# EUROPÄISCHE PATENTSCHRIFT

(11) **EP 3 534 709 B1**
(45) Veröffentlichungstag und Bekanntmachung des Hinweises auf die Patenterteilung: **16.06.2021**
(21) Anmeldenummer: 17797305.4
(22) Anmeldetag: 06.11.2017
(51) Int. Cl.: A01P 7/00, A01P 5/00, A01N 43/50, A01N 43/56, A01N 43/28, C07D 231/14, C07D 401/04, C07D 403/12, C07D 405/12, C07D 409/12, C07D 413/12

(54) **SUBSTITUIERTE SULFONYLAMIDE ZUR BEKÄMPFUNG TIERISCHER SCHÄDLINGE**
SUBSTITUTED SULFONYLAMIDE FOR COMBATING ANIMAL PESTS
SULFONYLAMIDES SUBSTITUÉS POUR LA LUTTE CONTRE LES RAVAGEURS

(30) Priorität: 07.11.2016 EP 16197465
(43) Veröffentlichungstag der Anmeldung: 11.09.2019
(73) Patentinhaber: Bayer Aktiengesellschaft, 51373 Leverkusen (DE); Bayer CropScience Aktiengesellschaft, 40789 Monheim am Rhein (DE)
(72) Erfinder: FÜßLEIN, Martin, 40225 Düsseldorf (DE); WROBLOWSKY, Heinz-Jürgen, 40764 Langenfeld (DE); KÜBBELER, Susanne, 40589 Düsseldorf (DE); HAGER, Dominik, 40789 Monheim (DE); KAUSCH-BUSIES, Nina, 51467 Bergisch Gladbach (DE); ANDREE, Roland, 40764 Langenfeld (DE); JANSEN, Johannes-Rudolf, 40789 Monheim (DE); SCHWARZ, Hans-Georg, 46282 Dorsten (DE); PORTZ, Daniela, 52391 Vettweiß (DE); ILG, Kerstin, 50670 Köln (DE); MALSAM, Olga, 51503 Rösrath (DE); EILMUS, Sascha, 42799 Leichlingen (DE); LÖSEL, Peter, 51371 Leverkusen (DE); HERRMANN, Stefan, 40764 Langenfeld (DE); BECKER, Angela, 06650 Opio (FR); VOERSTE, Arnd, 50674 Köln (DE); GÖRGENS, Ulrich, 40882 Ratingen (DE); LISHCHYNSKYI, Anton, 63225 Langen (DE); TURBERG, Andreas, 42781 Haan (DE)
(74) Vertreter: BIP Patents
(86) Internationale Anmeldenummer: PCT/EP2017/078280
(87) Internationale Veröffentlichungsnummer: WO 2018/083288

(56) Entgegenhaltungen:
- WO-A1-03/040107
- WO-A1-2015/169776
- WO-A2-2005/099705
- WO-A2-2010/129500

## Beschreibung

Die vorliegende Anmeldung betrifft die nicht-therapeutische Verwendung substituierter Sulfonylamide zur Bekämpfung von tierischen Schädlingen, insbesondere Nematoden, ein Mittel enthaltend substituierte Sulfonylamide zur Bekämpfung von tierischen Schädlingen, ein nicht-therapeutisches Verfahren zur Bekämpfung von tierischen Schädlingen und eine agrochemische Formulierung enthaltend die substituierten Sulfonylamide. Ebenfalls betrifft die Erfindung neue substituierte Sulfonylamide.

In der Literatur sind Sulfonylamide und deren Eignung als Wirkstoffe beispielsweise in den Patentanmeldungen WO 2005/099705, WO 2003/040107, WO 2014/077285 und WO 2014/023367 beschrieben. Darüber hinaus ist z.B. aus den Dokumenten WO 2010/129500, WO 2012/054233, WO 2013/055584, WO 2014/109933, WO 2015/007668, WO 2015/011082 und WO 2015/169776 bekannt, dass bestimmte Sulfonylamide als Nematizide verwendet werden können.

Aus der EP 2092824 sind zusätzlich auch Sulfonylamide bekannt, die als Insektizide verwendet werden können.

Moderne Nematizide und Insektizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen gleichwertigen oder zumindest in Bezug auf einzelne Aspekte verbesserten Eigenschaften.

Aufgabe der vorliegenden Erfindung war es, Verbindungen zur nicht-therapeutische Verwendung zur Bekämpfung von tierischen Schädlingen bereitzustellen, durch die das Spektrum der Schädlingsbekämpfungsmittel unter verschiedenen Aspekten ergänzt wird.

Gelöst wird die Aufgabe, sowie weitere nicht explizit genannte Aufgaben, die aus den hierin diskutierten Zusammenhängen ableitbar oder erschließbar sind, durch die nicht-therapeutische Verwendung einer Verbindung der Formel (I) 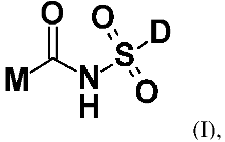 in der
M für einen Rest ausgewählt aus den Formeln (IIa-IIc) steht: 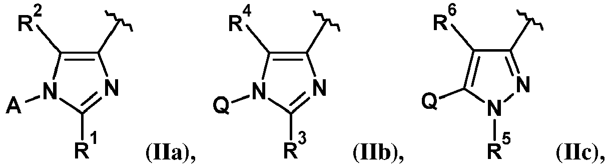 wobei
(Ausgestaltung 2-1)
R¹ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Al-koxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
   Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Al-koxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
   R² Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Al-koxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino ist,
   R³ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Al-koxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
      Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Al-koxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
      R⁴ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Al-koxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
      R⁵ Wasserstoff, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Al-koxy-(C₁-C₆)alkyl, (C₁-C₆)Alkylthio-(C₁-C₆)alkyl, Halogen(C₁-C₆)Alkylthio-(C₁-C₆)alkyl, (C₁-C₆)Al-kylsulfinyl-(C₁-C₆)alkyl, (C₁-C₆)Alkylsulfonyl-(C₁-C₆)alkyl, Halogen(C₁-C₆)Alkylsulfinyl-(C₁-C₆)alkyl oder Halogen(C₁-C₆)Alkylsulfonyl-(C₁-C₆)alkyl ist,
      R⁶ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Al-koxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
      A Halogen(C₁-C₆)alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C3)Alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Halogen(C₃-C₆)alkenyl oder
   ein gegebenenfalls einfach oder mehrfach, gleich oder verschieden substituierter Benzyl Rest ist, wobei der oder die Substituenten jeweils unabhängig voneinander ausgewählt sind aus:
      Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halo-genalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
      Q ein unsubstituierter oder mit einem oder mehreren Resten R⁷ substituierter Phenyl oder Pyridyl Rest ist,
   wobei der oder die Substituenten R⁷ jeweils unabhängig voneinander ausgewählt sind aus:
      Cyano, Halogen, Nitro, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino), Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
         und
      D ein unsubstituierter oder mit einem oder mehreren Resten R⁸ substituierter C₁-C₆-Alkyl, Phenyl, Thiophenyl, Isoxazolyl, Phenyl-(C₁-C₂)alkyl oder Benzdioxolyl Rest ist,
   wobei der oder die Substituenten R⁸ jeweils unabhängig voneinander ausgewählt sind aus:
      Cyano, Halogen, Nitro, Acetyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin bevorzugt ist eine Ausführungsform in der M für einen Rest ausgewählt aus den Formeln (IIa-IIc) steht und wobei (Ausgestaltung 2-2)
A Halogen(C₁-C₆)alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C3)Alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl,Halogen(C₁-C₆)alkylsulfonyl-(C₁-C₆)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
   und wobei R¹, R², R³, R⁴, R⁵, R⁶, R⁷, R⁸, Q und D definiert sind gemäß Ausgestaltung 2-1.
   Weiter besonders bevorzugt ist eine Ausführungsform (Ausgestaltung 3-1), in der M für einen Rest ausgewählt aus den Formeln (IIa-IIc) steht und wobei
   R¹ Wasserstoff, Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, oder unsubstituiertes, einfach oder mehrfach, gleich oder verschieden substituiertes Benzyl ist, wobei als Substituenten jeweils in Frage kommen: Cyano, Halogen, (C₁-C₄)Halogenalkyl, (C₁-C₄)Alkoxy, (C₁-C₄)Halogenalkoxy, (C₁-C₆)Alkylthio und/oder (C₁-C₆)Halogenalkylthio,
   R² Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
   R³ Wasserstoff, Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl oder(C₁-C₄)Alkylsulfonyl-(C₁-C₄)alkyl ist,
   R⁴ Wasserstoff, Cyano, Halogen, (C₁-C₄)Halogenalkyl, (C₁-C₄)Alkoxy, (C₁-C₄)Alkyl, (C₃-C₄)Cycloalkyl, (C₁-C₄)Halogenalkoxy, (C₁-C₆)Alkylthio oder (C₁-C₆)Halogenalkylthio ist,
   R⁵ Wasserstoff, Cyclopropyl, (C₁-C₄)Alkyl, Halogencyclopropyl, Halogen(C₁-C₃)Alkylcyclopropyl oder (C₁-C₄)Halogenalkyl ist,
   R⁶ Wasserstoff, Cyano, Halogen, (C₁-C₄)Halogenalkyl, (C₁-C₄)Alkoxy, (C₁-C₄)Halogenalkoxy, 4 (C₁-C₆)Alkylthio oder (C₁-C₆)Halogenalkylthio ist,
   A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder
   ein gegebenenfalls einfach oder mehrfach, gleich oder verschieden substituierter Benzyl Rest ist, wobei der oder die Substituenten jeweils unabhängig voneinander ausgewählt sind aus:
      Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkylthio und/oder (C₁-C₆)Halogenalkylthio,
      Q ein unsubstituierter oder mit einem oder mehreren Resten R⁷ substituierter Phenyl oder Pyridyl Rest ist,
   wobei der oder die Substituenten R⁷ jeweils unabhängig voneinander ausgewählt sind aus:
      Cyano, Halogen, (C₃-C₆)Cycloalkyl, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy,
         und
      D ein unsubstituierter oder mit einem oder mehreren Resten R⁸ substituierter C₁-C₆-Alkyl, Phenyl, Thiophenyl, Isoxazolyl, Benzyl oder Benzdioxolyl Rest ist,
   wobei der oder die Substituenten R⁸ jeweils unabhängig voneinander ausgewählt sind aus:
      Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin besonders bevorzugt ist eine Ausführungsform in der M für einen Rest ausgewählt aus den Formeln (IIa-IIc) steht und wobei (Ausgestaltung 3-2)
R⁴ Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkylthio und/oder (C₁-C₆)Halogenalkylthio,
   und wobei R¹, R², R³, R⁵, R⁶, R⁷, R⁸, Q und D definiert sind gemäß Ausgestaltung 3-1.

Weiter ganz besonders bevorzugt ist eine Ausführungsform (Ausgestaltung 4-1), in der M für einen Rest ausgewählt aus den Formeln (IIa-IIc) steht und wobei
R¹ Wasserstoff, Halogen, Cyclopropyl, (C₁-C₄)Alkyl, (C₁-C₄)Alkylthio, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen: Cyano, Halogen, (C₁-C₄)Halogenalkyl und/oder (C₁-C₄)Alkoxy,
R² Wasserstoff, Halogen, Cyano, (C₁-C₄)Alkyl, (C₁-C₄)Halogenalkyl ist,
R³ Wasserstoff, Halogen, Cyclopropyl, (C₁-C₄)Alkyl, Halogencyclopropyl oder(C₁-C₄)Halogenalkyl ist,
R⁴ Wasserstoff, Halogen, Cyano, (C₁-C₄)Alkyl, Cyclopropyl oder (C₁-C₄)Halogenalkyl ist,
R⁵ Wasserstoff, Cyclopropyl, (C₁-C₄)Alkyl oder Halogen(C₁-C₄)alkyl ist,
R⁶ Wasserstoff, Halogen, Cyano, (C₁-C₄)Alkyl oder (C₁-C₄)Halogenalkyl ist,
A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio,
   Q ein unsubstituierter oder mit einem oder mehreren Resten R⁷ substituierter Phenyl oder Pyridyl Rest ist,
wobei der oder die Substituenten R⁷ jeweils unabhängig voneinander ausgewählt sind aus:
   Cyano, Halogen, (C₃-C₆)Cycloalkyl, Halogen(C₃-C₆)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy
      und
   D ein unsubstituierter oder mit einem oder mehreren Resten R⁸ substituierter C₁-C₆-Alkyl, Phenyl, Thiophenyl, Isoxazolyl, Benzyl oder Benzdioxolyl Rest ist,
wobei der oder die Substituenten R⁸ jeweils unabhängig voneinander ausgewählt sind aus:
   Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₆)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin ganz besonders bevorzugt ist eine Ausführungsform in der M für einen Rest ausgewählt aus den Formeln (IIa-IIc) steht und wobei (Ausgestaltung 4-2)
R¹ Wasserstoff, Halogen, Cyclopropyl, (C₁-C₄)Alkyl, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen: Cyano, Halogen, (C₁-C₄)Halogenalkyl und/oder (C₁-C₄)Alkoxy,
R⁴ Wasserstoff, Halogen, Cyano, (C₁-C₄)Alkyl oder (C₁-C₄)Halogenalkyl ist,
A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio
   und wobei R², R³, R⁵, R⁶, R⁷, R⁸, Q und D definiert sind gemäß Ausgestaltung 4-1.

Weiter insbesondere bevorzugt ist eine Ausführunsgform (Ausgestaltung 5-1), in der M für einen Rest ausgewählt aus den Formeln (IIa-IIc) steht und wobei
R¹ Wasserstoff, Chlor, Brom, Methyl, Benzyl, 2-Chlorbenzyl, 3-Chlorbenzyl, 4-Chlorbenzyl, Trifluormethyl, Methylthio oder Isopropylthio ist,
R² Wasserstoff, Cyano, Chlor, Brom oder Iod ist,
R³ Wasserstoff, Chlor, Methyl, Isopropyl, Ethyl oder Brom ist,
R⁴ Wasserstoff, Chlor, Brom, Iod, Fluor, Difluormethyl, Isopropyl oder Cyclopropyl ist,
R⁵ Methyl oder 2,2,2-Trifluorethyl ist und
R⁶ Wasserstoff oder Chlor ist,
A ein Rest ausgewählt aus den Resten der Formeln (III1-III19) ist: 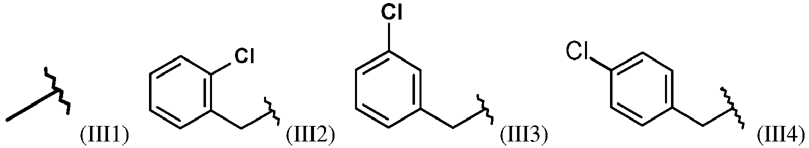 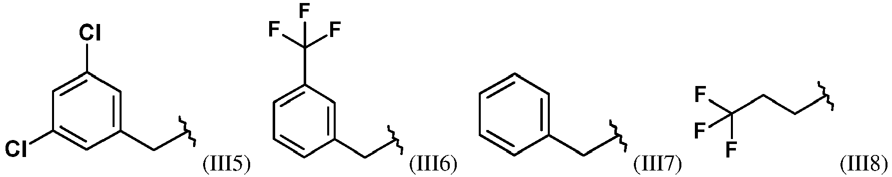 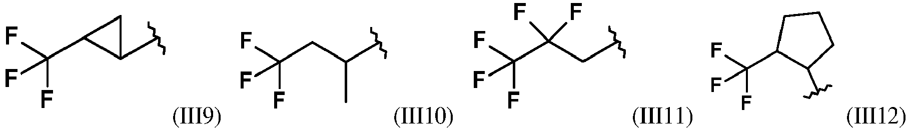 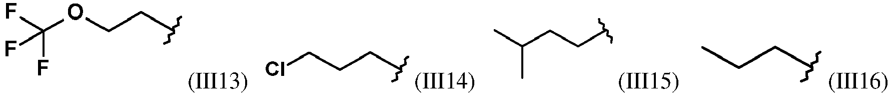 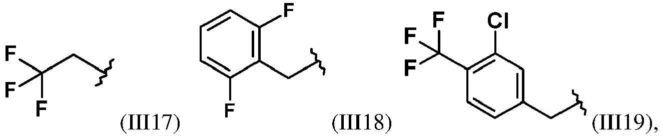
Q ein Rest ausgewählt aus den Resten der Formeln (IV1-IV40) ist: 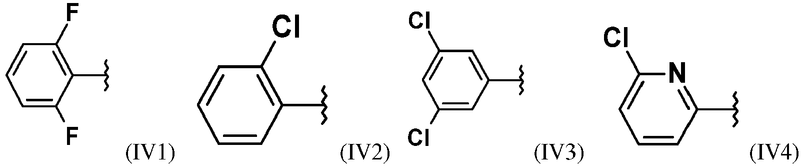 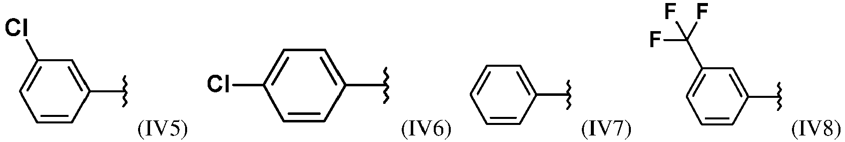 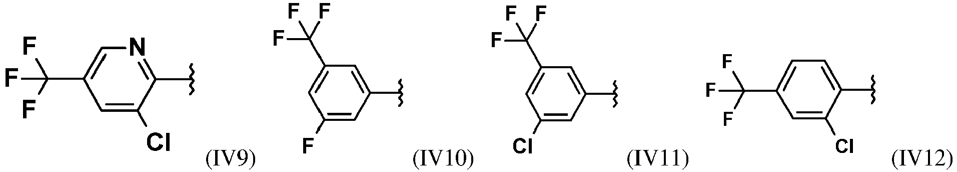 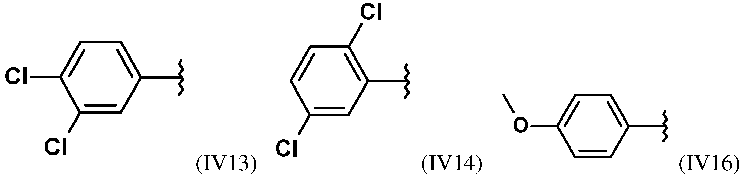 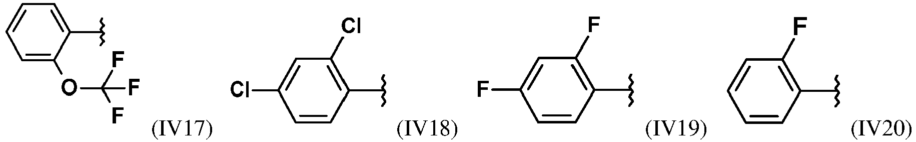 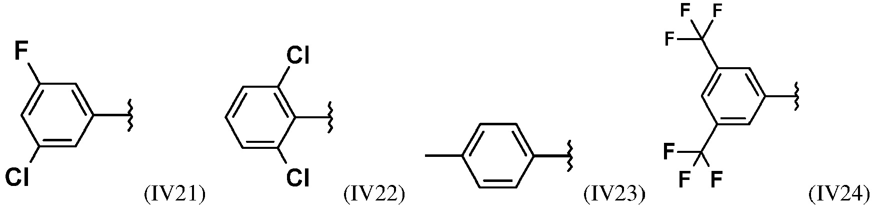 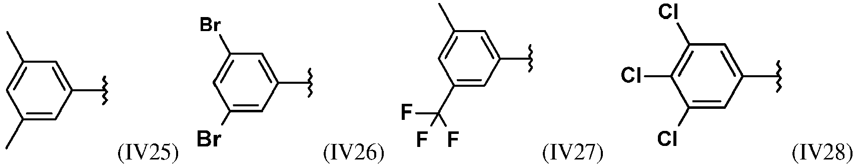 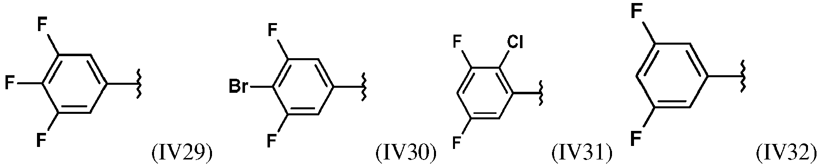 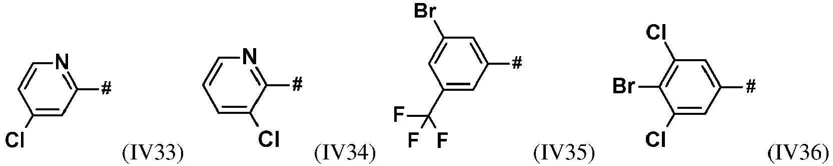 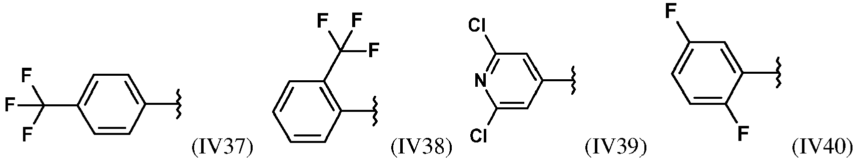 und
D ein Rest ausgewählt aus den Resten der Formeln (V1-V61) ist:

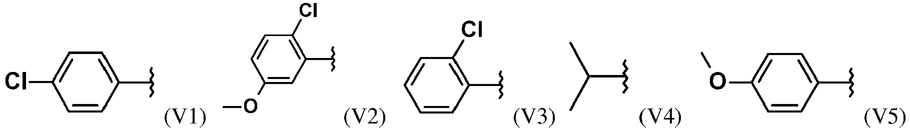

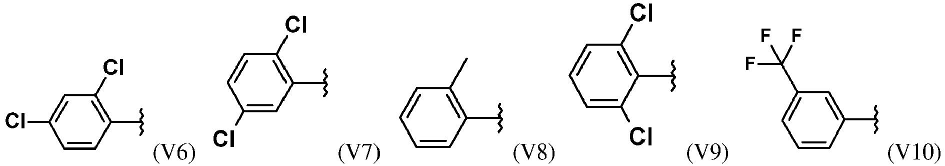

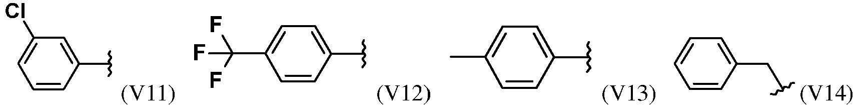

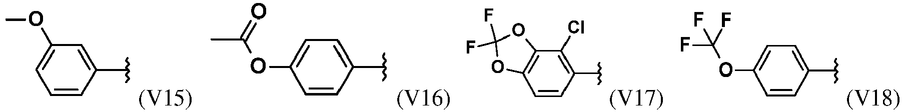

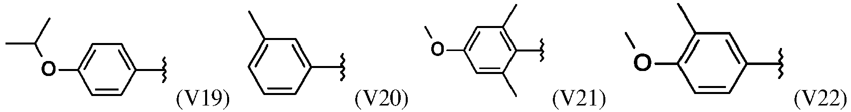

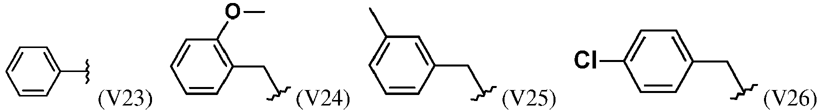

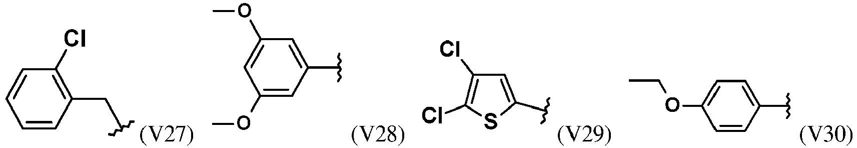

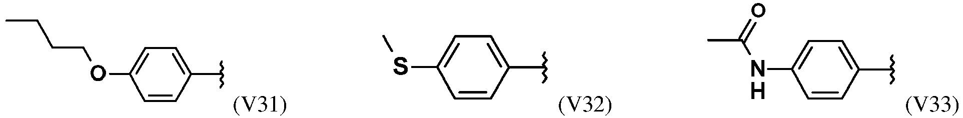

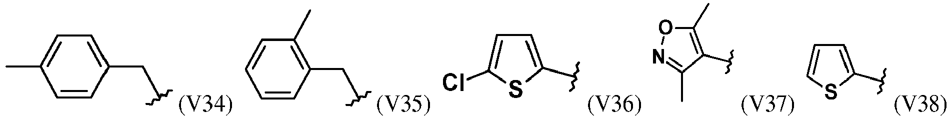

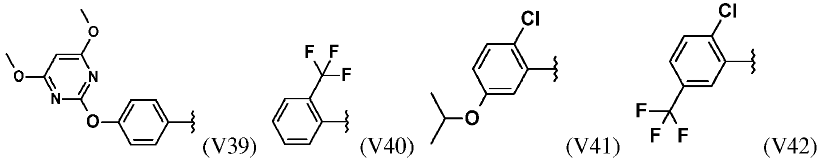

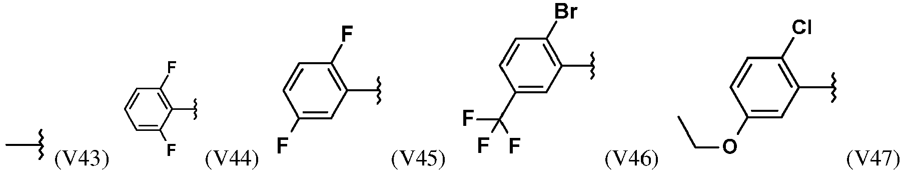

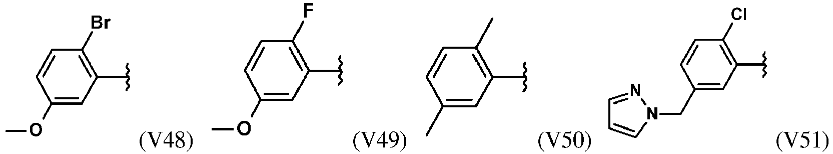

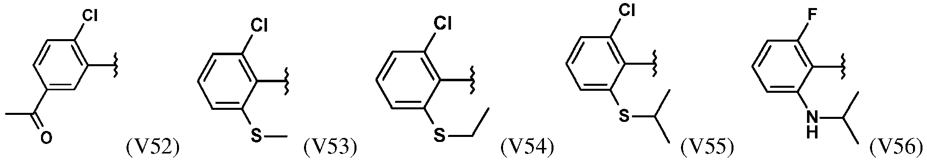

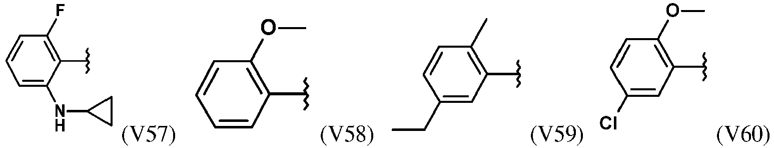

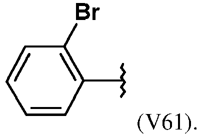

Weiterhin ganz besonders bevorzugt ist eine Ausführungsform in der M für einen Rest ausgewählt aus den Formeln (IIa-IIc) steht und wobei (Ausgestaltung 5-2)
R¹ Wasserstoff, Chlor, Brom, Methyl, Benzyl, 2-Chlorbenzyl, 3-Chlorbenzyl oder 4-Chlorbenzyl ist,
R² Wasserstoff, Cyano, Chlor, Brom oder Iod ist,
R³ Wasserstoff, Chlor, Methyl, Isopropyl oder Ethyl ist,
R⁴ Wasserstoff, Chlor, Brom oder Iod ist,
R⁵ Methyl ist und
R⁶ Wasserstoff oder Chlor ist,
A ein Rest ausgewählt aus den Resten der Formeln (III1-III18) ist: 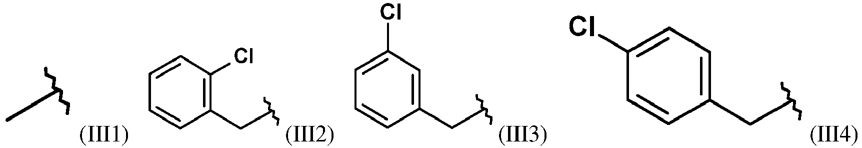 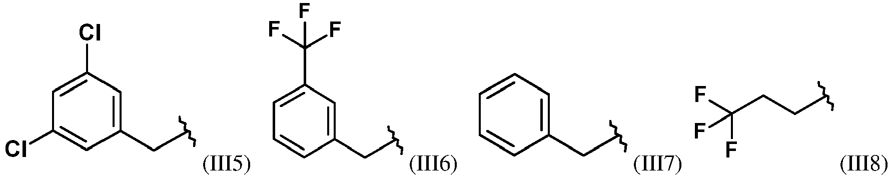 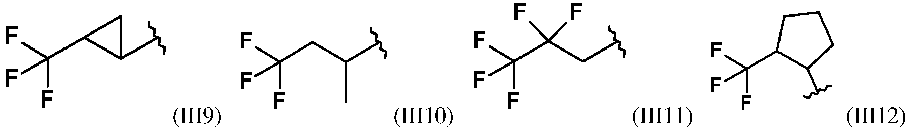 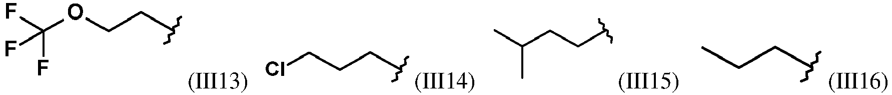 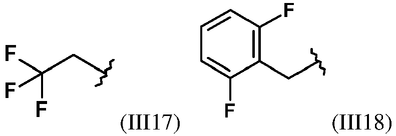
Q ein Rest ausgewählt aus den Resten der Formeln (IV1-IV36) ist: 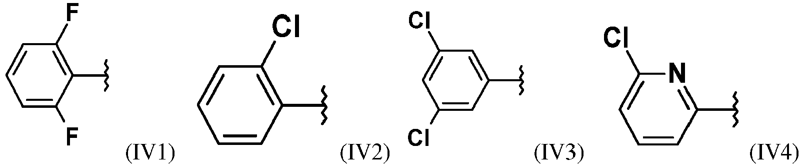 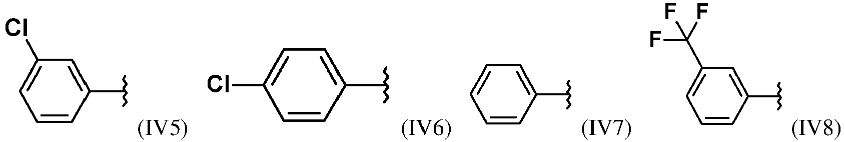 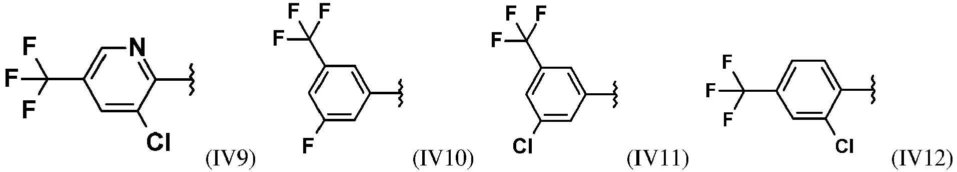 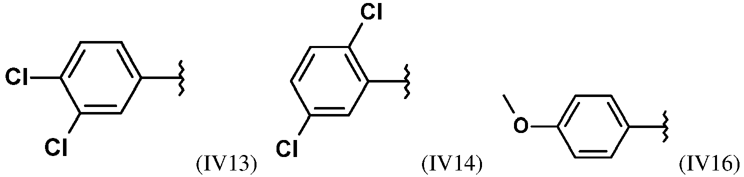 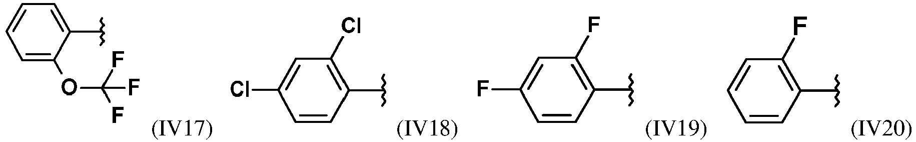 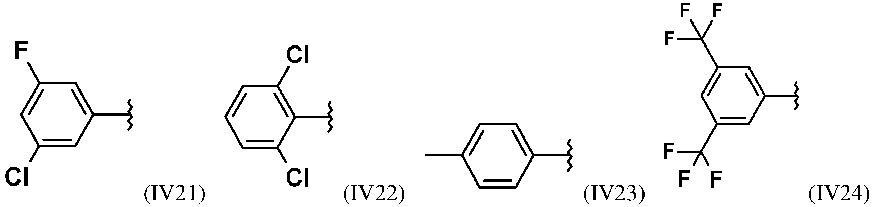 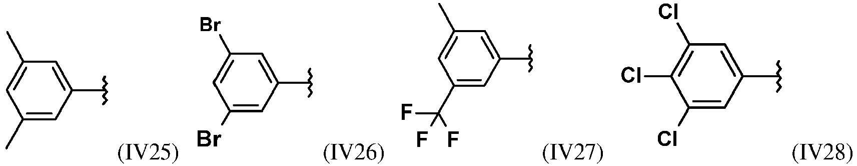 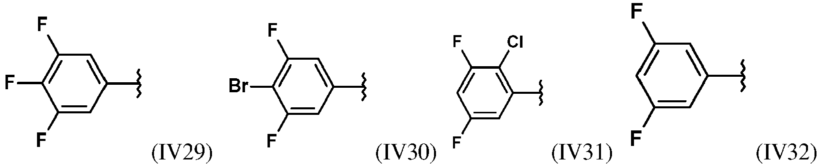 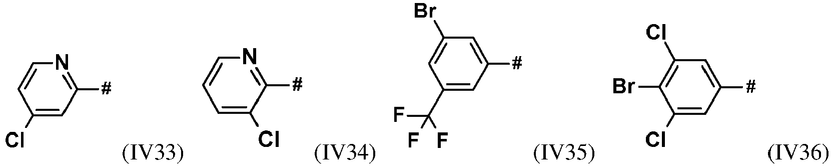 und
D ein Rest ausgewählt aus den Resten der Formeln (V1-V57) ist:

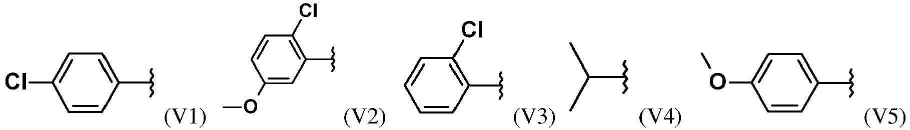

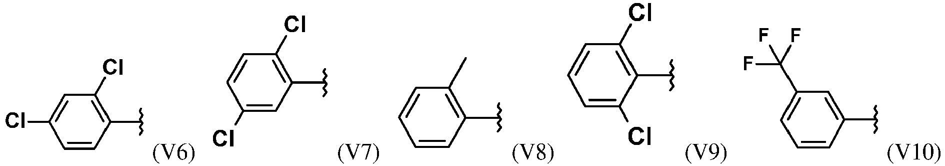

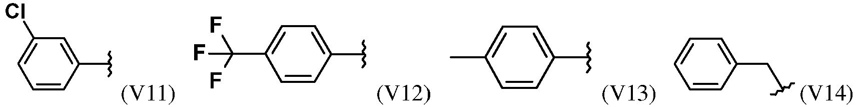

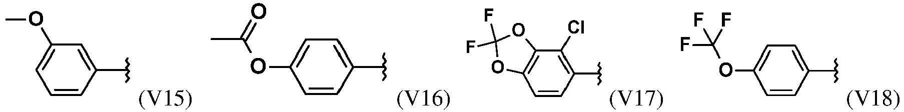

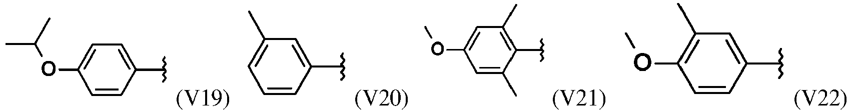

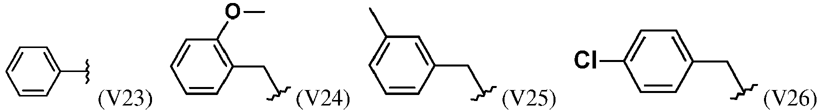

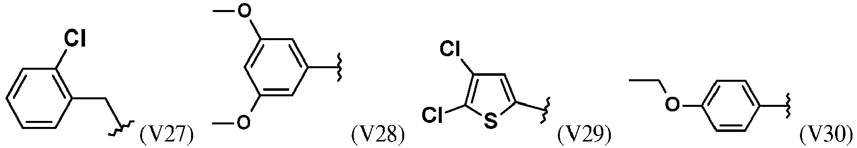

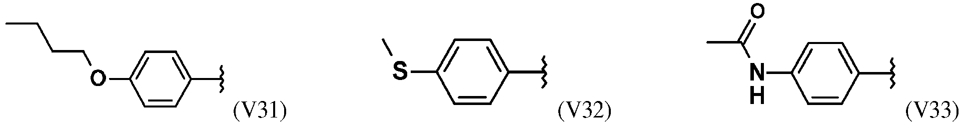

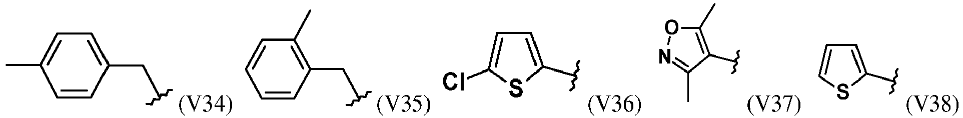

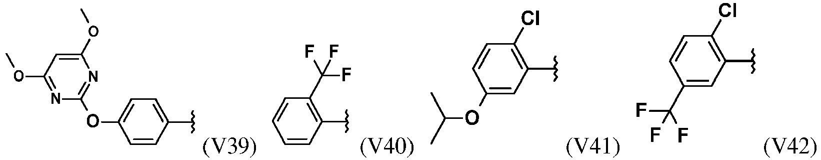

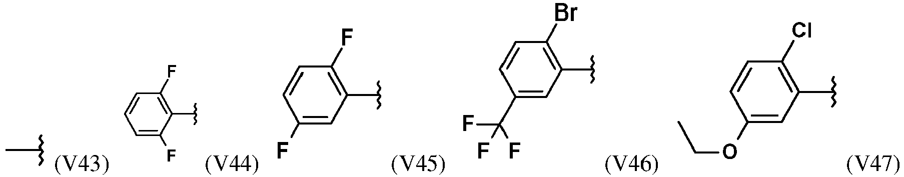

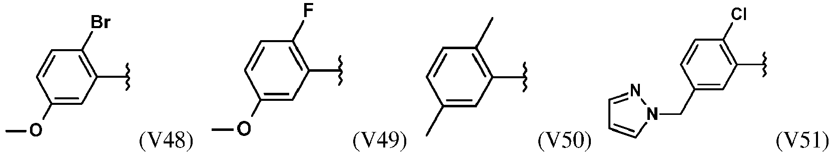

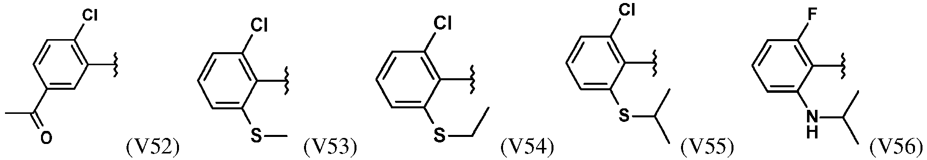

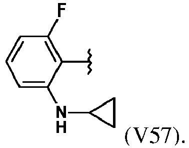

Im Folgenden steht *Ausgestaltung (x)* gleichbedeutend mit *Ausgestaltung (x-1) oder (x-2)* mit x = 1, 2, 3, 4 oder 5.

In einer bevorzugten Ausführungsform der Erfindung steht in Formeln (I) M für einen Rest ausgewählt aus den Formeln IIa oder IIb, wobei die Reste R¹, R², A, R³, R⁴, R⁷, R⁸, Q und D die in der Ausgestaltung (2) oder der Ausgestaltung (3) oder der Ausgestaltung (4) oder der Ausgestaltung (5) beschriebenen Bedeutungen haben.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸, Q und D die oben beschriebenen Bedeutungen, insbesondere die in Ausgestaltung (3) oder Ausgestaltung (4) oder Ausgestaltung (5) beschriebenen Bedeutungen haben und
A die in Ausgestaltung (2) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸, Q und D die oben beschriebenen Bedeutungen, insbesondere die in Ausgestaltung (2) oder Ausgestaltung (4) oder Ausgestaltung (5) beschriebenen Bedeutungen haben und
A die in Ausgestaltung (3) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸, Q und D die oben beschriebenen Bedeutungen, insbesondere die in Ausgestaltung (2) oder Ausgestaltung (3) oder Ausgestaltung (5) beschriebenen Bedeutungen haben und
A die in Ausgestaltung (4) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸, Q und D die oben beschriebenen Bedeutungen, insbesondere die in Ausgestaltung (2) oder Ausgestaltung (3) oder Ausgestaltung (4) beschriebenen Bedeutungen haben und
A die in Ausgestaltung (5) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁸, A und D die oben beschriebenen Bedeutungen, insbesondere die in Ausgestaltung (3) oder Ausgestaltung (4) oder Ausgestaltung (5) beschriebenen Bedeutungen haben und
Q die in Ausgestaltung (2) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁸, A und D die oben beschriebenen Bedeutungen, insbesondere die in Ausgestaltung (2) oder Ausgestaltung (4) oder Ausgestaltung (5) beschriebenen Bedeutungen haben und
Q und R⁷ die in Ausgestaltung (3) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁸, A und D die oben beschriebenen Bedeutungen, insbesondere die in Ausgestaltung (2) oder Ausgestaltung (3) oder Ausgestaltung (5) beschriebenen Bedeutungen haben und
Q und R⁷ die in Ausgestaltung (4) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁸, A und D die oben beschriebenen Bedeutungen, insbesondere die in Ausgestaltung (2) oder Ausgestaltung (3) oder Ausgestaltung (4) beschriebenen Bedeutungen haben und
Q und R⁷ die in Ausgestaltung (5) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (3) oder Ausgestaltung (4) oder Ausgestaltung (5) beschriebenen Bedeutungen haben und
D und R⁸ die in Ausgestaltung (2) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (2) oder Ausgestaltung (4) oder Ausgestaltung (5) beschriebenen Bedeutungen haben und
D und R⁸ die in Ausgestaltung (3) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (2) oder Ausgestaltung (3) oder Ausgestaltung (5) beschriebenen Bedeutungen haben und
D und R⁸ die in Ausgestaltung (4) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (2) oder Ausgestaltung (3) oder Ausgestaltung (4) beschriebenen Bedeutungen haben und
D und R⁸ die in Ausgestaltung (5) beschriebenen Bedeutungen hat.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸ Q und A die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
D für einen unsubstituierten oder ein- oder zweifach durch R⁸ substituierten Phenylrest steht.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, R⁸ Q und A die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
D für einen ein- oder zweifach durch R⁸ substituierten Phenylrest steht.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
D für einen unsubstituierten oder ein- oder zweifach durch R⁸ substituierten Phenylrest steht, wobei
R⁸ ausgewählt ist aus Halogen, (C₁-C₄)Alkyl, (C₁-C₄)Alkoxy, (C₁-C₄)Halogenalkyl oder (C₁-C₄)Alkylthio, besonders bevorzugt aus Fluor, Brom, Chlor, Methyl, Trifluormethyl, Methoxy, Ethoxy, Isopropoxy oder Methylthio.

In einer weiteren bevorzugten Ausführungsform betrifft die Erfindung die nicht-therapeutische Verwendung von Verbindungen der Formel (I), wobei M, R¹, R², R³, R⁴, R⁵, R⁶, R⁷, Q und A die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
D für einen ein- oder zweifach durch R⁸ substituierten Phenylrest steht, wobei
R⁸ ausgewählt ist aus Halogen, (C₁-C₄)Alkyl, (C₁-C₄)Alkoxy, (C₁-C₄)Halogenalkyl oder (C₁-C₄)Alkylthio, besonders bevorzugt aus Fluor, Brom, Chlor, Methyl, Trifluormethyl, Methoxy, Ethoxy, Isopropoxy oder Methylthio.

Weiterhin bevorzugt sind Verbindungen der Formeln (Ia) oder (Ib) oder (Ic), besonders bevorzugt (Ia) oder (Ib), 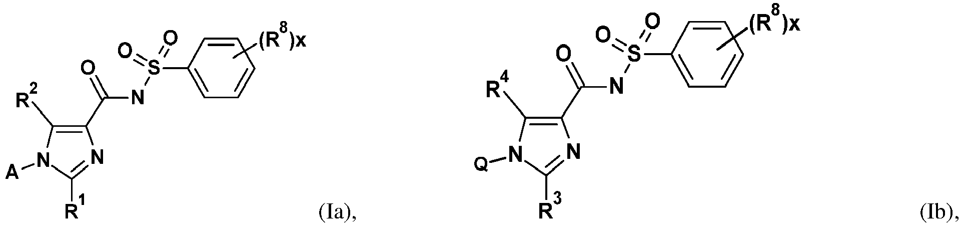 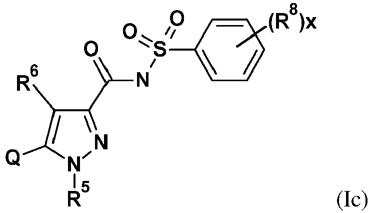 wobei R¹, R², R³, R⁴, R⁵, R⁶, R⁷, A, Q und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben
und x für 1 oder 2 steht.

Besonders bevorzugt sind Verbindungen der Formel (Ia) bei welchen gilt:
A steht für (C₁-C₄)Halogenalkyl oder (C₁-C₄)Halogenalkylcyclopropyl, bevorzugt für (C₁-C₄)Fluoralkyl oder (C₁-C₄)Fluoralkylcyclopropyl, ganz besonders bevorzugt für 3,3,3-Trifluorpropyl oder 2-Trifluormethylcyclopropyl,
x steht für 1 oder 2 und
R¹, R² und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Weiterhin besonders bevorzugt sind Verbindungen der Formel (Ia) bei welchen gilt:
R¹ steht für Halogen, besonders bevorzugt für Chlor oder Brom,
R² steht für Halogen, besonders bevorzugt für Chlor, Brom oder Iod,
x steht für 1 oder 2 und

A und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Besonders bevorzugt sind Verbindungen der Formel (Ib) bei welchen gilt:
Q steht für einen unsubstituierten oder ein- oder zweifach durch R⁷ substituierten Phenylrest, wobei
R⁷ ausgewählt ist aus Halogen, (C₁-C₄)Alkyl, (C₁-C₄)Alkoxy oder (C₁-C₄)Halogenalkyl, besonders bevorzugt aus Fluor, Brom, Chlor oder Trifluormethyl,
x steht für 1 oder 2 und
R³, R⁴ und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Weiterhin besonders bevorzugt sind Verbindungen der Formel (Ib) bei welchen gilt:
Q steht für einen unsubstituierten oder ein- oder zweifach durch R⁷ substituierten Phenylrest,
x steht für 1 oder 2 und
R³, R⁴, R⁷ und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Weiterhin besonders bevorzugt sind Verbindungen der Formel (Ib) bei welchen gilt:
R³ steht für Wasserstoff, Halogen oder (C₁-C₄)Alkyl, besonders bevorzugt für Wasserstoff, Chlor, Methyl, Isopropyl oder Ethyl,
R⁴ steht für Wasserstoff oder Halogen, besonders bevorzugt für Wasserstoff, Chlor, Brom oder Iod,
x steht für 0, 1 oder 2 und
Q und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Besonders bevorzugt sind Verbindungen der Formel (Ic) bei welchen gilt:
Q steht für einen unsubstituierten oder ein- oder zweifach durch R⁷ substituierten Phenylrest, wobei
R⁷ für Halogen, (C₁-C₄)Alkyl, (C₁-C₄)Alkoxy oder (C₁-C₄)Halogenalkyl, besonders bevorzugt für Fluor, Brom, Chlor oder Trifluormethyl steht,
x steht für 1 oder 2 und
R⁵, R⁶ und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Weiterhin besonders bevorzugt sind Verbindungen der Formel (Ic) bei welchen gilt:
Q steht für einen unsubstituierten oder ein- oder zweifach durch R⁷ substituierten Phenylrest,
x steht für 1 oder 2 und
R³, R⁴, R⁷ und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen.

Weiterhin besonders bevorzugt sind Verbindungen der Formel (Ic) bei welchen gilt:
R⁵ steht für (C₁-C₄)Alkyl, besonders bevorzugt für Methyl,
R⁶ steht für Wasserstoff oder Halogen, besonders bevorzugt für Wasserstoff oder Chlor,
x steht für 1 oder 2 und
Q und R⁸ haben die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

Im Folgenden umfasst der Begriff *Formel (I)* auch die speziellen Ausführungsformeln Formel (Ia), Formel (Ib) und Formel (Ic).

In einer weiteren bevorzugten Ausführungsform betrifft die Erfindung Verbindungen der Formel (I), wobei M, R², R³, R⁴, R⁵, R⁶, R⁷, A, Q, D und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
R¹ Hydroxy, Cyano, Carboxy, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₅)cycloalkyl, Halogen(C₃-C₅)cycloalkyl, Methyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alko-xyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, Halogen(C₁-C₆)Alkylthio-(C₁-C₆)alkyl, Halogen(C₁-C₆)Alkylsulfinyl-(C₁-C₆)alkyl, Halogen(C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Alkylsulfoximino, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino oder
gegebenenfalls einfach oder mehrfach, gleich oder verschieden substituiertes Aryl-(C₂-C₆)alkyl oder Hetaryl-(C₁-C₆)alkyl ist, wobei, im Fall von Hetaryl, gegebenenfalls mindestens eine Carbonylgruppe enthalten sein kann und wobei der oder die Substituenten jeweils unabhängig voneinander ausgewählt sind aus:
Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothio-carbonyl, (C₃-C₈)Cycloalkylamino und/oder (C₁-C₆)Alkylcarbonylamino.

In einer weiteren bevorzugten Ausführungsform betrifft die Erfindung Verbindungen der Formel (I), wobei M, R², R³, R⁴, R⁵, R⁶, R⁷, A, Q, D und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
R¹ Cyano, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₅)cycloalkyl, Halogen(C₃-C₅)cycloalkylMethyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist.

In einer weiteren bevorzugten Ausführungsform betrifft die Erfindung Verbindungen der Formel (I), wobei M, R², R³, R⁴, R⁵, R⁶, R⁷, A, Q, D und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
R¹ Cyano, Halogen, Cyclopropyl, Methyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl ist.

In einer weiteren bevorzugten Ausführungsform betrifft die Erfindung Verbindungen der Formel (I), wobei M, R², R³, R⁴, R⁵, R⁶, R⁷, A, Q, D und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
R¹ Halogen, Cyclopropyl, Methyl, (C₁-C₄)Alkylthio, Halogencyclopropyl oder (C₁-C₄)Halogenalkyl ist. In einer weiteren bevorzugten Ausführungsform betrifft die Erfindung Verbindungen der Formel (I), wobei M, R², R³, R⁴, R⁵, R⁶, R⁷, A, Q, D und R⁸ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben und
R¹ Chlor, Brom, Methyl, Trifluormethyl, Methylthio oder Isopropylthio ist.

In einer bevorzugten Ausführungsform der Erfindung werden die Verbindungen der Formel (I) gemäß den vorstehend beschriebenen Ausgestaltungen und bevorzugten Ausführungsformen, zur Bekämpfung von Nematoden verwendet.

Gemäß einer weiteren bevorzugten Ausführungsform der Erfindung ist die nicht-therapeutische Verwendung einer Verbindung gemäß Formel (I) gemäß den vorstehend beschriebenen Ausgestaltungen und bevorzugten Ausführungsformen, zum Schutz des Vermehrungsmaterials von Pflanzen vorgesehen.

Gegenstand der Erfindung ist auch ein Mittel, mit einem Gehalt von mindestens einer Verbindung gemäß Formel (I) gemäß den vorstehend beschriebenen Ausgestaltungen und bevorzugten Ausführungsformen, und üblichen Streckmitteln und/oder oberflächenaktiven Substanzen insbesondere zur Bekämpfung von tierischen Schädlingen, bevorzugt von Nematoden.

Ein weiterer Gegenstand der Erfindung ist ein Verfahren zur Bekämpfung von tierischen Schädlingen, bevorzugt Nematoden, bei dem man wenigstens eine Verbindung gemäß Formel (I) gemäß den vorstehend beschriebenen Ausgestaltungen und bevorzugten Ausführungsformen, oder ein erfindungsgemäßes Mittel auf die tierischen Schädlinge, bevorzugt Nematoden und/oder ihren Lebensraum einwirken lässt.

Gemäß der Ausführungsfrom des Verfahrens ist die chirurgische, therapeutische und diagnostische Behandlung des menschlichen oder tierischen Körpers ausgeschlossen.

Noch weiterer Gegenstand der Erfindung ist eine agrochemische Formulierung enthaltend wenigstens eine Verbindung gemäß Formel (I) gemäß den vorstehend beschriebenen Ausgestaltungen und bevorzugten Ausführungsformen, in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

Eine bevorzugte Asugestaltung der erfindungsgemäßen Formulierung enthält zusätzlich einen weiteren agrochemischen Wirkstoff.

Ebenfalls Gegenstand der Erfindung sind Verbindungen der Formel (I') 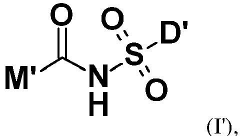 in der
M' für einen Rest ausgewählt aus den Formeln (IIa'-IIc') steht: 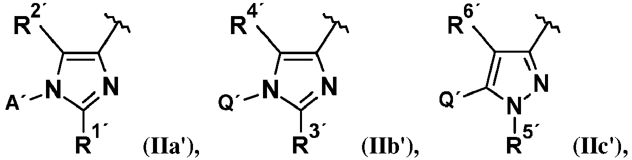 wobei
(Ausgestaltung 2'-1)
R^{1'} Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
   Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
   R^{2'} Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
   R^{3'} Cyano, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
      Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
      R^{4'} Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
      R⁵ (C₃-C₅)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkylthio-(C₁-C₆)alkyl, Halogen(C₁-C₆)alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylsulfinyl-(C₁-C₆)alkyl oder Halogen(C₁-C₆)alkylsulfonyl-(C₁-C₆)alkyl ist,
      R⁶ Wasserstoff, Cyano, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
      A' Halogen(C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C₃)alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Halogen(C₃-C₆)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
   Q' ein unsubstituierter oder mit einem oder mehreren Resten R^{7'} substituierter Phenyl oder Pyridyl Rest ist,
wobei der oder die Substituenten R^{7'} jeweils unabhängig voneinander ausgewählt sind aus:
   Cyano, Halogen, Nitro, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino), Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
      und
   D' ein unsubstituierter oder mit einem oder mehreren Resten R^{8'} substituierter C₁-C₆-Alkyl, Phenyl, Thiophenyl, Isoxazolyl, Phenyl-(C₁-C₂)alkyl oder Benzdioxolyl Rest ist,
wobei der oder die Substituenten R^{8'} jeweils unabhängig voneinander ausgewählt sind aus:
   Cyano, Halogen, Nitro, Acetyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin bevorzugt ist eine Ausführungsform in der M' für einen Rest ausgewählt aus den Formeln (IIa'-IIc') steht und wobei (Ausgestaltung 2'-2)
A' Halogen(C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C3)alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₅)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
   und wobei R^{1'}, R^{2'}, R^{3'}, R^{4'}, R^{5'}, R^{6'}, R^{7'}, R^{8'}, Q' und D' definiert sind gemäß gemäß Ausgestaltung 2'-1.

Weiter besonders bevorzugt ist eine Ausführungsform (Ausgestaltung 3'-1), in der M' für einen Rest ausgewählt aus den Formeln (IIa'-IIc') steht und wobei
R^{1'} Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, oder unsubstituiertes, einfach oder mehrfach, gleich oder verschieden substituiertes Benzyl ist, wobei als Substituenten jeweils in Frage kommen:
Cyano, Halogen, (C₁-C₄)Halogenalkyl, (C₁-C₄)Alkoxy, (C₁-C₄)Halogenalkoxy, (C₁-C₆)Alkylthio und/oder (C₁-C₆)Halogenalkylthio,
R^{2'} Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
R^{3'} Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, oder (C₁-C₄)Alkylsulfonyl-(C₁-C₄)alkyl ist,
R^{4'} Wasserstoff, Cyano, Halogen, (C₁-C₄)Halogenalkyl, (C₁-C₄)Alkoxy, (C₁-C₄)Alkyl, (C₃-C₄)Cycloalkyl, (C₁-C₄)Halogenalkoxy, (C₁-C₆)Alkylthio oder (C₁-C₆)Halogenalkylthio ist,
R⁵ Cyclopropyl, (C₁-C₄)Alkyl, Halogencyclopropyl, Halogen(C₁-C₃)Alkylcyclopropyl oder (C₁-C₄)Halogenalkyl ist,
R⁶ Wasserstoff, Cyano, Halogen, (C₁-C₄)Halogenalkyl, (C₁-C₄)Alkoxy, (C₁-C₄)Halogenalkoxy, 4 (C₁-C₆)Alkylthio oder (C₁-C₆)Halogenalkylthio ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C₃)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio und/oder (C₁-C₆)Halogenalkylthio,
   Q' ein unsubstituierter oder mit einem oder mehreren Resten R^{7'} substituierter Phenyl oder Pyridyl Rest ist,
wobei der oder die Substituenten R^{7'} jeweils unabhängig voneinander ausgewählt sind aus:
   Cyano, Halogen, (C₃-C₆)Cycloalkyl, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy,
      und
   D' ein unsubstituierter oder mit einem oder mehreren Resten R^{8'} substituierter C₁-C₆-Alkyl, Phenyl, Thiophenyl, Isoxazolyl, Benzyl oder Benzdioxolyl Rest ist,
wobei der oder die Substituenten R^{8'} jeweils unabhängig voneinander ausgewählt sind aus:
   Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin besonders bevorzugt ist eine Ausführungsform in der M' für einen Rest ausgewählt aus den Formeln (IIa'-IIc') steht und wobei (Ausgestaltung 3'-2)
R^{4'} Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C₃)alkoxy-(C₁-C₄)alkyl, (C₁-C₄)Alkylthio-(C₁-C₄)alkyl, (C₁-C₄)Alkylsulfinyl-(C₁-C₄)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio und/oder (C₁-C₆)Halogenalkylthio,
   und wobei R^{1'}, R^{2'}, R^{3'}, R^{5'}, R^{6'}, R^{7'}, R^{8'} Q' und D' definiert sind gemäß gemäß Ausgestaltung 3'-1.

Weiter ganz besonders bevorzugt ist eine Ausführungsform (Ausgestaltung 4'-1), in der M' für einen Rest ausgewählt aus den Formeln (IIa'-IIc') steht und wobei
R^{1'} Halogen, Cyclopropyl, (C₁-C₄)Alkyl, (C₁-C₄)Alkylthio, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen:Cyano, Halogen, (C₁-C₄)Halogenalkyl und/oder (C₁-C₄)Alkoxy,
R^{2'} Wasserstoff, Halogen, Cyano, (C₁-C₄)Alkyl, (C₁-C₄)Halogenalkyl ist,
R^{3'} Halogen, Cyclopropyl, (C₁-C₄)Alkyl, Halogencyclopropyl oder (C₁-C₄)Halogenalkyl ist,
R^{4'} Wasserstoff, Halogen, Cyano, (C₁-C₄)Alkyl, Cyclopropyl, oder (C₁-C₄)Halogenalkyl ist,
R^{5'} Cyclopropyl, (C₁-C₄)Alkyl oder Halogen(C₁-C₄)alkyl ist,
R⁶' Wasserstoff, Halogen, Cyano, (C₁-C₄)Alkyl oder (C₁-C₄)Halogenalkyl ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C₃)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio,
   Q' ein unsubstituierter oder mit einem oder mehreren Resten R^{7'} substituierter Phenyl oder Pyridyl Rest ist,
wobei der oder die Substituenten R^{7'} jeweils unabhängig voneinander ausgewählt sind aus:
   Cyano, Halogen, (C₃-C₆)Cycloalkyl, Halogen(C₃-C₆)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy
      und
   D' ein unsubstituierter oder mit einem oder mehreren Resten R^{8'} substituierter C₁-C₆-Alkyl, Phenyl, Thiophenyl, Isoxazolyl, Benzyl oder Benzdioxolyl Rest ist,
wobei der oder die Substituenten R^{8'} jeweils unabhängig voneinander ausgewählt sind aus:
   Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₆)Cycloalkylamino, (C₁-C₆)Alkylcarbonylamino, (1-Pyrazolyl)(C₁-C₃)alkyl und/oder (C₁-C₃)Alkoxypyrimidinyloxy.

Weiterhin besonders bevorzugt ist eine Ausführungsform in der M' für einen Rest ausgewählt aus den Formeln (IIa'-IIc') steht und wobei (Ausgestaltung 4'-2)
R^{1'} Halogen, Cyclopropyl, (C₁-C₄)Alkyl, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen:Cyano, Halogen, (C₁-C₄)Halogenalkyl und/oder (C₁-C₄)Alkoxy,
R^{2'} Wasserstoff, Halogen, Cyano, (C₁-C₄)Alkyl, (C₁-C₄)Halogenalkyl ist,
R^{3'} Halogen, Cyclopropyl, (C₁-C₄)Alkyl, Halogencyclopropyl oder (C₁-C₄)Halogenalkyl ist,
R^{4'} Wasserstoff, Halogen, Cyano, (C₁-C₄)Alkyl oder (C₁-C₄)Halogenalkyl ist,
R^{5'} Cyclopropyl, (C₁-C₄)Alkyl oder Halogen(C₁-C₄)alkyl ist,
R^{6'} Wasserstoff, Halogen, Cyano, (C₁-C₄)Alkyl oder (C₁-C₄)Halogenalkyl ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl oder
ein gegebenenfalls einfach oder mehrfach, gleich oder verschieden substituierter Benzyl Rest ist, wobei der oder die Substituenten jeweils unabhängig voneinander ausgewählt sind aus:
   Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio,
   und wobei R²', R³', R⁵', R⁶', R⁷', R⁸', Q' und D' definiert sind gemäß gemäß Ausgestaltung 4'-1.

Weiter insbesondere bevorzugt ist eine Ausführunsgform (Ausgestaltung 5'-1), in der M' für einen Rest ausgewählt aus den Formeln (IIa'-IIc') steht und wobei
R^{1'} Chlor, Brom, Methyl, Benzyl, 2-Chlorbenzyl, 3-Chlorbenzyl, 4-Chlorbenzyl, Trifluormethyl, Methylthio oder Isopropylthio ist,
R^{2'} Wasserstoff, Cyano, Chlor, Brom oder Iod ist,
R^{3'} Chlor, Methyl, Isopropyl, Ethyl oder Brom ist,
R^{4'} Wasserstoff, Chlor, Brom, Iod, Fluor, Difluormethyl, Isopropyl oder Cyclopropyl ist,
R^{5'} Methyl oder 2,2,2-Trifluorethyl ist,
R^{6'} Wasserstoff oder Chlor ist,
A' ein Rest ausgewählt aus den Resten der Formeln (III1-III19) ist: 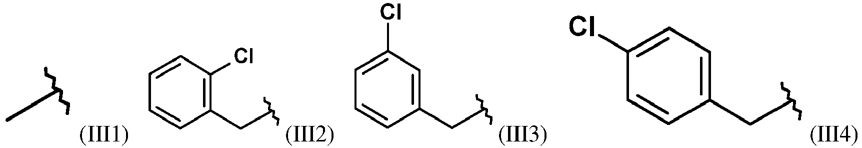 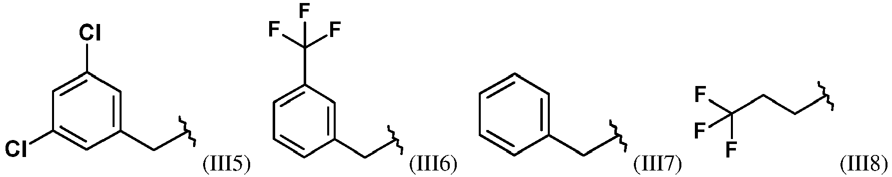 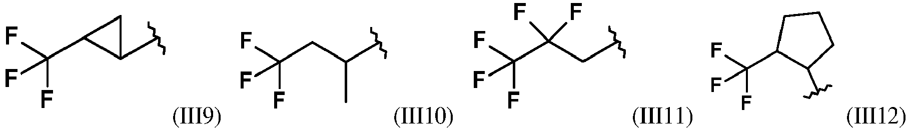 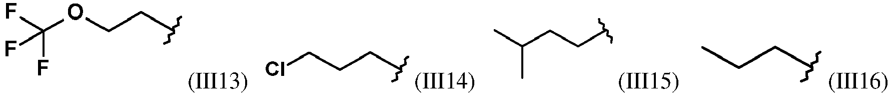 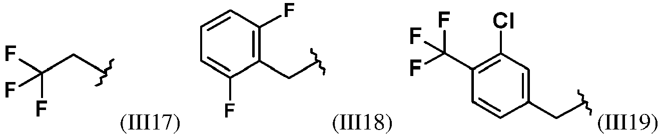
Q' ein Rest ausgewählt aus den Resten der Formeln (IV1-IV40) ist: 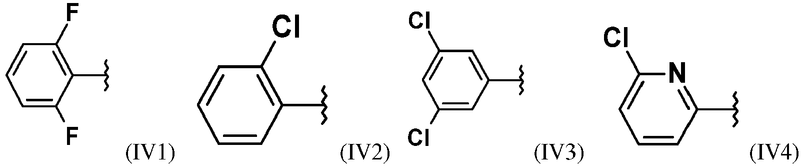 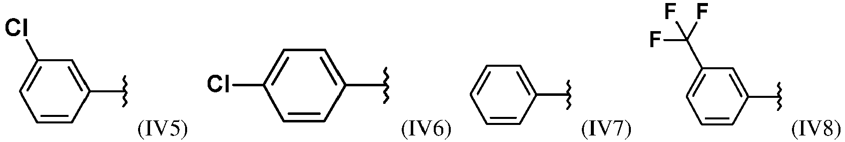 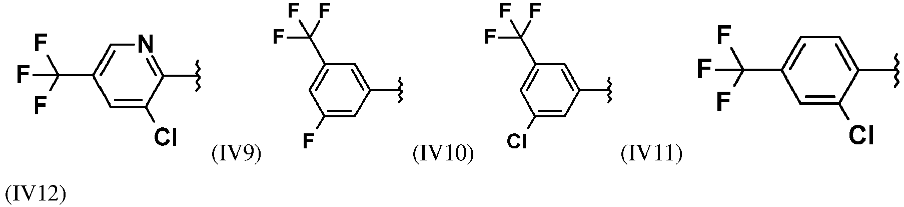 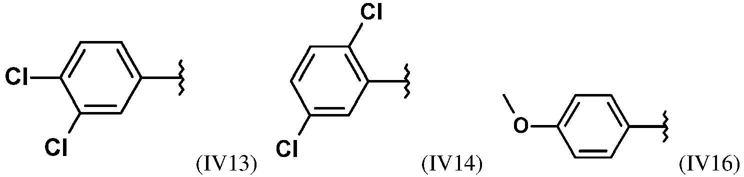 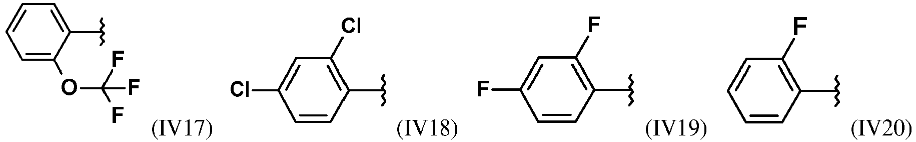 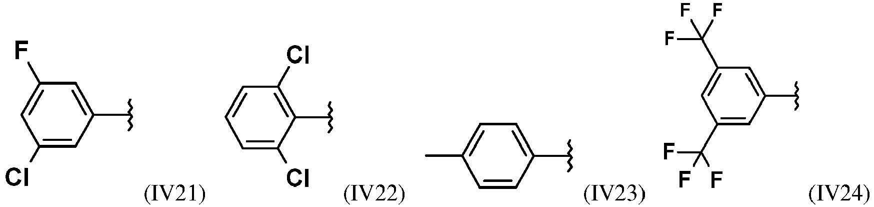 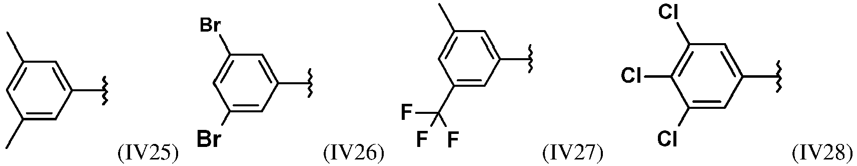 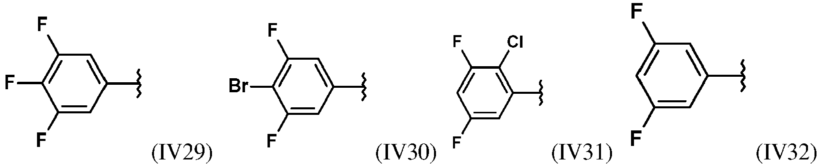 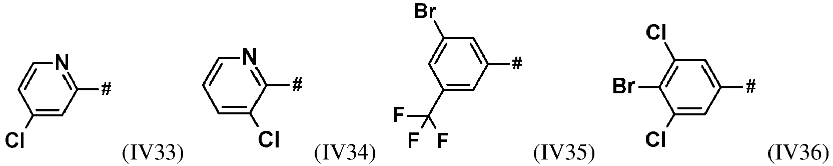 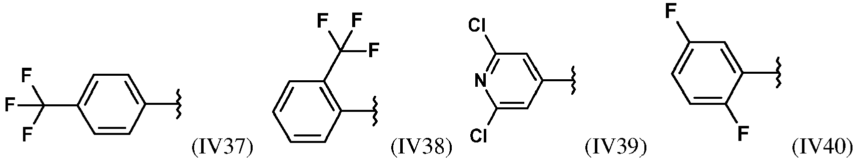 und
D' ein Rest ausgewählt aus den Resten der Formeln (V1-V61) ist:

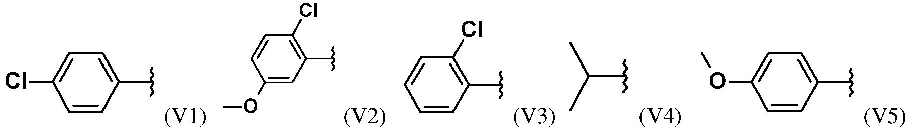

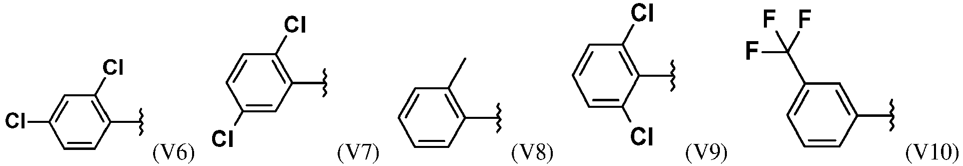

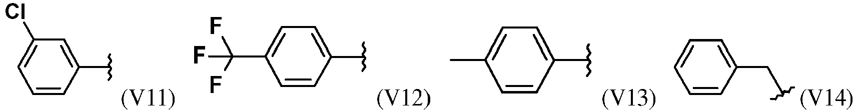

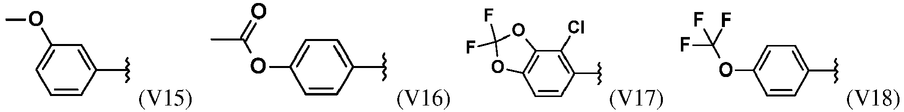

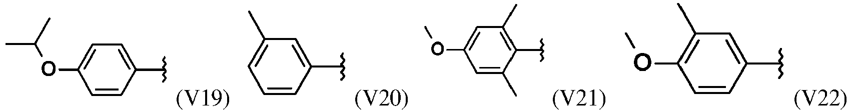

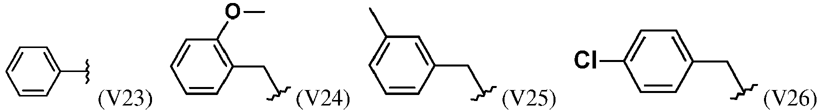

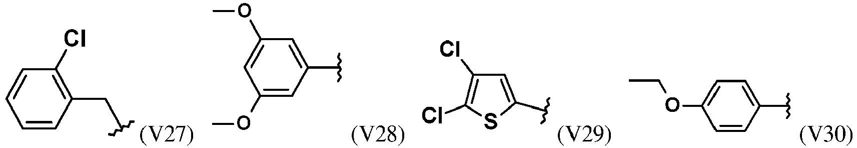

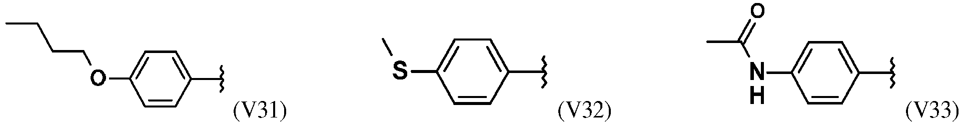

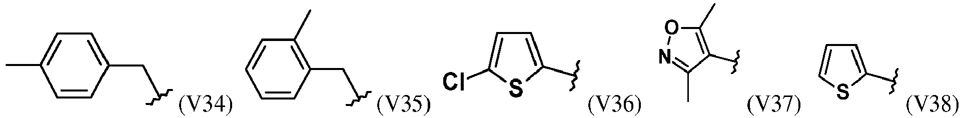

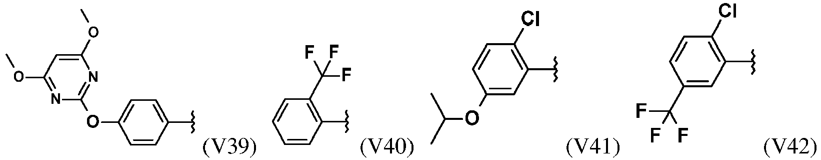

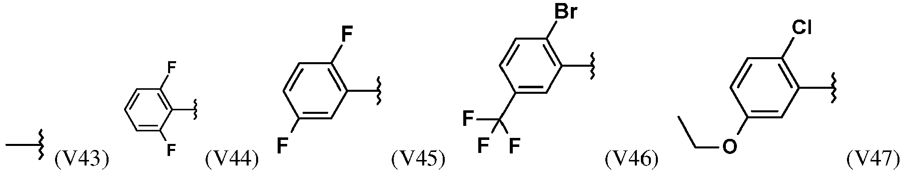

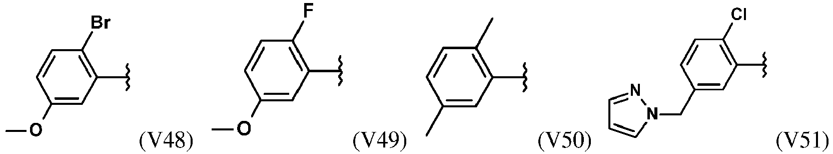

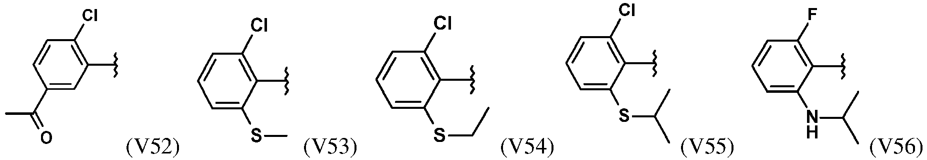

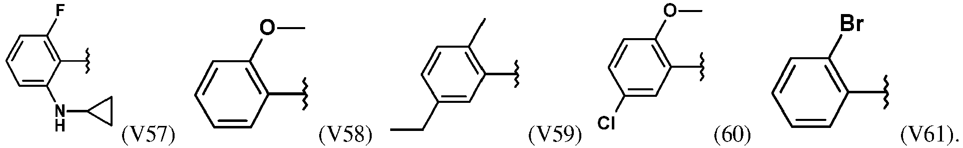

Weiterhin besonders bevorzugt ist eine Ausführungsform in der M' für einen Rest ausgewählt aus den Formeln (IIa'-IIc') steht und wobei (Ausgestaltung 5'-2)
R^{1'} Chlor, Brom, Methyl, Benzyl, 2-Chlorbenzyl, 3-Chlorbenzyl oder 4-Chlorbenzyl ist,
R^{2'} Wasserstoff, Cyano, Chlor, Brom oder Iod ist,
R^{3'} Chlor, Methyl, Isopropyl oder Ethyl ist,
R^{4'} Wasserstoff, Chlor, Brom oder Iod ist,
R^{5'} Methyl ist,
R^{6'} Wasserstoff oder Chlor ist,
A' ein Rest ausgewählt aus den Resten der Formeln (III1-III18) ist: 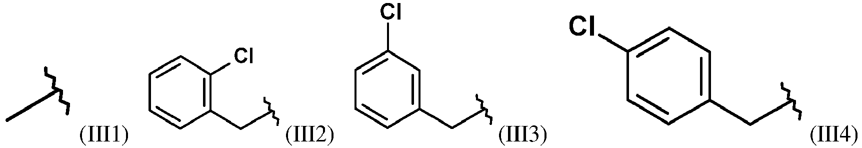 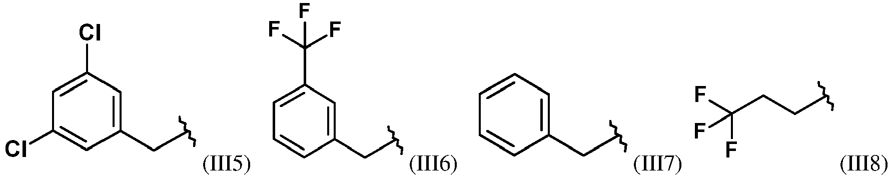 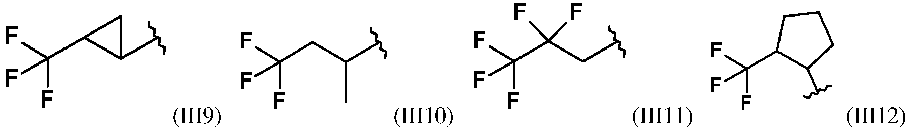 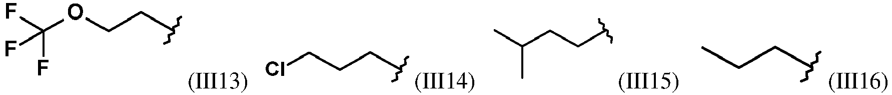 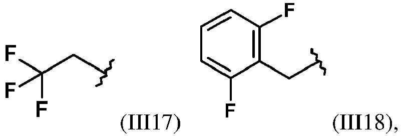
Q' ein Rest ausgewählt aus den Resten der Formeln (IV1-IV36) ist: 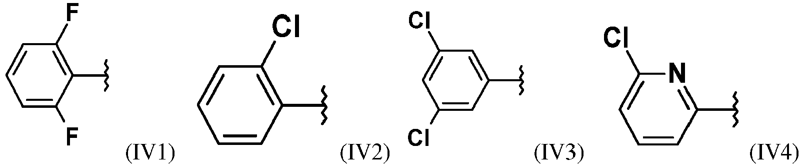 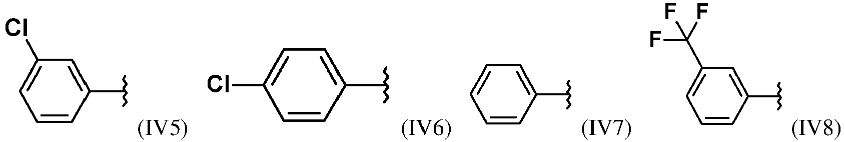 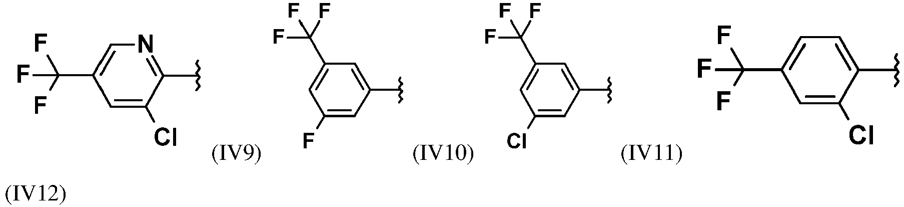 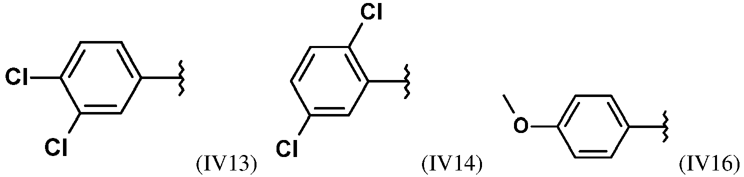 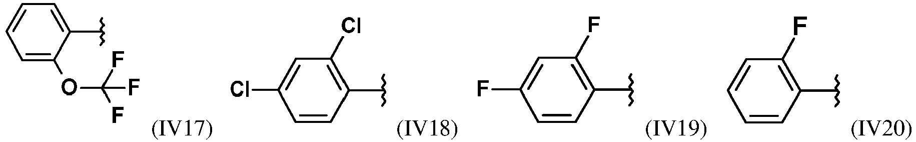 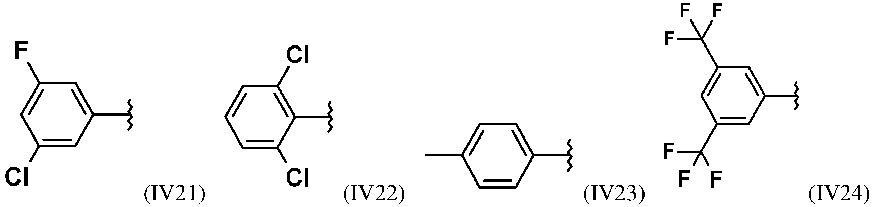 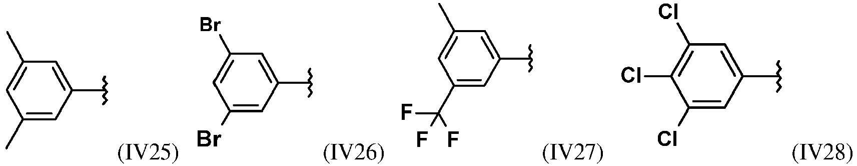 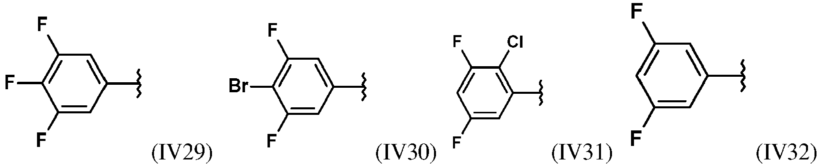 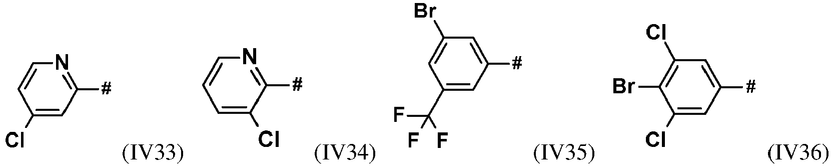 und
D' ein Rest ausgewählt aus den Resten der Formeln (V1-V57) ist:

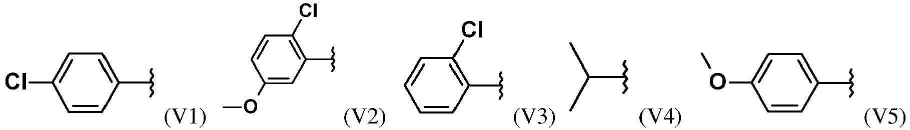

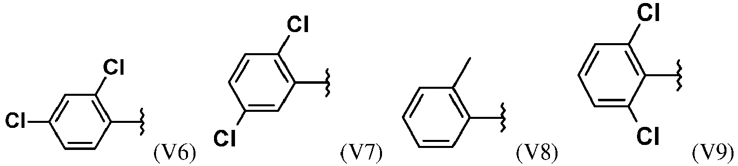

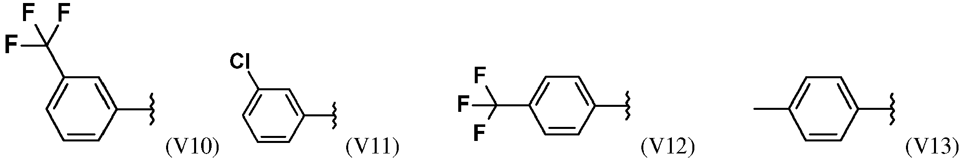

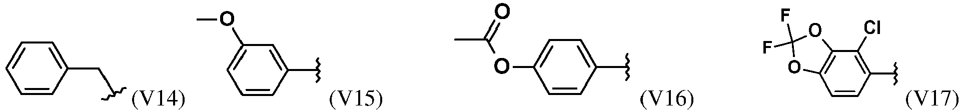

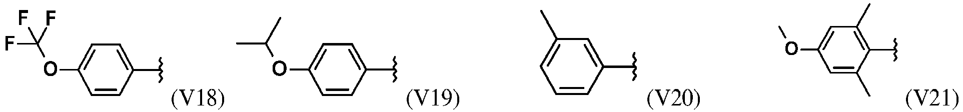

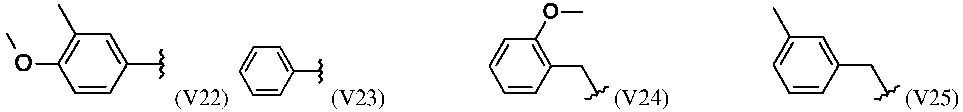

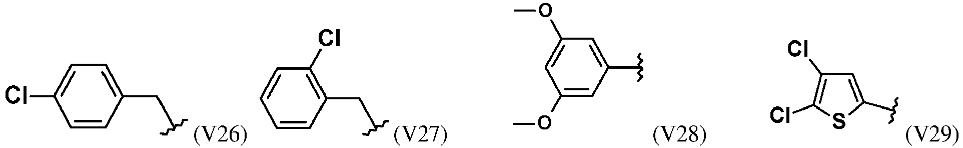

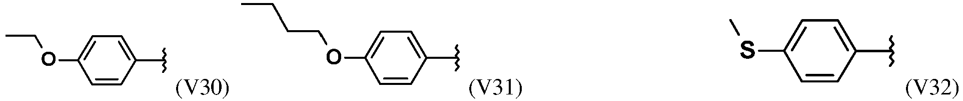

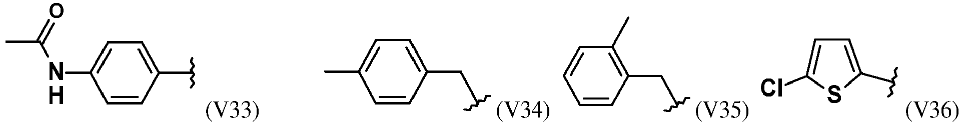

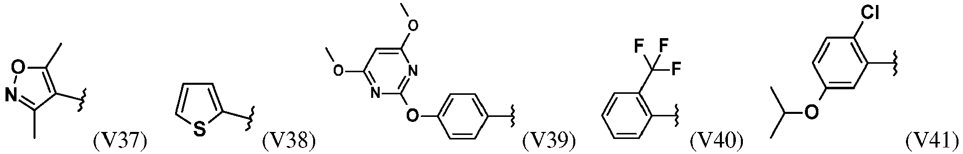

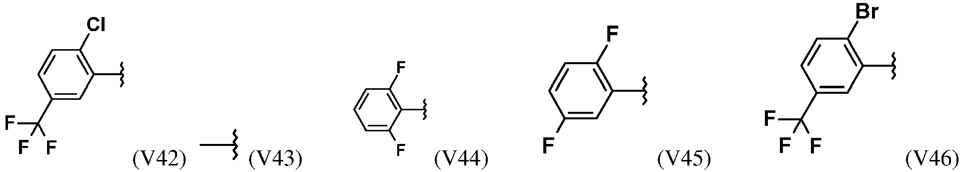

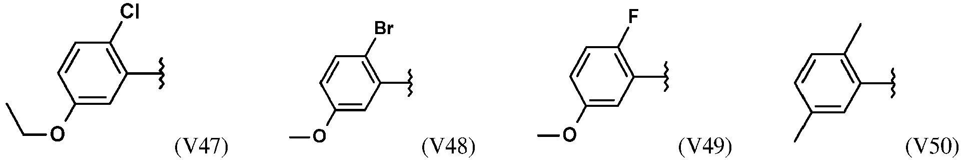

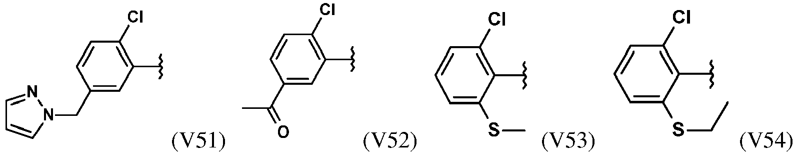

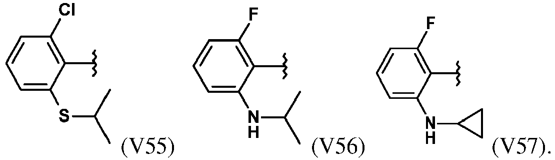

Im Folgenden steht *Ausgestaltung (x')* gleichbedeutend mit *Ausgestaltung (x'-1) oder (x'-2)* mit x' = 1', 2', 3', 4' oder 5'.

In einer bevorzugten Ausführungsform der Erfindung steht in Formeln (I') M' für einen Rest ausgewählt aus den Formeln IIa' oder IIb', wobei die Reste R¹', R²', A', R³', R⁴', R⁷', R⁸', Q' und D' die in der Ausgestaltung (2') oder der Ausgestaltung (3') oder der Ausgestaltung (4') oder der Ausgestaltung (5') beschriebenen Bedeutungen haben.

In einer weiteren bevorzugten Ausführungsform betrifft die Erfindung Verbindungen der Formel (I'), wobei M', R¹', R²', R³', R⁴', R⁵', R⁶', R⁷', R⁸', Q' und D' die oben beschriebenen Bedeutungen, insbesondere die in Ausgestaltung (3') oder Ausgestaltung (4') oder Ausgestaltung (5') beschriebenen Bedeutungen haben und
A' die in Ausgestaltung (2') beschriebenen Bedeutungen hat.

In einer weiteren bevorzugten Ausführungsform betrifft die Erfindung Verbindungen der Formel (I'), wobei M', R¹', R²' , R³', R⁴', R⁵', R⁶', R⁷' , R⁸', Q' und D' die oben beschriebenen Bedeutungen, insbesondere die in Ausgestaltung (2') oder Ausgestaltung (4') oder Ausgestaltung (5') beschriebenen Bedeutungen haben und
A' die in Ausgestaltung (3') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', R⁸', Q' und D' die oben beschriebenen Bedeutungen, insbesondere die in Ausgestaltung (2') oder Ausgestaltung (3') oder Ausgestaltung (5') beschriebenen Bedeutungen haben und
A' die in Ausgestaltung (4') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', R⁸', Q' und D' die oben beschriebenen Bedeutungen, insbesondere die in Ausgestaltung (2') oder Ausgestaltung (3') oder Ausgestaltung (4') beschriebenen Bedeutungen haben und
A' die in Ausgestaltung (5') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁸', A' und D' die oben beschriebenen Bedeutungen, insbesondere die in Ausgestaltung (3') oder Ausgestaltung (4') oder Ausgestaltung (5') beschriebenen Bedeutungen haben und
Q' und R⁷' die in Ausgestaltung (2') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁸', A' und D' die oben beschriebenen Bedeutungen, insbesondere die in Ausgestaltung (2') oder Ausgestaltung (4') oder Ausgestaltung (5') beschriebenen Bedeutungen haben und
Q' und R⁷' die in Ausgestaltung (3') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁸', A' und D' die oben beschriebenen Bedeutungen, insbesondere die in Ausgestaltung (2') oder Ausgestaltung (3') oder Ausgestaltung (5') beschriebenen Bedeutungen haben und
Q' und R⁷' die in Ausgestaltung (4') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁸', A' und D' die oben beschriebenen Bedeutungen, insbesondere die in Ausgestaltung (2') oder Ausgestaltung (3') oder Ausgestaltung (4') beschriebenen Bedeutungen haben und
Q' und R⁷' die in Ausgestaltung (5') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', A' und Q' die oben beschriebenen Bedeutungen, insbesondere die in Ausgestaltung (3') oder Ausgestaltung (4') oder Ausgestaltung (5') beschriebenen Bedeutungen haben und
D' und R⁸' die in Ausgestaltung (2') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', A' und Q' die oben beschriebenen Bedeutungen, insbesondere die in Ausgestaltung (2') oder Ausgestaltung (4') oder Ausgestaltung (5') beschriebenen Bedeutungen haben und
D' und R⁸' die in Ausgestaltung (3') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', A' und Q' die oben beschriebenen Bedeutungen, insbesondere die in Ausgestaltung (2') oder Ausgestaltung (3') oder Ausgestaltung (5') beschriebenen Bedeutungen haben und
D' und R⁸' die in Ausgestaltung (4') beschriebenen Bedeutungen hat.

In einer weiteren bevorzugten Ausführungsform betrifft die Erfindung Verbindungen der Formel (I'), wobei M', R¹', R²', R³', R⁴', R⁵', R⁶', R⁷', A' und Q' die oben beschriebenen Bedeutungen, insbesondere die in Ausgestaltung (2') oder Ausgestaltung (3') oder Ausgestaltung (4') beschriebenen Bedeutungen haben und
D' und R⁸' die in Ausgestaltung (5') beschriebenen Bedeutungen hat.

In einer weiteren bevorzugten Ausführungsform betrifft die Erfindung die Verwendung von Verbindungen der Formel (I'), wobei M', R¹', R²', R³', R⁴', R⁵', R⁶', R⁷', R⁸', Q' und A' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
D' für einen unsubstituierten oder ein- oder zweifach durch R⁸' substituierten Phenylrest steht.

In einer weiteren bevorzugten Ausführungsform betrifft die Erfindung die Verwendung von Verbindungen der Formel (I'), wobei M', R¹', R²', R³', R⁴', R⁵', R⁶', R⁷', R⁸', Q' und A' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
D' für einen ein- oder zweifach durch R⁸' substituierten Phenylrest steht.

In einer weiteren bevorzugten Ausführungsform betrifft die Erfindung die Verwendung von Verbindungen der Formel (I'), wobei M', R¹', R²', R³', R⁴', R⁵', R⁶', R⁷', R⁸', Q' und A' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
D' für einen unsubstituierten oder ein- oder zweifach durch R⁸' substituierten Phenylrest steht, wobei

R⁸' ausgewählt ist aus Halogen, (C₁-C₄)Alkyl, (C₁-C₄)Alkoxy, (C₁-C₄)Halogenalkyl oder (C₁-C₄)Alkylthio, besonders bevorzugt aus Fluor, Brom, Chlor, Methyl, Trifluormethyl, Methoxy, Ethoxy, Isopropoxy oder Methylthio.

In einer weiteren bevorzugten Ausführungsform betrifft die Erfindung die Verwendung von Verbindungen der Formel (I'), wobei M', R¹', R²', R³', R⁴', R⁵', R⁶', R⁷', R⁸', Q' und A' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
D' für einen ein- oder zweifach durch R⁸' substituierten Phenylrest steht, wobei
R⁸' ausgewählt ist aus Halogen, (C₁-C₄)Alkyl, (C₁-C₄)Alkoxy, (C₁-C₄)Halogenalkyl oder (C₁-C₄)Alkylthio, besonders bevorzugt aus Fluor, Brom, Chlor, Methyl, Trifluormethyl, Methoxy, Ethoxy, Isopropoxy oder Methylthio.

Weiterhin bevorzugt sind Verbindungen der Formeln (Ia') oder (Ib') oder (Ic'), besonders bevorzugt (Ia') oder (Ib'), 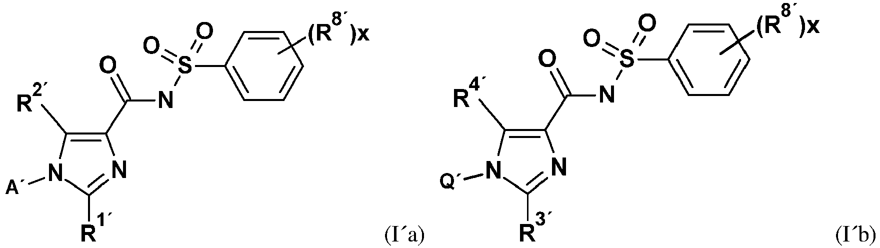 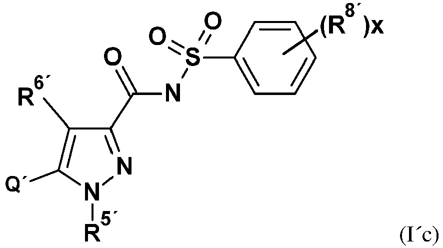 wobei R¹', R²', R³', R⁴', R⁵', R⁶', R⁷', A', Q' und R⁸' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben
und x für 1 oder 2 steht.

Besonders bevorzugt sind Verbindungen der Formel (Ia') bei welchen gilt:
A' steht für (C₁-C₄)Halogenalkyl oder (C₁-C₄)Halogenalkylcyclopropyl, bevorzugt für (C₁-C₄)Fluoralkyl oder (C₁-C₄)Fluoralkylcyclopropyl, ganz besonders bevorzugt für 3,3,3-Trifluorpropyl oder 2-Trifluormethylcyclopropyl,
x steht für 1 oder 2 und
R¹', R²' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Weiterhin besonders bevorzugt sind Verbindungen der Formel (Ia') bei welchen gilt:
R¹' steht für Halogen, besonders bevorzugt für Chlor oder Brom,
R²' steht für Halogen, besonders bevorzugt für Chlor, Brom oder Iod,
x steht für 1 oder 2 und
A' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Besonders bevorzugt sind Verbindungen der Formel (Ib') bei welchen gilt:
Q' steht für einen unsubstituierten oder ein- oder zweifach durch R⁷' substituierten Phenylrest, wobei
R⁷' ausgewählt ist aus Halogen, (C₁-C₄)Alkyl, (C₁-C₄)Alkoxy oder (C₁-C₄)Halogenalkyl, besonders bevorzugt aus Fluor, Brom, Chlor oder Trifluormethyl,
x steht für 1 oder 2 und
R³', R⁴' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Weiterhin besonders bevorzugt sind Verbindungen der Formel (Ib') bei welchen gilt:
Q' steht für einen unsubstituierten oder ein- oder zweifach durch R⁷' substituierten Phenylrest,
x steht für 1 oder 2 und
R³', R⁴', R⁷' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Weiterhin besonders bevorzugt sind Verbindungen der Formel (Ib') bei welchen gilt:
R³' steht für Wasserstoff, Halogen oder (C₁-C₄)Alkyl, besonders bevorzugt für Wasserstoff, Chlor, Methyl, Isopropyl oder Ethyl,
R⁴' steht für Wasserstoff oder Halogen, besonders bevorzugt für Wasserstoff, Chlor, Brom oder Iod,
x steht für 0, 1 oder 2 und
Q' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Besonders bevorzugt sind Verbindungen der Formel (Ic') bei welchen gilt:
Q' steht für einen unsubstituierten oder ein- oder zweifach durch R⁷' substituierten Phenylrest, wobei
R⁷' für Halogen, (C₁-C₄)Alkyl, (C₁-C₄)Alkoxy oder (C₁-C₄)Halogenalkyl, besonders bevorzugt für Fluor, Brom, Chlor oder Trifluormethyl steht,
x steht für 1 oder 2 und
R⁵', R⁶' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Weiterhin besonders bevorzugt sind Verbindungen der Formel (Ic') bei welchen gilt:
Q' steht für einen unsubstituierten oder ein- oder zweifach durch R⁷' substituierten Phenylrest,
x steht für 1 oder 2 und
R³', R⁴', R⁷' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen.

Weiterhin besonders bevorzugt sind Verbindungen der Formel (Ic') bei welchen gilt:
R⁵' steht für (C₁-C₄)Alkyl, besonders bevorzugt für Methyl,
R⁶' steht für Wasserstoff oder Halogen, besonders bevorzugt für Wasserstoff oder Chlor,
x steht für 1 oder 2 und
Q' und R⁸' haben die oben beschriebenen Bedeutungen, insbesondere die in Ausgestaltung (2') oder Ausgestaltung (3') oder Ausgestaltung (4') oder Ausgestaltung (5') beschriebenen Bedeutungen haben.

Im Folgenden umfasst der Begriff *Formel (I')* auch die speziellen Ausführungsformeln Formel (Ia'), Formel (Ib') und Formel (Ic').

In einer weiteren bevorzugten Ausführungsform betrifft die Erfindung Verbindungen der Formel (I'), wobei M', R²', R³', R⁴', R⁵', R⁶', R⁷', A', Q', D' und R⁸' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
R¹ Hydroxy, Cyano, Carboxy, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₅)cycloalkyl, Halogen(C₃-C₅)cycloalkyl, Methyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(Ci-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, Halogen(C₁-C₆)Alkylthio-(C₁-C₆)alkyl, Halogen(C₁-C₆)Alkylsulfinyl-(C₁-C₆)alkyl, Halogen(C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Alkylsulfoximino, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino oder
gegebenenfalls einfach oder mehrfach, gleich oder verschieden substituiertes Aryl-(C₂-C₆)alkyl oder Hetaryl-(C₁-C₆)alkyl ist, wobei, im Fall von Hetaryl, gegebenenfalls mindestens eine Carbonylgruppe enthalten sein kann und wobei der oder die Substituenten jeweils unabhängig voneinander ausgewählt sind aus:
Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino und/oder (C₁-C₆)Alkylcarbonylamino.

In einer weiteren bevorzugten Ausführungsform betrifft die Erfindung Verbindungen der Formel (I'), wobei M', R²', R³', R⁴', R⁵', R⁶', R⁷', A', Q', D' und R⁸' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
R¹ Cyano, Halogen, Nitro, (C₃-C₅)Cycloalkyl, (C₃-C₈)Cycloalkyloxy, (C₃-C₈)Cycloalkyl-(C₃-C₈)cycloalkyl, (C₁-C₆)Alkyl-(C₃-C₅)cycloalkyl, Halogen(C₃-C₅)cycloalkylMethyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist.

In einer weiteren bevorzugten Ausführungsform betrifft die Erfindung Verbindungen der Formel (I'), wobei M', R²', R³', R⁴', R⁵', R⁶', R⁷', A', Q', D' und R⁸' die oben beschriebenen Bedeutungen, insbesondere die in Ausgestaltung (1') oder Ausgestaltung (2') oder Ausgestaltung (3') oder Ausgestaltung (4') oder Ausgestaltung (5') beschriebenen Bedeutungen haben und R^{1'} Cyano, Halogen, Cyclopropyl, Methyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl ist.

In einer weiteren bevorzugten Ausführungsform betrifft die Erfindung Verbindungen der Formel (I'), wobei M', R²' , R³', R⁴', R⁵', R⁶', R⁷' , A', Q', D' und R⁸' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
R^{1'} Halogen, Cyclopropyl, Methyl, (C₁-C₄)Alkylthio, Halogencyclopropyl oder (C₁-C₄)Halogenalkyl ist.

In einer weiteren bevorzugten Ausführungsform betrifft die Erfindung Verbindungen der Formel (I'), wobei M', R²', R³', R⁴', R⁵', R⁶', R⁷', A', Q', D' und R⁸' die oben beschriebenen Bedeutungen, insbesondere die in Ausgestaltung (2') oder Ausgestaltung (3') oder Ausgestaltung (4') oder Ausgestaltung (5') beschriebenen Bedeutungen haben und
R¹ Chlor, Brom, Methyl, Trifluormethyl, Methylthio oder Isopropylthio ist.

Im Folgenden steht *Formel (I)* gleichbedeutend mit *Formel (I) oder (I').*

Definitionsgemäß ist, sofern nichts anderes angegeben ist, Halogen ausgewählt aus der Reihe Fluor, Chlor, Brom und Iod, bevorzugt wiederum aus der Reihe Fluor, Chlor und Brom.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, wird unter dem Begriff "Aryl" erfindungsgemäß ein mono-, bi- oder tricyclischer Rest mit 6 bis 14 Kohlenstoffatomen, wobei mindestens ein Zyklus aromatisch ist, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet "Hetaryl" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome und ist ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

### Erläuterung der Verfahren und Zwischenprodukte

Beispielhaft und ergänzend wird die Herstellung von Verbindungen der Formel (I) oder (I') in den folgenden Formelschemata erläutert. An dieser Stelle sei auch auf die Herstellungsbeispiele verwiesen.

Alle im Folgenden für Formel (I) gemachten Ausführungen gelten analog auch für Formel (I'). Alle angegebenen Reste wie beispielsweise M, D, Q etc. stehen im Folgenden auch für die jeweilige' Variante, also beispielsweise M', D', Q' etc.

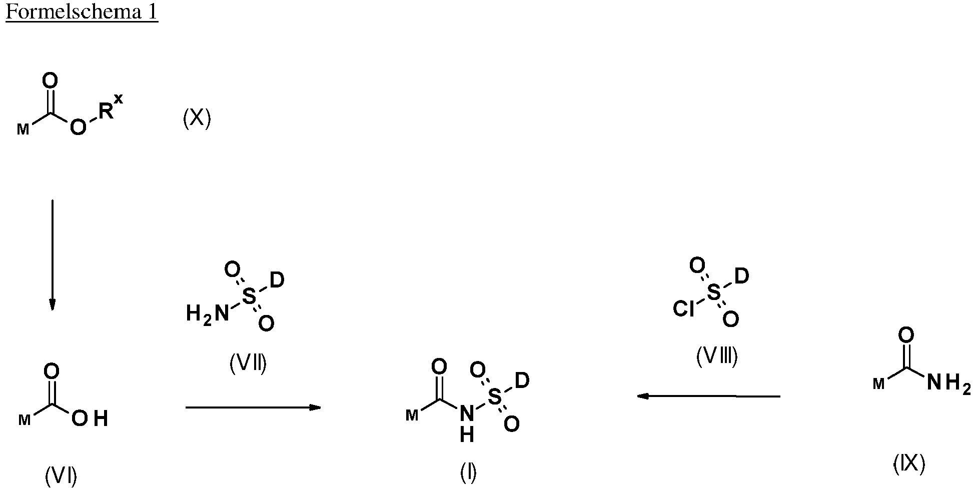

Die Herstellung von erfindungsgemäßen Verbindungen der Formel (I) erfolgt ganz allgemein entsprechend Formelschema 1 ausgehend von Carbonsäuren der Formel (VI) durch Umsetzung mit einem Kupplungsreagenz und Sulfonamiden der Formel (VII), siehe dazu beispielsweise WO2012/80447, WO2006/114313, WO2015/11082, WO2010/129500, US2008/227769 und WO2009/67108. Alternativ können die Verbindungen der Formel (I) auch durch Umsetzung eines Carbonsäureamids der Formel (IX) mit einem Sulfochlorid der Formel (VIII) in Gegenwart einer Base wie beispielsweise Natriumhydrid hergestellt werden, siehe dazu beispielsweise US2004/6143. Die benötigten Amide der Formel (IX) können aus den Säuren der Formel (VI) beispielsweise durch Umsetzung mit einem Kupplungsreagenz und Ammoniumacetat erhalten werden, siehe dazu beispielsweise US5300498.

Die benötigten Sulfonamide und Sulfochloride der Formel (VII) und (VIII) sind bekannt oder lassen sich nach im Allgemeinen bekannten Methoden herstellen. Dabei können die Sulfonamide aus den Sulfochloriden durch Umsetzung mit Ammoniak erhalten werden, siehe dazu WO2014/146490, Eur. J. Med. Chem. 2013, 62, 597-604; Bioorg. Med. Chem. 2005, 13, 7, 2459-2468.

### Weitere Beispiele sind:

3-Chlorbenzolsulfonylamid: Coll. Czech. Chem. Comm. 1984, 49, 5, 1182-1192
2-Chlorbenzolsulfonsäurechlorid: US5099025
2-Chlor-5-Methoxy-benzolsulfonylamid: WO2010/129500
Isopropylsulfonylamid: US542803

Die benötigten Carbonsäuren der Formel (VI) werden durch Hydrolyse aus den Estern der Formel (X) erhalten. Die Hydrolyse der Ester der Formel (X) zu den Säuren der Formel (VI) erfolgt gemäß allgemein bekannter Bedingungen (LiOH, H₂O, THF oder NaOH, EtOH).

Für die Ester der Formel (X) gilt, dass R^{x} zum Beispiel Alkyl (auch cyclisch) sein kann.

Die Herstellung derjenigen erfindungsgemäßen Verbindungen der Formel (I), in denen R¹, R², R³, R⁴, R⁶ unabhängig voneinander für Halogen stehen, lassen sich entweder entsprechend Formelschema 2 durch Umsetzung von geeigneten Verbindungen der Formel (I), in denen entsprechend R¹, R², R³, R⁴, R⁶ für Wasserstoff stehen, mit einem Halogenierungsmittel wie N-Brom-Succinimid oder Brom oder 1-chloromethyl-4-fluoro-1,4-diazoniabicyclo[2.2.2]octan bis(tetrafluoroborat) in einem Lösungsmittel wie Acetonitril erhalten.

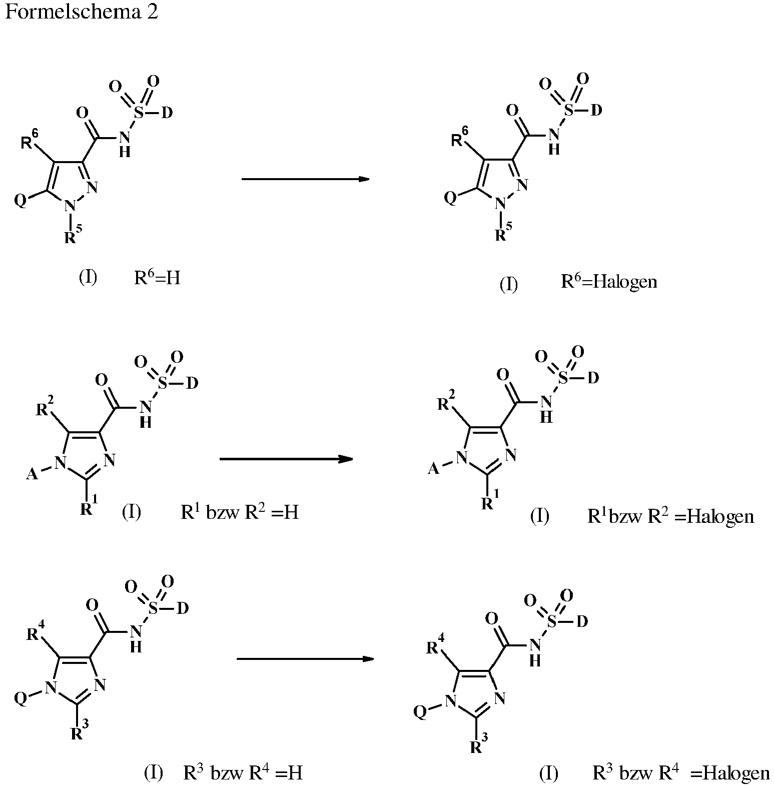

Alternativ kann die Einführung von Halogen an den genannten Positionen auch vor der in Formelschema 1 beschriebenen Kupplung an einem entsprechenden Intermediat wie beispielsweise dem Ester (X) in Analogie zu der in Formelschema 2 beschriebenen Umsetzung erfolgen, siehe dazu beispielsweise WO2014/191894 A1, Seite 63.

Die Ester der Formel (X) und die entsprechenden Säuren der Formel (VI) sind bekannt oder können nach im Allgemeinen bekannten Verfahren oder entsprechend der im Folgenden beschriebenen Verfahren hergestellt werden.

Beispiele für Ester der Formel (X) bzw. entsprechenden Säuren der Formel (VI) und ihre Herstellverfahren sind:
Für 1-Benzyl-1H-imidazol-4-carbonsäure siehe WO 2014125444 A1, Tetrahedron 2004, vol60, # 29, 6079

Für 1-(3-Chlorbenzyl)-1H-imidazol-4-carbonsäure und 1-[3-(Trifluormethyl)benzyl]-1*H*-imidazol-4-carbonsäure siehe Bioorganic and Medicinal Chemistry Letters, 2011 , vol. 21, # 6 p. 1621 - 162 Für 1-(3-Chlorphenyl)-1*H*-imidazol-4-carbonsäure und 1-[3-(Trifluormethyl)phenyl]-1*H*-imidazol-4-carbonsäure siehe US4952698 A1

Für 1-(4-Chlorphenyl)-1H-imidazol-4-carbonsäure siehe US2002/151715 A1,

Für 1-Phenyl-1H-imidazol-4-carbonsäure siehe US2009/239810 A1

Für 2-Methyl-1-phenyl-1H-imidazol-4-carbonsäure siehe Bioorganic and Medicinal Chemistry Letters, 2010, vol. 20, # 3 p. 1084 - 1089

Für Ethyl-1-benzyl-1H-imidazol-4-carboxylat siehe Organic Letters, 2002, vol. 4, # 23 p. 4133 - 4134 und WO2015/96884 A1 und US5089499 A1 und Chemical and Pharmaceutical Bulletin, 2006 , vol. 54, # 5 p. 706-710.

Für 5-(2,6-Difluorphenyl)-1-methyl-1H-pyrazol-3-carbonsäure siehe WO2014/108336 A1, Seite 58.

Ein Herstellverfahren für Ester der Formel (X) wird in Formelschema 3 berschrieben.

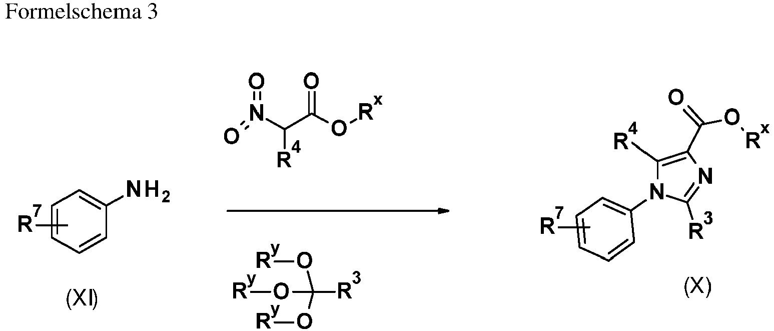

Die Umsetzung eines aromatischen Amins der Formel (XI) mit Nitroessigsäureester und einem orthoEster in Essigsäure mit einem Reduktionsmittel wie Eisen entsprechend beispielsweise US2005/256113 A1 Seite 20 oder US6642237 B1 Seite 115 führt zu einem geeigneten Ester der Formel (X).

Alternativ lassen sich entsprechend Formelschema 4 die Ester der Formel (X) durch Umsetzung des Enamins der Formel (XII) während übergangsmetallkatalysierter Hydrierung beispielsweise entsprechend WO2013/22818 A1Paragraph00400 oder Bioorganic and Medicinal Chemistry Letters, 2010 , vol. 20, # 3 p. 1084 - 1089 oder Bioorganic and Medicinal Chemistry Letters, 2011, vol. 21, # 21 p. 6515 - 6518 oder unter reduzierenden Bedingungen entsprechend WO2015/110369 A1 Seite 31erhalten.

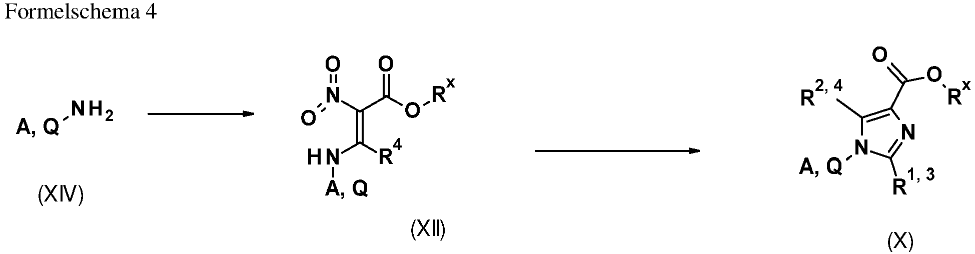

Die Enamine der Formel (XII) wiederum werden erhalten aus den Aminen der Formel (XIV) in einer Formelschema 3 ähnlichen Weise entsprechend Chemische Berichte, 1977, vol. 110, p. 2480 - 2493 oder WO2013/22818 A1 Paragraph 00372 oder durch direkte Umsetzung mit einem Nitroessigsäureenolether entsprechend WO2015/110369 A1, Seite 32 oder WO2013/22818 A1 Paragraph 00372. Die Nitroessigsäureenolether werden ihrerseits hergestellt gemäß beispielsweise WO2013/40790 Seite 100.

Weiterhin lassen sich Ester der Formel (X) durch Umsetzung eines geeigneten Imidazols der Formel (XIII) erhalten, dies geschieht entsprechend Formelschema 5 und beispielsweise entsprechend US2012/94837 A1 Seite 35, Molecules, 2013, vol. 18, # 11 p. 13385 - 13397, WO201O/33626 A1, Seite 133, WO2013/22818 A1 Paragraph 00341, US2006/194779 A1 Seite 13, US2016/185785 A1 Paragraph 2173, WO2006/135627 A2 Seite 140, WO2005/9965 A1 Seite 49, WO2012/143599 A1 Seite 46.

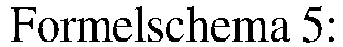

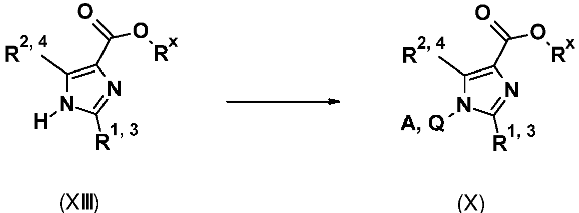

Einen weiteren Herstellungsweg zu Estern der Formel (X) beschreibt Formelschema 6.

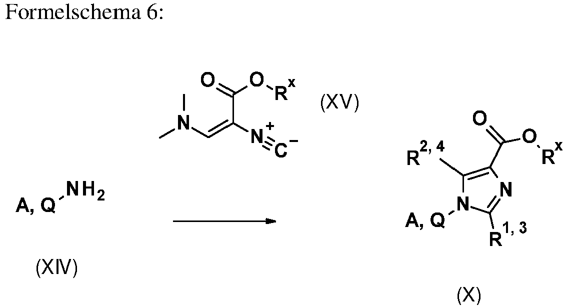

Die Umsetzung von Aminen der Formel (XIV) mit Isonitrilen der Formel (XV) gemäß beispielsweise WO2014/115077 A1 Seite 60, US2012/35168 A1 Seite 30, EP2548871 A1 Paragraph 0264, WO2014/191894 A1 Seite 62, WO2007/42546 A1 Seite 25, WO2015/96884 A1 Seite 39, Organic Letters, 2002, vol. 4, # 23 p. 4133 - 4134, WO2007/42545 A1 Seite 19, Bioorganic and Medicinal Chemistry Letters, 2011 , vol. 21, # 6 p. 1621 - 1625 führt zu den Estern der Formel (X).

Die benötigten Isonitrile sind bekannt beispielsweise aus Chemische Berichte, 1983, vol. 116, # 9 p. 3205 - 3211, WO2015/96884 A Seite 38 und WO2014/115077 A1 Seite 64.

Die Herstellung derjenigen Ester der Formel (Xa) mit R¹/R³ = gegebenenfalls mit einem entsprechenden Substituenten R^{z} substituiertes Benzyl erfolgt bevorzugt nach der Methode, die in Formelschema 7 beschrieben wird:

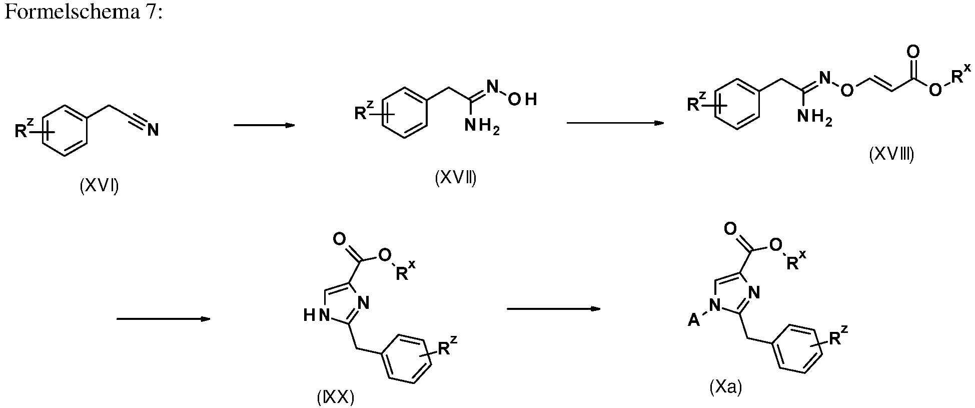

Aus den Nitrilen der Formel (XVI) werden mit Hydroxylamin beispielsweis mit Natriumhydrogencarbonat in Methanol/Wasser die Hydroxamsäuren der Formel (XVII) erhalten, siehe dazu WO2015/140130 A1, Seite 143 oder WO2013/49119 A1 Seite 39. Durch Umsetzung mit Propioloat erhält man zunächst beim Erwärmen in Ethanol das Intermediat der Formel (XVIII) und schließlich beim Erhitzen in einem hochsiedenden Lösungsmittel wie Diphenylether das Imidazol der Formel (IXX), siehe dazu WO2016/44441 A1 Seite 175 oder US2010/22599 A1 Seite 51oder WO2004/63169 Seite 137. Aus dem Imidazol der Formel (IXX) wird durch Umsetzung mit einem Alkylierungsmittel der Ester der Formel (X) erhalten, siehe dazu Journal of the American Chemical Society, 2014, vol. 136, # 34 p. 11914 - 11917 oder US2010/22599 A1, Seite 51.

Halogensubstituierte Ester der Formel (X) lassen sich sowohl über den in Formelschema 2 beschriebenen Prozeß als auch über den in Formelschema 8 beschriebenen Weg erhalten, außerdem lassen die nach Formelschema 8 erhaltenen Ester der Formel (X) sich weiter gemäß Formelschema 2 umsetzen, darüberhinaus beschreibt Formelschema 8 ganz allgemein einen weiteren Zugang zu Estern der Formel (X).

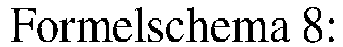

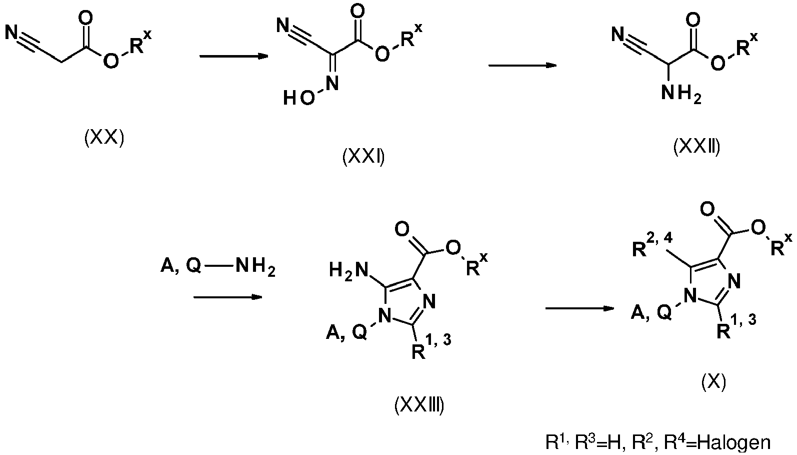

Aus Cyanessigsäureester (XX) wird durch Umsetzen mit Natriumnitrit das Oxim der Formel (XXI) erhalten, siehe dazu beispielsweise WO2010/140168 Seite 14. Reduktion, beispielsweise mit Natriumdithionit, ergibt das Amin der Formel (XXII), aus welchem unmittelbar durch Umsetzung mit einem Amin A/Q-N das Aminoimidazol der Formel (XXIII) erhalten wird, siehe dazu WO2011/79000 A1 Seite 36 oder Journal of Medicinal Chemistry, 1997, vol. 40, # 14 p. 2196 - 2210 oder European Journal of Medicinal Chemistry, 2012, vol. 49, p. 164 - 171 oder Journal of the Chemical Society, Perkin Transactions 1: Organic and Bio-Organic Chemistry (1972-1999), 1980 , p. 2316 - 2321. Schließlich werden nach dem Prinzip der Sandmeyer-Reaktion aus den Aminoimidazolen der Formel (XXIII) die Ester der Formel (X) erhalten, siehe dazu beispielsweise Journal of Medicinal Chemistry, 1991 , vol. 34, #3 p. 1187 - 1192 oder Journal of the Chemical Society, Perkin Transactions 1: Organic and Bio-Organic Chemistry (1972-1999), 1980 , p. 2310 - 2315.

Die Pyrazol-Ester der Formel (X) werden gemäß dem in Formelschema 9 beschriebenen Verfahren erhalten.

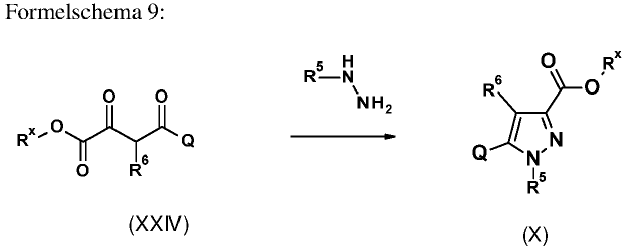

Die Herstellung von Estern der Formel (X) erfolgt ausgehend von einem entsprechenden Alkylhydrazin und einem Diketoester der Formel (XXIV). Diese Cyclisierung kann basen- oder säurekatalysiert erfolgen wie beispielsweise in US2007/287734 A1, 2007 oder US6020357 A1, 2000 beschrieben.

Die benötigten Diketoester der Formel (XXIV) sind bekannt oder lassen sich nach im Allgemeinen bekannten Methoden herstellen.

Ein weiterer Gegenstand der Erfindung sind Zwischenprodukte der Formeln (VIa), (Xa) und (IXa), 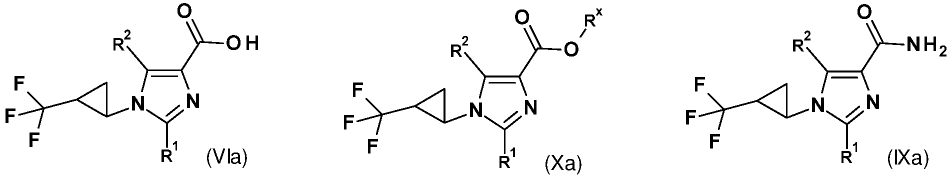 in welchen R¹ und R² die in Ausgestaltung 2 oder Ausgestaltung 3 oder Ausgestaltung 4 oder Ausgestaltung 5 genannten Bedeutungen haben und R^{x} für (C₁-C₆) Alkyl oder (C₃-C₈)Cycloalkyl steht.

Die Zwischenprodukte der Formel (VIa), (Xa) oder (IXa) können als geometrische und/oder als optisch aktive Isomere oder entsprechende Isomerengemische in unterschiedlicher Zusammensetzung vorliegen. Die Erfindung umfasst somit sowohl reine Stereoisomere, als auch beliebige Gemische dieser Isomere.

### Salze

Die Verbindungen der Formel (I) können auch als Salze, insbesondere Säureadditionssalze und Metallsalzkomplexe, vorliegen. Die Verbindungen der Formel (I) und deren Säureadditionssalze und Metallsalzkomplexe besitzen gute Wirksamkeit, insbesondere zur Bekämpfung von tierischen Schädlingen.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

### Isomere

Die Verbindungen der Formel (I) oder (I') können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) oder (I') auf tierische Schädlinge, insbesondere Nematoden und/oder ihren Lebensraum einwirken lässt. Bevorzugt wird die Bekämpfung der tierischen Schädlinge in der Land- und Forstwirtschaft und im Materialschutz durchgeführt. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) oder (I') als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) oder (I') eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) oder (I') können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Alphitobius diaperinus, Amphimallon solstitialis, Anobium punctatum, Anoplophora spp., z. B. Anoplophora glabripennis, Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita o-nukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens , Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditina und Spirurida) parasitieren und in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu bekämpfen.

Der Begriff "Nematoden bekämpfen" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneiern pro Bodenvolumen, Beweglichkeit der Nematoden zwischen einer mit der Verbindung der Formel (I) oder (I') behandelten Pflanze, Pflanzenteil oder dem behandelten Boden und einer unbehandelten Pflanze, Pflanzenteil oder unbehandeltem Boden (100 %) ermittelt. Vorzugsweise wird eine Verringerung um 25-50 % im Vergleich mit einer unbehandelten Pflanze, Pflanzenteil oder unbehandeltem Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Bekämpfung von Nematoden, wie hier beschrieben, beinhaltet ebenso die Bekämpfung der Nematoden-Vermehrung (Entwicklung von Zysten und/oder Eiern). Verbindungen der Formel (I) oder (I') können ebenso verwendet werden, um die Pflanzen oder Tiere gesund zu erhalten und können kurativ, präventiv oder systemisch zur Nematoden-Bekämpfung eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneiern pro Bodenvolumen, Beweglichkeit der Nematoden bestimmt werden.

Die Verwendung einer Verbindung der Formel (I) oder (I') kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Gelbe Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z. B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella ornata, Criconemella rusium, Criconemella xenoplax (= Mesocriconema xenoplax) und Criconemella spp.,

Criconemoides ferniae, Criconemoides onoense, Criconemoides ornatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Gelbe Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Halbparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) oder (I') eingesetzt werden kann, zählen Nematoden der Gattung Meloidogyne wie der Southern Root-Knot Nematode (Meloidogyne incognita), der Javanese Root-Knot Nematode (Meloidogyne javanica), der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie der Gelbe Kartoffelzystennematode (Globodera rostochiensis) und der Weiße Kartoffelzystennematode (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) oder (I') verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Paprika und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumen (Chrysantheme, Rose, Orchidee und dergleichen).

Die Verbindungen der Formel (I) oder (I') eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie auch Meloidogyne paranaensis, Rotylenchus spp., Xiphinema spp., Tylenchorhynchus spp. und Scutellonema spp..

Die Verbindungen der Formel (I) oder (I') eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie von Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) oder (I') eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und auch Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) oder (I') eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) oder (I') eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) oder (I') eignen sich besonders für die Bekämpfung von Nematoden des Maises, insbesondere von Belonolaimus longicaudatus, Paratrichodorus minor und auch Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) oder (I') eignen sich besonders für die Bekämpfung von Nematoden der Sojabohne, insbesondere von Pratylenchus brachyurus, Pratylenchus pratensis, Pratylenchus penetrans, Pratylenchus scribneri, Belonolaimus longicaudatus, Heterodera glycines, Hoplolaimus columbus und auch Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) oder (I') eignen sich besonders für die Bekämpfung von Nematoden des Tabaks, insbesondere von Meloidogyne incognita, Meloidogyne javanica und auch Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) oder (I') eignen sich besonders für die Bekämpfung von Nematoden von Zitrusgewächsen, insbesondere von Pratylenchus coffeae und auch Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis und Radopholus citrophilus, Hemicycliophora arenaria, Hemicycliophora nudata und Tylenchulus semipenetrans.

Die Verbindungen der Formel (I) oder (I') eignen sich besonders für die Bekämpfung von Nematoden der Banane, insbesondere von Pratylenchus coffeae, Radopholus similis und auch Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) oder (I') eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und auch Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides ornatum.

Die Verbindungen der Formel (I) oder (I') eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und auch Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) oder (I') eignen sich besonders für die Bekämpfung von Nematoden von Baumkulturen - Kernobst, insbesondere von Pratylenchus penetrans und auch Pratylenchus vulnus, Longidorus elongatus, Meloidogyne incognita und Meloidogyne hapla.

Die Verbindungen der Formel (I) oder (I') eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und von Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) oder (I') eignen sich besonders für die Bekämpfung von Nematoden in Baumkulturen, Zuckerrohr und Reis, insbesondere von Trichodorus spp., Criconemella spp. und auch Pratylenchus spp., Paratrichodorus spp., Meloidogyne spp., Helicotylenchus spp., Tylenchorhynchus spp., Aphelenchoides spp., Heterodera spp, Xiphinema spp. und Cacopaurus pestis.

Die Verbindungen der Formel (I) oder (I') können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I) oder (I') . Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) oder (I') weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) oder (I') mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder (I') oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) oder (I') und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) oder (I') mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I) oder (I'), besonders bevorzugt zwischen 0,01 und 95 Gew.-% der Verbindung der Formel (I) oder (I'), ganz besonders bevorzugt zwischen 0,5 und 90 Gew.-% der Verbindung der Formel (I) oder (I'), bezogen auf das Gewicht der Formulierung.

Der Gehalt an der Verbindung der Formel (I) oder (I') in den aus den Formulierungen bereiteten Anwendungsformen (insbesondere Schädlingsbekämpfungsmittel) kann in weiten Bereichen variieren. Die Konzentration der Verbindung der Formel (I) oder (I') in den Anwendungsformen kann üblicherweise zwischen 0,00000001 und 95 Gew.-% der Verbindung der Formel (I) oder (I'), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) oder (I') können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) oder (I') in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) oder (I') zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) oder (I') in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.
(3) Natrium-Kanal-Modulatoren, wie beispielsweise Pyrethroide, z. B. Acrinathrin, Allethrin, d-cis-trans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.
(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin oder Sulfoxaflor oder Flupyradifurone.
(5) Allosterische Modulatoren des nicotinischen Acetylcholin-Rezeptors (nAChR), wie beispielsweise Spinosyne, z. B. Spinetoram und Spinosad.
(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.
(7) Juvenilhormon-Mimetika, wie beispielsweise Juvenilhormon-Analoge, z. B. Hydropren, Kinopren und Methopren oder Fenoxycarb oder Pyriproxyfen.
(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.
(9) Modulatoren chordotonaler Organe, z. B. Pymetrozin oder Flonicamid.
(10) Milbenwachstumsinhibitoren, wie z. B. Clofentezin, Hexythiazox und Diflovidazin oder Etoxazol.
(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t*.-Pflanzenproteine: Cry1Ab, Cry1Ac, Cry1Fa, Cry1A.105, Cry2Ab, VIP3A, mCry3A, Cry3Ab, Cry3Bb, Cry34Ab1/35Ab1.
(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.
(13) Entkoppler der oxidativen Phoshorylierung durch Störung des Protonengradienten, wie beispielsweise Chlorfenapyr, DNOC und Sulfluramid.
(14) Blocker des nicotinischen Acetylcholinrezeptorkanals, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsdisruptor (insbesondere bei Dipteren, d. h. Zweiflüglern), wie beispielsweise Cyromazin.
(18) Ecdyson-Rezeptor-Agonisten, wie beispielsweise Chromafenozid, Halofenozid, Methoxyfenozid und Tebufenozid.
(19) Oktopamin-Rezeptor-Agonisten, wie beispielsweise Amitraz.
(20) Mitochondriale Komplex-III-Elektronentransportinhibitoren, wie beispielsweise Hydramethylnon oder Acequinocyl oder Fluacrypyrim.
(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).
(22) Blocker des spannungsabhängigen Natriumkanals, wie z. B. Indoxacarb oder Metaflumizone.
(23) Inhibitoren der Acetyl-CoA-Carboxylase, wie beispielsweise Tetron- und Tetramsäurederivate, z. B. Spirodiclofen, Spiromesifen und Spirotetramat.
(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.
(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.
(28) Ryanodinrezeptor-Modulatoren, wie beispielsweise Diamide, z. B. Chlorantraniliprol, Cyantraniliprol und Flubendiamid,
weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, Bio-Neem, Votivo), sowie folgende Verbindungen: 1-12-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{ 1-[(2E)-3-(4-chlorphenyl)prop-2-en-1-yl]piperidin-4-yl}-4-(trifluormethyl)phenyl] isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-l-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-cblor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxylphenoxylpropoxyl-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyllethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8) und N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9).

### Fungizide

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2- [4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yll(pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl]metbyl)-1H-1,2,4-triazol, (1.039) 1-1[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl] -1-(1H-1,2,4-triazol-1-yl)butan-2-ol, (1.054) 2- [4-(4-Chlorphenoxy)-2-(trifluormethyl)phenyl] -1-(1H-1,2,4-triazol-1-yl)pentan-2-ol, (1.055) 2- [4-(4-Chlorphenoxy)-2-(trifluormethyl)phenyl] -1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-1[3-(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-1[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{ [3-(1,1 ,2,2-tetrafluorethoxy)phenyl] sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4-{[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068)N'-(2,5-Dimethyl-4-{3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4-{[3-(Difluormethoxy)phenyl]sulfanyl}-2,5-dimethylphenyl)-N-ethyl-N-methylimidoformamid, (1.073) N'-(4- {3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-14-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorophenyl)ethoxy] -2-methylpyridin-3-yl} -N-ethyl-N-methylimidoformamid, (1.077) N'- { 5-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy]-2-methylpyridin-3-yl}-N-ethyl-N-methylimidoformamid, (1.081) Mefentrifluconazole, (1.082) Ipfentrifluconazole.
2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{ [4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-lH-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-iluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-lH-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.
3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{ [1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{ [1-(4-Chlor-2-fluorphenyl)-1H-pyrazol-3-yl]oxy}-2-(methoxyimino)-N,3-dimethylpent-3-enamid, (3.029) Methyl {5-[3-(2,4-dimethylphenyl)-1H-pyrazol-1-yl]-2-methylbenzyl}carbamate.
4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazole-3-carbonitrile.
6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.
7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.
(8) Inhibitoren der ATP-Produktion, beispielsweise (8.001) Silthiofam.
9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membran-Synthese, beispielsweise (10.001) Propamocarb, (10.002) Propamocarbhydrochlorid, (10.003) Tolclofos-Methyl.
11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.
12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).
13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, beispielsweise (14.001) Fluazinam, (14.002) Meptyldinocap.
15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarbfosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-|4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-15-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(diiluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat, (15.062) 5-Fluor-4-imino-3-methyl-1-[(4-methylphenyl)sulfonyl]-3,4-dihydropyrimidin-2(1H)-one.

### Biologische Schädlingsbekämpfungsmittel als Mischungskomponenten

Die Verbindungen der Formel (I) oder (I') können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii*), insbesondere Stamm KV01, *Metarhizium anisopliae,* insbesondere Stamm F52 (DSM3884/ ATCC 90448), *Metschnikowiafructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) oder (I') können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) oder (I') erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) oder (I') erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) oder (I') nach dem Ultra-Low-Volume-Verfahren auszubringen oder die Anwendungsform oder die Verbindung der Formel (I) oder (I') selbst in den Boden zu injizieren.

Eine bevorzugte direkte Behandlung der Pflanzen ist die Blattapplikation, d. h. die Verbindungen der Formel (I) oder (I') werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) oder (I') auch über das Wurzelwerk in die Pflanzen. Die Behandlung der Pflanzen erfolgt dann durch Einwirkung der Verbindungen der Formel (I) oder (I') auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) oder (I') getränkt, oder durch die Bodenapplikation, d. h. die erfindungsgemäßen Verbindungen der Formel (I) oder (I') werden in fester Form (z. B. in Form eines Granulats) in den Standort der Pflanzen eingebracht. Bei Wasserreiskulturen kann das auch durch Zudosieren der Verbindung der Formel (I) oder (I') in einer festen Anwendungsform (z. B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Be-handlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) oder (I') behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) oder (I') und einer Mischungskomponente behandelt wird. Es umfasst ferner auch ein Verfahren, in dem das Saatgut zu unterschiedlichen Zeiten mit einer Verbindung der Formel (I) oder (I') und einer Mischungskomponente behandelt wird.

Die Erfindung bezieht sich ebenfalls auf die Verwendung der Verbindungen der Formel (I) oder (I') zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) oder (I') behandelt wurde. Die Erfindung bezieht sich auch auf Saatgut, welches zur gleichen Zeit mit einer Verbindung der Formel (I) oder (I') und einer Mischungskomponente behandelt wurde. Die Erfindung bezieht sich weiterhin auf Saatgut, welches zu unterschiedlichen Zeiten mit einer Verbindung der Formel (I) oder (I') und einer Mischungskomponente behandelt wurde. Bei Saatgut, welches zu unterschiedlichen Zeiten mit einer Verbindung der Formel (I) oder (I') und einer Mischungskomponente behandelt wurde, können die einzelnen Substanzen in unterschiedlichen Schichten auf dem Saatgut vorhanden sein. Dabei können die Schichten, die eine Verbindung der Formel (I) oder (I') und Mischungskomponenten enthalten, gegebenenfalls durch eine Zwischenschicht getrennt sein. Die Erfindung bezieht sich auch auf Saatgut, bei dem eine Verbindung der Formel (I) oder (I') und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) oder (I') einem Filmcoating-Verfahren unterzogen wird, um Staubabrieb am Saatgut zu vermeiden.

Einer der auftretenden Vorteile, wenn eine Verbindung der Formel (I) oder (I') systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) oder (I') Keimung und Auflauf des behandelten Saatguts gefördert werden können.

Ebenso ist es als vorteilhaft anzusehen, dass Verbindungen der Formel (I) oder (I') insbesondere auch bei transgenem Saatgut eingesetzt werden können.

Verbindungen der Formel (I) oder (I') können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) oder (I') eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) oder (I') eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) oder (I') auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) oder (I') und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) oder (I') werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) oder (I') können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) oder (I') mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tri-stryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formu-lierungen enthalten sein können, kommen alle für derartige Zwecke in agrochemischen Mitteln ein-setzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zu-bereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) oder (I') in den Formulierungen und nach dem Saatgut. Die Aufwandmengen bei der Verbindung der Formel (I) oder (I') liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (I) oder (I') gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I) oder (I') , die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) oder (I') an Säugetiere verabreicht.

Gemäß einer weiteren bestimmten Ausführungsform werden die Verbindungen der Formel (I) oder (I') an Vögel, nämlich Stubenvögel oder insbesondere Geflügel, verabreicht.

Durch Verwendung der Verbindungen der Formel (I) oder (I') für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) oder (I') wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) oder (I') den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
Metamonada: aus der Ordnung Diplomonadida zum Beispiel Giardia spp., Spironucleus spp.

Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp. Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,

Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) oder (I') nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) oder (I') zur Verwendung als Arzneimittel.

Ein weiterer Aspekt bezieht sich auf die Verbindungen der Formel (I) oder (I') zur Verwendung als Antiendoparasitikum.

Ein weiterer spezieller Aspekt der Erfindung betrifft die Verbindungen der Formel (I) oder (I') zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) oder (I') zur Verwendung als Antiprotozoikum.

Ein weiterer Aspekt betrifft die Verbindungen der Formel (I) oder (I') zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) oder (I') und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) oder (I') mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) oder (I') bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) oder (I') bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) oder (I') mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;
Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(ethyl), Parathion(-ethyl), Methacrifos, Isopropyl-o-salicylat, Trichlorfon, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (I) oder (I') können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) oder (I') Resistenzbrechend sind.

Verbindungen der Formel (I) oder (I') sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) oder (I') zur Vektorbekämpfung, z. B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) oder (I') eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) oder (I') zusammen mit mindestens einem weiteren Insektizid und/oder mindestens einem Fungizid eingesetzt.

In einer weiteren Ausführungsform liegen die Verbindungen der Formel (I) oder (I') als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) oder (I') zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) oder (I') allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) oder (I') eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) oder (I') allein oder in Kombination mit anderen Wirk- und/oder Hilfsstoffen verwendet. Bevorzugt werden sie in Haushaltsinsektizid-Produkten verwendet. Die Verbindungen der Formel (I) oder (I') sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

Die folgenden Herstellungs- und Verwendungsbeispiele illustrieren die Erfindung, ohne sie zu beschränken.

### Methoden

Die Bestimmung der logP-Werte erfolgte gemäß EEC Directive 79/831 Annex V.A8 durch HPLC (High Performance Liquid Chromatography) an einer Phasenumkehrsäule (C18). Temperatur 43 °C. Die Eichung erfolgt mit unverzweigten Alkan-2-onen (mit 3 bis 16 Kohlenstoffatomen), deren logP Werte bekannt sind. Dabei sind die Bezeichnungen sauer und neutral analog zu nachfolgenden Definitionen zur Bestimmung von M⁺ für sauer und neutral definiert.

Die Bestimmung des M⁺ mit der LC-MS im sauren Bereich erfolgte bei pH 2,7 mit 0,1 % wässriger Ameisensäure und Acetonitril (enthält 0,1% Ameisensäure) als Eluenten; linearer Gradient von 10% Acetonitril bis 95% Acetonitril, Gerät: Agilent 1100 LC-System, Agilent MSD System, HTS PAL.

Die Bestimmung des M⁺ mit der LC-MS im neutralen Bereich erfolgte bei pH 7.8 mit 0,001 molarer wässriger Ammoniumhydrogencarbonat-Lösung und Acetonitril als Eluenten; linearer Gradient von 10 % Acetonitril bis 95 % Acetonitril.

b) Die Bestimmung der ¹H-NMR-Daten erfolgte mit einem Bruker Avance 400 ausgestattet mit einem Durchflussprobenkopf (60 µl Volumen), mit Tetramethylsilan als Referenz (0.0) und den Lösungsmitteln CD₃CN, CDCl₃ oder D₆-DMSO oder mit einem Bruker Avance III HD 300MHz Digital NMR mit einem 5mm Probenkopf.

Die NMR-Daten ausgewählter Beispiele werden entweder in klassischer Form (δ-Werte, Multiplettaufspaltung, Anzahl der H-Atome) oder als NMR-Peak-Listen aufgeführt.

### NMR-Peak-Listenverfahren

Die ¹H-NMR-Daten ausgewählter Beispiele werden in Form von ¹H-NMR-Peaklisten notiert. Zu jedem Signalpeak wird erst der δ -Wert in ppm und dann die Signalintensität in runden Klammern aufgeführt. Die δ -Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensität;);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren wurde Tetramethylsilan und/oder die chemische Verschiebung des Lösungsmittels benutzt, besondern im Falle von Spektren, die in DMSO gemessen wurden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

### Herstellung von Ethyl-1-methyl-5-[3-(trifluormethyl)phenyl]-1H-pyrazol-3-carboxylat

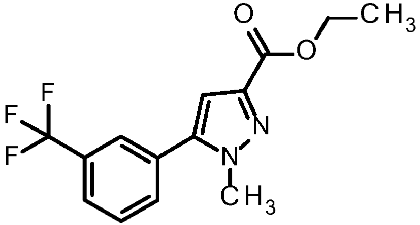

10 g (34.72 mmol) Ethyl-2,4-dioxo-4-[3-(trifluormethyl)phenyl]butanoat (bekannt aus WO2005/121134 A1) wurden in 200 mL Ethanol gelöst und mit 7.51 g (52.08 mmol) Methylhydrazinsulfat und 1 ml conc. HCl versetzt. Das Reaktionsgemisch wurde bei Raumtemperatur über Nacht gerührt und dann im Vakuum eingeengt. Der Rückstand wurde mit Essigsäureethylester gelöst, über Natriumsulfat getrocknet, filtriert und wiederum im Vakuum eingeengt. Der Rückstand wurde mittels Säulenchromatographie (Kieselgel, Laufmittel Petrolether:Essigsäureethylester = 5:1) vom isomeren Ethyl-l-methyl-3-[3-(trifluormethyl)phenyl]-1H-pyrazol-5-carboxylat abgetrennt. Es wurden 4.1 g (39.6 % d. Th.) isoliert.
LC-MS (M+1): 299; RT = 1.61 min

### Herstellung von 1-Methyl-5-[3-(trifluormethyl)phenyl]-1H-pyrazol-3-carbonsäure

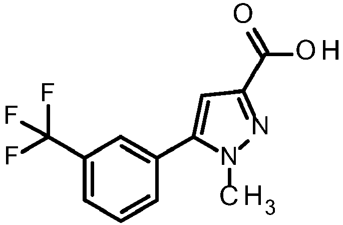

4.1 g (12.76 mmol) Ethyl-1-methyl-5-[3-(trifluormethyl)phenyl]-1H-pyrazol-3-carboxylat wurden in 100 ml Ethanol gelöst und mit 2.75 g (68.79 mmol) NaOH, gelöst in 30 mL Wasser, versetzt. Das Reaktionsgemisch wurde 3 h bei Raumtemperatur gerührt und anschließend im Vakuum eingeengt. Die verbleibende wässrige Lösung wurde mit IN HCl auf pH 2 angesäuert. Hierbei fiel ein Feststoff aus, der abgesaugt und an der Luft getrocknet wurde. Es wurden 2.7 g (78.4 % d. Th.) isoliert und ohne weitere Aufreinigung in der nächsten Stufe eingesetzt.
LC-MS (M+1): 271; RT = 1.44 min

### Herstellung von N-[(2,5-Dichlorphenyl)sulfonyl]-1-methyl-5-[3-(trifluormethyl)phenyl]-1H-pyrazol-3-carboxamid

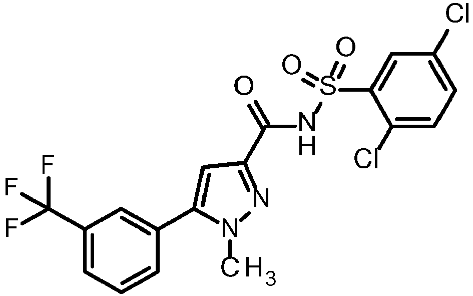

0.324 g (1.2 mmol) 1-Methyl-5-[3-(trifluormethyl)phenyl]-1H-pyrazol-3-carbonsäure und 0.407 g (1.8 mmol) 2,5-Dichlorbenzolsulfonamid wurden in 5 mL DMF gelöst und mit 0.684 g (1.8 mmol) HATU und 0.220 g (1.8 mmol) DMAP versetzt. Das Reaktionsgemisch wurde bei Raumtemperatur über Nacht gerührt und anschließend im Vakuum eingeengt. Der Rückstand wurde mittels präparativer HPLC chromatographiert. Es wurden 0.25 g eines farblosen Feststoffes isoliert (43.7 % d. Th.).
LC-MS: logP (sauer): 3.84 MH+: 478.0
1H-NMR (400MHz, D6-DMSO) δ ppm: 8.11 (s, 1H), 7.93 - 7.71 (m, 6H), 7.14 (s, 1H), 3.96 (s, 3H) - NH Proton nicht detektiert.

### Herstellung von Ethyl-5-amino-1-propyl-1H-imidazol-4-carboxylat

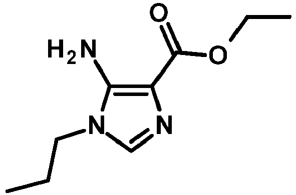

Eine Lösung von Ethyl-3-nitriloalaninat (10.2 g, 79.6 mmol, bekannt aus WO2008/59368) und (Diethoxymethoxy)ethan (13.9 g, 93.9 mmol) in Nitromethan (200 mL) wurde 45 Minuten bei Raumtemperatur gerührt. Anschließend wurde Propan-1-amin (4.71 g, 79.6 mmol) zugegeben und über Nacht bei Raumtemperatur gerührt. Das Lösungsmittel wurde entfernt und der Rückstand mittels Säulenchromatographie aufgereinigt um 4.20 g (21.3 mmol, 27%) des gewünschten Produkts zu erhalten.
logP (neutral): 0.9; MH+: 198.1; ¹H-NMR (400MHz, D6-DMSO) δ ppm: 7.11 (s, 1H), 6.00 (s, 2H), 4.15 (q, 2H), 3.75 (m, 2H), 1.63 (m, 2H), 1.24 (m, 3H), 0.83 (m, 3H).

### Herstellung von Ethyl-5-chlor-1-propyl-1H-imidazol-4-carboxylat

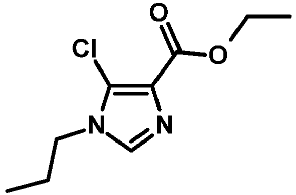

Einer wässrige HCl-Lösung (6 N, 200 mL) wurde auf -25 °C abgekühlt und nacheinander eine Lösung von Ethyl-5-amino-1-propyl-1H-imidazol-4-carboxylat (4.00 g, 20.3 mmol) in Acetonitril (140 mL) und eine Lösung von Natriumnitrit (7.00 g, 2101 mmol) in Wasser (25 mL) zugetropft. Das Gemisch wurde 5 Minuten bei -25 °C weitergerührt und anschließend eine Lösung von Kupfer(I)chlorid (10.3 g, 104 mmol) in wässriger HCl (6 N, 25 mL) zugegeben. Das Reaktionsgemisch wurde über 2 Stunden auf -10 °C erwärmt und anschließend mit gesättigter NaHCO₃-Lösung neutralisiert. Das Gemisch wurde mehrfach mit Dichlormethan extrahiert, die vereinte organische Phase mit Wasser gewaschen, getrocknet und im Vakuum eingeengt. Der Rückstand wurde mittels Säulenchromatographie auf gereinigt um das gewünschte Produkt (1.90 g, 43%) zu erhalten.
logP (neutral): 1.7; MH+: 217.1; ¹H-NMR (400MHz, D6-DMSO) δ ppm: 7.93 (s, 1H), 4.24 (q, 2H), 3.98 (m, 2H), 1.72 (m, 2H), 1.27 (m, 3H), 0.84 (m, 3H).

### Herstellung von 5-Chlor-1-propyl-1H-imidazol-4-carbonsäure

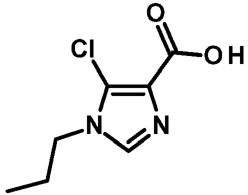

Eine Lösung von Ethyl-5-chlor-1-propyl-1H-imidazol-4-carboxylat (2.20 g, 10.2 mmol) und Natriumhydroxid (516 mg, 12.9 mmol) in Ethanol (30 mL) und Wasser (3 mL) wurde über Nacht bei Raumtemperatur gerührt. Das Lösungsmittel wurde im Vakuum entfernt, der Rückstand in Wasser gelöst und anschließend der pH der wässrigen Phase mit wässriger HCl-Lösung (2 N) auf 2 eingestellt. Der entstandene Feststoff wurde gesammelt, mit Wasser gewaschen und getrocknet um das gewünschte Produkt (1.40 g, 72%) zu erhalten.
logP (sauer): 0.6; MH+: 189.0; ¹H-NMR (400MHz, D6-DMSO) δ ppm: 7.90 (s, 1H), 3.97 (m, 2H), 1.72 (m, 2H), 0.84 (m, 3H).

### Herstellung von 5-Chlor-N-[(4-methoxyphenyl)sulfonyl]-1-propyl-1H-imidazol-4-carboxamid

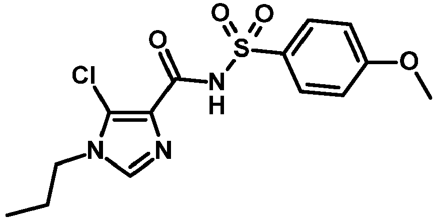

Einer Lösung von 5-Chlor-1-propyl-1H-imidazol-4-carbonsäure (100 mg, 0.53 mmol), N,N-Dimethylpyridin-4-amin (194 mg, 1.59 mmol) und 1-Ethyl-3-(3-dimethylaminopropyl)carbodiimid hydrochlorid (305 mg, 1.59 mmol) wurde 5 Minuten bei Raumtemperatur gerührt und anschließend 4-Methoxybenzolsulfonamid (99.3 mg, 0.53 mmol) zugegeben. Das Reaktionsgemisch wurde über Nacht bei Raumtemperatur weitergerührt. Anschließend wurde das Lösungsmittel im Vakuum entfernt, der Rückstand in wässriger HCl (2 N) gelöst und das Gemisch mehrfach mit Ethylacetat extrahiert. Die vereinte organische Phase wurde getrocknet und das Lösungsmittel entfernt. Der Rückstand wurde mittels Säulenchromatographie (RP18) aufgereinigt, um das gewünschte Produkt (100 mg, 52%) zu erhalten.
logP (neutral): 0.56; MH+: 358.0; ¹H-NMR (400MHz, D6-DMSO) δ ppm: 7.99 (s, 1H), 7.93 (m, 2H), 7.14 (m, 2H), 3.96 (m, 2H), 3.85 (s, 3H), 1.69 (m, 2H), 0.82 (m, 3H).

### Herstellung von Ethyl-(-3-[(2-chlorphenyl)amino]-2-nitroacrylat

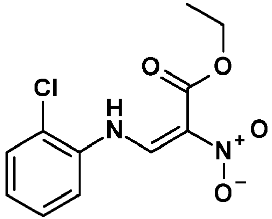

5,99g (45mmol) Nitroethylacetat und 4,775g (45mmol) Trimethylorthoformat wurden in 50ml Methanol gelöst, mit 5,74g(45mmol) 2-Chloranilin versetzt und über Nacht bei Rückfluß erhitzt. Der ausgefallene Feststoff wurde abgesaugt, mit Methanol gewaschen und getrocknet.
Ausbeute 7,4g (60% d. Th)
logP (HCOOH): 2,92; MH+: 271,0
¹H-NMR (400MHz, D6-DMSO) δ ppm: 1,3 (m 3H), 4,3 (m, 1H), 4,4 (m, 1H), 7,3 (m, 1H), 7,5 (m, 1H), 7,65 (m, 1H), 7,75 (m, 1H), 8,6 (m) 9,2 (m) (beide zusammen 1H), 11 (s) 11,5 (s) (beide zusammen 1H)

### Herstellung von Ethyl-1-(2-chlorphenyl)-2-methyl-1H-imidazol-4-carboxylat

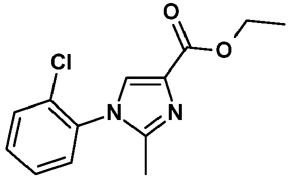

16,5g(60,96 mmol) Ethyl-(2E)-3-[(2-chlorphenyl)amino]-2-nitroacrylat wurden in 60ml Triethylorthoacetat bei 70°C und 2,5 bar Wasserstoff und 3,2g Platin auf Kohle (10%) 1,5h umgesetzt. Man saugte durch Celite ab, wusch mit Methanol nach und dampfte ein. Der Rückstand wurde an Kieselgel chromatographisch gereinigt (Cyclohexan/Ethylacetat).
Ausbeute 9,94g (59% d. Th)
logP (neutral): 2,06; MH+: 265,1
¹H-NMR (400MHz, D6-DMSO) δ ppm: 1,3 (t, 3H), 2,3 (s, 3H), 4,2-4,3 (q, 2H), 7,6-7,7 (m, 4H), 7,75 (m, 1H), 7,95 (s, 1H)

### Herstellung von 1-(2-Chlorphenyl)-2-methyl-1H-imidazol-4-carbonsäure

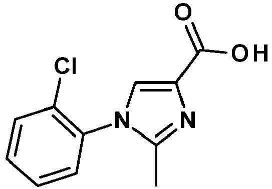

2,1g (7,9 mmol) Ethyl-1-(2-chlorphenyl)-2-methyl-1H-imidazol-4-carboxylat wurden bei -10°C mit 11,925g (47,6mmol) Bortribromid 2h gerührt. Dann wurde mit Wasser versetzt, 1h bei RT gerührt, das Dichlormethan abgedampft, über Nacht gerührt, abgesaugt, mit wäßrigem Ammoniak bis pH=7 versetzt und mit Ethylacetat extrahiert. Mit verdünnter Salzsäure wurde bis pH=5 versetzt, wiederum mit Ethylacetat extrahiert. Die vereinigten organischen Phasen wurden getrocknet uind eingedampft. Man erhielt so 0,65g (30% d.Th). Eine weitere Fraktion von 1g war durch Eindampfen der wäßrigen Phasen, verrühren mit THF, Absaugen und Eindampfen der THF-Phase zu gewinnen.
logP (HCOOH): 0.67; logP (neutral): -0,01 MH+: 237,0

### Herstellung von 1-(2-Chlorphenyl)-N-[(2-chlorphenyl)sulfonyl]-2-methyl-1H-imidazol-4-carboxamid

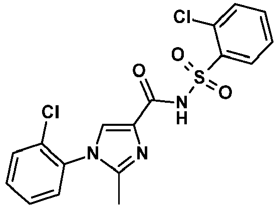

0,2g (0,845 mmol) 1-(2-Chlorphenyl)-2-methyl-1H-imidazol-4-carbonsäure wurd in THF mit 0,21g (1,27mmol) Carbonyldiimidazol 1b unter Rückfluß erhitzt, nach Abkühlen mit 0,243g (1,27mmol) 2-Chlorphenylsulfonsäureamid und 0,193g (1,27mmol) DBU versetzt. Nach Rühren über Nacht bei Raumtemperatur wurde eingedampft, in Dichlormethan und verdünnter Salzsäure gelöst. Die organische Phase wurde noch zweimal mit verdünnter Salzsäure gewaschen, getrocknet und eingedampft. Der Rückstand wurde mit Acetonitril verrührt, abgesaugt und getrocknet. Ausbeute 0,22g (63% d.Th.)
logP (neutral): 0.85; MH+: 410,0
¹H-NMR (400MHz, D6-DMSO) δ ppm: 2,4 (s, 3H), 7,5-7,8 (m, 7H), 8,1-8,2 (m, 2H)

### Herstellung von Methyl-1-(2,6-difluorbenzyl)-1H-imidazol-4-carboxylat

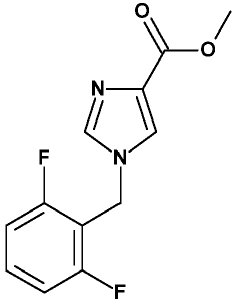

3,0 g (23,788 mmol) Imidazol-4-carbonsäuremethylester wurden in 100 ml Acetonitril vorgelegt, 3,29 g (23,788 mmol) Kaliumcarbonat zugefügt und über 20 Minuten bei Raumtemperatur gerührt. Anschliessend wurden 3,87 g (23,788 mmol) 2,6-Difluorbenzylchlorid gelöst in 5ml Acetonitril zugetropft und das Reaktionsgemisch vier Stunden bei 50°C gerührt. Nach dem Abkühlen wurde auf Natriumchloridlösung gegeben und mit Essigester ergiebig extrahiert. Die Chromatograpie an Kieselgel (Cyclohexan / Essigester 1/1) ergab 2,7 g (45% d.Th.) des gesuchten Produktes.
logP (sauer / neutral): 1.45 / 1.55; MH+: 253.1
1H-NMR (400MHz, D6-DMSO), δ ppm: 3,72 (s, 3H); 5,36 (s, 2H); 7,17-7,21 (m, 2H); 7,47-7,53 (m, 2H); 7,78 (s, 1H); 7,81 (s, 1H)

### Herstellung von 1-(2,6-Difluorbenzyl)-1H-imidazol-4-carbonsäure

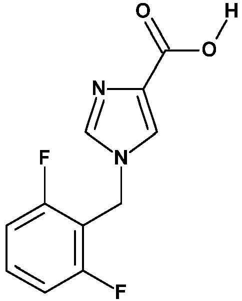

800 mg (3,172 mmol) Methyl-1-(2,6-difluorbenzyl)-1H-imidazol-4-carboxylat wurden in 10 ml THF p.a. vorgelegt und 266,2 mg (6.344 mmol) Lithiumhydroxidmonohydrat gelöst in 2 ml Wasser zugegeben. Der Ansatz wurde über vier Stunden bei Raumtemperatur gerührt. Nach dem Abziehen der flüchtigen Bestandteile wurde mit 2N HCl-Lösung auf pH 2 eingestellt, dabei kristallisierten 500 mg (66% d.Th.) 1-(2,6-Difluorbenzyl)-1H-imidazol-4-carbonsäure aus. Weitere 200 mg (26 % d.Th) des Produktes konnten durch Extraktion der wässrigen Phase gewonnen werden.
logP (sauer): 0,42 ; MH+: 239,1
1H-NMR (400MHz, D6-DMSO) δ ppm: 5,27 (s, 2H); 7,14-7,21 (m, 3H); 7,46-7,54 (m, 1H); 7,69 (s, 1H).

### Herstellung von N-[(2-Chlor-5-methoxyphenyl)sulfonyl]-1-(2,6-difluorbenzyl)-1H-imidazol-4-carboxamid

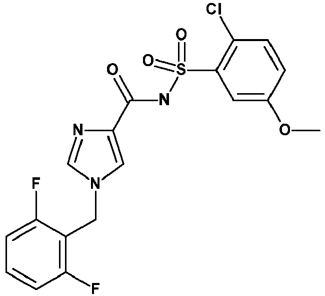

150 mg (0,63 mmol) 1-(2,6-Difluorbenzyl)-1H-imidazol-4-carbonsäure wurden in 20 ml Dichlormethan vorgelegt, dann 230,8 mg (1,889 mmol) 4-Dimethylaminopyridin sowie 362,2 mg (1,889 mmol) 1-(3-Dimethylaminopropyl)3-ethylcarbodiimidhydrochlorid (EDCxHCl) zugefügt. Es wurde fünf Minuten nachgerührt anschließend 139,59 mg (0,63 mmol) 2-Chlor-5-methoxybenzene-1-sulfonamid zugegeben und 18 Stunden bei Raumtemperatur gerührt. Danach wurde mit verdünnter Salzsäure versetzt, zweimal mit Dichlormethan extrahiert, die organische Phase mit ges. Kochsalzlösung gewaschen, über Natriumsulfat getrocknet und evaporiert. Nach Reinigung über präparative HPLC (Wasser/ Acetonitril) wurden 40 mg (14,4 % d. Th.) des N-[(2-Chlor-5-methoxyphenyl)sulfonyl]-1-(2,6-difluorbenzyl)-1H-imidazol-4-carboxamides erhalten.
logP (sauer): 2.18 ; MH+: 442,0

### Herstellung von Methyl-1-(3,3,3-trifluorpropyl)-1H-imidazol-4-carboxylat

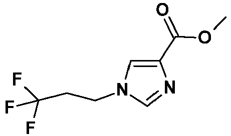

10,55g (68,46 mmol) Methyl-(2Z)-3-(dimethylamino)-2-isocyanoacrylat (kommerziell verfügbar, siehe auch WO2016/57924), 9,75g (66,2mmol) 2,2,2-Trifluorpropylamin-hydrochlorid (kommerziell verfügbar) und 9,27g (71,7mmol) Diisopropylethylamin wurden in 32g 1-Butanol im Autoklaven bei 95° 16° gerührt. Nach Abkühlen wurde die Mischung eingeengt, in Wasser / Ethylacetat gelöst, bei pH=8 extrahiert; die organische Phase wurde mit Na2SO4 getrocknet und eingeengt. Ausbeute 13,3g.
logP (HCOOH): 0,87; MH+: 223.1;

### Herstellung von Methyl-2,5-dibrom-1-(3,3,3-trifluorpropyl)-1H-imidazol-4-carboxylat

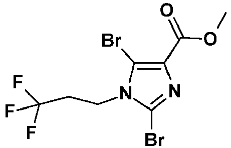

1,52g (6,84 mmol) Methyl-1-(3,3,3-trifluorpropyl)-1H-imidazol-4-carboxylat wurden in 50ml Acetonitril sukzessive mit 5g (27,8mmol) N-Brom-succinimid versetzt und insgesamt 2 Tage gerührt. Man versetzte mit wäßriger Natriumhydrogensulfit-Lösung, danach mit Ethylacetat. Bei pH=8 wurde 3 mal mit Ethylacetat extrahiert, die vereinigten organischen Phasen mit Na2SO4 getrocknet und eingengt. Der Rückstand wurde chromatographisch an Kieselgel mit Petrolether-Aceton gereinigt. Ausbeute 0,4g.
logP (HCOOH): 2,13 ; MH+: 380,9.

### Herstellung von 2,5-Dibrom-1-(3,3,3-trifluorpropyl)-1H-imidazol-4-carbonsäure

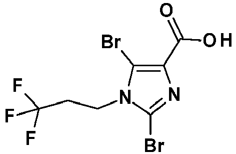

0,4g (1 mmol) Methyl-2,5-dibrom-1-(3,3,3-trifluorpropyl)-1H-imidazol-4-carboxylat wurden in Tetrahydrofuran gelöst und mit Natronlauge versetzt und die Mischung gerührt. Die Mischung wurde eingeengt, mit Wasser und verdünnter Salzsäure versetzt, das ausgefallene Produkt abgesaugt und getrocknet. Ausbeute 0,34g.
logP (HCOOH): 1,49 ; MH+: 366,9.

### Herstellung von 2,5-Dibrom-N-[(2-chlorphenyl)sulfonyl]-1-(3,3,3-trifluorpropyl)-1H-imidazol-4-carboxamid

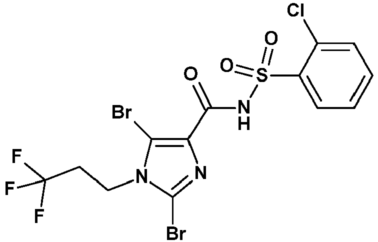

0,17g(0,46mmol) 2,5-Dibrom-1-(3,3,3-trifluorpropyl)-1H-imidazol-4-carbonsäure, 2-Chlorphenylsulfonsäureamid, 0,55g (2,86mmol) EDC-HCl und 0,3g 4-Dimethylaminopyridin (DMAP)n wurden in Tetrahydrofuran - Dichlormethan - Diisopropylamin gelöst und 3d gerührt. Die Mischung wurde eingeengt, in Ethylacetat, wäßrigem Natriumchlorid, Zitronensäure gelöst, 3 mal mit Ethylacetat extrahiert; die vereinigten organischen Phasen wurden mit Na2SO4 getrocknet und eingeengt. Der Rückstand wurde chromatographisch an Kieselgel RP-18, Wasser/Ameisensäure-Acetonitril gereinigt. Ausbeute 0,08g.
logP (HCOOH): 2,99 ; MH+: 539,6.

### Herstellung von Ethyl-5-amino-1-[2-(trifluormethyl)cyclopropyl]-1H-imidazol-4-carboxylat

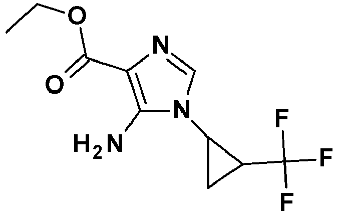

Zu einer Mischung von 30.0 g (97.9 mmol) Ethyl-3-nitriloalaninat (als Tosylat-Salz) und 15.0 g (101 mmol) HC(OEt)₃ in Acetonitril (500 mL) wurde 11.0 g (109 mmol) Triethylamin gegeben und die Lösung für 1 h unter Rückfluss erhitzt. 16.2 g (100 mmol) 2-(Trifluormethyl)cyclopropanamin (als Hydrochlorid), sowie eine weitere 11.0 g (109 mmol) Triethylamin wurden zugegeben und die Reaktionslösung für 1 h unter Rückfluss erhitzt. Alles flüchtige Material wurde im Vakuum entfernt und der Rückstand in 200 mL Wasser suspendiert. Der unlösliche Feststoff wurde abfiltriert und aus einer Mischung von Hexan/MTBE (7:3) umkristallisiert, um 12.0 g (45.6 mmol, 45%) des gewünschten Produktes zu erhalten.

### Herstellung von Ethyl-5-amino-2-chlor-1-[2-(trifluormethyl)cyclopropyl]-1H-imidazol-4-carboxylat

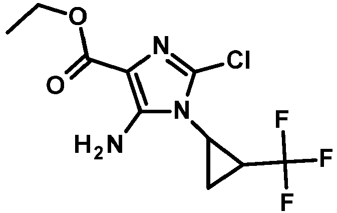

Zu einer Lösung von 12.0 g (45.6 mmol) Ethyl-5-amino-1-[2-(trifluormethyl)cyclopropyl]-1H-imidazol-4-carboxylat in Acetonitril (200 mL) wurde 9.30 g (54.6 mmol) CuCl₂·2H₂O gegeben und die Reaktionsmischung für 72 h unter Rückfluss erhitzt. Alles flüchtige Material wurde im Vakuum entfernt und der Rückstand mit Kaliumcarbonatlösung versetzt. Das Gemisch wurde mit Dichlormethan (3 x 300 mL) extrahiert und die vereinte organische Phase getrocknet (Na₂CO₃) and anschließend in Vakuum eingeengt. Der Rückstand wurde aus Hexan/Ethylacetat (9:1) umkristallisiert, um 7.50 g (25.2 mmol, 55%) des gewünschten Produkts zu erhalten.

### Herstellung von Ethyl-2,5-dichlor-1-[2-(trifluormethyl)cyclopropyl]-1H-imidazol-4-carboxylat

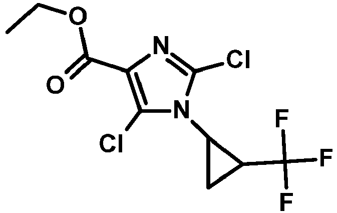

Eine Lösung von 7.0 g (23.5 mmol) Ethyl-5-amino-2-chlor-1-[2-(trifluormethyl)cyclopropyl]-1H-imidazol-4-carboxylat und 3.50 g (35.4 mmol) CuCl in Acetonitril (150 mL) wurde auf -10 °C abgekühlt und 3.65 g (35.4 mmol) *t*-BuONO über 30 min langsam zugetropft. Die Reaktionsmischung wurde auf Raumtemperatur erwärmt und für 1 h gerührt. Alles flüchtige Material wurde im Vakuum entfernt und der Rückstand mit Kaliumcarbonatlösung versetzt. Das Gemisch wurde mit Dichlormethan (3 x 100 mL) extrahiert. Die vereinte organische Phase wurde über Silicagel abfiltriert und anschließend in Vakuum eingeengt. Der Rückstand wurde aus Hexan umkristallisiert, um 3.40 g (10.7 mmol, 46%) des gewünschten Produkts zu erhalten.

### Herstellung von 2,5-Dichlor-1-[2-(trifluormethyl)cyclopropyl]-1H-imidazol-4-carbonsäure

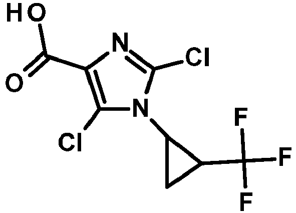

Zu einer Lösung von 3.40 g (10.7 mmol) Ethyl-2,5-dichlor-1-[2-(trifluormethyl)cyclopropyl]-1H-imidazol-4-carboxylat in THF (100 mL) wurde eine Lösung von 2.30 g (54.8 mmol) LiOH·H₂O in Wasser gegeben. Das Reaktionsgemisch wurde über Nacht bei Raumtemperatur gerührt. Anschließend wurde THF von der Mischung abdestilliert und die wässrige Phase mit IN HCl angesäuert. Der Feststoff wurde abfiltriert, aus Ethylacetat umkristallisiert und getrocknet. Das gewünschte Produkt wurde mit einer Ausbeute von 77% (2.40 g, 8.30 mmol) erhalten.
Ret_Time: 1.162 min
[M+H]⁺: 289.0

### Herstellung von N-[(2-Chlor-5-methoxyphenyl)sulfonyl]-1-(3,5-dichlorphenyl)-5-fluor-2-methyl-1H-imidazol-4-carboxamid

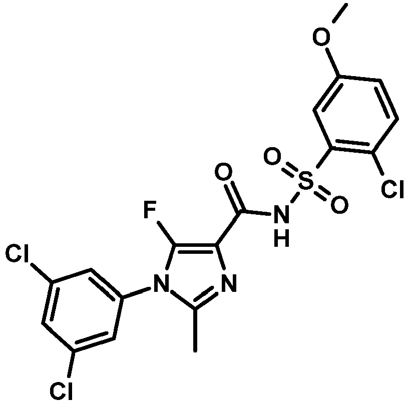

In einem Druckgefäß wurde zu einem Gemisch von N-[(2-Chlor-5-methoxyphenyl)sulfonyl]-1-(3,5-dichlorphenyl)-2-methyl-1*H*-imidazol-4-carboxamid (50 mg, 0.11 mmol) und 1-Chlormethyl-4-fluor-1,4-diazoniabicyclo[2.2.2]octanbis(tetrafluorborat) (149 mg, 0.42 mmol) unter Argon Acetonitril (2.5 ml) zugegeben. Das Reaktionsgemisch wurde 1.5 Stunden bei 80 °C kräftig gerührt, dann auf Raumtemperatur abgekühlt und filtriert. Der entstehende Niederschlag wurde zusätzlich mit Acetonitril (1 ml) gewaschen. Die Mutterlauge wurde anschließend im Vakuum zur Trockene eingeengt. Der Rückstand wurde mittels Säulenchromatographie (RP18) gereinigt,
Ausbeute (6 mg, 11%)
logP (sauer): 3.71; MH+: 494.0;

**Tabelle 1** führt in Folgenden weitere Verbindungen der Formel (I) auf, die analog zu den oben aufgeführten Beispielen hergestellt wurden. Die Synthese der Säurevorstufen erfolgte entweder wie oben beschrieben, oder die Säuren waren kommerziell erhältlich.

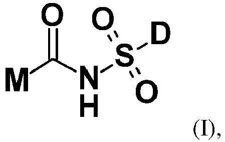

| **Beispiel Nr.** | **Struktur** | **NMR peak list** |
|---|---|---|
| I-001 | 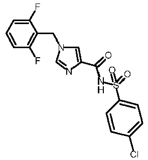 | Beispiel I-001: 1H-NMR(400.0 MHz, d6-DMSO): δ= 8.134(1.4);8.013(6.4);7.949(11.32);7.928(13.44);7.88(9.12);7.67(12.78);7.649(11.13);7.551(0 .85);7.534(1.95);7.53(2.01);7.513(3.82);7.496(2.06);7.492(2.35);7.475(1.02);7.214(0.99);7.20 5(6.21);7.185(9.96);7.164(5.31);7.155(0.89);5.754(10.6);5.353(16);4.278(0.33);4.243(0.34);4 .121(0.4);4.076(0.41);4.046(0.44);3.989(0.43);3.91(0.43);3.9(0.43);3.874(0.46);3.85(0.42);3. 816(0.41);3.791(0.47);3.777(0.39);3.744(0.44);3.716(0.55);2.677(0.59);2.672(0.82);2.668(0.6 1);2.542(0.33);2.525(1.97);2.507(102.25);2.503(140.59);2.498(108.84);2.334(0.67);2.33(0.88 );2.326(0.68);0.146(0.78);0.008(6.03);0(171.7);-0.008(8.75);-0.15(0.85) |
| I-002 | 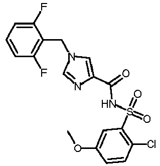 | Beispiel I-002: 1H-NMR(400.0 MHz, d6-DMSO): δ= 8.111(1.44);7.974(3.36);7.572(3.02);7.564(3.23);7.55(0.81);7.546(0.77);7.529(1.43);7.507(3. 16);7.485(2.91);7.232(1.85);7.224(3.34);7.21(1.91);7.203(4.86);7.183(1.93);7.173(0.37);5.37 4(5.96);3.831(16);2.671(0.34);2.506(45.04);2.502(58.65);2.498(44.46);2.329(0.35);2.074(0.4 3);0(17.74) |
| I-003 | 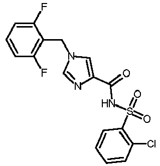 | Beispiel I-003: 1H-NMR(400.0 MHz, d6-DMSO): δ= 8.114(4.31);8.094(4.67);8.066(5.81);7.974(8.32);7.665(1.2);7.647(3.54);7.629(3.82);7.606(6. 79);7.583(4.06);7.562(4.65);7.545(3.68);7.528(3.7);7.508(2.32);7.49(0.98);7.232(1.06);7.223 (5.8);7.203(9.68);7.183(5.02);7.173(0.98);6.961(0.49);5.368(16);4.855(0.71);4.758(0.33);4.7 46(0.4);4.737(0.35);4.725(0.32);3.738(1.72);3.17(1.46);2.672(0.92);2.542(0.75);2.503(165.65 );2.33(1);0.147(0.85);0(177.13);-0.149(0.93) |
| I-004 | 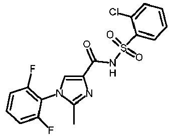 | Beispiel I-004: 1H-NMR(400.0 MHz, d6-DMSO): δ= 8.314(0.38);8.186(2.21);8.151(1.96);8.148(2.14);8.131(2.11);8.128(2.23);7.747(0.38);7.731(0 .85);7.726(0.81);7.709(1.71);7.704(1.09);7.693(1.03);7.687(1.56);7.683(1.66);7.672(0.85);7.6 62(1.91);7.65(3.09);7.634(1.09);7.611(1.28);7.607(1.18);7.591(1.81);7.574(0.81);7.57(0.76); 7.476(2.75);7.454(4.65);7.434(2.22);3.65(0.32);3.637(0.33);3.604(0.35);3.583(0.35);3.569(0. 35);3.547(0.35);3.512(0.35);3.488(0.35);3.435(0.33);2.675(0.77);2.67(1.11);2.666(0.84);2.52 4(2.1);2.506(136.37);2.501(181.02);2.497(136.48);2.384(0.32);2.332(0.95);2.328(1.27);2.324 (1);2.223(16);0(3.59) |
| I-005 | 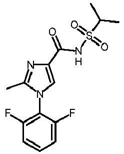 | Beispiel I-005: 1H-NMR(400.0 MHz, d6-DMSO): δ= 8.229(4.62);7.732(0.7);7.727(0.69);7.711(1.37);7.694(0.78);7.689(0.88);7.673(0.39);7.48(2.2 4);7.459(3.96);7.438(1.87);3.798(0.45);3.781(1.19);3.764(1.65);3.746(1.23);3.729(0.48);3.32 6(1.02);2.671(0.43);2.502(66.57);2.329(0.43);2.215(14.37);1.318(16);1.301(15.8);0.146(0.34) ;0(66.84);-0.15(0.35) |
| I-006 | 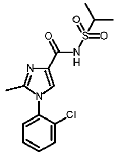 | Beispiel I-006: 1H-NMR(400.0 MHz, d6-DMSO): δ= 8.107(5.18);7.78(1.46);7.777(1.63);7.76(1.87);7.757(2.05);7.669(1.16);7.665(1.38);7.651(1.7 5);7.646(2.33);7.638(0.87);7.623(1.83);7.618(1.46);7.604(1.35);7.599(1.04);7.591(1.54);7.58 7(1.61);7.572(1.57);7.569(1.54);7.553(0.53);7.55(0.5);3.804(0.45);3.787(1.13);3.77(1.54);3.7 52(1.19);3.735(0.47);3.339(0.8);2.671(0.4);2.506(48.6);2.502(63.69);2.498(48.56);2.328(0.4) ;2.151(16);1.317(14.68);1.3(14.49);0.146(0.34);0.007(3.03);0(66.15);-0.15(0.35) |
| I-007 | 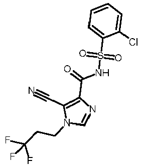 | Beispiel I-007: 1H-NMR(400.0 MHz, d6-DMSO): δ= 8.462(0.41);8.315(16);8.17(5.61);8.166(6.27);8.15(6.1);8.147(6.65);7.732(1.54);7.728(1.82); 7.709(4.79);7.694(4.88);7.69(5.37);7.667(9.46);7.65(4.28);7.647(4.19);7.64(4.38);7.636(4.21) ;7.619(5.95);7.602(2.59);7.598(2.61);4.626(0.42);4.608(0.33);4.447(7.03);4.43(14.63);4.413( 7.24);3.995(0.34);3.854(0.51);3.78(0.48);3.716(0.53);3.672(0.53);3.637(0.55);3.602(0.56);3.5 62(0.55);3.532(0.56);3.505(0.54);3.484(0.54);3.452(0.52);3.393(0.48);3.314(0.41);3.286(0.39 );3.235(0.36);3.228(0.35);3.186(0.36);3.021(1.06);3.004(2.17);2.993(3.22);2.976(5.97);2.966 (3.93);2.959(3.71);2.949(6.13);2.932(3.11);2.921(2.28);2.904(1.08);2.675(1.4);2.67(1.83);2.6 66(1.39);2.506(234.23);2.501(297.02);2.497(220.35);2.387(0.37);2.332(1.6);2.328(2.04);2.32 4(1.6);2.073(4.19);0.146(1.51);0.008(19.99);0(346.46);-0.008(18.84);-0.042(0.73);-0.064(0.37);-0.15(1.69) |
| I-008 | 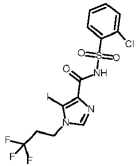 | Beispiel I-008: 1H-NMR(400.0 MHz, d6-DMSO): δ= 8.313(0.62);8.242(16);8.153(5.29);8.15(5.97);8.134(5.84);8.131(6.31);7.725(1.42);7.722(1.68 );7.702(4.65);7.688(5.03);7.684(5.53);7.666(9.47);7.649(3.79);7.646(3.47);7.635(4.09);7.632 (3.93);7.615(5.64);7.598(2.46);7.594(2.5);4.312(6.28);4.295(12.1);4.277(6.81);2.904(0.59);2. 885(1.5);2.876(2.58);2.858(4.72);2.848(3.36);2.841(3.14);2.83(4.95);2.82(1.77);2.813(2.66); 2.803(1.78);2.785(0.72);2.67(1.25);2.505(191.74);2.501(253.16);2.497(201.42);2.332(1.84);2 .328(2.26);2.21(0.42);2.139(0.33);2.135(0.33);2.073(1.4);0(6.04) |
| I-009 | 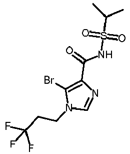 | Beispiel I-009: 1H-NMR(400.0 MHz, d6-DMSO): δ= 8.148(5.41);4.354(2.22);4.336(4.55);4.319(2.3);3.778(0.45);3.761(1.16);3.744(1.58);3.727(1. 2);3.71(0.47);3.319(4.42);2.928(0.61);2.918(0.97);2.901(1.78);2.89(1.12);2.884(1.04);2.873( 1.83);2.863(0.56);2.856(0.9);2.845(0.64);2.675(0.42);2.671(0.54);2.506(65.63);2.502(84.73); 2.498(65.01);2.328(0.55);1.301(16);1.284(15.77);0(0.48) |
| I-010 | 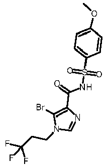 | Beispiel I-010: 1H-NMR(400.0 MHz, d6-DMSO): δ= 8.12(4.93);7.948(0.48);7.94(4.06);7.936(1.56);7.923(1.33);7.918(4.44);7.911(0.54);7.154(0.5 3);7.147(4.19);7.129(1.3);7.124(4.05);4.315(1.7);4.297(3.55);4.28(1.75);3.847(16);3.32(0.95) ;2.886(0.49);2.876(0.75);2.858(1.39);2.848(0.89);2.841(0.82);2.83(1.42);2.819(0.45);2.813(0. 71);2.803(0.51);2.671(0.36);2.51(21.3);2.506(41.07);2.502(54.7);2.497(42.04);2.328(0.36);2. 073(3.3);0(0.41) |
| I-011 | 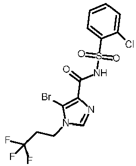 | Beispiel I-011: 1H-NMR(400.0 MHz, d6-DMSO): δ= 8.313(0.35);8.171(16);8.15(5.15);8.147(5.97);8.13(5.48);8.127(6.15);7.724(1.47);7.721(1.7); 7.704(4.7);7.701(4.8);7.686(5);7.683(5.46);7.665(9.29);7.648(3.58);7.645(3.36);7.63(3.8);7.6 27(3.83);7.61(5.55);7.593(2.35);7.59(2.43);4.323(6.42);4.305(12.81);4.288(6.81);3.952(0.33) ;3.932(0.33);3.894(0.34);3.848(0.38);3.816(0.36);3.782(0.37);3.754(0.38);3.69(0.38);3.676(0. 38);3.635(0.37);3.608(0.37);3.599(0.37);3.586(0.36);3.569(0.37);3.548(0.35);3.517(0.34);3.5 04(0.34);3.482(0.33);3.461(0.32);3.441(0.32);2.923(0.8);2.905(1.73);2.895(2.8);2.878(5.06); 2.868(3.55);2.861(3.43);2.85(5.27);2.833(2.78);2.822(2.01);2.805(0.86);2.671(0.95);2.667(0. 79);2.506(125.21);2.502(167.3);2.498(143.33);2.328(1.4);2.256(0.44);2.073(1.66);0(0.83) |
| I-012 | 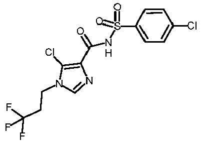 | Beispiel I-012: 1H-NMR(400.0 MHz, d6-DMSO): δ= 8.076(14.94);8.013(1.49);8.006(13.39);8.002(4.45);7.989(4.66);7.985(16);7.978(1.96);7.885( 0.36);7.863(0.44);7.742(1.82);7.736(14.47);7.714(12.49);7.68(0.33);4.318(5.9);4.3(12.74);4.2 83(6.17);4.146(0.34);4.116(0.36);4.099(0.38);4.086(0.37);4.066(0.36);4.059(0.38);4.044(0.38 );4.026(0.4);4.001(0.42);3.983(0.46);3.954(0.49);3.911(0.56);3.901(0.57);3.895(0.63);3.87(0. 62);3.851(0.64);3.836(0.68);3.813(0.7);3.754(0.96);3.746(0.97);3.738(1.03);3.375(42.67);3.1 94(3.39);3.002(1.03);2.967(0.81);2.94(0.71);2.924(1.29);2.908(2.08);2.897(2.96);2.88(5.3);2. 869(3.35);2.863(3.03);2.852(5.39);2.841(1.8);2.835(2.72);2.824(2.08);2.807(1.08);2.787(0.56 );2.769(0.53);2.751(0.47);2.739(0.48);2.717(0.91);2.7(0.45);2.682(2.72);2.678(3.73);2.673(2. 69);2.669(1.49);2.654(0.44);2.624(0.49);2.619(0.47);2.581(0.71);2.548(143.66);2.531(11.01); 2.517(208.17);2.513(409.48);2.509(534.67);2.504(390.07);2.5(190.65);2.462(0.79);2.446(0.6 4);2.413(0.42);2.401(0.66);2.374(0.69);2.34(2.64);2.335(3.47);2.331(2.56);1.512(0.43);1.305 (0.35);1.265(0.45);1.258(0.37);1.242(1.05);1.153(0.38);0.007(0.59) |
| I-013 | 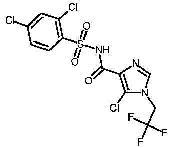 | Beispiel I-013: 1H-NMR(400.0 MHz, d6-DMSO): δ= 8.14(8.85);8.118(16);7.88(5.64);7.726(3.78);7.714(2.43);7.704(3.61);7.693(2.3);7.545(1.27); 7.54(1.35);7.4(0.84);7.395(0.86);7.379(0.75);7.373(0.75);7.208(0.63);7.08(0.69);6.953(0.67); 5.175(2.33);5.153(6.53);5.13(6.76);5.108(2.42);4.132(0.35);4.099(0.38);4.083(0.4);4.076(0.3 8);4.025(0.42);4.01(0.45);3.987(0.51);3.973(0.51);3.923(0.62);3.911(0.63);3.899(0.64);3.869 (0.71);3.832(0.83);3.634(2.42);3.374(153.96);3.193(5.22);3.002(1.31);2.985(1.06);2.916(0.82 );2.846(0.62);2.811(0.56);2.766(0.52);2.737(0.48);2.731(0.48);2.726(0.51);2.718(0.63);2.682 |
| | | (5.66);2.678(7.64);2.673(5.67);2.641(0.48);2.589(0.77);2.548(53.37);2.531(22.4);2.513(883.5 4);2.509(1144.63);2.504(842.05);2.5(418.8);2.411(0.83);2.375(0.68);2.34(5.63);2.336(7.47);2 .331(5.61);2.297(0.66);2.288(0.41);2.268(0.34);2.2(0.33);1.304(0.55);1.265(0.74);1.242(1.68) ;1.154(0.83);0.861(0.34);0.007(0.4) |
| I-014 | 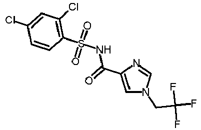 | Beispiel I-014: 1H-NMR(400.0 MHz, d6-DMSO): δ= 8.136(1.34);8.114(2.18);8.062(1.21);7.859(0.96);7.854(1.09);7.713(0.66);7.708(0.68);7.691(0 .62);7.686(0.65);5.177(0.91);5.154(0.98);5.131(0.35);3.452(16);2.684(0.41);2.679(0.56);2.67 4(0.41);2.549(11.21);2.532(1.7);2.519(31.46);2.515(62.36);2.51(81.38);2.506(58.53);2.501(2 7.81);2.341(0.37);2.337(0.52);2.332(0.35) |
| I-015 | 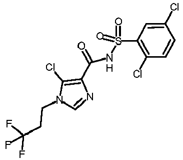 | Beispiel I-015: 1H-NMR(400.0 MHz, d6-DMSO): δ= 8.18(7.29);8.077(4.17);8.07(4.35);7.816(1.94);7.809(1.83);7.794(2.94);7.788(2.85);7.716(5.3 1);7.695(3.49);7.62(0.49);7.613(0.53);7.477(0.39);7.471(0.35);7.409(0.66);7.388(0.42);4.33( 2.35);4.313(5);4.296(2.48);3.555(16);2.949(0.34);2.932(0.67);2.921(1.03);2.904(1.96);2.894( 1.17);2.887(1.07);2.876(2.03);2.866(0.55);2.859(0.97);2.848(0.73);2.831(0.34);2.718(0.34);2. 682(0.58);2.678(0.77);2.673(0.55);2.548(81.38);2.531(2.19);2.518(42.82);2.513(85.39);2.509 (112.12);2.504(80.93);2.5(38.74);2.374(0.33);2.34(0.52);2.335(0.71);2.331(0.51) |
| I-016 | 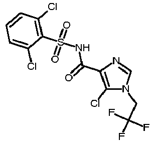 | Beispiel I-016: 1H-NMR(400.0 MHz, d6-DMSO): δ= 8.222(0.35);8.132(10.6);7.683(5.27);7.678(6.94);7.671(0.8);7.661(15.14);7.654(1.53);7.628(7 .28);7.619(0.62);7.611(4.44);7.604(3.27);7.596(0.35);7.588(2.28);5.18(1.76);5.158(5.69);5.13 5(6.01);5.113(2.09);3.489(16);3.003(0.66);2.719(0.74);2.688(0.54);2.684(0.97);2.679(1.25);2 .674(0.93);2.67(0.5);2.549(170.03);2.532(3.54);2.528(5.46);2.519(63.29);2.514(126.49);2.51( 165.68);2.505(117.84);2.501(54.66);2.376(0.59);2.346(0.36);2.341(0.78);2.337(1.07);2.332(0 .74);2.328(0.35) |
| I-017 | 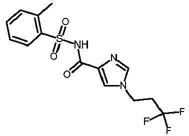 | Beispiel I-017: 1H-NMR(400.0 MHz, d6-DMSO): δ= 8.029(3.74);8.002(4.98);7.984(2.05);7.981(1.99);7.561(0.74);7.558(0.76);7.542(1.84);7.539(1 .78);7.523(1.21);7.52(1.14);7.431(1.16);7.412(1.85);7.394(0.84);7.374(2.07);7.355(1.66);4.33 6(2.37);4.318(5.03);4.301(2.51);3.708(0.37);3.393(81.35);3.105(0.38);3.001(0.33);2.963(0.48 );2.945(0.78);2.935(1.15);2.928(0.63);2.917(2.07);2.907(1.27);2.9(1.17);2.889(2.11);2.878(0. 63);2.872(1.05);2.862(0.8);2.844(0.41);2.718(0.38);2.687(0.7);2.682(1.41);2.678(1.9);2.673( 1.4);2.669(0.7);2.589(16);2.548(85.39);2.531(5.46);2.526(8.75);2.518(106.15);2.513(212.57); 2.509(278.75);2.504(201.24);2.5(96.41);2.374(0.34);2.345(0.64);2.34(1.34);2.335(1.83);2.33 1(1.32);2.326(0.64);1.242(0.4) |
| I-018 | 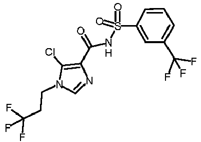 | Beispiel I-018: 1H-NMR(400.0 MHz, d6-DMSO): δ= 8.32(0.34);8.308(3.8);8.286(10.71);8.133(3.41);8.113(4.39);8.1(16);7.93(3.03);7.91(5.24);7.8 91(2.46);4.318(5.87);4.301(12.79);4.284(6.13);3.938(0.32);3.928(0.32);3.925(0.33);3.916(0.3 4);3.857(0.44);3.844(0.43);3.816(0.49);3.4(246.28);3.174(1.6);3.056(0.64);3.008(0.5);3.001( 0.52);2.974(0.43);2.955(0.41);2.924(1.04);2.906(1.91);2.896(2.72);2.89(1.52);2.879(5.19);2.8 68(3.09);2.862(2.86);2.851(5.35);2.84(1.54);2.834(2.55);2.823(2);2.806(0.96);2.718(0.58);2. 687(1.23);2.682(2.37);2.678(3.17);2.673(2.34);2.669(1.16);2.596(0.35);2.548(110.22);2.531( |
| | | 9.47);2.526(14.93);2.518(174.42);2.513(349.41);2.509(458.33);2.504(328.48);2.5(154.99);2. 442(0.33);2.425(0.85);2.374(0.48);2.345(1.07);2.34(2.23);2.335(3.03);2.331(2.18);2.326(1.01 );1.264(0.35);1.241(0.73) |
| I-019 | 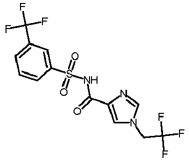 | Beispiel I-019: 1H-NMR(400.0 MHz, d6-DMSO): δ= 8.288(1.01);8.272(3.07);8.12(0.87);8.1(1.08);8.062(2.64);8.044(2.54);7.915(0.73);7.894(1.24) ;7.874(0.54);5.177(0.54);5.154(1.73);5.131(1.84);5.108(0.64);3.502(16);2.548(26.78);2.531(0 .82);2.526(1.27);2.518(16.16);2.513(32.31);2.509(42.28);2.504(30.25);2.5(14.34) |
| I-020 | 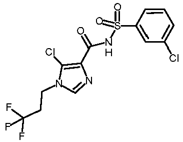 | Beispiel I-020: 1H-NMR(400.0 MHz, d6-DMSO): δ= 8.316(0.42);8.086(16);7.993(5.58);7.988(10.09);7.984(7.07);7.957(5.28);7.938(5.94);7.812(3. 81);7.789(5.21);7.696(5.99);7.676(9.27);7.656(3.86);5.756(1.32);4.315(6.81);4.298(14.61);4. 28(7.16);3.47(0.73);3.423(0.76);3.417(0.75);3.382(0.75);3.285(0.64);3.263(0.61);3.259(0.6); 3.187(0.51);3.109(0.37);2.921(1.02);2.904(2.02);2.893(3.05);2.876(5.76);2.865(3.62);2.86(3. 43);2.848(5.97);2.831(2.94);2.821(2.21);2.803(0.99);2.675(1.1);2.671(1.48);2.667(1.17);2.64 4(0.34);2.541(0.62);2.524(3.01);2.506(167.11);2.502(220.75);2.498(167.57);2.333(1.13);2.32 9(1.53);2.324(1.2);1.505(0.78);0.146(0.95);0.008(7.71);0(229.05);-0.008(12.48);-0.15(1.05) |
| I-021 | 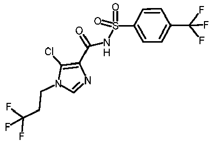 | Beispiel I-021: 1H-NMR(400.0 MHz, d6-DMSO): δ= 8.21(9.74);8.19(12.76);8.094(16);8.053(12.87);8.032(10.01);8.011(0.41);7.213(0.34);7.085(0. 39);6.957(0.38);4.319(6.94);4.302(15);4.285(7.31);4.12(0.34);4.089(0.32);4.072(0.32);4.04(0 .36);3.981(0.41);3.949(0.43);3.892(0.53);3.869(0.55);3.818(0.69);3.8(0.74);3.791(0.76);3.381 (822.34);3.067(1.11);3.001(0.88);2.967(0.65);2.924(1.39);2.906(2.42);2.896(3.4);2.879(6.26) ;2.868(3.94);2.862(3.62);2.851(6.5);2.834(3.19);2.823(2.48);2.806(1.27);2.786(0.47);2.777(0. 45);2.752(0.44);2.719(1.16);2.683(4.62);2.679(6.16);2.674(4.55);2.67(2.29);2.625(0.54);2.54 9(231.23);2.532(17.98);2.527(28.7);2.518(347.95);2.514(689.63);2.51(898.56);2.505(646.66) ;2.5(308.12);2.426(0.72);2.375(1.01);2.345(2.12);2.341(4.35);2.336(5.87);2.332(4.19);2.3(0.4 7);1.512(0.52);1.336(0.37);1.305(0.4);1.265(0.6);1.242(1.58);1.155(0.62) |
| I-022 | 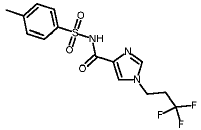 | Beispiel I-022: 1H-NMR(400.0 MHz, d6-DMSO): δ= 8.009(4.28);7.937(3.65);7.863(5.21);7.842(5.72);7.407(4.45);7.386(4.01);4.318(2.57);4.3(5.4 7);4.283(2.66);3.694(0.52);3.657(0.65);3.385(26.85);3.089(0.52);2.995(0.4);2.943(0.56);2.92 5(0.89);2.914(1.26);2.908(0.7);2.897(2.26);2.886(1.4);2.88(1.24);2.869(2.32);2.858(0.7);2.85 2(1.15);2.841(0.88);2.824(0.45);2.68(0.71);2.675(1.4);2.671(1.87);2.666(1.37);2.662(0.67);2. 541(6.44);2.524(5.88);2.519(9.35);2.511(106.54);2.506(211.15);2.502(275.58);2.497(197.66) ;2.492(93.42);2.381(16);2.337(0.7);2.333(1.38);2.328(1.83);2.324(1.33);2.319(0.64);1.235(0. 4);0(0.88) |
| I-023 | 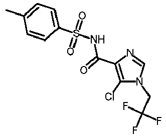 | Beispiel I-023: 1H-NMR(400.0 MHz, d6-DMSO): δ= 8.085(5.82);7.888(5.1);7.867(5.63);7.842(0.4);7.439(4.45);7.419(4.16);5.161(1.02);5.138(3.2 7);5.116(3.44);5.093(1.18);3.331(46.78);2.675(0.72);2.671(0.97);2.666(0.7);2.541(63.01);2.5 24(2.93);2.511(59.34);2.506(115.57);2.502(149.35);2.497(108.18);2.493(52.95);2.459(0.35); 2.395(16);2.368(0.36);2.333(0.74);2.329(0.98);2.324(0.73);0(0.37) |
| I-024 | 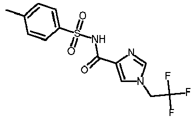 | Beispiel I-024: 1H-NMR(400.0 MHz, d6-DMSO): δ= 8.022(4.27);7.913(4.41);7.875(5.34);7.854(5.88);7.428(4.59);7.408(4.23);5.159(0.96);5.136(3 .07);5.113(3.24);5.09(1.12);3.574(0.64);3.526(1.06);3.358(4.5);2.676(0.56);2.671(0.73);2.666 (0.55);2.541(13.12);2.524(2.37);2.511(42.85);2.506(82.53);2.502(105.92);2.497(76.08);2.493 (36.33);2.389(16);2.333(0.56);2.329(0.7);2.324(0.52) |
| I-025 | 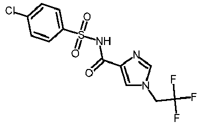 | Beispiel I-025: 1H-NMR(400.0 MHz, d6-DMSO): δ= 8.048(8.63);8.005(1.5);7.998(13.59);7.994(4.11);7.982(4.63);7.977(16);7.97(2.46);7.964(8.85 );7.733(1.87);7.726(15.79);7.721(4.39);7.709(4.12);7.704(13.1);7.698(1.41);5.174(1.9);5.152 (6.16);5.128(6.55);5.106(2.26);4.019(0.39);3.904(0.62);3.536(7.46);3.511(7.73);3.505(7.74); 3.174(0.93);3.066(0.48);3.057(0.46);3.053(0.45);3.02(0.39);3.002(0.56);2.964(0.35);2.718(0. 43);2.687(0.62);2.682(1.07);2.678(1.4);2.673(1.01);2.669(0.54);2.579(0.34);2.548(74.35);2.5 31(3.66);2.526(5.99);2.518(73.57);2.513(145.82);2.508(189.52);2.504(134.42);2.499(62.56); 2.344(0.45);2.34(0.92);2.335(1.25);2.331(0.87) |
| I-026 | 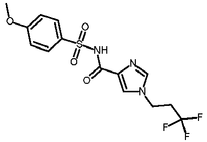 | Beispiel I-026: 1H-NMR(400.0 MHz, d6-DMSO): δ= 8.012(3.25);8.009(3.12);7.922(4.33);7.916(2.24);7.911(2.48);7.905(2.18);7.899(4.5);7.892(0. 74);7.126(4.2);7.121(1.41);7.108(1.54);7.104(3.92);7.096(0.52);4.316(1.77);4.299(3.77);4.28 1(1.93);3.837(16);2.922(0.59);2.912(0.84);2.894(1.53);2.884(0.98);2.877(0.93);2.867(1.53);2 .856(0.52);2.849(0.76);2.839(0.55);2.671(0.36);2.51(21.79);2.506(39.95);2.502(49.8);2.497(3 5.79);2.493(17.77);1.488(2);1.233(0.32);0.146(0.4);0(86.75);-0.008(4.78);-0.15(0.39) |
| I-027 | 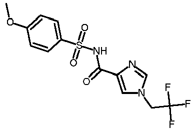 | Beispiel I-027: 1H-NMR(400.0 MHz, d6-DMSO): δ= 8.026(2.84);7.94(1.51);7.936(4.4);7.93(1.53);7.913(7.13);7.153(1.52);7.149(4.44);7.144(1.41) ;7.131(2.11);7.126(3.96);7.119(0.42);5.164(0.74);5.141(2.08);5.118(2.12);5.095(0.72);3.853( 5.43);3.848(16);3.664(0.41);3.367(6.38);2.682(0.44);2.677(0.5);2.672(0.36);2.552(16.99);2.5 48(57.02);2.517(35.83);2.513(58.88);2.508(67.24);2.504(45.88);2.499(20.42);2.34(0.37);2.33 5(0.44);1.502(0.5) |
| I-028 | 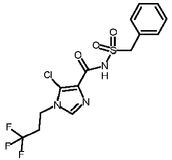 | Beispiel I-028: 1H-NMR(400.0 MHz, d6-DMSO): δ= 20.007(1.29);8.052(7);7.99(1.2);7.374(10.13);7.367(16);7.346(8.34);7.338(6);7.327(4.28);4.8 03(9.02);4.359(4.81);4.342(9.81);4.325(5.06);3.334(1103.06);3.216(3.24);2.973(1.43);2.944( 2.52);2.928(4.6);2.916(3.17);2.9(5);2.883(2.97);2.872(2.27);2.682(12.02);2.678(15.93);2.673 (11.77);2.548(85.22);2.531(45.08);2.518(943.45);2.513(1881.46);2.509(2461.77);2.504(1774 .37);2.5(849.47);2.34(11.74);2.335(15.4);2.331(10.87);2.295(1.36);1.512(2.91);1.335(1.22);1. 304(1.23);1.265(2.03);1.257(1.75);1.242(4.65);1.155(1.68);0.904(1.49);0.007(3.23) |
| I-029 | 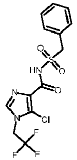 | Beispiel I-029: 1H-NMR(400.0 MHz, d6-DMSO): δ= 17.914(0.47);11.402(0.48);8.086(7.1);7.938(0.46);7.405(0.56);7.386(5.24);7.373(16);7.355(8. 38);7.346(5.62);7.336(3.75);7.316(0.74);5.221(0.59);5.211(1.82);5.189(5.67);5.167(5.89);5.1 44(2.33);5.121(0.53);4.809(10.15);3.905(0.52);3.866(0.51);3.794(0.51);3.73(0.61);3.704(0.47 );3.671(0.66);3.653(0.53);3.632(0.73);3.588(0.78);3.559(0.93);3.548(1.11);3.538(1.27);3.522 (1.33);3.496(1.69);3.482(1.66);3.344(2638.83);3.21(2.59);3.176(1.98);3.137(1.07);3.103(1.06 );3.098(0.99);3.082(0.98);3.031(0.77);3.01(0.73);2.937(0.66);2.918(0.48);2.898(0.5);2.887(0. 52);2.877(0.53);2.859(0.56);2.855(0.52);2.849(0.5);2.84(0.49);2.815(0.56);2.734(0.6);2.716( 0.63);2.682(6.69);2.678(8.95);2.673(6.46);2.604(0.9);2.586(0.6);2.548(97.21);2.517(557.81); 2.513(1063.86);2.509(1377.3);2.504(1008.9);2.44(1.86);2.373(1.08);2.34(6.76);2.335(8.96);2 .331(6.56);2.298(0.73);2.282(0.51);2.275(0.56);1.9(0.48);1.52(3.21);1.498(0.51);1.305(0.77); 1.265(0.98);1.252(1.14);1.242(2.37);1.156(1);0.908(0.56);0.86(0.54);-3.449(0.47) |
| I-030 | 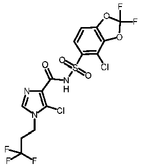 | Beispiel I-030: 1H-NMR(400.0 MHz, d6-DMSO): δ= 8.316(0.41);8.149(14.19);8.134(16);7.907(15.3);4.322(5.18);4.305(10.78);4.288(5.47);2.94(0. 64);2.922(1.38);2.912(2.17);2.895(4.15);2.884(2.65);2.878(2.44);2.867(4.32);2.85(2.11);2.83 9(1.56);2.822(0.67);2.676(0.74);2.671(0.99);2.667(0.74);2.524(2);2.507(126.58);2.502(162.8 6);2.498(120.15);2.333(0.85);2.329(1.08);2.325(0.84);0.146(0.39);0.008(3.13);0(92.07);-0.008(5.4);-0.15(0.44) |
| I-031 | 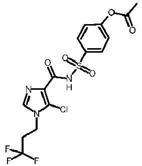 | Beispiel I-031: 1H-NMR(400.0 MHz, d6-DMSO): δ= 12.171(0.34);12.164(0.33);8.142(5.63);8.137(2.93);8.037(0.52);8.014(0.42);8.007(4.01);8.00 3(1.9);7.991(1.36);7.985(4.72);7.979(1.36);7.912(1.28);7.907(0.6);7.896(0.44);7.89(1.54);7.8 27(0.4);7.805(0.42);7.531(0.47);7.524(4.38);7.519(1.95);7.507(1.31);7.502(4.31);7.495(1.06) ;7.468(0.33);7.461(1.51);7.456(0.68);7.445(0.57);7.439(1.66);7.433(2.25);6.926(0.38);6.904( 0.39);4.4(2.43);4.383(5.3);4.366(2.61);4.288(0.36);3.328(11.51);2.985(0.34);2.968(0.7);2.958 (1.1);2.941(2.05);2.93(1.35);2.924(1.3);2.913(2.18);2.896(1.1);2.885(0.85);2.867(0.46);2.689 (0.32);2.675(0.38);2.671(0.55);2.666(0.45);2.524(1.37);2.51(27.22);2.506(58.61);2.502(82.35 );2.497(67.21);2.329(0.53);2.324(0.43);2.075(0.77);1.948(16);0.146(0.36);0.008(2.68);0(78.7 4);-0.007(9.88);-0.15(0.34) |
| I-032 | 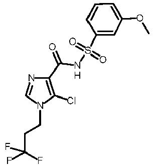 | Beispiel I-032: 1H-NMR(400.0 MHz, d6-DMSO): δ= 8.066(4.92);7.951(0.99);7.577(0.38);7.573(0.39);7.559(3.67);7.553(1.56);7.542(2.06);7.522(0 .58);7.508(1.59);7.505(1.75);7.501(1.33);7.289(0.83);7.283(1.24);7.276(0.76);7.271(0.74);7.2 65(1.23);7.259(0.66);4.312(1.78);4.295(3.73);4.278(1.78);3.826(16);2.902(0.53);2.891(0.81); 2.874(1.52);2.863(0.93);2.857(0.85);2.846(1.56);2.829(0.74);2.819(0.56);2.525(0.45);2.511(1 0.14);2.507(20.31);2.502(26.35);2.498(18.6);2.494(8.7);1.91(0.85);1.505(9.87);1.252(0.42);1. 236(0.42);1.111(3.28);0.898(0.4);0.008(0.41);0(11.12);-0.008(0.36) |
| I-033 | 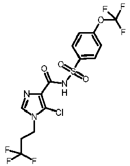 | Beispiel I-033: 1H-NMR(400.0 MHz, d6-DMSO): δ= 8.316(0.54);8.14(1.67);8.133(13.15);8.128(4.35);8.116(4.77);8.111(14.44);8.104(1.65);8.077( 16);7.65(8.39);7.629(7.69);4.312(5.51);4.295(11.74);4.278(5.68);3.383(0.74);2.918(0.85);2.9 01(1.66);2.89(2.49);2.873(4.67);2.862(2.92);2.856(2.68);2.846(4.79);2.828(2.34);2.818(1.75) ;2.801(0.83);2.676(1.15);2.671(1.5);2.667(1.13);2.541(1.25);2.511(89.16);2.507(170.43);2.50 |
| | | 2(217.14);2.498(154.39);2.494(73.8);2.334(1.03);2.329(1.36);2.325(1);0.008(2.09);0(44.44);-0.008(1.6) |
| I-034 | 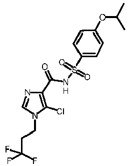 | Beispiel I-034: 1H-NMR(400.0 MHz, d6-DMSO): δ= 8.045(4.56);7.904(3.91);7.882(4.17);7.111(3.87);7.089(3.68);5.757(0.67);4.769(0.39);4.754(1 .03);4.738(1.4);4.723(1.04);4.708(0.4);4.307(1.66);4.29(3.57);4.273(1.73);4.097(0.66);3.34(0 .56);3.169(14.87);2.896(0.47);2.885(0.72);2.868(1.38);2.857(0.86);2.851(0.8);2.84(1.43);2.82 3(0.69);2.812(0.51);2.525(0.47);2.511(14.4);2.507(28.68);2.502(37.09);2.498(26.9);1.505(0.3 3);1.299(16);1.284(15.82);0.008(0.46);0(13.3);-0.008(0.51) |
| I-035 | 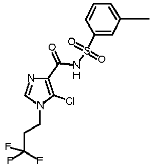 | Beispiel I-035: 1H-NMR(400.0 MHz, d6-DMSO): δ= 8.058(6.06);7.95(1.3);7.795(4.52);7.782(1.45);7.518(5.05);7.51(3.17);7.503(3.05);4.309(3.02) ;4.292(6.26);4.274(3.11);3.494(0.38);3.341(0.8);3.223(0.43);3.169(0.51);2.916(0.44);2.899(0. 86);2.888(1.35);2.871(2.39);2.86(1.63);2.854(1.44);2.843(2.46);2.826(1.23);2.816(0.91);2.79 8(0.43);2.506(36.91);2.502(46.23);2.498(34.29);2.403(16);1.505(12.95);1.251(0.52);1.236(0. 56);0.897(0.49);0.008(1.82);0.003(11.84);0(43.72);-0.008(2.22) |
| I-036 | 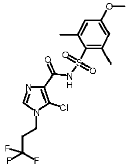 | Beispiel I-036: 1H-NMR(400.0 MHz, d6-DMSO): δ= 8.074(0.53);8.054(1.82);6.777(4.03);4.304(1.17);4.296(0.7);4.287(2.41);4.269(1.17);3.792(9. 72);3.776(1.54);3.329(4.12);2.901(0.39);2.89(0.56);2.873(1.01);2.862(0.66);2.857(0.67);2.84 5(1.02);2.829(0.51);2.818(0.38);2.675(0.32);2.67(0.42);2.632(16);2.584(1.23);2.506(45.22);2 .502(58.78);2.497(43.35);2.329(0.37);2.295(1.13);0.007(0.83);0(20.01);-0.008(0.93) |
| I-037 | 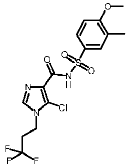 | Beispiel I-037: 1H-NMR(400.0 MHz, d6-DMSO): δ= 8.044(5.02);7.85(2.02);7.829(2.15);7.749(3.87);7.154(2.77);7.133(2.61);5.758(0.73);4.305(2. 84);4.289(5.27);4.272(2.87);3.878(16);3.503(0.47);3.331(11.57);2.911(0.52);2.884(1.51);2.86 7(2.43);2.855(2.06);2.84(2.4);2.824(1.44);2.795(0.49);2.672(0.84);2.503(115.35);2.33(0.85); 2.198(15.23);1.505(1.04);0(12.18);-0.003(11.3) |
| I-038 | 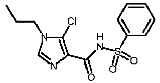 | Beispiel I-038: 1H-NMR(400.0 MHz, d6-DMSO): δ= 8.004(15.73);7.987(7.27);7.879(0.4);7.724(1.18);7.706(3.65);7.687(2.9);7.646(5.29);7.626(7. 31);7.608(2.8);3.976(4.43);3.959(8.1);3.941(4.56);3.612(0.33);3.594(0.37);3.578(0.35);3.507 (0.4);3.484(0.39);3.458(0.4);3.451(0.4);3.437(0.39);3.425(0.41);3.37(0.39);3.355(0.39);3.339 (0.37);3.33(0.37);3.318(0.36);3.304(0.36);3.261(0.32);2.672(0.38);2.507(40.6);2.503(52.43); |
| | | 2.499(40.87);2.33(0.37);1.741(0.57);1.723(2.61);1.705(5.06);1.686(5.12);1.668(2.73);1.65(0. 66);1.503(4.07);0.854(0.48);0.837(8.28);0.818(16);0.8(7.23);0(2.35) |
| I-039 | 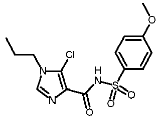 | Beispiel I-039: 1H-NMR(400.0 MHz, d6-DMSO): δ= 7.987(5.1);7.946(0.45);7.939(3.98);7.934(1.31);7.922(1.31);7.916(4.34);7.909(0.47);7.154(0. 49);7.147(4.17);7.142(1.35);7.129(1.26);7.124(3.98);7.117(0.42);5.757(3.88);3.975(1.85);3.9 58(3.33);3.94(1.85);3.846(16);2.511(9.85);2.507(19.79);2.503(25.9);2.498(18.68);2.494(8.95) ;1.722(1.13);1.704(2.16);1.686(2.16);1.668(1.15);1.503(2.97);0.836(4.1);0.817(7.74);0.798(3. 32);0(0.86) |
| I-040 | 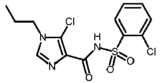 | Beispiel I-040: 1H-NMR(400.0 MHz, d6-DMSO): δ= 8.146(2.94);8.142(3.04);8.126(3.21);8.123(3.14);8.056(10.3);7.72(0.87);7.716(0.9);7.7(2.45); 7.697(2.32);7.683(3.13);7.679(3.02);7.667(4);7.663(5.04);7.647(1.99);7.643(1.44);7.626(2.31 );7.622(1.97);7.606(2.89);7.602(2.34);7.589(1.47);7.584(1.33);3.986(3.86);3.969(6.64);3.951 (3.97);2.671(0.43);2.511(28.82);2.507(55.68);2.502(71.76);2.498(52.5);2.494(26.14);2.334(0. 39);2.329(0.5);2.325(0.39);2.086(0.74);1.758(0.47);1.74(2.29);1.722(4.41);1.704(4.47);1.686 (2.44);1.667(0.57);1.503(2.57);0.854(7.61);0.835(16);0.817(7.11);0(2.36) |
| I-041 | 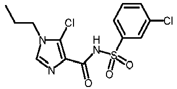 | Beispiel I-041: 1H-NMR(400.0 MHz, d6-DMSO): δ= 8.05(10.55);7.99(5.66);7.986(4.17);7.957(3.26);7.937(3.65);7.808(2.37);7.788(3.19);7.786(3. 2);7.694(3.63);7.674(5.52);7.654(2.35);5.757(0.5);3.987(4.4);3.969(8.05);3.951(4.56);2.672( 0.53);2.507(62.31);2.503(80.92);2.499(65.85);2.33(0.62);1.749(0.59);1.731(2.67);1.712(5.2); 1.694(5.27);1.676(2.84);1.658(0.73);1.503(1.59);0.843(8);0.824(16);0.806(7.4);0(2.04) |
| I-042 | 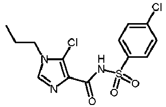 | Beispiel I-042: 1H-NMR(400.0 MHz, d6-DMSO): δ= 8.024(11.01);8.009(1.07);8.002(8.3);7.985(3);7.981(9.89);7.733(1.24);7.727(9.7);7.71(2.73); 7.705(8.35);5.757(1.75);3.983(4.19);3.965(7.61);3.947(4.36);3.877(0.34);3.871(0.35);3.822(0 .36);3.803(0.39);3.785(0.41);3.766(0.39);3.706(0.4);3.7(0.4);3.68(0.41);3.643(0.41);3.614(0. 41);3.61(0.4);3.588(0.4);3.548(0.38);3.477(0.35);2.672(0.37);2.508(42.46);2.503(57.16);2.49 9(44.25);2.33(0.43);2.326(0.35);1.745(0.49);1.727(2.45);1.709(4.84);1.691(4.92);1.673(2.63) ;1.654(0.61);1.503(1.07);0.839(7.72);0.821(16);0.802(7.21);0(1.71) |
| I-043 | 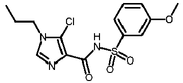 | Beispiel I-043: 1H-NMR(400.0 MHz, d6-DMSO): δ= 8.01(4.75);7.576(0.39);7.572(0.4);7.561(1.61);7.557(3.85);7.553(1.63);7.539(2.11);7.52(0.65) ;7.509(1.5);7.506(1.79);7.502(1.36);7.286(0.77);7.28(1.12);7.274(0.74);7.268(0.7);7.262(1.12 );7.256(0.65);5.757(0.44);3.981(1.69);3.963(3.03);3.946(1.74);3.826(16);2.511(10.27);2.507( 21.24);2.502(28.31);2.498(20.59);2.494(10.01);1.728(1.06);1.709(2.02);1.691(2.03);1.673(1. 09);1.503(2.03);0.841(3.47);0.823(7.3);0.804(3.18);0(0.87) |
| I-044 | 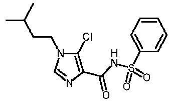 | Beispiel I-044: 1H-NMR(400.0 MHz, d6-DMSO): δ= 8.024(5.23);8.004(2.95);7.985(3.48);7.982(2.53);7.723(0.52);7.71(0.39);7.705(1.76);7.686(1. 34);7.644(2.52);7.625(3.5);7.607(1.33);5.756(0.64);4.021(1.9);4.003(2.66);3.984(1.96);3.411 (0.34);3.404(0.34);3.392(0.34);2.525(0.45);2.507(23.81);2.503(31.68);2.498(23.76);1.597(0.8 3);1.58(1.99);1.561(2.02);1.543(1.23);1.517(0.51);1.502(2.94);1.485(0.96);1.468(0.74);1.452 (0.39);0.919(0.87);0.9(16);0.884(14.96);0.008(0.73);0(21.16);-0.008(0.8) |
| I-045 | 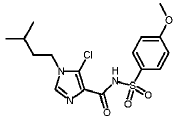 | Beispiel I-045: 1H-NMR(400.0 MHz, d6-DMSO): δ= 8.007(4.47);7.944(0.42);7.936(4);7.931(1.21);7.919(1.25);7.914(4.22);7.906(0.44);7.899(0.56 );7.152(0.44);7.145(4.01);7.14(1.23);7.128(1.18);7.122(3.77);7.115(0.39);5.756(0.38);4.02(1. 65);4.002(2.3);3.982(1.64);3.846(16);3.335(0.9);2.524(0.88);2.511(17.55);2.507(34.76);2.502 (45.86);2.498(33.46);2.493(15.98);1.596(0.8);1.579(1.78);1.56(1.74);1.542(1.04);1.514(0.49) ;1.502(5.08);1.482(0.82);1.465(0.64);1.448(0.32);0.918(1.81);0.9(15.23);0.883(13.99);0.008( 1.25);0(34.26);-0.008(1.11) |
| I-046 | 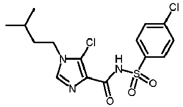 | Beispiel I-046: 1H-NMR(400.0 MHz, d6-DMSO): δ= 8.045(5.15);8.001(3.8);7.979(4.62);7.9(0.51);7.726(4.52);7.704(4);5.756(1.67);4.027(1.9);4.0 08(2.82);3.99(1.99);2.507(23.16);2.503(30.41);2.499(23.5);1.601(0.85);1.584(2.03);1.564(2.0 9);1.547(1.21);1.519(0.57);1.502(5.17);1.486(1.05);1.47(0.78);1.453(0.41);0.919(1.79);0.902 (16);0.886(14.03);0(18.62);-0.008(1) |
| I-047 | 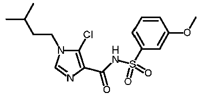 | Beispiel I-047: 1H-NMR(400.0 MHz, d6-DMSO): δ= 8.03(4.9);7.9(0.57);7.574(0.39);7.57(0.39);7.556(3.93);7.551(1.66);7.538(2.14);7.519(0.66);7 .507(1.6);7.504(1.86);7.5(1.41);7.285(0.79);7.279(1.18);7.273(0.76);7.267(0.74);7.262(1.17); 7.255(0.66);5.756(0.74);4.026(1.64);4.007(2.41);3.988(1.72);3.826(16);3.383(0.32);3.371(0.3 2);2.525(0.38);2.511(11.26);2.507(22.87);2.502(30.71);2.498(22.99);2.494(11.52);1.602(0.74 );1.585(1.76);1.566(1.77);1.548(1.06);1.521(0.5);1.502(5.53);1.489(0.92);1.472(0.68);1.455( 0.35);0.918(1.96);0.903(15.78);0.886(13.57);0.008(0.68);0(21.63);-0.008(0.9) |
| I-048 | 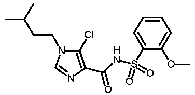 | Beispiel I-048: 1H-NMR(400.0 MHz, d6-DMSO): δ= 8.035(3.99);7.899(0.44);7.89(1.5);7.886(1.6);7.871(1.63);7.866(1.64);7.69(0.61);7.686(0.61); 7.669(1.18);7.651(0.75);7.647(0.71);7.239(1.81);7.218(1.66);7.164(0.98);7.144(1.8);7.126(0. 89);4.027(1.74);4.009(2.37);3.99(1.81);3.843(16);3.326(1.23);2.671(0.33);2.524(0.81);2.511( 18.94);2.506(38.1);2.502(50.8);2.497(37.43);2.493(18.16);2.328(0.34);1.611(0.78);1.595(1.9 1);1.576(1.93);1.557(1.25);1.538(0.48);1.522(0.79);1.502(4.06);1.489(0.73);1.472(0.36);0.91 8(1.67);0.909(15.85);0.903(2.65);0.892(14.89);0.008(1.18);0(35.28);-0.008(1.18) |
| I-049 | 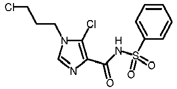 | Beispiel I-049: 1H-NMR(400.0 MHz, d6-DMSO): δ= 8.007(16);7.991(1.69);7.986(7.25);7.982(5.11);7.887(1.05);7.726(0.61);7.723(1.12);7.72(0.71 );7.71(0.83);7.705(3.61);7.699(1.1);7.689(1.75);7.686(2.88);7.683(1.52);7.645(4.99);7.628(3. 87);7.625(7.14);7.611(1.14);7.607(2.76);5.756(7.96);4.153(3.83);4.136(6.23);4.118(3.79);3.6 52(0.69);3.642(4.81);3.637(1.84);3.626(9.38);3.611(4.91);2.672(0.38);2.525(0.94);2.511(21.2 8);2.507(43.19);2.502(57.2);2.498(41.66);2.494(20.09);2.329(0.39);2.192(1.11);2.176(3.26);2 .158(4.18);2.141(3.1);2.125(0.98);1.504(9.15);1.249(0.33);1.234(0.37);0.008(2.11);0(60.33);-0.008(2.11) |
| I-050 | 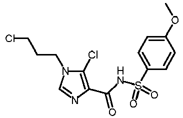 | Beispiel I-050: 1H-NMR(400.0 MHz, d6-DMSO): δ= 7.992(5.24);7.945(0.44);7.937(4.05);7.933(1.38);7.92(1.43);7.915(4.38);7.908(0.5);7.887(0.8 1);7.153(0.48);7.146(4.28);7.141(1.43);7.128(1.31);7.123(4.09);7.116(0.47);5.756(3.04);4.15 2(1.88);4.135(3.13);4.117(1.79);3.846(16);3.652(0.49);3.64(2.3);3.625(4.4);3.609(2.27);3.36 4(0.35);3.347(0.37);3.329(0.34);3.318(0.35);3.304(0.35);2.511(12.83);2.507(25.27);2.502(33. 16);2.498(24.66);2.19(0.58);2.174(1.64);2.157(2.17);2.14(1.51);2.124(0.45);1.504(7);1.234(0 .32);0.008(1.17);0(29.62);-0.008(1.24) |
| I-051 | 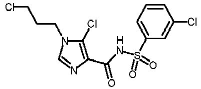 | Beispiel I-051: 1H-NMR(400.0 MHz, d6-DMSO): δ= 8.316(0.44);8.133(0.35);8.046(16);7.991(4.52);7.987(8.22);7.982(5.36);7.957(3.32);7.954(4.1 8);7.951(2.87);7.937(3.79);7.935(4.58);7.931(3.31);7.808(2.64);7.806(3.06);7.803(2.81);7.80 1(2.61);7.788(3.81);7.786(4.02);7.783(4.04);7.781(3.42);7.692(5.51);7.672(8.42);7.652(3.54) ;5.756(1.34);4.162(5.58);4.144(9.22);4.126(5.71);4.063(0.35);3.847(0.33);3.836(0.37);3.828( 0.34);3.821(0.39);3.807(0.37);3.796(0.35);3.777(0.36);3.768(0.37);3.751(0.37);3.726(0.38);3. 72(0.38);3.711(0.39);3.648(7.35);3.633(14.25);3.617(7.56);3.569(0.43);3.507(0.47);3.497(0.4 );3.49(0.4);3.486(0.41);3.476(0.39);3.455(0.41);3.44(0.44);3.424(0.39);3.405(0.37);3.38(0.38 );3.363(0.46);3.346(0.46);3.34(0.39);3.328(0.42);3.313(0.42);3.298(0.36);3.187(0.47);3.169( 10.49);2.891(0.42);2.731(0.33);2.676(0.72);2.672(0.97);2.667(0.73);2.663(0.39);2.644(0.51); 2.525(2.67);2.511(52.6);2.507(102.88);2.502(133.57);2.498(97.06);2.494(47.13);2.334(0.69); 2.329(0.94);2.325(0.72);2.199(1.65);2.183(4.96);2.165(6.44);2.149(4.78);2.133(1.51);1.504(2 .18);0.146(0.5);0.008(4.84);0(118.45);-0.008(4.78);-0.02(0.35);-0.15(0.53) |
| I-052 | 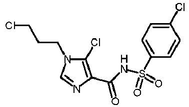 | Beispiel I-052: 1H-NMR(400.0 MHz, d6-DMSO): δ= 8.026(16);8.014(0.38);8.007(1.48);8.001(12.23);7.996(4.06);7.984(4.4);7.979(14.84);7.973(1. 78);7.733(1.71);7.726(13.83);7.721(4.22);7.71(3.86);7.705(12.01);7.698(1.38);5.756(3.28);4. 159(4.77);4.141(7.87);4.123(5.01);3.645(6.25);3.63(12.49);3.614(6.64);2.672(0.42);2.525(0.4 6);2.512(27.74);2.508(57.41);2.503(76.77);2.498(56.26);2.494(27.64);2.334(0.47);2.33(0.62); 2.325(0.48);2.196(1.38);2.18(4.23);2.162(5.56);2.146(4.26);2.129(1.44);2.087(3.01);1.504(1. 11);0.008(1.34);0(48.26);-0.008(1.95) |
| I-053 | 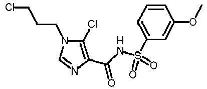 | Beispiel I-053: 1H-NMR(400.0 MHz, d6-DMSO): δ= 8.012(4.91);7.887(0.6);7.574(0.36);7.57(0.37);7.555(3.77);7.55(1.58);7.538(2.11);7.518(0.64) ;7.507(1.5);7.503(1.74);7.499(1.33);7.284(0.77);7.278(1.15);7.272(0.73);7.266(0.7);7.26(1.14 );7.254(0.64);5.756(0.57);4.157(1.73);4.139(2.85);4.122(1.77);3.826(16);3.646(2.23);3.636(1 .02);3.63(4.15);3.62(0.71);3.614(2.16);2.524(0.63);2.511(14.13);2.507(28.61);2.502(37.86);2. 498(27.49);2.493(13.19);2.196(0.5);2.18(1.44);2.162(1.82);2.145(1.34);2.129(0.41);1.504(5.1 );0.008(0.66);0(18.97);-0.008(0.63) |
| I-054 | 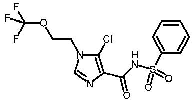 | Beispiel I-054: 1H-NMR(601.6 MHz, d6-DMSO): δ= 8.029(6.48);8.005(3.66);7.993(4.16);7.99(3.26);7.918(1.11);7.722(0.78);7.71(2.05);7.697(1.4 2);7.645(2.81);7.632(4.16);7.619(1.82);4.405(0.65);4.386(16);3.344(1.25);2.615(0.37);2.525( 0.58);2.522(0.71);2.518(0.78);2.509(22.13);2.506(47.73);2.504(66.53);2.5(49.91);2.498(24.5) ;2.388(0.38);1.508(8.77);1.252(0.38);1.242(0.41);0.899(0.38);0.005(2.38);0(65.85);-0.006(3.06) |
| I-055 | 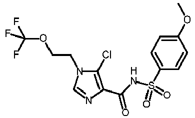 | Beispiel I-055: 1H-NMR(601.6 MHz, d6-DM50): δ= 8.017(4.64);7.952(0.36);7.938(4.01);7.935(1.31);7.927(1.38);7.923(4.32);7.918(1.88);7.147(3 .94);7.144(1.32);7.132(3.84);5.761(3.38);4.406(0.7);4.386(12.72);3.847(16);3.351(1.58);2.52 2(0.44);2.51(14.21);2.507(29.6);2.504(39.99);2.501(28.87);2.498(13.38);1.509(11.37);1.253( 0.52);1.242(0.51);0.899(0.5);0.005(1.63);0(44.51);-0.006(1.59) |
| I-056 | 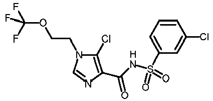 | Beispiel I-056: 1H-NMR(400.0 MHz, d6-DMSO): δ= 8.046(4.67);7.995(1.43);7.99(2.77);7.986(1.92);7.96(1.39);7.948(0.34);7.94(1.56);7.914(0.55) ;7.81(0.96);7.807(0.94);7.789(1.29);7.786(1.35);7.695(1.67);7.675(2.58);7.655(1.1);5.757(3.3 |
| | | 1);4.392(16);2.672(0.35);2.525(1.09);2.512(18.25);2.507(36.04);2.503(47.06);2.498(33.84);2. 494(16.01);1.79(0.41);1.509(4.34);1.256(0.35);1.24(0.64);1.236(0.63);0.008(0.45);0(10.82) |
| I-057 | 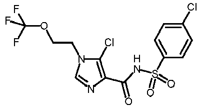 | Beispiel I-057: 1H-NMR(400.0 MHz, d6-DMSO): δ= 8.035(6.38);8.011(0.56);8.005(4.73);8(1.61);7.988(1.67);7.983(5.73);7.977(0.72);7.735(0.68) ;7.729(5.42);7.724(1.73);7.712(1.5);7.707(4.7);7.701(0.57);5.757(1.2);4.39(16);2.672(0.36);2 .525(0.92);2.511(19.25);2.507(38.52);2.502(50.69);2.498(36.69);2.494(17.56);1.509(0.6);0.0 08(0.44);0(12.17);-0.008(0.36) |
| I-058 | 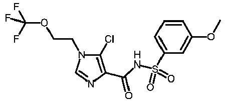 | Beispiel I-058: 1H-NMR(400.0 MHz, d6-DMSO): δ= 8.027(3.64);7.948(0.4);7.914(1.91);7.578(0.39);7.574(0.39);7.563(1.51);7.559(3.43);7.54(1.7 7);7.521(0.58);7.509(1.41);7.506(1.69);7.502(1.28);7.286(0.64);7.281(0.94);7.274(0.64);7.26 8(0.59);7.262(0.88);7.257(0.53);5.757(0.56);4.403(1.27);4.39(13.95);3.827(15.56);3.345(0.55 );3.315(0.52);2.525(0.86);2.511(16.37);2.507(32.72);2.502(43.04);2.498(31.16);2.493(14.97); 1.509(16);1.256(0.6);1.24(0.62);0.9(0.57);0.008(0.37);0(10.91);-0.008(0.36) |
| I-059 | 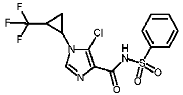 | Beispiel I-059: 1H-NMR(400.0 MHz, d6-DMSO): δ= 8(1.56);7.989(1.44);7.97(1.46);7.927(0.38);7.893(1.9);7.694(0.62);7.676(0.51);7.635(0.99);7. 616(1.35);7.597(0.53);5.756(1.76);3.864(0.35);3.852(0.7);3.844(0.87);3.832(0.85);3.823(0.58 );3.335(0.61);2.716(0.52);2.711(0.5);2.702(0.52);2.676(0.38);2.671(0.37);2.506(25.84);2.502 (32.11);2.498(23.81);1.85(0.49);1.834(0.67);1.824(0.64);1.809(0.47);1.606(0.54);1.589(1.07) ;1.569(1.01);1.552(0.42);1.503(16);1.247(0.59);1.232(0.61);0.896(0.52);0(35.67) |
| I-060 | 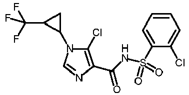 | Beispiel I-060: 1H-NMR(400.0 MHz, d6-DMSO): δ= 8.145(1.09);8.14(1.14);8.125(1.2);8.121(1.19);8.041(3.22);7.927(0.4);7.892(1.86);7.699(0.8); 7.695(0.77);7.681(0.98);7.677(0.95);7.66(1.3);7.657(1.71);7.64(0.74);7.637(0.56);7.627(0.81) ;7.623(0.66);7.607(1);7.589(0.5);7.585(0.44);5.756(3.57);3.877(0.49);3.866(0.72);3.857(0.97 );3.848(0.79);3.845(0.79);3.836(0.62);3.831(0.6);3.822(0.39);2.75(0.39);2.742(0.54);2.733(0. 51);2.725(0.6);2.718(0.49);2.708(0.38);2.701(0.38);2.524(0.77);2.511(15.43);2.506(31.05);2. 502(40.96);2.497(29.49);2.493(14.04);1.871(0.32);1.858(0.48);1.853(0.52);1.844(0.7);1.833( 0.63);1.817(0.43);1.614(0.53);1.598(0.96);1.577(0.91);1.569(0.46);1.56(0.38);1.503(16);1.24 7(0.67);1.232(0.69);0.896(0.54);0.008(2.27);0(61.06);-0.009(2.25) |
| I-061 | 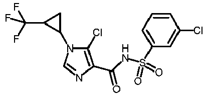 | Beispiel I-061: 1H-NMR(400.0 MHz, d6-DMSO): δ= 8.316(0.51);8.04(16);7.984(4.47);7.979(8.43);7.974(5.46);7.951(3.19);7.949(4.03);7.945(2.72 );7.931(3.67);7.928(4.66);7.925(3.37);7.893(1.08);7.811(2.59);7.808(2.98);7.806(2.71);7.803 (2.55);7.79(3.77);7.788(3.85);7.785(3.94);7.783(3.3);7.693(5.3);7.673(8.17);7.653(3.42);3.87 8(1.95);3.867(2.86);3.858(3.84);3.848(2.98);3.837(2.2);3.82(0.64);3.81(0.61);3.755(0.6);3.74 3(0.63);3.532(1.13);3.507(1.25);3.486(1.19);3.476(1.19);3.461(1.19);3.378(1.06);3.292(0.82) ;3.259(0.72);3.187(0.8);3.169(1.86);3.123(0.44);3.116(0.43);3.076(0.37);3.055(0.36);3.039(0. 35);3.021(0.33);2.762(0.45);2.752(0.88);2.744(1.07);2.735(1.51);2.727(1.97);2.718(1.84);2.7 09(2.07);2.701(1.58);2.692(1.19);2.683(1);2.676(1.01);2.671(1.12);2.666(0.93);2.524(2.46);2 .511(53.15);2.507(107.03);2.502(141.26);2.498(102.11);2.493(49.3);2.338(0.41);2.333(0.76); 2.329(1);2.324(0.73);2.32(0.4);1.859(1.3);1.842(1.87);1.831(2.16);1.821(1.72);1.818(1.68);1. 805(1.35);1.616(1.73);1.598(3.43);1.578(3.35);1.561(1.4);1.503(8.96);1.248(0.81);1.232(0.89 );0.896(0.64);0.146(0.85);0.008(7.25);0(197.4);-0.008(7.73);-0.033(0.33);-0.15(0.9) |
| I-062 | 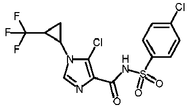 | Beispiel I-062: 1H-NMR(400.0 MHz, d6-DMSO): δ= 8.316(0.49);8.02(14.48);7.995(1.68);7.988(13.15);7.984(5.03);7.972(4.87);7.967(16);7.961(2. 22);7.927(0.37);7.893(0.67);7.723(14.1);7.706(4.19);7.702(12.32);3.875(1.99);3.864(3.18);3. 854(4.17);3.845(3.22);3.834(2.23);3.822(0.39);3.64(0.45);3.508(0.83);3.371(1.11);2.747(0.97 );2.738(1.18);2.73(1.71);2.721(2.24);2.713(2.06);2.704(2.35);2.696(1.76);2.687(1.32);2.676( 1.45);2.671(1.43);2.667(1.03);2.662(0.66);2.524(2.78);2.511(63.98);2.506(128.75);2.502(169 .38);2.497(122.76);2.493(59.51);2.333(0.9);2.329(1.18);2.324(0.89);1.855(1.5);1.838(2.22);1. 827(2.56);1.818(2.04);1.814(1.98);1.801(1.62);1.612(2);1.594(4.05);1.574(4.04);1.557(1.67); 1.503(5.58);1.247(0.57);1.232(0.66);0.896(0.45);0.146(0.63);0.008(5.51);0(150.86);-0.008(5.68);-0.15(0.66) |
| I-063 | 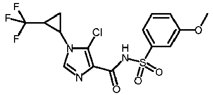 | Beispiel I-063: 1H-NMR(400.0 MHz, d6-DMSO): δ= 7.835(0.72);7.456(0.8);7.438(1.64);7.419(2.64);7.402(1.02);7.094(0.72);5.756(2.92);3.791(16 );3.335(2.97);3.201(0.39);2.786(0.47);2.767(0.33);2.671(0.89);2.667(0.86);2.65(0.61);2.569( 0.41);2.506(46.23);2.502(58.17);2.498(45.07);2.328(0.35);1.989(0.44);1.813(0.68);1.803(0.7 6);1.791(0.67);1.776(0.42);1.582(0.43);1.564(0.9);1.546(0.9);1.528(0.53);1.506(0.45);1.487( 0.33);1.329(0.41);1.309(0.42);0.901(0.81);0.883(1.57);0.865(0.69);0(11.03) |
| I-064 | 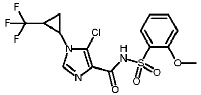 | Beispiel I-064: 1H-NMR(400.0 MHz, d6-DMSO): δ= 8.018(3.01);7.888(1.55);7.884(1.64);7.868(1.67);7.864(1.69);7.684(0.56);7.666(1.15);7.647(0 .67);7.236(1.73);7.215(1.57);7.161(0.96);7.142(1.74);7.123(0.85);3.876(0.57);3.866(0.95);3.8 56(1.37);3.841(16);3.324(1.45);2.753(0.33);2.745(0.49);2.736(0.63);2.727(0.6);2.719(0.69);2 .71(0.52);2.702(0.4);2.675(0.39);2.671(0.43);2.51(25.51);2.506(49.83);2.502(64.42);2.497(46 .61);2.493(22.68);2.328(0.41);2.324(0.32);1.867(0.44);1.85(0.68);1.839(0.79);1.83(0.63);1.82 6(0.62);1.813(0.48);1.615(0.58);1.598(1.2);1.578(1.18);1.561(0.49);1.503(1.94);0.008(2.17); 0(50.56);-0.008(1.88) |
| I-065 | 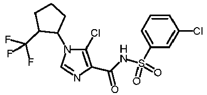 | Beispiel I-065: 1H-NMR(400.0 MHz, d6-DMSO): δ= 8.185(9.64);8.001(5.45);7.997(7.79);7.992(4.84);7.967(4.15);7.947(4.51);7.817(3.24);7.797(4 .23);7.794(3.94);7.702(4.41);7.682(6.67);7.662(2.84);5.757(16);4.891(1.39);4.871(3.72);4.85 1(3.73);4.831(1.41);3.732(0.36);3.689(0.39);3.654(0.42);3.607(0.44);3.508(0.5);3.478(0.48); 3.464(0.48);3.428(0.47);3.354(0.59);3.327(1.06);3.306(2.02);3.285(2.32);3.259(1.97);3.238(1 .02);3.213(0.49);3.186(0.36);2.676(0.6);2.672(0.7);2.507(90.09);2.503(102.22);2.499(72.85); 2.334(0.75);2.33(0.88);2.325(0.75);2.296(2.51);2.276(4.81);2.257(4.02);2.234(1.46);2.202(0. 38);2.147(1.02);2.13(1.87);2.125(1.99);2.119(1.79);2.109(1.48);2.097(2.49);2.076(1.3);2.071 (1.2);2.018(1.66);2.011(1.66);1.996(1.81);1.98(1.59);1.966(1.95);1.944(2.38);1.934(1.32);1.9 22(2.31);1.912(1.83);1.9(1.16);1.889(1.58);1.868(0.67);1.757(0.52);1.734(1.57);1.711(2.23); 1.704(1.71);1.688(1.69);1.682(1.87);1.66(1.05);1.509(0.48);0(6.71) |
| I-066 | 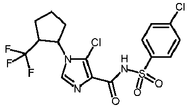 | Beispiel I-066: 1H-NMR(400.0 MHz, d6-DMSO): δ= 8.169(12.39);8.01(13.32);7.993(5.23);7.989(16);7.734(15.38);7.712(13.45);5.757(14.9);4.887 (1.55);4.867(4.45);4.847(4.56);4.827(1.69);3.624(0.44);3.51(0.55);3.442(0.57);3.423(0.57);3. 395(0.56);3.384(0.56);3.35(0.69);3.325(1.23);3.303(2.26);3.282(2.44);3.277(2.6);3.256(2.31) ;3.235(1.12);3.23(1.11);3.209(0.59);2.677(0.54);2.673(0.73);2.668(0.58);2.508(77.08);2.504( 103.06);2.499(79.63);2.335(0.6);2.33(0.84);2.326(0.68);2.293(2.4);2.288(2.38);2.272(4.94);2. 254(4.11);2.232(1.54);2.199(0.4);2.145(1.16);2.128(1.8);2.124(2.09);2.118(1.89);2.108(1.39) ;2.096(2.54);2.092(2.62);2.075(1.39);2.07(1.43);2.017(1.66);2.01(1.66);1.995(1.82);1.978(1.5 6);1.963(2.11);1.942(2.74);1.932(1.33);1.92(2.62);1.91(2.09);1.899(1.22);1.887(1.89);1.866( |
| | | 0.77);1.756(0.57);1.733(1.78);1.71(2.43);1.703(1.94);1.687(1.64);1.68(2.21);1.658(1.25);1.50 9(0.64);0(8.23) |
| I-067 | 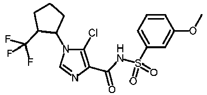 | Beispiel I-067: 1H-NMR(400.0 MHz, d6-DMSO): δ= 8.144(2.63);8.134(0.33);8.012(1.27);7.575(0.41);7.571(0.37);7.56(1.23);7.556(2.76);7.535(1. 75);7.516(0.65);7.505(1.71);7.501(1.33);7.278(0.62);7.274(0.87);7.267(0.66);7.26(0.59);7.25 5(0.84);7.249(0.57);5.757(5.56);4.881(0.41);4.861(1.25);4.841(1.27);4.821(0.45);3.826(14.05 );3.347(0.52);3.322(0.67);3.3(0.94);3.274(0.98);3.253(0.83);3.232(0.45);2.891(0.52);2.732(0. 41);2.52(0.38);2.512(11.53);2.507(25.05);2.503(34.31);2.498(25.91);2.494(13.43);2.292(0.69 );2.271(1.35);2.253(1.14);2.23(0.44);2.128(0.58);2.123(0.57);2.117(0.53);2.106(0.53);2.101( 0.57);2.096(0.64);2.091(0.61);2.075(0.33);2.069(0.34);2.016(0.5);2.007(0.5);1.99(0.56);1.973 (0.54);1.963(0.62);1.951(0.43);1.942(0.76);1.93(0.53);1.92(0.81);1.909(0.58);1.898(0.46);1.8 87(0.46);1.733(0.43);1.724(0.43);1.71(0.59);1.702(0.58);1.693(0.39);1.688(0.41);1.68(0.55); 1.509(16);1.257(0.65);1.241(0.7);0.902(0.33) |
| I-068 | 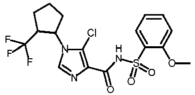 | Beispiel I-068: 1H-NMR(400.0 MHz, d6-DMSO): 6= 8.157(2);8.012(1.28);7.892(1.27);7.888(1.41);7.872(1.4);7.868(1.45);7.686(0.45);7.682(0.47) ;7.664(0.95);7.646(0.55);7.643(0.54);7.238(1.44);7.217(1.32);7.164(0.79);7.145(1.45);7.127( 0.72);5.757(2.84);4.879(0.52);4.859(1.5);4.839(1.52);4.819(0.55);3.822(13.64);3.328(1.92);3. 324(1.91);3.309(1.5);3.302(1.63);3.281(1.19);3.276(1.16);3.255(0.9);3.235(0.41);3.229(0.39) ;2.525(0.54);2.52(0.8);2.511(13.43);2.507(28.01);2.502(37.44);2.498(27.42);2.494(13.55);2.3 05(0.67);2.283(1.46);2.274(1.01);2.264(1.41);2.242(0.71);2.132(0.65);2.127(0.68);2.121(0.52 );2.111(0.44);2.105(0.62);2.1(0.85);2.095(0.67);2.078(0.44);2.073(0.37);2.015(0.51);1.995(0. 54);1.984(0.5);1.969(0.76);1.948(0.85);1.938(0.38);1.927(0.79);1.917(0.62);1.905(0.34);1.89 4(0.55);1.738(0.52);1.715(0.8);1.708(0.49);1.693(0.62);1.685(0.55);1.663(0.37);1.509(16);1. 256(0.47);1.241(0.49) |
| I-069 | 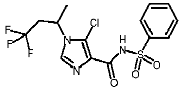 | Beispiel I-069: 1H-NMR(400.0 MHz, d6-DMSO): δ= 8.317(0.37);8.28(9.49);8.165(1.54);8.004(6.81);7.986(7.71);7.983(5.99);7.724(1.13);7.706(3. 57);7.687(2.84);7.645(5.47);7.626(7.6);7.608(2.94);4.708(0.94);4.697(1.35);4.691(1.42);4.68 1(1.56);4.673(1.44);4.668(1.3);4.656(1);3.504(0.43);3.336(2.04);3.186(0.66);3.178(0.67);3.1 7(0.63);3.151(1.11);3.138(0.79);3.124(1.37);3.112(1.58);3.099(1.14);3.086(1.9);3.072(0.59); 3.06(1.37);3.032(0.74);3.02(0.56);3.002(1.32);2.991(1.6);2.974(1.44);2.963(2.12);2.952(0.92) ;2.946(0.72);2.935(1.29);2.924(0.82);2.908(0.34);2.763(0.36);2.676(0.58);2.672(0.77);2.667( 0.59);2.507(86.72);2.502(113.92);2.498(86.42);2.334(0.59);2.329(0.81);2.325(0.6);1.989(0.4 2);1.514(13.15);1.505(16);1.497(13.18);1.235(0.33);1.11(0.77);0.008(1.26);0(32.96); 0.008(1.73) |
| I-070 | 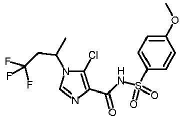 | Beispiel I-070: 1H-NMR(400.0 MHz, d6-DMSO): δ= 8.267(4.13);8.166(1.52);7.938(4.01);7.916(4.27);7.146(4.1);7.124(3.91);5.758(0.66);4.708(0. 56);4.697(0.81);4.691(0.86);4.681(0.93);4.673(0.86);4.657(0.55);3.846(16);3.331(1.48);3.17( 0.66);3.149(0.57);3.132(0.46);3.123(0.7);3.11(0.85);3.098(0.58);3.084(0.96);3.059(0.65);3.03 1(0.38);3.002(0.68);2.991(0.81);2.974(0.78);2.963(1.1);2.953(0.52);2.935(0.67);2.925(0.42); |
| | | 2.507(33.29);2.503(41.68);2.499(32.31);1.513(7.21);1.505(15.24);1.497(6.96);1.251(0.36);1. 236(0.43);0(9.73) |
| I-071 | 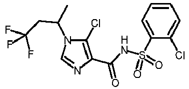 | Beispiel I-071: 1H-NMR(400.0 MHz, d6-DMSO): δ= 8.313(13.31);8.166(0.51);8.149(4.38);8.145(4.64);8.129(4.86);8.125(4.89);7.723(1.14);7.719( 1.22);7.702(3.57);7.685(4.08);7.682(4.11);7.665(7.26);7.649(2.84);7.628(3.15);7.624(2.83);7. 608(4.39);7.59(2.02);7.587(1.85);4.733(0.36);4.716(1.21);4.705(1.65);4.699(1.78);4.688(1.91 );4.681(1.83);4.664(1.29);4.648(0.39);3.189(0.45);3.162(1.12);3.15(0.75);3.136(1.55);3.123( 1.82);3.111(1.32);3.098(2.22);3.084(0.67);3.072(1.65);3.044(0.7);3.028(0.62);3.012(1.47);3.0 01(1.8);2.984(1.64);2.972(2.43);2.962(1.14);2.945(1.51);2.934(0.99);2.916(0.43);2.905(0.39) ;2.676(0.66);2.672(0.87);2.667(0.66);2.507(90.75);2.503(118.13);2.498(89.08);2.334(0.6);2.3 29(0.8);2.325(0.61);2.075(1.63);1.532(15.77);1.514(16);1.505(5.58);0.008(1.28);0(32.05);-0.008(1.66) |
| I-072 | 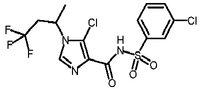 | Beispiel I-072: 1H-NMR(400.0 MHz, d6-DMSO): δ= 8.314(7.22);8.166(1.2);7.996(1.79);7.992(3.57);7.987(2.51);7.962(1.88);7.941(2.1);7.815(1.3 5);7.812(1.3);7.81(1.25);7.795(1.8);7.792(1.86);7.699(2.32);7.679(3.59);7.659(1.54);4.721(0. 54);4.709(0.73);4.704(0.84);4.694(0.92);4.686(0.88);4.68(0.81);4.669(0.62);3.157(0.53);3.14 4(0.42);3.131(0.79);3.118(0.88);3.106(0.72);3.092(1.05);3.08(0.39);3.066(0.74);3.039(0.41); 3.01(0.66);2.999(0.84);2.982(0.76);2.971(1.14);2.96(0.54);2.953(0.44);2.942(0.69);2.932(0.4 6);2.645(0.68);2.526(0.54);2.512(15.27);2.508(31.63);2.504(43.08);2.499(33.41);2.076(4.13); 1.521(7.91);1.505(16);1.252(0.38);1.236(0.39);0(2.22) |
| I-073 | 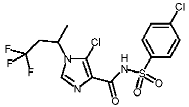 | Beispiel I-073: 1H-NMR(400.0 MHz, d6-DMSO): δ= 8.317(0.45);8.299(14.82);8.004(11.49);7.983(13.58);7.954(0.77);7.731(13.24);7.709(11.36);5 .758(2.76);4.734(0.38);4.717(1.26);4.706(1.74);4.7(1.91);4.691(2.19);4.682(2.02);4.665(1.42) ;4.65(0.47);3.763(0.38);3.682(0.5);3.676(0.52);3.584(0.71);3.464(0.97);3.456(0.98);3.435(1); 3.401(1.03);3.384(1.01);3.316(0.92);3.286(0.85);3.201(0.65);3.182(0.82);3.155(1.45);3.142(1 .11);3.128(1.91);3.116(2.18);3.103(1.69);3.09(2.67);3.064(1.94);3.037(1.14);3.025(0.86);3.00 8(1.66);2.997(1.99);2.98(1.84);2.969(2.58);2.958(1.35);2.941(1.64);2.93(1.14);2.912(0.59);2. 892(4.26);2.733(3.59);2.673(0.73);2.508(76.36);2.504(98.83);2.5(80.12);2.33(0.78);1.91(1.71 );1.518(15.42);1.501(16);0(3.95) |
| I-074 | 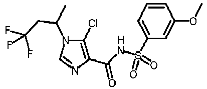 | Beispiel I-074: 1H-NMR(400.0 MHz, d6-DMSO): δ= 8.287(3.88);8.165(0.78);7.576(0.38);7.572(0.4);7.558(3.99);7.54(1.98);7.521(0.6);7.503(2.04) ;7.287(0.73);7.281(1.16);7.275(0.72);7.269(0.7);7.264(1.07);7.257(0.62);4.714(0.4);4.703(0.5 6);4.697(0.58);4.687(0.66);4.679(0.6);4.674(0.56);4.662(0.44);3.827(16);3.335(0.64);3.154(0 .42);3.142(0.32);3.128(0.56);3.115(0.63);3.103(0.5);3.089(0.76);3.063(0.54);3.006(0.48);2.99 5(0.58);2.978(0.51);2.967(0.78);2.956(0.35);2.939(0.48);2.671(0.33);2.507(38.41);2.502(50.8 5);2.498(38.64);2.329(0.33);2.075(3.14);1.518(5.68);1.505(8.59);0(2.03) |
| I-075 | 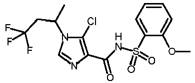 | Beispiel 1-075: 1H-NMR(400.0 MHz, d6-DMSO): δ= 8.29(4.49);7.893(1.58);7.89(1.67);7.874(1.74);7.87(1.75);7.691(0.71);7.687(0.73);7.67(1.49); 7.652(0.88);7.648(0.84);7.241(2.15);7.22(1.97);7.166(1.17);7.146(2.15);7.127(1.08);4.714(0. 44);4.703(0.6);4.697(0.65);4.686(0.72);4.679(0.67);4.662(0.48);3.835(16);3.441(0.37);3.332( 5.41);3.157(0.49);3.146(0.35);3.131(0.64);3.119(0.72);3.106(0.54);3.093(0.86);3.067(0.61);3. 041(0.35);3.012(0.54);3.001(0.66);2.984(0.6);2.973(0.89);2.962(0.43);2.945(0.54);2.934(0.35 |
| | | );2.507(38.45);2.502(49.32);2.498(37.94);2.33(0.34);1.532(5.69);1.515(5.89);1.505(3.07);0.0 08(2.96);0(56.38) |
| I-076 | 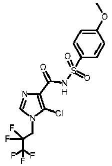 | Beispiel I-076: 1H-NMR(400.0 MHz, d6-DMSO): δ= 8.092(3.6);7.952(0.4);7.944(4);7.94(1.34);7.927(1.28);7.922(4.38);7.914(0.47);7.163(0.43);7. 155(4.06);7.15(1.37);7.138(1.19);7.133(3.89);7.125(0.44);5.215(1.03);5.175(2.18);5.135(1.12 );3.849(16);3.332(9.32);2.672(0.42);2.525(1.16);2.511(24.89);2.507(50.85);2.502(67.51);2.49 8(50.17);2.494(25.19);2.329(0.43);2.325(0.33);0.008(1.94);0(60.71);-0.008(2.25) |
| I-077 | 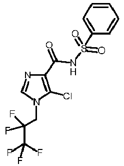 | Beispiel I-077: 1H-NMR(400.0 MHz, d6-DMSO): δ= 12.026(0.34);8.317(0.52);8.103(16);8.011(12.58);7.993(14.77);7.989(11.02);7.735(2.19);7.72 2(1.65);7.716(7.01);7.698(5.41);7.654(10.22);7.635(14.39);7.617(5.42);5.757(1.07);5.216(5); 5.176(10.66);5.136(5.51);3.33(4.73);2.676(1.36);2.671(1.92);2.666(1.42);2.662(0.7);2.524(4. 63);2.52(7.28);2.511(108.27);2.506(223.69);2.502(298.62);2.497(219.98);2.493(108.76);2.39 (0.53);2.333(1.41);2.329(1.97);2.324(1.46);1.305(0.33);0.146(1.49);0.008(11.21);0(338.45);-0.009(12.73);-0.15(1.5) |
| I-078 | 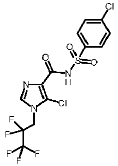 | Beispiel I-078: 1H-NMR(400.0 MHz, d6-DMSO): δ= 8.313(0.44);8.133(1.39);8.1(4.21);8.004(4.74);7.982(5.65);7.73(4.92);7.709(4.31);5.217(1.54) ;5.177(3.26);5.137(1.69);3.807(0.35);3.778(0.4);3.768(0.45);3.733(0.47);3.709(0.54);3.621(0. 9);3.359(238.96);3.134(1.01);3.108(0.8);3.001(0.39);2.961(0.32);2.677(1.49);2.672(2.05);2.6 68(1.52);2.566(0.36);2.526(4.87);2.512(107.63);2.508(220.15);2.504(293.62);2.499(217.05); 2.495(108.14);2.335(1.42);2.33(1.95);2.326(1.44);2.074(16);0.146(0.74);0.008(5.27);0(164.1 6);-0.008(6.01);-0.06(0.48);-0.15(0.76) |
| I-079 | 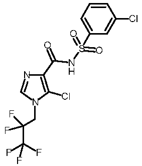 | Beispiel I-079: 1H-NMR(400.0 MHz, d6-DMSO): δ= 8.124(16);8(5.69);7.995(10.83);7.991(7.45);7.971(5.6);7.951(6.13);7.831(3.47);7.828(4.09);7 .826(3.86);7.824(3.56);7.81(4.94);7.808(5.35);7.806(5.49);7.803(4.65);7.709(6.77);7.689(10. 52);7.669(4.46);5.759(7.55);5.228(4.66);5.189(9.78);5.149(5.07);3.509(0.44);3.428(0.52);3.4 16(0.53);3.372(0.54);3.357(0.56);3.329(0.51);3.303(0.51);3.29(0.49);3.274(0.47);3.254(0.45) ;3.235(0.41);2.677(0.7);2.673(0.95);2.668(0.72);2.526(2.63);2.513(51.2);2.508(103.46);2.504 (136.32);2.499(99.62);2.495(48.82);2.335(0.64);2.33(0.87);2.326(0.64);2.087(3.32);2.077(3.1 5);0.146(0.35);0.008(2.74);0(79.8);-0.008(2.86);-0.15(0.34) |
| I-080 | 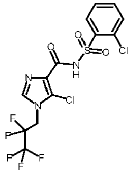 | Beispiel I-080: 1H-NMR(400.0 MHz, d6-DMSO): δ= 8.317(0.42);8.157(6.45);8.153(7.03);8.144(1.52);8.137(8);8.133(9.15);8.127(16);7.733(1.6);7 .729(1.74);7.713(4.7);7.71(4.56);7.695(5.53);7.692(5.57);7.677(7.67);7.673(10.09);7.657(4.0 9);7.653(3.22);7.635(4.37);7.632(4.14);7.624(1.19);7.615(5.82);7.598(2.82);7.594(2.73);5.22 2(4.72);5.182(9.99);5.162(0.88);5.142(5.18);5.123(0.38);3.38(1.12);3.353(1.14);3.304(1.04); 2.68(0.5);2.676(1.11);2.671(1.56);2.667(1.15);2.662(0.54);2.525(3.58);2.52(5.81);2.511(86.4 2);2.507(178.72);2.502(238.58);2.498(174.53);2.493(84.84);2.338(0.58);2.334(1.18);2.329(1. |
| | | 63);2.325(1.22);2.32(0.62);2.075(6.51);0.146(0.58);0.008(4.39);0(142.8);-0.008(4.97);-0.15(0.6) |
| I-081 | 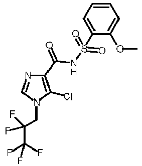 | Beispiel I-081: 1H-NMR(400.0 MHz, d6-DMSO): δ= 8.119(3.76);7.898(1.54);7.894(1.68);7.879(1.7);7.875(1.74);7.697(0.73);7.693(0.76);7.675(1. 31);7.672(1.12);7.658(0.92);7.653(0.9);7.244(2);7.223(1.8);7.167(1.12);7.149(1.98);7.131(0. 98);7.129(0.96);5.757(1.36);5.221(1.09);5.182(2.33);5.141(1.21);3.847(16);3.332(17.86);2.67 6(0.33);2.672(0.47);2.667(0.34);2.525(1.17);2.52(1.74);2.511(25.47);2.507(52.53);2.502(69.9 8);2.498(51.49);2.493(25.28);2.329(0.45);2.324(0.33);2.075(1.82);0.008(2.06);0(62.5);-0.008(2.13) |
| I-082 | 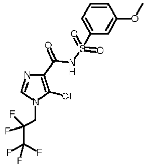 | Beispiel I-082: 1H-NMR(400.0 MHz, d6-DMSO): δ= 8.109(3.81);7.584(0.39);7.58(0.44);7.566(4.2);7.549(2.19);7.53(0.61);7.506(2.24);7.298(0.83) ;7.292(1.36);7.286(0.83);7.281(0.83);7.275(1.27);7.269(0.72);5.222(1.18);5.182(2.49);5.142( 1.28);3.83(16);3.336(3.14);2.672(0.38);2.507(44.76);2.503(58.48);2.498(45.39);2.33(0.37);0( 43.29) |
| I-083 | 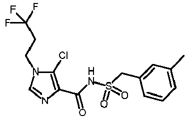 | Beispiel I-083: 1H-NMR(400.0 MHz, d6-DMSO): δ= 8.051(7.03);7.949(0.37);7.268(1.12);7.249(3.08);7.23(2.47);7.184(2.38);7.165(1.64);7.141(3. 6);7.122(2.4);7.103(1.8);4.745(9.04);4.358(2.52);4.34(5.6);4.323(2.95);4.292(0.44);3.329(0.9 4);2.964(0.36);2.947(0.74);2.936(1.16);2.92(2.21);2.909(1.54);2.903(1.46);2.892(2.37);2.881 (0.91);2.875(1.27);2.864(0.97);2.846(0.47);2.675(0.45);2.671(0.63);2.666(0.52);2.524(1.89); 2.51(32.85);2.506(67.07);2.502(91.68);2.497(74.5);2.493(45.33);2.333(0.46);2.328(0.63);2.3 24(0.52);2.309(0.45);2.264(16);2.074(1.07);1.505(3.41);0.146(0.55);0.008(5.1);0(118.57);-0.008(12.81);-0.15(0.54) |
| I-084 | 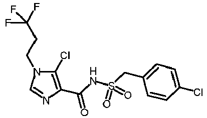 | Beispiel I-084: 1H-NMR(400.0 MHz, d6-DMSO): δ= 8.054(14.14);7.949(0.33);7.462(0.85);7.456(8.98);7.452(3.36);7.44(3.57);7.435(14.17);7.429( 1.94);7.357(1.55);7.351(12.47);7.335(2.83);7.33(8.53);4.815(16);4.353(4.56);4.336(10);4.318 (4.83);4.291(0.34);3.507(0.33);3.487(0.34);3.351(0.9);3.344(0.92);3.186(0.33);2.965(0.61);2. 947(1.26);2.937(1.93);2.92(3.83);2.909(2.24);2.903(2.07);2.892(4.02);2.881(1.07);2.875(1.93 );2.864(1.47);2.847(0.68);2.762(1.73);2.675(0.64);2.671(0.91);2.666(0.7);2.524(2.12);2.519( 3.16);2.51(51.92);2.506(109.01);2.502(146.75);2.497(108.22);2.492(53.34);2.338(0.38);2.33 3(0.73);2.328(1.02);2.324(0.78);2.075(7.23);1.505(2.94);1.202(0.33);0.986(0.63);0.146(0.36) ;0.008(2.56);0(88.71);-0.009(2.94);-0.15(0.38) |
| I-085 | 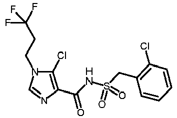 | Beispiel I-085: 1H-NMR(400.0 MHz, d6-DMSO): δ= 8.058(12.04);7.949(0.58);7.515(3.01);7.51(3.6);7.494(6.35);7.492(6.57);7.476(4.02);7.47(4.4) ;7.428(1.33);7.423(1.69);7.41(3.95);7.404(3.36);7.389(4.9);7.384(5.57);7.37(3.55);7.366(3.19 );7.352(1.11);7.348(0.94);5.757(1.41);4.964(16);4.358(4.44);4.34(9.67);4.323(4.7);4.309(0.4 2);4.292(0.57);3.505(0.55);3.338(2.34);2.968(0.66);2.951(1.29);2.94(1.96);2.923(3.74);2.912 (2.26);2.906(2.08);2.895(3.9);2.884(1.14);2.878(1.9);2.868(1.45);2.85(0.68);2.675(0.91);2.67 1(1.26);2.666(0.93);2.524(3.14);2.519(4.79);2.51(67.59);2.506(137.75);2.502(182.31);2.497( 133.72);2.493(65.52);2.333(0.85);2.328(1.19);2.324(0.86);1.505(5.36);1.235(0.33);0.146(0.4 6);0.008(3.28);0(103.8);-0.008(3.34);-0.15(0.44) |
| I-086 | 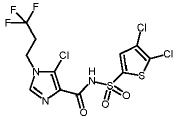 | Beispiel I-086: 1H-NMR(400.0 MHz, d6-DMSO): δ= 8.307(13.23);8.294(0.8);8.285(0.45);7.786(16);7.764(0.67);7.752(0.43);4.763(0.49);4.668(0.5 9);4.545(0.64);4.534(0.66);4.516(0.67);4.501(0.65);4.443(0.62);4.437(0.62);4.354(5.96);4.33 7(12.17);4.32(6.3);4.22(0.46);4.151(0.42);4.134(0.37);2.955(0.67);2.938(1.48);2.927(2.35);2. 91(4.49);2.9(2.86);2.894(2.71);2.882(4.67);2.865(2.3);2.855(1.68);2.838(0.72);2.672(0.81);2. 507(100.21);2.503(134.01);2.499(106.24);2.33(0.94);2.076(2.92);0.008(1.96);0(55.07);-0.008(2.98) |
| I-087 | 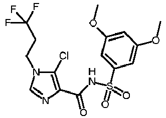 | Beispiel I-087: 1H-NMR(400.0 MHz, d6-DMSO): δ= 8.134(0.35);8.058(1.37);7.953(1.7);7.103(2.72);7.097(2.85);6.809(0.59);6.804(0.97);4.313(0. 81);4.296(1.75);4.279(0.85);3.808(16);2.891(12.71);2.877(0.71);2.866(0.43);2.86(0.4);2.849( 0.71);2.832(0.35);2.732(10.81);2.525(0.51);2.511(11.47);2.507(23.32);2.502(30.9);2.498(22. 77);2.494(11.35);0.008(1.35);0(37.94);-0.008(1.35);-0.008(1.35) |
| I-088 | 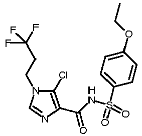 | Beispiel I-088: 1H-NMR(400.0 MHz, d6-DMSO): δ= 8.045(9.94);7.927(0.85);7.919(8.24);7.914(2.69);7.902(2.58);7.897(8.95);7.89(0.92);7.133(0. 93);7.126(8.33);7.121(2.8);7.108(2.49);7.103(8);7.096(0.87);4.306(3.36);4.289(7.3);4.272(3. 5);4.15(2.11);4.132(7.03);4.115(7.11);4.097(2.19);3.332(1.31);2.913(0.46);2.896(0.95);2.885 (1.45);2.868(2.83);2.857(1.7);2.851(1.57);2.84(2.92);2.829(0.82);2.823(1.39);2.812(1.04);2.7 95(0.46);2.676(0.37);2.671(0.52);2.667(0.37);2.525(1.26);2.511(28.44);2.507(57.12);2.502(7 5.26);2.498(55.11);2.494(27.12);2.334(0.36);2.329(0.48);2.324(0.36);2.075(1.04);1.361(7.54) ;1.344(16);1.326(7.34);0.008(2.07);0(58.49);-0.008(2.04) |
| I-089 | 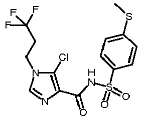 | Beispiel I-089: 1H-NMR(400.0 MHz, d6-DMSO): δ= 8.054(16);7.885(12.85);7.864(14.64);7.464(13.98);7.442(12.89);4.308(5.8);4.291(12.37);4.27 4(6.07);3.343(0.66);3.325(0.66);3.32(0.64);2.914(0.82);2.897(1.72);2.886(2.67);2.869(4.88); 2.858(3.16);2.852(2.96);2.841(5.03);2.824(2.51);2.814(1.82);2.797(0.82);2.731(0.52);2.671(0 .78);2.541(53.26);2.506(86.17);2.502(110.65);2.498(84.81);2.362(0.34);2.329(0.76);2.075(0. 8);0.146(0.38);0(80.22);-0.008(4.53);-0.15(0.39) |
| I-090 | 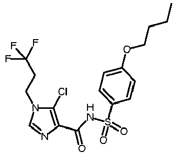 | Beispiel I-090: 1H-NMR(400.0 MHz, d6-DMSO): δ= 8.133(0.82);8.037(1.29);7.952(2.59);7.911(1.25);7.889(1.36);7.127(1.24);7.104(1.22);4.303(0 .59);4.286(1.26);4.269(0.62);4.078(0.7);4.061(1.45);4.045(0.73);3.33(3.05);2.89(16);2.866(0. 58);2.855(0.36);2.849(0.35);2.838(0.54);2.731(14.16);2.506(36.36);2.502(47.94);2.498(37.91 );1.728(0.53);1.711(0.71);1.692(0.59);1.458(0.44);1.439(0.75);1.42(0.74);1.402(0.42);0.947( 1.48);0.929(2.89);0.91(1.29);0.008(1.02);0(25.4) |
| I-091 | 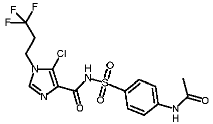 | Beispiel I-091: 1H-NMR(400.0 MHz, d6-DMSO): δ= 10.378(2.55);8.047(5.63);7.924(3.69);7.906(1.38);7.902(5.21);7.78(4.72);7.758(3.62);4.305(1 .73);4.288(3.79);4.271(1.82);3.348(0.75);3.339(0.76);2.894(0.5);2.883(0.77);2.866(1.5);2.855 (0.89);2.849(0.83);2.838(1.56);2.827(0.44);2.821(0.75);2.81(0.56);2.672(0.32);2.525(0.72);2. 52(1.12);2.511(18.22);2.507(37.95);2.502(50.84);2.498(37.55);2.494(18.68);2.329(0.33);2.08 5(16);2.075(1.36);0.008(1.61);0(49.84);-0.008(1.82) |
| 1-092 | 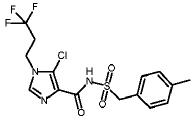 | Beispiel I-092: 1H-NMR(400.0 MHz, d6-DMSO): δ= 8.047(7.68);7.218(2.52);7.198(7.95);7.178(6.94);7.158(2.3);4.744(8.83);4.354(2.51);4.336(5. 44);4.319(2.62);3.334(1.43);3.235(0.43);2.966(0.35);2.949(0.7);2.938(1.08);2.921(2.07);2.91 (1.24);2.904(1.15);2.893(2.16);2.882(0.61);2.876(1.02);2.866(0.78);2.848(0.34);2.675(0.36); 2.67(0.5);2.666(0.35);2.524(1.06);2.51(29.82);2.506(59.34);2.501(77.66);2.497(56.61);2.493( 27.64);2.333(0.4);2.328(0.52);2.324(0.41);2.289(16);2.074(0.61);0(4.74) |
| 1-093 | 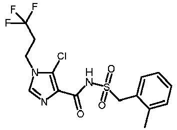 | Beispiel I-093: 1H-NMR(400.0 MHz, d6-DMSO): δ= 8.075(6.87);7.292(0.32);7.288(0.38);7.272(1.37);7.269(1.7);7.256(4.56);7.254(4.93);7.242(2. 93);7.201(1.26);7.197(1.17);7.181(1.23);7.176(0.8);7.167(0.45);7.161(0.46);4.827(8.99);4.36 4(2.22);4.347(4.88);4.33(2.34);3.337(0.81);3.332(0.8);3.308(0.74);3.247(0.43);2.954(0.62);2. 944(0.95);2.926(1.84);2.916(1.11);2.909(1.03);2.899(1.92);2.888(0.54);2.882(0.91);2.871(0.6 9);2.67(0.35);2.524(0.76);2.51(19.91);2.506(40.43);2.501(53.73);2.497(39.94);2.492(19.95);2 .406(16);2.328(0.37);2.074(1.08);0(3.39) |
| 1-094 | 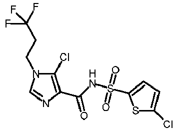 | Beispiel I-094: 1H-NMR(400.0 MHz, d6-DMSO): δ= 8.132(16);7.687(10.27);7.677(10.85);7.278(11.03);7.268(10.45);4.336(5.19);4.318(11.43);4.3 01(5.52);2.94(0.57);2.923(1.32);2.912(2.08);2.895(4.23);2.884(2.46);2.878(2.24);2.867(4.46) ;2.856(1.13);2.85(2.07);2.839(1.56);2.822(0.66);2.672(0.48);2.667(0.34);2.525(0.65);2.52(1.1 5);2.512(29.24);2.507(62.28);2.503(84.46);2.498(63.09);2.494(31.94);2.334(0.44);2.329(0.61 );2.325(0.47);2.076(0.51);0.146(0.33);0.008(1.99);0(80.54);-0.008(3.41);-0.15(0.4) |
| 1-095 | 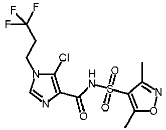 | Beispiel I-095: 1H-NMR(400.0 MHz, d6-DMSO): δ= 8.104(5.36);4.328(1.74);4.311(3.78);4.294(1.83);2.918(0.46);2.907(0.71);2.89(1.42);2.879(0. 82);2.872(0.76);2.862(1.49);2.851(0.38);2.845(0.78);2.834(0.53);2.682(16);2.525(0.53);2.52( 0.8);2.511(14.05);2.507(29.38);2.502(39.54);2.498(29.32);2.493(14.61);2.383(15.67);0.008(1 .07);0(36.46);-0.008(1.33) |
| 1-096 | 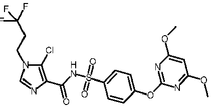 | Beispiel I-096: 1H-NMR(400.0 MHz, d6-DMSO): δ= 8.064(2.8);8.056(2.52);8.034(2.48);7.516(2.27);7.495(2.13);6.068(2.72);6.066(2.91);4.313(1. 05);4.296(2.19);4.279(1.14);3.784(16);3.34(0.6);2.904(0.35);2.892(0.53);2.876(0.92);2.864(0 .69);2.848(0.96);2.832(0.52);2.821(0.38);2.502(40.48);2.5(40.59);0(25.74);-0.002(24.96) |
| I-097 | 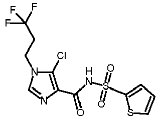 | Beispiel I-097: 1H-NMR(400.0 MHz, d6-DMSO): δ= 8.068(16);8.024(7.74);8.015(6.82);8.012(8.52);7.818(8.44);7.813(8.85);7.809(9.74);7.211(5.1 1);7.208(4.95);7.198(9.56);7.189(6.33);7.187(5.36);4.318(7.75);4.302(15.93);4.285(8.6);3.92 (0.38);3.875(0.38);3.856(0.41);3.841(0.43);3.79(0.49);3.716(0.59);3.642(0.71);3.633(0.72);3. 603(0.77);3.566(0.84);3.505(0.91);3.491(0.9);3.426(0.93);3.406(0.91);3.368(0.89);3.345(0.86 );3.276(0.76);3.255(0.73);3.066(0.46);2.988(0.37);2.968(0.34);2.924(1.27);2.907(2.51);2.897 (3.91);2.88(6.64);2.865(5.36);2.853(6.95);2.836(3.97);2.826(2.95);2.809(1.31);2.67(1.69);2.5 01(248.98);2.498(243.16);2.376(0.36);2.328(1.76);0.145(0.71);-0.001(140.93);-0.002(132.28);-0.15(0.78) |
| I-098 | 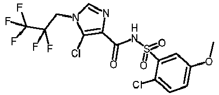 | Beispiel I-098: 1H-NMR(400.0 MHz, d6-DMSO): δ= 8.129(3.51);8.055(0.69);7.598(2.75);7.59(2.97);7.582(2.54);7.56(2.89);7.436(0.57);7.418(0.3 4);7.4(0.65);7.304(1.44);7.297(1.47);7.282(1.27);7.275(1.26);5.313(1.43);5.237(0.33);5.225( 1.04);5.197(0.69);5.185(2.2);5.157(0.37);5.145(1.14);3.855(16);3.385(1.37);3.366(1.15);2.67 6(0.38);2.672(0.54);2.667(0.41);2.525(1.1);2.511(29.5);2.507(61.38);2.503(82.33);2.498(62.1 2);2.334(0.42);2.329(0.56);2.325(0.44);0(5.68) |
| I-099 | 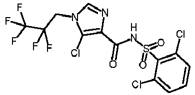 | Beispiel I-099: 1H-NMR(400.0 MHz, d6-DMSO): δ= 8.318(0.82);8.121(9.35);8.055(0.95);7.666(5.2);7.662(6.37);7.644(16);7.61(5.73);7.593(3.56) ;7.587(2.68);7.57(1.73);7.456(0.42);7.438(0.81);7.418(0.44);7.4(0.87);7.382(0.46);7.364(0.34 );7.347(0.32);5.313(1.93);5.237(0.49);5.22(3.54);5.198(1.11);5.18(7.43);5.157(0.7);5.14(3.82 );3.788(0.32);3.766(0.36);3.761(0.38);3.647(0.67);3.629(0.71);3.619(0.77);3.602(0.81);3.484 (1.51);3.382(2.04);3.127(0.56);2.676(1.54);2.671(2.28);2.667(1.68);2.525(3.47);2.511(140.4) ;2.507(294.05);2.502(395.65);2.498(296.22);2.494(152.8);2.352(0.6);2.333(2.25);2.329(3.01) ;2.325(2.37);2.275(0.42);2.244(0.39);2.234(0.34);2.075(7.93);2.063(0.34);1.598(0.34);1.581( 0.46);1.563(0.35);1.36(0.33);1.342(0.57);1.324(0.58);1.306(0.39);1.171(0.48);1.15(0.54);1.13 (0.45);0.92(0.89);0.902(1.75);0.892(0.38);0.884(0.84);0.873(0.55);0.008(1.5);0(65.25);-0.008(3.36);-0.15(0.34) |
| I-100 | 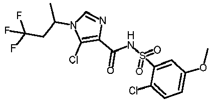 | Beispiel I-100: 1H-NMR(400.0 MHz, d6-DMSO): δ= 8.322(4.89);7.593(2.82);7.585(3.02);7.577(2.71);7.555(3.08);7.297(1.57);7.289(1.5);7.275(1. 35);7.267(1.31);4.722(0.34);4.71(0.46);4.705(0.49);4.698(0.51);4.694(0.53);4.687(0.51);4.68 2(0.47);4.67(0.37);3.854(16);3.137(0.43);3.125(0.5);3.112(0.36);3.098(0.63);3.074(0.46);3.0 15(0.41);3.004(0.51);2.987(0.46);2.976(0.69);2.948(0.42);2.525(0.49);2.512(13.67);2.508(28. 43);2.503(37.83);2.498(28.25);2.494(14.34);2.076(3.33);1.536(4.51);1.518(4.52);0(4.56) |
| I-101 | | Beispiel I-101: 1H-NMR(400.0 MHz, d6-DMSO): δ= 8.355(12.88);8.317(0.34);7.665(5.26);7.66(6.69);7.642(16);7.607(6.57);7.59(4.05);7.584(3.02 );7.567(2.11);4.744(0.34);4.728(0.98);4.716(1.3);4.71(1.4);4.703(1.44);4.699(1.5);4.692(1.47 );4.688(1.34);4.675(1.09);4.658(0.41);4.24(0.34);4.205(0.36);4.177(0.37);4.163(0.38);4.146( 0.41);4.136(0.41);4.12(0.57);4.078(0.56);4.053(0.46);4.042(0.46);4.022(0.47);4.004(0.47);3.9 94(0.48);3.982(0.48);3.934(0.49);3.919(0.49);3.86(0.49);3.831(0.63);3.778(0.44);3.764(0.44) ;3.704(0.4);3.666(0.37);3.618(0.33);3.164(0.8);3.152(0.48);3.137(1.11);3.125(1.37);3.113(0.9 3);3.099(1.69);3.086(0.44);3.074(1.26);3.046(0.62);3.033(0.41);3.016(1.1);3.005(1.38);2.988 |
| | 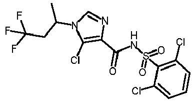 | (1.24);2.977(1.87);2.966(0.79);2.949(1.14);2.938(0.71);2.676(0.61);2.672(0.89);2.667(0.65); 2.525(1.23);2.52(2.36);2.511(53.34);2.507(112.08);2.502(150.71);2.498(112.4);2.494(57.27); 2.334(0.87);2.329(1.14);2.325(0.91);1.538(12.72);1.521(12.85);0(10.3);-0.008(0.47) |
| I-102 | 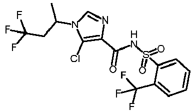 | Beispiel I-102: 1H-NMR(400.0 MHz, d6-DMSO): δ= 8.334(16);8.318(4.89);8.011(3.16);7.992(4.82);7.974(1.63);7.958(3.93);7.955(3.64);7.939(3.2 1);7.936(2.72);7.925(3.48);7.907(3.45);7.888(1.12);4.723(1.05);4.711(1.44);4.706(1.55);4.69 5(1.7);4.688(1.61);4.683(1.49);4.671(1.17);4.654(0.38);3.188(0.32);3.162(0.92);3.149(0.56); 3.135(1.29);3.123(1.54);3.11(1.07);3.096(1.97);3.084(0.52);3.071(1.44);3.043(0.76);3.031(0. 48);3.015(1.23);3.003(1.55);2.987(1.39);2.975(2.1);2.964(0.92);2.947(1.27);2.937(0.8);2.92( 0.33);2.676(0.65);2.672(0.95);2.668(0.72);2.525(1.73);2.507(121.81);2.503(162.61);2.498(12 5.34);2.334(0.97);2.33(1.25);2.325(1.01);2.076(4.87);1.531(14.19);1.514(14.34);0(5.28) |
| I-103 | 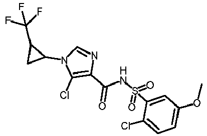 | Beispiel I-103: 1H-NMR(400.0 MHz, d6-DMSO): δ= 8.051(4.47);7.587(2.67);7.579(2.83);7.568(2.42);7.546(2.82);7.29(1.42);7.282(1.36);7.268(1. 24);7.26(1.18);3.884(0.7);3.873(1.01);3.864(1.52);3.854(16);3.844(0.98);3.796(0.33);3.773(0 .33);3.753(0.32);3.738(0.35);3.67(0.44);3.637(0.39);3.628(0.39);3.617(0.39);3.596(0.39);3.54 8(0.37);3.508(0.34);2.758(0.42);2.75(0.55);2.741(0.49);2.733(0.58);2.724(0.42);2.716(0.32); 2.671(0.34);2.525(0.69);2.511(19.41);2.507(39.29);2.502(51.65);2.498(37.71);2.494(18.62);2 .329(0.37);1.874(0.37);1.858(0.54);1.847(0.63);1.837(0.5);1.832(0.49);1.82(0.39);1.618(0.47) ;1.601(0.97);1.581(0.98);1.564(0.41);0(4.47) |
| I-104 | 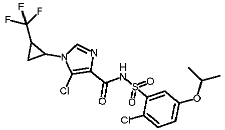 | Beispiel I-104: 1H-NMR(400.0 MHz, d6-DMSO): δ= 8.05(4.9);7.546(2.8);7.538(3.03);7.533(2.78);7.511(3.1);7.265(1.42);7.258(1.38);7.243(1.22); 7.236(1.19);4.714(0.4);4.699(1.02);4.684(1.39);4.669(1.03);4.654(0.42);3.886(0.67);3.874(0. 97);3.865(1.27);3.856(1);3.844(0.74);3.693(0.35);3.657(0.38);3.616(0.4);3.593(0.4);3.557(0. 39);3.55(0.39);3.507(0.38);3.497(0.36);3.489(0.36);3.487(0.36);3.45(0.33);2.757(0.44);2.748 (0.56);2.74(0.52);2.732(0.6);2.723(0.44);2.714(0.33);2.671(0.37);2.525(0.68);2.52(1.12);2.51 1(20.43);2.507(42.85);2.502(57.22);2.498(42.1);2.493(20.87);2.329(0.4);1.873(0.38);1.856(0. 56);1.845(0.65);1.832(0.51);1.819(0.41);1.619(0.48);1.602(1.02);1.582(1.04);1.564(0.42);1.3 13(16);1.298(15.92);0(4.87) |
| I-105 | 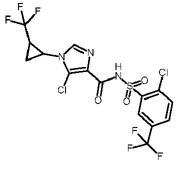 | Beispiel I-105: 1H-NMR(400.0 MHz, d6-DMSO): δ= 8.329(7.22);8.324(7.73);8.198(16);8.096(3.45);8.091(3.49);8.075(4.37);8.07(4.33);7.92(7.03) ;7.9(5.62);4.301(0.48);3.965(3.44);3.9(4.67);3.889(5.68);3.88(6.54);3.87(5.37);3.859(4.22);2. 778(0.86);2.77(1.06);2.761(1.6);2.753(2.12);2.744(1.95);2.735(2.25);2.727(1.68);2.718(1.22) ;2.71(0.94);2.676(0.86);2.672(1.23);2.667(0.93);2.525(2.5);2.52(4.29);2.512(66.93);2.507(13 8.75);2.503(184.71);2.498(136.9);2.494(68.66);2.334(0.94);2.33(1.31);2.325(0.99);1.884(1.4 2);1.867(2.15);1.856(2.53);1.846(1.96);1.842(1.97);1.83(1.56);1.627(1.86);1.61(3.89);1.59(3. 97);1.573(1.61);0.008(0.51);0(16.5);-0.008(0.65) |
| I-106 | 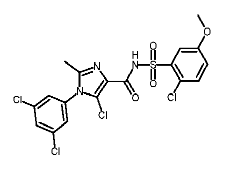 | Beispiel 1-106: 1H-NMR(400.0 MHz, CDCl3): δ= 7.852(2.68);7.844(2.76);7.582(1.74);7.578(3.09);7.574(1.92);7.387(2.56);7.365(2.95);7.261(9 .41);7.169(6.26);7.165(6.36);7.079(1.55);7.071(1.55);7.057(1.36);7.049(1.34);5.3(2.4);3.887( 16);2.279(15.6);1.562(0.83);0(9.45) |
| I-107 | 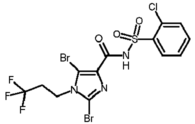 | Beispiel 1-107: 1H-NMR(400.0 MHz, d6-DMSO): δ= 8.14(0.87);8.136(0.88);8.12(0.97);8.116(0.93);7.704(0.71);7.686(0.83);7.682(0.8);7.666(1.43) ;7.649(0.58);7.627(0.62);7.623(0.54);7.607(0.86);7.589(0.41);7.586(0.36);4.304(0.88);4.287( 1.67);4.269(0.93);3.351(0.35);2.843(0.41);2.826(0.69);2.816(0.49);2.808(0.44);2.798(0.7);2.7 8(0.37);2.506(25.26);2.502(32.43);2.497(23.42);2.086(16);0.008(1.52);0(34.62);-0.008(1.59) |
| I-108 | 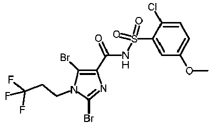 | Beispiel I-108: 1H-NMR(400.0 MHz, d6-DMSO): δ= 7.582(3.02);7.574(5.36);7.552(2.81);7.293(1.42);7.286(1.39);7.271(1.24);7.264(1.19);4.307(1 .5);4.289(2.86);4.272(1.58);3.852(16);3.343(1.24);2.856(0.43);2.847(0.71);2.83(1.2);2.819(0. 84);2.812(0.76);2.802(1.22);2.784(0.63);2.774(0.45);2.671(0.37);2.506(48.63);2.502(63.66);2 .497(45.96);2.328(0.37);2.086(2.72);0.146(0.36);0.008(2.86);0(76.31);-0.008(3.08);-0.15(0.37) |
| I-109 | 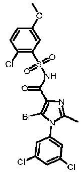 | Beispiel I-109: 1H-NMR(400.0 MHz, d6-DMSO): δ= 7.927(2.01);7.923(3.68);7.918(2.25);7.787(7.58);7.783(7.26);7.605(3);7.597(3.23);7.586(2.93 );7.564(3.3);7.292(1.68);7.284(1.66);7.27(1.5);7.262(1.44);5.757(3.16);3.857(16);2.506(19.4 8);2.502(25.65);2.498(20.48);2.256(14.03);0(1.07) |
| I-110 | 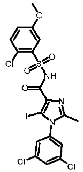 | Beispiel I-110: 1H-NMR(400.0 MHz, d6-DMSO): δ= 7.925(2.11);7.921(3.82);7.916(2.26);7.727(7.68);7.722(7.45);7.605(3.1);7.597(3.26);7.581(2. 81);7.559(3.29);7.285(1.68);7.277(1.61);7.263(1.47);7.255(1.41);3.862(16);2.506(25.28);2.50 2(32.51);2.497(24.73);2.297(13.61);2.256(0.47);2.074(1.6);0.008(1.28);0(33.1);-0.008(1.9) |
| I-111 | 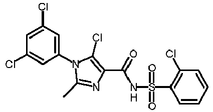 | Beispiel I-111: 1H-NMR(400.0 MHz, d6-DMSO): δ= 8.156(1.64);8.153(1.67);8.136(1.84);8.133(1.78);7.924(1.96);7.919(3.82);7.914(2.2);7.808(7. 89);7.803(7.31);7.722(0.37);7.718(0.38);7.702(1.29);7.698(1.14);7.685(1.74);7.681(1.81);7.6 76(2.36);7.672(2.76);7.656(0.98);7.627(1.13);7.623(0.96);7.607(1.49);7.59(0.78);7.586(0.68) ;3.616(0.43);2.675(0.39);2.671(0.52);2.666(0.4);2.524(1.09);2.51(34.27);2.506(68.84);2.502( 89.08);2.497(63.06);2.333(0.4);2.328(0.54);2.324(0.4);2.236(16);1.481(0.79);0.146(0.37);0.0 08(3.19);0(90.36);-0.008(3.44);-0.15(0.39) |
| I-112 | 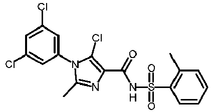 | Beispiel I-112: 1H-NMR(400.0 MHz, d6-DMSO): δ= 8.032(1.77);8.012(1.88);7.918(1.85);7.913(3.71);7.908(2.24);7.787(7.51);7.783(7.4);7.588(0. 7);7.569(1.68);7.55(1.08);7.455(1.05);7.436(1.69);7.417(0.84);7.406(1.9);7.387(1.53);2.671( 0.36);2.626(13.1);2.506(44.34);2.502(59.7);2.497(44.58);2.329(0.36);2.226(16);2.074(3.91);0 .008(1.24);0(34.39) |
| I-113 | 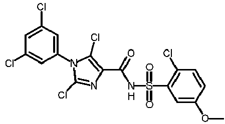 | Beispiel I-113: 1H-NMR(400.0 MHz, d6-DMSO): δ= 7.975(1.64);7.97(3.4);7.966(2.15);7.909(7.13);7.905(6.37);7.602(2.95);7.595(3.41);7.591(2.5 5);7.568(2.49);7.297(1.28);7.29(1.24);7.275(1.11);7.268(1.07);5.756(0.46);3.856(16);3.357(1 .03);2.67(0.44);2.506(61.21);2.502(79.57);2.497(58.27);2.333(0.36);2.328(0.47);2.086(15.26) ;0.007(1.74);0(43.27) |
| I-114 | 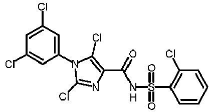 | Beispiel I-114: 1H-NMR(400.0 MHz, d6-DMSO): δ= 8.159(3.74);8.139(4.2);7.968(7.45);7.964(4.67);7.903(16);7.898(14.1);7.728(0.79);7.708(2.73 );7.68(5.63);7.663(1.84);7.63(2.22);7.611(3.19);7.594(1.45);5.756(6.74);3.346(4.92);3.286(1. 18);2.671(1.25);2.666(0.96);2.506(175.48);2.502(220.52);2.497(158.05);2.328(1.28);1.294(0. 36);1.236(0.49);0.146(0.52);0.008(4.85);0(121.56);-0.008(5.47);-0.15(0.55) |
| I-115 | 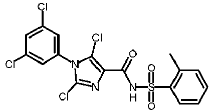 | Beispiel I-115: 1H-NMR(400.0 MHz, d6-DMSO): δ= 12.628(0.46);8.034(2.23);8.015(2.38);7.972(2.22);7.967(4.51);7.962(2.82);7.885(9.24);7.88(8 .81);7.594(0.83);7.576(1.99);7.557(1.3);7.46(1.29);7.44(2.07);7.42(1.15);7.412(2.3);7.393(1. 81);5.756(3.58);3.328(6.58);3.286(0.33);2.67(0.63);2.629(16);2.506(77.92);2.501(104.72);2.4 97(79.36);2.328(0.6);0.008(2.05);0(56.12) |
| I-116 | 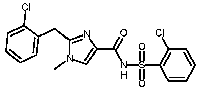 | Beispiel I-116: 1H-NMR(400.0 MHz, d6-DMSO): δ= 8.132(6.22);8.087(1.63);8.083(1.71);8.068(1.77);8.064(1.77);7.953(5.97);7.596(0.69);7.592(0 .51);7.576(1.24);7.572(1.17);7.559(2.11);7.555(3.52);7.549(3.22);7.534(1.11);7.525(1.42);7.5 2(1.14);7.501(2.6);7.495(1.57);7.488(1.04);7.483(1.66);7.478(2.01);7.35(0.39);7.345(0.56);7. 332(1.6);7.326(1.84);7.323(1.81);7.316(3.18);7.305(2.04);7.3(1.7);7.286(0.58);7.282(0.36);7. 119(1.57);7.114(1.09);7.103(1.25);7.096(1.22);4.287(7.28);4.056(0.51);4.038(1.06);4.02(1.07 );4.003(0.56);3.945(0.32);3.874(0.35);3.864(0.36);3.851(0.36);3.812(0.5);3.784(0.38);3.609( 16);2.523(0.84);2.51(19.53);2.506(39.2);2.502(51.54);2.497(37.26);2.493(18.11);1.988(3.54); 1.193(0.9);1.175(1.78);1.157(0.86);0.008(1.15);0(30.67);-0.008(1.19) |
| I-117 | 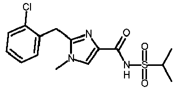 | Beispiel 1-117: 1H-NMR(400.0 MHz, d6-DMSO): δ= 8.006(5.44);7.49(1.32);7.478(1.18);7.474(0.86);7.467(1.72);7.325(0.37);7.314(3.11);7.306(1. 91);7.304(2.12);7.301(1.94);7.298(2.09);7.29(3.3);7.279(0.42);7.142(1.46);7.131(1.18);7.128 (1.13);7.118(1.13);4.189(8.17);3.765(0.61);3.748(1.34);3.73(1.81);3.713(1.41);3.696(0.72);3. 68(0.37);3.655(0.36);3.61(16);3.552(0.54);3.545(0.54);3.507(0.58);3.491(0.59);3.483(0.6);3. 473(0.6);3.431(0.64);3.388(0.54);2.67(0.34);2.506(42.55);2.501(56.87);2.497(42.75);2.328(0. 39);1.283(15.35);1.266(15.21);0(0.75) |
| I-118 | 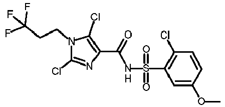 | Beispiel I-118: 1H-NMR(400.0 MHz, d6-DMSO): δ= 7.582(2.95);7.575(3.48);7.572(3.27);7.55(3.07);7.293(1.49);7.285(1.45);7.271(1.3);7.263(1.2 4);4.291(1.44);4.274(3.15);4.257(1.51);3.851(16);3.362(0.42);2.886(0.4);2.875(0.61);2.858(1 .2);2.847(0.74);2.841(0.68);2.83(1.25);2.813(0.59);2.802(0.45);2.524(0.7);2.51(15.8);2.506(3 2.98);2.501(45.88);2.497(34.71);2.492(17.09);2.073(1.37);0(3.16) |
| I-119 | 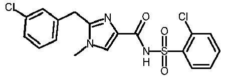 | Beispiel I-119: 1H-NMR(400.0 MHz, d6-DMSO): δ= 8.083(1.79);8.08(1.89);8.063(2.01);8.061(2.03);7.87(5.4);7.569(0.41);7.553(1.25);7.533(4.17) ;7.528(3.69);7.51(1.85);7.49(1.6);7.474(0.72);7.469(0.6);7.388(0.8);7.367(4.67);7.35(4.41);7. 344(3.16);7.329(0.56);7.324(0.72);7.269(0.32);7.25(0.86);7.231(0.83);7.204(1.98);7.186(2.07 );7.163(0.84);7.144(0.4);4.23(7.44);3.621(16);2.756(0.98);2.506(29.37);2.502(37.62);2.3(3.5 1);1.989(0.71);1.175(0.39);0(4.74) |
| 1-120 | 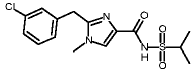 | Beispiel I-120: 1H-NMR(400.0 MHz, d6-DMSO): δ= 8.132(2.26);7.959(5.54);7.376(0.68);7.371(0.33);7.358(1.15);7.355(1.75);7.342(0.52);7.336(2 .33);7.32(4.42);7.317(5.23);7.303(0.9);7.3(0.87);7.204(1.71);7.185(1.35);4.141(7.04);3.776(0 .54);3.759(1.27);3.742(1.73);3.725(1.32);3.708(0.6);3.575(16);3.456(0.44);3.433(0.44);3.415 (0.44);3.397(0.49);3.314(0.34);2.67(0.39);2.524(1.07);2.51(22.5);2.506(45.13);2.502(59.17); 2.497(42.88);2.493(20.95);2.328(0.35);1.988(0.6);1.292(15.27);1.275(15.03);1.233(0.42);1.2 16(0.33);0.008(0.4);0(10.5);-0.008(0.38) |
| I-121 | 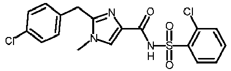 | Beispiel I-121: 1H-NMR(400.0 MHz, d6-DMSO): δ= 8.132(2.48);8.078(1.71);8.074(1.79);8.059(1.86);8.055(1.86);7.869(5.31);7.567(0.33);7.564(0 .35);7.548(1.19);7.544(1.2);7.528(4.04);7.523(3.64);7.509(1.17);7.504(1.78);7.499(1.03);7.48 5(1.49);7.48(1.08);7.469(0.72);7.464(0.62);7.409(4.25);7.388(5.87);7.279(5.09);7.258(3.69); 4.223(7.08);4.164(0.41);4.145(0.44);4.121(0.46);4.083(0.49);4.067(0.5);4.056(0.54);4.038(0. 56);4.02(0.57);4.009(0.54);4.002(0.54);3.988(0.55);3.981(0.55);3.922(0.56);3.84(0.56);3.781 (0.54);3.75(0.44);3.605(16);2.675(0.35);2.67(0.48);2.666(0.35);2.506(68.49);2.501(90.05);2. 497(66.4);2.332(0.45);2.328(0.58);2.324(0.45);0.008(0.38);0(9.87);-0.008(0.48) |
| I-122 | 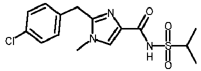 | Beispiel I-122: 1H-NMR(400.0 MHz, d6-DMSO): δ= 8.133(6.19);7.951(5.71);7.389(3.87);7.368(5.47);7.268(4.69);7.247(3.33);4.121(7.38);3.774(0 .58);3.757(1.28);3.74(1.72);3.722(1.35);3.705(0.62);3.558(16);3.502(0.34);3.472(0.34);3.456 (0.33);3.449(0.33);3.379(0.36);2.671(0.38);2.506(44.48);2.502(57.74);2.497(41.9);2.329(0.36 );1.988(0.83);1.291(15.24);1.274(14.97);1.175(0.45);0.008(0.38);0(9.25);-0.008(0.36) |
| I-123 | 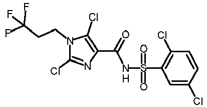 | Beispiel I-123: 1H-NMR(400.0 MHz, d6-DMSO): δ= 8.068(13.58);8.062(14.32);7.819(5.17);7.812(4.87);7.798(7.78);7.791(7.6);7.719(14.82);7.69 7(9.69);4.292(7.44);4.275(16);4.258(7.83);2.902(0.91);2.885(1.99);2.874(3.12);2.858(6.09);2 .846(3.84);2.841(3.6);2.83(6.31);2.813(3.05);2.802(2.24);2.785(0.98);2.676(0.58);2.672(0.8); 2.668(0.6);2.525(1.65);2.507(98.82);2.502(137.67);2.498(106.15);2.334(0.62);2.329(0.86);2. 325(0.65);2.074(2.88);0.008(0.89);0(28.85);-0.008(1.39) |
| I-124 | 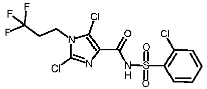 | Beispiel I-124: 1H-NMR(400.0 MHz, d6-DMSO): δ= 8.138(6.2);8.119(6.67);7.992(0.74);7.972(0.77);7.719(1.69);7.7(5.08);7.681(5.53);7.663(9.56) ;7.644(3.97);7.623(4.62);7.604(7.73);7.586(3.14);7.536(0.51);7.518(0.73);7.501(0.33);5.753( 16);4.289(6.72);4.272(13.72);4.255(6.93);4.056(0.4);4.039(1.2);4.021(1.2);4.003(0.43);3.353 (0.9);3.275(0.57);2.898(0.93);2.882(1.96);2.87(3.06);2.854(5.52);2.842(3.91);2.826(5.63);2.8 |
| | | 09(2.92);2.798(2.07);2.782(0.88);2.671(0.52);2.502(77.56);2.329(0.45);1.989(4.86);1.235(0.5 8);1.193(1.3);1.175(2.54);1.158(1.26);0(3.73) |
| I-125 | 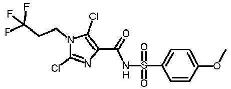 | Beispiel I-125: 1H-NMR(400.0 MHz, d6-DMSO): δ= 11.931(0.63);7.925(4.07);7.903(4.36);7.761(0.47);7.739(0.5);7.196(0.53);7.145(4.14);7.123(3 .93);7.093(0.51);7.071(0.46);5.754(3.3);4.286(1.7);4.27(3.58);4.253(1.78);4.038(0.53);4.021( 0.54);3.846(16);3.824(2.04);3.32(5.15);2.868(0.46);2.856(0.74);2.84(1.38);2.828(0.91);2.812 (1.42);2.795(0.7);2.784(0.5);2.506(20.92);2.502(27.3);2.498(20.26);1.989(2.21);1.193(0.58); 1.175(1.15);1.158(0.56);0(1.51) |
| I-126 | 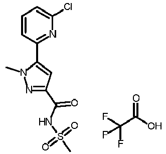 | Beispiel I-126: 1H-NMR(400.0 MHz, d6-DMSO): δ= 8.036(1.34);8.016(3.14);7.996(2.02);7.911(3.52);7.892(2.62);7.584(3.16);7.564(2.91);7.44(3. 97);4.213(16);4.153(0.83);4.107(0.38);4.099(0.38);4.074(0.39);4.032(0.5);4.002(0.48);3.974( 0.52);3.969(0.54);3.933(0.62);3.903(6.38);3.74(1.55);3.508(6.73);3.45(7.38);3.302(4.16);3.27 6(14.48);3.169(2.83);2.944(0.73);2.909(0.54);2.89(0.46);2.88(0.44);2.844(0.36);2.695(0.77); 2.672(0.96);2.542(1.9);2.507(127.41);2.503(164.89);2.499(132.47);2.33(1.36);2.198(0.36);2. 178(0.43);2.157(0.37);1.265(0.36);1.259(0.36);1.235(0.68);0(4.6) |
| I-127 | 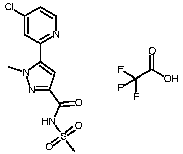 | Beispiel I-127: 1H-NMR(400.0 MHz, d6-DMSO): δ= 8.693(3.34);8.68(3.47);8.08(3.95);8.076(4.1);7.61(2);7.606(2.14);7.596(2.03);7.593(2.05);7.5 29(4.14);4.595(0.46);4.232(16);4.206(0.99);4.178(0.42);4.156(0.41);4.144(0.39);4.098(0.42); 4.053(0.52);4.012(0.49);4(0.51);3.989(0.52);3.962(0.55);3.957(0.59);3.943(0.6);3.902(9.74); 3.83(1.01);3.764(1.36);3.418(16.39);3.318(20.48);3.219(4.27);3.169(13.35);2.993(1.08);2.91( 1.19);2.892(0.77);2.87(0.58);2.771(0.38);2.75(0.33);2.731(0.34);2.695(0.52);2.69(0.9);2.672( 1.54);2.647(0.44);2.55(2.78);2.543(2.75);2.507(217.6);2.503(278.75);2.499(217.29);2.33(2.1 6);2.297(0.62);2.276(0.56);2.263(0.52);2.23(0.49);2.198(0.47);2.178(0.49);2.157(0.43);2.106 (0.36);2.094(0.33);2.088(0.33);2.042(0.32);1.258(0.44);1.235(1.04);1.213(0.34);1.194(0.39); 1.184(0.36);1.178(0.34);0(8.31) |
| I-128 | 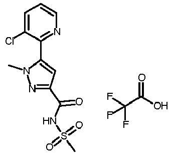 | Beispiel I-128: 1H-NMR(400.0 MHz, d6-DMSO): δ= 11.914(0.64);8.722(1.8);8.713(1.8);8.71(1.8);8.184(1.67);8.164(1.78);7.597(1.48);7.585(1.48) ;7.576(1.42);7.565(1.4);7.321(5.7);7.204(1.52);7.076(1.73);6.948(1.53);4.596(0.44);4.03(0.33 );3.949(16);3.924(0.78);3.911(1.09);3.903(5.09);3.878(0.68);3.872(0.67);3.829(0.99);3.768(1 .25);3.526(7.06);3.509(6.86);3.355(15.8);3.324(1.53);3.304(1.34);3.219(0.91);3.169(8.4);3.13 (0.44);3.075(0.35);2.91(0.6);2.672(0.89);2.542(1.28);2.507(113.97);2.503(144.31);2.499(111. 51);2.33(0.83);0(1.5) |
| I-129 | 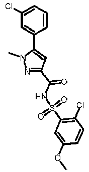 | Beispiel I-129: 1H-NMR(400.0 MHz, d6-DMSO): δ= 7.67(2.99);7.617(2.97);7.609(3.11);7.586(2.36);7.563(3.91);7.555(8.42);7.539(1.09);7.306(1. 4);7.299(1.35);7.284(1.23);7.277(1.14);7.065(4.56);3.941(14.79);3.902(1.84);3.86(16);3.338( 64.09);3.169(0.58);2.672(0.55);2.507(73.01);2.503(92.19);2.499(71.15);2.329(0.51);0(4.17) |
| I-130 | 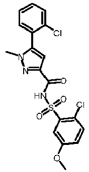 | Beispiel I-130: 1H-NMR(400.0 MHz, d6-DMSO): δ= 7.681(1.95);7.661(2.76);7.622(3.05);7.615(3.18);7.591(2.72);7.584(1.29);7.57(3.88);7.552(0. 95);7.546(1.42);7.538(1.15);7.524(3.41);7.518(3.94);7.499(1.61);7.48(0.45);7.31(1.44);7.303 (1.4);7.288(1.24);7.281(1.17);6.966(4.37);3.902(2.58);3.86(16);3.728(14.96);3.713(1.08);3.3 42(99.08);3.169(0.69);2.672(0.6);2.503(104.25);2.329(0.57);1.299(0.4);1.259(0.66);1.244(0.5 2);1.236(0.34);0(3.46) |
| I-131 | 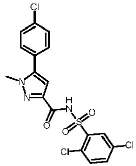 | Beispiel I-131: 1H-NMR(400.0 MHz, d6-DMSO): δ= 8.101(2.29);8.095(2.39);7.831(0.86);7.825(0.83);7.809(1.3);7.803(1.26);7.73(2.36);7.708(1.5 7);7.596(16);7.023(3.73);3.928(11.01);3.902(1.96);3.509(0.43);3.349(20.18);3.17(0.71);2.672 (0.38);2.508(49.34);2.503(63.19);2.499(47.1);2.33(0.38);0(2.47) |
| I-132 | 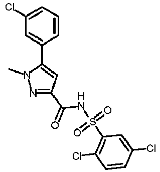 | Beispiel I-132: 1H-NMR(400.0 MHz, d6-DMSO): δ= 8.102(3.32);8.096(3.46);7.83(1.23);7.824(1.2);7.809(1.85);7.803(1.83);7.731(3.28);7.709(2.1 8);7.67(3.31);7.569(1.3);7.554(8.9);7.539(1.24);7.053(5.23);4.597(0.33);3.942(16);3.902(2.3 3);3.353(41.64);3.17(0.65);2.673(0.54);2.508(72.11);2.504(90.62);2.5(70.45);2.33(0.53);0(2. 46) |
| I-133 | 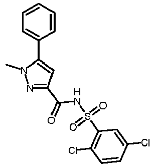 | Beispiel I-133: 1H-NMR(400.0 MHz, d6-DMSO): δ= 8.104(3.26);8.098(3.42);7.833(1.14);7.828(1.08);7.812(1.7);7.806(1.62);7.734(3.06);7.712(2. 02);7.572(1.77);7.553(4.77);7.528(4.18);7.509(2.97);7.504(2.34);7.487(1.39);7.47(0.38);6.99 6(4.29);3.932(16);3.903(2.14);3.508(0.44);3.345(6.58);3.169(0.37);2.671(0.53);2.506(67.41); 2.502(86.42);2.498(66.85);2.329(0.48);0(3.49) |
| I-134 | 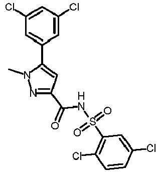 | Beispiel I-134: 1H-NMR(601.6 MHz, d6-DMSO): δ= 8.09(2.74);8.086(2.72);7.803(0.77);7.792(0.96);7.755(1.92);7.752(3.56);7.749(2.09);7.71(1.6 7);7.696(1.33);7.681(8.84);7.678(8.1);7.081(1.6);3.947(16);3.336(2.23);2.616(0.5);2.613(0.6 7);2.61(0.49);2.522(2.86);2.519(3.52);2.516(4.08);2.507(40.71);2.504(79.18);2.501(104.2);2. 498(75.78);2.495(35.83);2.388(0.5);2.385(0.66);2.382(0.47);1.908(1.07);0.005(0.49);0(10.44) ;-0.006(0.33) |
| I-135 | 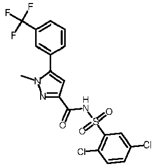 | Beispiel I-135: 1H-NMR(400.0 MHz, d6-DMSO): δ= 8.107(3.05);8.101(3.41);7.927(3.34);7.914(1.86);7.894(2.14);7.873(1.47);7.853(2.19);7.831(1 .26);7.809(1.89);7.79(1.68);7.771(2.18);7.751(0.91);7.734(2.69);7.712(1.78);7.135(3.91);3.95 5(16);3.902(3.86);3.509(0.55);3.346(15.26);3.169(2.28);2.672(0.67);2.507(86.95);2.503(112. 52);2.499(88.88);2.33(0.62);0(5.25) |
| I-136 | 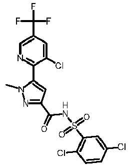 | Beispiel I-136: 1H-NMR(400.0 MHz, d6-DMSO): δ= 9.109(3.91);8.711(3.96);8.103(3.36);8.097(3.46);7.81(1.16);7.79(1.73);7.716(2.55);7.695(1.7 1);7.413(2.72);4.022(0.33);3.984(16);3.902(5.4);3.508(0.81);3.343(33.51);3.169(1.08);2.859( 0.41);2.672(0.93);2.507(121.65);2.503(152.84);2.499(116.39);2.329(0.83);1.249(0.33);1.235( 0.48);0.94(0.44);0.923(0.44);0(7.67) |
| I-137 | 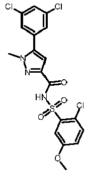 | Beispiel I-137: 1H-NMR(400.0 MHz, d6-DMSO): δ= 7.755(4.09);7.712(0.45);7.683(8.73);7.611(3.26);7.604(3.44);7.578(2.1);7.556(2.43);7.292(1. 63);7.274(1.43);7.102(3.54);4.222(0.32);4.022(0.33);4.006(0.35);3.95(14.52);3.902(3.72);3.8 57(16);3.816(0.51);3.674(0.32);3.338(390.16);2.672(1.34);2.503(214.55);2.329(1.21);1.237(0 .45);0.94(0.45);0.923(0.48);0.912(0.41);0(4.52) |
| I-138 | 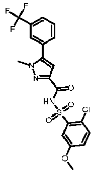 | Beispiel I-138: 1H-NMR(400.0 MHz, d6-DMSO): δ= 7.927(3.03);7.916(1.74);7.896(1.89);7.874(1.3);7.854(1.91);7.792(1.44);7.772(1.91);7.753(0. 73);7.621(2.95);7.614(3.06);7.589(2.41);7.566(2.81);7.31(1.53);7.302(1.47);7.287(1.33);7.28 (1.24);7.143(4.93);3.955(14.91);3.902(2.7);3.862(16);3.341(80.08);3.17(0.92);2.672(0.55);2. 507(76.32);2.503(93.25);2.33(0.52);0(3.36) |
| I-139 | 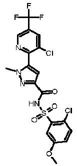 | Beispiel I-139: 1H-NMR(400.0 MHz, d6-DMSO): δ= 9.112(3.03);8.713(3.05);7.626(2.93);7.618(3.04);7.586(1.81);7.564(2.09);7.454(2.94);7.307(1 .17);7.3(1.19);7.286(1.04);7.279(0.99);3.988(14.57);3.968(0.39);3.902(3.1);3.862(16);3.51(0. 34);3.342(111.04);3.17(1.98);2.672(0.65);2.508(87.69);2.504(110.16);2.5(83.97);2.33(0.59);0 (3.38) |
| I-140 | 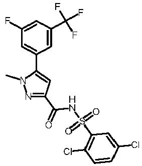 | Beispiel I-140: 1H-NMR(400.0 MHz, d6-DMSO): δ= 8.104(3.27);8.098(3.48);7.879(1.45);7.856(2.83);7.833(2.57);7.828(1.93);7.811(4.89);7.732(3 .17);7.71(2.13);7.177(4.85);4.596(0.51);3.98(16);3.902(2.9);3.351(51.88);3.17(0.62);2.673(0. 65);2.508(84.71);2.504(108.82);2.499(82.27);2.33(0.63);0(3.58) |
| I-141 | 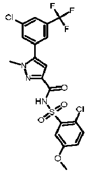 | Beispiel I-141: 1H-NMR(400.0 MHz, d6-DMSO): δ= 8.036(2.91);8.009(2.78);7.93(2.87);7.617(2.94);7.609(3.06);7.585(1.82);7.563(2.12);7.306(1. 16);7.299(1.18);7.284(1.01);7.277(1);7.188(3.34);3.968(14.23);3.903(2.93);3.86(16);3.333(3 6.85);2.671(0.62);2.507(80.21);2.502(103.14);2.498(78.51);2.329(0.56);0(4.69) |
| I-142 | 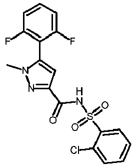 | Beispiel I-142: 1H-NMR(400.0 MHz, d6-DMSO): δ= 8.165(2.23);8.146(2.36);7.712(1.43);7.695(3.05);7.674(4.15);7.663(3.54);7.637(1.27);7.624(1 .45);7.605(1.95);7.586(0.88);7.357(3.18);7.337(5.28);7.317(2.66);7.054(2.12);5.139(0.37);3.9 02(11.16);3.777(16);3.575(0.98);3.508(0.39);3.338(97.18);3.304(6.08);3.286(3.04);3.169(0.8) ;3.048(0.34);2.88(0.52);2.866(0.99);2.852(0.92);2.838(0.5);2.696(7.08);2.676(0.79);2.672(1.0 3);2.668(0.82);2.542(0.75);2.512(64.74);2.508(130.84);2.503(173.6);2.499(129.04);2.494(65. 84);2.334(0.72);2.33(0.98);2.325(0.75);2.197(0.8);2.177(1.54);2.157(1.07);1.938(0.38);1.921 (0.92);1.918(0.76);1.902(1.06);1.882(0.85);1.235(0.44);0(7.59) |
| I-143 | 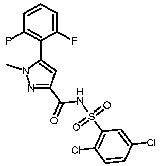 | Beispiel I-143: 1H-NMR(400.0 MHz, d6-DMSO): δ= 7.968(3.31);7.662(0.57);7.641(1.48);7.624(2.35);7.605(1.69);7.586(0.64);7.492(2.29);7.463(2 .65);7.443(1.33);7.321(2.54);7.301(4.08);7.282(2.17);7.053(0.39);7.035(0.41);6.67(0.6);6.652 (0.93);6.632(2.01);6.024(0.43);6.016(0.45);4.107(0.33);4.1(0.35);3.902(16);3.67(11.06);3.50 8(0.74);3.493(0.43);3.479(0.61);3.469(0.69);3.465(0.67);3.458(0.68);3.434(1.08);3.338(345.9 9);3.267(2.8);3.222(0.55);3.173(1.8);3.164(1.79);2.892(0.53);2.731(0.33);2.695(0.33);2.69(0. 5);2.676(1.47);2.672(2.01);2.668(1.53);2.542(6.09);2.507(274.83);2.503(360.79);2.499(268.1 7);2.451(0.43);2.334(1.49);2.33(2.05);2.325(1.51);1.298(0.34);1.259(0.47);1.249(0.66);1.236 (1.16);0.008(0.62);0(18.57);-0.008(0.76) |
| I-144 | 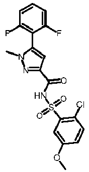 | Beispiel I-144: 1H-NMR(400.0 MHz, d6-DMSO): δ= 7.681(0.83);7.676(0.79);7.66(1.68);7.644(2.04);7.627(1.47);7.607(0.68);7.577(2.91);7.474(0. 43);7.452(0.52);7.414(0.75);7.403(0.71);7.349(1.88);7.336(3);7.33(3.21);7.317(4.44);7.297(2 .32);7.118(0.58);7.049(0.46);7.031(0.43);6.828(0.37);6.755(1.94);6.025(0.4);6.016(0.42);3.90 3(16);3.82(13.05);3.801(1.79);3.737(6.4);3.717(6.83);3.508(0.57);3.479(0.61);3.47(0.69);3.4 66(0.7);3.459(0.69);3.434(1);3.338(177.88);3.292(5.15);3.268(2.83);3.252(1.71);3.17(1.55);3 .147(0.42);3.024(1.29);2.988(3.05);2.853(2.22);2.677(0.96);2.672(1.28);2.668(0.97);2.542(1. 62);2.512(88.83);2.508(175.36);2.503(229.74);2.499(170.48);2.334(0.95);2.33(1.27);2.326(0. 95);1.259(0.37);1.25(0.53);1.235(1.13);0.008(0.39);0(12.45);-0.008(0.55) |
| I-145 | 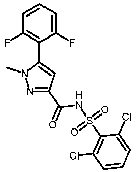 | Beispiel I-145: 1H-NMR(400.0 MHz, d6-DMSO): δ= 7.684(0.56);7.666(1.26);7.647(1.87);7.628(1.35);7.609(0.65);7.598(0.34);7.545(1.77);7.526(2 .72);7.443(0.81);7.428(0.99);7.409(0.78);7.388(0.46);7.338(2.93);7.318(4.67);7.298(2.37);6.8 24(0.35);6.819(0.35);3.902(16);3.821(0.46);3.723(8.26);3.508(0.67);3.49(0.53);3.479(0.72);3 .47(0.8);3.466(0.83);3.458(0.9);3.434(1.36);3.337(139.16);3.268(2.78);3.169(2.06);2.85(9.93) ;2.69(0.66);2.676(0.94);2.672(1.25);2.668(0.95);2.542(1.44);2.507(172);2.503(225.06);2.499( 168.13);2.334(0.91);2.33(1.28);2.325(0.97);1.259(0.32);1.249(0.4);1.236(0.73);0.008(0.37);0 (11.46);-0.008(0.52) |
| I-146 | 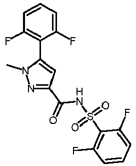 | Beispiel I-146: 1H-NMR(400.0 MHz, d6-DMSO): δ= 7.659(0.69);7.64(1.7);7.622(2.63);7.602(1.8);7.584(0.7);7.425(1.85);7.353(0.46);7.342(0.64); 7.32(3.67);7.3(5.83);7.28(3);7.03(2.78);7.009(4.45);6.99(2.36);6.915(0.38);6.67(0.73);6.654( 0.85);6.602(3.19);5.845(0.47);5.836(0.4);5.791(0.54);4.614(0.34);4.596(0.58);4.577(0.33);3.9 02(10.67);3.82(0.55);3.762(0.88);3.665(16);3.543(0.34);3.508(0.83);3.487(0.7);3.477(0.66);3 .342(602.47);3.267(1.36);3.22(0.45);3.175(0.73);3.162(0.69);2.689(0.49);2.676(1.46);2.672(2 );2.566(0.34);2.542(7.36);2.507(273.24);2.503(359.09);2.499(271.98);2.334(1.5);2.33(2.02);2 .326(1.56);1.298(0.45);1.258(0.69);1.248(0.63);1.235(1.27);0.007(0.44);0(13.17) |
| I-147 | 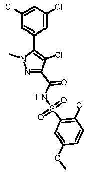 | Beispiel I-147: 1H-NMR(400.0 MHz, d6-DMSO): δ= 7.777(1.8);7.772(3.59);7.767(2.14);7.608(7.16);7.603(7.11);7.554(2.72);7.546(2.79);7.312(1. 66);7.29(1.91);6.993(0.98);6.986(1.02);6.972(0.91);6.964(0.87);5.756(4.46);3.791(16);3.759( 13.41);3.323(34.63);2.671(0.45);2.565(2.31);2.506(56.32);2.502(74.46);2.497(56.62);2.329(0 .42);2.324(0.33);1.989(1.24);1.235(0.71);1.192(0.36);1.175(0.64);1.157(0.35);0.854(0.32);0.0 08(2.64);0(64.84);-0.15(0.32) |
| I-148 | 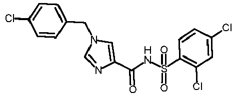 | Beispiel I-148: 1H-NMR(400.0 MHz, d6-DMSO): δ= 8.586(3.13);8.318(0.35);8.063(7.14);8.042(7.95);8.026(8.63);8.022(8.23);7.978(0.45);7.956(0 .48);7.855(0.46);7.85(0.47);7.73(0.8);7.716(6.08);7.711(6.54);7.639(0.32);7.617(0.4);7.607(4 .44);7.601(4.07);7.585(3.98);7.58(3.76);7.478(6.07);7.473(2.49);7.462(3.7);7.457(16);7.452( 2.83);7.429(13.82);7.424(3.38);7.413(2.21);7.408(5.37);5.301(15.47);4.756(0.35);4.72(0.36); 4.67(0.4);4.625(0.4);4.616(0.41);4.596(0.42);4.547(0.42);4.502(0.41);4.454(0.39);4.427(0.37) ;4.348(0.32);2.944(0.82);2.784(0.67);2.676(0.52);2.672(0.73);2.668(0.54);2.525(2.48);2.52(4. 01);2.512(41.71);2.507(83.46);2.503(108.93);2.498(78.6);2.494(37.98);2.334(0.57);2.33(0.77 );2.325(0.56);1.958(0.71);0(2.9) |
| I-149 | 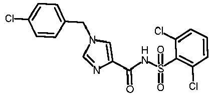 | Beispiel I-149: 1H-NMR(601.6 MHz, d6-DMSO): δ= 8.551(1.53);7.97(3.42);7.609(0.38);7.569(0.34);7.505(7.7);7.492(11.73);7.476(0.95);7.472(8. 47);7.469(3.12);7.462(3.91);7.458(16);7.454(2.24);7.435(0.39);7.429(3.85);7.423(11.94);7.41 7(4.1);7.415(4.14);7.412(3.59);7.409(6.9);7.403(2.25);7.36(0.36);7.347(0.49);5.297(14.63);3. 616(0.52);3.419(124.87);3.107(0.95);3.012(0.64);2.944(1.75);2.784(1.22);2.62(0.85);2.617(1. 88);2.614(2.63);2.61(1.88);2.607(0.87);2.541(0.7);2.538(0.48);2.523(5.74);2.52(6.98);2.517( 6.8);2.508(135.6);2.505(293.61);2.502(407.76);2.499(296.55);2.496(136.11);2.392(0.78);2.3 89(1.78);2.386(2.52);2.383(1.79);2.38(0.8);2.349(0.52);1.957(1.26);1.909(1.45);0.096(0.67); 0.005(5.53);0(191.57);-0.006(5.64);-0.009(0.35);-0.1(0.68) |
| I-150 | 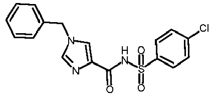 | Beispiel I-150: 1H-NMR(400.0 MHz, d6-DMSO): δ= 8.215(2.7);7.973(7.76);7.971(7.29);7.935(9.04);7.914(10.43);7.644(9.04);7.622(7.73);7.538(0 .56);7.391(1.87);7.388(1.47);7.37(5.06);7.356(10.05);7.34(6.95);7.333(11.61);7.325(5.47);7.3 15(5.16);5.259(16);2.676(0.4);2.671(0.56);2.667(0.4);2.525(1.88);2.511(36.3);2.507(71.01);2 .502(92.19);2.498(68.32);2.494(35.07);2.333(0.54);2.329(0.69);2.325(0.53);0.008(1.2);0(35.8 6);-0.008(2.06) |
| I-151 | 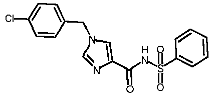 | Beispiel I-151: 1H-NMR(400.0 MHz, d6-DMSO): δ= 8.036(4.94);7.995(8.79);7.967(7.38);7.948(7.91);7.838(0.35);7.822(0.37);7.687(1.32);7.669(3 .9);7.651(3.16);7.609(5.96);7.59(8.43);7.571(3.46);7.446(7.34);7.424(10.98);7.347(10.45);7.3 26(6.9);5.246(16);2.672(0.47);2.503(68.54);2.33(0.49);1.356(0.43);0(10.23) |
| I-152 | 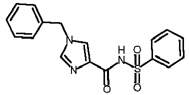 | Beispiel I-152: 1H-NMR(400.0 MHz, d6-DMSO): δ= 8.055(4.04);7.993(7.98);7.991(7.92);7.965(6.81);7.946(7.51);7.943(5.95);7.682(1.2);7.663(3. 83);7.645(3);7.604(5.76);7.585(8.1);7.567(3.23);7.389(1.19);7.385(1.8);7.367(4.94);7.362(3. 75);7.35(7.12);7.334(3.33);7.331(3.78);7.318(11.36);7.298(4.91);5.759(0.32);5.245(16);2.507 (35.67);2.503(45.85);2.498(34.74);0(9.01) |
| I-153 | 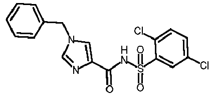 | Beispiel I-153: 1H-NMR(400.0 MHz, d6-DMSO): δ= 8.855(4.05);8.014(6.81);7.997(4.83);7.992(4.69);7.609(2.34);7.604(2.34);7.588(3.49);7.533(4 .92);7.512(2.92);7.403(16);7.383(4.76);5.326(12.22);2.672(0.36);2.504(49.08);2.33(0.4);2.07 6(2.1);0(0.85) |
| I-154 | 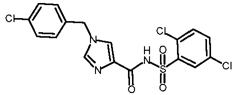 | Beispiel I-154: 1H-NMR(400.0 MHz, d6-DMSO): δ= 8.816(3.36);8.037(7.04);8.033(6.94);8.002(6.14);7.996(6.27);7.623(2.65);7.617(2.51);7.602(4 .42);7.595(4.38);7.545(8.05);7.524(4.62);7.486(3.11);7.48(1.66);7.47(2.78);7.464(16);7.452( 13.84);7.446(2.38);7.436(1.32);7.43(2.78);5.322(13.31);2.676(0.43);2.672(0.62);2.667(0.44); 2.525(2.85);2.512(36.35);2.507(71.95);2.503(93.8);2.498(68.05);2.494(33.38);2.334(0.45);2. 33(0.61);2.325(0.46);0(3.86) |
| I-155 | 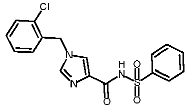 | Beispiel I-155: 1H-NMR(400.0 MHz, d6-DMSO): δ= 8(5.17);7.977(6.84);7.959(8.06);7.955(6.08);7.947(8.32);7.944(7.79);7.697(1.26);7.684(0.97) ;7.678(4.05);7.673(1.36);7.66(3.07);7.618(5.75);7.598(7.94);7.588(1.05);7.584(1.55);7.58(3.0 9);7.569(0,44);7.524(2.9);7.518(3.24);7.504(2.74);7.5(4.24);7.415(1.02);7.41(1.4);7.396(3.36 );7.391(3.71);7.384(3.31);7.378(5.9);7.372(3.21);7.366(4.01);7.362(3.95);7.347(1.35);7.343( 1.01);7.228(3.3);7.223(2.55);7.211(2.73);7.205(2.54);5.76(1.9);5.368(16);2.526(0.93);2.512( 13.02);2.508(25.89);2.504(33.93);2.499(24.83);2.495(12.34);1.357(1.3);0(0.97) |
| I-156 | 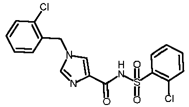 | Beispiel I-156: 1H-NMR(400.0 MHz, d6-DMSO): δ= 8.202(3.88);8.116(3.4);8.112(3.59);8.096(3.65);8.093(3.69);7.995(7.72);7.657(0.98);7.653(1. 06);7.637(2.98);7.62(3.6);7.616(3.67);7.602(5.88);7.585(2.42);7.574(2.76);7.57(2.36);7.554( 3.7);7.542(3.18);7.537(4.22);7.523(2.93);7.519(3.81);7.437(0.98);7.432(1.31);7.419(3.41);7.4 13(3.98);7.41(3.71);7.403(5.45);7.392(4.3);7.387(3.58);7.373(1.22);7.369(0.88);7.302(3.61); 7.285(2.56);7.279(2.3);5.398(16);2.672(0.46);2.506(57.16);2.503(70.75);2.329(0.51);0(0.7) |
| I-157 | 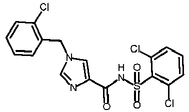 | Beispiel I-157: 1H-NMR(400.0 MHz, d6-DMSO): δ= 8.538.66);7.954(7.85);7.95(7.54);7.606(0.37);7.548(7.05);7.545(8.23);7.531(4.53);7.527(16 );7.476(5.31);7.459(3.83);7.453(3.31);7.445(1.61);7.436(2.63);7.432(3.69);7.427(3.83);7.42( 3.21);7.414(5.37);7.408(3.3);7.401(4.19);7.397(3.97);7.383(1.57);7.378(1.19);7.374(0.48);7.3 3(3.85);7.326(2.75);7.313(2.73);7.307(2.27);5.435(15.19);2.676(0.42);2.671(0.57);2.667(0.41 );2.525(1.77);2.511(32.22);2.507(63.26);2.502(81.92);2.498(59.27);2.493(28.65);2.334(0.41); 2.329(0.56);2.325(0.41);2.075(0.47);0.008(1.61);0(42.43);-0.008(1.69) |
| I-158 | 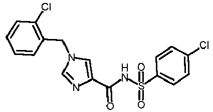 | Beispiel I-158: 1H-NMR(400.0 MHz, d6-DMSO): δ= 8.128(4.09);7.96(8.06);7.938(16);7.674(8.88);7.652(7.63);7.528(2.48);7.523(2.76);7.508(2.89 );7.505(3.6);7.421(1);7.416(1.26);7.403(2.92);7.398(3.21);7.384(4.42);7.379(2.95);7.37(3.34) ;7.367(3.14);7.352(1.18);7.348(0.97);7.24(3.12);7.236(2.7);7.223(2.56);7.218(2.34);5.382(14. 14);2.673(0.35);2.508(43.28);2.504(54.52);2.5(41.79);2.331(0.41);0(0.62) |
| I-159 | 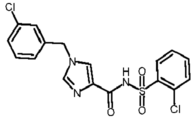 | Beispiel I-159: 1H-NMR(400.0 MHz, d6-DMSO): δ= 8.318(0.56);8.296(3.23);8.108(3.53);8.104(3.74);8.088(3.97);8.084(4.13);8.074(8.28);8.071(8 .24);7.64(1);7.636(1.18);7.62(2.85);7.616(2.78);7.603(3.85);7.599(3.75);7.588(4.5);7.584(6.2 6);7.568(2.51);7.562(3.32);7.557(2.37);7.542(3.45);7.538(2.83);7.524(1.75);7.52(1.62);7.492 (5.67);7.446(0.43);7.426(8.02);7.422(4.31);7.416(8.59);7.413(9.74);7.407(0.78);7.402(0.61); 7.348(0.54);7.345(0.6);7.337(2.37);7.333(2.39);7.326(3.35);7.315(1.63);7.312(1.47);5.285(16 );2.512(14.68);2.508(29.98);2.503(40.28);2.499(29.82);2.494(14.83);0.008(1.08);0(32.15);-0.008(1.45) |
| I-160 | 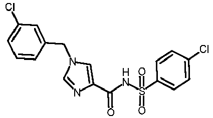 | Beispiel I-160: 1H-NMR(400.0 MHz, d6-DMSO): δ= 8.19(3.18);8.008(5.92);7.944(7.37);7.923(8.87);7.839(1.63);7.835(0.61);7.818(2.07);7.67(2.2 6);7.665(1);7.649(8.01);7.629(6.27);7.474(2.13);7.459(6.51);7.428(0.45);7.418(0.66);7.409(8 .9);7.396(10.38);7.378(0.33);7.357(0.34);7.31(0.66);7.302(2.58);7.3(2.56);7.291(3.75);7.281( 1.89);7.278(1.75);5.264(16);2.508(24.63);2.504(31.81);2.5(23.76);1.357(1.03);0.007(0.89);0( 21.16);-0.001(20.42);-0.008(1.13) |
| I-161 | 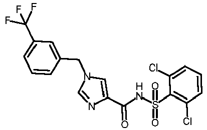 | Beispiel I-161: 1H-NMR(400.0 MHz, d6-DMSO): δ= 9.106(0.36);8.699(3.57);8.096(9.01);7.925(4.79);7.856(6.32);7.752(2.88);7.733(4.25);7.712(3 .03);7.695(4.89);7.664(4.05);7.645(4.54);7.628(4.48);7.609(6.1);7.538(2.77);7.528(7.06);7.52 1(3.04);7.509(14.06);7.499(1.78);7.457(4.7);7.44(3.55);7.435(3.11);7.418(1.93);7.394(0.77); 7.376(1.68);7.331(0.61);7.315(0.45);7.309(0.39);5.419(16);2.674(0.5);2.504(87.53);2.33(0.6) ;0(19.54) |
| I-162 | 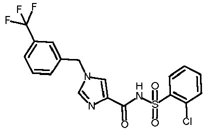 | Beispiel I-162: 1H-NMR(400.0 MHz, d6-DMSO): δ= 8.329(2.65);8.102(12.23);8.099(9.41);8.087(4.24);8.083(4.2);7.993(1.47);7.99(1.53);7.974(1. 7);7.97(1.69);7.807(5.56);7.736(2.23);7.719(3.54);7.672(1.34);7.653(8.01);7.65(8.39);7.638( 4.2);7.634(5.79);7.617(7.15);7.609(3.81);7.604(5.72);7.6(4.23);7.588(5.33);7.585(7.03);7.568 (2.71);7.562(3.54);7.558(2.5);7.542(4.76);7.538(4.02);7.525(2.87);7.523(2.68);7.521(2.87);7. 505(0.86);7.501(0.77);5.76(0.82);5.385(16);2.677(0.46);2.672(0.66);2.668(0.49);2.512(40.64) ;2.508(79.78);2.503(104.25);2.499(78.06);2.495(41.04);2.334(0.58);2.33(0.76);2.325(0.6);0.0 08(1.43);0(36.75);-0.008(1.89) |
| I-163 | 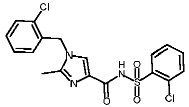 | Beispiel I-163: 1H-NMR(601.6 MHz, d6-DMSO): δ= 8.051(1.63);8.048(1.71);8.038(1.7);8.036(1.69);7.989(0.74);7.986(0.76);7.976(0.81);7.973(0. 79);7.708(5.35);7.649(0.46);7.647(0.54);7.636(1.05);7.634(1.07);7.618(0.59);7.615(0.63);7.6 06(1.15);7.601(1.95);7.593(0.57);7.59(0.49);7.566(1.6);7.564(1.74);7.552(1.94);7.55(2.02);7. 534(0.56);7.531(0.58);7.52(1.09);7.509(1.43);7.507(1.42);7.498(1.82);7.496(1.94);7.492(2.22 );7.49(2.87);7.479(0.96);7.476(0.54);7.468(1.28);7.465(1.01);7.455(1.49);7.452(1.16);7.444( 1.37);7.441(1.42);7.431(1.56);7.428(1.57);7.418(1.1);7.415(1.02);7.402(1.19);7.399(1.23);7.3 89(1.84);7.387(1.87);7.377(0.79);7.374(0.74);7.153(1.46);7.15(1.45);7.14(1.36);7.138(1.29); 5.383(7.17);3.501(5.79);2.617(0.41);2.614(0.58);2.611(0.41);2.523(0.94);2.52(1.2);2.517(1.2 7);2.508(30.18);2.505(65.05);2.502(89.79);2.499(65.02);2.496(30.21);2.456(16);2.389(0.44); 2.386(0.59);2.383(0.43);0.005(1.92);0(62.21);-0.006(2.13) |
| I-164 | 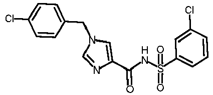 | Beispiel I-164: 1H-NMR(601.6 MHz, d6-DMSO): δ= 8.357(1.16);8.018(9.06);8.015(8.96);7.937(3.49);7.934(6.11);7.931(3.83);7.875(2.37);7.873(3 );7.871(2.31);7.862(2.64);7.86(3.19);7.858(2.48);7.711(2.05);7.709(1.99);7.704(0.71);7.698( 2.52);7.696(2.5);7.606(3.64);7.593(5.91);7.586(0.34);7.58(2.64);7.456(1.02);7.451(8.82);7.44 8(3.14);7.44(3.78);7.437(13.47);7.433(1.76);7.381(10.74);7.378(3.35);7.37(2.76);7.367(7.13) ;5.278(16);2.615(0.46);2.612(0.33);2.524(0.81);2.521(1.02);2.518(1.01);2.509(23.05);2.506(5 1.07);2.503(71.11);2.5(51.75);2.497(23.91);2.39(0.34);2.387(0.46);2.384(0.32);0.005(1.93);0 (63.51);-0.006(2.23) |
| I-165 | 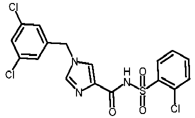 | Beispiel I-165: 1H-NMR(400.0 MHz, d6-DMSO): δ= 8.317(0.35);8.272(3.7);8.113(3.79);8.109(4.16);8.101(9.48);8.097(9.34);8.094(4.84);8.09(4.2 9);7.994(1.52);7.99(1.52);7.974(1.79);7.97(1.74);7.657(0.77);7.653(1.16);7.65(1.16);7.645(1. 21);7.637(2.59);7.633(3.9);7.629(3.2);7.625(4.54);7.621(5.45);7.617(7.98);7.612(10.08);7.60 8(9.52);7.604(3.86);7.597(4.94);7.593(6.8);7.588(1.48);7.584(1.16);7.577(2.65);7.573(1.95); 7.569(3.48);7.565(2.52);7.55(3.51);7.546(2.85);7.541(1.7);7.537(1.55);7.532(2.03);7.528(1.8) ;7.523(1.29);7.522(1.59);7.518(1.51);7.5(16);7.495(14.29);5.758(0.76);5.276(15.01);4.51(0.3 3);4.467(0.36);4.445(0.37);4.436(0.38);4.416(0.39);4.388(0.4);4.372(0.4);4.363(0.41);4.356( 0.41);4.353(0.41);4.317(0.41);4.29(0.41);4.249(0.4);4.144(0.34);4.111(0.33);2.676(0.5);2.672 (0.72);2.667(0.51);2.525(1.73);2.52(2.61);2.512(37.49);2.507(77.1);2.503(103.45);2.498(75.6 1);2.494(35.95);2.334(0.53);2.33(0.74);2.325(0.54);1.356(1.42);0.146(0.36);0.008(2.61);0(90 .79);-0.008(2.91);-0.15(0.37) |
| I-166 | 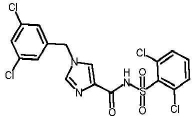 | Beispiel I-166: 1H-NMR(400.0 MHz, d6-DMSO): δ= 8.602(3.61);8.086(9.09);8.083(8.82);7.636(3.44);7.631(6.79);7.627(4.16);7.608(0.48);7.544(1 6);7.539(15.06);7.536(8.44);7.532(7.68);7.514(15.21);7.462(5.92);7.445(4.22);7.44(3.48);7.4 22(2.41);7.375(0.34);5.301(15.77);4.832(0.32);4.778(0.36);4.758(0.37);4.709(0.4);4.694(0.4) ;4.674(0.4);4.653(0.41);4.633(0.41);4.611(0.41);4.582(0.41);4.576(0.41);4.564(0.41);4.558(0. 41);4.542(0.41);4.536(0.4);4.513(0.39);4.483(0.38);4.391(0.32);2.676(0.56);2.672(0.77);2.66 7(0.56);2.542(0.46);2.525(2.2);2.512(41.49);2.507(82.19);2.503(107.19);2.498(77.89);2.494( 37.74);2.334(0.53);2.33(0.73);2.325(0.53);2.076(2.98);0.008(1.57);0(44.22);-0.008(1.6) |
| I-167 | 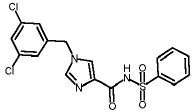 | Beispiel I-167: 1H-NMR(400.0 MHz, d6-DMSO): δ= 8.05(10.33);7.971(4.73);7.953(5.45);7.95(4.11);7.91(3.01);7.892(3.63);7.889(2.7);7.84(2.22); 7.837(2.44);7.821(2.61);7.817(2.54);7.742(0.64);7.724(1.76);7.705(1.41);7.691(0.97);7.673(2 .81);7.656(4.41);7.637(3.69);7.613(4.69);7.593(10.69);7.588(7.07);7.575(3.3);7.568(3.03);7.5 57(0.63);7.553(0.78);7.545(0.58);7.449(9.47);7.445(8.78);7.354(4.08);6.871(0.54);5.245(10.6 4);3.746(0.39);3.728(1.2);3.694(0.43);3.564(16);3.509(0.76);3.465(0.52);3.431(0.47);3.412(0 |
| | | .47);3.404(0.47);3.377(0.51);3.362(0.44);3.338(0.49);3.326(0.47);3.272(0.38);3.255(0.41);3.2 36(0.34);3.169(0.42);2.676(0.64);2.672(0.77);2.668(0.61);2.507(79.01);2.502(97.25);2.498(7 2.62);2.329(0.69);2.325(0.52);2.184(0.84);1.356(5.83);1.234(0.47);0(36.01);-0.008(2.1) |
| I-168 | 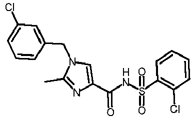 | Beispiel I-168: 1H-NMR(400.0 MHz, d6-DMSO): δ= 8.05(1.74);8.047(1.78);8.031(1.91);8.029(1.94);7.896(7.42);7.497(1.08);7.492(1.22);7.48(4.2 2);7.475(4.75);7.463(5.15);7.444(2.09);7.438(6.29);7.429(3.79);7.425(5.42);7.405(0.36);7.28 1(1.18);7.277(1.22);7.272(1.04);7.266(1.77);7.259(0.99);7.255(0.91);5.294(8.18);2.671(0.38) ;2.525(1.32);2.511(21.87);2.507(42.43);2.502(54.62);2.498(39.56);2.493(19.23);2.463(16);2. 329(0.38);2.075(2.78);0.008(0.98);0(23.85);-0.008(0.87) |
| I-169 | 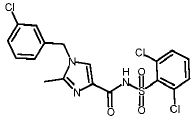 | Beispiel I-169: 1H-NMR(400.0 MHz, d6-DMSO): δ= 8.317(0.59);7.878(14.93);7.497(5.56);7.457(0.96);7.445(15.95);7.442(16);7.438(5.84);7.432( 8.14);7.424(15.33);7.411(0.88);7.357(5.74);7.34(4.41);7.335(3.85);7.318(3.04);7.308(2.26);7. 304(2.43);7.294(3.18);7.286(1.79);7.282(1.58);5.312(14.66);3.675(0.33);3.64(0.33);3.601(0.3 4);3.567(0.33);3.518(0.32);2.676(1.05);2.671(1.41);2.666(1.19);2.541(1.02);2.524(5.67);2.51 1(74.88);2.506(147.24);2.502(201.91);2.498(132.66);2.493(63.96);2.333(0.98);2.329(1.23);2. 324(0.89);2.32(0.42);2.075(0.61);0.146(0.43);0.008(4.6);0(99.54);-0.008(3.71);-0.15(0.43) |
| I-170 | 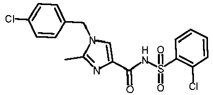 | Beispiel I-170: 1H-NMR(400.0 MHz, d6-DMSO): δ= 8.043(2.2);8.025(2.38);7.88(6.25);7.509(0.66);7.488(2.13);7.475(9.92);7.454(8.16);7.442(2.4 7);7.426(1.01);7.42(0.83);7.361(5.81);7.34(4.16);5.291(8.75);4.181(0.33);4.176(0.34);4.158( 0.35);4.103(0.37);4.077(0.38);4.068(0.38);4.05(0.38);4.013(0.39);3.962(0.39);3.95(0.39);3.90 2(0.38);3.889(0.37);3.87(0.37);3.851(0.37);3.847(0.37);3.835(0.36);3.823(0.35);3.801(0.35); 3.787(0.34);2.671(0.67);2.611(0.36);2.502(78.91);2.45(16);2.329(0.57);2.076(1.63);0(1.75) |
| I-171 | 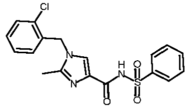 | Beispiel I-171: 1H-NMR(400.0 MHz, d6-DMSO): δ= 7.935(4.36);7.917(4.96);7.914(3.88);7.841(0.47);7.837(0.53);7.821(0.55);7.816(0.61);7.767(5 .34);7.709(0.46);7.706(0.48);7.632(0.65);7.613(1.97);7.595(1.88);7.588(1.31);7.564(3.68);7.5 57(3.25);7.553(3.53);7.544(4.81);7.538(3.68);7.534(3.77);7.527(1.92);7.432(0.91);7.427(1.1) ;7.413(2.33);7.409(2.39);7.394(1.85);7.389(1.87);7.385(2.16);7.381(2.19);7.366(2.9);7.362(3. 27);7.348(1.18);7.344(1.09);7.048(1.74);7.032(1.59);6.87(0.51);5.76(6.89);5.339(9.78);2.512 (18.98);2.507(42.66);2.503(60.79);2.498(49.26);2.494(28.08);2.379(16);2.334(0.69);2.329(0. 8);2.325(0.69);2.183(0.94);1.355(5.72);0(2.25) |
| I-172 | 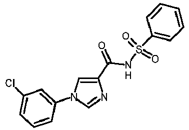 | Beispiel I-172: 1H-NMR(400.0 MHz, d6-DMSO): δ= 8.569(13.72);8.566(16);8.51(14.55);8.507(14.36);8.318(0.34);8.022(12.8);8.003(14.18);8(11. 37);7.922(6.74);7.916(12.71);7.912(7.79);7.73(2.64);7.721(5.15);7.717(6.08);7.711(8.84);7.7 01(6.71);7.696(9.23);7.653(10.84);7.633(15.11);7.615(5.76);7.591(5.44);7.57(11.55);7.55(6.9 9);7.507(6.86);7.486(4.04);5.759(1.4);3.503(0.44);3.47(0.47);3.367(0.49);3.341(0.49);3.212( 0.39);2.676(0.82);2.672(1.07);2.668(0.86);2.507(108.9);2.503(144.5);2.499(112.39);2.334(0. 68);2.33(0.95);2.326(0.74);1.233(0.33);1.18(0.53);0(1.41) |
| I-173 | 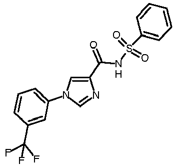 | Beispiel I-173: 1H-NMR(400.0 MHz, d6-DMSO): δ= 8.653(13.23);8.65(14.09);8.581(13.69);8.578(12.81);8.319(0.39);8.149(8.54);8.064(2.6);8.05 8(3.63);8.049(4.51);8.044(4.39);8.036(3.23);8.025(12.54);8.006(13.69);8.003(10.28);7.95(0.3 3);7.848(0.47);7.841(2.81);7.837(3.25);7.821(3.61);7.817(3.84);7.808(1.72);7.798(16);7.792( 7.15);7.789(6.85);7.784(7.5);7.764(1.09);7.731(2.18);7.718(1.74);7.712(7.18);7.707(2.46);7.6 94(5.6);7.654(10.28);7.634(14.13);7.616(5.55);7.603(1.59);7.593(2.26);7.587(5.8);7.576(1.33 );7.568(3.7);7.557(0.67);7.553(0.95);7.546(0.72);7.356(5.12);6.87(0.88);6.65(0.46);5.759(1.6 );3.529(0.41);3.406(0.61);3.337(0.71);3.326(0.7);3.311(0.68);3.226(0.5);3.206(0.48);3.187(0. 44);2.676(1.1);2.672(1.48);2.667(1.17);2.507(153.35);2.503(200.07);2.498(150.26);2.334(0.9 7);2.33(1.33);2.325(0.97);2.184(1.46);1.355(9.64);1.259(0.49);1.234(4.33);1.19(0.39);1.17(0. 45);0.854(0.55);0.836(0.33);0.008(0.83);0(20.14);-0.008(1.03) |
| I-174 | 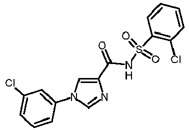 | Beispiel I-174: 1H-NMR(400.0 MHz, d6-DMSO): δ= 8.634(14.75);8.631(16);8.562(13.93);8.56(13.46);8.179(6.38);8.176(6.96);8.16(7.11);8.156(7. 32);7.922(7.14);7.918(13.12);7.913(7.88);7.724(6.43);7.72(6.33);7.704(11.61);7.701(11.33);7 .687(7.09);7.683(7.05);7.671(9.04);7.667(11.97);7.651(4.78);7.648(3.66);7.635(5.4);7.632(4. 59);7.615(7.25);7.607(6.84);7.598(3.78);7.594(3.66);7.587(12.3);7.566(7.27);7.524(7.43);7.5 04(4.27);7.502(4.25);5.759(2.17);2.678(0.39);2.673(0.52);2.669(0.39);2.508(61.23);2.504(79. 62);2.5(60.74);2.336(0.39);2.331(0.53);0(0.91) |
| I-175 | 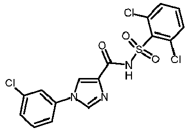 | Beispiel I-175: 1H-NMR(400.0 MHz, d6-DMSO): δ= 8.689(6.64);8.628(8.74);8.626(8.01);7.942(4.05);7.937(7.25);7.932(4.32);7.739(2.73);7.736(2 .59);7.719(3.47);7.716(3.48);7.65(5.4);7.647(6.42);7.629(16);7.614(3.39);7.608(1.04);7.594( 6.84);7.586(6.2);7.574(4.62);7.569(4.27);7.563(3.3);7.545(2.68);7.538(4.62);7.518(2.45);2.67 2(0.49);2.507(60.09);2.503(76.51);2.499(58.37);2.33(0.51);2.077(4.32);0(0.73) |
| I-176 | 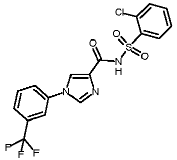 | Beispiel I-176: 1H-NMR(400.0 MHz, d6-DMSO): δ= 8.713(13.25);8.632(11.99);8.184(5.38);8.181(5.85);8.164(6.12);8.161(6.51);8.148(8.67);8.06 3(3.53);8.054(4.09);8.048(4.29);8.041(2.73);8.03(0.63);7.817(16);7.801(6.95);7.782(1.09);7. 727(1.49);7.723(1.61);7.707(4.83);7.705(4.73);7.689(5.56);7.686(5.54);7.67(9.86);7.654(3.91 );7.639(4.39);7.636(3.79);7.619(5.96);7.602(2.61);7.598(2.4);5.76(0.62);2.674(0.58);2.509(6 7.25);2.505(86.27);2.501(67.01);2.331(0.58);0(0.95) |
| I-177 | 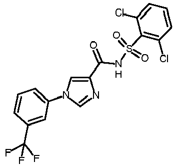 | Beispiel I-177: 1H-NMR(400.0 MHz, d6-DMSO): δ= 8.76(6.51);8.712(9.37);8.709(8.53);8.173(5.55);8.078(2.57);8.067(2.45);8.062(3.5);8.056(1.9 1);8.045(0.35);8.041(0.33);7.839(1.08);7.825(11.05);7.809(4.09);7.789(1.02);7.654(6.07);7.6 5(7.29);7.632(16);7.608(0.54);7.588(6.62);7.571(4.46);7.566(3.59);7.549(2.48);2.513(19.32); 2.509(36.87);2.505(47.67);2.5(35.72);2.078(1.55);0(0.62) |
| I-178 | 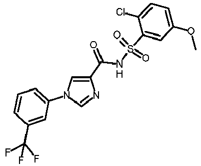 | Beispiel I-178: 1H-NMR(400.0 MHz, d6-DMSO): δ= 8.706(3.4);8.64(3.1);8.638(3.07);8.146(2.27);8.068(0.66);8.062(0.93);8.052(1.1);8.047(1.16); 8.04(0.74);7.828(0.38);7.818(4.26);7.803(1.87);7.627(2.71);7.62(2.91);7.573(2.56);7.551(3.0 4);7.291(1.53);7.283(1.51);7.269(1.34);7.261(1.31);3.861(16);3.818(0.63);2.508(27.43);2.503 (35.74);2.499(27.52);0.008(0.41);0.001(10.16);0(9.96) |
| I-179 | 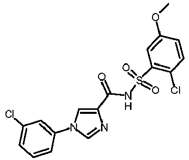 | Beispiel I-179: 1H-NMR(400.0 MHz, d6-DMSO): δ= 8.603(3.16);8.6(3.49);8.564(3.22);8.562(2.95);7.921(1.65);7.916(3.05);7.911(1.82);7.724(1.0 9);7.721(1.04);7.704(1.43);7.701(1.45);7.669(0.37);7.666(0.37);7.622(2.89);7.614(3.12);7.60 5(1.54);7.585(2.81);7.565(1.77);7.557(2.79);7.535(3.19);7.521(1.76);7.5(0.98);7.274(1.62);7. 267(1.59);7.252(1.42);7.245(1.38);5.756(1.13);3.856(16);3.817(0.67);2.508(13.53);2.504(17. 6);2.499(13.34);0(4.35) |
| I-180 | 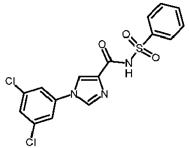 | Beispiel I-180: 1H-NMR(400.0 MHz, d6-DMSO): δ= 8.624(7.06);8.621(8.15);8.556(7.58);8.553(7.48);8.03(0.64);8.022(6.52);8.009(1.8);8.004(7.9 1);8.001(5.78);7.951(15.01);7.947(16);7.731(1.26);7.728(0.87);7.719(0.9);7.713(4.08);7.708( 1.37);7.698(1.95);7.694(3.39);7.689(4.48);7.685(7.06);7.68(3.85);7.654(5.68);7.634(7.91);7.6 2(1.3);7.616(3.06);5.757(0.34);2.526(1.02);2.512(14.35);2.508(28.62);2.504(37.85);2.499(28. 09);2.495(14.3);2.076(2.93);0(1.83) |
| I-181 | 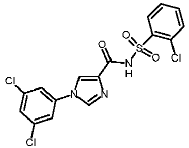 | Beispiel I-181: 1H-NMR(400.0 MHz, d6-DMSO): δ= 8.677(7.08);8.674(7.6);8.588(7.4);8.585(7.09);8.177(3.31);8.173(3.61);8.158(3.72);8.154(3.8 1);7.996(0.4);7.959(0.52);7.954(0.75);7.945(15.23);7.941(16);7.732(0.37);7.727(1.12);7.723( 1.18);7.707(6.4);7.703(9.31);7.699(4.14);7.69(3.68);7.686(3.6);7.672(4.45);7.668(6.02);7.652 (2.52);7.649(1.86);7.637(2.78);7.633(2.39);7.617(3.26);7.614(2.89);7.608(0.64);7.6(1.77);7.5 96(1.57);5.756(2.37);2.677(0.33);2.672(0.46);2.668(0.36);2.525(1.64);2.512(25.94);2.507(51. 37);2.503(67.92);2.498(50.69);2.494(26.35);2.33(0.44);2.325(0.32);0(1.62) |
| I-182 | 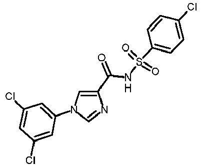 | Beispiel I-182: 1H-NMR(400.0 MHz, d6-DMSO): δ= 8.633(6.82);8.63(7.89);8.582(7.34);8.579(6.81);8.026(1.03);8.019(8.86);8.014(3.22);8.002(3. 13);7.997(10.81);47.991(1.64);7.959(15.23);7.955(16);7.74(1.32);7.733(10.48);7.728(3.52);7.7 17(2.92);7.712(9.25);7.705(1.41);7.694(3.78);7.69(6.78);7.686(3.65);2.527(0.93);2.513(13.69 );2.509(27.84);2.504(37.29);2.5(27.86);2.495(14.46);2.077(0.65);0(1.61) |
| I-183 | 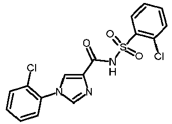 | Beispiel I-183: 1H-NMR(400.0 MHz, d6-DMSO): δ= 8.327(13.03);8.324(13.94);8.182(16);8.161(5.92);8.158(6.51);7.758(4.67);7.753(5.26);7.739( 4.95);7.735(6.58);7.728(2.21);7.724(1.98);7.708(4.71);7.705(4.58);7.691(5.84);7.687(6.24);7. 678(7.72);7.674(10.3);7.663(5);7.658(7.48);7.646(6.4);7.64(7.92);7.633(4.61);7.624(1.32);7. 616(5.66);7.599(3.79);7.596(4.6);7.583(5.75);7.578(5.55);7.568(6.91);7.565(8.33);7.563(8.17 |
| | | );7.559(5.77);7.55(5.38);7.545(4.87);7.531(1.7);7.527(1.4);2.672(0.33);2.507(40.13);2.503(5 3.01);2.498(41.87);2.33(0.35);2.075(0.65);0(0.63) |
| I-184 | 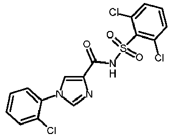 | Beispiel I-184: 1H-NMR(400.0 MHz, d6-DMSO): δ= 8.331(7.88);8.328(10.63);8.313(6.85);7.914(1.15);7.765(3.17);7.76(3.57);7.746(3.36);7.742(4 .36);7.707(0.41);7.704(0.44);7.686(2.93);7.681(2.78);7.668(4.26);7.661(7.36);7.656(7.21);7.6 38(16);7.627(1.32);7.625(1.3);7.612(1.35);7.606(3.44);7.596(7.24);7.588(4.03);7.579(5.26);7 .575(8.55);7.57(6.21);7.557(5.9);7.553(3.8);7.535(1.59);7.518(0.69);7.514(0.62);7.496(0.45); 7.394(0.4);7.391(0.47);7.373(0.97);7.328(0.45);2.672(0.33);2.524(1.07);2.511(19.56);2.507(3 8.86);2.502(50.98);2.498(37.89);2.329(0.33);2.075(4.52);1.356(2.06);0.008(0.37);0(9.13);-0.008(0.37) |
| I-185 | 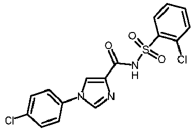 | Beispiel I-185: 1H-NMR(400.0 MHz, d6-DMSO): δ= 8.578(9.38);8.575(10.62);8.514(8.49);8.511(8.12);8.175(4.3);8.171(4.73);8.155(4.78);8.152(4 .96);7.775(0.99);7.768(10.51);7.762(3.89);7.751(4.19);7.745(15.86);7.738(2.12);7.719(1.28); 7.715(1.39);7.698(3.47);7.695(3.5);7.681(4.68);7.677(4.71);7.665(6.09);7.661(9.18);7.652(16 );7.646(6.56);7.641(3.26);7.635(4.08);7.63(14.4);7.611(4.39);7.607(3.97);7.593(2.28);7.589( 2.11);2.676(0.33);2.672(0.46);2.667(0.34);2.525(1.38);2.512(26.48);2.507(53.86);2.503(71.8 6);2.498(53.36);2.494(27.25);2.334(0.35);2.33(0.49);2.325(0.37);2.075(0.65);0(1.07) |
| I-186 | 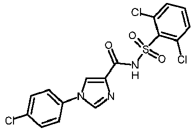 | Beispiel I-186: 1H-NMR(400.0 MHz, d6-DMSO): δ= 8.657(6.12);8.57(8.71);8.567(8.43);7.913(1.21);7.792(0.98);7.785(8.91);7.78(3.63);7.768(3.9 8);7.763(12.97);7.756(2.06);7.669(1.61);7.662(12.84);7.656(4.42);7.64(13.58);7.628(2.29);7. 619(16);7.606(2.38);7.575(6.48);7.558(4.36);7.553(3.58);7.536(3.11);7.518(0.63);7.514(0.56) ;7.496(0.37);2.676(0.44);2.671(0.62);2.667(0.46);2.511(35.34);2.507(68.53);2.502(89.62);2.4 98(67.81);2.494(36.4);2.334(0.42);2.329(0.59);2.325(0.44);0(1.08) |
| I-187 | 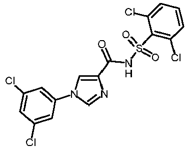 | Beispiel I-187: 1H-NMR(400.0 MHz, d6-DMSO): δ= 8.676(16);7.958(10.92);7.954(11.47);7.72(2.84);7.715(5);7.711(2.79);7.66(3.58);7.656(4.46); 7.638(10.52);7.606(0.38);7.598(4.08);7.581(2.65);7.575(2.09);7.558(1.41);2.508(28.54);2.50 3(37.83);2.499(29.08);2.076(4.51);0(3.96) |
| I-188 | 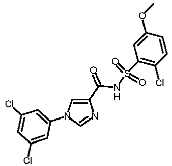 | Beispiel I-188: 1H-NMR(400.0 MHz, d6-DMSO): δ= 8.672(3.36);8.593(3.32);7.945(6.44);7.941(7.09);7.71(1.59);7.706(2.91);7.702(1.7);7.618(2.7 3);7.611(2.95);7.573(2.53);7.551(2.98);7.293(1.51);7.285(1.5);7.271(1.33);7.263(1.31);3.859 (16);2.507(30.03);2.502(39.85);2.498(31.03);0(3.42) |
| I-189 | 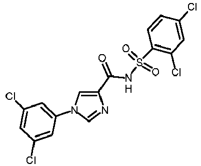 | Beispiel I-189: 1H-NMR(400.0 MHz, d6-DMSO): δ= 8.668(9.05);8.657(8.23);8.158(6.64);8.136(7.39);7.958(15.04);7.953(16);7.873(6.28);7.868(6. 88);7.723(4.43);7.718(7.66);7.714(7.69);7.71(4.16);7.701(3.89);7.696(3.71);4.191(0.99);3.85 9(0.35);2.672(0.4);2.508(43.86);2.504(57.67);2.499(43.28);2.33(0.37);2.076(1.97);0(0.88) |
| I-190 | 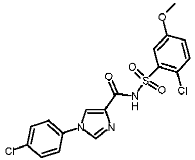 | Beispiel I-190: 1H-NMR(400.0 MHz, d6-DMSO): δ= 8.578(3.05);8.575(3.68);8.531(2.81);8.529(2.75);7.776(0.34);7.768(3.35);7.763(1.33);7.752(1 .38);7.746(5.08);7.739(0.72);7.662(0.58);7.655(4.97);7.65(1.6);7.638(1.18);7.633(3.58);7.62( 2.87);7.613(3.07);7.566(2.71);7.544(3.18);7.284(1.6);7.277(1.58);7.262(1.4);7.254(1.36);5.75 6(0.71);3.857(16);3.816(0.92);2.507(19.96);2.503(26.67);2.498(20.21);0(0.62) |
| I-191 | 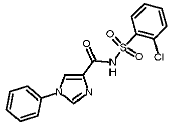 | Beispiel I-191: 1H-NMR(400.0 MHz, d6-DMSO): δ= 8.572(10.48);8.568(13.77);8.521(10.54);8.316(0.37);8.176(5.39);8.172(6.23);8.156(5.94);8.1 52(6.54);7.722(7.87);7.719(11.47);7.698(16);7.691(6.59);7.677(5.8);7.674(6.41);7.663(7.32); 7.659(10.4);7.643(3.73);7.64(3.12);7.628(4.43);7.624(4.18);7.608(6.11);7.606(6.08);7.604(5. 8);7.591(4.19);7.586(9.23);7.567(12.68);7.546(8.13);7.471(4.98);7.452(7.16);7.434(2.66);2.6 76(0.79);2.671(1.1);2.667(0.85);2.524(3.28);2.511(58.67);2.507(119.1);2.502(159.53);2.498( 122.19);2.333(0.75);2.329(1.04);2.325(0.82);2.075(1.49);0.008(0.83);0(23.52);-0.008(1.22) |
| I-192 | 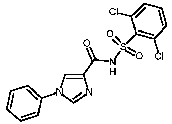 | Beispiel I-192: 1H-NMR(400.0 MHz, d6-DMSO): δ= 8.704(6.2);8.566(9.7);7.915(0.64);7.743(7.58);7.724(10.03);7.634(6.72);7.631(6.77);7.613(16 );7.594(5.1);7.575(9.73);7.567(7.31);7.555(6.36);7.55(5.39);7.544(3.81);7.527(2.36);7.519(0. 64);7.487(3.6);7.469(4.97);7.451(1.91);7.374(0.38);5.757(0.64);2.672(0.4);2.507(52.27);2.50 3(59.73);2.33(0.37);2.076(0.39);0(6.95) |
| I-193 | 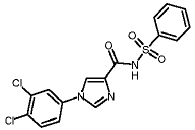 | Beipiel I-193: 1H-NMR(400.0 MHz, d6-DMSO): δ= 8.577(13.95);8.574(14.56);8.515(14.63);8.512(13.05);8.15(12.13);8.143(12.56);8.02(11.72);8 .002(14.21);7.998(10.26);7.844(8.77);7.822(16);7.81(0.62);7.805(0.59);7.773(8.5);7.767(8.06 );7.751(4.81);7.745(4.81);7.729(2.3);7.716(1.7);7.71(7.45);7.705(2.32);7.695(3.7);7.692(5.77 );7.651(10.32);7.632(14.57);7.617(2.49);7.614(5.68);2.676(0.47);2.672(0.66);2.667(0.48);2.5 25(1.86);2.511(41.9);2.507(82.68);2.503(108.26);2.498(80.04);2.494(40.51);2.443(0.45);2.33 4(0.6);2.33(0.8);2.325(0.62);1.76(0.34);0.008(0.69);0(19.18);-0.008(0.76) |
| I-194 | 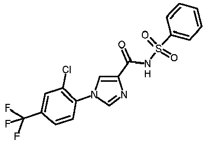 | Beispiel I-194: 1H-NMR(400.0 MHz, d6-DMSO): δ= 8.309(15.18);8.306(15.55);8.229(10.28);8.226(10.44);8.185(15.34);8.182(14.53);8.027(13.54 );8.008(15.73);8.004(11.56);7.962(4.35);7.958(4.39);7.941(7.01);7.937(6.93);7.877(10.44);7. 856(6.61); 7. 734(2.47);7.722(1.95);7.716(8.13);7 .698(6.24);7 .657(11.48);7 .637(16);7 .619(6.1 5);7.586(0.35);2.675(0.82);2.671(1.07);2.667(0.8);2.506(110.67);2.502(142.26);2.498(104.54 );2.333(0.68);2.329(0.93);2.324(0.67);2.074(1.01);1.356(0.33);0.008(1.1);0(25.01);-0.008(0.93) |
| I-195 | 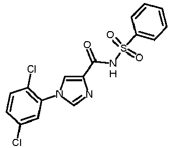 | Beispiel I-195: 1H-NMR(400.0 MHz, d6-DMSO): δ= 8.267(10.14);8.264(10.68);8.142(9.76);8.139(9.51);8.025(8.34);8.011(2.37);8.006(9.94);8.00 3(7.22);7.891(0.35);7.86(8.71);7.854(9.35);7.841(0.6);7.837(0.59);7.822(0.61);7.817(0.64);7. 776(7.38);7.754(10.99);7.734(1.59);7.721(1.19);7.716(5.25);7.71(1.69);7.697(4.1);7.67(6.3); 7.664(6.23);7.656(7.71);7.649(5.03);7.642(6.47);7.64(6.44);7.636(10.59);7.618(4.09);7.601(0 .48);7.591(0.45);7.586(1.06);7.566(0.67);7.349(0.85);6.871(0.68);6.64(0.34);5.756(16);3.618 (0.97);3.611(0.69);3.607(0.94);3.601(2.39);3.595(1.04);3.591(0.75);3.585(1.06);2.675(0.33); 2.671(0.47);2.666(0.35);2.524(0.99);2.511(28.25);2.506(57.68);2.502(77.4);2.497(58.15);2.4 93(29.82);2.444(0.38);2.333(0.44);2.329(0.58);2.324(0.45);2.184(1.11);1.776(0.97);1.768(1.0 8);1.759(2.82);1.751(1.13);1.743(1);1.356(7.58);1.233(0.4);1.099(0.37);0.008(0.67);0(22.9);-0.008(0.97) |
| I-196 | 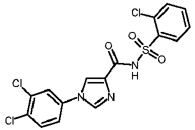 | Beispiel I-196: 1H-NMR(400.0 MHz, d6-DMSO): δ= 8.633(14.18);8.63(15.09);8.557(13.95);8.554(13.1);8.176(6.35);8.172(6.75);8.156(7.39);8.15 2(7.99);8.147(13.44);8.141(13.34);7.859(9.95);7.837(16);7.773(8.56);7.766(8.07);7.751(5.4); 7.744(5.34);7.724(1.87);7.72(1.95);7.704(5.32);7.7(5.03);7.686(6.69);7.682(6.56);7.669(8.51) ;7.666(11.37);7.649(4.66);7.646(3.41);7.634(5.31);7.63(4.4);7.614(6.47);7.603(1.09);7.597(3. 3);7.593(2.95);2.676(0.63);2.672(0.84);2.667(0.62);2.524(2.89);2.511(47.9);2.507(92.21);2.5 02(120.17);2.498(88.12);2.334(0.58);2.329(0.79);2.325(0.58);2.075(0.76);0.008(0.95);0(22.9) ;-0.008(0.82) |
| I-197 | 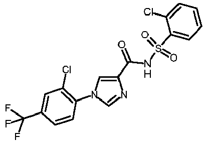 | Beispiel I-197: 1H-NMR(400.0 MHz, d6-DMSO): δ= 8.396(15.86);8.393(15.86);8.241(10.54);8.238(10.67);8.231(16);8.228(14.98);8.182(6.77);8.1 79(7.03);8.163(7.42);8.159(7.31);7.994(0.47);7.99(0.51);7.975(4.19);7.971(4.04);7.954(7.34) ;7.95(7.09);7.907(10.81);7.887(5.97);7.734(1.91);7.73(1.96);7.714(5.65);7.71(5.12);7.697(7.2 5);7.693(7.01);7.683(9.3);7.679(11.87);7.663(4.59);7.659(3.03);7.652(0.56);7.641(5.17);7.63 7(4.67);7.621(6.76);7.604(4.14);7.6(3.24);2.676(0.48);2.671(0.64);2.667(0.47);2.524(2.05);2. 511(38.6);2.507(74.25);2.502(95.95);2.498(69.86);2.494(34.35);2.334(0.48);2.329(0.63);2.32 4(0.47);1.356(0.53);1.291(0.33);0.008(0.67);0(18.14);-0.008(0.63) |
| I-198 | 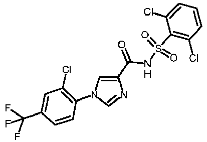 | Beispiel I-198: 1H-NMR(400.0 MHz, d6-DMSO): δ= 8.395(8.44);8.393(9.15);8.308(7.84);8.306(7.77);8.247(5.99);8.245(6.09);7.982(2.18);7.978(2 .14);7.961(4.42);7.957(4.44);7.924(6.62);7.904(3.25);7.671(5.56);7.667(6.67);7.649(16);7.62 4(0.33);7.609(6.46);7.592(4.17);7.586(3.2);7.569(2.19);2.675(0.44);2.671(0.57);2.666(0.44); 2.506(66.98);2.502(86.76);2.498(65.66);2.471(0.49);2.467(0.84);2.462(0.86);2.333(0.42);2.3 29(0.57);2.324(0.44);2.074(1.29);0.007(1.84);0(37.47);-0.036(0.34) |
| I-199 | 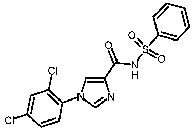 | Beispiel I-199: 1H-NMR(400.0 MHz, d6-DMSO): δ= 8.234(8.4);8.232(7.95);8.112(8.2);8.109(7.29);8.022(7.03);8.004(8.33);8(6.01);7.957(5.68);7. 952(5.82);7.731(1.35);7.718(1.19);7.713(4.3);7.708(1.49);7.694(3.35);7.676(1.83);7.654(16); 7.651(11.67);7.646(8.07);7.634(8.81);7.624(2.15);7.619(1.88);7.616(3.45);2.675(0.48);2.671( 0.64);2.666(0.45);2.51(44.44);2.506(79.95);2.502(100.66);2.497(74.08);2.333(0.55);2.328(0. 72);2.324(0.53);2.074(1.08);0.008(2.22);0(37.07);-0.008(1.81) |
| I-200 | 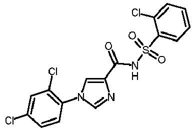 | Beispiel 1-200: 1H-NMR(400.0 MHz, d6-DMSO): δ= 8.316(10.38);8.314(10.17);8.175(4.83);8.172(5.07);8.164(9.92);8.162(9.37);8.156(5.48);8.15 2(5.02);7.97(7.59);7.965(7.55);7.728(1.24);7.724(1.28);7.708(8.17);7.686(16);7.676(6.84);7. 672(8.1);7.665(7.56);7.66(7.44);7.644(2.88);7.638(3.55);7.635(3.85);7.631(3.06);7.615(4.49) ;7.598(2.19);7.594(1.9);2.671(0.59);2.506(70.98);2.502(88.74);2.498(65.85);2.329(0.59);2.07 4(2);0(34.72);-0.008(1.53) |
| I-201 | 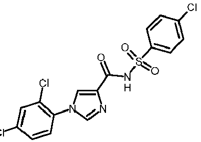 | Beispiel I-201: 1H-NMR(400.0 MHz, d6-DMSO): δ= 8.249(2.83);8.246(2.85);8.144(2.61);8.02(3.04);7.999(3.62);7.959(2.05);7.955(1.9);7.838(1.6 4);7.816(2.1);7.733(3.49);7.711(3.01);7.682(0.7);7.667(2.53);7.661(4.01);7.655(2.75);7.65(2. 83);7.646(2.1);7.634(0.45);7.628(0.47);7.464(2.15);2.502(20.99);2.075(16);1.356(1.59);0(5.4 9) |
| I-202 | 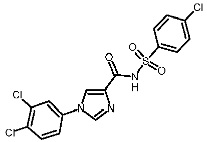 | Beispiel I-202: 1H-NMR(400.0 MHz, d6-DMSO): δ= 8.582(14.08);8.544(12.79);8.542(12.78);8.157(9.79);8.151(10.94);8.015(13.36);7.994(16);7.8 48(6.24);7.826(11.62);7.805(0.54);7.78(6.82);7.774(7.13);7.758(3.74);7.752(4.08);7.729(15.2 7);7.708(13.57);2.672(0.9);2.503(139.2);2.33(0.97);2.075(0.51);0(29.77) |
| I-203 | 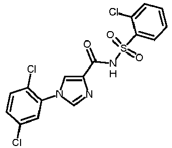 | Beispiel I-203: 1H-NMR(400.0 MHz, d6-DMSO): δ= 8.348(14.01);8.346(14.11);8.212(0.47);8.209(0.52);8.192(12.71);8.19(12.42);8.177(6.14);8.1 74(6.21);8.158(6.41);8.154(6.35);8.071(0.33);8.068(0.32);7.993(2.43);7.99(2.43);7.974(2.75) ;7.97(2.67);7.894(11.26);7.888(11.71);7.872(0.45);7.79(9.29);7.778(0.75);7.768(13.48);7.757 (0.66);7.731(1.7);7.727(1.75);7.711(5.14);7.708(4.72);7.694(6.51);7.69(6.47);7.68(16);7.676( 13.73);7.659(8.58);7.652(7.18);7.636(7.2);7.619(7.38);7.602(10.69);7.587(1.79);7.583(1.53); 7.573(0.37);7.54(1.92);7.536(1.82);7.521(2.54);7.519(2.36);7.503(1.28);7.499(1.18);6.871(0. 93);6.641(0.45);4.273(0.41);4.255(0.46);3.204(0.37);2.675(0.8);2.671(1.07);2.666(0.81);2.50 6(129.34);2.502(164.23);2.497(122.78);2.445(0.41);2.411(0.37);2.333(0.87);2.328(1.12);2.32 4(0.86);2.183(1.49);2.074(10.78);1.356(10.16);1.304(0.44);1.286(0.88);1.268(0.45);0.008(2.1 3);0(46.31);-0.008(2.09) |
| I-204 | 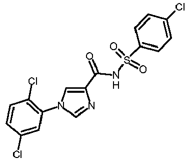 | Beispiel I-204: 1H-NMR(400.0 MHz, d6-DMSO): δ= 8.316(0.39);8.273(9.32);8.27(10.47);8.167(9.71);8.164(9.82);8.025(1.68);8.019(13.65);8.014( 4.7);8.002(4.81);7.997(15.98);7.991(2.03);7.866(9.59);7.86(10.05);7.78(8.62);7.758(12.65);7. 74(2.04);7.733(14.97);7.729(5.01);7.716(4.24);7.712(12.79);7.705(1.61);7.675(7.24);7.669(6. 72);7.653(4.79);7.647(4.6);2.68(0.41);2.675(0.79);2.671(1.08);2.666(0.79);2.524(3.45);2.519 (5.35);2.511(58.82);2.506(117.66);2.502(154.6);2.497(111.46);2.493(53.29);2.338(0.35);2.33 3(0.75);2.329(1.05);2.324(0.76);2.32(0.36);2.074(16);0.146(0.68);0.008(6);0(158.33);-0.009(5.16);-0.15(0.67) |
| I-205 | 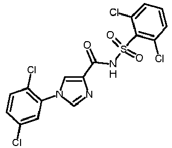 | Beispiel I-205: 1H-NMR(400.0 MHz, d6-DMSO): δ= 8.35(8.63);8.348(8.9);8.288(7.54);7.917(7.5);7.911(7.97);7.796(5.98);7.774(8.76);7.704(0.74) ;7.689(4.99);7.683(4.74);7.667(8.01);7.662(9.23);7.644(16);7.626(0.57);7.603(6.24);7.586(3. 99);7.58(3.07);7.564(2.1);7.394(0.51);7.39(0.57);7.372(1.4);7.328(0.55);7.311(0.37);5.756(0. 99);2.671(0.5);2.506(66.56);2.502(84.39);2.498(63.11);2.332(0.47);2.328(0.6);1.045(1.18);1. 03(1.15);0(2.52) |
| I-206 | 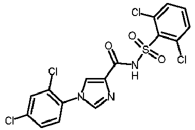 | Beispiel I-206: 1H-NMR(400.0 MHz, d6-DMSO): δ= 8.31(8.61);8.265(7.53);7.979(6.84);7.974(7.16);7.911(0.46);7.728(4.62);7.706(9.76);7.674(6. 18);7.669(6.31);7.659(6.36);7.654(8.69);7.648(4.52);7.637(16);7.606(1.03);7.595(5.79);7.578 (3.97);7.573(3.36);7.556(2.11);2.671(0.93);2.505(159.4);2.502(205.79);2.498(168.82);2.328( 1.93);2.257(0.44);2.25(0.43);2.074(1.6);0.146(0.45);0(125.22);-0.15(0.86) |
| I-207 | 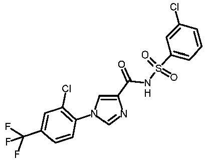 | Beispiel I-207: 1H-NMR(400.0 MHz, d6-DMSO): δ= 8.341(11.96);8.24(15.09);8.015(8.46);7.978(5.26);7.97(4.28);7.959(6.31);7.949(5.94);7.889(7 .67);7.868(4.73);7.842(1.27);7.823(3.61);7.803(4.9);7.801(4.95);7.771(0.85);7.706(5.76);7.68 7(8.68);7.667(3.41);7.638(0.9);7.619(1.11);7.599(0.47);7.545(0.44);7.515(2);7.474(0.33);7.4 16(0.39);3.803(0.49);3.788(1.21);3.773(1.61);3.758(1.23);3.743(0.51);2.672(0.54);2.503(95.9 4);2.329(0.76);1.356(0.97);1.046(16);1.03(15.94);0(2.05) |
| I-208 | 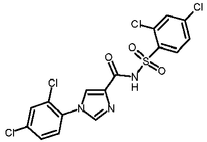 | Beispiel I-208: 1H-NMR(400.0 MHz, d6-DMSO): δ= 8.316(12.75);8.313(13);8.247(10.06);8.244(8.91);8.157(10.55);8.135(11.64);7.974(10.02);7.9 68(9.82);7.874(10.23);7.869(10.42);7.721(8.52);7.719(8.28);7.716(7.33);7.697(16);7.682(0.3 5);7.671(8.72);7.665(7.85);7.649(3.56);7.644(3.63);2.672(0.38);2.525(1.35);2.512(22.53);2.5 07(44.42);2.503(58.04);2.498(41.88);2.494(20.12);2.33(0.38);2.075(8.3);0(8.62);-0.008(0.32) |
| I-209 | 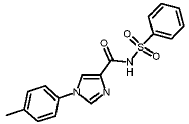 | Beispiel I-209: 1H-NMR(400.0 MHz, d6-DMSO): δ= 8.456(5.39);8.453(5.99);8.4(4.86);8.397(4.48);8.02(4.59);8.007(1.32);8.002(5.65);7.998(3.9); 7.724(0.86);7.72(0.57);7.711(0.64);7.705(2.84);7.7(0.88);7.69(1.36);7.687(2.25);7.684(1.24); 7.647(4.04);7.628(5.62);7.613(0.91);7.61(2.15);7.585(5.54);7.564(6.67);7.354(5.29);7.333(4. 32);5.756(1.14);2.511(12.45);2.507(25.69);2.502(34.34);2.498(25.82);2.494(13.34);2.351(16) ;2.329(0.56);2.325(0.44);0(4.92) |
| I-210 | 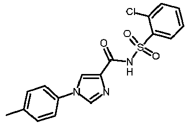 | Beispiel I-210: 1H-NMR(400.0 MHz, d6-DMSO): δ= 8.517(4.66);8.514(5.23);8.481(3.93);8.169(2.37);8.166(2.51);8.15(2.58);8.146(2.59);7.705(0. 64);7.701(0.68);7.685(1.86);7.681(1.77);7.668(2.54);7.664(2.48);7.653(3.14);7.649(4.16);7.6 33(1.67);7.629(1.19);7.619(1.98);7.615(1.61);7.596(7.16);7.574(6.81);7.37(5.18);7.349(4.32) ;5.754(1.62);2.512(17.73);2.507(36.66);2.502(49.17);2.498(36.97);2.494(19.2);2.36(16);2.33 9(0.53);2.334(0.54);2.329(0.59);2.325(0.49);2.074(12.98);0(6.73);-0.008(0.35) |
| I-211 | 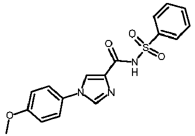 | Beispiel I-211: 1H-NMR(400.0 MHz, d6-DMSO): δ= 8.399(3.17);8.396(3.63);8.341(2.73);8.021(2.9);8.003(3.3);8(2.4);7.722(0.51);7.709(0.39);7.7 03(1.72);7.698(0.54);7.685(1.34);7.646(2.51);7.626(3.66);7.617(3.96);7.611(1.83);7.609(1.88 );7.599(1.37);7.594(4.22);7.586(0.46);7.104(0.42);7.095(4.06);7.09(1.31);7.078(1.24);7.072( 3.78);7.064(0.34);5.757(0.58);3.804(16);2.508(10.01);2.503(13.02);2.499(9.68);0(1.71) |
| I-212 | 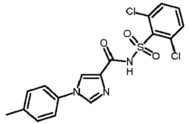 | Beispiel I-212: 1H-NMR(400.0 MHz, d6-DMSO): δ= 8.687(2.42);8.514(6.04);8.511(5.77);7.912(0.4);7.623(9.23);7.619(6.8);7.601(16);7.554(4.21) ;7.537(2.89);7.532(2.23);7.515(1.67);7.38(5.05);7.359(4.18);5.756(2.06);3.601(0.33);2.676(0. 34);2.671(0.46);2.666(0.33);2.524(1.42);2.52(2.23);2.511(25.9);2.506(51.98);2.502(68.02);2. 497(48.13);2.493(22.48);2.364(15.53);2.333(0.39);2.329(0.48);2.324(0.34);2.074(0.38);1.76( 0.4);0(7.56) |
| I-213 | 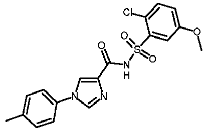 | Beispiel I-213: 1H-NMR(400.0 MHz, d6-DMSO): δ= 8.526(2.41);8.523(3.83);8.513(1.93);7.619(2.86);7.612(3.04);7.606(0.51);7.599(3.03);7.582(1 .17);7.578(3.75);7.557(2.65);7.535(3.1);7.373(2.87);7.353(2.36);7.274(1.56);7.266(1.49);7.25 2(1.35);7.244(1.31);5.756(0.39);3.854(16);3.816(0.43);2.524(0.57);2.52(0.93);2.511(10.85);2 .507(21.97);2.502(29);2.498(20.87);2.493(10);2.362(8.74);0(3.15) |
| I-214 | 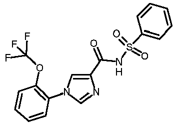 | Beispiel I-214: 1H-NMR(400.0 MHz, d6-DMSO): δ= 8.288(13.78);8.285(14.53);8.2(0.35);8.174(13.16);8.171(12.73);8.065(0.37);8.061(0.41);8.03 9(1.29);8.031(10.46);8.027(5.52);8.018(2.98);8.013(13.22);8.009(8.96);7.94(0.42);7.937(0.41 );7.844(0.73);7.84(0.75);7.824(0.8);7.82(0.87);7.751(0.36);7.737(7.03);7.735(5.18);7.719(11. 43);7.704(3.49);7.7(5.6);7.697(3.3);7.689(1.22);7.686(1.15);7.672(9.84);7.665(6.34);7.659(1 6);7.656(9.48);7.643(7.21);7.64(14.57);7.624(6.87);7.622(7.03);7.618(5.17);7.609(2.9);7.605 (4.29);7.602(3.28);7.599(3.09);7.593(1.01);7.588(3.06);7.582(2.55);7.577(0.42);7.572(0.46); 7.568(0.9);7.354(0.96);6.872(0.82);3.349(0.34);3.332(0.43);2.672(0.34);2.525(0.85);2.512(22 .76);2.508(47);2.503(63.01);2.498(46.53);2.494(23.4);2.444(0.49);2.432(0.36);2.416(0.42);2. 334(0.37);2.33(0.49);2.325(0.37);2.185(1.39);2.076(9.81);1.989(0.5);1.654(0.4);1.357(10.01) ;1.291(0.47);1.287(0.34);1.27(0.64);1.252(0.33);0(6.58) |
| I-215 | 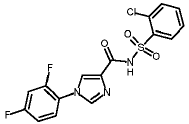 | Beispiel I-215: 1H-NMR(400.0 MHz, d6-DMSO): δ= 8.386(1.59);8.382(2.76);8.379(1.68);8.32(0.39);8.268(0.71);8.265(0.76);8.236(2.56);8.192(0. 34);8.176(1.39);8.172(1.66);8.165(0.61);8.156(1.7);8.152(1.75);8.146(0.44);7.823(0.56);7.80 8(0.61);7.801(1.16);7.786(1.15);7.778(0.65);7.764(0.6);7.728(0.4);7.724(0.45);7.708(1.14);7. 704(1.18);7.69(1.69);7.686(1.59);7.68(1);7.674(2.38);7.67(2.98);7.662(0.65);7.657(1.3);7.65 4(1.44);7.651(1.71);7.645(0.89);7.636(1.26);7.631(1.35);7.623(0.88);7.616(1.39);7.614(1.33) ;7.612(1.38);7.607(0.55);7.598(0.83);7.594(0.78);7.59(0.46);7.353(0.41);7.349(0.46);7.346(0. 42);7.343(0.4);7.329(0.73);7.327(0.75);7.323(0.72);7.31(0.39);7.307(0.41);7.303(0.37);7.3(0. 34);6.944(0.35);4.148(0.71);4.13(0.75);4.119(0.51);4.101(0.48);2.524(0.81);2.511(13.18);2.5 07(26.29);2.502(34.61);2.498(25.14);2.493(12.22);2.075(16);1.363(0.49);1.345(1.01);1.328(0 .47);1.298(0.74);1.28(1.63);1.263(0.8);0(2.7) |
| I-216 | 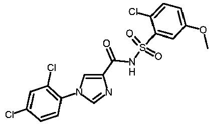 | Beispiel I-216: 1H-NMR(400.0 MHz, d6-DMSO): δ= 8.309(1.95);8.172(2.3);8.17(2.36);7.973(2.64);7.968(2.64);7.711(1.55);7.69(3.93);7.668(2.41) ;7.662(2.24);7.646(0.98);7.641(0.96);7.617(2.81);7.609(2.94);7.575(2.17);7.553(2.55);7.292( 1.26);7.284(1.22);7.27(1.11);7.262(1.08);3.856(16);2.675(0.6);2.67(0.84);2.666(0.6);2.524(2. 18);2.519(3.55);2.51(48.99);2.506(99.26);2.501(131.21);2.497(94.91);2.492(46.04);2.333(0.6 9);2.328(0.93);2.324(0.69);2.074(0.79);0.008(0.41);0(14.4);-0.008(0.49) |
| I-217 | 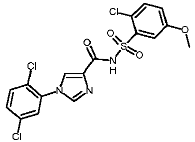 | Beispiel I-217: 1H-NMR(400.0 MHz, d6-DMSO): δ= 8.348(3.66);8.2(3.48);7.899(2.91);7.893(3.08);7.791(2.35);7.769(3.38);7.682(1.98);7.676(1.9 2);7.66(1.38);7.654(1.33);7.62(2.91);7.612(3.16);7.582(2.6);7.56(3.01);7.298(1.63);7.29(1.61 );7.276(1.44);7.268(1.39);3.858(16);3.816(1.17);2.502(54.62);2.328(0.56);0(2.28) |
| I-218 | 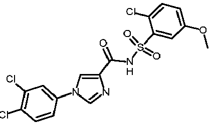 | Beispiel I-218: 1H-NMR(400.0 MHz, d6-DMSO): δ= 8.632(2.86);8.629(3.3);8.567(3.06);8.564(3.02);8.148(2.97);8.141(3.03);7.862(2.28);7.84(3.6) ;7.772(1.89);7.766(1.78);7.751(1.19);7.744(1.19);7.618(2.76);7.611(2.89);7.57(2.63);7.548(3. 09);7.29(1.54);7.282(1.49);7.268(1.33);7.26(1.28);3.857(16);2.524(0.49);2.511(15.53);2.506( 32.06);2.502(43.06);2.498(32.35);2.493(16.76);2.329(0.32);2.074(1.31);0.008(0.87);0(31.62); -0.008(1.56) |
| I-219 | 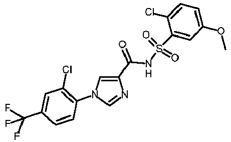 | Beispiel I-219: 1H-NMR(400.0 MHz, d6-DMSO): δ= 8.395(2.73);8.392(2.91);8.238(4.87);8.235(3.68);7.976(0.75);7.972(0.75);7.955(1.46);7.951(1 .48);7.91(2.21);7.889(1.21);7.624(2.75);7.616(2.91);7.584(2.59);7.562(3.05);7.301(1.51);7.29 3(1.47);7.279(1.32);7.271(1.29);3.86(16);2.524(0.85);2.511(16.7);2.506(33.21);2.502(43.53); 2.497(31.86);2.493(15.86);2.074(0.56);0.008(1.33);0(34.46);-0.008(1.34) |
| I-220 | 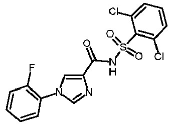 | Beispiel I-220: 1H-NMR(400.0 MHz, d6-DMSO): δ= 8.421(4.27);8.418(7.63);8.414(4.79);8.372(6.3);7.912(0.4);7.75(1.79);7.73(3.74);7.711(2);7.6 63(5.7);7.658(6.89);7.641(16);7.628(0.52);7.625(0.53);7.606(1.07);7.6(6.81);7.583(4.47);7.5 77(3.85);7.572(2.13);7.567(3.84);7.558(6.72);7.541(4.7);7.536(4.48);7.52(0.92);7.514(0.43); 7.429(2.1);7.424(1.23);7.419(1.25);7.414(1.88);7.408(2.66);7.395(1.56);7.387(1.46);5.756(0. 54);2.675(0.35);2.671(0.54);2.666(0.36);2.524(0.9);2.511(40.33);2.506(83.33);2.502(111.5);2 .497(82.29);2.493(41.49);2.333(0.7);2.329(0.91);2.324(0.72);0.146(0.33);0.008(2.45);0(86.53 );-0.008(4);-0.15(0.45) |
| I-221 | 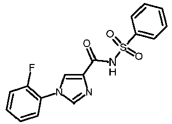 | Beispiel I-221: 1H-NMR(400.0 MHz, d6-DMSO): δ= 9.089(0.41);8.381(0.37);8.377(0.38);8.34(14.51);8.232(9.28);8.229(13.03);8.027(13.14);8.02 6(13.17);8.008(16);8.004(10.82);7.844(2.38);7.84(2.52);7.825(2.69);7.82(2.67);7.734(2.71);7 .716(11.29);7.698(13.01);7.677(4.07);7.657(11.51);7.637(15.42);7.619(6.25);7.606(1.13);7.6 04(1.17);7.593(1.97);7.588(4.69);7.577(1.19);7.569(3.05);7.549(7.52);7.538(11.37);7.522(8.2 1);7.518(7.85);7.502(1.31);7.423(0.47);7.412(3.81);7.407(2.38);7.402(2.52);7.397(3.52);7.39 1(5.03);7.379(3.11);7.37(2.87);7.356(3.91);6.874(0.34);4.493(0.7);4.476(0.72);3.789(0.32);3. 739(0.35);3.696(0.35);3.677(0.36);3.654(0.36);3.637(0.36);3.618(0.36);3.595(0.37);3.588(0.3 |
| | | 7);3.547(0.37);3.542(0.38);3.527(0.37);3.509(0.38);3.5(0.38);3.473(0.37);3.468(0.37);3.458( 0.37);3.44(0.36);3.42(0.36);3.414(0.35);3.373(0.34);3.359(0.34);3.352(0.33);2.672(0.56);2.66 8(0.43);2.508(57.56);2.503(71.18);2.499(51.06);2.33(0.46);2.326(0.34);2.185(0.6);2.076(1.4) ;1.39(0.67);1.373(1.39);1.357(4.24);1.269(0.33);1.236(0.4);0.008(0.64);0(12.13);-0.008(0.52) |
| I-222 | 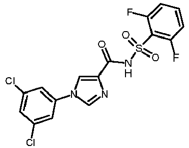 | Beispiel 1-222: 1H-NMR(400.0 MHz, d6-DMSO): δ= 8.712(7.97);8.669(9.03);7.97(16);7.967(15.07);7.776(0.77);7.76(1.75);7.754(1.89);7.74(3.24) ;7.719(8.34);7.705(1.63);7.314(4.74);7.291(7.71);7.268(4.62);5.756(0.48);2.502(84.36);2.329 (1.04) ;2.07 4(5. 26) ;2.061(0. 71) ;0.006(0.82) ;-0.002(25.97) ;-0.014(2.86) |
| I-223 | 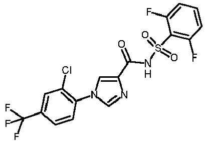 | Beispiel I-223: 1H-NMR(400.0 MHz, d6-DMSO): δ= 8.384(14.26);8.381(16);8.339(12.82);8.336(11.76);8.316(0.36);8.252(9.08);8.248(9.12);7.986 (3.53);7.982(3.5);7.965(6.74);7.961(6.58);7.925(10.18);7.904(5.2);7.792(1.11);7.777(2.51);7. 771(2.37);7.762(1.77);7.756(4.48);7.75(1.79);7.741(2.45);7.734(2.82);7.72(1.15);7.329(7.31) ;7.307(11.1);7.284(6.59);5.756(2.48);2.671(0.39);2.511(45.8);2.506(105.16);2.502(147.77);2. 497(113.37);2.493(60.57);2.333(1.25);2.329(1.56);2.324(1.32);2.075(2.04);1.018(0.44);0.008 (0.39);0(62.45);-0.009(3.75);-0.15(0.38) |
| I-224 | 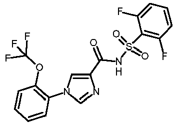 | Beispiel I-224: 1H-NMR(400.0 MHz, d6-DMSO): δ= 8.356(16);8.338(13.37);8.316(0.75);7.984(0.65);7.782(8.12);7.764(10.77);7.762(10.49);7.748 (5.34);7.733(3.11);7.727(3.28);7.711(2.3);7.692(14.09);7.68(10.35);7.659(2.21);7.655(2.58); 7.642(5.59);7.634(4.02);7.627(3.57);7.622(5.07);7.616(3.75);7.607(2.59);7.6(2.26);7.322(8.2 9);7.299(13.07);7.276(7.51);7.242(0.39);5.756(1.63);4.492(0.32);4.458(0.33);4.427(0.35);4.4 14(0.34);4.383(0.36);4.363(0.36);4.314(0.36);4.296(0.38);4.278(0.46);4.268(0.41);4.258(0.44 );4.242(0.4);4.2(0.37);4.164(0.42);4.147(0.56);4.13(0.56);4.113(0.4);4.062(0.37);4.038(0.35); 4.002(0.35);3.991(0.34);3.96(0.34);3.919(0.33);2.675(1.43);2.671(1.97);2.666(1.51);2.506(23 5.6);2.502(301.64);2.497(231.43);2.333(1.73);2.328(2.24);2.324(1.77);2.074(0.57);1.288(0.6 1);0.146(0,4);0(94.66);-0.15(0.47) |
| I-225 | 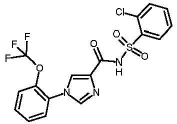 | Beispiel I-225: 1H-NMR(400.0 MHz, d6-DMSO): δ= 8.378(8.93);8.207(11.65);8.178(5.89);8.174(6.96);8.158(6.44);8.154(7.32);7.768(5.75);7.752( 7.39);7.748(7.91);7.727(1.41);7.724(1.67);7.707(5.43);7.704(5.89);7.685(16);7.682(14.99);7. 677(12.9);7.672(12.98);7.669(11.59);7.665(8.45);7.656(4.49);7.652(4.05);7.648(4.11);7.644( 3.17);7.635(9.5);7.629(5.9);7.616(10.86);7.6(5.37);7.595(4.02);3.508(0.34);2.675(1.71);2.671 (2.29);2.666(1.68);2.524(8.87);2.51(128.94);2.506(252.17);2.502(328.38);2.497(239.45);2.49 3(118.33);2.333(1.64);2.328(2.2);2.324(1.64);1.234(0.67);0.146(0.33);0.008(3.13);0(74.11);-0.008(2.56) |
| I-226 | 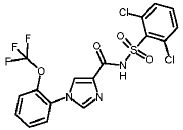 | Beispiel I-226: 1H-NMR(400.0 MHz, d6-DMSO): δ= 8.387(7.82);8.384(8.86);8.312(6.65);8.309(6.72);7.787(3.54);7.77(4.21);7.766(4.21);7.708(0. 69);7.705(0.68);7.691(6.83);7.678(4.92);7.674(4.32);7.669(6.32);7.665(7.46);7.657(1.93);7.6 53(2.17);7.647(16);7.642(4.66);7.635(2.45);7.626(2.19);7.622(2.92);7.619(2.22);7.616(2.25); 7.605(7.64);7.6(1.92);7.588(4.5);7.582(3.51);7.565(2.49);2.676(0.33);2.671(0.46);2.667(0.33) ;2.524(1.57);2.511(26.22);2.507(51.57);2.502(67.06);2.498(48.25);2.493(23.3);2.329(0.44);2. 076(2.89);1.291(0.41);0.008(0.74);0(18.37);-0.008(0.6) |
| I-227 | 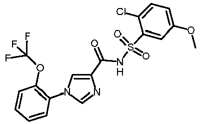 | Beispiel I-227: 1H-NMR(400.0 MHz, d6-DMSO): δ= 8.392(2.91);8.389(3.03);8.228(2.8);8.225(2.76);7.774(1.19);7.756(1.53);7.752(1.57);7.684(2. 52);7.671(1.64);7.667(1.37);7.651(0.6);7.647(0.48);7.638(1.24);7.631(0.93);7.622(3.48);7.61 8(1.63);7.615(3.92);7.602(0.65);7.596(0.52);7.583(2.63);7.561(3.07);7.553(0.41);7.531(0.38) ;7.481(0.37);7.473(0.38);7.3(1.57);7.292(1.5);7.278(1.36);7.27(1.31);3.86(16);3.816(2.17);2. 524(0.63);2.511(13.78);2.506(27.6);2.502(36.26);2.497(26.35);2.493(12.98);0.008(0.37);0(11 .09);-0.008(0.41) |
| I-228 | 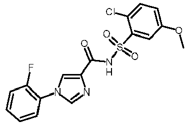 | Beispiel I-228: 1H-NMR(400.0 MHz, d6-DMSO): δ= 8.427(2.77);8.289(2.57);7.74(0.72);7.72(1.4);7.701(0.81);7.624(2.88);7.616(3.01);7.58(2.73); 7.563(1.96);7.558(3.69);7.553(2.43);7.537(1.3);7.532(2);7.426(0.77);7.42(0.48);7.413(0.68); 7.405(1.02);7.394(0.71);7.384(0.53);7.298(1.62);7.29(1.57);7.276(1.43);7.268(1.39);3.86(16) ;3.816(0.42);2.524(0.61);2.511(9.94);2.507(19.17);2.502(24.63);2.498(17.81);2.493(8.72);2.0 76(1.53);1.292(0.32);0(6.25) |
| I-229 | 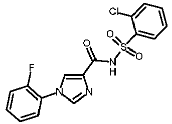 | Beispiel I-229: 1H-NMR(400.0 MHz, d6-DMSO): δ= 8.431(9.3);8.428(16);8.424(9.73);8.314(0.85);8.311(0.92);8.281(8.78);8.278(14.22);8.209(0.5 5);8.182(7.71);8.178(8.27);8.17(1.06);8.162(8.45);8.158(8.54);7.738(4.03);7.731(2.8);7.726( 3.13);7.718(8.04);7.71(7);7.707(6.67);7.699(5.27);7.693(9.21);7.689(8.64);7.678(10.75);7.67 4(14.3);7.666(1.23);7.658(5.63);7.654(3.79);7.639(6.33);7.635(5.35);7.62(7.35);7.616(6.06); 7.608(1.2);7.602(4.28);7.598(3.68);7.591(0.48);7.586(0.51);7.561(9.19);7.551(11.33);7.536(6 .67);7.53(10.5);7.516(1.48);7.496(0.45);7.492(0.54);7.476(0.46);7.473(0.7);7.452(0.41);7.434 (0.66);7.424(4.21);7.419(2.52);7.412(3.74);7.403(5.37);7.398(2.55);7.392(3.73);7.382(2.93); 7.37(0.34);7.283(0.47);7.264(0.37);7.094(0.43);7.091(0.41);4.139(0.89);4.122(0.89);2.676(0. 44);2.672(0.6);2.667(0.43);2.525(2.11);2.512(36.46);2.507(71.89);2.503(93.68);2.498(67.67); 2.494(32.89);2.334(0.47);2.33(0.62);2.325(0.46);2.076(5.31);1.31(0.94);1.292(2.02);1.275(0. 91);0.008(0.95);0(24.11);-0.008(0.86) |
| I-230 | 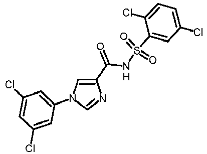 | Beispiel 1-230: 1H-NMR(400.0 MHz, d6-DMSO): δ= 8.756(5.68);8.754(6.05);8.675(7.61);8.672(7.24);8.092(6.52);8.086(6.76);7.971(15.48);7.967( 16);7.787(3.12);7.781(2.9);7.766(4.69);7.76(4.63);7.731(4.03);7.727(7.13);7.722(3.8);7.712( 0.43);7.706(0.49);7.702(0.66);7.694(8.37);7.672(5.35);5.758(4.01);2.672(0.39);2.525(1.64);2. 512(24.56);2.507(47.36);2.503(61.01);2.498(44.22);2.494(21.78);2.329(0.39);0.008(0.41);0(9 .27);-0.008(0.34) |
| I-231 | 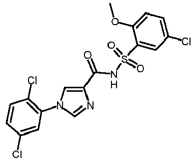 | Beispiel I-231: 1H-NMR(400.0 MHz, d6-DMSO): δ= 8.35(3.29);8.347(3.47);8.153(3.58);8.15(3.63);7.886(3.26);7.879(3.44);7.843(3.01);7.836(3.4 9);7.792(2.8);7.771(4.12);7.765(1.87);7.758(1.5);7.742(1.87);7.736(1.67);7.681(2.36);7.675( 2.24);7.659(1.6);7.653(1.59);7.306(2.89);7.284(2.63);3.904(0.32);3.875(16);2.524(1.05);2.51 (15);2.506(29.28);2.502(38.09);2.497(27.76);2.492(13.66);2.075(1.25);0(3) |
| I-232 | 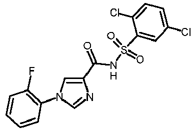 | Beispiel I-232: 1H-NMR(400.0 MHz, d6-DMSO): δ= 8.454(8.99);8.415(7.33);8.412(11.51);8.408(6.34);8.268(0.52);8.265(0.5);8.093(12.67);8.087( 12.79);8.073(0.38);8.066(0.4);7.79(6.23);7.783(5.65);7.768(9.27);7.762(9.21);7.757(2.89);7.7 54(2.94);7.737(5.19);7.735(5.97);7.717(3.14);7.714(3.44);7.707(1.13);7.697(16);7.676(10.06) ;7.652(0.52);7.631(0.34);7.599(0.45);7.595(0.44);7.584(1.09);7.578(2.98);7.574(3.62);7.57(5. 11);7.563(9.96);7.561(8.99);7.544(6.25);7.539(4.99);7.524(1.2);7.518(0.61);7.431(3.27);7.42 7(1.96);7.422(1.94);7.416(3.45);7.41(4.11);7.402(1.61);7.396(2.43);7.389(2.12);4.145(0.59); 4.127(0.59);2.68(0.37);2.675(0.91);2.671(1.29);2.666(0.9);2.662(0.35);2.524(3.94);2.519(6.3 2);2.511(76.16);2.506(154.6);2.502(204.65);2.497(147.55);2.493(70.92);2.338(0.53);2.333(1. 04);2.328(1.42);2.324(1.03);2.32(0.52);2.075(1.57);1.303(0.56);1.286(1.22);1.268(0.55);0.14 6(0.32);0.008(2.69);0(93.34);-0.008(3.48);-0.15(0.39) |
| I-233 | 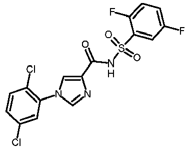 | Beispiel I-233: 1H-NMR(400.0 MHz, d6-DMSO): δ= 8.355(13.39);8.33(16);7.911(12.37);7.905(12.81);7.799(9.58);7.777(14.22);7.763(3.06);7.754 (3.82);7.75(4.02);7.742(5.25);7.736(4.13);7.731(3.67);7.722(3.04);7.696(8.47);7.69(8.19);7.6 75(5.94);7.668(6.68);7.659(2.8);7.645(3.31);7.637(4.56);7.627(3.38);7.617(3.19);7.609(1.68) ;7.546(3.13);7.536(3.41);7.522(5.09);7.512(4.98);7.499(2.38);7.489(2.05);2.671(1.16);2.502( 205.79);2.354(0.42);2.328(1.62);2.075(0.35);0(55.61);-0.15(0.32) |
| I-234 | 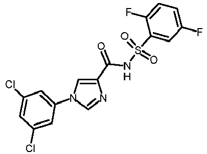 | Beispiel I-234: 1H-NMR(400.0 MHz, d6-DMSO): δ= 8.75(5.42);8.747(5.65);8.665(7.43);8.661(6.96);7.974(15.53);7.97(16);7.763(1.21);7.755(1.55 );7.75(1.55);7.742(2.12);7.736(1.73);7.729(4.83);7.724(7.73);7.72(4.19);7.661(0.64);7.652(1. 09);7.641(1.06);7.638(1.15);7.629(1.75);7.619(1.23);7.609(1.42);7.601(0.7);7.536(1.47);7.52 6(1.55);7.513(2.37);7.502(2.34);7.49(1.07);7.48(0.98);5.758(1.62);2.672(0.33);2.525(1.18);2. 512(21.03);2.507(41.82);2.503(54.82);2.498(39.76);2.494(19.36);2.33(0.37);0(1.01) |
| I-235 | 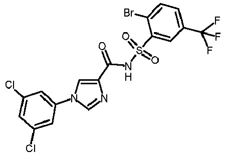 | Beispiel 1-235: 1H-NMR(400.0 MHz, d6-DMSO): δ= 8.88(5.25);8.878(4.89);8.687(7.57);8.684(6.68);8.374(4.38);8.368(4.13);8.076(3.24);8.055(4. 28);7.987(16);7.983(15.51);7.942(2.62);7.936(2.45);7.92(1.89);7.915(1.75);7.744(4.04);7.739 (6.78);7.735(3.23);2.672(0.36);2.526(1.29);2.512(21.62);2.508(42.52);2.503(55.32);2.499(39. 88);2.494(19.24);2.33(0.36);0(1.15) |
| I-236 | 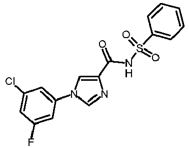 | Beispiel I-236: 1H-NMR(400.0 MHz, d6-DMSO): δ= 8.609(13.97);8.606(15.8);8.552(15.17);8.549(14.14);8.028(1.28);8.02(12.55);8.016(6.63);8.0 07(3.46);8.002(16);7.998(11);7.839(9.61);7.799(3.56);7.793(5.95);7.788(3.28);7.774(3.61);7. 768(5.96);7.763(3.24);7.734(1.25);7.731(2.4);7.728(1.62);7.718(1.74);7.712(7.68);7.707(2.48 );7.697(3.58);7.694(6.21);7.691(3.48);7.653(10.66);7.649(5.2);7.636(7.8);7.633(15.09);7.619 (2.46);7.615(5.86);7.613(4.04);7.548(3.46);7.544(5.31);7.539(3.24);7.527(3.64);7.522(5.42); 7.517(3.13);3.336(0.42);3.32(0.42);3.221(0.33);2.676(0.78);2.671(1.03);2.667(0.78);2.524(3. 78);2.511(57.22);2.507(112.01);2.502(145.53);2.498(104.69);2.493(49.93);2.333(0.67);2.329 (0.91);2.324(0.64);2.076(1.48);0(1.71) |
| I-237 | 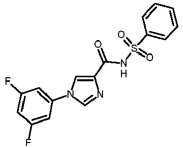 | Beispiel I-237: 1H-NMR(400.0 MHz, d6-DMSO): δ= 8.583(11.84);8.58(13.96);8.558(0.63);8.54(14.54);8.537(14.22);8.506(0.43);8.503(0.41);8.01 6(13.58);7.998(16);7.994(12.22);7.722(2.38);7.71(2.01);7.704(8.25);7.699(3.77);7.682(12.36) ;7.678(10.75);7.662(10.19);7.656(8.9);7.646(12.38);7.626(15.9);7.608(6.06);7.376(1.75);7.37 (2.89);7.365(1.7);7.352(3.67);7.347(5.77);7.342(3.2);7.329(1.97);7.324(2.96);7.318(1.54);5.7 58(0.58);4.132(0.46);4.115(0.45);2.676(0.8);2.672(1.03);2.667(0.79);2.511(57.79);2.507(109. 2);2.503(140.14);2.498(101.76);2.494(49.95);2.334(0.66);2.329(0.91);2.325(0.65);1.356(0.45 );1.338(0.88); 1.321(0.4);0(2.47) |
| I-238 | 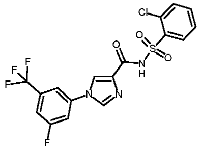 | Beispiel I-238: 1H-NMR(400.0 MHz, d6-DMSO): δ= 8.744(14.77);8.741(16);8.656(15.92);8.652(15.58);8.181(7.07);8.177(7.8);8.161(7.94);8.157( 8.24);8.124(5.61);8.12(3.86);8.1(5.71);8.095(3.82);8.06(11.23);7.815(5.16);7.794(5.27);7.728 (2);7.724(2.23);7.708(5.88);7.705(5.83);7.691(7.53);7.687(7.7);7.674(9.74);7.67(13.15);7.65 4(5.44);7.65(4.19);7.639(5.91);7.635(5.28);7.619(7.3);7.602(3.89);7.598(3.55);5.757(2.77);4. 222(0.4);4.205(0.4);2.676(0.79);2.672(1.09);2.667(0.82);2.511(69.54);2.507(134.14);2.503(1 74.13);2.498(128.67);2.494(65.53);2.334(0.94);2.33(1.22);2.325(0.94);2.075(0.55);1.366(0.5 1);0.008(2.64);0(56.6);-0.008(2.57) |
| I-239 | 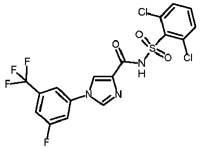 | Beispiel I-239: 1H-NMR(400.0 MHz, d6-DMSO): δ= 8.743(16);8.134(2.09);8.129(1.4);8.11(2.11);8.104(1.42);8.078(4.15);7.913(4.55);7.828(1.93) ;7.807(1.97);7.662(4.16);7.658(5.17);7.64(12.01);7.628(3.64);7.626(3.93);7.607(7.68);7.599( 4.96);7.582(3.08);7.576(2.44);7.559(1.67);7.536(2.89);7.518(2.16);7.514(1.92);7.496(1.36);5. 756(0.89);2.511(26.6);2.507(60.01);2.502(84.2);2.498(65.97);2.494(36.51);2.334(0.7);2.329( 0.88);2.325(0.75);2.075(0.62);0(27.95);-0.008(2.11) |
| I-240 | 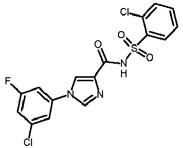 | Beispiel I-240: 1H-NMR(400.0 MHz, d6-DMSO): δ= 8.661(15.17);8.658(16);8.585(15.53);8.583(14.96);8.177(6.95);8.173(7.45);8.157(7.66);8.153 (7.82);7.832(12);7.796(4.1);7.791(6.42);7.786(3.8);7.771(4.15);7.766(6.43);7.761(3.74);7.72 7(1.95);7.723(2.12);7.707(6.1);7.704(5.89);7.69(7.04);7.686(7.13);7.672(9.71);7.669(12.3);7. 653(5.09);7.637(5.57);7.633(5.04);7.616(7.38);7.6(3.57);7.596(3.29);7.564(4.05);7.56(6.17); 7.555(4.01);7.543(4.35);7.538(6.28);7.534(3.81);5.758(1.85);2.676(0.32);2.672(0.56);2.668(0 .36);2.507(108.03);2.503(141.16);2.499(111.02);2.334(1.07);2.329(1.31);2.326(1.11);0(34.54 );-0.058(0.33) |
| I-241 | 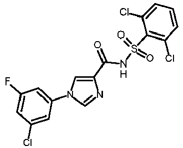 | Beispiel I-241: 1H-NMR(400.0 MHz, d6-DMSO): δ= 8.674(9.54);8.666(11.38);7.915(0.62);7.846(6.89);7.808(2.47);7.802(3.67);7.783(2.49);7.778( 3.65);7.661(6.07);7.657(6.96);7.639(16);7.607(1.36);7.599(6.45);7.582(4.76);7.576(5.76);7.5 72(4.28);7.559(3.18);7.555(2.96);7.55(3.72);7.537(0.62);7.518(0.35);7.374(0.45);5.757(4.96) ;2.673(0.39);2.508(49.08);2.504(60.12);2.5(45.06);2.33(0.37);0(16) |
| I-242 | 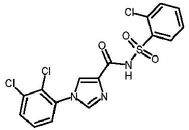 | Beispiel I-242: 1H-NMR(400.0 MHz, d6-DMSO): δ= 8.335(15.84);8.332(16);8.316(0.33);8.193(15.3);8.19(14.7);8.178(7.21);8.174(7.33);8.158(7.5 6);8.154(7.41);7.87(7.34);7.866(7.84);7.85(8.54);7.846(8.61);7.73(1.92);7.726(2.02);7.709(5. 75);7.706(5.4);7.692(7.67);7.688(7.72);7.68(9.74);7.676(12.38);7.666(6.85);7.661(9.19);7.65 6(3.89);7.646(11.05);7.642(10.08);7.636(6.3);7.632(5.02);7.617(6.6);7.613(5.33);7.599(3.83) ;7.595(3.91);7.59(9.81);7.57(13.81);7.55(5.56);2.676(0.47);2.671(0.68);2.667(0.46);2.511(47. 69);2.507(92.01);2.502(118.97);2.498(86.89);2.493(43.53);2.333(0.66);2.329(0.85);2.324(0.6 5);2.075(3.3);1.074(0.41);0.008(3.04);0(68.59);-0.008(3.31);-0.15(0.33) |
| I-243 | 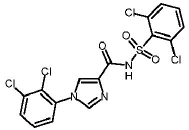 | Beispiel I-243: 1H-NMR(400.0 MHz, d6-DMSO): δ= 8.341(9.48);8.337(10.53);8.293(7.75);8.29(6.9);7.879(4.69);7.875(5.19);7.859(5.49);7.855(5. 62);7.686(3.96);7.682(4.37);7.666(11.73);7.661(11.19);7.654(0.87);7.644(14.82);7.643(16);7. 602(7.97);7.597(6.45);7.585(5.33);7.577(9.72);7.562(2.96);7.557(3.84);5.756(0.47);2.676(0.3 4);2.671(0.47);2.667(0.34);2.525(1.65);2.52(2.57);2.511(27.81);2.507(56.15);2.502(73.6);2.4 98(52.65);2.493(24.85);2.334(0.35);2.329(0.48);2.324(0.34);2.075(0.8);1.073(0.5);0.008(1.17 );0(34.45);-0.008(1.12) |
| I-244 | 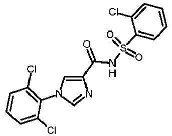 | Beispiel I-244: 1H-NMR(400.0 MHz, d6-DMSO): δ= 8.255(8.13);8.252(8.54);8.181(3.6);8.177(3.84);8.161(4.01);8.157(3.93);8.136(8.15);8.132(7. 87);7.769(8.56);7.768(9.74);7.748(16);7.737(1.17);7.733(1.11);7.717(2.9);7.713(2.89);7.7(4. 17);7.696(4.23);7.689(5.09);7.685(6.42);7.669(2.38);7.665(1.41);7.645(6.37);7.642(3.63);7.6 38(2.79);7.626(5.73);7.623(7.26);7.618(2.97);7.604(5.2);7.6(2.14);2.524(0.77);2.511(25.52); 2.506(52.73);2.502(70.76);2.497(52.08);2.493(26.16);2.333(0.46);2.328(0.58);2.324(0.46);2. 075(0.73);0.008(1.29);0(47.45);-0.009(2.17) |
| I-245 | 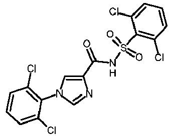 | Beispiel I-245: 1H-NMR(400.0 MHz, d6-DMSO): δ= 8.264(7.21);8.261(8.07);8.206(7.23);8.203(6.86);7.912(0.47);7.775(8.78);7.773(9.54);7.753(1 6);7.677(5.65);7.673(7);7.655(15.03);7.652(7.48);7.632(4.89);7.629(4.65);7.624(0.84);7.615( 6.68);7.61(3.85);7.606(1.13);7.598(4.19);7.592(3.2);7.575(2.24);7.536(0.37);2.676(0.45);2.67 1(0.62);2.666(0.45);2.524(2.17);2.511(37.39);2.506(75.57);2.502(99.5);2.497(71.78);2.493(3 4.61);2.333(0.49);2.329(0.66);2.324(0.49);2.074(0.46);1.355(0.35);0.008(2.16);0(61.89);-0.009(2.26) |
| I-246 | 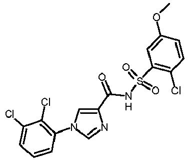 | Beispiel I-246: 1H-NMR(400.0 MHz, d6-DMSO): δ= 8.341(3.46);8.338(3.42);8.206(3.31);8.203(3.13);7.872(1.61);7.868(1.72);7.852(1.87);7.848(1 .89);7.67(1.34);7.666(1.44);7.65(2.26);7.646(2.06);7.622(2.9);7.614(3.05);7.592(2.14);7.583( 2.88);7.572(3.08);7.561(3.29);7.552(1.39);7.298(1.65);7.291(1.59);7.276(1.43);7.268(1.36);3. 859(16);3.816(0.6);2.506(24.8);2.502(32.04);2.498(24.09);2.075(0.95);0.008(0.51);0(11.45) |
| I-247 | 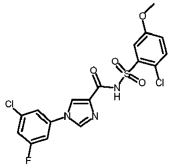 | Beispiel I-247: 1H-NMR(400.0 MHz, d6-DMSO): δ= 8.662(2.69);8.658(3.03);8.594(2.9);8.591(2.84);7.83(2.2);7.796(0.79);7.79(1.28);7.785(0.73); 7.771(0.8);7.766(1.3);7.76(0.73);7.619(2.89);7.611(3.22);7.574(2.76);7.567(0.98);7.562(1.35) ;7.557(0.98);7.552(3.54);7.546(1.04);7.541(1.31);7.536(0.76);7.53(0.34);7.294(1.59);7.286(1. 56);7.272(1.39);7.264(1.35);3.858(16);3.816(1.3);2.524(0.52);2.511(14.91);2.507(31);2.502( 41.93);2.498(32.02);2.493(16.99);2.329(0.34);2.075(1.78);0.008(0.52);0(18.02);-0.008(1.05) |
| I-248 | 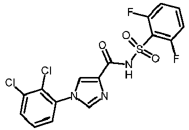 | Beispiel I-248: 1H-NMR(400.0 MHz, d6-DMSO): δ= 8.357(0.33);8.355(0.33);8.33(16);7.883(2.97);7.88(3.27);7.863(3.44);7.859(3.56);7.782(0.52) ;7.767(1.15);7.761(1.16);7.746(2.03);7.731(1.2);7.725(1.3);7.709(0.58);7.687(2.5);7.684(2.79 );7.667(4.02);7.664(3.76);7.6(3.65);7.58(5.35);7.56(2.21);7.321(3.35);7.298(5.11);7.276(2.88 );2.671(0.32);2.506(41.42);2.502(53.83);2.498(41.02);2.329(0.36);2.075(2.1);1.076(0.33);0.0 08(0.96);0(19.24) |
| I-249 | 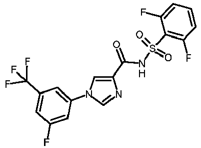 | Beispiel 1-249: 1H-NMR(400.0 MHz, d6-DMSO): δ= 8.781(3.46);8.778(3.81);8.747(5.03);8.744(4.15);8.147(1.54);8.142(0.97);8.122(1.54);8.117(0 .99);8.094(2.9);7.985(0.58);7.83(1.32);7.809(1.33);7.78(0.38);7.765(0.86);7.759(0.78);7.749( 0.58);7.744(1.49);7.738(0.57);7.728(0.84);7.722(1.01);7.707(0.46);7.317(2.39);7.295(3.6);7.2 72(2.16);6.871(0.51);2.525(0.43);2.52(0.77);2.512(14.92);2.507(31.15);2.503(42.55);2.498(3 1.65);2.494(15.55);2.184(0.84);2.075(16);1.369(0.37);1.356(6.43);0(5.57) |
| I-250 | 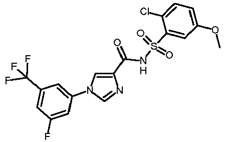 | Beispiel I-250: 1H-NMR(400.0 MHz, d6-DMSO): δ= 8.748(3.05);8.745(3.16);8.666(3.1);8.662(2.88);8.124(1.05);8.12(0.68);8.1(1.07);8.095(0.68); 8.06(2.08);7.819(0.95);7.798(0.96);7.624(2.93);7.616(3.05);7.577(2.71);7.555(3.2);7.297(1.6 8);7.29(1.59);7.275(1.43);7.267(1.37);3.861(16);3.842(0.33);3.817(1.36);2.512(11.87);2.507( 22.97);2.503(30.26);2.498(22.58);2.494(11.28);0.008(0.32);0(7.89) |
| I-251 | 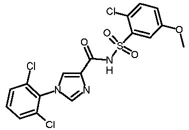 | Beispiel I-251: 1H-NMR(400.0 MHz, d6-DMSO): δ= 8.259(2.91);8.256(3.08);8.143(2.97);8.14(2.88);7.77(3.63);7.75(6.11);7.647(2.13);7.628(1.97) ;7.624(3.93);7.616(3.11);7.606(1.42);7.593(2.7);7.571(3.12);7.306(1.59);7.299(1.53);7.284(1. 41);7.277(1.34);3.858(16);3.841(0.44);3.816(0.6);2.524(0.5);2.511(15.73);2.506(32.58);2.502 (43.63);2.497(32.68);2.493(16.84);2.328(0.33);2.074(3.12);0.008(0.5);0(16.14);-0.008(0.81) |
| I-252 | 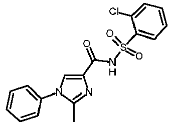 | Beispiel I-252: 1H-NMR(400.0 MHz, d6-DMSO): δ= 8.133(0.53);8.113(1.82);8.11(1.86);8.093(2.01);8.091(2.01);8.073(6.49);8.044(0.35);7.618(0. 95);7.613(0.99);7.606(2.28);7.597(3.25);7.584(8.87);7.578(11.58);7.567(3.35);7.559(5.98);7. 54(1.32);7.532(1.63);7.527(1.01);7.512(1.51);7.496(0.9);7.491(0.61);7.476(0.36);7.373(0.46) ;2.671(0.34);2.506(41.91);2.502(53.13);2.497(40.46);2.415(0.9);2.374(16);2.329(0.4);2.074(1 .06);0(17.66) |
| I-253 | 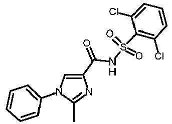 | Beispiel I-253: 1H-NMR(400.0 MHz, d6-DMSO): δ= 8.044(4.97);7.623(0.5);7.616(1.39);7.612(2.04);7.606(11.77);7.59(0.6);7.579(0.39);7.497(2.2 5);7.494(2.43);7.476(4.6);7.41(1.82);7.393(1.4);7.388(1.2);7.371(0.87);2.524(0.6);2.511(11.5 4);2.506(23.06);2.502(30.46);2.497(22.38);2.493(10.99);2.415(11);2.074(16) |
| I-254 | 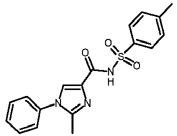 | Beispiel I-254: 1H-NMR(400.0 MHz, d6-DMSO): δ= 8.047(5.81);7.875(2.95);7.871(1.07);7.859(1.09);7.854(3.32);7.723(0.84);7.718(5.4);7.714(1. 93);7.702(2.29);7.697(6.57);7.693(1.02);7.594(0.42);7.59(0.73);7.585(0.39);7.579(0.52);7.57 3(2.06);7.568(1.29);7.558(1.14);7.555(2.77);7.548(0.63);7.545(0.67);7.54(1.31);7.536(1.16); 7.53(0.38);7.523(1.44);7.514(2.73);7.51(2.92);7.504(0.9);7.498(0.93);7.494(1.75);7.49(1.25); 7.406(2.49);7.386(2.42);7.375(4.22);7.374(4.69);7.354(4.15);7.27(5.19);3.767(0.59);3.601(1. 45);3.562(1.32);2.526(0.35);2.521(0.56);2.512(7.16);2.508(14.5);2.503(19.26);2.499(14.08);2 .494(6.86);2.386(8.42);2.372(16);2.309(12.57);2.184(0.37);2.073(3.64);1.357(2.63);0(2) |
| I-255 | 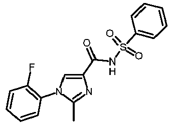 | Beispiel I-255: 1H-NMR(400.0 MHz, d6-DMSO): δ= 8.08(6.36);8.079(6.28);8.012(0.51);8.004(4.2);8.001(2.15);7.991(1.23);7.986(5.53);7.983(3.7 6);7.843(2.56);7.84(2.72);7.836(1.09);7.834(1.06);7.829(0.99);7.824(2.63);7.819(3.06);7.711 (0.48);7.708(0.87);7.705(0.57);7.695(0.65);7.689(2.64);7.684(0.83);7.674(1.33);7.671(2.2);7. 668(1.19);7.639(1.89);7.632(4.49);7.628(2.5);7.622(2.38);7.619(3.8);7.616(5.2);7.613(6.58); 7.605(2.66);7.602(2.39);7.6(2.64);7.595(3.99);7.592(4.21);7.587(5.38);7.582(2.44);7.576(1); 7.571(1.44);7.568(2.93);7.56(0.54);7.554(1.65);7.552(2.07);7.545(0.89);7.533(1.05);7.529(2. 02);7.526(1.62);7.507(0.93);7.504(0.81);7.424(1.41);7.422(1.37);7.406(2.01);7.403(2.33);7.3 86(1.07);7.383(1.06);7.354(3.25);6.872(0.71);2.512(8.92);2.507(19.73);2.503(27.38);2.498(2 0.8);2.494(10.77);2.22(16);2.184(1.34);2.075(4.15);1.357(8.71);0(3.34) |
| I-256 | 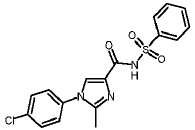 | Beispiel I-256: 1H-NMR(400.0 MHz, d6-DMSO): δ= 8.062(7.52);7.984(3.12);7.971(0.96);7.966(3.87);7.963(2.68);7.842(1.05);7.839(1.13);7.828(0 .41);7.823(1.13);7.818(1.25);7.681(0.57);7.668(0.51);7.662(1.96);7.652(3.88);7.646(2.46);7.6 35(2.14);7.63(6.65);7.622(1.25);7.61(2.93);7.602(1.06);7.59(4.3);7.576(1.73);7.57(7.54);7.55 3(1.54);7.548(3.74);7.541(0.45);7.353(1.68);6.871(0.67);2.511(7.97);2.507(17.48);2.502(24.2 1);2.498(18.6);2.493(9.85);2.363(0.46);2.311(16);2.287(0.59);2.184(1.16);2.075(2.47);1.356( 7.89);1.104(0.57);1.089(0.59);0(2.48) |
| I-257 | 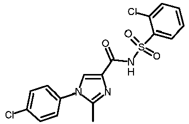 | Beispiel I-257: 1H-NMR(400.0 MHz, d6-DMSO): δ= 8.116(1.89);8.113(1.95);8.091(7.64);7.68(3.31);7.675(1.52);7.658(7.01);7.621(6.92);7.604(2. 06);7.599(3.53);7.589(1.59);7.586(1.46);7.568(4.01);7.564(3.49);7.548(1.11);7.541(1.59);7.5 36(1.13);7.521(1.65);7.505(0.76);7.5(0.64);2.506(24.75);2.502(31.41);2.498(23.97);2.361(16) ;2.329(0.32);2.074(14.72);0(7.05) |
| I-258 | 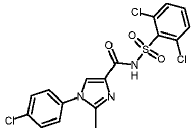 | Beispiel I-258: 1H-NMR(400.0 MHz, d6-DMSO): δ= 8.067(6.57);7.912(2.65);7.702(2.81);7.68(7.18);7.654(7.09);7.631(3.03);7.627(2.25);7.626(2. 41);7.606(3.42);7.536(1.21);7.517(1.09);7.514(1.04);7.503(3.97);7.483(7.05);7.42(2.35);7.40 2(1.9);7.399(1.7);7.38(1.07);7.372(0.37);2.502(44.72);2.402(16);2.328(0.33);2.074(1.47);0(1 6.92) |
| I-259 | 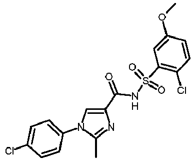 | Beispiel I-259: 1H-NMR(400.0 MHz, d6-DMSO): δ= 8.084(5.92);7.685(2.47);7.68(0.98);7.669(1.36);7.663(5.72);7.657(0.78);7.634(0.86);7.628(5. 44);7.622(1.28);7.611(1.11);7.606(2.7);7.599(0.36);7.592(2.86);7.584(2.9);7.457(2.17);7.435 (2.52);7.161(1.26);7.153(1.19);7.139(1.08);7.131(1.03);3.825(16);3.816(1.67);2.524(0.6);2.5 19(1.02);2.511(13.69);2.506(27.95);2.502(37.32);2.497(27.34);2.492(13.36);2.369(12.44);2.0 74(8.41);0.008(0.77);0(24.03);-0.009(0.91) |
| I-260 | 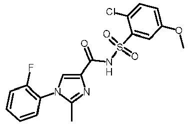 | Beispiel I-260: 1H-NMR(400.0 MHz, d6-DMSO): δ= 8.13(3.82);7.695(0.87);7.675(1.79);7.656(1.53);7.632(1.17);7.618(0.82);7.598(2.84);7.591(2. 91);7.578(1.31);7.554(1.57);7.531(0.88);7.508(2.28);7.487(2.63);7.445(1.16);7.425(1.83);7.4 07(0.85);7.217(1.51);7.21(1.47);7.195(1.37);7.188(1.27);4.132(0.35);4.017(0.68);3.838(16);3 .816(2.17);3.759(1.41);3.654(0.96);3.502(0.41);2.671(0.56);2.502(74.17);2.329(0.65);2.278(1 1.34);0(0.76) |
| I-261 | 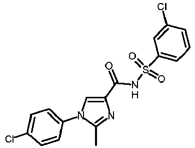 | Beispiel I-261: 1H-NMR(400.0 MHz, d6-DMSO): δ= 8.062(7.41);7.936(2.9);7.932(1.99);7.866(1.67);7.847(1.94);7.679(4.3);7.657(8.01);7.638(0.4 1);7.608(6.43);7.586(4.18);7.57(2.81);7.55(1.17);7.515(0.38);2.506(32.45);2.502(47.08);2.49 8(40);2.358(16);2.329(1.18);2.192(0.38);2.074(0.4);0(3.27) |
| I-262 | 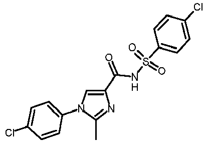 | Beispiel I-262: 1H-NMR(400.0 MHz, d6-DMSO): δ= 8.052(7.21);7.958(0.55);7.951(4.62);7.946(1.55);7.935(1.61);7.93(5.44);7.923(0.69);7.836(0. 36);7.814(0.45);7.674(0.41);7.667(4.12);7.662(1.51);7.651(2.39);7.646(11.32);7.629(1.46);7. 624(4.11);7.618(0.62);7.597(0.87);7.591(6.42);7.585(1.7);7.574(1.31);7.568(3.64);7.561(0.39 );7.465(0.34);2.671(0.33);2.525(1.16);2.511(17.18);2.507(33.61);2.502(43.79);2.498(31.25);2 .493(14.5);2.362(0.34);2.337(16);2.075(0.6);0.008(0.55);0(13.96);-0.008(0.35) |
| I-263 | 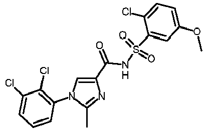 | Beispiel I-263: 1H-NMR(400.0 MHz, d6-DMSO): δ= 8.108(4.26);7.919(1.43);7.916(1.58);7.899(1.69);7.895(1.73);7.719(1.23);7.716(1.32);7.699(1 .9);7.696(1.76);7.62(1.88);7.608(0.62);7.599(5.31);7.591(2.98);7.58(1.25);7.551(0.33);7.529( 0.42);7.52(2.48);7.498(2.89);7.48(0.33);7.472(0.33);7.228(1.47);7.22(1.42);7.206(1.28);7.198 (1.25);3.839(16);3.816(2.24);3.61(5.48);2.671(0.4);2.511(22.6);2.507(43.89);2.502(57.08);2. 498(42);2.493(20.77);2.329(0.36);2.208(12.56);2.074(0.95) |
| I-264 | 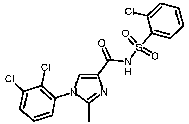 | Beispiel I-264: 1H-NMR(400.0 MHz, d6-DMSO): δ= 8.14(1.61);8.136(1.67);8.12(1.76);8.116(1.82);8.106(6.07);7.916(1.97);7.912(2.12);7.895(2.3 3);7.891(2.31);7.71(1.66);7.706(1.77);7.69(2.67);7.687(2.37);7.67(0.4);7.666(0.41);7.65(1.3); 7.646(1.21);7.633(2.06);7.629(2.44);7.627(2.54);7.622(2.98);7.616(2.79);7.607(1.12);7.602(0 .77);7.596(3.91);7.586(1.41);7.582(1.18);7.576(1.82);7.569(1.12);7.567(1.47);7.562(1.12);7.5 5(0.84);7.545(0.72);3.722(1.38);3.714(1.39);3.476(0.35);2.676(0.35);2.671(0.47);2.666(0.33) ;2.524(1.16);2.52(1.99);2.511(25.66);2.506(51.58);2.502(69.08);2.497(50.82);2.493(24.13);2. 333(0.38);2.328(0.5);2.324(0.36);2.197(16);2.074(1.01);0.008(0.66);0(19.65);-0.008(0.63) |
| I-265 | 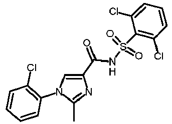 | Beispiel 1-265: 1H-NMR(400.0 MHz, d6-DMSO): δ= 8.087(8.93);7.806(1.72);7.803(1.85);7.786(2.23);7.783(2.27);7.752(1.53);7.748(1.65);7.733(2 .06);7.729(2.11);7.679(0.89);7.675(0.98);7.66(1.96);7.656(1.66);7.64(1.41);7.636(1.16);7.618 (1.58);7.614(1.6);7.606(0.51);7.598(1.95);7.595(1.92); 7 .579(0. 76);7 .576(0. 7);7 .551(2.94);7.5 48(3.38);7.53(6.74);7.472(2.5);7.455(1.82);7.45(1.55);7.433(1.07);7.372(0.6);2.524(0.59);2.5 19(1.05);2.51(19.48);2.506(40.36);2.501(54.17);2.497(40.3);2.492(20.32);2.328(0.4);2.255(1 6);2.074(7.57);0.008(0.61);0(24.32);-0.008(1.11) |
| I-266 | 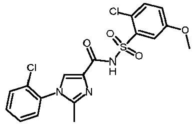 | Beispiel I-266: 1H-NMR(400.0 MHz, d6-DMSO): δ= 8.084(4.36);7.79(0.94);7.787(1.03);7.77(1.21);7.767(1.26);7.714(0.82);7.709(0.9);7.694(1.15) ;7.69(1.24);7.659(0.54);7.654(0.58);7.64(1.23);7.635(1.03);7.62(1.24);7.61(4.2);7.601(3.48); 7.597(1.62);7.593(2.53);7.59(1.3);7.582(1.21);7.578(1.17);7.563(0.46);7.559(0.43);7.552(2.7 2);7.531(3.29);7.525(1.12);7.51(1.74);7.488(2.06);7.48(2.67);7.473(2.75);7.307(0.46);7.299( 0.44);7.285(0.41);7.277(0.39);7.215(0.99);7.208(0.97);7.194(2.42);7.186(2.32);7.172(1.42);7. 164(1.36);6.871(0.49);5.757(8.06);4.041(0.35);4.023(1.12);4.006(1.14);3.988(0.4);3.837(14.0 8);3.816(16);2.511(12.06);2.506(26.18);2.502(35.89);2.497(26.99);2.493(13.9);2.205(8.93);2. 183(0.94);1.355(5.79);1.101(1.31);1.083(2.79);1.066(1.42);0(4.93) |
| I-267 | 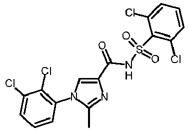 | Beispiel I-267: 1H-NMR(400.0 MHz, d6-DMSO): δ= 8.111(7.67);7.94(1.94);7.937(2.07);7.92(2.29);7.916(2.33);7.756(1.72);7.752(1.8);7.736(2.48) ;7.732(2.23);7.67(0.53);7.637(2.47);7.617(3.89);7.596(1.7);7.564(2.9);7.561(3.28);7.543(7.07 );7.488(2.59);7.47(1.85);7.466(1.55);7.448(1.08);5.756(0.82);4.04(0.33);4.022(0.33);2.524(0. 51);2.519(0.95);2.511(19.31);2.506(40.1);2.502(53.77);2.497(39.58);2.493(19.76);2.328(0.39 );2.258(16);1.102(0.45);1.084(0.94);1.066(0.45);0.008(0.62);0(24.31);-0.008(1.07) |
| I-268 | 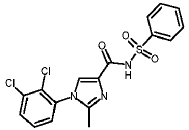 | Beispiel I-268: 1H-NMR(400.0 MHz, d6-DMSO): δ= 8.06(7.51);8.009(3.15);7.991(3.88);7.987(2.82);7.909(1.83);7.896(2.25);7.892(4.16);7.888(2. 02);7.876(2.06);7.872(2.12);7.745(0.39);7.726(1.21);7.721(0.44);7.711(0.99);7.708(1.16);7.7 (0.48);7.694(1.91);7.689(0.63);7.676(1.55);7.66(1.91);7.656(2.04);7.652(1.65);7.636(5.73);7. 632(3.44);7.617(4.22);7.598(3.73);7.587(0.59);7.578(3.44);7.567(0.38);7.558(1.37);7.352(0.3 5);5.757(4.91);4.038(0.95);4.021(3.01);4.003(3.05);3.985(1.01);3.601(0.44);2.525(0.41);2.50 7(26.28);2.502(34.4);2.498(25.27);2.494(12.58);2.184(0.36);2.15(16);2.135(0.38);1.759(0.51) |
| | | ;1.356(2.36);1.116(3.11);1.099(6.46);1.089(0.65);1.081(3.06);0.008(0.54);0(16.4);-0.008(0.62) |
| I-269 | 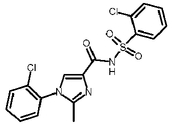 | Beispiel I-269: 1H-NMR(400.0 MHz, d6-DMSO): δ= 8.137(1.91);8.134(1.89);8.118(2.02);8.114(1.93);8.079(6.44);7.787(1.84);7.783(1.86);7.767(2 .32);7.763(2.24);7.703(1.58);7.698(1.66);7.684(2.17);7.679(2.26);7.654(1.36);7.65(1.19);7.63 5(3.38);7.631(2.22);7.621(2.72);7.616(4.86);7.612(4.17);7.597(2.63);7.593(2.09);7.578(3.24) ;7.574(2.73);7.558(2.2);7.542(0.73);7.537(0.6);2.506(27.94);2.502(35.47);2.497(26.17);2.193 (16);2.075(0.36);0.008(0.6);0(12.83);-0.008(0.66) |
| I-270 | 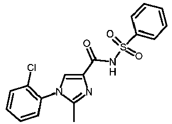 | Beispiel I-270: 1H-NMR(400.0 MHz, d6-DMSO): δ= 8.021(6.4);8.007(3.92);7.989(4.17);7.764(1.71);7.761(1.68);7.744(2.37);7.707(0.72);7.688(2. 08);7.67(1.75);7.632(4.63);7.612(7.17);7.593(2.98);7.576(1.85);7.572(1.88);7.556(1.59);7.55 4(1.74);7.538(0.6);7.535(0.56);2.506(22.49);2.502(27.94);2.498(21.9);2.141(16);0(8.48) |
| I-271 | 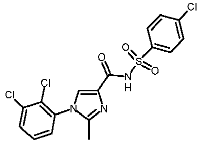 | Beispiel I-271: 1H-NMR(400.0 MHz, d6-DMSO): δ= 8.078(5.04);8.003(0.47);7.996(3.34);7.992(1.19);7.98(1.28);7.975(3.87);7.969(0.53);7.914(2. 08);7.907(1.51);7.903(1.51);7.898(0.95);7.893(2.72);7.886(1.88);7.883(1.59);7.84(0.86);7.82 4(0.35);7.819(1.12);7.745(0.38);7.738(2.61);7.734(0.83);7.722(0.74);7.717(2.05);7.71(0.4);7. 705(0.6);7.699(3.68);7.694(1.29);7.682(1.2);7.677(3.33);7.669(2.24);7.652(2.09);7.648(2.35) ;7.609(1.71);7.588(2.35);7.568(0.93);7.47(0.87);4.055(0.7);4.038(2.24);4.02(2.26);4.002(0.73 );3.602(0.42);2.513(6.15);2.509(12.02);2.504(15.61);2.5(11.61);2.176(10.59);2.077(16);1.759 (0.5);1.357(1.89);1.133(2.29);1.124(0.39);1.116(4.7);1.109(0.49);1.098(2.23);1.058(0.4);0.00 8(0.43);0(11.67);-0.008(0.55) |
| I-272 | 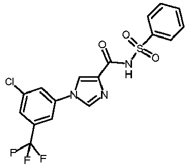 | Beispiel I-272: 1H-NMR(400.0 MHz, d6-DMSO): δ= 8.718(4.01);8.714(4.19);8.633(4.13);8.629(3.81);8.284(2.84);8.28(1.73);8.172(2.57);8.027(3. 26);8.014(1.06);8.009(4.21);8.006(2.97);7.94(2.63);7.911(1.28);7.898(0.46);7.893(1.77);7.89 (1.33);7.734(0.8);7.731(0.62);7.728(0.99);7.722(0.76);7.716(2.18);7.71(1.1);7.706(0.57);7.70 1(1.1);7.698(1.72);7.694(0.93);7.657(3.44);7.641(4.02);7.638(4.59);7.623(1.4);7.62(1.92);4.0 4(0.69);4.022(2.17);4.005(2.19);3.987(0.71);2.526(0.44);2.513(9.71);2.509(18.95);2.504(24.4 8);2.5(17.78);2.495(8.69);2.077(16);1.357(1.48);1.118(2.25);1.106(0.98);1.1(4.7);1.09(0.84); 1.083(2.21);0.008(0.62);0(15.59);-0.008(0.57) |
| I-273 | 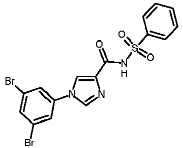 | Beispiel I-273: 1H-NMR(400.0 MHz, d6-DMSO): δ= 8.62(6.78);8.616(7.4);8.544(7.49);8.541(7.11);8.138(0.48);8.134(0.5);8.089(15.72);8.085(16) ;8.02(6.51);8.002(7.91);7.998(5.67);7.908(6.92);7.904(8.6);7.9(4.26);7.891(3.92);7.887(2.96) ;7.842(0.33);7.839(0.36);7.823(0.46);7.818(0.43);7.743(0.67);7.74(0.5);7.73(1.72);7.725(2.33 );7.717(1.24);7.711(4.29);7.706(2.78);7.696(2.04);7.693(3.17);7.658(3.78);7.653(6.12);7.637 (6.28);7.633(8.37);7.619(2.46);7.615(3.34);7.588(0.58);7.568(0.35);7.353(0.45);5.758(4.43); 4.037(1.44);4.019(4.52);4.001(4.57);3.984(1.51);3.618(0.82);3.612(0.63);3.608(0.82);3.602(1 .92);3.596(0.84);3.591(0.61);3.585(0.86);3.338(0.37);3.32(0.4);2.672(0.42);2.512(25.86);2.50 8(49.57);2.503(63.84);2.499(46.21);2.494(22.48);2.334(0.34);2.33(0.44);2.325(0.32);2.184(0. |
| | | 45);1.776(0.78);1.768(0.85);1.76(2.2);1.751(0.83);1.743(0.73);1.356(3.11);1.22(0.51);1.205( 0.44);1.201(0.36);1.117(5.08);1.104(1.84);1.099(10.45);1.089(1.5);1.081(4.96);1.057(0.32);0. 008(1.56);0(35.51);-0.008(1.25) |
| I-274 | 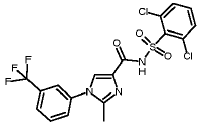 | Beispiel I-274: 1H-NMR(400.0 MHz, d6-DMSO): δ= 8.159(4.37);8.074(3.54);7.976(1.89);7.955(3.36);7.93(2.59);7.87(1.9);7.851(2.38);7.831(0.98) ;7.625(0.47);7.606(0.62);7.535(0.44);7.514(4.02);7.495(6.43);7.432(2.1);7.412(2.01);7.392(1. 06);2.671(0.78);2.502(147.96);2.412(16);2.328(1.57);2.255(0.47);1.077(0.55);1.059(0.33);0( 1.08) |
| I-275 | 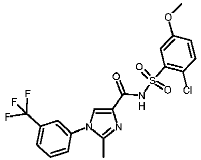 | Beispiel I-275: 1H-NMR(400.0 MHz, d6-DMSO): δ= 8.186(5.05);8.041(2.39);7.954(1.2);7.933(2.21);7.908(1.62);7.857(1.39);7.837(1.69);7.817(0. 62);7.598(2.92);7.591(3.03);7.474(2.08);7.452(2.4);7.178(1.26);7.171(1.26);7.156(1.11);7.14 9(1.07);3.832(16);2.507(24.66);2.502(31.26);2.498(23.97);2.384(12.65);2.075(0.91) |
| I-276 | 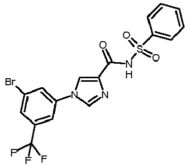 | Beispiel I-276: 1H-NMR(400.0 MHz, d6-DMSO): δ= 8.713(10.18);8.71(11.17);8.624(10.88);8.621(10.77);8.566(0.35);8.563(0.42);8.489(0.36);8.4 86(0.37);8.392(8.34);8.201(7.79);8.038(8.48);8.025(10.07);8.007(11.43);8.004(9.01);7.91(3.9 5);7.892(4.86);7.888(3.9);7.824(0.33);7.819(0.34);7.745(0.86);7.733(2.26);7.726(3);7.721(2. 22);7.715(6.08);7.709(3.6);7.696(4.61);7.656(10.06);7.64(11.21);7.636(13.38);7.622(3.94);7. 618(5.46);7.594(0.37);7.588(0.52);7.568(0.32);7.352(0.36);6.872(0.37);5.758(8.47);4.039(1.8 2);4.021(5.64);4.003(5.72);3.986(1.92);3.858(0.39);3.618(0.65);3.608(0.72);3.602(1.59);3.58 5(0.74);3.368(0.35);3.342(0.5);3.324(0.56);3.307(0.33);3.227(0.35);3.211(0.35);2.677(0.4);2. 672(0.56);2.668(0.43);2.508(67.25);2.504(88.21);2.499(67.62);2.463(0.48);2.445(0.43);2.418 (0.37);2.335(0.47);2.33(0.62);2.326(0.49);2.184(0.63);2.087(16);1.776(0.64);1.767(1.24);1.7 6(1.8);1.751(0.76);1.743(0.65);1.733(0.6);1.648(0.44);1.559(0.34);1.546(0.34);1.541(0.35);1. 356(4.21);1.232(0.39);1.221(0.4);1.196(0.53);1.178(0.84);1.159(0.46);1.117(6.09);1.105(2.21 );1.1(12.56);1.09(1.75);1.082(6.01);1.057(0.38);0.008(2.63);0(53.51) |
| I-277 | 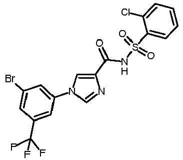 | Beispiel I-277: 1H-NMR(400.0 MHz, d6-DMSO): δ= 8.754(16);8.651(15.83);8.384(12.39);8.19(12.15);8.18(8.51);8.176(8.16);8.159(7.97);8.156(7. 9);8.093(0.33);8.055(12.51);7.726(1.89);7.724(2.07);7.706(6.37);7.704(6.48);7.689(7.07);7.6 87(7.11);7.669(12.04);7.652(5.26);7.637(5.56);7.635(5.05);7.619(7.7);7.617(7.7);7.615(6.97) ;7.6(3.65);7.598(3.48);4.012(0.64);3.995(0.65);3.975(0.33);2.671(1.5);2.505(191.62);2.502(2 32.82);2.498(179.36);2.328(1.72);2.075(0.52);1.091(0.52);1.073(1.04);1.055(0.53);0.145(0.4 3);0(88.55);-0.002(77.76);-0.008(6.15);-0.151(0.45) |
| I-278 | 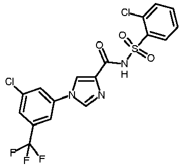 | Beispiel I-278: 1H-NMR(400.0 MHz, d6-DMSO): δ= 8.759(14.22);8.756(14.5);8.659(14.91);8.656(13.64);8.316(0.42);8.282(5.78);8.277(10.46);8. 273(5.89);8.181(6.87);8.177(7.25);8.161(16);8.157(13.16);7.958(9.04);7.729(2.09);7.725(2.2) ;7.72(0.51);7.711(3.67);7.709(5.24);7.705(5.46);7.701(1.09);7.691(7.43);7.687(7.09);7.674(8. 54);7.67(11.43);7.654(4.76);7.65(3.22);7.639(5.61);7.635(4.46);7.621(4.86);7.62(6.08);7.618 (4.88);7.616(4.87);7.608(0.53);7.602(3.68);7.598(3.17);5.757(5);4.013(0.68);3.995(0.69);2.6 8(0.55);2.676(1.08);2.671(1.46);2.667(1.07);2.662(0.54);2.541(0.65);2.525(4.35);2.52(6.63); 2.511(75.66);2.507(152.9);2.502(201.28);2.498(142.4);2.493(66.07);2.338(0.44);2.334(0.97); 2.329(1.34);2.324(0.96);2.32(0.42);1.091(0.61);1.073(1.31);1.055(0.6);0.146(0.49);0.008(4.1 9);0(130.47);-0.008(4.11);-0.15(0.51) |
| I-279 | 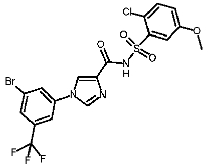 | Beispiel I-279: 1H-NMR(400.0 MHz, d6-DMSO): δ= 8.761(3.3);8.759(3.41);8.665(3.36);8.663(3.23);8.385(2.68);8.192(2.53);8.06(2.67);7.624(2.8 6);7.616(2.99);7.577(2.68);7.555(3.16);7.297(1.65);7.289(1.6);7.275(1.43);7.267(1.36);5.757 (0.4);3.861(16);3.84(0.56);3.817(0.43);2.507(21.59);2.503(27.5);2.499(20.76);0(9.25) |
| I-280 | 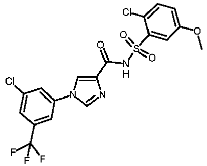 | Beispiel I-280: 1H-NMR(400.0 MHz, d6-DMSO): δ= 8.76(2.81);8.758(2.95);8.668(2.95);8.665(2.81);8.277(2.35);8.272(1.43);8.16(2.1);7.959(2.14) ;7.623(2.89);7.616(3.01);7.576(2.6);7.554(3.06);7.296(1.56);7.288(1.49);7.274(1.35);7.266(1. 3);5.756(0.53);3.86(16);3.839(0.38);2.525(0.65);2.511(14.73);2.507(29.55);2.502(39.03);2.49 8(28.67);2.494(14.21);0.008(0.58);0(16.38);-0.008(0.63) |
| I-281 | 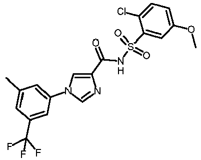 | Beispiel I-281: 1H-NMR(400.0 MHz, d6-DMSO): δ= 8.702(4.68);8.625(4.37);7.934(4.22);7.916(4.21);7.653(4.16);7.625(3.81);7.618(3.62);7.573(2 .71);7.551(3.11);7.291(2.17);7.284(2.06);7.269(1.89);7.262(1.64);5.759(0.33);3.86(16);3.817 (0.85);2.503(38.31);2.474(15.6);2.33(0.36);1.085(0.35);0(13.63) |
| I-282 | 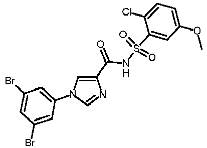 | Beispiel I-282: 1H-NMR(400.0 MHz, d6-DMSO): δ= 8.679(3.92);8.591(3.85);8.082(7.56);8.079(6.98);7.925(3.33);7.619(2.94);7.612(3.21);7.576(2 .65);7.554(3.18);7.295(1.67);7.288(1.68);7.273(1.47);7.266(1.42);3.86(16);3.84(0.76);3.817( 0.55);2.506(23.78);2.503(27.74);0(17.81) |
| I-283 | 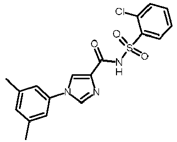 | Beispiel I-283: 1H-NMR(400.0 MHz, d6-DMSO): δ= 8.552(2.12);8.549(2.19);8.484(1.82);8.173(1.1);8.169(1.14);8.153(1.2);8.149(1.18);7.693(0.8 7);7.689(0.77);7.675(1.16);7.671(1.11);7.661(1.43);7.657(1.91);7.642(0.74);7.637(0.51);7.62 6(0.88);7.622(0.7);7.606(1.05);7.589(0.56);7.585(0.48);7.342(3.8);7.084(1.66);2.511(7.8);2.5 07(15.88);2.502(21.01);2.498(15.35);2.493(7.58);2.339(16);2.075(0.95);0.008(0.42);0(14.06) ;-0.009(0.61) |
| I-284 | 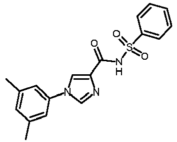 | Beispiel I-284: 1H-NMR(400.0 MHz, d6-DMSO): δ= 8.473(2.64);8.47(2.79);8.409(2.38);8.407(2.24);8.024(2.29);8.006(2.81);8.002(1.97);7.727(0. 44);7.715(0.38);7.709(1.36);7.704(0.46);7.694(0.74);7.69(1.12);7.688(0.64);7.651(1.97);7.63 5(1.63);7.632(2.7);7.618(0.52);7.614(1.05);7.335(4.24);7.065(1.88);2.512(4.43);2.508(8.15); 2.503(10.26);2.499(7.5);2.495(3.74);2.328(16);0(7.18) |
| I-285 | 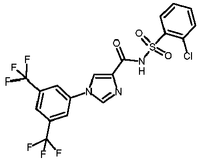 | Beispiel I-285: 1H-NMR(400.0 MHz, d6-DMSO): δ= 8.822(4.6);8.722(6.89);8.506(16);8.316(0.57);8.184(7.32);8.156(4.21);7.716(1.06);7.697(2.95 );7.679(3.44);7.662(5.49);7.644(2.47);7.631(2.96);7.611(3.82);7.594(1.79);3.816(0.33);3.602 (0.95);3.506(1.84);3.368(1.65);3.21(1.01);2.977(0.35);2.671(3.01);2.502(430.59);2.328(3.12) ;2.3(1.18);1.76(0.34);1.234(2.24);0.852(0.35);0(41.45) |
| I-286 | 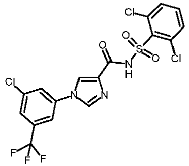 | Beispiel I-286: 1H-NMR(400.0 MHz, d6-DMSO): δ= 8.756(11.23);8.746(9.89);8.289(7.56);8.178(7.35);7.969(7.37);7.657(7);7.639(16);7.598(5.83) ;7.581(4.02);7.575(3.34);7.558(2.05);2.672(0.49);2.503(102.61);2.377(0.33);2.33(0.88);2.075 (1.62);0(9.75) |
| I-287 | 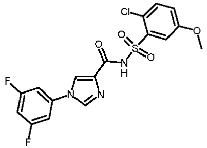 | Beispiel I-287: 1H-NMR(400.0 MHz, d6-DMSO): δ= 8.647(3.72);8.645(3.68);8.59(3.8);8.587(3.43);7.674(2.54);7.658(2.53);7.619(2.93);7.612(3.1 9);7.576(2.61);7.554(3.11);7.395(0.71);7.372(1.38);7.354(0.51);7.348(0.72);7.295(1.64);7.28 7(1.69);7.273(1.43);7.265(1.45);5.758(0.49);3.858(16);3.84(0.51);3.817(0.58);2.506(25.6);2. 503(31.51);2.499(24.85);0(1.72) |
| I-288 | 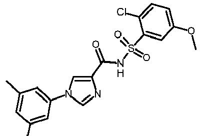 | Beispiel 1-288: 1H-NMR(400.0 MHz, d6-DMSO): δ= 8.557(2.42);8.554(2.74);8.511(2.02);7.622(2.13);7.614(2.22);7.563(2.03);7.541(2.38);7.344(4 .18);7.28(1.23);7.272(1.19);7.258(1.07);7.25(1.03);7.087(1.85);3.857(11.87);2.508(9.77);2.50 3(12.71);2.499(9.49);2.34(16);0(1.03) |
| I-289 | 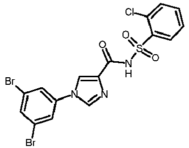 | Beispiel I-289: 1H-NMR(400.0 MHz, d6-DMSO): δ= 8.678(7.11);8.676(8.11);8.582(7.05);8.579(7.47);8.178(3.29);8.174(3.74);8.158(3.64);8.155(3 .94);8.084(14.23);8.08(16);7.926(3.99);7.922(6.88);7.919(4.47);7.728(0.93);7.724(1.09);7.70 8(3.14);7.704(3.15);7.69(3.47);7.687(3.64);7.674(4.51);7.67(6.14);7.654(2.52);7.65(2.07);7.6 38(2.93);7.634(2.77);7.618(3.63);7.601(1.94);7.597(1.78);5.759(3.87);2.508(33.23);2.504(43. 05);2.5(33.22);1.074(0.57);1.066(0.49);1.05(0.47);0(3.25) |
| I-290 | 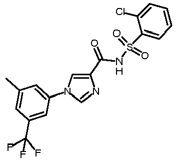 | Beispiel I-290: 1H-NMR(400.0 MHz, d6-DMSO): δ= 8.699(4.91);8.61(4.45);8.181(2.14);8.178(2.28);8.162(2.38);8.158(2.42);7.934(3.5);7.915(3.6 3);7.725(0.61);7.721(0.67);7.705(1.95);7.702(1.86);7.688(2.24);7.684(2.28);7.672(3.09);7.66 8(3.86);7.651(4.95);7.637(1.97);7.633(1.65);7.617(2.3);7.6(1.11);7.596(1.03);5.759(0.52);2.5 07(27.11);2.503(35);2.499(27.67);2.472(16);0(2.19) |
| I-291 | 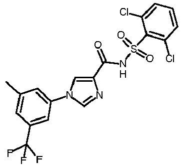 | Beispiel I-291: 1H-NMR(400.0 MHz, d6-DMSO): δ= 8.738(3.66);8.694(4.68);8.692(4.56);7.957(3.48);7.933(3.57);7.664(3.92);7.65(3.43);7.647(3. 9);7.628(8.05);7.607(0.56);7.585(3.01);7.568(2.14);7.562(1.82);7.545(1.14);2.672(0.37);2.50 6(43.72);2.502(53.9);2.474(16);2.329(0.4);2.076(1.23);0(16.33) |
| I-292 | 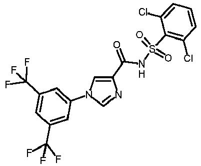 | Beispiel I-292: 1H-NMR(400.0 MHz, d6-DMSO): δ= 8.845(8.4);8.843(9.02);8.823(8.04);8.524(14.94);8.202(6.49);7.664(6.04);7.66(6.86);7.642(16 );7.601(6.25);7.584(4.24);7.578(3.29);7.561(2.19);2.672(0.64);2.668(0.48);2.507(80.93);2.50 3(100.42);2.499(75.03);2.334(0.51);2.33(0.65);2.326(0.5);2.075(4.76);0(1.28) |
| I-293 | 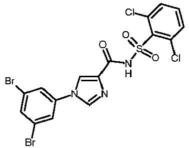 | Beispiel I-293: 1H-NMR(400.0 MHz, d6-DMSO): δ= 8.671(16);8.096(12.44);8.092(12.79);7.937(3.71);7.934(5.87);7.93(3.38);7.658(4.04);7.654(4. 86);7.636(10.78);7.606(0.41);7.595(4.17);7.578(2.8);7.572(2.23);7.556(1.43);2.672(0.38);2.5 07(50.21);2.503(62.81);2.498(46.96);2.334(0.35);2.33(0.42);2.325(0.33);2.076(7.97);0(1.46) |
| I-294 | 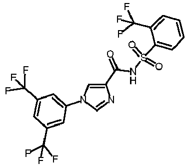 | Beispiel I-294: 1H-NMR(400.0 MHz, d6-DMSO): δ= 8.841(6.23);8.782(5.88);8.519(10.84);8.374(2.82);8.354(3.04);8.195(4.72);8.015(2.32);7.995( 3.41);7.978(1.23);7.961(2.82);7.943(2.19);7.926(2.43);7.908(2.51);7.89(0.89);2.671(0.34);2.5 02(83.24);2.329(0.76);2.075(16);0(1.48) |
| I-295 | 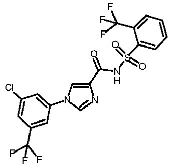 | Beispiel I-295: 1H-NMR(400.0 MHz, d6-DMSO): δ= 8.754(15.24);8.752(15.35);8.704(16);8.368(7.5);8.349(8.2);8.317(0.73);8.287(13.48);8.173(1 2.57);8.011(6.06);7.994(8.73);7.992(8.83);7.973(4.01);7.959(15.95);7.939(6.37);7.923(6.2);7. 904(6.4);7.886(2.09);7.807(0.43);7.722(1.32);4.373(0.33);4.342(0.35);4.327(0.36);4.307(0.37 );4.288(0.4);4.268(0.39);4.256(0.4);4.248(0.4);4.226(0.41);4.22(0.41);4.112(0.49);4.083(0.51 );4.074(0.54);4.045(0.55);4.027(0.62);4.01(0.63);3.99(0.61);3.94(0.66);3.852(0.71);3.774(0.7 4);3.762(0.74);3.737(0.75);3.718(0.76);3.673(0.78);3.624(0.74);3.615(0.74);3.584(0.73);3.57 5(0.74);3.568(0.72);3.506(0.78);3.444(0.65);3.423(0.62);3.409(0.6);3.394(0.6);3.387(0.59);3. 38(0.62);3.368(0.63);3.322(0.6);3.284(0.51);3.226(0.49);3.21(0.5);3.195(0.43);3.129(0.38);3. 089(0.34);3.051(0.32);2.676(2.29);2.671(2.94);2.667(2.21);2.649(0.33);2.507(329.4);2.502(4 12.18);2.498(301.44);2.333(2.07);2.329(2.7);2.325(2);2.184(0.33);2.075(1.18);1.356(2.21);1. 234(0.52);0.146(1.01);0.008(11.85);0(214.47);-0.008(10.2);-0.15(1.03) |
| I-296 | 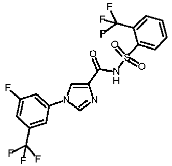 | Beispiel I-296: 1H-NMR(400.0 MHz, d6-DMSO): δ= 8.749(14.87);8.746(16);8.724(0.53);8.707(14.81);8.704(13.08);8.371(6.45);8.352(6.96);8.135 (5.27);8.13(3.47);8.111(5.32);8.106(3.45);8.076(10.35);8.017(5);8.015(5.11);7.999(7.37);7.9 95(7.64);7.981(2.43);7.978(2.64);7.962(6.71);7.959(5.74);7.943(5.38);7.939(4.29);7.927(5.4) ;7.909(5.58);7.89(1.89);7.822(4.79);7.801(4.85);7.726(0.46);5.759(2.29);2.678(0.4);2.673(0.6 );2.669(0.41);2.526(2.06);2.513(40.5);2.509(78.28);2.504(101.16);2.5(74.15);2.495(36.95);2. 335(0.57);2.331(0.74);2.326(0.57);2.077(0.51);1.366(0.37);0(1.73) |
| I-297 | 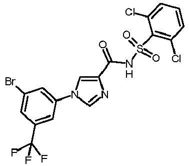 | Beispiel I-297: 1H-NMR(400.0 MHz, d6-DMSO): δ= 8.756(7.34);8.745(6.08);8.397(4.83);8.208(4.55);8.068(4.83);7.661(4.2);7.657(5.05);7.639(11 .65);7.598(4.55);7.581(3);7.575(2.37);7.558(1.57);2.672(0.39);2.507(46.02);2.503(59.01);2.4 99(44.73);2.33(0.37);2.075(16);0(1.58) |
| I-298 | 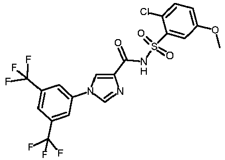 | Beispiel I-298: 1H-NMR(400.0 MHz, d6-DMSO): δ= 8.847(3.17);8.844(3.26);8.745(3.17);8.742(3.02);8.508(5.44);8.193(2.38);7.629(2.9);7.621(3. 05);7.609(0.58);7.58(2.77);7.558(3.25);7.53(0.4);7.474(0.33);7.3(1.68);7.292(1.61);7.278(1.4 6);7.27(1.39);3.863(16);3.84(0.59);3.817(1.91);2.507(26.65);2.503(34.55);2.498(26.04);2.075 (0.37);1.356(0.42);0(1.57) |
| I-299 | 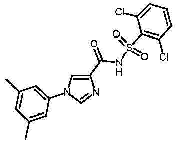 | Beispiel I-299: 1H-NMR(400.0 MHz, d6-DMSO): δ= 8.673(1.29);8.539(2.4);8.536(2.28);7.631(1.69);7.627(2.08);7.609(4.37);7.563(1.7);7.546(1.1 3);7.54(0.92);7.523(0.63);7.371(4);7.103(1.8);5.757(0.59);2.507(20.82);2.502(26.68);2.498(1 9.76);2.364(0.46);2.342(16);2.075(0.73);0(0.79) |
| I-300 | 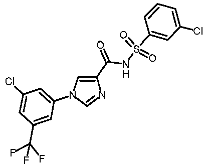 | Beispiel I-300: 1H-NMR(400.0 MHz, d6-DMSO): δ= 8.74(14.49);8.737(14.37);8.686(14.45);8.683(12.78);8.316(0.43);8.297(11.18);8.189(10.54);8 .014(11.23);8.009(7.44);7.971(6.19);7.95(16);7.816(4.11);7.814(4.66);7.812(4.46);7.796(5.96 );7.794(6.39);7.791(6.43);7.7(7.11);7.68(11.07);7.66(4.68);7.609(0.36);3.817(1.08);2.672(0.7 2);2.507(122.22);2.503(156.92);2.499(120.82);2.33(1.4);2.076(0.67);0(2.36) |
| I-301 | 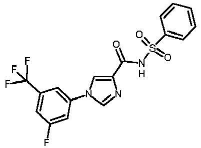 | Beispiel I-301: 1H-NMR(400.0 MHz, d6-DMSO): δ= 8.7(14.34);8.697(15.34);8.676(0.5);8.673(0.51);8.628(15.48);8.624(14.12);8.594(0.48);8.591( 0.44);8.317(0.37);8.131(2.47);8.126(4.67);8.121(2.99);8.107(2.43);8.102(4.66);8.097(3.01);8. 072(8.81);8.032(1.37);8.024(12.18);8.011(3.45);8.006(16);8.003(10.87);7.841(0.66);7.838(0. 7);7.822(0.71);7.817(0.8);7.797(4.1);7.776(4.11);7.735(1.31);7.732(2.46);7.729(1.59);7.72(1. 81);7.714(7.79);7.708(2.38);7.698(3.73);7.695(6.35);7.692(3.52);7.654(10.71);7.638(7.95);7. 635(15.2);7.621(2.8);7.617(6.11);7.605(0.52);7.602(0.6);7.592(0.49);7.587(1.13);7.568(0.7); 7.352(0.87);6.871(0.34);4.215(0.5);4.198(0.52);3.368(0.41);3.342(0.46);3.323(0.49);3.307(0. 39);3.28(0.4);3.257(0.41);3.227(0.36);3.212(0.42);3.194(0.45);2.681(0.42);2.676(0.81);2.672 (1.08);2.667(0.81);2.663(0.4);2.525(3.17);2.52(4.87);2.512(58.59);2.507(117.98);2.503(154.6 2);2.498(109.19);2.494(50.72);2.334(0.7);2.33(0.97);2.325(0.69);2.184(0.55);2.088(0.32);2.0 75(4.83);1.378(0.54);1.36(1.56);1.356(4.49);1.343(0.55);0.008(1.67);0(48.59);-0.009(1.33) |
| I-302 | 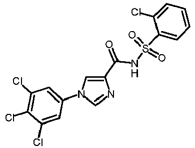 | Beispiel I-302: 1H-NMR(400.0 MHz, d6-DMSO): δ= 8.68(4.31);8.678(4.64);8.592(4.44);8.59(4.42);8.186(16);8.176(2.52);8.172(2.41);8.156(2.33) ;8.152(2.38);7.728(0.56);7.724(0.61);7.708(1.7);7.704(1.66);7.69(2.07);7.686(2.08);7.672(2.7 5);7.669(3.64);7.652(1.52);7.649(1.17);7.637(1.64);7.633(1.43);7.617(2.11);7.6(1.04);7.596( 0.97);2.507(33.39);2.503(44.69);2.498(34.27);2.329(0.36);0.008(1.43);0(48.35);-0.008(3.08) |
| I-303 | 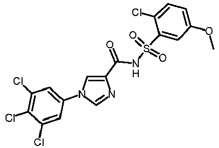 | Beispiel I-303: 1H-NMR(400.0 MHz, d6-DMSO): δ= 8.678(2.53);8.675(2.75);8.598(2.94);8.595(2.82);8.186(13.25);7.617(2.82);7.609(3.11);7.573( 2.54);7.551(3.12);7.293(1.48);7.285(1.44);7.271(1.3);7.263(1.26);3.858(16);3.816(0.87);2.52 5(0.42);2.511(14.82);2.507(30.52);2.502(40.54);2.498(29.57);2.493(14.56);2.075(10.37);0.00 8(1.6);0(48.45);-0.008(2.09) |
| I-304 | 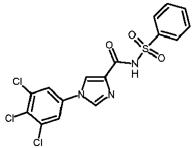 | Beispiel I-304: 1H-NMR(400.0 MHz, d6-DMSO): δ= 8.627(3.56);8.56(3.57);8.194(9.01);8.018(3.42);7.999(3.63);7.727(0.69);7.709(1.72);7.691(1. 5);7.65(2.59);7.631(3.5);7.612(1.49);2.672(0.4);2.503(43.81);2.075(16);0.822(0.34);0(24.23) |
| I-305 | 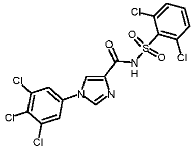 | Beispiel I-305: 1H-NMR(400.0 MHz, d6-DMSO): δ= 8.667(9.75);8.197(16);7.656(4.03);7.652(4.28);7.634(9.79);7.594(3.64);7.577(2.6);7.571(2.05 );7.554(1.29);2.503(59.31);2.33(0.57);2.075(0.87);0(26.27) |
| I-306 | 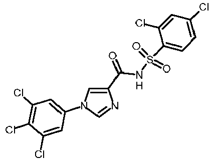 | Beispiel I-306: 1H-NMR(400.0 MHz, d6-DMSO): δ= 8.675(5.49);8.653(4.86);8.651(4.59);8.197(16);8.157(3.88);8.136(4.29);7.875(3.95);7.87(4.39 );7.724(2.64);7.719(2.63);7.703(2.31);7.698(2.35);2.507(42.1);2.503(53.9);2.499(43.58);2.33 (0.4);2.076(0.63);0(28.13) |
| I-307 | 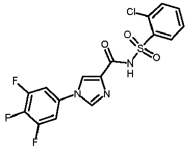 | Beispiel I-307: 1H-NMR(400.0 MHz, d6-DMSO): δ= 11.062(0.61);8.578(15.27);8.511(16);8.474(2.43);8.405(2.34);8.316(0.53);8.171(8.4);8.167(8. 79);8.151(9.23);8.148(9.21);7.989(0.68);7.969(0.91);7.963(0.91);7.943(8.32);7.927(9.16);7.9 2(8.95);7.905(7.94);7.885(0.76);7.719(2.56);7.702(7.15);7.685(7.85);7.681(8.24);7.664(14.03 );7.647(5.93);7.632(6.97);7.612(9.15);7.594(4.12);7.591(3.88);7.541(0.49);7.536(0.51);7.521 (0.63);7.374(0.44);7.368(0.52);7.36(0.51);7.353(0.52);7.347(0.5);7.34(0.52);7.332(0.47);7.07 5(0.85);7.058(0.84);5.756(0.33);4.262(0.46);4.244(0.46);3.844(0.35);3.761(0.36);3.695(0.39) ;3.673(0.4);3.625(0.43);3.602(0.43);3.365(0.4);3.349(0.39);2.671(2.38);2.506(275.11);2.502( 343.38);2.498(260.71);2.328(2.32);2.074(1.17);1.381(0.6);1.364(0.36);1.356(0.49);0.146(0.8 6);0(171.61);-0.15(0.84) |
| I-308 | 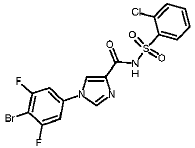 | Beispiel I-308: 1H-NMR(400.0 MHz, d6-DMSO): δ= 8.658(9.1);8.642(7.53);8.586(10.66);8.316(0.37);8.17(7.61);8.152(7.92);7.988(0.33);7.972(0. 36);7.887(6.78);7.867(6.68);7.7(6.49);7.682(7.84);7.666(11.7);7.646(5.92);7.63(6.28);7.611( 8.09);7.593(3.95);7.523(3.92);7.518(4.05);7.499(3.8);7.494(3.72);7.333(6.54);7.072(0.43);4.2 9(3.33);4.273(8.15);4.255(8.18);4.238(3.27);4.209(0.95);4.191(0.83);4.14(0.54);4.123(0.55); 4.067(0.57);4.022(0.58);3.971(0.61);3.813(0.73);3.772(0.74);3.76(0.74);3.662(0.78);3.639(0. 77);3.552(0.75);3.507(0.73);3.451(0.68);3.44(0.68);3.362(0.63);3.226(0.44);2.671(1.69);2.50 2(272.34);2.329(2.61);2.182(0.5);2.171(0.48);2.162(0.44);2.074(1);1.41(8.48);1.393(16);1.37 5(8.66);1.304(0.5);1.285(0.37);1.232(1.03);0.146(0.42);0(87.01);-0.15(0.64) |
| I-309 | 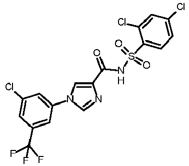 | Beispiel I-309: 1H-NMR(400.0 MHz, d6-DMSO): δ= 8.744(15.47);8.72(16);8.316(0.9);8.288(13.97);8.175(13.49);8.158(13.87);8.137(15.01);7.977 (2.15);7.964(13.56);7.868(12.95);7.864(13.13);7.853(1.48);7.848(1.22);7.721(9.53);7.716(8.1 6);7.699(7.73);7.694(7.06);7.643(0.68);7.638(0.6);7.622(0.58);7.617(0.54);4.296(0.36);4.289 (0.34);4.26(0.35);4.252(0.35);4.198(0.37);4.143(0.39);4.134(0.41);4.1(0.41);4.085(0.4);4.056 (0.49);4.052(0.42);4.038(0.62);4.02(0.65);4.003(0.5);3.991(0.44);3.963(0.42);3.953(0.42);3.8 99(0.43);3.89(0.41);3.873(0.42);3.871(0.41);3.824(0.41);3.81(0.42);3.793(0.4);3.776(0.4);3.7 37(0.4);3.728(0.38);3.708(0.39);3.682(0.37);3.676(0.37);3.656(0.37);3.64(0.36);3.629(0.37); 3.576(0.35);3.559(0.34);3.54(0.34);3.507(0.44);3.478(0.34);2.676(1.75);2.671(2.25);2.667(1. 72);2.506(283.08);2.502(352.01);2.498(261.23);2.376(0.37);2.333(1.93);2.329(2.41);2.075(0. 64);1.989(1.17);1.341(0.44);1.233(1.22);1.193(0.36);1.175(0.64);1.157(0.32);0.146(0.5);0.00 8(7.66);0(121.9);-0.15(0.65) |
| I-310 | 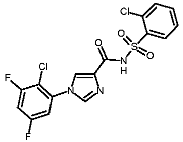 | Beispiel I-310: 1H-NMR(400.0 MHz, d6-DMSO): δ= 8.374(11.5);8.198(11.06);8.177(5.06);8.157(5.3);8.146(0.38);8.084(0.59);7.994(1.78);7.991(1 .9);7.972(1.93);7.841(1.83);7.834(2.01);7.818(3.37);7.811(3.4);7.795(1.88);7.788(1.81);7.73( 1.53);7.711(4.63);7.697(7.8);7.693(7.75);7.678(10.74);7.661(3.24);7.652(1.96);7.636(5.31);7. 62(6.71);7.603(8.79);7.587(1.48);7.552(0.32);7.54(1.27);7.536(1.35);7.52(1.8);7.519(1.84);7. 503(0.8);7.499(0.85);6.871(0.85);6.638(0.43);5.755(16);2.671(0.39);2.502(60.86);2.329(0.4); 2.183(1.3);2.074(0.48);1.97(1.07);1.356(8.33);1.234(0.39);1.221(0.56);1.202(0.78);1.184(0.4 3);0.019(1.61);0(45.53) |
| I-311 | 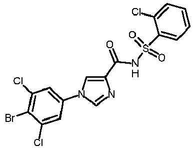 | Beispiel I-311: 1H-NMR(400.0 MHz, d6-DMSO): δ= 9.382(0.46);8.687(4.04);8.684(4.43);8.599(4.18);8.596(4.14);8.205(0.57);8.178(1.96);8.174(2 .12);8.158(2.35);8.154(2.62);8.145(16);7.838(0.43);7.739(2.23);7.729(0.6);7.725(0.6);7.708( 1.61);7.705(1.56);7.691(1.99);7.687(1.96);7.673(2.6);7.669(3.46);7.653(1.55);7.649(1.18);7.6 38(1.61);7.634(1.53);7.618(1.95);7.601(1);7.597(0.89);2.526(0.6);2.512(11.49);2.508(22.41); 2.504(29.08);2.5(21.23);2.495(10.58);2.076(6.09);0.008(0.76);0(17.2);-0.008(0.71) |
| I-312 | 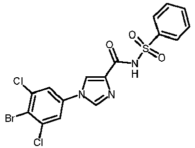 | Beispiel I-312: 1H-NMR(400.0 MHz, d6-DMSO): δ= 9.382(0.64);8.628(3.7);8.565(4);8.562(3.88);8.154(16);8.016(3.71);7.998(4.25);7.994(3.27);7 .741(3.05);7.727(0.73);7.709(2.09);7.69(1.65);7.649(3.05);7.63(4.21);7.612(1.6);3.39(0.34);3 .355(0.43);3.336(0.45);3.317(0.47);2.671(0.58);2.667(0.44);2.524(2.37);2.506(70.19);2.502(9 2.03);2.498(68.77);2.329(0.59);2.324(0.45);2.075(1.1);0.017(0.42);0.008(1.78);0(44.86);-0.008(2.23) |
| I-313 | 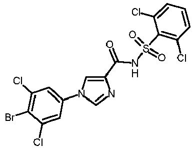 | Beispiel I-313: 1H-NMR(400.0 MHz, d6-DMSO): δ= 9.889(0.47);8.678(5.52);8.673(5.18);8.156(16);7.841(2.07);7.659(2.7);7.655(3.32);7.637(7.9) ;7.597(3.12);7.58(2.01);7.574(1.56);7.557(1.08);2.511(21.05);2.507(47.98);2.502(67.23);2.49 8(51.9);2.494(28.52);2.334(0.58);2.329(0.7);2.325(0.61);2.075(0.84);0(10.97) |
| I-314 | 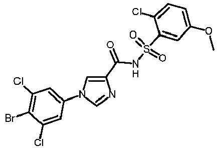 | Beispiel I-314: 1H-NMR(400.0 MHz, d6-DMSO): δ= 8.686(3.2);8.684(3.35);8.604(3.28);8.145(10.78);7.617(2.96);7.609(3.67);7.575(2.6);7.553(3. 34);7.53(0.54);7.48(0.45);7.473(0.48);7.296(1.57);7.288(1.56);7.273(1.39);7.266(1.36);6.746 (0.37);3.859(16);3.84(0.67);3.834(0.51);3.816(2.78);2.507(34.48);2.503(45.3);2.498(35.02);2 .33(0.37);2.075(7.66);0(7.16) |
| I-315 | 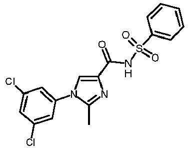 | Beispiel I-315: 1H-NMR(400.0 MHz, d6-DMSO): δ= 8.098(2.86);7.983(3.02);7.965(3.52);7.961(2.72);7.816(1.61);7.811(3.19);7.807(1.99);7.738(7 .78);7.734(7.15);7.687(0.46);7.669(1.5);7.651(1.28);7.614(2.39);7.595(3.33);7.577(1.31);3.40 5(1.22);2.676(0.58);2.671(0.79);2.667(0.58);2.541(0.6);2.524(1.89);2.511(48.22);2.507(94.36 );2.502(122.04);2.498(90.44);2.338(16);2.075(0.46);0.146(0.72);0.008(6.83);0(157.45);-0.008(6.77);-0.15(0.74) |
| I-316 | 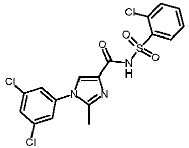 | Beispiel I-316: 1H-NMR(400.0 MHz, d6-DMSO): δ= 8.147(5.49);8.126(1.7);8.122(1.86);8.106(1.84);8.103(1.95);7.852(1.71);7.847(3.46);7.843(2. 21);7.781(8.29);7.777(7.64);7.639(0.42);7.636(0.46);7.619(1.33);7.616(1.32);7.602(1.96);7.5 96(2.73);7.591(3.18);7.576(1.06);7.564(1.38);7.56(1.13);7.544(1.59);7.528(0.75);7.523(0.68) ;5.757(2.83);2.507(36.42);2.502(48.75);2.498(37.81);2.38(16);2.329(0.4);2.086(8.8);1.234(2. 38);0.008(1.88);0(53.1);-0.008(3.26) |
| I-317 | 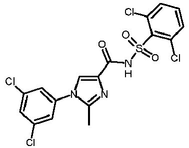 | Beispiel I-317: 1H-NMR(400.0 MHz, d6-DMSO): δ= 8.317(0.38);8.107(2.33);7.888(1.61);7.883(3.23);7.879(2.02);7.811(8.17);7.807(7.56);7.781(0 .38);7.777(0.33);7.527(2.88);7.525(3.3);7.506(6.66);7.446(2.2);7.429(1.66);7.424(1.51);7.407 (0.98);5.757(2.28);4.043(0.34);3.964(0.41);3.945(0.42);3.902(0.49);3.84(0.53);3.824(0.55);3. 808(0.57);3.79(0.57);3.766(0.57);3.749(0.58);3.729(0.57);3.684(0.55);3.524(0.38);3.465(0.32 );2.675(0.86);2.671(1.21);2.666(0.89);2.524(2.98);2.519(4.6);2.511(64.91);2.506(131.89);2.5 02(175.24);2.497(129.55);2.493(64.73);2.416(16);2.379(0.8);2.339(0.78);2.333(0.94);2.328(1 .26);2.324(0.93);2.075(2.76);1.233(0.51);0.146(1.01);0.008(7.42);0(212.52);-0.008(8.42);-0.15(1.01) |
| I-318 | 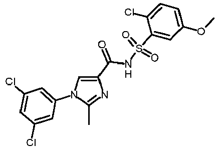 | Beispiel I-318: 1H-NMR(400.0 MHz, d6-DMSO): δ= 8.126(2.13);7.856(1.37);7.852(2.82);7.847(1.78);7.785(6.79);7.78(6.25);7.59(2.84);7.583(2.9 7);7.481(1.96);7.459(2.28);7.187(1.11);7.18(1.1);7.166(0.98);7.158(0.96);3.832(16);2.525(0. 41);2.52(0.68);2.511(11.73);2.507(24.37);2.502(32.61);2.498(24.42);2.494(12.4);2.385(12.4); 2.086(2.71);0.008(1.38);0(43.49);-0.008(1.93) |
| I-319 | 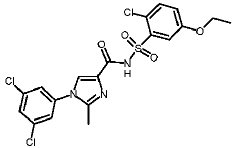 | Beispiel I-319: 1H-NMR(400.0 MHz, d6-DMSO): δ= 8.146(4.73);7.859(1.74);7.855(3.64);7.85(2.23);7.787(8.91);7.782(8.11);7.573(3.55);7.565(3. 71);7.47(2.68);7.449(3.14);7.177(1.49);7.169(1.47);7.155(1.32);7.147(1.3);4.121(1.28);4.104 (4.1);4.087(4.19);4.069(1.41);2.671(0.39);2.524(0.55);2.52(1.07);2.511(26.13);2.507(54.68); 2.502(73.02);2.498(53.95);2.493(27.02);2.385(16);2.333(0.46);2.329(0.58);2.324(0.46);2.086 |
| | | (11.15);1.373(4.5);1.355(9.92);1.338(4.54);0.146(0.41);0.008(3.12);0(103.31);-0.009(4.33);-0.025(0.37);-0.15(0.49) |
| I-320 | 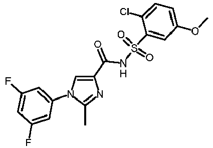 | Beispiel I-320: 1H-NMR(400.0 MHz, d6-DMSO): δ= 8.316(0.43);8.098(1.6);7.589(2.64);7.581(2.74);7.535(0.59);7.53(0.64);7.51(1.28);7.5(2.06);7 .496(1.98);7.479(3.6);7.456(2.22);7.184(1.11);7.177(1.04);7.162(0.97);7.155(0.9);3.83(16);3. 816(1.37);3.647(1.19);3.564(1.43);3.492(1.2);3.478(1.15);3.392(0.66);3.376(0.6);3.253(0.35) ;2.676(0.86);2.671(1.17);2.666(0.88);2.524(3.21);2.51(64.6);2.506(127.85);2.502(167.49);2.4 97(123.97);2.4(12.29);2.333(0.86);2.329(1.15);2.324(0.86);2.074(2.16);0.146(0.93);0.008(7.7 );0(203.6);-0.008(8.47);-0.064(0.39);-0.15(0.97) |
| I-321 | 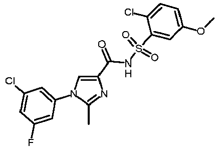 | Beispiel I-321: 1H-NMR(400.0 MHz, d6-DMSO): δ= 8.112(2.1);7.71(0.65);7.705(1.17);7.7(0.85);7.688(0.69);7.683(1.2);7.678(0.87);7.652(2.4);7. 636(1.02);7.63(1.28);7.626(0.72);7.612(1.19);7.608(1.42);7.602(0.79);7.59(2.81);7.582(2.94) ;7.479(2.18);7.472(0.4);7.457(2.36);7.186(1.27);7.178(1.2);7.164(1.14);7.156(1.04);3.831(16 );3.816(1.34);2.524(0.98);2.511(19.36);2.507(38.26);2.502(50.46);2.498(38.47);2.392(12.62); 2.329(0.37);2.075(10.87);0.008(2.56);0(59.35);-0.008(3.61) |
| I-322 | 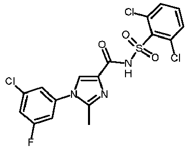 | Beispiel I-322: 1H-NMR(400.0 MHz, d6-DMSO): δ= 8.104(3.86);7.744(0.86);7.739(1.5);7.734(1.07);7.722(0.9);7.717(1.55);7.712(1.09);7.683(2.9 8);7.661(1.22);7.656(1.6);7.651(0.92);7.638(1.19);7.633(1.61);7.628(1.02);7.529(2.97);7.527 (3.43);7.508(6.66);7.449(2.33);7.432(1.78);7.427(1.61);7.409(1.03);2.507(36.76);2.502(48.4) ;2.498(37.03);2.427(16);2.329(0.4);0.008(2.55);0(58.15);-0.008(3.42) |
| I-323 | 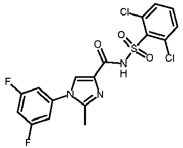 | Beispiel I-323: 1H-NMR(400.0 MHz, d6-DMSO): δ= 8.11(5.36);7.575(0.82);7.552(1.94);7.547(1.91);7.532(7.41);7.513(10.4);7.455(2.34);7.437(1. 99);7.415(1.06);2.672(0.43);2.502(69.02);2.44(16);2.329(0.53);2.075(1.86);0(60.18);-0.15(0.35) |
| I-324 | 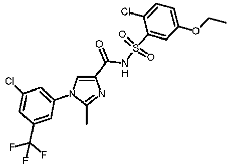 | Beispiel I-324: 1H-NMR(400.0 MHz, d6-DMSO): δ= 8.211(6.62);8.128(3.09);8.124(2.42);8.112(2.85);8.052(2.83);7.578(3.64);7.571(3.76);7.477(2 .88);7.455(3.36);7.184(1.64);7.176(1.6);7.162(1.43);7.154(1.38);4.125(1.22);4.107(4.08);4.09 (4.15);4.072(1.31);2.512(11.61);2.507(24.42);2.503(32.9);2.498(24.87);2.494(12.92);2.392(1 6);1.374(4.54);1.357(9.81);1.34(4.64);0.008(0.37);0(15.85);-0.008(0.82) |
| I-325 | 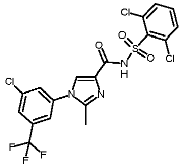 | Beispiel I-325: 1H-NMR(400.0 MHz, d6-DMSO): δ= 8.18(5.41);8.147(6.12);8.086(3.1);7.535(3.07);7.533(3.44);7.514(7.02);7.455(2.37);7.438(1.7 8);7.433(1.61);7.416(1.04);4.226(0.33);4.181(0.36);4.097(0.44);4.074(0.46);4.061(0.47);4.01 4(0.51);3.99(0.51);3.932(0.52);3.907(0.51);3.835(0.5);3.741(0.36);2.676(0.58);2.671(0.76);2. 667(0.59);2.506(77.45);2.502(100.14);2.498(75.17);2.423(16);2.333(0.5);2.328(0.77);2.086(1 .87);0.146(0.72);0.008(7.46);0(138.09);-0.008(7.15);-0.15(0.66) |
| I-326 | 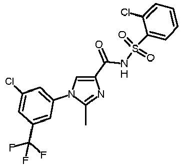 | Beispiel I-326: 1H-NMR(400.0 MHz, d6-DMSO): δ= 8.213(6.04);8.128(3.11);8.124(3.25);8.119(2.43);8.108(4.13);8.044(2.78);7.647(0.34);7.643(0 .36);7.626(1.2);7.623(1.15);7.61(1.8);7.606(2.05);7.602(2.38);7.597(3.07);7.582(1.03);7.578( 0.65);7.571(1.32);7.566(1.04);7.551(1.5);7.547(1.21);7.534(0.76);7.53(0.68);2.525(0.53);2.51 1(18.69);2.507(38.34);2.502(51.28);2.498(38.88);2.494(20.65);2.387(16);2.334(0.33);2.329(0 .43);2.325(0.35);2.086(0.38);0.146(0.33);0.008(2.47);0(74.23);-0.008(4.4);-0.15(0.39) |
| I-327 | 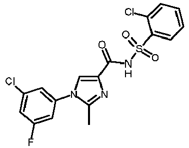 | Beispiel I-327: 1H-NMR(400.0 MHz, d6-DMSO): δ= 8.146(5.75);8.128(1.73);8.124(1.83);8.108(1.84);8.105(1.86);7.709(0.91);7.704(1.57);7.698(1 .1);7.687(0.91);7.682(1.59);7.677(1.13);7.652(3.09);7.636(1.58);7.631(1.74);7.625(1.47);7.62 2(1.53);7.618(1.43);7.613(1.56);7.605(2.67);7.602(2.77);7.598(2.66);7.593(3.08);7.578(0.97) ;7.574(0.62);7.566(1.29);7.562(1.02);7.547(1.51);7.542(1.17);7.53(0.71);7.525(0.64);2.525(0. 41);2.511(10.57);2.507(21.16);2.503(27.78);2.498(20.9);2.391(16);2.075(0.91);0.008(1.31);0 (38.59);-0.008(2.08) |
| I-328 | 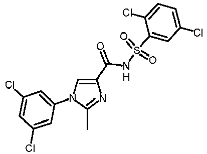 | Beispiel I-328: 1H-NMR(400.0 MHz, d6-DMSO): δ= 8.111(5.05);8.021(3.37);8.015(3.48);7.905(1.82);7.9(3.57);7.896(2.17);7.818(8.71);7.814(8.1 1);7.797(0.45);7.706(0.34);7.702(0.39);7.618(1.29);7.612(1.25);7.597(2.39);7.59(2.4);7.55(4. 38);7.529(2.28);2.676(0.39);2.671(0.54);2.667(0.41);2.524(1.48);2.511(33.39);2.506(66.19);2 .502(86.52);2.498(64.7);2.493(33.34);2.432(16);2.334(0.54);2.329(0.64);2.325(0.46);2.075(0. 48);0.146(0.56);0.008(4.92);0(123.6);-0.008(6.25);-0.15(0.58) |
| I-329 | 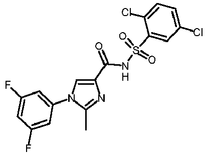 | Beispiel I-329: 1H-NMR(400.0 MHz, d6-DMSO): δ= 8.109(6.8);8.025(3.19);8.019(3.41);7.624(1.3);7.617(1.29);7.602(2.49);7.596(2.89);7.57(1.59) ;7.565(1.34);7.555(4.88);7.547(1.48);7.54(3.3);7.534(4.75);7.522(3.13);2.507(22.77);2.502(3 0.06);2.498(23.99);2.456(16);2.075(7.79);0.008(1.52);0(34.98) |
| I-330 | 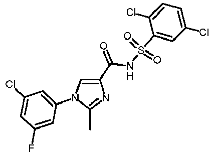 | Beispiel I-330: 1H-NMR(400.0 MHz, d6-DMSO): δ= 8.115(7.15);8.025(3.34);8.018(3.46);7.797(0.35);7.764(0.92);7.759(1.58);7.754(1.09);7.742(0 .94);7.737(1.61);7.732(1.11);7.701(1.72);7.696(2.93);7.668(1.17);7.663(1.59);7.658(0.88);7.6 46(1.17);7.64(1.63);7.635(0.9);7.622(1.33);7.615(1.28);7.6(2.42);7.594(2.42);7.554(4.5);7.53 |
| | | 2(2.37);2.525(0.48);2.511(10.61);2.507(21.51);2.503(28.48);2.498(21.28);2.494(10.87);2.446 (16);2.075(2.48);0.008(1.38);0(40.01);-0.008(1.99) |
| I-331 | 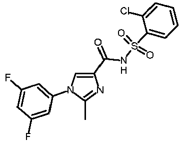 | Beispiel I-331: 1H-NMR(400.0 MHz, d6-DMSO): δ= 8.123(2.28);8.118(6.32);8.104(1.87);8.101(1.88);7.635(0.44);7.632(0.46);7.615(1.33);7.611(1 .33);7.598(1.98);7.592(2.66);7.587(3.1);7.572(1.02);7.567(0.62);7.561(1.36);7.556(1.03);7.54 1(1.64);7.537(1.53);7.531(0.88);7.524(1.27);7.52(0.94);7.512(1.06);7.506(1.79);7.498(2.73); 7.493(2.41);7.478(3.54);2.542(0.84);2.525(0.49);2.511(11.7);2.507(22.9);2.503(29.65);2.498( 21.83);2.494(10.96);2.397(16);0.008(1.8);0(41.22);-0.008(1.81) |
| I-332 | 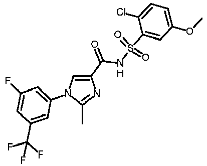 | Beispiel I-332: 1H-NMR(400.0 MHz, d6-DMSO): δ= 8.162(5.17);7.966(2.38);7.944(2.67);7.924(2.86);7.614(0.56);7.593(2.94);7.585(3.06);7.553(0 .44);7.531(0.35);7.479(2.63);7.457(2.82);7.185(1.59);7.177(1.5);7.163(1.41);7.155(1.29);3.83 2(16);3.816(2.1);2.503(33.08);2.499(26.75);2.397(13.5);2.34(0.33);2.329(0.36);2.075(4.41);0 (0.73) |
| I-333 | 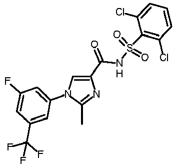 | Beispiel I-333: 1H-NMR(400.0 MHz, d6-DMSO): δ= 8.179(4.96);8.002(2.5);7.98(2.2);7.971(3.76);7.538(3.23);7.535(3.44);7.516(6.74);7.458(2.27) ;7.44(1.73);7.436(1.51);7.418(0.93);2.671(0.41);2.507(51.25);2.502(66.98);2.498(51.38);2.43 3(16);2.334(0.37);2.329(0.48);0(7.86);-0.063(0.56) |
| I-334 | 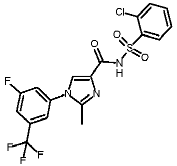 | Beispiel I-334: 1H-NMR(400.0 MHz, d6-DMSO): δ= 8.217(6.29);8.134(1.67);8.13(1.81);8.114(1.84);8.111(1.92);7.971(2.51);7.948(2.78);7.927(2. 88);7.65(0.37);7.646(0.39);7.63(1.29);7.627(1.21);7.613(1.9);7.609(2.19);7.605(2.5);7.601(3. 19);7.586(1.03);7.581(0.66);7.574(1.33);7.569(1.05);7.554(1.59);7.537(0.75);7.533(0.69);5.7 58(5.97);2.542(26.85);2.525(0.5);2.507(33.05);2.503(43.37);2.498(32.81);2.398(16);2.33(0.3 5);0.008(1.02);0(30.91) |
| I-335 | 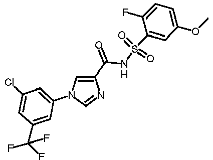 | Beispiel I-335: 1H-NMR(400.0 MHz, d6-DMSO): δ= 8.741(2.14);8.679(2.43);8.677(2.39);8.283(2.22);8.169(2.04);7.96(2.05);7.422(1);7.414(1.23) ;7.408(1.19);7.4(1.75);7.378(1.55);7.354(1.1);7.314(0.63);7.305(1.07);7.297(0.67);7.292(0.51 );7.283(0.61);7.274(0.34);3.83(16);3.795(0.36);3.646(0.64);3.441(1.89);2.676(0.68);2.672(0. 95);2.667(0.73);2.542(1.71);2.525(2.03);2.52(3.44);2.511(53.35);2.507(109.91);2.502(145.72 );2.498(108.59);2.494(55.2);2.334(0.75);2.329(1.01);2.325(0.79);0.008(0.5);0(15.79);-0.008(0.65) |
| I-336 | 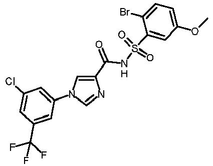 | Beispiel I-336: 1H-NMR(400.0 MHz, d6-DMSO): δ= 8.768(2.98);8.672(3.16);8.67(3.1);8.278(2.59);8.16(2.37);7.963(2.43);7.738(2.64);7.717(2.93) ;7.666(2.76);7.659(2.87);7.213(1.45);7.206(1.41);7.191(1.35);7.184(1.3);5.757(0.49);3.856(1 6);3.814(0.82);2.672(0.39);2.507(47.54);2.503(62);2.498(46.74);2.334(0.36);2.329(0.46);2.32 5(0.36) |
| I-337 | 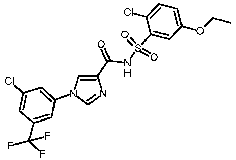 | Beispiel I-337: 1H-NMR(400.0 MHz, d6-DMSO): δ= 8.754(5.41);8.663(6.43);8.278(5.44);8.162(5.07);7.959(5.08);7.601(5.87);7.594(6);7.553(4.69 );7.531(5.65);7.272(2.76);7.264(2.68);7.25(2.45);7.242(2.33);5.757(0.57);4.15(2.31);4.133(7. 09);4.116(7.21);4.098(2.5);4.086(0.62);4.069(0.55);4.052(0.4);4.028(0.34);3.962(0.4);3.948( 0.42);3.931(0.44);3.911(0.45);3.857(0.52);3.667(0.78);3.633(0.79);3.618(0.8);3.602(0.8);3.56 6(0.81);3.479(0.75);3.463(0.7);3.31(0.45);3.299(0.42);3.269(0.4);2.676(0.65);2.672(0.88);2.6 68(0.65);2.507(103.12);2.503(131.52);2.498(97.87);2.334(0.81);2.329(1.02);2.325(0.79);2.07 5(0.37);1.382(7.61);1.365(16);1.347(7.51);1.321(0.35);0.146(0.39);0.008(3.72);0(85.73);-0.008(4.34);-0.058(0.38);-0.15(0.45) |
| I-338 | 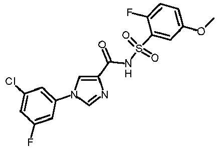 | Beispiel I-338: 1H-NMR(400.0 MHz, d6-DMSO): δ= 8.654(3.03);8.651(3.46);8.611(3.18);8.608(2.98);7.838(2.37);7.801(0.81);7.796(1.33);7.79(0. 74);7.776(0.83);7.771(1.34);7.766(0.74);7.565(0.84);7.56(1.28);7.556(0.79);7.544(0.87);7.53 9(1.29);7.534(0.75);7.423(1.1);7.415(1.38);7.409(1.39);7.401(1.57);7.381(2.04);7.357(1.52); 7.318(0.86);7.308(1.39);7.3(0.85);7.295(0.6);7.286(0.79);7.277(0.42);5.757(0.58);3.831(16); 3.794(0.63);2.509(15.5);2.504(20.8);2.5(15.97);0.008(0.76);0(29.87) |
| I-339 | 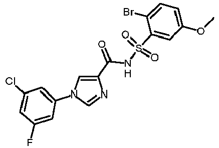 | Beispiel I-339: 1H-NMR(400.0 MHz, d6-DMSO): δ= 8.676(3.17);8.673(3.48);8.6(3.3);8.598(3.32);7.832(2.65);7.799(0.89);7.794(1.4);7.774(0.9);7 .769(1.4);7.737(2.7);7.715(2.99);7.663(2.9);7.656(2.96);7.57(0.88);7.565(1.33);7.548(0.9);7. 544(1.32);7.212(1.53);7.204(1.49);7.19(1.41);7.183(1.35);3.855(16);3.815(0.52);2.507(31.15) ;2.503(40.49);2.499(31.48);2.076(0.65);0.008(1.52);0(30.93) |
| I-340 | 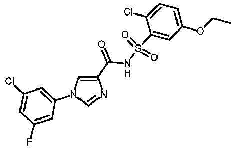 | Beispiel I-340: 1H-NMR(400.0 MHz, d6-DMSO): δ= 20.008(0.51);8.316(5.08);5.92(0.55);3.621(0.51);3.541(0.75);3.501(0.66);3.467(0.79);3.454(0 .86);3.428(0.98);3.329(1735.33);2.938(0.51);2.88(0.67);2.845(0.56);2.671(16);2.506(1891.72 );2.502(2420.97);2.499(1964.31);2.329(15.21);0.146(7.6);0(1523.4);-0.058(1.3);-0.149(7.58) |
| I-341 | 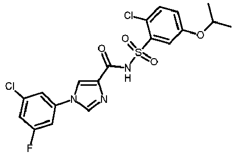 | Beispiel I-341: 1H-NMR(400.0 MHz, d6-DMSO): δ= 8.676(3.22);8.674(3.43);8.596(3.42);8.593(3.25);7.835(2.46);7.799(0.84);7.794(1.39);7.789(0 .78);7.775(0.85);7.77(1.4);7.764(0.77);7.586(2.82);7.578(2.99);7.564(0.89);7.56(1.31);7.555( 0.82);7.543(1);7.537(3.89);7.515(3.22);7.266(1.55);7.258(1.5);7.243(1.34);7.236(1.3);4.724( 0.41);4.709(1.08);4.694(1.48);4.679(1.12);4.664(0.46);2.508(22);2.503(30.27);2.499(23.58);1 .319(15.74);1.304(16);0(13.79);-0.008(0.84) |
| I-342 | 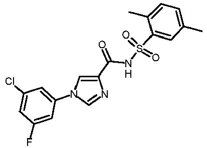 | Beispiel I-342: 1H-NMR(400.0 MHz, d6-DMSO): δ= 8.609(4.86);8.55(5.03);7.846(3.97);7.83(3.88);7.791(1.27);7.786(1.98);7.782(1.15);7.767(1.3) ;7.762(1.98);7.757(1.12);7.55(1.31);7.546(1.93);7.528(1.36);7.524(1.93);7.393(1.64);7.375(2. 28);7.282(3.43);7.263(2.41);5.758(0.45);2.56(16);2.527(1.38);2.508(27.71);2.504(34.57);2.5( 26);2.369(15.3);2.326(0.75);2.076(1.13);0(8.34) |
| I-343 | 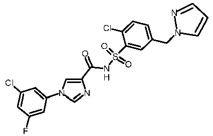 | Beispiel I-343: 1H-NMR(400.0 MHz, d6-DMSO): δ= 8.647(6.33);8.595(6.7);8.592(6.55);8.317(0.42);8.042(5.27);8.036(5.54);7.904(5.57);7.899(5. 55);7.831(5.25);7.795(1.83);7.79(2.99);7.784(1.67);7.77(1.86);7.765(3.01);7.76(1.68);7.635( 4.85);7.614(6.25);7.565(1.81);7.56(2.75);7.556(1.72);7.544(1.88);7.539(2.8);7.534(1.65);7.51 3(5.18);7.509(5.39);7.469(2.95);7.464(3.04);7.449(2.36);7.443(2.43);6.315(3.97);6.31(6.71); 6.305(4.1);5.49(16);3.96(0.32);3.83(0.51);3.768(0.57);3.717(0.68);3.587(0.86);3.573(0.87);3. 543(0.87);3.463(0.77);3.415(0.68);3.357(0.57);3.297(0.45);3.268(0.41);2.676(0.87);2.671(1.2 4);2.667(0.94);2.542(11.93);2.524(2.8);2.511(68.4);2.507(139.65);2.502(186.3);2.498(141.15 );2.333(1.04);2.329(1.4);2.325(1.09);2.075(2.89);0.146(0.43);0.008(3.95);0(105.04);-0.008(5.12);-0.15(0.51) |
| I-344 | 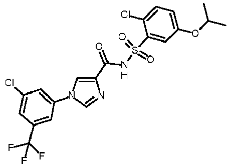 | Beispiel I-344: 1H-NMR(400.0 MHz, d6-DMSO): δ= 8.757(1.71);8.66(2.53);8.282(2.55);8.165(2.32);7.955(2.29);7.587(2.71);7.58(2.9);7.531(1.79) ;7.508(2.11);7.258(1.03);7.25(1.03);7.236(0.9);7.228(0.89);4.723(0.4);4.709(1.01);4.694(1.37 );4.678(1.03);4.664(0.42);2.676(0.41);2.671(0.57);2.667(0.42);2.542(0.4);2.525(1.29);2.511( 31.6);2.507(65.29);2.502(87.8);2.498(66.99);2.334(0.44);2.329(0.61);2.325(0.47);2.075(0.47) ;1.32(15.99);1.304(16);0.008(1.19);0(38.45);-0.008(1.98) |
| I-345 | 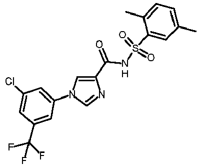 | Beispiel I-345: 1H-NMR(400.0 MHz, d6-DMSO): δ= 8.695(2.8);8.615(4.28);8.317(0.35);8.273(4.19);8.159(3.89);7.942(3.75);7.844(3.72);7.388(1. 44);7.369(1.98);7.278(2.88);7.258(2.06);3.332(1.45);2.891(0.35);2.676(0.78);2.671(1.05);2.6 67(0.8);2.56(16.81);2.542(0.64);2.524(2.97);2.507(115.31);2.502(150.78);2.498(113.21);2.36 |
| | | 8(16);2.333(0.8);2.329(1.04);2.325(0.81);0.146(0.39);0.008(3.47);0(89.58);-0.008(3.98);-0.15(0.42) |
| I-346 | 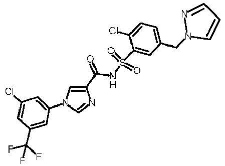 | Beispiel I-346: 1H-NMR(400.0 MHz, d6-DMSO): δ= 8.755(6.69);8.752(7.25);8.673(6.95);8.67(6.72);8.277(5.54);8.161(5.1);8.051(5.31);8.046(5.6 2);7.959(5.19);7.905(5.46);7.9(5.66);7.639(5.14);7.619(6.64);7.514(5.31);7.511(5.55);7.476( 3.07);7.47(3.14);7.455(2.45);7.45(2.54);6.317(3.97);6.312(6.69);6.307(4.2);5.494(16);2.672( 0.44);2.542(1);2.525(0.34);2.521(0.89);2.512(29.39);2.507(62.85);2.503(85.79);2.498(66.24); 2.334(0.52);2.33(0.69);2.325(0.56);2.076(0.62);0.008(1.21);0(56.73);-0.008(3.22) |
| I-347 | 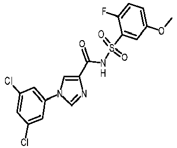 | Beispiel I-347: 1H-NMR(400.0 MHz, d6-DMSO): δ= 8.662(2.64);8.659(3.02);8.611(2.85);8.608(2.75);7.952(6.16);7.947(6.68);7.711(1.45);7.707(2 .7);7.703(1.52);7.419(1.03);7.411(1.29);7.405(1.35);7.402(1.3);7.398(1.4);7.378(1.85);7.355( 1.37);7.315(0.77);7.306(1.28);7.298(0.79);7.293(0.57);7.284(0.74);7.275(0.4);3.83(16);2.52( 0.43);2.511(15.64);2.507(33.69);2.503(46.24);2.498(36.08);2.494(19.65);2.329(0.38);0.008(0 .62);0(28.15);-0.008(1.66) |
| I-348 | 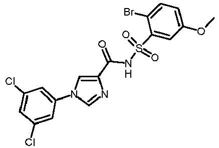 | Beispiel I-348: 1H-NMR(400.0 MHz, d6-DMSO): δ= 8.685(3.27);8.598(3.52);7.946(6.64);7.942(7.24);7.735(2.56);7.713(4.6);7.71(4.04);7.661(2.7 4);7.654(2.85);7.21(1.43);7.202(1.43);7.188(1.34);7.18(1.3);3.854(16);3.712(0.33);3.705(0.3 3);3.669(0.41);3.647(0.35);3.618(0.34);3.596(0.35);3.553(0.34);3.52(0.34);3.507(0.33);3.502 (0.33);2.672(0.56);2.542(1.49);2.502(90.69);2.498(72.7);2.329(0.65);0(31.85) |
| I-349 | 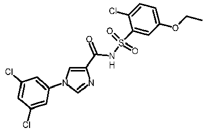 | Beispiel I-349: 1H-NMR(400.0 MHz, d6-DMSO): δ= 8.68(8.51);8.596(8.59);8.002(0.33);7.944(15.95);7.707(6.91);7.598(6);7.591(6.61);7.555(5.11 );7.533(6.09);7.51(0.36);7.274(3.34);7.267(3.53);7.252(3.08);7.245(3.07);4.149(2.66);4.132( 7.66);4.115(7.97);4.098(3.19);4.069(0.71);4.053(0.49);4.009(0.35);3.996(0.35);3.963(0.35);3. 948(0.35);3.932(0.34);2.672(0.41);2.503(93.39);2.329(0.9);1.382(7.73);1.364(16);1.347(8.59) ;1.232(0.41);1.185(0.33);0(6.98) |
| I-350 | 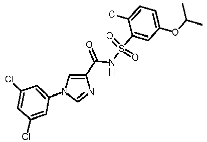 | Beispiel I-350: 1H-NMR(400.0 MHz, d6-DMSO): δ= 8.672(1.55);8.588(2.5);7.95(7.06);7.946(7.34);7.706(1.63);7.702(2.78);7.698(1.68);7.582(2.6 4);7.574(2.82);7.528(1.61);7.506(1.89);7.255(0.95);7.248(1.01);7.233(0.89);7.226(0.87);5.75 7(0.58);4.721(0.4);4.706(1);4.691(1.34);4.676(1.03);4.661(0.42);2.672(0.67);2.667(0.51);2.5 42(28.24);2.507(80.26);2.502(103.69);2.498(77.83);2.334(0.55);2.329(0.72);2.325(0.56);1.31 8(16);1.303(15.97);0.146(0.49);0.008(4.65);0(105.64);-0.008(5.45);-0.15(0.52) |
| I-351 | 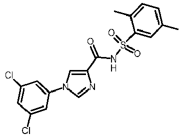 | Beispiel I-351: 1H-NMR(400.0 MHz, d6-DMSO): δ= 8.624(4.78);8.622(5.23);8.552(5.39);8.549(5.4);7.943(10);7.939(10.66);7.844(4.05);7.697(2.7 1);7.693(4.59);7.689(2.69);7.661(0.56);7.393(1.74);7.374(2.4);7.301(0.97);7.282(3.69);7.263 (2.51);7.252(0.65);3.586(0.34);3.552(0.41);3.365(1.14);3.343(1.15);2.672(0.6);2.558(16.79); 2.527(3.66);2.507(59.14);2.503(75.2);2.499(58.2);2.368(16);2.325(2.39);2.076(0.86);0(7.7) |
| I-352 | 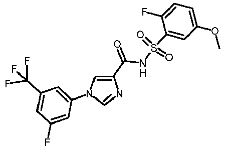 | Beispiel I-352: 1H-NMR(400.0 MHz, d6-DMSO): δ= 8.738(4.04);8.681(3.93);8.13(1.78);8.106(1.66);8.071(3.31);7.818(1.65);7.798(1.61);7.425(1. 9);7.417(2.34);7.411(2.42);7.404(2.4);7.382(2.37);7.358(1.65);7.318(1.5);7.31(1.95);7.301(1. 3);7.287(1.11);7.279(0.56);3.832(16);3.794(0.79);2.504(47.2);2.33(0.37);0(3.22) |
| I-353 | 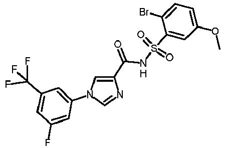 | Beispiel I-353: 1H-NMR(400.0 MHz, d6-DMSO): δ= 8.757(3.49);8.67(3.52);8.128(1.32);8.103(1.33);8.06(2.74);7.822(1.27);7.801(1.31);7.739(2.5 9);7.717(2.86);7.667(2.92);7.66(3.14);7.214(1.48);7.206(1.52);7.192(1.43);7.184(1.42);3.856 (16);3.815(0.44);2.503(48.5);2.499(39.97);2.33(0.37);0(4.43) |
| I-354 | 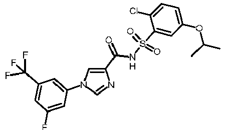 | Beispiel I-354: 1H-NMR(400.0 MHz, d6-DMSO): δ= 8.758(3.44);8.668(3.5);8.665(3.44);8.129(1.27);8.105(1.29);8.066(2.64);7.817(1.21);7.795(1. 24);7.59(2.83);7.583(2.99);7.538(2.59);7.516(3.05);7.266(1.54);7.259(1.51);7.244(1.33);7.23 7(1.29);4.727(0.43);4.712(1.11);4.697(1.51);4.682(1.13);4.666(0.46);2.508(28.28);2.504(36.8 9);2.5(28.73);1.321(16);1.306(15.95);0(3.46) |
| I-355 | 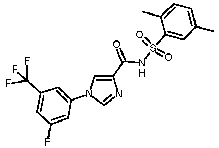 | Beispiel I-355: 1H-NMR(400.0 MHz, d6-DMSO): δ= 8.696(4.57);8.693(4.92);8.647(0.33);8.644(0.36);8.622(5.29);8.619(5.06);8.571(0.32);8.119(1 .78);8.094(1.8);8.061(3.63);7.849(3.8);7.803(1.67);7.782(1.7);7.394(1.6);7.375(2.22);7.284(3 .34);7.264(2.37);3.52(0.42);3.351(0.92);2.677(0.37);2.672(0.51);2.668(0.4);2.562(16);2.526( 1.82);2.508(52.66);2.503(68.07);2.499(51.33);2.37(15.28);2.326(0.69);2.076(1.71);0(7.38) |
| I-356 | 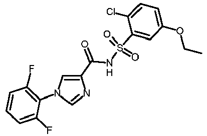 | Beispiel I-356: 1H-NMR(400.0 MHz, d6-DMSO): δ= 8.368(6.03);8.214(5.54);7.683(0.53);7.667(1.21);7.662(1.13);7.646(2.44);7.629(1.27);7.624(1 .67);7.608(0.97);7.601(5.88);7.594(6.2);7.567(5.65);7.545(6.62);7.459(4.21);7.438(7.15);7.41 7(3.24);7.286(3.32);7.278(3.25);7.264(2.91);7.256(2.85);4.149(2.19);4.132(7.15);4.115(7.27) ;4.097(2.38);2.672(0.33);2.507(43.31);2.503(57.64);2.498(44.61);2.33(0.43);2.326(0.35);2.07 6(1.69);1.381(7.61);1.364(16);1.346(7.57);0.008(1.29);0(36.55);-0.008(2.06) |
| 1-357 | 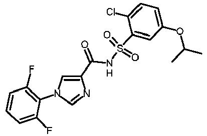 | Beispiel 1-357: 1H-NMR(400.0 MHz, d6-DMSO): δ= 8.374(2.6);8.216(2.56);8.214(2.5);7.667(0.58);7.662(0.52);7.651(0.43);7.646(1.16);7.64(0.52) ;7.629(0.56);7.624(0.78);7.608(0.35);7.584(2.79);7.577(2.99);7.55(2.62);7.528(3.09);7.46(1.9 6);7.439(3.35);7.418(1.49);7.278(1.48);7.27(1.46);7.255(1.28);7.248(1.27);5.758(0.71);4.724 (0.4);4.709(1.05);4.694(1.44);4.679(1.08);4.664(0.43);2.542(0.79);2.508(22.92);2.503(30.83) ;2.499(23.32);1.318(15.97);1.303(16);0.008(0.38);0(17.45);-0.008(1.03) |
| I-358 | 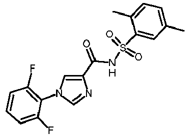 | Beispiel I-358: 1H-NMR(400.0 MHz, d6-DMSO): δ= 8.297(4.06);8.183(3.86);8.18(3.91);7.846(3.71);7.675(0.43);7.659(0.93);7.653(0.83);7.643(0. 69);7.638(1.88);7.632(0.83);7.621(0.91);7.616(1.26);7.6(0.56);7.45(3.17);7.429(5.3);7.408(2. 47);7.4(2.04);7.38(2.16);7.377(2.22);7.288(3.45);7.269(2.45);2.569(16);2.526(0.74);2.512(10 .97);2.508(22.24);2.503(29.64);2.499(22.44);2.369(15.36);2.326(0.34);2.076(1.54);0.008(0.8 2);0(21.75);-0.008(0.89) |
| I-359 | 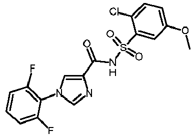 | Beispiel I-359: 1H-NMR(400.0 MHz, d6-DMSO): δ= 8.369(2.94);8.216(2.66);7.667(0.61);7.662(0.59);7.646(1.22);7.623(3.38);7.615(3.23);7.588(2 .71);7.566(3.16);7.46(2.01);7.438(3.41);7.418(1.55);7.308(1.65);7.3(1.63);7.285(1.45);7.278( 1.42);3.861(16);2.508(17.87);2.503(24.23);2.499(19.49);2.076(0.38);0.008(0.38);0(12.68) |
| I-360 | 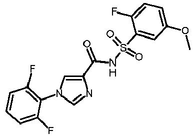 | Beispiel 1-360: 1H-NMR(400.0 MHz, d6-DMSO): δ= 8.337(1.77);8.21(2.08);7.666(0.62);7.661(0.52);7.65(0.39);7.645(1.09);7.629(0.53);7.623(0.7 4);7.458(1.85);7.437(3.16);7.42(1.33);7.412(2.75);7.407(1.56);7.398(1.35);7.389(1.73);7.365 (1.21);7.325(0.7);7.316(1.16);7.307(0.72);7.303(0.54);7.294(0.68);7.285(0.36);3.832(16);3.7 93(0.41);2.672(0.35);2.524(0.66);2.511(20.59);2.507(42.62);2.502(57.29);2.498(43.89);2.329 (0.4);0.008(1.03);0(34.15);-0.008(1.72) |
| I-361 | 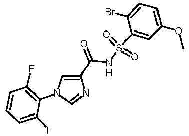 | Beispiel I-361: 1H-NMR(400.0 MHz, d6-DMSO): δ= 8.38(2.7);8.222(2.47);8.22(2.45);7.749(2.89);7.727(3.19);7.668(3.48);7.661(3.4);7.653(0.5);7 .647(1.17);7.642(0.51);7.63(0.55);7.626(0.77);7.61(0.34);7.461(1.94);7.44(3.27);7.419(1.46); 7.225(1.61);7.217(1.57);7.203(1.49);7.195(1.46);3.857(16);2.525(0.33);2.512(7.5);2.508(15.4 3);2.503(20.57);2.499(15.46);2.494(7.92);0.008(0.48);0(15.06);-0.008(0.66) |
| I-362 | 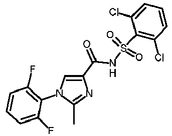 | Beispiel I-362: 1H-NMR(400.0 MHz, d6-DMSO): δ= 8.212(6.35);7.767(0.41);7.751(0.93);7.746(0.9);7.729(1.69);7.713(0.97);7.708(1.08);7.692(0. 49);7.624(3.39);7.62(3.71);7.602(8.62);7.558(3.16);7.541(2.2);7.536(1.79);7.519(1.24);7.493 (2.75);7.472(4.74);7.451(2.24);2.507(26.4);2.504(32.56);2.5(25.09);2.273(16);0(43.65) |
| I-363 | 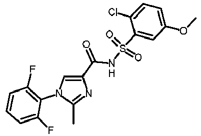 | Beispiel I-363: 1H-NMR(400.0 MHz, d6-DMSO): δ= 8.206(4.05);7.737(0.56);7.731(0.49);7.721(0.38);7.715(1.07);7.71(0.44);7.699(0.52);7.694(0. 71);7.604(2.76);7.597(2.92);7.559(2.69);7.537(3.16);7.482(1.81);7.461(2.86);7.44(1.41);7.27 5(1.57);7.267(1.53);7.253(1.37);7.245(1.34);3.849(16);2.542(1.69);2.521(0.51);2.512(9.59);2 .508(20.77);2.503(28.25);2.498(21.1);2.494(10.72);2.234(10.21);0(11.08);-0.008(0.52) |
| I-364 | 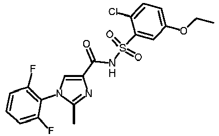 | Beispiel I-364: 1H-NMR(400.0 MHz, d6-DMSO): δ= 9.562(1.03);7.68(0.44);7.67(0.41);7.654(0.85);7.648(0.77);7.632(1.66);7.616(0.81);7.611(1.0 9);7.595(0.47);7.515(4.2);7.507(4.32);7.475(5.61);7.417(2.85);7.397(4.62);7.376(2.28);7.266 (4.05);7.245(4.66);7.032(0.93);6.948(2.32);6.94(2.25);6.927(2.04);6.919(2);4.062(1.53);4.04 5(5.07);4.027(5.14);4.01(1.6);3.56(2.77);3.546(2.11);3.537(2.73);3.489(2.21);3.474(3.95);3.4 6(2.36);3.342(2.66);3.255(2.85);2.672(0.44);2.667(0.4);2.654(2.48);2.628(2.49);2.542(14.42) ;2.525(0.89);2.511(23.19);2.507(46.64);2.503(60.67);2.498(44.27);2.33(0.39);2.1(16);1.941(0 .77);1.926(1.95);1.912(3.01);1.897(1.86);1.883(0.68);1.672(1.76);1.659(2.02);1.647(1.69);1.6 41(1.76);1.623(2.77);1.61(3.48);1.599(3.08);1.359(5.5);1.342(11.72);1.325(5.37);0.008(0.78) ;0(23.6);-0.008(0.88) |
| I-365 | 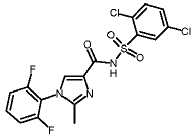 | Beispiel I-365: 1H-NMR(400.0 MHz, d6-DMSO): δ= 8.212(4.98);8.058(3.99);8.054(4.02);7.774(0.45);7.754(1.27);7.729(2.91);7.723(3.03);7.707(3 .57);7.703(3.54);7.646(3.99);7.625(2.44);7.498(2.96);7.476(5.21);7.455(2.56);4.764(0.38);4.6 95(0.46);4.596(0.61);4.581(0.62);4.52(0.69);4.489(0.71);4.473(0.73);4.425(0.73);4.409(0.72) ;4.384(0.7);4.325(0.65);4.307(0.63);4.299(0.61);4.273(0.57);4.167(0.43);4.133(0.39);4.084(0. 33);4.069(0.33);2.671(0.51);2.503(78.99);2.329(0.81);2.295(16);2.075(0.35);0(40.32) |
| I-366 | 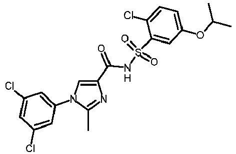 | Beispiel I-366: 1H-NMR(400.0 MHz, d6-DMSO): δ= 8.151(2.24);7.933(4.46);7.804(2.94);7.744(6.83);7.74(6.27);7.596(0.42);7.543(2.65);7.536(2. 77);7.378(2.28);7.356(2.65);7.254(5.57);7.082(1.45);7.075(1.45);7.06(1.31);7.053(1.27);4.66 (0.48);4.645(1.1);4.63(1.48);4.615(1.12);4.599(0.5);3.477(0.36);2.671(0.34);2.502(74.18);2.3 53(12.69);2.075(0.89);1.933(0.33);1.614(0.36);1.303(14.62);1.288(16);1.272(2.27);0(58.54);-0.15(0.4) |
| I-367 | 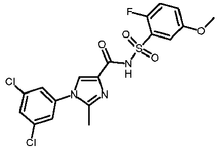 | Beispiel I-367: 1H-NMR(400.0 MHz, d6-DMSO): δ= 8.11(3.65);7.859(2.98);7.783(7.01);7.666(0.59);7.383(1.35);7.375(1.74);7.37(1.58);7.362(1.4) ;7.282(0.83);7.26(2.16);7.236(1.34);7.189(1);7.181(1.6);7.172(1.08);7.159(0.94);4.21(0.35);4 .19(0.37);4.13(0.41);4.085(0.44);4.08(0.44);4.067(0.45);4.064(0.45);4.039(0.45);4.02(0.46);3 .98(0.53);3.901(0.44);3.884(0.43);3.859(0.43);3.802(16);3.793(3.21);2.671(0.38);2.502(60.41 );2.386(13.24);2.332(0.6);2.074(1.81);1.177(0.39);-0.001(32.73) |
| I-368 | 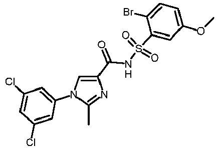 | Beispiel I-368: 1H-NMR(400.0 MHz, d6-DMSO): δ= 8.155(5.2);7.863(1.53);7.858(3.17);7.854(1.99);7.792(7.31);7.788(6.73);7.655(2.42);7.636(3. 41);7.634(3.55);7.629(3.3);7.114(1.29);7.106(1.27);7.092(1.2);7.085(1.18);3.83(16);3.814(0. 46);2.507(24);2.502(32.32);2.498(24.33);2.392(13.38);0.008(0.61);0(23.56);-0.008(1.15) |
| I-369 | 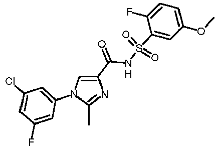 | Beispiel I-369: 1H-NMR(400.0 MHz, d6-DMSO): δ= 8.129(5.01);7.721(2.19);7.7(2.16);7.661(4.27);7.636(2.29);7.614(2.07);7.38(2.65);7.294(1.19) ;7.271(2.52);7.248(1.71);7.192(2.23);7.171(1.35);4.946(0.32);4.911(0.34);4.898(0.34);4.862( 0.37);4.85(0.38);4.839(0.38);4.806(0.41);4.786(0.41);4.774(0.42);4.757(0.44);4.746(0.44);4.7 18(0.45);4.711(0.44);4.666(0.46);4.639(0.46);4.631(0.46);4.586(0.46);4.575(0.45);4.494(0.42 );4.467(0.41);4.446(0.39);4.43(0.38);4.42(0.38);4.412(0.37);4.389(0.35);4.332(0.32);3.805(1 6);2.504(38.7);2.402(14.33);2.332(0.46);2.076(4.54);0(7.54) |
| I-370 | 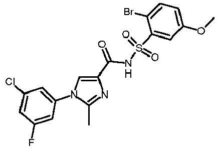 | Beispiel I-370: 1H-NMR(400.0 MHz, d6-DMSO): δ= 8.154(5.66);7.719(0.77);7.714(1.44);7.709(1);7.698(0.79);7.693(1.47);7.688(1.01);7.658(4.33 );7.646(1.32);7.637(5.41);7.63(3.45);7.623(1.22);7.618(1.45);7.613(0.78);7.118(1.36);7.11(1. 38);7.096(1.27);7.088(1.29);3.831(16);3.814(0.56);2.507(23.86);2.503(31.71);2.498(24.17);2. 403(13.62);2.075(10.95);0.008(0.39);0(11.35) |
| I-371 | 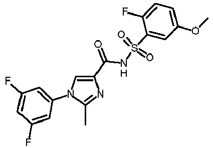 | Beispiel I-371: 1H-NMR(400.0 MHz, d6-DMSO): δ= 8.126(5.25);7.55(1.63);7.527(3.17);7.506(5.64);7.491(4.66);7.38(2.74);7.297(1.27);7.274(2.5 9);7.251(1.76);7.195(2.28);3.806(16);2.504(28.88);2.413(14.81);2.332(0.38);2.076(3.5);-0.001(5.65) |
| I-372 | 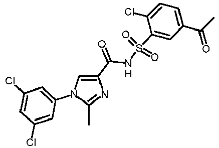 | Beispiel I-372: 1H-NMR(400.0 MHz, d6-DMSO): δ= 8.576(3.14);8.571(3.3);8.11(6.03);8.102(1.99);8.086(1.68);8.081(1.69);7.89(1.95);7.886(3.38) ;7.881(2.17);7.808(7.71);7.803(7.14);7.701(2.82);7.68(2.6);2.632(16);2.507(38.54);2.503(49. 34);2.498(38.77);2.418(14);2.333(0.35);2.329(0.42);2.325(0.36);2.319(0.38);2.075(1.44);1.17 9(0.56);1.175(0.55);0(17.69) |
| I-373 | 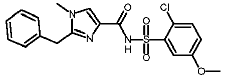 | Beispiel I-373: 1H-NMR(400.0 MHz, d6-DMSO): δ= 7.823(3.68);7.563(2.76);7.555(2.91);7.393(2.11);7.371(2.52);7.366(0.82);7.363(1.1);7.346(2. 91);7.327(2.5);7.289(1.1);7.286(0.88);7.271(1.46);7.264(0.41);7.248(2.57);7.245(2.89);7.227 (2.09);7.093(1.2);7.085(1.21);7.071(1.06);7.063(1.05);4.252(4.32);3.815(0.73);3.805(16);3.6 12(11.31);2.52(0.59);2.511(18.62);2.507(40.58);2.502(55.15);2.498(41.21);2.493(21.02);2.32 9(0.41);2.324(0.33);2.075(6.06);0.008(0.5);0(22.09);-0.008(0.97) |
| I-374 | 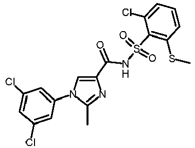 | Beispiel I-374: 1H-NMR(400.0 MHz, d6-DMSO): δ= 8.139(3.44);7.843(1.97);7.839(3.59);7.834(2.14);7.776(9.1);7.771(7.75);7.489(0.93);7.469(2. 29);7.449(1.74);7.359(2.05);7.34(1.51);7.317(2.24);7.298(1.83);5.753(6.87);2.675(0.41);2.67 (0.58);2.666(0.38);2.54(1.87);2.524(1.19);2.51(42.72);2.506(82.03);2.501(105.19);2.496(77.4 );2.492(38.5);2.431(13.16);2.373(16);2.332(0.76);2.328(0.91);2.323(0.73);2.086(0.47);0.008( 1.15);0(29.86);-0.008(1.26) |
| I-375 | 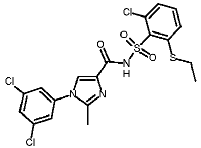 | Beispiel I-375: 1H-NMR(400.0 MHz, d6-DMSO): δ= 8.152(5.15);7.84(1.77);7.836(3.47);7.832(2.28);7.773(8.26);7.769(7.62);7.742(0.42);7.737(0. 39);7.47(0.76);7.449(2.22);7.43(2.54);7.414(2.7);7.397(1.18);7.32(2.07);7.318(2.08);7.302(1. 69);7.299(1.62);2.984(0.98);2.966(3.46);2.947(3.64);2.929(1.21);2.54(3.9);2.506(46.87);2.50 1(63.77);2.497(51.17);2.372(16);2.352(1.34);2.333(0.65);2.328(0.75);1.285(4.25);1.267(9.26) ;1.249(4.38);1.183(0.5);1.12(1.96);0(13.33) |
| I-376 | 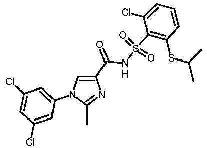 | Beispiel I-376: 1H-NMR(400.0 MHz, d6-DMSO): δ= 8.16(2.74);7.834(2.03);7.83(3.72);7.825(2.31);7.766(8.64);7.761(7.77);7.73(0.32);7.522(1.05) ;7.503(2.1);7.477(1.46);7.458(2.16);7.445(0.33);7.438(1.05);7.348(1.86);7.346(1.9);7.329(1.4 );7.327(1.36);5.753(16);3.672(0.35);3.656(0.92);3.64(1.26);3.623(0.98);3.606(0.43);2.524(0. 37);2.511(11.84);2.506(24.22);2.502(32.17);2.497(24.27);2.493(12.52);2.372(15.1);1.988(1.0 2);1.397(15.99);1.306(1.08);1.282(0.98);1.269(15.59);1.252(15.35);1.224(0.94);1.207(0.88);1 .193(0.45);1.188(0.66);1.175(0.71);1.172(0.51);1.158(0.33);0.008(0.6);0(23.33);-0.008(1.25) |
| I-377 | 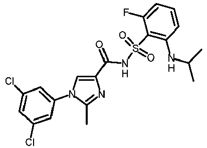 | Beispiel I-377: 1H-NMR(400.0 MHz, d6-DMSO): δ= 8.159(4.77);7.828(1.74);7.824(3.36);7.819(2.21);7.761(8.02);7.757(7.33);7.341(0.53);7.32(1. 13);7.303(1.15);7.283(0.58);6.907(0.46);6.606(1.8);6.584(1.72);6.368(0.98);6.348(1.06);6.34 1(1.15);6.321(0.95);3.738(0.54);3.723(0.71);3.711(0.59);2.541(0.64);2.506(21.51);2.502(28.3 );2.497(21.94);2.358(14.67);2.333(0.38);2.328(0.34);1.218(15.91);1.202(16);0(2.11) |
| I-378 | 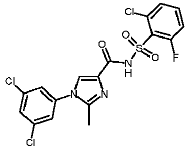 | Beispiel I-378: 1H-NMR(400.0 MHz, d6-DMSO): δ= 8.106(4.8);7.882(1.93);7.877(3.7);7.873(2.42);7.808(8.7);7.803(8.12);7.544(0.57);7.53(0.69); 7.523(1.32);7.51(1.35);7.503(0.93);7.489(0.79);7.374(2.29);7.354(1.74);7.305(1.01);7.28(1.3 2);7.259(0.83);2.506(27.17);2.502(36.23);2.497(28.06);2.416(16);2.074(2.27);0.008(1.15);0( 32.22) |
| I-379 | 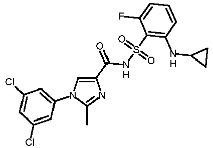 | Beispiel I-379: 1H-NMR(400.0 MHz, d6-DMSO): δ= 8.141(6.18);7.837(1.95);7.832(3.86);7.828(2.37);7.764(9.11);7.76(8.24);7.4(0.54);7.379(1.15) ;7.363(1.16);7.343(0.6);7.164(1.49);6.943(2.09);6.922(1.94);6.464(0.97);6.444(1.04);6.437(1. 1);6.416(093);2.524(0.51);2.51(10.03);2.506(20.15);2.502(26.68);2.497(20.01);2.493(10.6);2 .364(16);2.074(5.52);0.844(0.64);0.832(2.14);0.827(2.75);0.815(2.7);0.81(2.34);0.799(0.77); 0.51(0.78);0.5(2.5);0.494(2.78);0.492(2.75);0.485(2.47);0.474(0.72);0.008(0.61);0(18.68);-0.008(0.76) |
| I-380 | 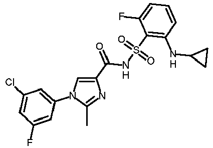 | Beispiel I-380: 1H-NMR(400.0 MHz, d6-DMSO): δ= 9.57(0.84);7.729(2.1);7.562(0.78);7.557(1.5);7.552(1.11);7.541(0.72);7.536(1.62);7.53(2.53); 7.523(3.78);7.518(6.89);7.509(1.71);7.504(0.75);7.491(1.16);7.486(1.55);7.48(0.73);7.147(0. 61);7.132(0.76);7.127(1.26);7.111(1.26);7.107(0.83);7.091(0.66);6.765(2.05);6.744(1.85);6.2 53(0.97);6.232(1.06);6.228(1.1);6.208(0.92);5.754(10.25);3.561(2.26);3.548(1.7);3.538(2.2); 3.49(1.74);3.475(3.12);3.46(1.82);3.325(2.04);3.268(1.65);3.254(2.72);3.24(1.66);2.659(2.09) ;2.633(2.05);2.54(13.89);2.524(0.66);2.51(12.31);2.506(24.58);2.501(32.65);2.497(24.41);2.4 92(12.32);2.399(0.44);2.391(0.67);2.383(0.91);2.377(0.92);2.368(0.74);2.361(0.49);2.29(16); 1.943(0.61);1.928(1.6);1.913(2.52);1.899(1.53);1.884(0.57);1.673(1.42);1.66(1.63);1.643(1.6 5);1.625(2.13);1.613(2.7);1.6(2.41);0.762(0.64);0.75(2.04);0.745(2.63);0.734(2.6);0.728(2.15 );0.718(0.74);0.45(0.77);0.44(2.5);0.434(2.6);0.431(2.58);0.425(2.35);0.414(0.68);0.008(0.33 );0(9.51); -0.008(0.36) |
| I-381 | 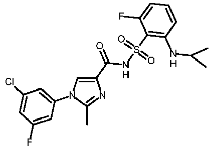 | Beispiel I-381: 1H-NMR(400.0 MHz, d6-DMSO): δ= 8.156(3.76);7.684(0.75);7.679(1.39);7.673(1.01);7.662(0.75);7.657(1.4);7.652(1.03);7.627(2. 79);7.614(1.36);7.609(1.53);7.604(0.77);7.591(1.11);7.586(1.49);7.581(0.81);7.342(0.49);7.3 21(1.03);7.304(1.02);7.284(0.54);6.902(0.36);6.607(1.65);6.585(1.56);6.369(0.9);6.349(0.94) ;6.341(1.01);6.321(0.86);5.752(2.91);3.739(0.48);3.724(0.63);3.709(0.51);2.67(0.38);2.523(0. 75);2.519(1.23);2.51(21.55);2.506(46.67);2.501(65.76);2.496(51.9);2.492(27.46);2.367(14.99 );2.332(0.44);2.328(0.53);2.323(0.44);1.398(3.95);1.217(15.95);1.202(16);1.177(0.68);1.161( 0.6);0.008(0.34);0(13.53);-0.008(0.59) |
| I-382 | 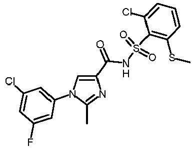 | Beispiel I-382: 1H-NMR(400.0 MHz, d6-DMSO): δ= 7.789(0.44);7.633(0.68);7.628(1.19);7.623(0.97);7.607(1.18);7.602(1.09);7.589(2.67);7.58(1. 38);7.574(1.32);7.556(1);7.551(1.26);7.546(0.68);7.356(0.44);7.336(0.95);7.316(0.71);7.24(1 .13);7.22(0.89);7.205(1.28);7.186(0.95);5.752(10.12);4.038(0.97);4.02(0.99);4.003(0.34);2.51 (6.39);2.506(14.18);2.502(20.14);2.497(15.98);2.493(8.66);2.432(0.32);2.37(0.75);2.345(16); 1.988(4.25);1.397(2.85);1.193(1.13);1.175(2.26);1.158(1.14);0(4.93) |
| I-383 | 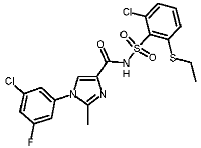 | Beispiel I-383: 1H-NMR(400.0 MHz, d6-DMSO): δ= 8.177(0.55);8.147(4.57);7.695(0.85);7.69(1.56);7.685(1.1);7.673(0.98);7.668(1.74);7.663(1.3) ;7.64(3.17);7.628(1.48);7.622(1.66);7.618(0.87);7.604(1.53);7.6(1.84);7.594(1.04);7.471(0.75 );7.45(2.19);7.431(2.56);7.415(2.61);7.398(1.02);7.322(1.98);7.318(1.99);7.303(1.61);7.3(1.6 );4.056(0.71);4.038(2.17);4.02(2.21);4.003(0.76);2.985(1.05);2.967(3.59);2.948(3.72);2.93(1. 25);2.506(33.03);2.501(45.97);2.497(36.25);2.382(16);2.365(2.02);2.333(0.37);2.328(0.46);2. 324(0.38);1.988(9.32);1.285(4.03);1.267(8.83);1.249(4.16);1.236(0.55);1.226(0.65);1.208(1.1 3);1.2(0.35);1.193(2.62);1.181(0.48);1.175(4.93);1.163(0.37);1.157(2.51);0(8.22);-0.008(0.49) |
| I-384 | 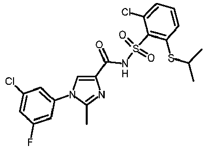 | Beispiel I-384: 1H-NMR(400.0 MHz, d6-DMSO): δ= 7.622(1.03);7.601(1.05);7.577(2.59);7.542(1.23);7.364(0.74);7.322(0.62);7.214(0.62);4.056(1 .29);4.038(3.8);4.02(3.86);4.003(1.36);3.558(0.68);3.543(0.8);3.528(0.74);2.67(0.39);2.524(0 .53);2.51(21.43);2.506(45);2.501(62.32);2.497(48.72);2.492(25.83);2.344(10.8);1.988(16);1.3 98(0.33);1.291(1.1);1.258(15.12);1.241(15.16);1.22(0.66);1.204(0.49);1.193(4.44);1.175(8.56 );1.157(4.28);0(11.37);-0.008(0.52) |
| I-385 | 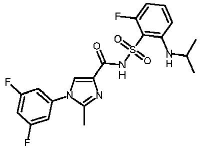 | Beispiel I-385: 1H-NMR(400.0 MHz, d6-DMSO): δ= 8.153(4.21);7.512(0.68);7.506(0.54);7.493(0.97);7.488(1.74);7.48(2.73);7.46(3.54);7.344(0.5 3);7.322(1.1);7.306(1.09);7.285(0.55);6.897(0.39);6.609(1.8);6.587(1.7);6.371(1);6.351(1.03) ;6.343(1.1);6.324(0.95);3.801(0.36);3.739(0.93);3.725(1.11);3.711(0.99);3.656(0.57);3.646(0. 57);3.604(0.61);3.573(0.64);3.556(0.64);3.54(0.64);3.517(0.63);3.492(0.61);3.48(0.6);3.33(0. 37);3.321(0.36);2.675(0.6);2.671(0.81);2.666(0.61);2.541(1.94);2.524(2.15);2.506(93.16);2.5 02(121.85);2.497(90.71);2.377(15.25);2.332(0.63);2.328(0.83);2.324(0.63);1.217(16);1.202(1 5.9);0.146(0.47);0.008(3.78);0(104.97);-0.007(4.95);-0.15(0.54) |
| I-386 | 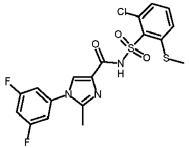 | Beispiel I-386: 1H-NMR(400.0 MHz, d6-DMSO): δ= 8.134(5.62);7.532(0.43);7.527(0.79);7.521(0.68);7.503(2.09);7.494(3.94);7.475(5.39);7.453(2 .12);7.364(2.3);7.344(1.63);7.32(2.38);7.301(1.9);5.753(8.72);2.67(0.37);2.54(5.29);2.505(53 .53);2.501(70.7);2.497(54.86);2.434(13.75);2.393(16);2.37(1.04);2.332(0.49);2.328(0.59);0.0 07(2.15);0(60.26);-0.15(0.34) |
| I-387 | 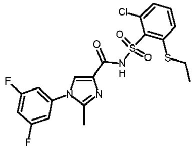 | Beispiel I-387: 1H-NMR(400.0 MHz, d6-DMSO): δ= 8.141(4.28);7.524(0.73);7.518(0.67);7.5(2.01);7.493(3.29);7.473(4.81);7.452(2.46);7.433(2.5 5);7.416(2.65);7.399(1.11);7.323(2.02);7.319(2.02);7.304(1.66);7.301(1.6);5.753(6.21);3.896 (0.35);3.768(0.38);3.733(0.38);3.69(0.37);3.621(0.33);2.986(1.11);2.967(3.48);2.949(3.59);2. 931(1.26);2.675(0.57);2.67(0.76);2.666(0.58);2.541(5.92);2.506(94.61);2.501(124.08);2.497( 96.06);2.391(16);2.372(0.99);2.332(0.8);2.328(0.98);2.324(0.79);2.073(0.37);1.284(4.21);1.2 66(9.07);1.248(4.18);0.146(0.5);0.008(6.1);0(114.82);-0.008(8.82);-0.041(0.59);-0.15(0.54) |
| I-388 | 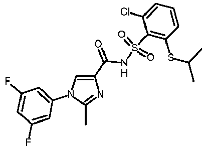 | Beispiel I-388: 1H-NMR(400.0 MHz, d6-DMSO): δ= 8.164(6.09);7.531(1.27);7.512(3.24);7.486(5.02);7.468(6.42);7.446(1.51);7.356(2.54);7.337(1 .97);5.754(7.12);3.678(0.42);3.662(1.09);3.645(1.49);3.629(1.12);3.613(0.45);2.542(3.3);2.50 3(16.73);2.395(15.14);2.074(0.42);1.268(16);1.252(15.55);0(15.77) |
| I-389 | 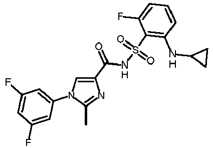 | Beispiel I-389: 1H-NMR(400.0 MHz, d6-DMSO): δ= 8.136(5.7);7.526(0.36);7.521(0.72);7.516(0.57);7.498(1.57);7.493(1.81);7.481(2.93);7.478(2. 95);7.463(3.39);7.404(0.6);7.383(1.28);7.367(1.27);7.347(0.66);7.153(1.65);6.946(2.16);6.92 4(2.03);6.468(1.03);6.447(1.13);6.44(1.24);6.42(1.02);5.753(9.27);2.541(5.12);2.506(51.19); 2.501(68.76);2.497(55.44);2.434(0.59);2.384(16);2.332(0.57);2.328(0.69);2.324(0.6);2.073(0. |
| | | 88);0.844(0.57);0.828(2.88);0.816(2.88);0.811(2.63);0.8(0.98);0.51(0.71);0.5(2.59);0.492(3.1 4);0.486(2.85);0.474(0.95);0.007(1.86);0(57.62);-0.15(0.36) |
| I-390 | 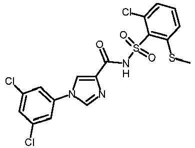 | Beispiel I-390: 1H-NMR(400.0 MHz, d6-DMSO): δ= 8.656(3.7);8.569(4.04);8.566(4.01);7.934(9.04);7.93(10.08);7.707(2.21);7.703(4.08);7.698(2. 23);7.553(1.31);7.533(3.03);7.513(2.4);7.421(2.46);7.4(1.74);7.372(2.53);7.37(2.59);7.353(2. 11);7.35(2.09);7.236(0.35);3.718(0.33);3.664(0.38);3.522(0.48);3.47(0.49);3.367(0.4);3.313( 0.36);2.675(0.5);2.671(0.71);2.666(0.52);2.541(75.39);2.524(1.96);2.51(39.78);2.506(81.89); 2.502(109.78);2.497(81.6);2.493(41.35);2.471(16);2.444(2.19);2.367(0.39);2.333(0.62);2.328 (0.79);2.324(0.6);0.146(0.5);0.008(3.92);0(114.17);-0.008(4.92);-0.15(0.51) |
| I-391 | 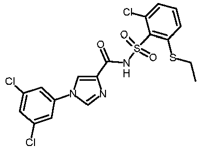 | Beispiel I-391: 1H-NMR(400.0 MHz, d6-DMSO): δ= 8.668(7.76);8.571(8.5);7.934(15.5);7.702(6.42);7.53(1.4);7.509(4.3);7.49(4.91);7.476(5.77);7 .458(2.07);7.373(4.44);7.354(3.5);5.754(0.42);3.775(0.33);3.735(0.35);3.724(0.35);3.677(0.3 8);3.655(0.38);3.579(0.4);3.554(0.4);3.513(0.4);3.447(0.39);3.397(0.36);3.383(0.36);3.351(0. 34);3.023(2.28);3.005(6.98);2.987(7.18);2.968(2.5);2.711(0.5);2.671(0.79);2.541(99.26);2.50 2(129.3);2.368(0.7);2.328(0.98);1.278(7.69);1.26(16);1.242(7.65);1.12(0.4);0.146(0.44);0(92. 3);-0.15(0.51) |
| I-392 | 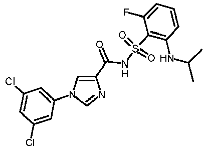 | Beispiel I-392: 1H-NMR(400.0 MHz, d6-DMSO): δ= 8.673(3.8);8.671(3.67);8.564(4.1);7.942(7.51);7.938(7.53);7.695(2.02);7.691(3.37);7.687(1.8 2);7.389(0.62);7.368(1.24);7.351(1.25);7.331(0.65);6.654(2.04);6.632(1.93);6.409(1.11);6.38 9(1.15);6.381(1.24);6.361(1.07);3.79(0.39);3.775(0.81);3.759(1.05);3.744(0.83);3.728(0.43); 2.542(8.8);2.507(27.98);2.503(35.81);2.498(26.94);1.229(16);1.213(15.83);0.008(1.32);0(30. 77) |
| I-393 | 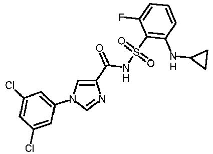 | Beispiel I-393: 1H-NMR(400.0 MHz, d6-DMSO): δ= 8.658(7.19);8.656(7.1);8.573(7.98);8.57(7.09);7.941(15.98);7.937(16);7.698(4.16);7.694(6.87 );7.689(3.7);7.464(1.18);7.448(1.62);7.443(2.47);7.427(2.47);7.423(1.74);7.407(1.31);7.055( 2.51);6.99(4.27);6.968(3.96);6.518(2.04);6.498(2.18);6.49(2.32);6.47(2.01);5.754(2.59);2.712 (0.58);2.676(0.37);2.672(0.51);2.667(0.36);2.542(141.79);2.524(3.35);2.52(4.05);2.507(66.22 );2.502(85.72);2.498(64.14);2.368(0.76);2.334(0.52);2.329(0.65);2.325(0.52);0.862(1.23);0.8 5(4.35);0.846(5.4);0.834(5.4);0.829(4.6);0.817(1.54);0.53(1.52);0.519(5.01);0.513(5.75);0.50 5(4.96);0.493(1.49);0.146(0.36);0.008(3.41);0(84.69);-0.149(0.42) |
| I-394 | 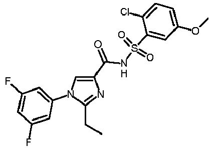 | Beispiel I-394: 1H-NMR(400.0 MHz, d6-DMSO): δ= 8.169(5.68);7.611(2.78);7.603(2.98);7.545(0.38);7.536(3.06);7.522(0.76);7.514(4.1);7.494(2. 88);7.489(2.79);7.475(2.75);7.458(0.4);7.248(1.56);7.24(1.56);7.226(1.4);7.218(1.38);3.848( 16);2.73(1.08);2.712(3.51);2.693(3.62);2.674(1.3);2.507(19.59);2.502(25.58);2.498(19.83);1. 154(3.81);1.135(7.89);1.116(3.67);0.008(1.05);0(23.23) |
| I-395 | 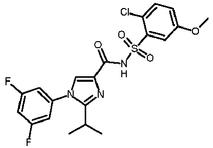 | Beispiel I-395: 1H-NMR(400.0 MHz, d6-DMSO): δ= 8.157(5.92);7.621(2.87);7.613(3.01);7.556(3.38);7.533(4.26);7.527(1.14);7.514(0.71);7.508(0 .97);7.502(2.56);7.497(2.2);7.483(2.55);7.269(1.64);7.261(1.6);7.247(1.43);7.239(1.39);3.854 (16);3.027(0.36);3.01(1.01);2.993(1.42);2.976(1.06);2.959(0.41);2.712(0.8);2.543(116.95);2. 507(11.75);2.503(15.6);2.499(12.27);2.368(0.89);1.167(13.53);1.15(13.29);0.008(0.67);0(16. 49) |
| I-396 | 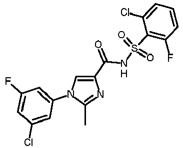 | Beispiel I-396: 1H-NMR(400.0 MHz, d6-DMSO): δ= 8.122(6.71);7.742(0.86);7.738(1.54);7.733(1.09);7.721(0.9);7.716(1.58);7.711(1.11);7.685(3. 19);7.661(1.19);7.656(1.64);7.652(0.95);7.638(1.18);7.633(1.67);7.629(0.96);7.552(0.58);7.5 39(0.67);7.532(1.34);7.519(1.38);7.512(0.96);7.498(0.84);7.381(2.32);7.361(1.81);7.314(1.08 );7.289(1.36);7.266(0.91);2.506(17.22);2.502(22.74);2.498(17.51);2.431(16);2.074(1.74);0.00 8(1.56);0(29.91) |
| I-397 | 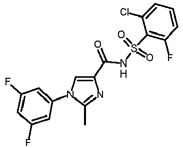 | Beispiel I-397: 1H-NMR(400.0 MHz, d6-DMSO): δ= 8.118(6.7);7.578(0.48);7.572(0.82);7.566(0.62);7.555(1.3);7.55(1.87);7.543(2.11);7.532(3.57) ;7.526(3.36);7.521(3.1);7.513(3.77);7.501(1.26);7.383(2.26);7.363(1.74);7.317(1.05);7.291(1. 29);7.27(0,86);2.506(23.03);2.502(30.08);2.498(22.76);2.441(16);0.008(1.71);0(40.03);-0.008(2.6) |
| I-398 | 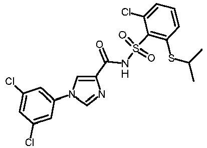 | Beispiel I-398: 1H-NMR(400.0 MHz, d6-DMSO): δ= 8.679(2.42);8.578(3.34);8.314(0.39);7.941(7.56);7.937(8.07);7.704(1.86);7.7(3.29);7.696(1.8 9);7.582(1.13);7.562(2.15);7.531(1.44);7.511(2.27);7.491(1.1);7.4(2.06);7.381(1.5);5.754(1.3 1);3.71(0.48);3.693(1.09);3.677(1.43);3.66(1.12);3.644(0.56);3.521(0.37);3.352(0.77);3.203( 0.41);2.71(0.33);2.675(0.87);2.67(1.19);2.666(0.93);2.54(64.72);2.523(3.01);2.506(134.31);2. 501(179.37);2.497(135.53);2.366(0.34);2.332(0.83);2.328(1.15);2.324(0.87);1.292(0.35);1.27 6(0.42);1.254(16);1.238(15.93);1.152(0.41);1.136(0.41);1.122(1.11);0.008(2.24);0(65.38);-0.008(2.83) |
| I-399 | 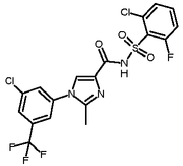 | Beispiel I-399: 1H-NMR(400.0 MHz, d6-DMSO): δ= 8.182(5.84);8.144(5.11);8.082(3.07);7.554(0.57);7.541(0.67);7.534(1.33);7.52(1.35);7.513(0. 93);7.5(0.81);7.383(2.31);7.363(1.77);7.316(1.05);7.291(1.33);7.269(0.88);2.506(30.27);2.50 2(39.92);2.497(30.72);2.423(16);0.007(2);0(43.65) |
| I-400 | 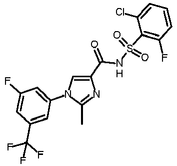 | Beispiel I-400: 1H-NMR(400.0 MHz, d6-DMSO): δ= 8.188(5.9);7.998(3.23);7.971(4.79);7.559(0.55);7.545(0.71);7.539(1.37);7.525(1.39);7.518(1. 04);7.505(0.83);7.387(2.4);7.367(1.86);7.32(1.16);7.295(1.49);7.273(0.96);2.67(0.37);2.502( 61.54);2.498(50.54);2.434(16);2.329(0.42);0.146(0.32);0(67.08);-0.15(0.4) |
| I-401 | 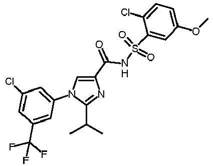 | Beispiel I-401: 1H-NMR(400.0 MHz, d6-DMSO): δ= 8.216(5.29);8.136(2.81);8.132(2.84);8.125(2.71);8.055(2.5);7.622(2.9);7.614(2.99);7.555(2.6 1);7.533(3.05);7.268(1.59);7.261(1.52);7.246(1.38);7.239(1.31);5.754(0.33);3.855(16);2.968( 0.34);2.951(0.95);2.934(1.34);2.917(0.99);2.9(0.38);2.542(45.04);2.526(0.76);2.507(18.68);2. 503(24.25);2.498(18.15);1.166(13.06);1.149(12.86);0(4.49) |
| I-402 | 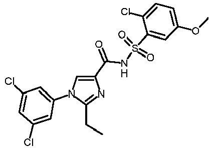 | Beispiel I-402: 1H-NMR(400.0 MHz, d6-DMSO): δ= 8.214(4.42);7.982(2.4);7.961(2.29);7.932(2.28);7.62(2.84);7.613(2.96);7.556(2.52);7.534(2.9 7);7.27(1.51);7.262(1.45);7.248(1.32);7.24(1.27);3.854(16);2.992(0.33);2.975(0.92);2.958(1. 3);2.941(0.96);2.924(0.36);2.541(49.08);2.511(17.68);2.506(35.01);2.502(46.12);2.498(34.44 );2.329(0.33);1.164(12.87);1.147(12.72);0(6.12) |
| I-403 | 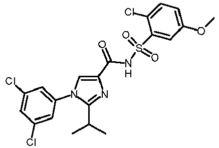 | Beispiel I-403: 1H-NMR(400.0 MHz, d6-DMSO): δ= 8.159(4.82);7.87(1.77);7.866(3.24);7.861(1.9);7.785(7.39);7.781(6.46);7.618(2.92);7.61(2.96) ;7.551(2.58);7.529(3.01);7.264(1.57);7.256(1.49);7.242(1.36);7.234(1.27);3.852(16);2.98(0.3 4);2.964(0.96);2.946(1.34);2.929(1);2.912(0.37);2.712(0.7);2.542(134.45);2.525(1.7);2.507(2 3.73);2.502(30.18);2.498(22.24);2.467(0.34);2.368(0.77);1.166(13.14);1.149(12.87);0.008(0. 36);0(6.9);-0.008(0.34) |
| I-404 | 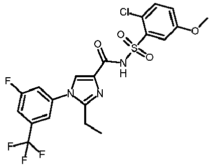 | Beispiel I-404: 1H-NMR(400.0 MHz, d6-DMSO): δ= 8.234(4.77);7.966(3.41);7.944(3.47);7.916(2.96);7.612(2.79);7.605(2.93);7.538(2.47);7.516(2 .86);7.25(1.53);7.242(1.5);7.228(1.32);7.22(1.27);3.849(16);2.717(1.09);2.698(3.5);2.68(3.73 );2.661(1.29);2.541(21.34);2.502(60.58);2.328(0.44);1.157(3.87);1.139(7.85);1.12(3.75);0(11 .22) |
| I-405 | 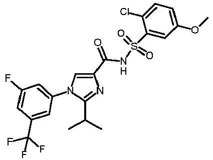 | Beispiel I-405: 1H-NMR(400.0 MHz, d6-DMSO): δ= 8.178(5.58);7.855(1.64);7.85(3.16);7.846(1.95);7.776(7.17);7.771(6.52);7.61(2.94);7.602(3.0 5);7.533(2.6);7.511(3.03);7.244(1.58);7.237(1.52);7.222(1.39);7.215(1.33);5.754(0.82);3.847 (16);2.704(1.03);2.685(3.37);2.666(3.52);2.647(1.1);2.542(0.87);2.507(17.15);2.502(21.96);2 .498(16.29);1.155(3.8);1.136(7.96);1.117(3.63);0(4.08) |
| I-406 | 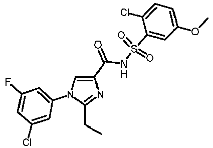 | Beispiel I-406: 1H-NMR(400.0 MHz, d6-DMSO): δ= 8.175(5.7);7.711(0.77);7.706(1.35);7.701(0.95);7.689(0.81);7.684(1.36);7.679(0.93);7.643(2. 63);7.629(1.18);7.623(1.45);7.618(0.91);7.611(3.19);7.603(3.9);7.596(0.93);7.534(2.64);7.51 2(3.09);7.246(1.58);7.238(1.54);7.224(1.39);7.216(1.33);3.848(16);3.817(0.49);2.717(1.06);2 .699(3.39);2.68(3.5);2.661(1.14);2.542(39.66);2.511(8.22);2.507(15.62);2.502(20.2);2.498(14 .97);1.155(3.82);1.136(7.97);1.117(3.64);0(4.06) |
| I-407 | 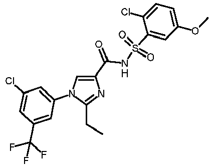 | Beispiel I-407: 1H-NMR(400.0 MHz, d6-DMSO): δ= 8.159(5.13);7.728(0.9);7.723(1.47);7.718(1);7.706(0.9);7.702(1.43);7.696(0.91);7.652(2.96); 7.638(1.23);7.633(1.48);7.628(0.92);7.618(3.47);7.61(4.26);7.553(2.58);7.531(2.96);7.266(1. 62);7.258(1.52);7.244(1.36);7.236(1.26);3.853(16);3.004(0.39);2.986(1.01);2.969(1.41);2.952 (1.05);2.935(0.4);2.541(38.26);2.506(22.29);2.502(28.53);2.498(21.68);1.166(13.62);1.148(1 3.29);0.008(0.32);0(5.82) |
| I-408 | 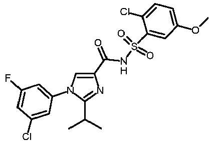 | Beispiel I-408: 1H-NMR(400.0 MHz, d6-DMSO): δ= 8.234(4.9);8.119(3.18);8.115(3.09);8.107(3.13);8.038(2.89);7.612(2.89);7.605(2.98);7.537(2. 48);7.515(2.88);7.248(1.55);7.241(1.49);7.226(1.34);7.219(1.27);3.849(16);2.703(1.06);2.685 (3.46);2.666(3.72);2.647(1.18);2.541(4.61);2.502(47.04);2.329(0.32);1.159(3.77);1.14(7.85); 1.121(3.7);0(8.92) |
| I-409 | 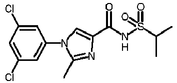 | Beispiel I-409: 1H-NMR(400.0 MHz, d6-DMSO): δ= 8.216(5.16);7.819(1.61);7.815(3.37);7.81(2.24);7.766(7.69);7.762(6.78);3.791(0.45);3.774(1. 15);3.756(1.58);3.739(1.19);3.722(0.5);3.477(0.37);3.346(0.7);3.259(0.46);2.506(38.81);2.50 1(50.46);2.497(37.5);2.356(16);2.333(0.41);2.328(0.42);2.324(0.34);2.073(9.63);1.308(14.48) ;1.291(14.28) |
| I-410 | 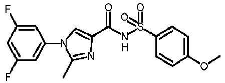 | Beispiel I-410: 1H-NMR(400.0 MHz, d6-DMSO): δ= 8.083(4.59);7.937(0.41);7.929(3.85);7.924(1.32);7.912(1.25);7.907(4.29);7.9(0.48);7.486(0.5 7);7.48(0.41);7.463(1.22);7.458(1.36);7.446(2.1);7.442(2.06);7.427(2.42);7.138(0.44);7.131( 3.9);7.126(1.35);7.113(1.21);7.108(3.83);7.101(0.46);3.842(16);3.444(0.35);3.401(0.36);3.34 3(0.33);2.524(0.86);2.51(18.15);2.506(35.94);2.501(47.03);2.497(34.13);2.492(16.63);2.35(1 4);2.332(0.34);2.328(0.41);2.073(0.32) |
| I-411 | 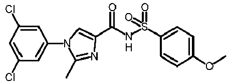 | Beispiel I-411: 1H-NMR(400.0 MHz, d6-DMSO): δ= 8.105(5.63);7.939(0.49);7.932(4.13);7.927(1.37);7.914(1.37);7.909(4.41);7.902(0.51);7.807(1 .47);7.802(2.95);7.798(1.82);7.728(6.58);7.723(6.05);7.142(0.49);7.134(4.16);7.116(1.31);7.1 12(3.97);7.104(0.46);3.843(16);3.361(0.48);3.343(0.48);3.332(0.48);2.675(0.45);2.67(0.6);2. |
| | | 666(0.45);2.51(37.34);2.506(73.3);2.501(96.45);2.497(71.78);2.329(14.24);2.073(1.11);0.008 (0.49);0(12.36);-0.008(0.51) |
| I-412 | 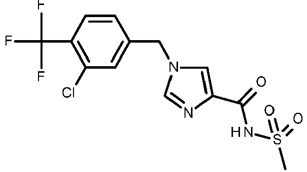 | I-412: ¹H-NMR(400.0 MHz, d₆-DMSO): |
| | | δ=8.1462(9.6);8.0276(8.7);7.8997(5.6);7.8793(6.1);7.7263(7.4);7.4540(3.9);7.43 35(3.5);5.3929(16.0);3.4611(0.6);3.3984(0.4);3.2895(39.6);3.1138(0.4);2.6715(0. 7);2.5066(85.5);2.5024(112.4);2.4980(85.9);2.3292(0.7);2.3247(0.6);2.0748(0.3); 0.1458(0.3);0.0074(3.1);-0.0002(71.1);-0.1498(0.3) |
| I-413 | 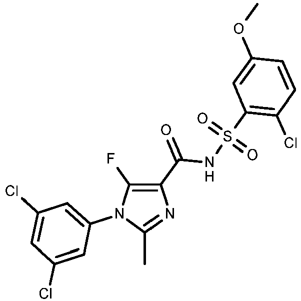 | I-413: ¹H-NMR(601.6 MHz, CD3CN): |
| | | δ= 7.7567(1.6);7.7516(1.7);7.7251(0.9);7.7220(1.8);7.7190(1.1);7.5456(1.7);7.53 09(1.9);7.4976(2.7);7.4948(3.0);7.2457(1.0);7.2406(1.0);7.2310(0.9);7.2259(0.9); 4.9467(0.4);3.9023(11.0);2.5347(16.0);2.2305(4.7);1.9923(2.5);1.9842(1.1);1.98 01(1.2);1.9762(5.5);1.9722(9.2);1.9681(13.1);1.9639(9.2);1.9598(5.0) |
| I-414 | 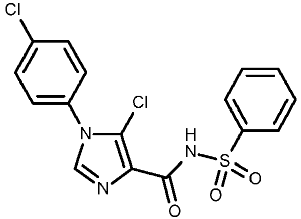 | I-414: ¹H-NMR(400.0 MHz, d₆-DMSO): |
| | | δ= 8.2181(15.8);8.0234(8.3);8.0052(9.9);8.0016(7.3);7.7305(1.4);7.7179(1.1);7.7 120(4.9);7.7069(1.7);7.6929(13.4);7.6879(4.6);7.6762(4.4);7.6708(16.0);7.6637( 2.5);7.6556(7.1);7.6360(9.8);7.6219(1.7);7.6181(3.8);7.5971(2.0);7.5900(15.6);7. 5847(4.8);7.5732(3.6);7.5680(10.4);7.5609(1.2);3.3780(0.7);3.3421(0.7);3.1862( 0.6);3.1692(0.7);2.6762(0.4);2.6715(0.5);2.6671(0.4);2.5248(1.1);2.5112(29.7);2. 5070(60.6);2.5025(80.4);2.4980(59.1);2.4937(29.2);2.3337(0.4);2.3291(0.5);2.32 47(0.4);2.0754(0.6);-0.0002(8.4) |
| I-415 | 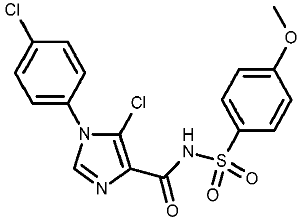 | I-415: ¹H-NMR(400.0 MHz, d₆-DMSO): |
| | | 6= 8.2107(5.3);8.0963(1.6);7.9651(0.4);7.9573(3.9);7.9398(1.2);7.9350(4.3);7.92 81(0.5);7.7012(0.5);7.6940(3.8);7.6891(1.4);7.6722(5.8);7.6653(0.7);7.5991(1.6); 7.5889(5.4);7.5835(1.8);7.5774(1.1);7.5722(1.2);7.5671(3.6);7.5599(0.4);7.1671( 0.4);7.1598(4.1);7.1374(3.9);7.1302(0.5);3.8521(16.0);3.3280(1.5);2.5106(18.1); 2.5065(36.6);2.5020(48.7);2.4976(35.9);1.5303(11.3);-0.0002(7.7) |
| I-416 | 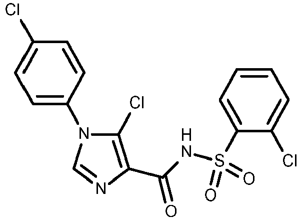 | I-416: ¹H-NMR(400.0 MHz, d₆-DMSO): |
| | | δ=8.2301(5.4);8.2226(2.9);8.1607(3.1);8.1413(3.4);8.0249(0.8);8.0064(1.0);7.71 57(1.2);7.6964(9.4);7.6744(16.0);7.6579(1.9);7.6541(2.0);7.6389(1.4);7.6201(2.9 );7.6128(12.3);7.5910(9.4);7.5743(0.7);7.5691(1.2);3.5073(0.5);3.3858(0.5);3.23 40(1.3);3.1866(0.6);2.6755(0.7);2.6713(0.9);2.6669(0.7);2.5065(102.1);2.5022(1 33.0);2.4978(99.8);2.3332(0.6);2.3290(0.8);2.3245(0.6);2.0750(0.8);-0.0002(6.5) |
| I-417 | 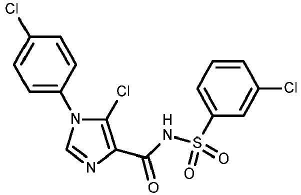 | I-417: ¹H-NMR(400.0 MHz, d₆-DMSO): |
| | | δ=8.2479(16.0);8.0171(4.2);8.0126(7.9);8.0079(5.3);7.9809(3.9);7.9771(2.8);7.9 611(4.4);7.8248(2.8);7.8221(2.7);7.8198(2.6);7.8047(3.8);7.8018(3.9);7.7116(5.2 );7.6993(10.1);7.6919(9.2);7.6828(4.7);7.6775(15.5);7.6717(5.0);7.6058(2.2);7.5 988(14.9);7.5936(4.6);7.5820(3.6);7.5769(9.8);7.5698(1.1);5.7578(5.4);3.5708(0. 3);3.5524(0.3);3.5050(0.4);3.4862(0.4);3.4627(0.4);3.4576(0.4);3.4517(0.4);3.44 58(0.4);3.3891(0.4);3.3717(0.4);3.3567(0.4);3.3453(0.4);3.3014(0.4);3.2844(0.4); 3.2767(0.4);3.2614(0.4);3.2449(0.4);3.1873(0.3);2.8908(2.4);2.7312(2.0);2.6762( 0.6);2.6717(0.9);2.6671(0.7);2.5111(50.0);2.5070(95.9);2.5026(124.6);2.4981(92 .9);2.3381(0.3);2.3336(0.6);2.3294(0.8);2.3249(0.6);2.0864(0.4);0.1458(0.8);0.00 77(9.5);-0.0002(178.6);-0.0085(8.9);-0.1498(0.8) |
| I-418 | 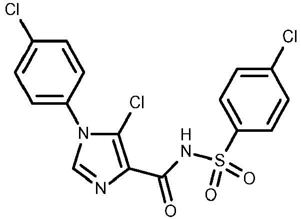 | I-418: ¹H-NMR(400.0 MHz, d₆-DMSO): |
| | | δ=8.2352(16.0);8.0295(1.2);8.0230(10.8);8.0183(3.7);8.0061(3.8);8.0013(12.9); 7.9949(1.6);7.9531(2.0);7.7503(1.5);7.7440(12.3);7.7392(4.0);7.7270(3.5);7.722 3(10.7);7.7159(1.3);7.7043(1.0);7.6971(9.3);7.6920(3.3);7.6805(3.9);7.6752(14.4 );7.6682(1.8);7.6019(1.7);7.5948(14.2);7.5895(4.0);7.5780(3.2);7.5729(9.3);7.56 56(0.9);5.7573(4.4);3.5071(0.4);3.4346(0.4);3.3791(0.5);3.3663(0.5);3.3153(0.5); 3.2874(0.4);3.2196(0.4);3.1978(0.3);3.1873(0.4);2.8912(15.1);2.7319(12.4);2.67 63(0.6);2.6719(0.8);2.6671(0.6);2.5251(1.9);2.5116(43.2);2.5073(86.9);2.5027(1 13.9);2.4982(83.0);2.4939(40.6);2.3340(0.6);2.3295(0.8);2.3251(0.6);0.1460(0.9) ;0.0078(7.3);-0.0002(195.6);-0.0086(7.6);-0.1497(0.9) |
| I-419 | 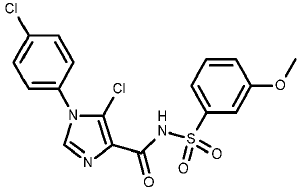 | I-419: ¹H-NMR(400.0 MHz, d₆-DMSO): |
| | | δ=8.2280(5.0);7.6966(3.3);7.6749(5.3);7.5952(5.6);7.5733(6.9);7.5528(2.3);7.53 11(2.0);7.5258(2.4);7.2901(1.2);7.2852(1.0);7.2769(0.8);7.2717(1.2);7.2667(0.8); 3.8351(16.0);3.3344(1.0);3.2339(0.5);3.2171(0.4);2.6711(0.4);2.5062(46.9);2.50 20(63.0);2.4978(50.7);2.3286(0.4);2.0751(0.6);0.8935(0.4);0.0078(2.4);-0.0002(55.4) |
| I-420 | 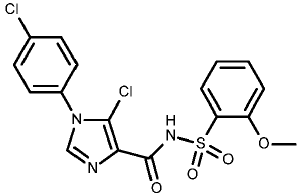 | I-420: ¹H-NMR(400.0 MHz, d₆-DMSO): |
| | | δ= 8.2335(4.8);7.9136(1.5);7.9093(1.7);7.8939(1.7);7.8896(1.8);7.7069(0.4);7.69 99(4.0);7.6947(1.6);7.6830(2.3);7.6778(7.0);7.6709(0.9);7.6617(0.8);7.6575(0.8); 7.6174(0.7);7.6103(5.7);7.6049(1.6);7.5936(1.2);7.5883(3.5);7.5811(0.4);7.2606( 1.8);7.2399(1.7);7.1714(1.0);7.1530(1.8);7.1350(0.9);7.1332(0.9);5.7573(2.8);3.8 819(16.0);3.3280(3.1);2.6753(0.3);2.6711(0.4);2.5243(1.0);2.5196(1.6);2.5108(2 2.8);2.5064(46.8);2.5019(62.0);2.4973(45.3);2.4928(22.1);2.3286(0.4);0.0080(2. 3);-0.0002(71.9);-0.0086(2.6) |
| I-421 | 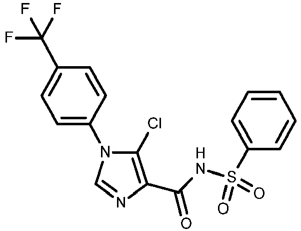 | I-421: ¹H-NMR(400.0 MHz, d₆-DMSO): |
| | | δ=8.3170(0.4);8.2972(16.0);8.0300(9.4);8.0158(11.2);8.0122(14.9);8.0089(10.1) ;7.9946(10.7);7.8154(10.0);7.7946(8.5);7.7384(1.5);7.7194(4.8);7.7015(3.9);7.66 25(7.3);7.6430(10.1);7.6251(3.7);3.3332(4.6);2.8906(0.7);2.7308(0.6);2.6803(0.4 );2.6759(0.9);2.6713(1.2);2.6668(0.9);2.6623(0.4);2.5247(3.0);2.5199(4.7);2.511 2(68.5);2.5068(141.2);2.5023(188.4);2.4977(138.5);2.4933(68.0);2.3336(0.9);2.3 290(1.3);2.3245(0.9);2.0752(8.4);0.1459(0.5);0.0080(3.5);-0.0002(113.1);-0.0085(4.0);-0.1498(0.5) |
| I-422 | 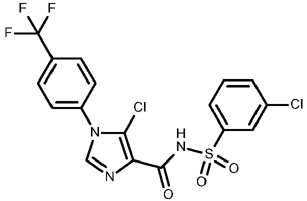 | I-422: ¹H-NMR(400.0 MHz, d₆-DMSO): |
| | | δ=8.3192(16.0);8.0217(10.2);8.0168(9.6);8.0119(5.5);8.0011(8.4);7.9879(2.9);7. 9854(3.6);7.9814(2.4);7.9683(3.2);7.9657(3.8);7.9617(2.8);7.9531(0.6);7.8232(1 0.0);7.8028(9.1);7.7153(4.6);7.6955(7.1);7.6755(3.0);5.7580(4.1);3.6027(0.4);3.5 339(0.5);3.3680(1.0);3.3561(1.0);3.3481(1.0);3.2528(0.7);3.1873(0.5);3.0524(0.3 );2.8908(4.2);2.7311(3.4);2.6800(0.5);2.6760(0.9);2.6714(1.2);2.6669(0.9);2.524 8(3.3);2.5113(64.6);2.5069(128.6);2.5024(167.4);2.4978(121.0);2.4934(58.0);2.3 378(0.4);2.3336(0.8);2.3291(1.1);2.3246(0.8);0.0079(1.0);-0.0002(30.8);-0.0085(1.0) |
| I-423 | 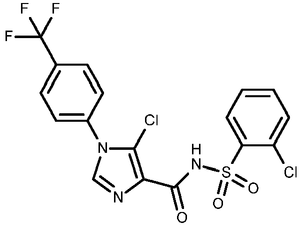 | I-423: ¹H-NMR(400.0 MHz, d₆-DMSO): |
| | | δ=8.3211(16.0);8.1774(3.9);8.1736(4.2);8.1575(4.3);8.1540(4.3);8.0213(6.9);8.0 000(8.5);7.8411(8.1);7.8203(6.7);7.7394(1.0);7.7355(1.0);7.7193(3.1);7.7155(3.0 );7.7021(4.3);7.6982(4.3);7.6920(5.4);7.6879(6.8);7.6722(2.4);7.6681(1.4);7.644 2(2.9);7.6399(2.5);7.6265(2.7);7.6243(3.5);7.6201(2.8);7.6072(2.0);7.6027(1.8);5 .7578(4.8);3.5230(0.5);3.5062(0.5);3.4259(0.6);3.4100(0.7);3.3816(0.7);3.3536(0 .7);3.3361(0.7);3.3060(0.6);3.3025(0.6);3.2902(0.6);3.2088(0.4);3.1869(0.5);3.17 11(0.4);2.8905(0.4);2.6801(0.4);2.6757(0.9);2.6711(1.3);2.6666(0.9);2.6622(0.4); 2.5245(2.8);2.5197(4.4);2.5110(70.8);2.5066(145.1);2.5021(192.3);2.4975(140.6 );2.4930(68.7);2.3377(0.5);2.3334(1.0);2.3288(1.3);2.3243(1.0);2.3198(0.5);0.00 79(1.0);-0.0002(36.8);-0.0085(1.3) |
| I-424 | 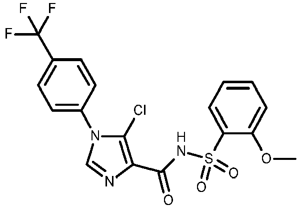 | I-424: ¹H-NMR(400.0 MHz, d₆-DMSO): |
| | | δ=8.3102(5.1);8.0229(2.8);8.0016(3.4);7.9190(1.6);7.9148(1.7);7.8993(1.7);7.89 51(1.8);7.8348(3.2);7.8140(2.7);7.7065(0.7);7.7023(0.7);7.6845(1.3);7.6669(0.8); 7.6628(0.8);7.2660(1.9);7.2453(1.7);7.1761(1.0);7.1577(1.9);7.1395(0.9);3.8876( 16.0);3.3271(6.5);2.6753(0.4);2.6709(0.6);2.6663(0.4);2.5242(1.5);2.5106(33.9); 2.5064(67.5);2.5019(88.3);2.4973(64.7);2.4929(31.9);2.3330(0.4);2.3287(0.6);2. 3241(0.4);2.0860(7.2);2.0750(0.3);0.0080(0.5);-0.0002(15.2);-0.0085(0.6) |
| I-425 | 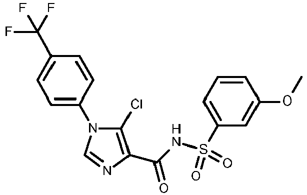 | I-425: ¹H-NMR(400.0 MHz, d₆-DMSO): |
| | | δ=8.3040(5.3);8.0202(2.5);7.9990(3.1);7.8205(2.9);7.7998(2.5);7.6001(0.6);7.59 60(0.5);7.5843(1.6);7.5806(2.8);7.5768(3.2);7.5583(2.2);7.5373(1.8);7.5310(2.0); 7.5269(1.5);7.3012(0.8);7.2970(1.1);7.2903(0.8);7.2821(0.8);7.2776(1.0);7.2717( 0.7);5.7579(0.7);3.8381(16.0);3.3340(1.0);2.6711(0.4);2.5244(1.2);2.5109(26.3); 2.5067(51.0);2.5022(65.9);2.4977(48.4);2.4936(24.2);2.3334(0.3);2.3289(0.4);2. 3245(0.3);2.0862(0.8);0.0078(0.4);-0.0002(10.8);-0.0085(0.4) |
| I-426 | 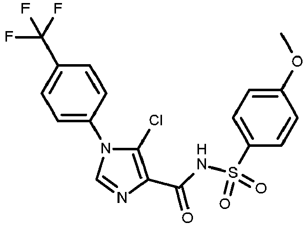 | I-426: ¹H-NMR(400.0 MHz, d₆-DMSO): |
| | | δ=8.2884(6.1);8.0171(2.6);7.9958(3.1);7.9712(0.4);7.9637(4.1);7.9589(1.4);7.94 64(1.2);7.9414(4.5);7.9341(0.5);7.8144(3.0);7.7936(2.5);7.1725(0.4);7.1650(4.3); 7.1601(1.4);7.1476(1.2);7.1426(4.1);7.1352(0.4);5.7579(1.3);3.8548(16.0);3.328 4(1.3);2.5247(0.8);2.5199(1.2);2.5112(17.4);2.5068(36.0);2.5023(48.2);2.4978(3 5.8);2.4934(17.9);-0.0002(7.9) |
| I-427 | 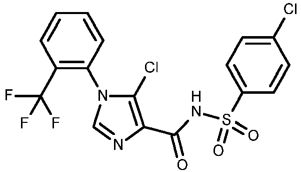 | I-427: ¹H-NMR(400.0 MHz, d₆-DMSO): |
| | | δ=8.2664(10.6);8.0477(5.0);8.0403(13.8);8.0351(6.1);8.0237(5.1);8.0186(16.0); 8.0124(2.1);7.9651(1.6);7.9461(4.2);7.9291(3.3);7.8957(3.4);7.8765(4.4);7.8574( 1.6);7.7814(5.5);7.7612(5.9);7.7543(15.6);7.7498(5.3);7.7374(4.0);7.7326(13.5); 7.7265(1.7);3.3624(0.4);3.3520(0.4);2.6771(0.4);2.6725(0.6);2.6682(0.4);2.5258( 1.1);2.5120(30.5);2.5078(63.3);2.5034(85.6);2.4989(64.8);2.4948(33.3);2.3344(0 .4);2.3301(0.6);2.3258(0.4);-0.0003(3.9) |
| I-428 | 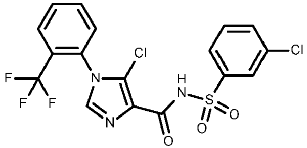 | I-428: ¹H-NMR(400.0 MHz, d₆-DMSO): |
| | | δ=8.2742(16.0);8.0500(6.4);8.0295(14.4);8.0246(15.2);8.0199(9.8);7.9966(7.2); 7.9767(8.0);7.9489(6.7);7.9300(5.4);7.8963(5.5);7.8772(7.0);7.8583(2.6);7.8370( 5.1);7.8344(4.8);7.8320(4.6);7.8169(6.8);7.8140(6.9);7.8120(6.2);7.7867(8.7);7.7 675(7.0);7.7224(9.0);7.7025(14.0);7.6825(6.0);3.3562(2.4);3.1185(0.6);3.0497(0. 4);2.6759(1.4);2.6715(1.9);2.6671(1.4);2.5248(4.4);2.5112(104.4);2.5069(211.0); 2.5024(280.2);2.4980(208.7);2.4938(105.0);2.3336(1.4);2.3292(1.8);2.3247(1.4); -0.0002(8.1) |
| I-429 | 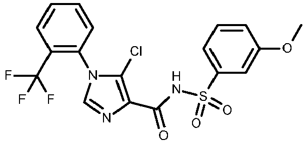 | I-429: ¹H-NMR(400.0 MHz, d₆-DMSO): |
| | | δ=8.2577(2.5);8.0472(1.0);8.0276(1.3);7.9632(0.4);7.9463(1.1);7.9439(1.1);7.92 75(0.9);7.8937(0.9);7.8747(1.2);7.8561(0.4);7.7809(1.4);7.7616(1.1);7.6111(0.7); 7.6071(0.5);7.5953(1.3);7.5916(2.4);7.5875(1.6);7.5845(2.0);7.5650(2.2);7.5422( 1.4);7.5364(1.9);7.5322(1.4);7.3085(0.8);7.3048(0.9);7.3022(0.9);7.2981(0.8);7.2 890(0.7);7.2852(0.8);7.2831(0.8);7.2789(0.7);3.8415(16.0);3.3285(1.4);2.6711(0. 4);2.5245(0.8);2.5197(1.2);2.5111(19.7);2.5066(41.4);2.5020(56.1);2.4975(42.0); 2.4930(21.1);2.3289(0.4) |
| I-430 | 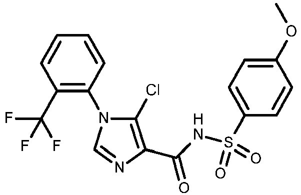 | I-430: ¹H-NMR(400.0 MHz, d₆-DMSO): |
| | | δ=8.2410(2.9);8.0447(1.2);8.0250(1.4);7.9810(0.4);7.9735(4.1);7.9685(1.4);7.95 62(1.4);7.9511(4.6);7.9438(1.7);7.9251(1.0);7.8916(1.0);7.8726(1.3);7.8536(0.5); 7.7726(1.6);7.7534(1.3);7.1771(0.5);7.1696(4.3);7.1646(1.4);7.1521(1.2);7.1471( 4.1);7.1397(0.4);3.8573(16.0);3.3283(1.8);2.5246(0.6);2.5112(15.1);2.5068(31.0) ;2.5022(41.3);2.4977(30.7);2.4933(15.3);2.0752(2.5) |
| I-431 | 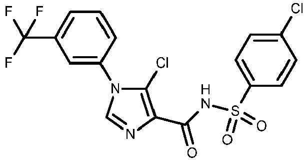 | I-431: ¹H-NMR(400.0 MHz, d₆-DMSO): |
| | | δ=8.3541(0.5);8.3165(0.7);8.3059(16.0);8.0376(6.4);8.0329(5.1);8.0255(12.0);8. 0207(4.1);8.0087(4.0);8.0038(14.1);7.9974(2.0);7.9805(3.0);7.9623(4.0);7.8977( 1.9);7.8934(1.4);7.8814(4.0);7.8774(6.1);7.8671(5.2);7.8483(4.1);7.8284(1.4);7.7 518(1.6);7.7454(12.9);7.7406(4.2);7.7284(3.6);7.7236(11.2);7.7173(1.4);3.3399( 1.5);3.2521(1.1);3.1867(0.5);2.6758(1.1);2.6712(1.5);2.6667(1.1);2.6620(0.6);2.5 246(3.8);2.5198(5.6);2.5111(78.5);2.5067(161.9);2.5021(217.0);2.4976(159.6);2. 4931(77.6);2.3378(0.4);2.3335(1.0);2.3289(1.4);2.3243(1.0);-0.0002(1.2) |
| I-432 | 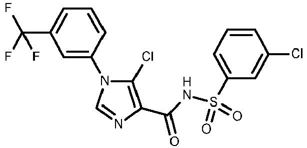 | I-432: ¹H-NMR(400.0 MHz, d₆-DMSO): |
| | | δ=8.3128(16.0);8.0400(9.5);8.0170(5.5);8.0125(10.7);8.0080(7.5);7.9805(9.3);7. 9626(10.8);7.9007(3.0);7.8803(8.8);7.8685(7.5);7.8496(6.3);7.8296(2.3);7.8205( 3.7);7.7975(5.2);7.7096(6.2);7.6897(9.6);7.6697(4.1);3.8907(0.3);3.8617(0.4);3.8 426(0.4);3.7979(0.4);3.7781(0.4);3.7713(0.5);3.7448(0.5);3.6977(0.6);3.6602(0.8 );3.3446(15.1);3.1416(1.5);3.0532(0.9);2.8910(0.4);2.8671(0.4);2.8561(0.3);2.77 20(0.3);2.6755(3.2);2.6710(4.4);2.6666(3.3);2.5243(10.3);2.5106(232.9);2.5064( 480.8);2.5019(646.2);2.4974(490.4);2.4933(250.0);2.3331(3.2);2.3287(4.4);2.324 1(3.2);1.2349(0.4);0.1459(4.1);0.0486(0.4);0.0413(0.4);0.0079(30.8);-0.0002(913.4);-0.0085(37.5);-0.0203(1.4);-0.0284(0.7);-0.0364(0.4);-0.1498(4.1) |
| I-433 | 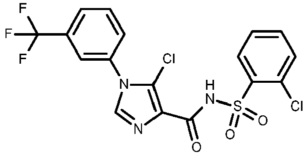 | I-433: ¹H-NMR(601.6 MHz, d₆-DMSO): |
| | | δ=19.9752(1.6);8.3222(16.0);8.1720(6.7);8.1697(6.9);8.1588(7.4);8.1565(7.1);8. 0645(10.1);7.9782(5.4);7.9654(6.6);7.9153(4.3);7.9025(6.6);7.8646(5.6);7.8514( 7.8);7.8384(3.2);7.7281(2.0);7.7148(5.3);7.7028(5.2);7.6862(9.1);7.6748(4.3);7.6 361(4.1);7.6229(6.4);7.6111(3.1);3.4151(35.0);2.6148(3.7);2.5208(8.9);2.5178(8. 8);2.5058(300.0);2.5030(390.8);2.5001(280.0);2.3870(2.6);0.0965(1.8);-0.0002(399.9);-0.0057(16.6);-0.1001(1.8) |
| I-434 | 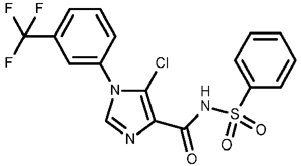 | I-434: ¹H-NMR(601.6 MHz, d₆-DMSO): |
| | | δ=19.9713(1.2);8.2966(16.0);8.0343(11.7);8.0260(15.5);8.0135(15.4);7.9747(6.1 );7.9620(7.5);7.8897(4.2);7.8759(8.6);7.8593(7.1);7.8462(8.2);7.8331(3.1);7.728 8(2.8);7.7165(7.6);7.7042(5.4);7.6562(10.4);7.6432(15.6);7.6304(6.9);3.3561(42. 1);2.6144(2.4);2.5053(245.0);2.5026(321.0);2.4998(240.8);2.3868(2.2);0.0962(1. 0);-0.0002(204.2);-0.1000(1.0) |
| I-435 | 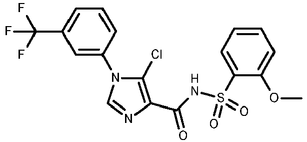 | I-435: ¹H-NMR(400.0 MHz, d₆-DMSO): |
| | | δ=8.3108(4.6);8.0542(2.4);7.9825(1.2);7.9636(1.6);7.9179(2.1);7.9140(2.5);7.89 80(3.2);7.8942(3.7);7.8712(1.7);7.8517(1.8);7.8320(0.6);7.7049(0.6);7.7012(0.7); 7.6835(1.4);7.6656(0.8);7.6619(0.8);7.2676(2.0);7.2467(1.8);7.1759(1.1);7.1571( 2.0);7.1384(1.0);3.8866(16.0);3.8402(0.5);3.3273(6.7);2.6758(0.7);2.6711(0.5);2. 6666(0.4);2.5240(1.1);2.5062(56.3);2.5018(74.5);2.4974(55.6);2.3328(0.4);2.328 6(0.5);2.3242(0.4);0.1459(0.5);0.0079(3.5);-0.0002(102.4);-0.0084(4.3);-0.1498(0.5) |
| I-436 | 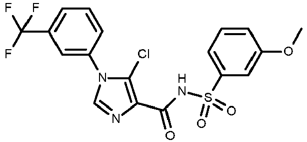 | I-436: ¹H-NMR(601.6 MHz, d₆-DMSO): |
| | | δ=8.3046(4.3);8.0389(1.9);7.9772(1.0);7.9643(1.2);7.8952(0.7);7.8813(1.4);7.86 33(1.2);7.8504(1.4);7.8372(0.5);7.5945(0.8);7.5814(2.0);7.5717(1.6);7.5586(2.0); 7.5456(0.8);7.5354(1.4);7.5316(1.9);7.5286(1.4);7.2925(0.8);7.2793(0.8);3.8378( 16.0);3.3325(1.0);2.6143(0.4);2.5236(0.6);2.5205(0.7);2.5174(0.7);2.5085(20.8); 2.5056(45.6);2.5026(63.4);2.4996(46.2);2.4966(21.5);2.3867(0.4);0.0965(0.4);0. 0052(2.8);-0.0002(87.6);-0.0057(2.8);-0.1001(0.4) |
| I-437 | 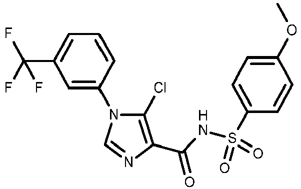 | I-437: ¹H-NMR(601.6 MHz, d₆-DMSO): |
| | | δ=8.2861(3.0);8.0308(1.9);7.9744(1.0);7.9580(3.9);7.9430(3.6);7.8878(0.7);7.87 40(1.5);7.8602(1.3);7.8473(1.4);7.8341(0.5);7.1587(3.2);7.1438(3.0);3.8536(16.0 );3.3323(5.9);2.6143(0.5);2.5236(0.8);2.5205(1.0);2.5174(1.0);2.5086(28.1);2.50 56(61.2);2.5026(85.0);2.4996(60.8);2.4966(27.5);2.3867(0.5);0.0965(0.5);0.0052 (3.6);-0.0002(115.2);-0.0058(3.6);-0.1000(0.5) |
| I-438 | 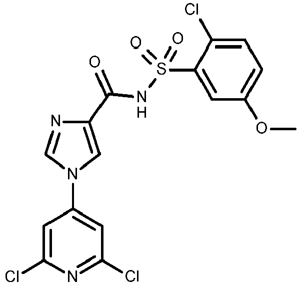 | I-438: ¹H-NMR(400.0 MHz, d₆-DMSO): |
| | | δ= 8.8253(3.0);8.8225(3.4);8.7705(3.4);8.7679(3.2);8.1312(11.2);7.6178(2.9);7.6 101(3.0);7.5809(2.6);7.5589(3.0);7.3033(1.6);7.2957(1.5);7.2814(1.4);7.2736(1.3 );3.8596(16.0);3.1687(10.7);2.5064(31.8);2.5021(41.9);2.4978(31.3);0.0073(0.5); -0.0002(13.3) |
| I-439 | 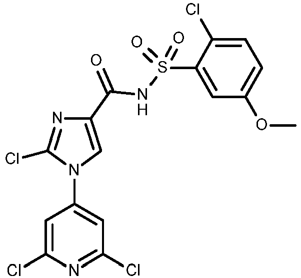 | I-439: ¹H-NMR(400.0 MHz, d₆-DMSO): |
| | | δ=8.3808(6.0);8.1035(1.1);8.0141(12.4);7.6120(2.9);7.6043(3.2);7.5929(3.0);7.5 857(0.4);7.5708(3.1);7.5633(0.4);7.3066(1.6);7.2989(1.5);7.2846(1.4);7.2768(1.3 );3.8618(16.0);3.8181(0.4);3.1888(0.4);3.1685(0.5);2.6705(0.3);2.5650(0.4);2.50 59(41.9);2.5015(55.8);2.4972(41.7);2.3279(0.3);0.0072(0.3);-0.0003(8.7);-0.0084(0.4) |
| I-440 | 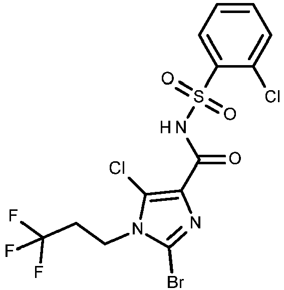 | I-440: ¹H-NMR(400.0 MHz, d₆-DMSO): |
| | | δ= 8.1411(4.9);8.1377(5.6);8.1213(5.4);8.1180(5.8);7.7249(1.3);7.7212(1.5);7.70 18(4.2);7.6872(4.4);7.6837(4.9);7.6660(8.5);7.6494(3.1);7.6465(3.1);7.6280(3.2); 7.6250(3.4);7.6079(4.9);7.5907(2.1);7.5875(2.2);5.7559(16.0);4.3081(4.9);4.290 7(10.7);4.2728(6.7);4.2540(1.5);3.3411(3.8);2.8872(0.8);2.8710(1.9);2.8596(2.5); 2.8552(2.4);2.8429(4.6);2.8318(3.0);2.8266(3.4);2.8149(4.4);2.7980(2.3);2.7869( 1.5);2.7698(0.6);2.6755(0.6);2.6711(0.8);2.6667(0.6);2.5498(0.6);2.5105(53.5);2. 5065(102.9);2.5021(134.1);2.4977(100.8);2.3333(0.6);2.3288(0.8);2.3245(0.6);1. 2348(0.7);0.0077(2.4);-0.0002(48.6);-0.0077(2.4) |
| I-441 | 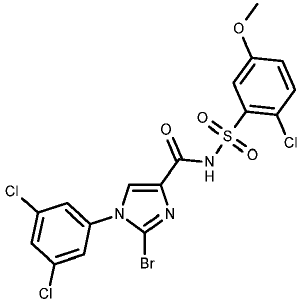 | I-441: ¹H-NMR(400.0 MHz, d₆-DMSO): |
| | | δ= 8.3430(5.5);7.9035(1.8);7.8989(3.5);7.8944(2.2);7.8022(7.3);7.7977(7.0);7.61 40(2.9);7.6063(3.2);7.5977(2.7);7.5757(3.0);7.3060(1.5);7.2984(1.5);7.2839(1.4); 7.2763(1.3);3.8637(16.0);2.6705(0.4);2.5649(0.4);2.5058(45.2);2.5015(58.5);2.4 971(43.5);2.3283(0.3);1.2581(1.1);-0.0001(1.2) |
| I-442 | 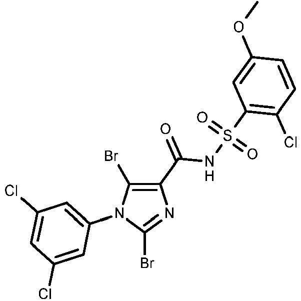 | I-442: ¹H-NMR(400.0 MHz, d₆-DMSO): |
| | | δ= 8.3450(0.5);7.9670(1.6);7.9626(3.0);7.9580(1.8);7.8882(1.4);7.8836(1.2);7.86 79(5.6);7.8633(5.2);7.8027(0.5);7.7981(0.5);7.6021(3.1);7.5949(5.1);7.5735(2.8); 7.3005(1.6);7.2929(1.4);7.2785(1.4);7.2708(1.2);3.8581(16.0);3.3491(0.9);2.670 5(0.3);2.5058(45.2);2.5015(58.8);2.4971(43.2);2.3282(0.4);2.0737(6.1);-0.0002(1.3) |
| I-443 | 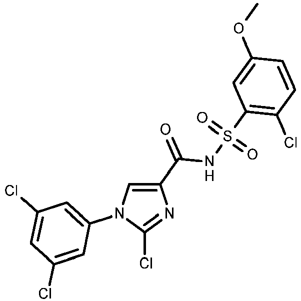 | I-443: ¹H-NMR(400.0 MHz, d₆-DMSO): |
| | | δ= 8.4048(0.8);8.2933(6.2);7.8990(1.8);7.8945(3.4);7.8899(2.4);7.8823(0.7);7.83 22(1.3);7.8216(7.3);7.8171(7.1);7.6156(3.0);7.6080(3.6);7.5978(3.0);7.5757(3.2); 7.3083(1.8);7.3007(1.8);7.2862(1.6);7.2785(1.6);3.8627(16.0);2.6989(0.5);2.565 7(6.2);2.5067(33.0);2.5024(43.3);2.4982(35.6);-0.0002(3.5) |
| I-444 | 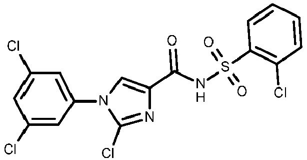 | I-444: ¹H-NMR(400.0 MHz, d₆-DMSO): |
| | | δ= 8.3920(0.4);8.2875(13.8);8.1750(3.1);8.1713(3.4);8.1551(3.4);8.1516(3.6);7.8 954(3.7);7.8908(7.5);7.8862(4.7);7.8281(0.8);7.8234(0.9);7.8148(16.0);7.8102(1 5.0);7.7380(0.8);7.7340(0.9);7.7177(2.7);7.7141(2.6);7.7006(3.5);7.6968(3.6);7.6 895(4.5);7.6855(5.7);7.6696(2.1);7.6657(1.4);7.6431(2.3);7.6389(2.1);7.6232(3.0 );7.6193(2.5);7.6061(1.6);7.6017(1.5);2.6716(0.4);2.5070(44.0);2.5025(57.0);2.4 980(42.6);2.3290(0.3);1.0459(0.3);1.0307(0.3);0.0079(0.3);-0.0002(7.8) |
| I-445 | 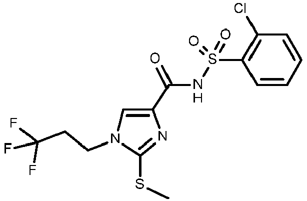 | I-445: ¹H-NMR(400.0 MHz, d₆-DMSO): |
| | | δ= 8.1572(4.8);8.1496(1.6);8.1460(1.6);8.1297(1.6);8.1263(1.6);7.7212(0.4);7.71 76(0.4);7.7010(1.3);7.6835(1.5);7.6799(1.4);7.6655(2.0);7.6626(2.5);7.6463(1.0); 7.6262(1.1);7.6227(0.9);7.6062(1.5);7.5887(0.7);7.5854(0.6);4.2226(1.7);4.2054( 3.6);4.1881(1.8);2.8738(0.4);2.8633(0.7);2.8459(1.4);2.8351(0.9);2.8289(0.8);2.8 181(1.4);2.8008(0.7);2.7906(0.5);2.6161(16.0);2.5063(21.0);2.5021(26.6);2.4979 (19.9);2.0858(1.1);-0.0002(5.2) |
| I-446 | 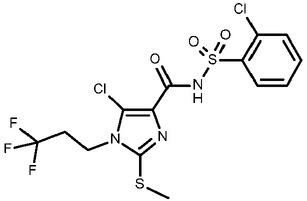 | I-446: ¹H-NMR(400.0 MHz, d₆-DMSO): |
| | | δ= 8.1584(1.5);8.1549(1.6);8.1386(1.6);8.1352(1.7);7.7377(0.4);7.7343(0.4);7.71 76(1.3);7.7156(1.2);7.7000(1.5);7.6964(1.4);7.6795(2.7);7.6632(1.1);7.6406(1.1); 7.6374(0.9);7.6206(1.6);7.6031(0.7);7.5998(0.6);4.2095(1.7);4.1922(3.4);4.1748( 1.7);2.8244(0.5);2.8137(0.7);2.7966(1.4);2.7858(0.9);2.7793(0.8);2.7687(1.4);2.7 513(0.7);2.7409(0.5);2.7014(16.0);2.5066(19.0);2.5025(24.2);2.4986(18.2);2.074 3(1.4);0.0078(0.5);-0.0002(10.5) |
| I-447 | 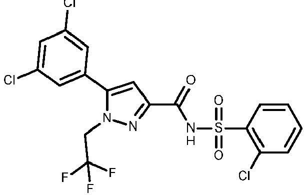 | I-447: ¹H-NMR(400.0 MHz, d₆-DMSO): |
| | | δ= 8.1880(3.4);8.1687(3.7);7.8089(3.9);7.8045(7.4);7.8002(4.5);7.7428(0.9);7.72 45(2.7);7.7051(3.1);7.6880(5.4);7.6709(2.2);7.6448(16.0);7.6404(14.6);7.6297(3. 6);7.6120(1.4);7.1951(7.9);5.2346(1.5);5.2128(4.6);5.1908(4.7);5.1687(1.6);3.38 08(1.7);2.6720(1.0);2.5069(133.8);2.5027(175.4);2.4988(133.6);2.3295(1.0);2.07 45(4.6);0.0077(1.1);-0.0002(32.0);-0.0081(1.4) |
| I-448 | 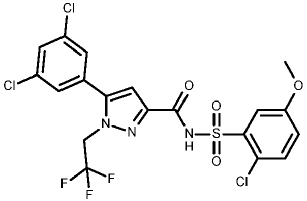 | I-448: ¹H-NMR(400.0 MHz, d₆-DMSO): |
| | | δ= 7.8094(1.9);7.8049(3.3);7.8004(1.9);7.6450(7.0);7.6405(6.4);7.6283(3.0);7.62 06(3.1);7.5969(2.3);7.5748(2.7);7.3168(1.4);7.3092(1.3);7.2948(1.2);7.2870(1.1); 7.1958(4.0);5.2331(0.7);5.2114(2.1);5.1895(2.2);5.1669(0.7);3.8640(16.0);3.352 4(5.2);2.6717(0.4);2.5069(62.0);2.5026(78.7);2.4987(57.3);2.3293(0.5);2.0743(1. 7);1.3094(0.3);0.0078(1.3);-0.0001(26.3) |
| I-449 | 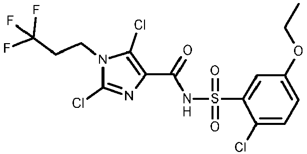 | I-449: ¹H-NMR(400.0 MHz, d₆-DMSO): |
| | | δ= 7.5605(6.0);7.5524(8.2);7.5286(5.5);7.2704(2.8);7.2628(2.7);7.2484(2.4);7.24 08(2.3);5.7559(12.7);4.2897(3.3);4.2728(7.0);4.2560(3.4);4.1359(2.1);4.1186(6.8 );4.1012(6.9);4.0839(2.2);3.3391(7.8);2.9024(0.4);2.8856(0.9);2.8749(1.4);2.857 9(2.7);2.8467(1.8);2.8411(1.7);2.8301(2.8);2.8133(1.4);2.8022(1.0);2.7852(0.4);2 .6749(0.7);2.6706(1.0);2.6661(0.7);2.5099(60.1);2.5059(118.9);2.5015(157.1);2. 4970(117.8);2.3327(0.7);2.3282(1.0);2.3237(0.7);2.0085(0.5);1.9895(0.5);1.3744 (7.5);1.3570(16.0);1.3396(7.4);1.2354(2.9);0.8540(0.7);0.0077(2.3);-0.0003(58.1);-0.0083(2.8) |
| I-450 | 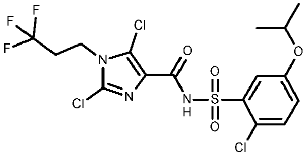 | I-450: ¹H-NMR(400.0 MHz, d₆-DMSO): |
| | | δ= 7.5390(2.9);7.5321(4.6);7.5108(2.5);7.2617(1.2);7.2542(1.2);7.2397(1.0);7.23 21(1.0);5.7546(0.4);4.7057(0.4);4.6912(1.0);4.6761(1.4);4.6612(1.0);4.6464(0.4); 4.2913(1.6);4.2745(3.4);4.2577(1.6);3.3370(162.7);2.8846(0.4);2.8738(0.7);2.85 70(1.3);2.8455(0.9);2.8410(0.8);2.8290(1.4);2.8122(0.7);2.8015(0.5);2.6759(0.6); 2.6713(0.8);2.6668(0.6);2.5243(2.5);2.5109(55.8);2.5067(112.1);2.5023(148.0);2 .4979(108.4);2.3333(0.7);2.3290(0.8);2.3245(0.7);1.3090(16.0);1.2940(15.9);1.2 350(0.6);0.0079(1.5);-0.0002(43.1);-0.0082(1.8) |
| I-451 | 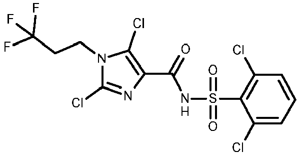 | I-451: ¹H-NMR(400.0 MHz, d₆-DMSO): |
| | | δ= 7.6681(5.1);7.6633(6.4);7.6456(16.0);7.6121(6.4);7.5957(3.9);7.5890(2.8);7.5 727(1.9);7.3939(0.6);7.3901(0.7);7.3719(1.6);7.3276(0.6);7.3109(0.4);5.7564(6.2 );4.2896(4.4);4.2727(9.3);4.2558(4.6);3.6095(0.3);3.5919(0.4);3.3974(3.0);3.231 3(0.5);2.9046(0.7);2.8881(1.4);2.8767(2.0);2.8599(3.7);2.8485(2.5);2.8440(2.3);2 .8320(3.8);2.8154(1.9);2.8042(1.4);2.7867(0.6);2.6748(1.2);2.6702(1.5);2.6661(1 .2);2.5057(184.4);2.5014(240.1);2.4972(183.0);2.3326(1.0);2.3280(1.3);1.2355(1 .5);0.8539(0.4);0.1459(0.4);-0.0003(77.3);-0.1497(0.4) |
| I-452 | 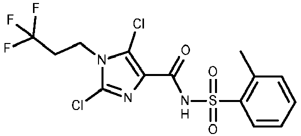 | I-452: ¹H-NMR(400.0 MHz, d₆-DMSO): |
| | | δ= 12.4480(0.4);8.0141(2.1); 7 .9966(2.2); 7.9943(2.2); 7.5874(0.9); 7.5 712(2.0); 7.5 685(2.0);7.5523(1.3);7.5501(1.3);7.4529(1.3);7.4339(2.1);7.4150(1.0);7.3961(2.2 );7.3773(1.8);5.7561(3.4);4.2839(2.4);4.2671(5.0);4.2503(2.4);3.3285(5.4);2.872 7(0.6);2.8615(1.0);2.8449(1.9);2.8334(1.2);2.8283(1.2);2.8170(2.0);2.8002(1.0);2 .7892(0.7);2.7719(0.3);2.6748(0.3);2.6705(0.4);2.6658(0.4);2.5959(16.0);2.5059( 55.4);2.5015(73.4);2.4971(55.3);2.3282(0.4);1.2351(0.7);0.0079(1.1);-0.0002(27.7) |
| I-453 | 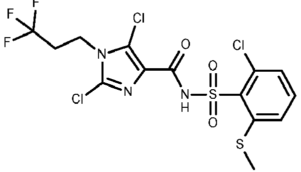 | I-453: ¹H-NMR(400.0 MHz, d₆-DMSO): |
| | | δ= 7.4874(1.6);7.4701(1.3);7.3693(2.2);7.3491(1.9);7.3271(2.3);7.3088(1.8);5.75 64(14.7);5.3252(0.4);4.2785(4.6);4.2616(9.2);4.2448(4.5);4.0381(0.6);4.0204(0.6 );3.3244(2.3);2.8936(0.6);2.8769(1.3);2.8661(2.1);2.8493(3.7);2.8379(2.6);2.833 1(2.4);2.8214(3.8);2.8047(1.9);2.7935(1.4);2.7764(0.6);2.7167(0.5);2.6748(0.5);2 .6709(0.6);2.6665(0.4);2.5238(1.8);2.5061(70.3);2.5017(90.0);2.4974(63.9);2.43 72(16.0);2.3326(0.4);2.3285(0.6);2.3246(0.4);2.0278(0.4);2.0097(0.8);1.9889(3.2 );1.9738(0.4);1.4576(0.3);1.2990(1.2);1.2842(1.0);1.2587(2.2);1.2358(8.0);1.192 2(0.9);1.1746(1.4);1.1569(0.7);0.8699(0.6);0.8539(1.6);0.8368(0.7);-0.0002(0.6) |
| I-454 | 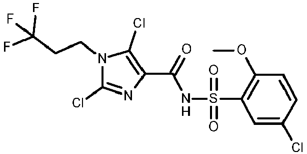 | I-454: ¹H-NMR(400.0 MHz, d₆-DMSO): |
| | | δ= 7.8028(3.1);7.7961(3.8);7.7519(1.5);7.7452(1.2);7.7296(1.6);7.7229(1.3);7.28 88(2.6);7.2664(2.4);5.7558(1.0);4.2921(1.8);4.2753(3.8);4.2584(1.8);3.8511(16.0 );3.3266(13.0);2.8854(0.5);2.8744(0.7);2.8578(1.4);2.8463(0.9);2.8414(0.8);2.82 99(1.5);2.8132(0.7);2.8018(0.5);2.6750(0.4);2.6706(0.5);2.6661(0.4);2.5236(1.5); 2.5101(31.7);2.5059(64.1);2.5014(84.8);2.4970(61.9);2.4928(30.6);2.3323(0.4);2 .3282(0.5);2.3240(0.4);0.0078(1.2);-0.0002(31.6);-0.0085(1.2) |
| I-455 | 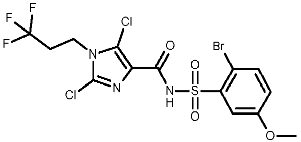 | I-455: ¹H-NMR(400.0 MHz, d₆-DMSO): |
| | | δ= 7.7329(2.4);7.7110(2.6);7.6248(2.8);7.6170(2.9);7.2080(1.3);7.2003(1.3);7.18 60(1.2);7.1784(1.2);4.2914(1.6);4.2746(3.4);4.2576(1.6);3.8454(16.0);3.3351(6.9 );2.8878(0.4);2.8769(0.7);2.8603(1.3);2.8487(0.8);2.8435(0.8);2.8324(1.4);2.815 4(0.7);2.8045(0.5);2.6749(0.4);2.6705(0.6);2.6660(0.4);2.5236(1.6);2.5100(35.7); 2.5059(72.6);2.5015(96.8);2.4970(71.8);2.3325(0.4);2.3282(0.6);2.3238(0.4);1.2 353(1.5);0.8542(0.4);0.0078(1.2);-0.0002(33.1);-0.0080(1.5) |
| I-456 | 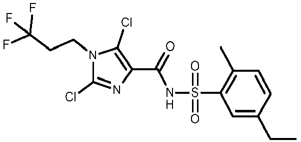 | I-456: ¹H-NMR(400.0 MHz, d₆-DMSO): |
| | | δ=12.3723(0.7);7.8339(4.5);7.8304(4.6);7.8035(0.4);7.4301(1.9);7.4107(2.5);7.3 301(0.4);7.3029(3.7);7.2833(2.7);4.2854(3.3);4.2686(6.8);4.2517(3.3);3.3240(62. 5);2.9940(0.4);2.9753(0.4);2.8902(0.4);2.8733(0.9);2.8626(1.4);2.8456(2.6);2.83 45(1.8);2.8296(1.6);2.8179(2.7);2.8010(1.3);2.7898(1.0);2.7731(0.4);2.6983(1.6); 2.6793(5.3);2.6706(1.7);2.6605(5.3);2.6415(1.7);2.5471(21.0);2.5233(3.5);2.505 8(136.5);2.5014(179.9);2.4970(133.0);2.3622(1.8);2.3326(0.8);2.3282(1.1);2.323 9(0.8);1.2355(1.7);1.2112(7.6);1.1924(16.0);1.1734(7.3);1.1370(0.6);1.1186(1.2); 1.0998(0.6);0.8538(0.4);0.0078(2.2);-0.0002(61.2);-0.0082(2.7) |
| I-457 | 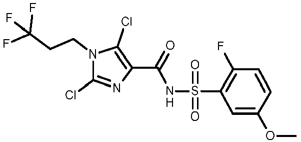 | I-457: ¹H-NMR(400.0 MHz, d₆-DMSO): |
| | | δ= 7.4008(0.7);7.3831(1.3);7.3761(2.5);7.3694(1.3);7.3613(1.4);7.3540(1.2);7.31 55(0.7);7.3061(1.1);7.2979(0.7);7.2837(0.6);7.2752(0.3);4.2900(1.5);4.2731(3.1); 4.2561(1.5);3.8208(16.0);3.3383(3.6);2.8812(0.4);2.8701(0.6);2.8537(1.2);2.842 0(0.8);2.8370(0.7);2.8256(1.2);2.8089(0.6);2.7977(0.4);2.6750(0.4);2.6706(0.5);2 .6663(0.4);2.5234(1.6);2.5101(31.4);2.5059(63.6);2.5014(84.5);2.4970(61.8);2.4 926(30.4);2.3328(0.4);2.3282(0.5);2.3236(0.4);1.2354(0.9);0.0080(1.1);-0.0001(31.2);-0.0083(1.3) |
| I-458 | 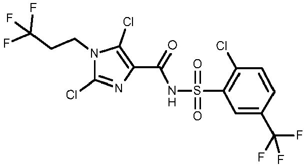 | I-458: ¹H-NMR(400.0 MHz, d₆-DMSO): |
| | | δ= 8.3182(10.9);8.1062(4.7);8.0846(5.7);7.9333(8.2);7.9120(6.6);7.8362(1.0);7.4 314(0.5);7.4139(0.6);7.3067(0.8);7.2865(0.6);5.7562(0.6);5.3247(0.4);5.3131(0.3 );4.2843(7.9);4.2675(16.0);4.2508(8.1);4.0885(0.4);3.9755(0.4);3.9634(0.4);3.94 10(0.5);3.9048(0.5);3.8820(0.5);3.8559(0.5);3.7910(0.7);3.7679(0.7);3.7346(0.9); 3.3909(10.3);3.1075(1.1);3.1021(1.0);3.0312(0.7);2.9946(0.7);2.9765(0.6);2.894 0(1.4);2.8776(2.7);2.8667(3.9);2.8504(6.7);2.8391(5.0);2.8229(6.8);2.8058(3.7);2 .7947(2.7);2.7781(1.4);2.7394(0.4);2.6981(0.7);2.6708(3.6);2.6438(0.7);2.5984(0 .6);2.5475(5.8);2.5019(552.7);2.3629(0.6);2.3284(3.2);2.0252(0.4);2.0097(0.7);1. 9898(0.8);1.2359(4.3);1.2120(1.7);1.1929(2.6);1.1740(1.2);0.8696(0.5);0.8533(0. 9);0.8361(0.5);0.1458(0.7);0.0000(141.3);-0.1492(0.8) |
| I-459 | 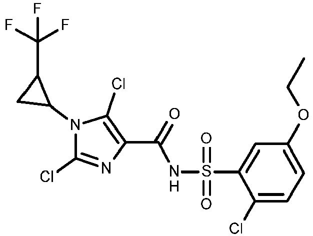 | I-459: ¹H-NMR(400.0 MHz, d₆-DMSO): |
| | | δ= 7.5569(6.1);7.5488(7.5);7.5463(7.1);7.5238(6.4);7.2680(3.2);7.2604(3.1);7.24 60(2.8);7.2383(2.7);5.7543(1.3);4.1360(2.2);4.1187(7.1);4.1013(7.2);4.0840(2.3); 3.7889(1.2);3.7772(2.0);3.7685(2.6);3.7594(1.9);3.7479(1.3);3.3431(2.7);2.8148( 0.6);2.8062(0.7);2.7978(1.1);2.7891(1.4);2.7807(1.3);2.7720(1.4);2.7634(1.1);2.7 547(0.8);2.7461(0.6);2.6706(0.5);2.5056(60.1);2.5014(77.7);2.4973(58.3);2.3281 (0.5);2.0088(0.5);1.9899(0.5);1.7761(0.8);1.7591(2.5);1.7402(2.8);1.7239(2.1);1. 7134(1.8);1.7003(1.7);1.6882(1.7);1.6720(0.7);1.3751(7.7);1.3578(16.0);1.3404( 7.5);1.2922(0.3);1.2356(3.1);0.8540(0.7);0.0073(1.9);-0.0002(43.0) |
| I-460 | 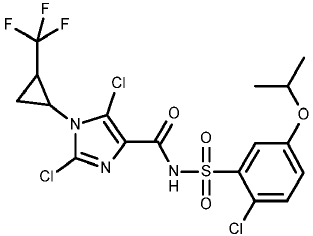 | I-460: ¹H-NMR(400.0 MHz, d₆-DMSO): |
| | | δ= 7.5371(2.9);7.5299(5.3);7.5081(3.0);7.2631(1.5);7.2555(1.4);7.2410(1.2);7.23 35(1.2);5.7541(1.5);4.7093(0.4);4.6944(1.0);4.6794(1.4);4.6644(1.1);4.6492(0.4); 3.7910(0.6);3.7790(0.9);3.7705(1.2);3.7617(0.9);3.7497(0.6);3.3395(2.3);2.7979( 0.5);2.7891(0.6);2.7808(0.6);2.7717(0.6);2.7631(0.5);2.7548(0.4);2.5060(31.9);2. 5016(41.3);2.4972(30.0);1.7772(0.4);1.7598(1.1);1.7407(1.2);1.7248(1.0);1.7129 (0.8);1.7004(0.8);1.6885(0.7);1.3100(16.0);1.2950(15.8);1.2356(0.4);0.0078(1.0); -0.0002(24.6);-0.0083(1.2) |
| I-461 | 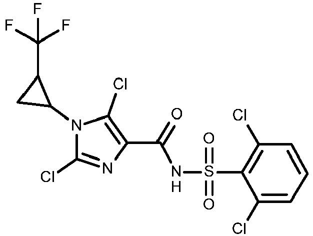 | I-461: ¹H-NMR(400.0 MHz, d₆-DMSO): |
| | | δ= 7.6626(5.1);7.6578(6.4);7.6404(16.0);7.6069(6.2);7.5905(3.8);7.5837(2.8);7.5 674(1.8);3.7885(1.5);3.7766(2.5);3.7680(3.2);3.7592(2.4);3.7473(1.7);3.3631(1.5 );2.8164(0.8);2.8076(0.9);2.7996(1.4);2.7909(1.8);2.7824(1.6);2.7737(1.8);2.765 2(1.4);2.7567(1.0);2.7480(0.8);2.6704(0.7);2.6662(0.6);2.5058(90.0);2.5015(115. 6);2.4973(85.6);2.3282(0.7);2.0736(10.0);1.7767(1.0);1.7595(3.0);1.7405(3.5);1. 7237(2.4);1.7146(2.2);1.7016(2.1);1.6891(2.0);1.6728(0.8);0.0077(3.1);-0.0002(65.0) |
| I-462 | 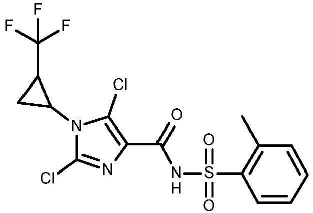 | I-462: ¹H-NMR(400.0 MHz, d₆-DMSO): |
| | | δ= 12.4360(0.4);8.0057(2.1);7.9859(2.2);7.5805(0.8);7.5620(1.9);7.5431(1.2);7.4 473(1.3);7.4282(2.0);7.4092(0.9);7.3878(2.2);7.3691(1.8);5.7542(3.7);3.7792(0.8 );3.7674(1.2);3.7588(1.6);3.7499(1.2);3.7381(0.8);3.3229(14.9);2.7946(0.4);2.78 57(0.5);2.7776(0.7);2.7691(0.9);2.7607(0.8);2.7519(0.9);2.7433(0.7);2.7349(0.5); 2.7260(0.4);2.6700(0.5);2.6662(0.4);2.5874(16.0);2.5056(64.1);2.5012(83.5);2.4 969(61.2);2.3326(0.4);2.3281(0.5);2.3239(0.4);1.7704(0.5);1.7532(1.5);1.7340(1. 7);1.7162(0.9);1.7105(0.9);1.6966(1.0);1.6835(1.0);1.6714(1.0);1.6554(0.4);1.23 56(0.6);0.0079(1.9);-0.0002(45.6);-0.0081(2.0) |
| I-463 | 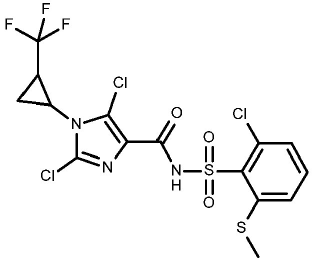 | I-463: ¹H-NMR(400.0 MHz, d₆-DMSO): |
| | | δ= 7.6643(0.4);7.5467(2.0);7.5264(4.7);7.5064(3.6);7.4895(0.4);7.4837(0.4);7.47 95(0.5);7.4594(0.4);7.4043(4.0);7.3838(3.0);7.3596(4.5);7.3578(4.5);7.3403(3.5); 7.3382(3.4);5.7561(16.0);3.7912(1.2);3.7791(2.0);3.7706(2.5);3.7620(1.9);3.750 0(1.3);3.3500(1.6);2.8190(0.6);2.8108(0.7);2.8023(1.0);2.7936(1.4);2.7851(1.3);2 .7764(1.4);2.7678(1.1);2.7594(0.8);2.7506(0.6);2.7186(0.8);2.6742(0.3);2.6698(0 .4);2.6344(0.8);2.5230(1.4);2.5053(55.7);2.5009(72.6);2.4965(53.3);2.4627(22.6) ;2.4402(1.6);2.4180(1.9);2.3321(0.3);2.3276(0.4);2.3233(0.3);1.7753(0.8);1.7582 (2.5);1.7390(2.8);1.7223(1.9);1.7137(1. 7);1.7006(1.6);1.6882(1.6);1.6719(0.7);1. 3779(0.5);1.2352(1.0);1.1173(7.8);1.0999(0.8);1.0952(0.5);-0.0002(0.7) |
| I-464 | 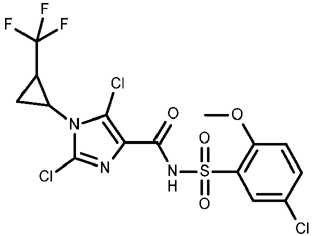 | I-464: ¹H-NMR(400.0 MHz, d₆-DMSO): |
| | | δ= 7.7975(3.1);7.7907(3.8);7.7504(1.8);7.7436(1.4);7.7281(1.8);7.7213(1.6);7.28 58(2.9);7.2634(2.6);3.8542(16.0);3.7923(0.6);3.7803(1.0);3.7717(1.3);3.7630(0.9 );3.7511(0.7);3.6511(0.8);3.3303(8.8);2.8061(0.4);2.7977(0.5);2.7893(0.7);2.780 6(0.6);2.7720(0.7);2.7633(0.5);2.7548(0.4);2.6749(0.4);2.6705(0.5);2.6660(0.3);2 .5100(30.6);2.5059(57.5);2.5014(73.7);2.4969(54.4);2.3326(0.3);2.3282(0.4);2.3 238(0.3);2.0733(2.6);1.7784(0.4);1.7610(1.2);1.7417(1.3);1.7244(1.2);1.7108(0.8 );1.6980(08);1.6860(0.8);1.6693(0.3);0.0079(0.8);-0.0002(18.8);-0.0084(0.8) |
| I-465 | 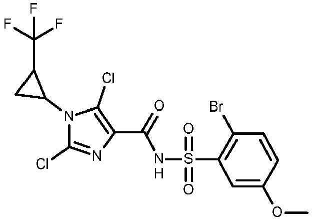 | I-465: ¹H-NMR(400.0 MHz, d₆-DMSO): |
| | | δ= 7.7295(2.6);7.7076(2.9);7.6221(2.9);7.6144(3.0);7.2065(1.5);7.1988(1.4);7.18 46(1.4);7.1768(1.3);3.8464(16.0);3.7931(0.6);3.7813(1.0);3.7725(1.2);3.7633(0.9 );3.7519(0.6);3.3390(2.3);2.8111(0.4);2.8032(0.5);2.7943(0.7);2.7858(0.6);2.777 1(0.7);2.7686(0.5);2.7603(0.4);2.5057(33.6);2.5015(43.6);2.4973(32.4);2.0734(2. 8);1.7771(0.4);1.7601(1.2);1.7410(1.3);1.7238(0.9);1.7162(0.8);1.7034(0.8);1.69 07(0.8);0.0075(1.0);-0.0002(23.3) |
| I-466 | 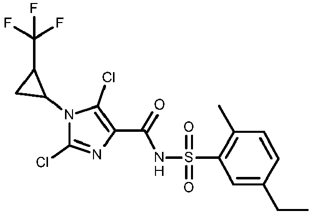 | I-466: ¹H-NMR(400.0 MHz, d₆-DMSO): |
| | | δ= 12.3682(0.7);7.8278(4.4);7.8240(4.4);7.7995(0.4);7.4264(1.9);7.4228(1.8);7.4 071(2.4);7.4036(2.4);7.3273(0.4);7.3071(0.4);7.2973(3.8);7.2778(2.8);5.7564(2.0 );3.7847(1.0);3.7728(1.7);3.7642(2.2);3.7554(1.5);3.7435(1.0);3.3257(14.2);2.98 80(0.5);2.9691(0.5);2.7991(0.5);2.7909(0.6);2.7821(0.9);2.7735(1.2);2.7649(1.1); 2.7562(1.2);2.7474(0.9);2.7390(0.7);2.7305(0.5);2.6963(1.7);2.6773(5.1);2.6586( 5.0);2.6395(1.7);2.5386(20.6);2.5232(2.4);2.5096(47.2);2.5054(93.6);2.5010(121 .3);2.4966(86.4);2.3599(1.8);2.3321(0.5);2.3278(0.7);2.3233(0.5);1.7731(0.7);1.7 559(2.1);1.7367(2.3);1.7191(1.2);1.7118(1.1);1.6982(1.3);1.6849(1.4);1.6730(1.3 );1.6570(0.6);1.2353(0.8);1.2098(7.5);1.1908(16.0);1.1719(7.2);1.1468(0.6);1.12 82(1.3);1.1096(0.6);-0.0002(1.2) |
| I-467 | 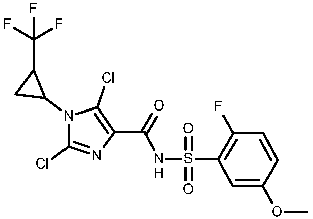 | I-467: ¹H-NMR(400.0 MHz, d₆-DMSO): |
| | | δ= 7.3992(0.8);7.3762(2.8);7.3690(1.6);7.3628(1.3);7.3533(2.0);7.3147(0.8);7.30 54(1.3);7.2972(0.7);7.2924(0.6);7.2831(0.7);7.2745(0.3);5.7565(1.3);3.8206(16.0 );3.7880(0.6);3.7765(0.9);3.7677(1.1);3.7589(0.8);3.7471(0.6);3.3594(0.6);2.789 7(0.4);2.7810(0.6);2.7727(0.6);2.7640(0.6);2.7553(0.5);2.7470(0.3);2.5054(38.3); 2.5010(50.3);2.4966(36.7);1.7788(0.4);1.7616(1.1);1.7427(1.2);1.7238(0.8);1.70 80(0.7);1.6947(0.7);1.6823(0.7);-0.0002(0.4) |
| I-468 | 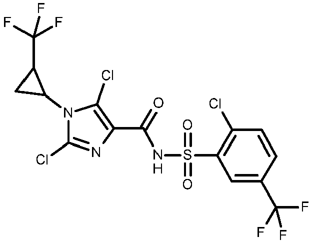 | I-468: ¹H-NMR(400.0 MHz, d₆-DMSO): |
| | | δ= 8.3204(15.0);8.3156(16.0);8.1646(0.5);8.1173(6.8);8.1124(6.7);8.0963(8.5);8. 0914(8.4);7.9382(13.5);7.9174(11.1);7.8363(0.5);7.7512(0.3);7.6130(0.5);7.5927 (0.4);5.7566(2.2);3.9885(0.4);3.9482(0.5);3.7852(4.5);3.7733(7.0);3.7646(8.9);3. 7562(6.7);3.7440(5.0);3.4571(2.3);2.8080(2.0);2.7991(2.4);2.7912(3.5);2.7823(4. 5);2.7739(4.2);2.7651(4.8);2.7561(3.7);2.7480(2.7);2.7392(2.2);2.7317(0.9);2.72 17(0.7);2.6747(2.8);2.6704(3.7);2.6658(2.8);2.5236(10.8);2.5100(226.1);2.5058( 460.3);2.5013(608.0);2.4968(439.0);2.4926(215.2);2.3324(2.5);2.3280(3.5);2.323 6(2.6);1.7758(2.7);1.7588(7.8);1.7394(8.4);1.7214(6.8);1.7069(4.9);1.6937(5.0);1 .6814(4.9);1. 6652(2.2);1.2352(1.4);0.8543(0.4);-0.0002(5.9) |
| I-469 | 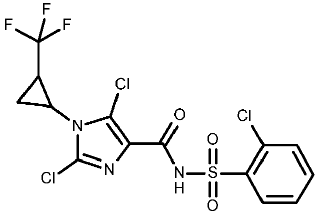 | I-469: ¹H-NMR(400.0 MHz, d₆-DMSO): |
| | | δ= 12.6175(0.4);12.6100(0.3);8.1349(7.7);8.1312(8.2);8.1152(8.6);8.1114(8.6);7. 7200(2.3);7.7160(2.4);7.6999(6.4);7.6963(5.8);7.6822(7.5);7.6783(7.2);7.6610(1 0.4);7.6578(13.1);7.6413(5.8);7.6379(4.5);7.6242(5.9);7.6206(5.0);7.6044(7.8);7. 5867(3.8);7.5830(3.3);5.7565(16.0);5.3364(0.4);5.3250(0.6);5.3133(0.4);3.7875( 2.8);3.7754(4.6);3.7668(6.0);3.7582(4.4);3.7462(3.1);3.5069(0.3);3.3517(3.8);2.8 286(0.3);2.8116(1.3);2.8027(1.6);2.7945(2.4);2.7859(3.2);2.7774(2.9);2.7686(3.3 );2.7600(2.5);2.7516(1.8);2.7428(1.4);2.7346(0.5);2.7252(0.4);2.6748(0.9);2.670 1(1.2);2.6659(0.9);2.5234(3.8);2.5099(82.1);2.5057(164.0);2.5012(213.3);2.4967 (152.3);2.3324(1.0);2.3279(1.3);2.3236(1.0);2.0739(0.8);2.0274(0.7);2.0088(1.2); 1.9900(1.2);1.9739(0.6);1.7747(1.9);1.7576(5.8);1.7383(6.3);1.7214(5.8);1.7087( 3.8);1.6958(3.8);1.6835(3.7);1.6673(1.6);1.4747(0.3);1.4571(0.4);1.2919(0.7);1.2 357(6.9);0.8708(0.6);0.8539(1.8);0.8365(0.7);-0.0001(2.1) |
| I-470 | 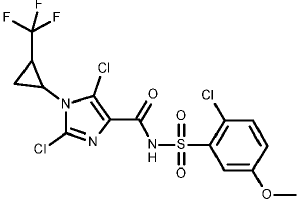 | I-470: ¹H-NMR(400.0 MHz, d₆-DMSO): |
| | | δ= 7.5755(2.9);7.5672(4.4);7.5438(2.8);7.2878(1.4);7.2801(1.3);7.2657(1.2);7.25 80(1.2);5.7564(1.6);3.8497(16.0);3.7903(0.5);3.7783(0.8);3.7697(1.1);3.7609(0.8 );3.7491(0.6);3.3493(1.2);2.7991(0.4);2.7905(0.6);2.7822(0.6);2.7735(0.6);2.765 1(0.5);2.7564(0.3);2.6702(0.4);2.5231(1.1);2.5054(48.2);2.5010(62.8);2.4966(45. 0);2.3278(0.4);1.7772(0.4);1.7601(1.1);1.7410(1.2);1.7246(1.0);1.7129(0.7);1.69 94(0.7);1.6879(0.7);-0.0002(0.6) |
| I-471 | 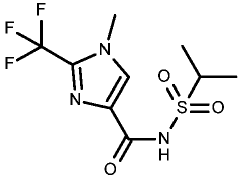 | I-471: ¹H-NMR(400.0 MHz, d₆-DMSO): |
| | | δ= 11.6208(0.6);8.2908(4.9);3.8638(7.9);3.8620(8.2);3.7770(0.4);3.7598(1.2);3.7 426(1.6);3.7254(1.2);3.7083(0.4);3.3318(8.3);2.5239(0.5);2.5191(0.8);2.5105(13. 4);2.5061(28.6);2.5015(38.8);2.4969(27.6);2.4923(12.8);1.3035(16.0);1.2863(15. 7);1.2329(1.5);1.2159(1.4);-0.0002(3.0) |
| I-472 | 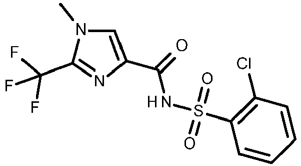 | I-472: ¹H-NMR(400.0 MHz, d₆-DMSO): |
| | | δ= 8.2731(9.7);8.1601(3.2);8.1561(3.4);8.1403(3.5) ;8.1364(3.6);7.7304(1.0); 7.72 64(1.0);7.7104(2.5);7.7065(2.5);7.6927(3.2);7.6886(3.1);7.6712(4.1);7.6677(5.4); 7.6513(2.4);7.6477(1.8);7.6335(2.5);7.6297(2.1);7.6137(3.0);7.6102(2.5);7.5959( 1.6);7.5921(1.5);5.7543(4.0);3.8457(15.9);3.8439(16.0);3.3435(1.3);2.5251(0.6); 2.5118(14.9);2.5073(31.6);2.5028(42.4);2.4982(30.4);2.4937(14.4);-0.0002(0.7) |
| I-473 | 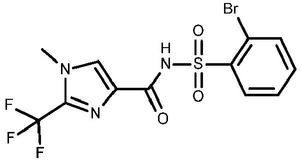 | I-473: ¹H-NMR(400.0 MHz, d₆-DMSO): |
| | | δ= 8.2857(9.5);8.1874(3.1);8.1830(3.4);8.1678(3.4);8.1635(3.5);7.8534(3.1);7.85 01(3.3);7.8341(3.8);7.8308(3.9);7.6770(1.3);7.6738(1.5);7.6582(3.3);7.6548(3.2); 7.6390(2.6);7.6355(2.4);7.6200(2.4);7.6153(2.7);7.6007(3.0);7.5962(3.1);7.5819( 1.3);7.5774(1.2);3.8463(16.0);3.3460(1.2);2.5248(0.6);2.5114(15.3);2.5070(32.5) ;2.5025(44.0);2.4979(31.9);2.4934(15.3);-0.0002(0.9) |
| I-474 | 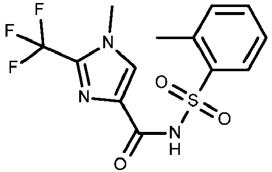 | I-474: ¹H-NMR(400.0 MHz, d₆-DMSO): |
| | | δ= 12.4383(0.4);8.2148(6.9);8.0240(1.9);8.0210(2.1);8.0042(2.1);8.0011(2.2);7.5 933(0.8);7.5900(0.9);7.5746(2.0);7.5713(2.1);7.5559(1.4);7.5526(1.3);7.4549(1.3 );7.4352(1.9);7.4162(0.9);7.3995(2.2);7.3806(1.8);7.3569(0.6);5.7539(1.2);3.835 5(10.2);3.8334(10.4);3.3292(4.6);2.6003(16.0);2.5877(1.5);2.5249(0.4);2.5201(0. 7);2.5116(10.4);2.5071(22.3);2.5025(30.2);2.4979(21.7);2.4933(10.2);1.9888(1.0 );1.1753(0.5);-0.0002(0.6) |
| I-475 | 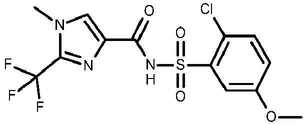 | I-475: ¹H-NMR(400.0 MHz, d₆-DMSO): |
| | | δ= 8.2689(3.5);7.5987(2.7);7.5910(2.8);7.5744(2.4);7.5524(2.8);7.2995(1.4);7.29 17(1.4);7.2774(1.2);7.2696(1.2);4.0385(0.4);4.0207(0.4);3.8527(16.0);3.8461(6.8 );3.8442(6.6);3.8164(0.5);3.3358(2.2);2.5243(0.4);2.5111(10.4);2.5066(22.2);2.5 021(30.0);2.4975(21.6);2.4929(10.3);1.9886(1.9);1.1930(0.5);1.1752(1.0);1.1574 (0.5);-0.0002(0.5) |
| I-476 | 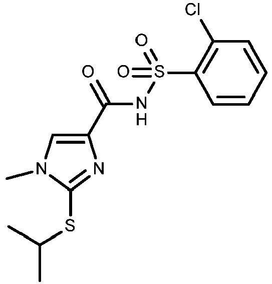 | I-476: ¹H-NMR(400.0 MHz, d₆-DMSO): |
| | | δ= 8.1397(1.5);8.1364(1.6);8.1200(1.6);8.1167(1.6);8.0649(3.5);7.6954(0.4);7.67 84(1.2);7.6613(1.4);7.6580(1.4);7.6431(2.4);7.6267(0.9);7.6097(1.0);7.6064(0.9); 7.5898(1.4);7.5726(0.6);7.5689(0.6);5.7575(0.7);3.6826(0.4);3.6657(1.1);3.6490( 1.5);3.6320(1.3);3.6171(14.4);3.1860(0.8);2.7653(0.6);2.5068(13.9);2.5027(17.9) ;2.4985(13.1);1.2924(16.0);1.2755(15.7);-0.0002(1.8) |
| I-477 | 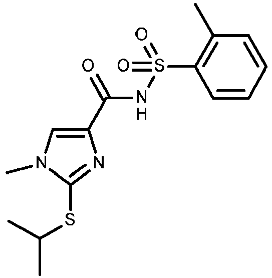 | I-477: ¹H-NMR(400.0 MHz, d₆-DMSO): |
| | | δ= 8.0267(4.4);8.0120(1.8);7.9920(1.9);7.5805(0.7);7.5620(1.8);7.5434(1.2);7.44 79(1.0);7.4288(1.7);7.4097(0.8);7.3918(1.9);7.3729(1.5);5.7572(0.6);3.6800(0.5); 3.6632(1.2);3.6464(1.6);3.6296(1.3);3.6106(14.6);2.5965(12.5);2.5057(23.4);2.5 021(28.0);1.2908(16.0);1.2739(15.8);-0.0002(2.3) |
| I-478 | 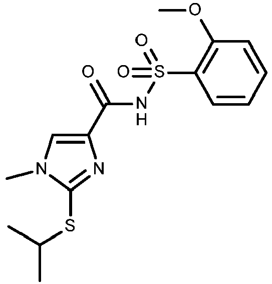 | I-478: ¹H-NMR(400.0 MHz, d₆-DMSO): |
| | | δ= 8.0515(4.2);7.8882(1.5);7.8845(1.5);7.8685(1.6);7.8647(1.6);7.6803(0.7);7.67 67(0.7);7.6590(1.4);7.6409(0.8);7.6373(0.8);7.2303(2.1);7.2095(1.9);7.1579(1.1); 7.1387(2.0);7.1197(1.0);3.8382(14.9);3.7027(0.4);3.6859(1.1);3.6690(1.5);3.652 3(1.2);3.6353(0.5);3.6050(14.3);3.3305(0.4);2.6705(0.3);2.5053(48.4);2.5012(62. 0);2.4971(45.6);2.3283(0.4);1.3108(16.0);1.2939(15.8);-0.0003(4.8) |
| I-479 | 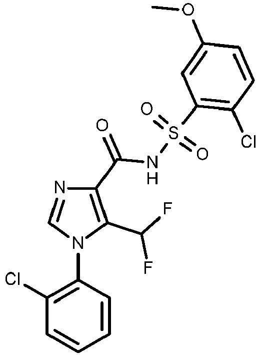 | I-479: ¹H-NMR(400.0 MHz, d₆-DMSO): |
| | | δ= 8.2906(3.6);7.7490(1.7);7.7292(2.4);7.7127(1.6);7.6929(1.9);7.6454(1.0);7.62 56(2.2);7.6198(3.3);7.6119(3.6);7.5963(2.5);7.5741(2.9);7.5671(1.6);7.5458(1.8); 7.5292(1.1);7.4022(1.3);7.2992(1.5);7.2917(1.5);7.2767(1.7);7.2703(1.8);4.0768( 0.3);4.0339(0.4);3.9025(14.7);3.8546(16.0);3.8160(1.0);3.7204(1.4);3.6910(1.4); 3.6820(1.4);3.6703(1.5);3.5092(1.0);3.4168(0.5);3.3816(0.5);3.3406(0.4);3.3236( 0.4);3.3061(0.3);3.1688(3.2);2.6713(0.8);2.5403(0.7);2.5061(106.9);2.5022(136. 9);2.4984(107.1);2.3288(08);1.2346(0.3);-0.0002(3.6) |
| I-480 | 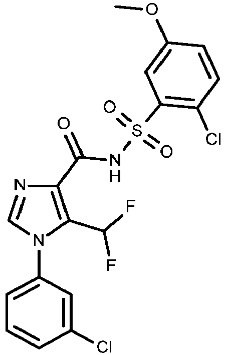 | I-480: ¹H-NMR(601.6 MHz, d₆-DMSO): |
| | | δ= 8.2989(1.9);7.7403(1.2);7.7371(2.4);7.7338(1.4);7.6675(0.7);7.6658(0.8);7.66 43(0.8);7.6626(0.7);7.6541(1.1);7.6524(1.3);7.6509(1.3);7.6492(1.1);7.6120(3.2); 7.6067(3.0);7.6003(2.6);7.5869(1.4);7.5676(1.8);7.5529(2.3);7.5351(0.8);7.4920( 0.4);7.2716(1.0);7.2665(1.0);7.2569(0.9);7.2518(0.9);3.8519(16.0);3.4170(26.6); 3.1696(0.6);2.6162(0.4);2.6132(0.6);2.6101(0.4);2.5224(1.1);2.5193(1.4);2.5162( 1.4);2.5075(31.0);2.5044(65.5);2.5014(90.9);2.4983(66.5);2.4953(31.8);2.3886(0 .4);2.3856(0.6);2.3825(0.4);1.9085(1.2);-0.0002(3.8) |
| I-481 | 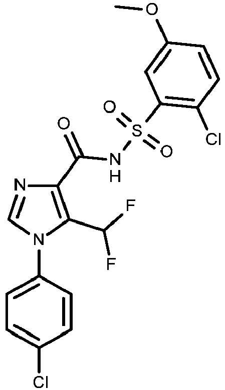 | I-481: ¹H-NMR(400.0 MHz, d₆-DMSO): |
| | | δ= 8.2993(2.4);7.6668(1.8);7.6453(3.6);7.6119(2.4);7.6045(5.0);7.5817(3.5);7.55 91(1.9);7.4578(0.9);7.3284(0.5);7.2864(1.0);7.2797(1.0);7.2647(0.9);7.2576(0.9); 4.0307(0.4);3.9024(16.0);3.8527(10.7);3.7398(1.3);3.5071(0.5);3.1688(7.3);2.67 10(0.6);2.5059(75.6);2.5021(95.4);2.4981(72.5);2.3285(0.5);-0.0002(2.6) |
| I-482 | 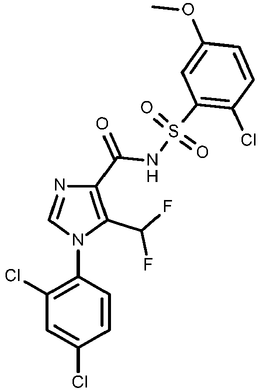 | I-482: ¹H-NMR(400.0 MHz, d₆-DMSO): |
| | | δ= 8.2685(2.9);7.9836(2.5);7.9786(2.7);7.7732(1.4);7.7518(2.1);7.6748(1.5);7.66 98(1.6);7.6538(1.1);7.6486(1.1);7.6151(2.3);7.6077(2.4);7.5890(1.9);7.5670(2.2); 7.5502(0.6);7.4194(1.0);7.2923(1.6);7.2854(1.5);7.2703(1.1);7.2636(1.1);4.0311( 0.3);3.9024(16.0);3.8521(12.9);3.7048(1.3);3.6668(1.4);3.6312(1.4);3.5079(1.1); 3.3236(0.4);3.1688(2.4);2.6713(0.7);2.5413(0.4);2.5062(90.8);2.5022(119.0);2.4 981(93.4);2.3292(0.7);1.2354(0.4);-0.0002(3.4) |
| I-483 | 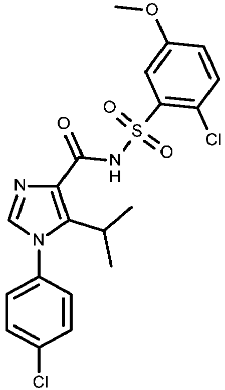 | I-483: ¹H-NMR(400.0 MHz, d₆-DMSO): |
| | | δ= 8.0517(2.6);7.6829(2.6);7.6614(3.6);7.6152(2.4);7.6077(2.4);7.5791(1.9);7.55 74(5.6);7.5362(2.6);7.2774(1.0);7.2699(1.1);7.2553(1.0);7.2477(0.9);3.9025(16.0 );3.8571(12.3);3.8159(0.6);3.5960(1.8);3.5082(1.3);3.4789(1.0);3.4188(0.6);3.32 44(0.8);3.2678(0.4);3.1691(1.3);3.0566(0.7);3.0389(0.9);3.0214(0.7);2.6718(0.4); 2.5067(58.5);2.5026(75.8);2.4988(58.5);2.3293(0.5);1.2938(0.5);1.2357(0.4);1.2 237(0.9);1.2059(0.9);1.1116(9.4);1.0940(9.3);-0.0001(2.0) |
| I-484 | 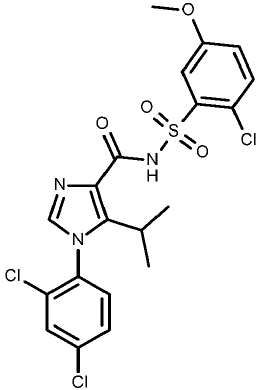 | 1-484: ¹H-NMR(400.0 MHz, d₆-DMSO): |
| | | δ= 8.0120(3.0);8.0066(3.1);7.9787(4.1);7.7716(1.9);7.7503(3.2);7.6948(1.9);7.68 94(1.8);7.6736(1.1);7.6682(1.1);7.6184(2.9);7.6108(3.1);7.6010(2.4);7.5790(2.7); 7.2976(1.4);7.2900(1.4);7.2756(1.3);7.2679(1.2);3.9025(13.0);3.8597(16.0);3.81 81(0.6);3.6750(1.2);3.5859(1.3);3.5654(1.3);3.5591(1.3);3.5081(1.2);3.3258(0.5); 3.3015(0.4);3.1687(0.5);2.9036(0.3);2.8854(0.8);2.8682(1.1);2.8505(0.8);2.8326( 0.3);2.6711(0.5);2.5062(76.5);2.5020(98.9);2.4978(74.8);2.3288(0.7);1.1301(6.1) ;1.1125(6.0);1.0382(5.9);1.0206(5.8);-0.0002(2.5) |
| I-485 | 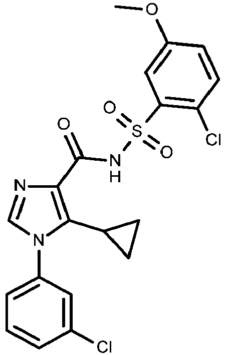 | I-485: ¹H-NMR(400.0 MHz, d₆-DMSO): |
| | | δ= 8.0939(3.3);7.7645(2.0);7.6169(2.6);7.6094(4.2);7.5901(3.6);7.5735(2.2);7.56 76(3.0);7.2941(1.0);7.2865(1.0);7.2720(0.9);7.2643(0.9);3.9023(16.0);3.8640(11. 3);3.8249(0.4);3.8152(0.4);3.7880(0.6);3.7257(0.8);3.6673(1.1);3.5479(2.0);3.50 87(1.8);3.3542(0.5);3.3242(0.9);3.2675(0.5);3.1687(1.4);2.6705(0.4);2.5061(57.7 );2.5020(74.8);2.4978(56.9);2.3287(0.4);1.9028(0.4);1.8953(0.5);1.8897(0.4);1.8 815(0.9);1.8680(0.5);1.8605(0.5);0.7186(0.4);0.7068(1.4);0.7022(1.5);0.6858(1.4 );0.6809(1.5);0.6703(0.5);0.3910(0.5);0.3761(1.8);0.3628(1.7);0.3509(0.4);-0.0002(1.6) |
| I-486 | 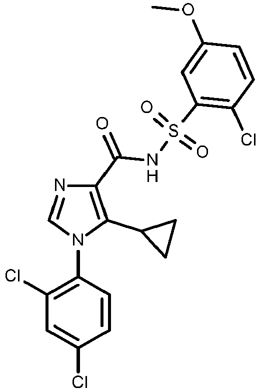 | I-486: ¹H-NMR(400.0 MHz, d₆-DMSO): |
| | | δ= 7.9937(2.2);7.9883(2.3);7.9742(3.3);7.7763(1.4);7.7550(2.2);7.6837(1.3);7.67 84(1.3);7.6626(0.8);7.6571(0.8);7.6159(2.1);7.6083(2.3);7.6036(2.0);7.5814(1.9); 7.3053(1.0);7.2977(1.0);7.2833(0.9);7.2757(0.9);3.9025(16.0);3.8619(10.8);3.78 01(0.4);3.7515(0.5);3.6768(0.8);3.6217(1.0);3.5597(1.2);3.5468(1.2);3.5370(1.2); 3.5073(1.2);3.4579(1.0);3.4307(0.8);3.3982(0.7);3.3239(0.6);3.3050(0.4);3.2667( 0.4);3.1685(0.9);2.6709(0.4);2.5059(64.1);2.5018(83.0);2.4977(64.0);2.3285(0.5) ;1.7120(0.4);1.7040(0.5);1.6982(0.4);1.6906(0.9);1.6830(0.4);1.6771(0.6);1.6695 (0.5);1.1215(0.3);0.6708(1.3);0.6658(1.5);0.6495(1.3);0.6444(1.5);0.4854(0.9);-0.0002(2.3) |
| I-487 | 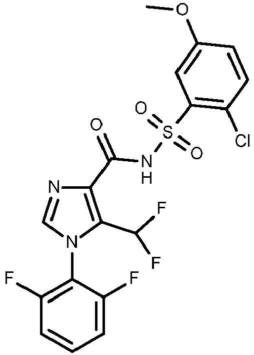 | I-487: ¹H-NMR(601.6 MHz, d₆-DMSO): |
| | | δ= 8.3632(2.0);7.7534(0.5);7.7497(0.4);7.7391(0.8);7.7284(0.4);7.7248(0.5);7.61 70(2.8);7.6118(2.9);7.5778(1.8);7.5632(2.1);7.4502(1.5);7.4363(2.3);7.4228(1.2); 7.2850(1.0);7.2799(1.0);7.2704(0.9);7.2652(0.9);3.8518(16.0);3.4648(0.5);3.169 3(1.5);2.6154(0.3);2.6124(0.4);2.6093(0.3);2.5217(0.8);2.5186(1.0);2.5155(1.0);2 .5067(23.8);2.5037(51.0);2.5006(71.0);2.4975(52.0);2.4945(24.9);2.3878(0.3);2. 3848(0.4);2.3817(0.3);1.9083(0.9);-0.0002(3.2) |
| I-488 | 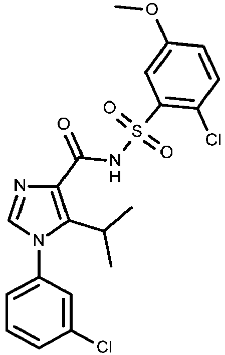 | I-488: ¹H-NMR(601.6 MHz, d₆-DMSO): |
| | | δ= 8.0078(0.4);7.7039(1.9);7.6820(1.1);7.6685(1.6);7.6669(1.4);7.6276(1.7);7.61 43(3.2);7.6114(3.3);7.6062(3.2);7.6011(1.5);7.5531(0.8);7.5387(0.9);7.4791(1.3); 7.4662(1.1);7.2443(0.6);7.2324(0.6);3.9007(15.6);3.8521(16.0);3.7287(0.3);3.45 08(1.2);3.1690(0.8);3.0714(0.5);2.6151(0.5);2.6121(0.7);2.6091(0.5);2.5213(1.1); 2.5183(1.4);2.5152(1.5);2.5062(43.2);2.5033(89.6);2.5003(121.9);2.4973(89.8);2 .4944(43.8);2.3875(0.6);2.3845(0.8);2.3815(0.6);1.9079(1.4);1.1196(13.2);1.107 8(13.4);1.0975(0.6);-0.0002(5.1) |
| I-489 | 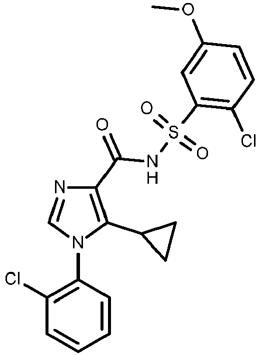 | I-489: ¹H-NMR(400.0 MHz, d₆-DMSO): |
| | | δ= 7.9905(4.0);7.7655(1.2);7.7481(1.6);7.7456(1.6);7.7163(1.0);7.7125(1.2);7.69 72(1.4);7.6935(1.4);7.6431(0.6);7.6393(0.6);7.6194(3.5);7.6116(2.9);7.6045(3.1); 7.5821(3.1);7.5614(1.2);7.5587(1.3);7.5425(0.4);7.3049(1.2);7.2972(1.2);7.2828( 1.1);7.2752(1.0);3.9024(16.0);3.8868(0.6);3.8629(13.5);3.8520(1.2);3.8160(0.5); 3.7605(0.6);3.7213(0.7);3.7071(0.8);3.6787(0.9);3.6435(0.8);3.6358(0.9);3.6080( 0.9);3.5712(0.8);3.5076(0.7);3.4587(0.5);3.4132(0.5);3.3972(0.5);3.3820(0.4);3.3 243(0.4);3.1687(1.4);2.6711(0.5);2.5063(62.0);2.5020(80.4);2.4977(59.3);2.3287 (0.4);2.3244(0.3);1.7360(0.6);1.7279(0.6);1.7223(0.4);1.7146(1.1);1.7065(0.4);1. 7010(0.6);1.6933(0.6);0.6431(1.4);0.6382(1.7);0.6219(1.4);0.6168(1.6);0.4861(1. 4);-0.0001(2.5) |
| I-490 | 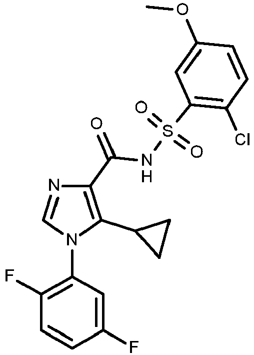 | I-490: ¹H-NMR(400.0 MHz, d₆-DMSO): |
| | | δ= 8.0358(3.9);7.7661(0.5);7.7588(0.6);7.7513(0.7);7.7444(1.1);7.7375(0.7);7.73 04(0.7);7.7231(0.6);7.6411(0.4);7.6291(0.5);7.6167(3.5);7.6088(3.4);7.6020(3.0); 7.5799(3.0);7.5527(0.4);7.5438(0.7);7.5338(0.7);7.5231(0.9);7.5015(0.4);7.3082( 1.4);7.3007(1.3);7.2862(1.2);7.2785(1.2);3.9023(16.0);3.8647(14.2);3.8163(0.4); 3.7878(0.6);3.7254(0.8);3.6962(0.8);3.6673(1.1);3.5084(2.6);3.4788(2.5);3.3242( 1.4);3.2862(0.7);3.2676(0.9);3.1905(0.4);3.1686(1.4);2.6711(0.6);2.5062(73.8);2. 5021(97.6);2.4978(77.6);2.3289(0.6);1.7762(0.5);1.7685(0.6);1.7552(1.0);1.7422 (0.6);1.7345(0.6);0.7060(0.6);0.6943(1.8);0.6893(2.1);0.6734(1.9);0.6682(2.0);0. 6575(0.7);0.4327(0.7);0.4175(2.4);0.4083(2.1);0.4040(2.3);0.3926(0.6);-0.0002(2.0) |
| I-491 | 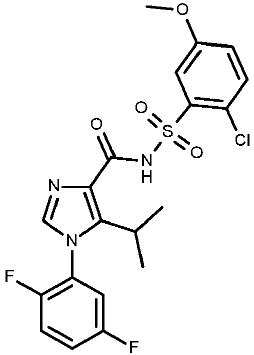 | I-491: ¹H-NMR(400.0 MHz, d₆-DMSO): |
| | | δ= 8.0151(4.5);7.7813(0.5);7.7733(0.6);7.7660(0.7);7.7594(1.1);7.7530(0.7);7.74 54(0.6);7.7380(0.6);7.6618(0.3);7.6499(0.4);7.6389(1.0);7.6269(1.1);7.6170(3.6); 7.6095(3.4);7.6022(3.5);7.5916(0.9);7.5801(3.5);7.5721(1.0);7.5639(0.6);7.5494( 0.4);7.3025(1.4);7.2948(1.4);7.2804(1.3);7.2728(1.2);3.9024(13.1);3.8614(16.0); 3.8159(0.4);3.7883(0.5);3.6766(1.0);3.5092(3.0);3.3245(0.9);3.2673(0.5);3.1855( 0.3);3.1685(1.9);3.0127(0.4);2.9951(0.8);2.9778(1.0);2.9603(0.8);2.9435(0.4);2.6 709(0.6);2.6665(0.4);2.5063(74.7);2.5020(98.1);2.4977(73.3);2.3287(0.6);1.0920 (6.6);1.0746(6.5);-0.0002(2.3) |
| I-492 | 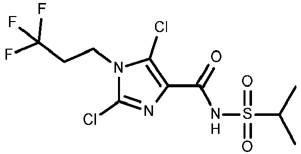 | I-492: ¹H-NMR(400.0 MHz, d₆-DMSO): |
| | | δ= 11.5588(1.1);4.3264(2.2);4.3094(4.7);4.2926(2.3);3.9021(4.4);3.7524(0.4);3.7 353(1.0);3.7181(1.4);3.7010(1.0);3.6832(0.4);3.3254(58.4);3.2674(0.4);2.9113(0. 6);2.9006(1.0);2.8839(1.8);2.8723(1.2);2.8674(1.2);2.8559(1.8);2.8394(0.9);2.82 82(0.6);2.6711(0.7);2.5063(93.4);2.5021(119.4);2.4978(92.3);2.3290(0.7);1.2920 (16.0);1.2749(15.9);1.2324(0.5);-0.0002(1.0) |

### Anwendungsbeispiele

### Boophilus microplus -Injektionstest

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: I-035, I-059, I-284

Bei diesem Test zeigen z.B. die folgenden Verbindungen der Herstellungsbeispiele eine Wirkung von 90% bei einer Aufwandmenge von 20µg/Tier: 1-032

Bei diesem Test zeigen z.B. die folgenden Verbindungen der Herstellungsbeispiele eine Wirkung von 80% bei einer Aufwandmenge von 20µg/Tier: 1-268

Bei diesem Test zeigen z.B. die folgenden Verbindungen der Herstellungsbeispiele eine Wirkung von 70% bei einer Aufwandmenge von 20µg/Tier: I-060

### Ctenocephalides felis - Oraltest

### Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 70% bei einer Aufwandmenge von 100ppm: I-032

### Lucilia cuprina - Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-035

### Diabrotica balteata - Sprühtest

| | |
|---|---|
| Lösungsmittel: | 78 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Vorgequollene Weizenkörner (*Triticum aestivum*) werden in einer mit Agar und etwas Wasser gefüllten Multiwell-Platte für einen Tag inkubiert (5 Saatkörner pro Kavität). Die gekeimten Weizenkörner werden mit einer Wirkstoffzubereitung der gewünschten Konzentration gespritzt. Anschließend wird jede Kavität mit 10-20 Käferlarven von *Diabrotica balteata* infiziert.

Nach 7 Tagen wird die Wirkung in % bestimmt. Dabei bedeutet 100 %, dass alle Maispflanzen wie in der unbehandelten, nicht infizierten Kontrolle gewachsen sind; 0 % bedeutet, dass keine Maispflanze gewachsen ist.

Bei diesem Test zeigen z. B. die folgenden Verbindungen der Herstellungsbeispiele Wirkung von 100% bei einer Aufwandmenge von 160µg/Kavität: I-118

### Meloidogyne incognita- Test

### Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100% bei einer Aufwandmenge von 20ppm: I-005, I-006, I-008, I-012, I-028, I-036, I-037, I-039, 1-041, 1-042, I-043, I-044, I-045, I-046, I-047, I-057, I-059, I-063, I-064, I-069, I-070, I-071, I-072, I-073, I-074, I-077, I-078, I-079, I-082, I-084, I-089, I-093, I-101, I-102, I-103, I-104, I-106, I-107, I-108, I-109, I-110, I-111, I-112, I-113, I-114, I-115, I-118, I-120, I-124, I-125, I-126, I-134, I-136, I-137, I-138, I-139, I-140, I-142, I-146, I-147, I-148, I-150, I-151, I-152, I-153, I-155, I-156, I-158, I-159, I-161, I-162, I-165, I-166, I-168, I-170, I-171, I-172, I-173, I-174, I-175, I-176, I-177, I-178, I-180, I-181, I-184, I-185, I-187, I-188, I-190, I-191, I-192, I-193, I-194, I-195, I-196, I-197, I-198, I-199, I-200, I-203, I-204, I-205, I-206, I-207, I-208, I-210, I-211, I-215, I-220, I-222, I-223, I-225, I-226, I-227, I-229, I-230, I-231, I-232, I-233, I-239, I-240, I-242, I-243, I-244, I-245, I-246, I-247, I-248, I-249, I-250, I-252, I-253, I-254, I-255, I-256, I-259, I-261, I-262, I-266, I-267, I-268, I-271, I-275, I-286, I-287, I-288, I-290, I-293, I-301, I-302, I-307, I-310, I-315, I-316, I-317, I-318, I-319, I-320, I-322, I-323, I-324, I-326, I-327, I-329, I-330, I-331, I-332, I-334, I-336, I-337, I-338, I-339, I-340, I-341, I-345, I-347, I-348, I-349, I-350, I-351, I-352, I-353, I-354, I-355, I-358, I-365, I-366, I-367, I-369, I-372, I-374, I-375, I-378, I-379, I-380, I-381, I-382, I-383, I-384, I-385, I-386, I-387, I-388, I-389, I-390, I-391, I-392, I-393, I-394, I-405, I-406, I-407, I-408, I-409, I-410, I-411, I-412, I-413, I-414, I-416, I-417, I-418, I-419, I-420, I-421, I-422, I-423, I-424, I-425, I-427, I-433, I-434, I-435, I-436, I-440, I-441, I-442, I-443, I-444, I-448, I-449, I-450, I-452, I-453, I-454, I-455, I-456, I-457, I-458, I-459, I-460, I-461, I-462, I-463, I-465, I-466, I-467, I-468, I-469, I-470, I-474, I-487, I-491

Bei diesem Test zeigen z. B. die folgenden Verbindungen der Herstellungsbeispiele Wirkung von 90% bei einer Aufwandmenge von 20ppm: I-001, I-002, I-003, I-004, I-009, I-010, I-014, I-015, 1-016, 1-017, I-018, I-019, I-020, I-021, I-022, I-023, I-024, I-025, I-026, I-027, I-030, I-031, I-032, I-033, I-034, I-035, I-038, I-048, I-049, I-050, I-051, I-052, I-053, I-054, I-055, I-056, I-060, I-061, I-062, I-065, I-066, I-068, I-076, I-080, I-081, I-083, I-085, I-088, I-090, I-091, I-094, I-095, I-096, I-097, I-098, I-100, I-105, I-116, I-117, I-119, I-122, I-123, I-129, I-130, I-131, I-132, I-135, I-141, I-143, I-144, I-154, I-157, I-163, I-164, I-167, I-169, I-179, I-182, I-183, I-186, I-189, I-209, I-212, I-213, I-214, I-216, I-217, I-219, I-221, I-224, I-228, I-235, I-236, I-237, I-238, I-241, I-251, I-257, I-263, I-264, I-265, I-269, I-270, I-272, I-273, I-274, I-278, I-279, I-280, I-281, I-282, I-283, I-285, I-289, I-291, I-292, I-294, I-295, I-296, I-297, I-298, I-299, I-303, I-304, I-305, I-306, I-308, I-309, I-311, I-312, I-313, I-314, I-321, I-325, I-328, I-335, I-342, I-343, I-344, I-359, I-360, I-361, I-362, I-363, I-364, I-368, I-371, I-376, I-377, I-395, I-396, I-398, I-399, I-400, I-402, I-403, I-404, I-415, I-426, I-428, I-429, I-430, I-431, I-432, I-437, I-439, I-445, I-446, I-447, I-451, I-464, I-471, I-472, I-473, I-475, I-477, I-479, I-480, I-482, I-485, I-488, I-489, I-490

Bei diesem Test zeigen z. B. die folgenden Verbindungen der Herstellungsbeispiele Wirkung von 80% bei einer Aufwandmenge von 20 ppm: I-201

Bei diesem Test zeigen z. B. die folgenden Verbindungen der Herstellungsbeispiele Wirkung von 70% bei einer Aufwandmenge von 20 ppm: I-007, I-011, I-013, I-029, I-040, I-058, I-067, I-075, I-086, I-087, I-092, I-099, I-121, I-127, I-128, I-133, I-145, I-160, I-202, I-218, I-234, I-258, I-260, I-276, I-277, I-284, I-300, I-333, I-346, I-370, I-373, I-397, I-401, I-476, I-478, I-481, I-483, I-484, I-486

Bei diesem Test zeigen z. B. die folgenden Verbindungen der Herstellungsbeispiele Wirkung von 90% bei einer Aufwandmenge von 4 ppm: 1-145, 1-149, 1-356, 1-357

### Aphis gossypii - Oraltest

### Lösungsmittel: 100 Gewichtsteile Aceton

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL4IInsektenmedium (33% + 15% Zucker) auf eine Endvolumen von 200 µl aufgefüllt. Anschließend werden die Platten mit Parafilm verschlossen, durch den eine gemischte Population der Baumwollblattlaus *(Aphis gossypii),* die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 100ppm: I-412

### Myzus persicae - Oraltest

### Lösungsmittel: 100 Gewichtsteile Aceton

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus *(Myzus persicae),* die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 100ppm: 1-250, 1-253, 1-254, I-264

Bei diesem Test zeigen z. B. die folgenden Verbindungen der Herstellungsbeispiele Wirkung von 100% bei einer Aufwandmenge von 20ppm: I-284, I-316, I-387, I-392

Bei diesem Test zeigen z. B. die folgenden Verbindungen der Herstellungsbeispiele Wirkung von 90% bei einer Aufwandmenge von 20ppm: I-116, I-170, I-266, I-268, I-286, I-291, I-317

Bei diesem Test zeigen z. B. die folgenden Verbindungen der Herstellungsbeispiele Wirkung von 70% bei einer Aufwandmenge von 20ppm: I-004, I-007, I-127, I-269, I-460

Bei diesem Test zeigen z. B. die folgenden Verbindungen der Herstellungsbeispiele Wirkung von 100% bei einer Aufwandmenge von 4ppm: I-106

### Myus persicae - Sprühtest

| | |
|---|---|
| Lösungsmittel: | 78 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 500g/ha: I-006, I-120, I-122, I-127, I-158, I-253, I-254, I-268, I-317, I-392, 1-401

Bei diesem Test zeigen z. B. die folgenden Verbindungen der Herstellungsbeispiele Wirkung von 70% bei einer Aufwandmenge von 500g/ha: I-010, I-042, I-059, I-060, I-085, I-116, I-171, I-214, I-220, I-264, I-269, I-314, I-346, I-385, I-387, I-473

Bei diesem Test zeigen z. B. die folgenden Verbindungen der Herstellungsbeispiele Wirkung von 90% bei einer Aufwandmenge von 100g/ha: I-010

### Nezara viridula -Sprühtest

| | |
|---|---|
| Lösungsmittel: | 78,0 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Gerstenpflanzen *(Hordeum vulgare),* die mit Larven der Grünen Reiswanze (*Nezara viridula*) infiziert sind, werden mit einer Wirkstoffzubereitung der gewünschten Konzentration gespritzt.

Nach 4 Tagen wird die Wirkung in % bestimmt. Dabei bedeutet 100 %, dass alle Reiswanzen abgetötet wurden; 0 % bedeutet, dass keine Reiswanzen abgetötet wurden.

Bei diesem Test zeigen z. B. die folgenden Verbindungen der Herstellungsbeispiele Wirkung von 100% bei einer Aufwandmenge von 500 g/ha: 1-032, 1-035

### Nilaparvata lugens -Test

| | |
|---|---|
| Lösungsmittel: | 78,0 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Reispflanzen *(Oryza sativa)* werden mit der Wirkstoffzubereitung der gewünschten Konzentration gespritzt und anschließend mit der Braunrückigen Reiszikade (*Nilaparvata lugens*) infiziert.

Nach 4 Tagen wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z. B. die folgenden Verbindungen der Herstellungsbeispiele Wirkung von 100% bei einer Aufwandmenge von 500g/ha: I-032, I-035, I-070, I-107, I-456

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500 g/ha: 1-035, I-300

Bei diesem Test zeigen z. B. die folgenden Verbindungen der Herstellungsbeispiele Wirkung von 83% bei einer Aufwandmenge von 500 g/ha: 1-032, 1-037, I-059

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: I-059, I-060, I-156

### Tetranychus urticae - Sprühtest, OP-resistent

| | |
|---|---|
| Lösungsmittel: | 78,0 GewichtsteileAceton |
| Emulgator : | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: 1-122, I-172

Bei diesem Test zeigen z. B. die folgenden Verbindungen der Herstellungsbeispiele eine Wirkung von 90% bei einer Aufwandmenge von 500g/ha: I-028, I-032, I-059, I-060, I-073, I-083, I-121, I-170, I-171, I-173, I-373, I-492

Bei diesem Test zeigen z. B. die folgenden Verbindungen der Herstellungsbeispiele eine Wirkung von 80% bei einer Aufwandmenge von 500g/ha: 1-483

Bei diesem Test zeigen z. B. die folgenden Verbindungen der Herstellungsbeispiele eine Wirkung von 70% bei einer Aufwandmenge von 500g/ha: 1-085, 1-160, 1-197, 1-255, 1-330, I-391

### Myzus persicae - Sprühtest

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen *(Capsicum annuum),* die stark von der Grünen Pfirsichblattlaus *(Myzus persicae)* befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 99% bei einer Aufwandmenge von 100ppm: 1-387

Bei diesem Test zeigen z. B. die folgenden Verbindungen der Herstellungsbeispiele Wirkung von 95% bei einer Aufwandmenge von 100ppm: I-316

### Absetzbeispiele

### Meloidogyne incognita -Test

| | | |
|---|---|---|
| Lösungsmittel: | 7 | Gewichtsteile Dimethylformamid |
| Emulgator: | 2,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration, wobei das Erdvolumen in das gedrencht wird, mitberücksichtigt werden muss. Es ist darauf zu achten, dass in der Erde eine Konzentration von 20 ppm Emulgator nicht überschritten wird. Zur Herstellung weiterer Testkonzentrationen wird mit Wasser verdünnt.

Mit Erde (lehmiger Sand) gefüllte Töpfe werden mit der Wirkstofflösung angegossen. Eine Ei-Larven-Suspension des südlichen Wurzelgallenälchens *(Meloidogyne incognita)* wird hinzugegeben, die Erdoberfläche mit Salatsamen bestreut und mit Quarzsand abgedeckt. Die Salatsamen keimen und die Pflänzchen entwickeln sich. An den Wurzeln entwickeln sich die Gallen.

Nach 21 Tagen wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele überlegene Wirksamkeit gegenüber dem Stand der Technik: siehe Tabellen

| Substanz | Struktur | Tierart | Konzentration | % Wirkung |
|---|---|---|---|---|
| Bsp 1-137 erfindungsgemäß | 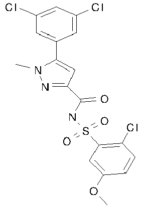 | MELGIN | 1 ppm | 85 21dat |
| Bsp Stand der Technik gemäß WO2015/169776 | 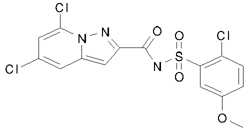 | MELGIN | 1 ppm | 0 21dat |

| Substanz | Struktur | Tierart | Konzentration | % Wirkung |
|---|---|---|---|---|
| Bsp I-141 erfindungsgemäß | 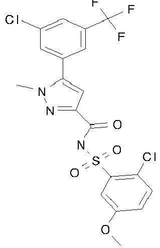 | MELGIN | 8 ppm | 99 21dat |
| Bsp Stand der Technik gemäß WO2015/169776 | 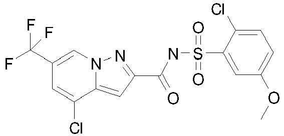 | MELGIN | 8 ppm | 30 21dat |

| Substanz | Struktur | Tierart | Konzentration | % Wirkung |
|---|---|---|---|---|
| Bsp I-113 erfindungsgemäß | 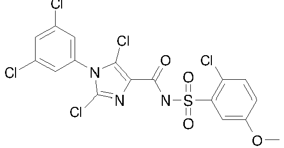 | MELGIN | 0,5 ppm | 100 21dat |
| Bsp 1-470 erfindungsgemäß | 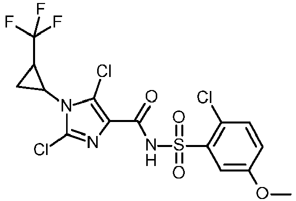 | MELGIN | 0,5 ppm | 100 21dat |
| Bsp Stand der Technik gemäß WO2010/129500 | 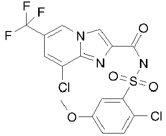 | MELGIN | 0,5 ppm | 55 21dat |

## Patentansprüche

1. Nicht-therapeutische Verwendung einer Verbindung der Formel (I) 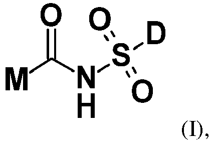 in der
M für einen Rest ausgewählt aus den Formeln (IIa-IIc) steht: 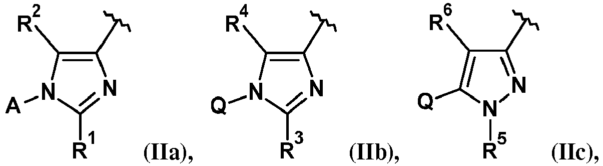 wobei
R¹ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
R² Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Al-koxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino ist,
R³ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaniinocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
R⁴ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
R⁵ Wasserstoff, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkylthio-(C₁-C₆)alkyl, Halogen(C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)Alkylsulfinyl-(C₁-C₆)alkyl oder Halogen(C₁-C₆)Alkylsulfonyl-(C₁-C₆)alkyl ist,
R⁶Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
A Halogen(C₁-C₆)alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C₃)Alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Halogen(C₃-C₆)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
Q ein unsubstituierter oder mit einem oder mehreren Resten R⁷ substituierter Phenyl oder Pyridyl Rest ist,
wobei der oder die Substituenten R⁷ jeweils unabhängig voneinander ausgewählt sind aus:
Cyano, Halogen, Nitro, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl, (C₁-C₆)Alkylcarbonylamino), Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
und
D ein unsubstituierter oder mit einem oder mehreren Resten R⁸ substituierter C₁-C₆-Alkyl, Phenyl, Thiophenyl, Isoxazolyl, Phenyl-(C₁-C₂)alkyl oder Benzdioxolyl Rest ist,
wobei der oder die Substituenten R⁸ jeweils unabhängig voneinander ausgewählt sind aus:
Cyano, Halogen, Nitro, Acetyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaniinocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy,
zur Bekämpfung von tierischen Schädlingen.

2. Nicht-therapeutische Verwendung gemäß Anspruch 1, **dadurch gekennzeichnet, dass** R¹ Wasserstoff, Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, oder unsubstituiertes, einfach oder mehrfach, gleich oder verschieden substituiertes Benzyl ist, wobei als Substituenten jeweils in Frage kommen: Cyano, Halogen, (C₁-C₄)Halogenalkyl, (C₁-C₄)Alkoxy, (C₁-C₄)Halogenalkoxy, (C₁-C₆)Alkylthio und/oder (C₁-C₆)Halogenalkylthio,
R² Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
R³ Wasserstoff, Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl oder(C₁-C₄)Alkylsulfonyl-(C₁-C₄)alkyl ist,
R⁴ Wasserstoff, Cyano, Halogen, (C₁-C₄)Halogenalkyl, (C₁-C₄)Alkoxy, (C₁-C₄)Alkyl, (C₃-C₄)Cycloalkyl, (C₁-C₄)Halogenalkoxy, (C₁-C₆)Alkylthio oder (C₁-C₆)Halogenalkylthio ist,
R⁵ Wasserstoff, Cyclopropyl, (C₁-C₄)Alkyl, Halogencyclopropyl, Halogen(C₁-C₃)Alkylcyclopropyl oder (C₁-C₄)Halogenalkyl ist,
R⁶ Wasserstoff, Cyano, Halogen, (C₁-C₄)Halogenalkyl, (C₁-C₄)Alkoxy, (C₁-C₄)Halogenalkoxy, 4 (C₁-C₆)Alkylthio oder (C₁-C₆)Halogenalkylthio ist,
A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkylthio und/oder (C₁-C₆)Halogenalkylthio,
Q ein unsubstituierter oder mit einem oder mehreren Resten R⁷ substituierter Phenyl oder Pyridyl Rest ist,
wobei der oder die Substituenten R⁷ jeweils unabhängig voneinander ausgewählt sind aus:
Cyano, Halogen, (C₃-C₆)Cycloalkyl, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy,
und
D ein unsubstituierter oder mit einem oder mehreren Resten R⁸ substituierter C₁-C₆-Alkyl, Phenyl, Thiophenyl, Isoxazolyl, Benzyl oder Benzdioxolyl Rest ist,
wobei der oder die Substituenten R⁸ jeweils unabhängig voneinander ausgewählt sind aus:
Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaniinocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₈)Cycloalkylamino, (C₁-C₆)Alkylcarbonylamino), (1-Pyrazolyl)(C₁-C₃)alkyl und/oder (C₁-C₃)Alkoxypyrimidinyloxy.

3. Nicht-therapeutische Verwendung gemäß Anspruch 1, **dadurch gekennzeichnet, dass** R¹ Wasserstoff, Halogen, Cyclopropyl, (C₁-C₄)Alkyl, (C₁-C₄)Alkylthio, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen: Cyano, Halogen, (C₁-C₄)Halogenalkyl und/oder (C₁-C₄)Alkoxy,
R² Wasserstoff, Halogen, Cyano, (C₁-C₄)Alkyl, (C₁-C₄)Halogenalkyl ist,
R³ Wasserstoff, Halogen, Cyclopropyl, (C₁-C₄)Alkyl, Halogencyclopropyl oder(C₁-C₄)Halogenalkyl ist,
R⁴ Wasserstoff, Halogen, Cyano, (C₁-C₄)Alkyl, Cyclopropyl oder (C₁-C₄)Halogenalkyl ist, R⁵ Wasserstoff, Cyclopropyl, (C₁-C₄)Alkyl oder Halogen(C₁-C₄)alkyl ist,
R⁶ Wasserstoff, Halogen, Cyano, (C₁-C₄)Alkyl oder (C₁-C₄)Halogenalkyl ist,
A Halogen(C₁-C₄)alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C3)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio,
Q ein unsubstituierter oder mit einem oder mehreren Resten R⁷ substituierter Phenyl oder Pyridyl Rest ist,
wobei der oder die Substituenten R⁷ jeweils unabhängig voneinander ausgewählt sind aus:
Cyano, Halogen, (C₃-C₆)Cycloalkyl, Halogen(C₃-C₆)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy
und
D ein unsubstituierter oder mit einem oder mehreren Resten R⁸ substituierter C₁-C₆-Alkyl, Phenyl, Thiophenyl, Isoxazolyl, Benzyl oder Benzdioxolyl Rest ist,
wobei der oder die Substituenten R⁸ jeweils unabhängig voneinander ausgewählt sind aus:
Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaniinocarbonyl, Di-(C₁-C₆)alkyl-aminocarbonyl, (C₂-C₆)Alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₆)Cycloalkylamino, (C₁-C₆)Alkylcarbonylamino), (1-Pyrazolyl) (C₁-C₃)Alkyl und/oder (C₁-C₃)Alkoxypyrimidinyloxy.

4. Nicht-therapeutische Verwendung gemäß Anspruch 1, **dadurch gekennzeichnet, dass**
R¹ Wasserstoff, Chlor, Brom, Methyl, Benzyl, 2-Chlorbenzyl, 3-Chlorbenzyl, 4-Chlorbenzyl, Trifluormethyl, Methylthio oder Isopropylthio ist,
R² Wasserstoff, Cyano, Chlor, Brom oder Iod ist,
R³ Wasserstoff, Chlor, Methyl, Isopropyl, Ethyl oder Brom ist,
R⁴ Wasserstoff, Chlor, Brom, Iod, Fluor, Difluormethyl, Isopropyl oder Cyclopropyl ist,
R⁵ Methyl oder 2,2,2-Trifluorethyl ist und
R⁶ Wasserstoff oder Chlor ist,
A ein Rest ausgewählt aus den Resten der Formeln (III1-III19) ist: 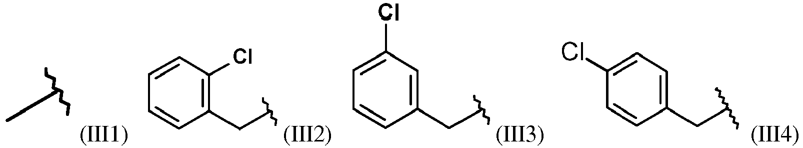 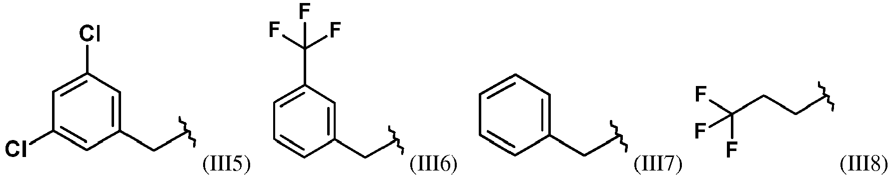 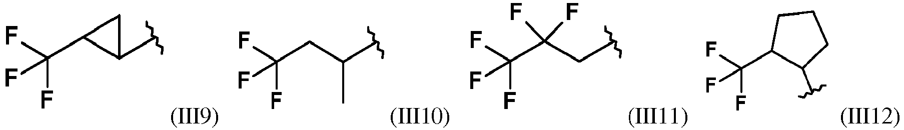 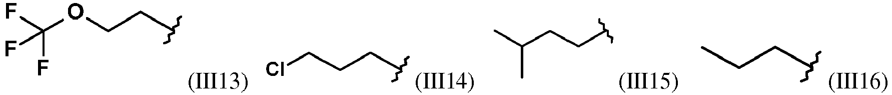 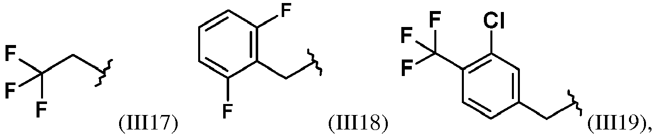
Q ein Rest ausgewählt aus den Resten der Formeln (IV1-IV40) ist: 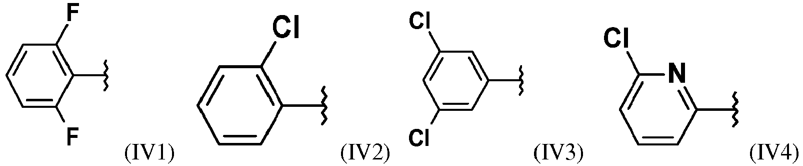 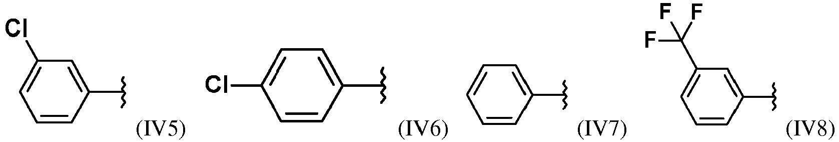 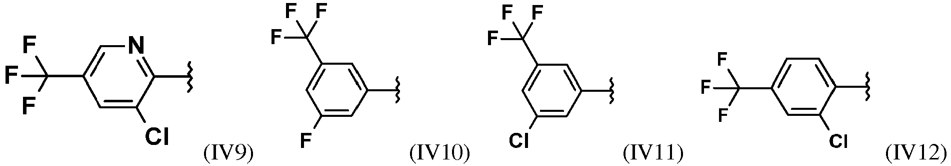 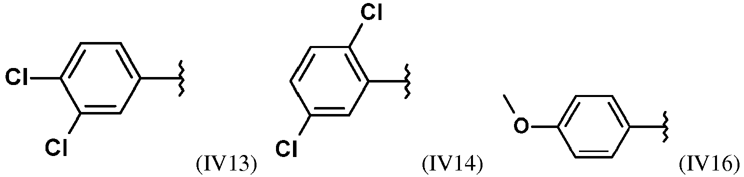 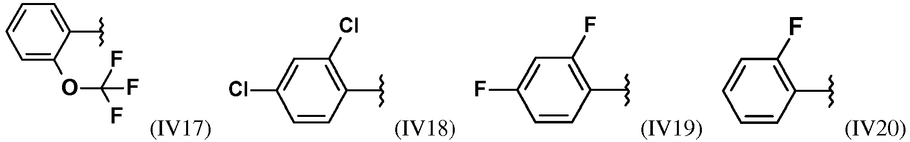 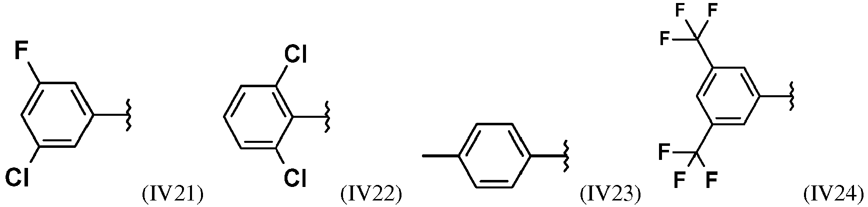 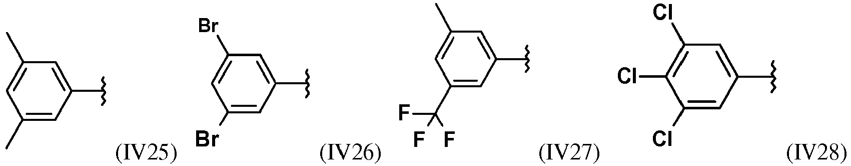 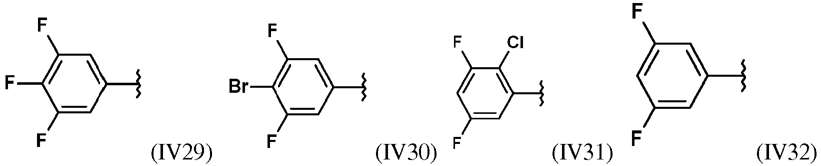 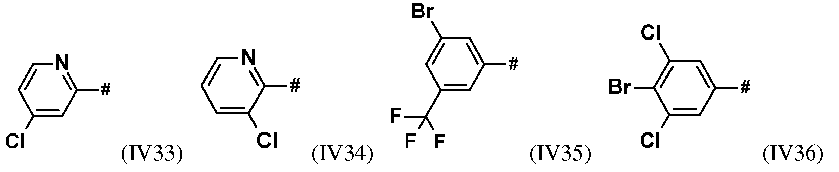 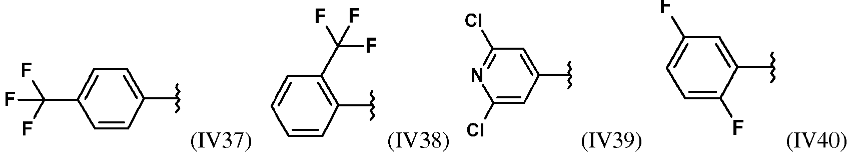 und
D ein Rest ausgewählt aus den Resten der Formeln (V1-V61) ist:

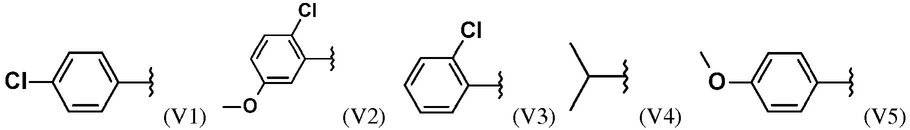

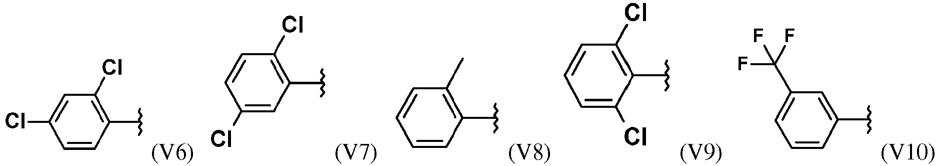

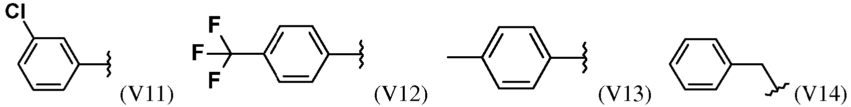

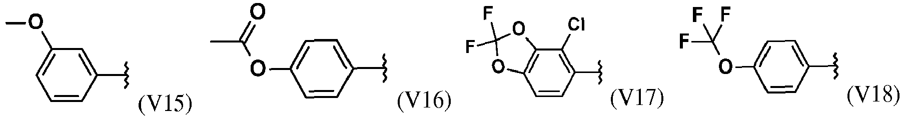

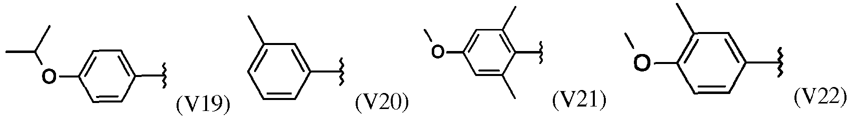

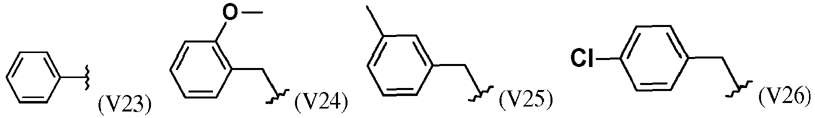

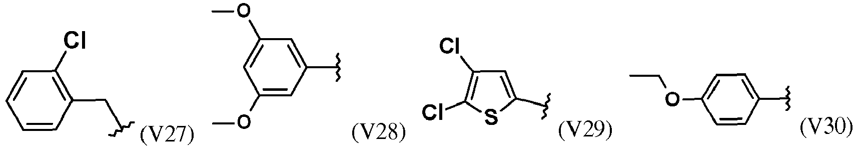

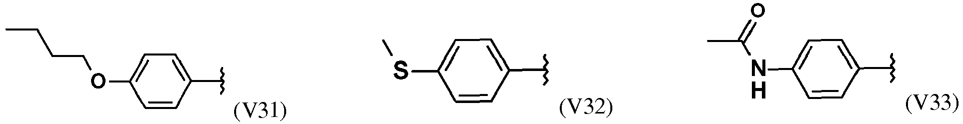

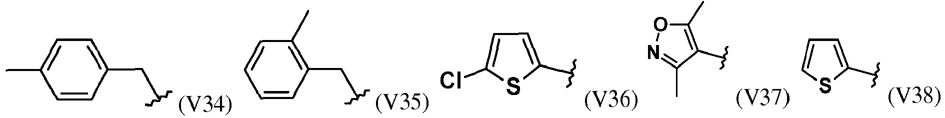

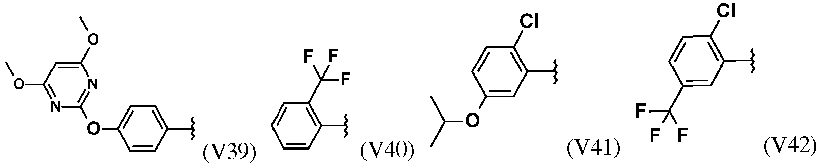

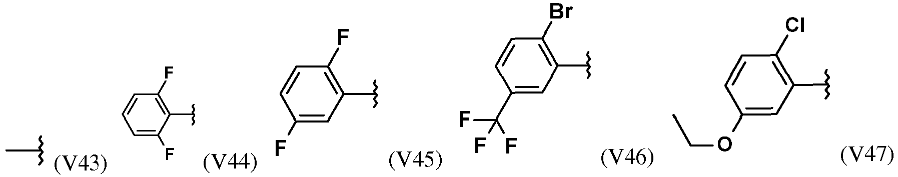

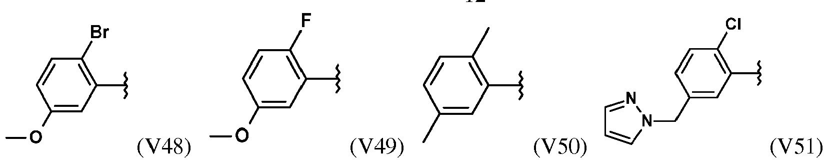

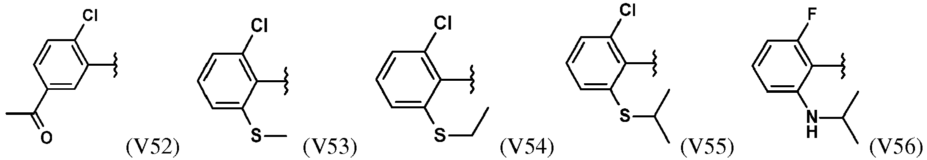

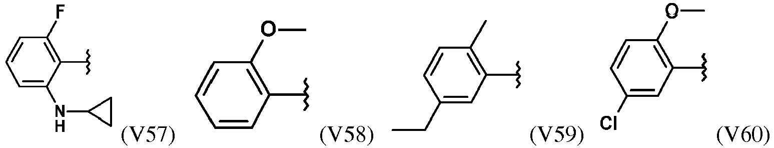

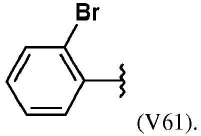

5. Nicht-therapeutische Verwendung gemäß einem der Ansprüche 1 bis 4, **dadurch gekennzeichnet, dass** in Formel (I) M für einen Rest ausgewählt aus den Formeln IIa oder IIb steht, wobei die Reste R¹, R², A, R³, R⁴, R⁷, R⁸, Q und D die in Anspruch 1, 2, 3 oder 4 beschriebenen Bedeutungen haben.

6. Nicht-therapeutische Verwendung gemäß einem der Ansprüche 1 bis 4, **dadurch gekennzeichnet, dass** Verbindungen der Formeln (Ia), (Ib) oder (Ic) verwendet werden, 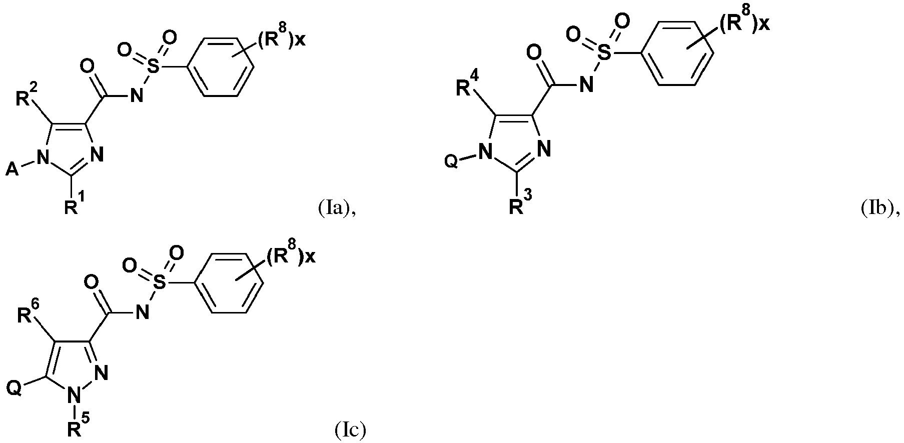 wobei die Reste R¹, R², R³, R⁴, R⁵, R⁶, R⁷, A, Q und R⁸ die in Anspruch 1, 2, 3 oder 4 beschriebenen Bedeutungen haben.

7. Nicht-therapeutische Verwendung gemäß einem der Ansprüche 1 bis 6, **dadurch gekennzeichnet, dass** es sich bei den tierischen Schädlingen um Nematoden handelt.

8. Nicht-therapeutische Verwendung gemäß einem der Ansprüche 1 bis 7 zum Schutz des Vermehrungsmaterials von Pflanzen.

9. Mittel, mit einem Gehalt von mindestens einer Verbindung gemäß Formel (I) oder der Formeln (Ia), (Ib) oder (Ic), welche gemäß einem der Ansprüche 1 bis 6 definiert ist, und üblichen Streckmitteln und/oder oberflächenaktiven Substanzen, insbesondere zur Bekämpfung von tierischen Schädlingen, bevorzugt von Nematoden.

10. Verfahren zur Bekämpfung von tierischen Schädlingen, bevorzugt Nematoden, bei dem man wenigstens eine Verbindung gemäß Formel (I oder der Formeln (Ia), (Ib) oder (Ic), welche gemäß einem der Ansprüche 1 bis 6 definiert ist, oder ein erfindungsgemäßes Mittel gemäß Anspruch 9, auf die tierischen Schädlinge, bevorzugt Nematoden und/oder ihren Lebensraum einwirken lässt, wobei die chirurgische, therapeutische und diagnostische Behandlung des menschlichen oder tierischen Körpers ausgeschlossen ist.

11. Agrochemische Formulierung enthaltend wenigstens eine Verbindung gemäß Formel (I) oder Formeln (Ia), (Ib) oder (Ic), welche gemäß einem der Ansprüche 1 bis 6 definiert ist, in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

12. Agrochemische Formulierung gemäß Anspruch 11, **dadurch gekennzeichnet, dass** diese zusätzlich einen weiteren agrochemischen Wirkstoff enthält.

13. Verbindungen der Formel (I') 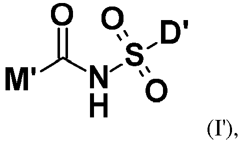 in der
M' für einen Rest ausgewählt aus den Formeln (IIa'-IIc') steht: 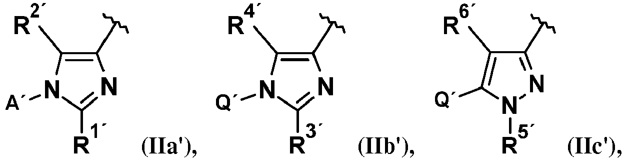 wobei
R^{1'} Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaniinocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
R^{2'} Wasserstoff, Cyano, Halogen, Nitro, (C₃-C₈)Cycloalkyl, (C₃-C₈)Cycloalkyloxy, (C₃-C₁)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
R^{3'} Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaniinocarbonyl, (C₁-C₆)Alkylcarbonylamino) oder unsubstituiertes, einfach oder mehrfach, gleich oder verschieden substituiertes Benzyl ist, wobei als Substituenten jeweils in Frage kommen:
Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl und/oder (C₁-C₆)Alkylsulfonyl-(C₁-C₆)alkyl,
R^{4'} Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
R^{5'} (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkylthio-(C₁-C₆)alkyl, Halogen(C₁-C₆)alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylsulfinyl-(C₁-C₆)alkyl oder Halogen(C₁-C₆)alkylsulfonyl-(C₁-C₆)alkyl ist,
R⁶ Wasserstoff, Cyano, Halogen, Nitro, (C₃-C₈)Cycloalkyl, (C₃-C₈)Cycloalkyloxy, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaminocarbonyl oder (C₁-C₆)Alkylcarbonylamino) ist,
A' Halogen(C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, Halogen(C₁-C3)alkoxy-(C₁-C₄)alkyl (C₁-C₆)Alkylthio-(C₁-C₆)alkyl, (C₁-C₆)Alkylsulfinyl-(C₁-C₆)alkyl, Halogen(C₁-C₆)alkylthio-(C₁-C₆)alkyl, Halogen(C₁-C₆)alkylsulfinyl-(C₁-C₆)alkyl, Halogen(C₁-C₆)alkylsulfonyl-(C₁-C₆)alkyl, Halogen(C₃-C₆)alkenyl oder
ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
Q' ein unsubstituierter oder mit einem oder mehreren Resten R^{7'} substituierter Phenyl oder Pyridyl Rest ist,
wobei der oder die Substituenten R^{7'} jeweils unabhängig voneinander ausgewählt sind aus:
Cyano, Halogen, Nitro, (C₃-C₆)Cycloalkyl, (C₃-C₆)Cycloalkyloxy, (C₃-C₆)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl-(C₁-C₆)alkyl, (C₁-C₆)Alkylsulfonyl-(C₁-C₆)alkyl, (C₁-C₆)Alkoxycarbonyl, (C₁-C₆)Halogenalkoxycarbonyl, (C₁-C₆)Alkylaniinocarbonyl, (C₁-C₆)Alkylcarbonylamino), Halogen(C₁-C₆)alkylthio-(C₁-C₆)alkyl, Halogen(C₁-C₆)alkylsulfinyl-(C₁-C₆)alkyl und/oder Halogen(C₁-C₆)alkylsulfonyl-(C₁-C₆)alkyl,
und
D' ein unsubstituierter oder mit einem oder mehreren Resten R^{8'} substituierter C₁-C₆-Alkyl, Phenyl, Thiophenyl, Isoxazolyl, Phenyl-(C₁-C₂)alkyl oder Benzdioxolyl Rest ist,
wobei der oder die Substituenten R^{8'} jeweils unabhängig voneinander ausgewählt sind aus:
Cyano, Halogen, Nitro, Acetyl, (C₃-C₈)Cycloalkyl, (C₃-C₈)Cycloalkyloxy, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaniinocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

14. Verbindungen der Formel (I') gemäß Anspruch 13, **dadurch gekennzeichnet, dass** R^{1'} Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl, oder unsubstituiertes, einfach oder mehrfach, gleich oder verschieden substituiertes Benzyl ist, wobei als Substituenten jeweils in Frage kommen:
Cyano, Halogen, (C₁-C₄)Halogenalkyl, (C₁-C₄)Alkoxy, (C₁-C₄)Halogenalkoxy, (C₁-C₆)Alkylthio und/oder (C₁-C₆)Halogenalkylthio,
R^{2'} Wasserstoff, Cyano, Halogen, (C₁-C₄)Halogenalkyl, (C₁-C₄)Alkoxy, (C₁-C₄)Halogenalkoxy, (C₁-C₆)Alkylthio oder (C₁-C₆)Halogenalkylthio ist,
R^{3'} Cyano, Halogen, Cyclopropyl, (C₁-C₄)Alkyl, Halogencyclopropyl, Halogen(C₁-C₃)Alkylcyclopropyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylthio-(C₁-C₄)alkyl, (C₁-C₄)Alkylsulfinyl-(C₁-C₄)alkyl, oder (C₁-C₄)Alkylsulfonyl-(C₁-C₄)alkyl ist,
R^{4'} Wasserstoff, Cyano, Halogen, (C₁-C₄)Halogenalkyl, (C₁-C₄)Alkoxy, (C₁-C₄)Alkyl, (C₃-C₄)Cycloalkyl, (C₁-C₄)Halogenalkoxy, (C₁-C₆)Alkylthio oder (C₁-C₆)Halogenalkylthio ist,
R^{5'} Cyclopropyl, (C₁-C₄)Alkyl, Halogencyclopropyl, Halogen(C₁-C₃)Alkylcyclopropyl oder (C₁-C₄)Halogenalkyl ist,
R^{6'} Wasserstoff, Cyano, Halogen, (C₁-C₄)Halogenalkyl, (C₁-C₄)Alkoxy, (C₁-C₄)Halogenalkoxy, 4 (C₁-C₆)Alkylthio oder (C₁-C₆)Halogenalkylthio ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C₃)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Cyanoalkyl, (C₁-C₆)Alkoxy-(C₁-C₆)alkyl, (C₁-C₆)Alkoxy, (C₁-C₆)Halogenalkoxy, (C₁-C₆)Cyanoalkoxy, C₁-C₆)Alkoxy-(C₁-C₆)alkoxy, (C₁-C₆)Alkylthio und/oder (C₁-C₆)Halogenalkylthio,
Q' ein unsubstituierter oder mit einem oder mehreren Resten R⁷' substituierter Phenyl oder Pyridyl Rest ist,
wobei der oder die Substituenten R⁷' jeweils unabhängig voneinander ausgewählt sind aus:
Cyano, Halogen, (C₃-C₆)Cycloalkyl, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy,
und
D' ein unsubstituierter oder mit einem oder mehreren Resten R⁸' substituierter C₁-C₆-Alkyl, Phenyl, Thiophenyl, Isoxazolyl, Benzyl oder Benzdioxolyl Rest ist,
wobei der oder die Substituenten R⁸' jeweils unabhängig voneinander ausgewählt sind aus:
Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₈)Cycloalkylamino, (C₁-C₆)Alkylcarbonylamino, (1-Pyrazolyl)(C₁-C₃)alkyl und/oder (C₁-C₃)Alkoxypyrimidinyloxy.

15. Verbindungen der Formel (I') gemäß Anspruch 13, **dadurch gekennzeichnet, dass**
R^{1'} Halogen, Cyclopropyl, (C₁-C₄)Alkyl, (C₁-C₄)Alkylthio, Halogencyclopropyl, (C₁-C₄)Halogenalkyl oder unsubstituiertes oder einfach oder mehrfach, gleich oder verschieden substituiertes Benzyl ist, wobei als Substituenten jeweils in Frage kommen:Cyano, Halogen, (C₁-C₄)Halogenalkyl und/oder (C₁-C₄)Alkoxy,
R^{2'} Wasserstoff, Halogen, Cyano, (C₁-C₄)Alkyl, (C₁-C₄)Halogenalkyl ist,
R^{3'} Halogen, Cyclopropyl, (C₁-C₄)Alkyl, Halogencyclopropyl oder (C₁-C₄)Halogenalkyl ist,
R^{4'} Wasserstoff, Halogen, Cyano, (C₁-C₄)Alkyl, Cyclopropyl, oder (C₁-C₄)Halogenalkyl ist,
R^{5'} Cyclopropyl, (C₁-C₄)Alkyl oder Halogen(C₁-C₄)alkyl ist,
R^{6'} Wasserstoff, Halogen, Cyano, (C₁-C₄)Alkyl oder (C₁-C₄)Halogenalkyl ist,
A' Halogen(C₁-C₄)Alkyl-(C₃-C₄)cycloalkyl, Halogen(C₃-C₆)cycloalkyl, (C₁-C₆)Alkyl, (C₁-C₅)Halogenalkyl, (C₁-C₆)Cyanoalkyl, (C₁-C₄)Alkoxy-(C₁-C₄)alkyl, Halogen(C₁-C₃)alkoxy-(C₁-C₄)alkyl, (C₁-C₄)Alkylthio-(C₁-C₄)alkyl, (C₁-C₄)Alkylsulfinyl-(C₁-C₄)alkyl, Halogen(C₃-C₄)alkenyl oder ein gegebenenfalls einfach oder mehrfach, gleich oder verschieden substituierter Benzyl Rest ist, wobei der oder die Substituenten jeweils unabhängig voneinander ausgewählt sind aus:
Cyano, Halogen, (C₃-C₆)Cycloalkyl, (C₃-C₆)Cycloalkyloxy, Halogen(C₃-C₈)cycloalkyl, (C₁-C₆)Alkyl, (C₁-C₆)Halogenalkyl, (C₁-C₆)Alkoxy-(C₁-C₆)alkyl, (C₁-C₆)Alkoxy, (C₁-C₆)Halogenalkoxy, (C₁-C₆)Alkylthio und/oder (C₁-C₆)Halogenalkylthio,
Q' ein unsubstituierter oder mit einem oder mehreren Resten R⁷' substituierter Phenyl oder Pyridyl Rest ist,
wobei der oder die Substituenten R⁷' jeweils unabhängig voneinander ausgewählt sind aus:
Cyano, Halogen, (C₃-C₆)Cycloalkyl, Halogen(C₃-C₆)cycloalkyl, (C₁-C₆)Alkyl, (C₁-C₆)Halogenalkyl, (C₁-C₆)Alkoxy-(C₁-C₆)alkyl, (C₁-C₆)Alkoxy und/oder (C₁-C₆)Halogenalkoxy
und
D' ein unsubstituierter oder mit einem oder mehreren Resten R⁸' substituierter C₁-C₆-Alkyl, Phenyl, Thiophenyl, Isoxazolyl, Benzyl oder Benzdioxolyl Rest ist,
wobei der oder die Substituenten R⁸' jeweils unabhängig voneinander ausgewählt sind aus:
Cyano, Halogen, Acetyl, (C₃-C₆)Cycloalkyl, (C₃-C₆)Cycloalkyloxy, (C₃-C₆)Cycloalkyl-(C₃-C₈)Cycloalkyl, (C₁-C₆)Alkyl-(C₃-C₆)cycloalkyl, Halogen(C₃-C₆)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₁-C₆)Alkoxy, (C₁-C₆)Halogenalkoxy, (C₁-C₆)Cyanoalkoxy, (C₁-C₆)Alkoxy-(C₁-C₆)alkoxy, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, (C₃-C₆)Cycloalkylamino, (C₁-C₆)Alkylcarbonylamino, (1-Pyrazolyl)(C₁-C₃)alkyl und/oder (C₁-C₃)Alkoxypyrimidinyloxy.

16. Verbindungen der Formel (I') gemäß Anspruch 13, **dadurch gekennzeichnet, dass**
R^{1'} Chlor, Brom, Methyl, Benzyl, 2-Chlorbenzyl, 3-Chlorbenzyl, 4-Chlorbenzyl, Trifluormethyl, Methylthio oder Isopropylthio ist,
R^{2'} Wasserstoff, Cyano, Chlor, Brom oder Iod ist,
R^{3'} Chlor, Methyl, Isopropyl, Ethyl oder Brom ist,
R^{4'} Wasserstoff, Chlor, Brom, Iod, Fluor, Difluormethyl, Isopropyl oder Cyclopropyl ist,
R^{5'} Methyl oder 2,2,2-Trifluorethyl ist,
R^{6'} Wasserstoff oder Chlor ist,
A' ein Rest ausgewählt aus den Resten der Formeln (III1-III19) ist: 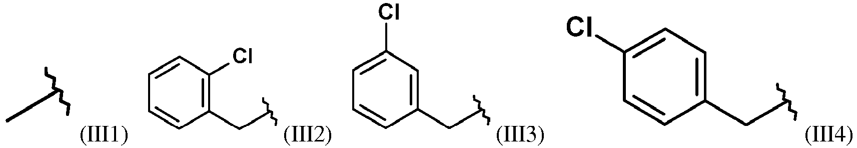 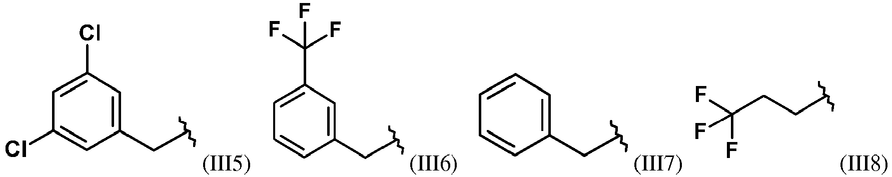 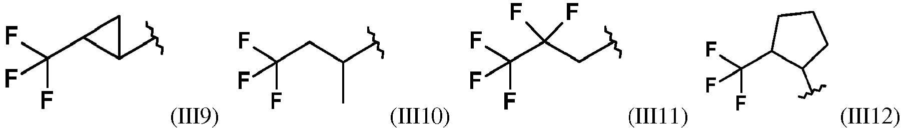 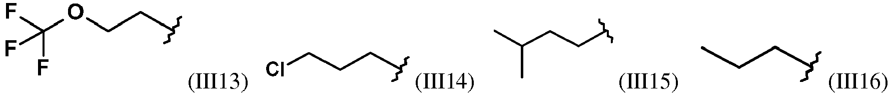 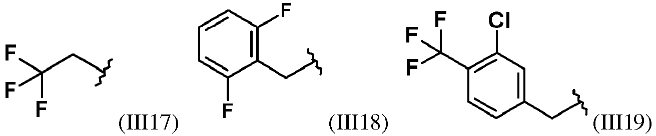
Q' ein Rest ausgewählt aus den Resten der Formeln (IV1-IV40) ist: 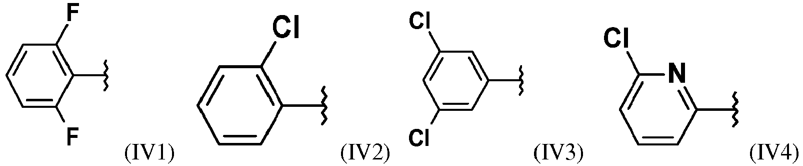 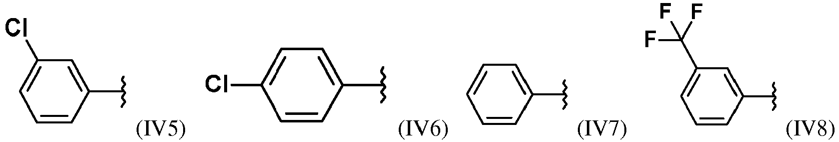 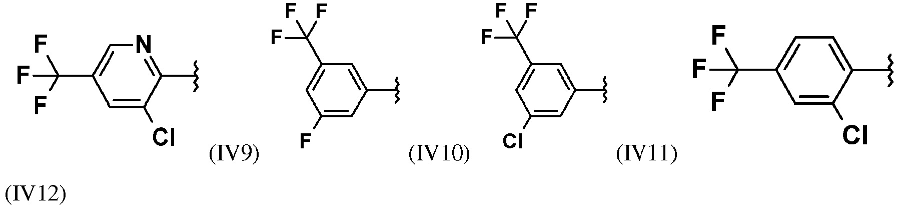 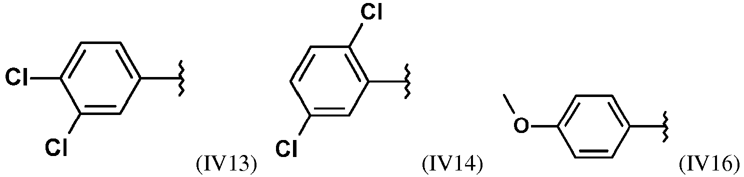 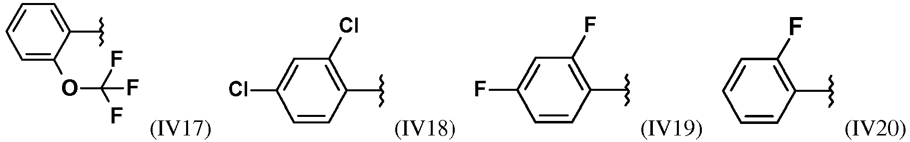 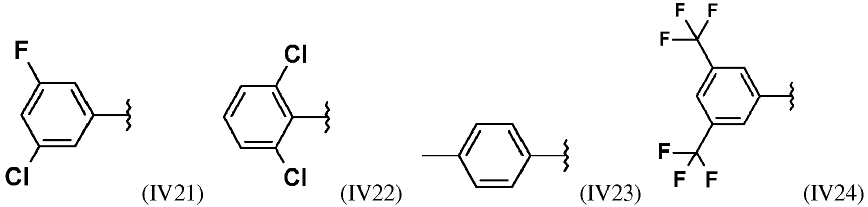 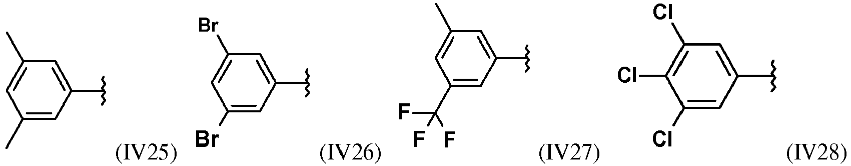 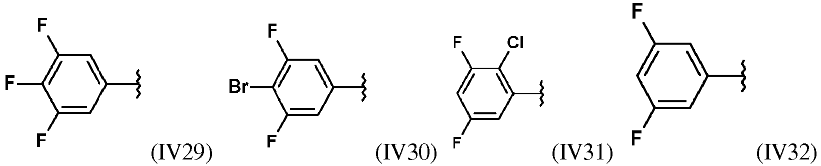 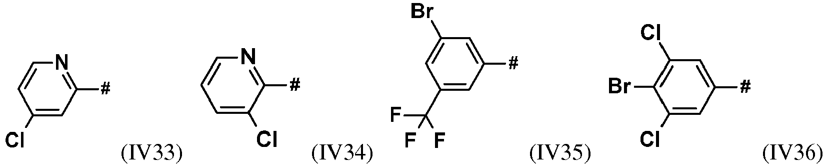 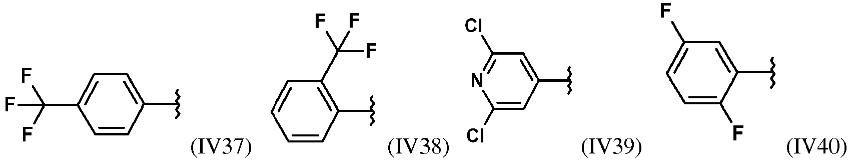 und
D' ein Rest ausgewählt aus den Resten der Formeln (V1-V61) ist:

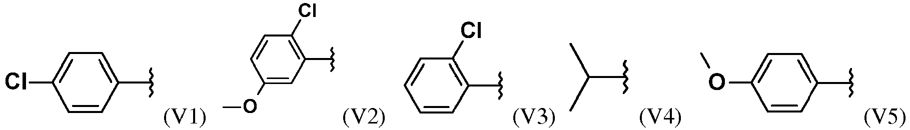

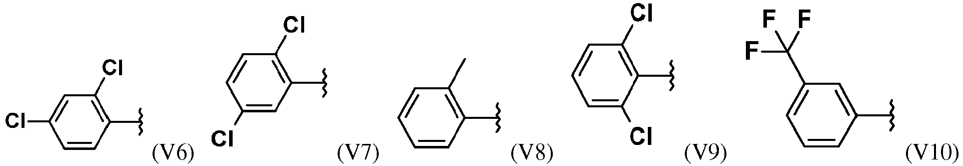

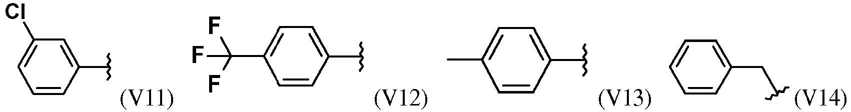

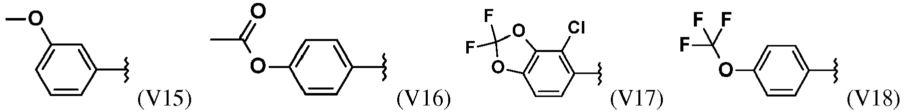

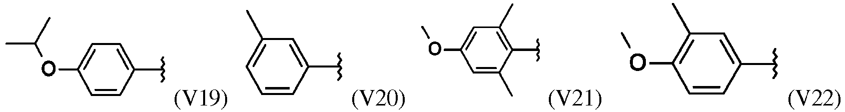

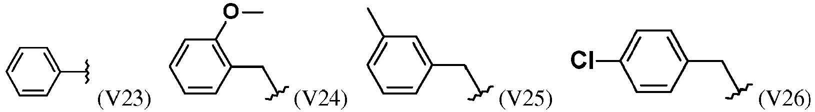

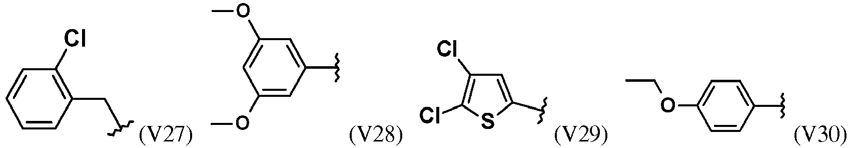

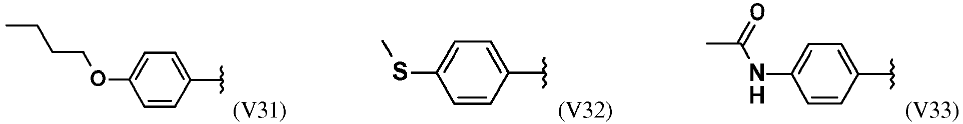

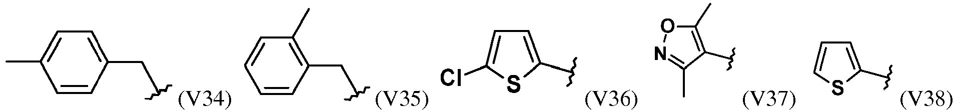

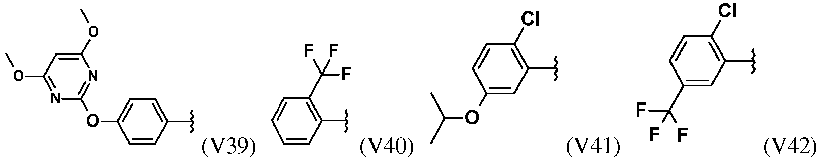

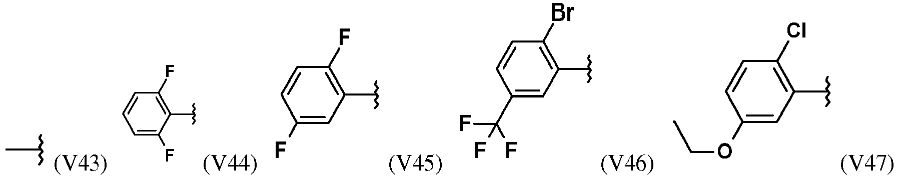

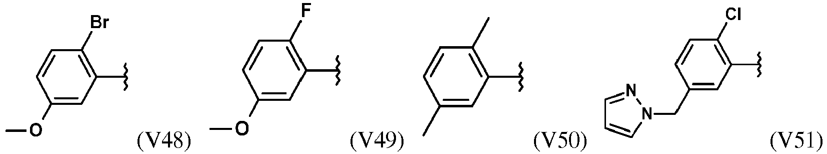

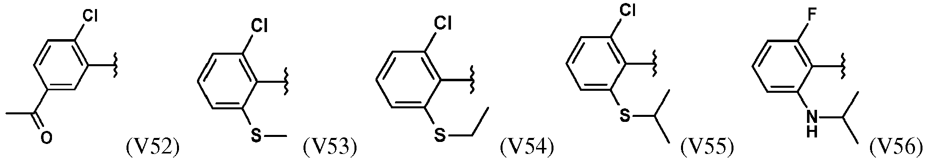

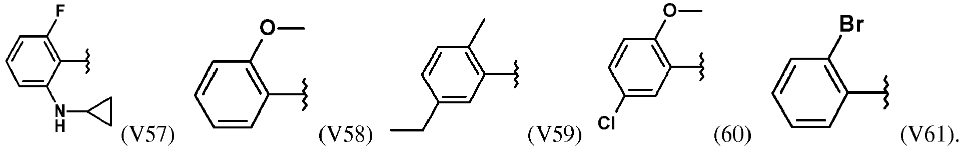

17. Verbindungen der Formel (I') gemäß einem der Ansprüche 13 bis 16, **dadurch gekennzeichnet, dass** in Formeln (I') M' für einen Rest ausgewählt aus den Formeln IIa' oder IIb' steht, wobei die Reste R¹', R²', A', R³', R⁴', R⁷', R⁸', Q' und D' die in Anspruch 13,14,15 oder 16 beschriebenen Bedeutungen haben.

18. Verbindungen der Formeln (Ia'), (Ib') oder (Ic') gemäß einem der Ansprüche 13 bis 16, 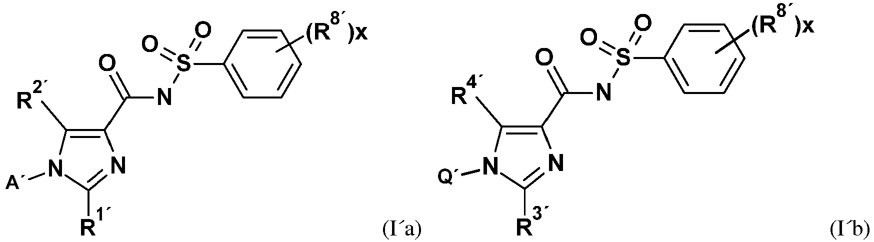 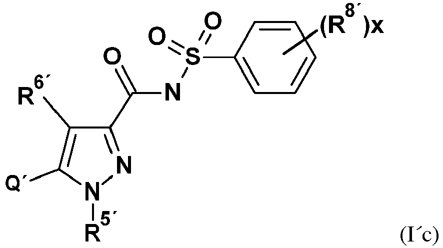 wobei die Reste R¹', R²', R³', R⁴', R⁵', R⁶', R⁷', A', Q' und R⁸' die in Anspruch 13,14,15 oder 16 beschriebenen Bedeutungen haben.

19. Zwischenprodukte der Formeln (VIa), (Xa) und (IXa), 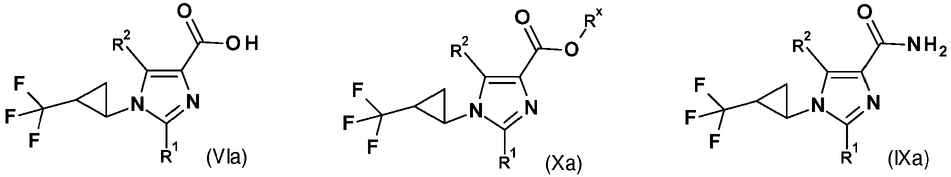 in welchen R¹ und R² die in Anspruch 2, 3 oder 4 genannten Bedeutungen haben und R^{x} für (C₁-C₆) Alkyl oder (C₃-C₈)Cycloalkyl steht.

## Claims

1. Non-therapeutic use of a compound of the formula (I) 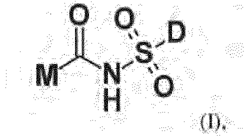 in which
M represents a radical selected from the formulae (IIa-IIc): 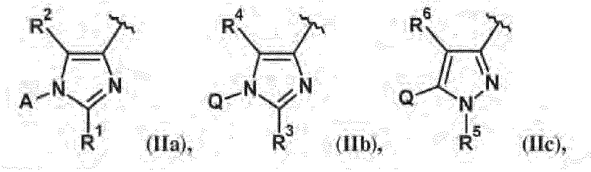 where
R¹ represents hydrogen, cyano, halogen, nitro, (C₃-C₈)-cycloalkyl, (C₃-C₈) -cycloalkyloxy, (C₃-C₈) -cycloalkyl-(C₃-C₈) -cycloalkyl, (C₁-C₆) -alkyl- (C₃-C₈) -cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy-(C₁-C₆)-alkyl, (C₁-C₆)-alkoxy, (C₁-C₆) -haloalkoxy, (C₁-C₆)-cyanoalkoxy, (C₁-C₆) -alkoxy- (C₁-C₆) -alkoxy, (C₁-C₆)-alkoxyimino, (C₁-C₆) -alkylthio, (C₁-C₆) -haloalkylthio, (C₁-C₆) -alkoxy- (C₁-C₆) -alkylthio, (C₁-C₆) -alkylthio-(C₁-C₆) -alkyl, (C₁-C₆) -alkylsulfinyl- (C₁-C₆) -alkyl, (C₁-C₆) -alkylsulfonyl- (C₁-C₆) -alkyl, (C₁-C₆) -alkoxycarbonyl, (C₁-C₆) -haloalkoxycarbonyl, (C₁-C₆) -alkylaminocarbonyl, (C₁-C₆)-alkylcarbonylamino or benzyl which is unsubstituted or mono- or polysubstituted by identical or different substituents, possible substituents being in each case:
cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl- (C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆) -alkoxy- (C₁-C₆) -alkyl, (C₁-C₆) -alkoxy, (C₁-C₆) -haloalkoxy, (C₁-C₆) -cyanoalkoxy, (C₁-C₆) -alkoxy-(C₁-C₆) -alkoxy, (C₁-C₆) -alkylthio, (C₁-C₆) -haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl-(C₁-C₆)-alkyl and/or (C₁-C₆)-alkylsulfonyl- (C₁-C₆)-alkyl,
R² represents hydrogen, cyano, halogen, nitro, (C₃-C₈)-cycloalkyl, (C₃-C₈) -cycloalkyloxy, (C₃-C₈) -cycloalkyl-(C₃-C₈)-cycloalkyl, (C₁-C₆)-alkyl- (C₃-C₈)-cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy-(C₁-C₆)-alkyl, (C₁-C₆)-alkoxy, (C₁-C₆)-haloalkoxy, (C₁-C₆)-cyanoalkoxy, (C₁-C₆) -alkoxy- (C₁-C₆) -alkoxy, (C₁-C₆)-alkoxyimino, (C₁-C₆) -alkylthio, (C₁-C₆) -haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl- (C₁-C₆)-alkyl, (C₁-C₆)-alkylsulfonyl- (C₁-C₆)-alkyl, (C₁-C₆)-alkoxycarbonyl, (C₁-C₆) -haloalkoxycarbonyl, (C₁-C₆) -alkylaminocarbonyl or (C₁-C₆)-alkylcarbonylamino,
R³ represents hydrogen, cyano, halogen, nitro, (C₃-C₈)-cycloalkyl, (C₃-C₈) -cycloalkyloxy, (C₃-C₈) -cycloalkyl-(C₃-C₈)-cycloalkyl, (C₁-C₆)-alkyl- (C₃-C₈)-cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy-(C₁-C₆)-alkyl, (C₁-C₆)-alkoxy, (C₁-C₆)-haloalkoxy, (C₁-C₆)-cyanoalkoxy, (C₁-C₆) -alkoxy- (C₁-C₆) -alkoxy, (C₁-C₆)-alkoxyimino, (C₁-C₆) -alkylthio, (C₁-C₆) -haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl- (C₁-C₆)-alkyl, (C₁-C₆)-alkylsulfonyl- (C₁-C₆)-alkyl, (C₁-C₆)-alkoxycarbonyl, (C₁-C₆) -haloalkoxycarbonyl, (C₁-C₆) -alkylaminocarbonyl, (C₁-C₆)-alkylcarbonylamino or benzyl which is unsubstituted or mono- or polysubstituted by identical or different substituents, possible substituents being in each case:
cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl- (C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆) -alkoxy- (C₁-C₆) -alkyl, (C₁-C₆) -alkoxy, (C₁-C₆) -haloalkoxy, (C₁-C₆) -cyanoalkoxy, (C₁-C₆) -alkoxy-(C₁-C₆) -alkoxy, (C₁-C₆) -alkylthio, (C₁-C₆) -haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl-(C₁-C₆)-alkyl and/or (C₁-C₆)-alkylsulfonyl- (C₁-C₆)-alkyl,
R⁴ represents hydrogen, cyano, halogen, nitro, (C₃-C₈)-cycloalkyl, (C₃-C₈) -cycloalkyloxy, (C₃-C₈) -cycloalkyl-(C₃-C₈)-cycloalkyl, (C₁-C₆)-alkyl- (C₃-C₈)-cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy-(C₁-C₆)-alkyl, (C₁-C₆)-alkoxy, (C₁-C₆)-haloalkoxy, (C₁-C₆)-cyanoalkoxy, (C₁-C₆) -alkoxy- (C₁-C₆) -alkoxy, (C₁-C₆)-alkoxyimino, (C₁-C₆) -alkylthio, (C₁-C₆) -haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl- (C₁-C₆)-alkyl, (C₁-C₆)-alkylsulfonyl- (C₁-C₆)-alkyl, (C₁-C₆)-alkoxycarbonyl, (C₁-C₆) -haloalkoxycarbonyl, (C₁-C₆) -alkylaminocarbonyl or (C₁-C₆)-alkylcarbonylamino,
R⁵ represents hydrogen, (C₃-C₈)-cycloalkyl, (C₃-C₈)-cycloalkyl- (C₃-C₈)-cycloalkyl, (C₁-C₆)-alkyl- (C₃-C₈)-cycloalkyl, halo-(C₃-C₈)-cycloalkyl, (C₁-C₆)-alkyl, (C₁-C₆) -haloalkyl, (C₁-C₆) -cyanoalkyl, (C₁-C₆) -alkoxy-(C₁-C₆) -alkyl, (C₁-C₆) -alkylthio- (C₁-C₆) -alkyl, halo-(C₁-C₆)-alkylthio- (C₁-C₆)-alkyl, (C₁-C₆)-alkylsulfinyl-(C₁-C₆)-alkyl, (C₁-C₆)-alkylsulfonyl-(C₁-C₆)-alkyl, halo-(C₁-C₆)-alkylsulfinyl-(C₁-C₆)-alkyl or halo- (C₁-C₆)-alkylsulfonyl- (C₁-C₆)-alkyl,
R⁶ represents hydrogen, cyano, halogen, nitro, (C₃-C₈)-cycloalkyl, (C₃-C₈) -cycloalkyloxy, (C₃-C₈) -cycloalkyl-(C₃-C₈)-cycloalkyl, (C₁-C₆)-alkyl- (C₃-C₈)-cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy-(C₁-C₆)-alkyl, (C₁-C₆)-alkoxy, (C₁-C₆) -haloalkoxy, (C₁-C₆)-cyanoalkoxy, (C₁-C₆) -alkoxy- (C₁-C₆) -alkoxy, (C₁-C₆)-alkoxyimino, (C₁-C₆) -alkylthio, (C₁-C₆) -haloalkylthio, (C₁-C₆) -alkoxy- (C₁-C₆) -alkylthio, (C₁-C₆) -alkylthio-(C₁-C₆) -alkyl, (C₁-C₆) -alkylsulfinyl- (C₁-C₆) -alkyl, (C₁-C₆) -alkylsulfonyl- (C₁-C₆) -alkyl, (C₁-C₆) -alkoxycarbonyl, (C₁-C₆) -haloalkoxycarbonyl, (C₁-C₆) -alkylaminocarbonyl or (C₁-C₆)-alkylcarbonylamino,
A represents halo-(C₁-C₆)-alkyl-(C₃-C₈)-cycloalkyl, halo-(C₃-C₈) -cycloalkyl, (C₁-C₆) -alkyl, (C₁-C₆) -haloalkyl, (C₁-C₆) -cyanoalkyl, (C₁-C₆) -alkoxy- (C₁-C₆) -alkyl, halo-(C₁-C₃)-alkoxy-(C₁-C₄)-alkyl, (C₁-C₆) -alkylthio (C₁-C₆) - alkyl, (C₁-C₆) -alkylsulfinyl- (C₁-C₆) -alkyl, halo- (C₁-C₆) - alkylthio- (C₁-C₆) -alkyl, halo- (C₁-C₆) -alkylsulfinyl-(C₁-C₆) -alkyl, halo- (C₁-C₆) -alkylsulfonyl- (C₁-C₆) -alkyl, halo-(C₃-C₆)-alkenyl or
a benzyl radical which is optionally mono- or polysubstituted by identical or different substituents, the substituent(s) being in each case independently of one another selected from:
cyano, halogen, (C₃-C₆)-cycloalkyl, (C₃-C₆)-cycloalkyloxy, (C₃-C₆)-cycloalkyl- (C₃-C₈) -cycloalkyl, (C₁-C₆)-alkyl- (C₃-C₈) -cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -alkoxy- (C₁-C₆) -alkyl, (C₁-C₆) -alkoxy, (C₁-C₆)-haloalkoxy, (C₁-C₆) -cyanoalkoxy, (C₁-C₆) -alkoxy- (C₁-C₆) - alkoxy, (C₁-C₆) -alkoxyimino, (C₁-C₆) -alkylthio, (C₁-C₆)-haloalkylthio, (C₁-C₆) -alkoxy- (C₁-C₆) -alkylthio, (C₁-C₆)-alkylthio- (C₁-C₆) -alkyl, (C₁-C₆) -alkylsulfinyl- (C₁-C₆) - alkyl, (C₁-C₆) -alkylsulfonyl- (C₁-C₆) -alkyl, halo- (C₁-C₆) - alkylthio- (C₁-C₆) -alkyl, halo- (C₁-C₆) -alkylsulfinyl-(C₁-C₆) -alkyl and/or halo- (C₁-C₆) -alkylsulfonyl- (C₁-C₆) - alkyl,
Q represents a phenyl or pyridyl radical which is unsubstituted or substituted by one or more radicals R⁷, where the substituent (s) R⁷ are in each case independently of one another selected from:
cyano, halogen, nitro, (C₃-C₆)-cycloalkyl, (C₃-C₆)-cycloalkyloxy, (C₃-C₆)-cycloalkyl- (C₃-C₈) -cycloalkyl, (C₁-C₆)-alkyl- (C₃-C₈) -cycloalkyl, halo- (C₃-C₈) -cycloalkyl, (C₁-C₆) -alkyl, (C₁-C₆) -haloalkyl, (C₁-C₆) -cyanoalkyl, (C₁-C₆) -hydroxyalkyl, (C₁-C₆) -alkoxy- (C₁-C₆) -alkyl, (C₁-C₆) -alkoxy, (C₁-C₆) -haloalkoxy, (C₁-C₆) -cyanoalkoxy, (C₁-C₆) -alkoxy- (C₁-C₆) -alkoxy, (C₁-C₆) -alkoxyimino, (C₁-C₆) -alkylthio, (C₁-C₆) -haloalkylthio, (C₁-C₆) -alkoxy-(C₁-C₆) -alkylthio, (C₁-C₆) -alkylthio- (C₁-C₆) -alkyl, (C₁-C₆) -alkylsulfinyl- (C₁-C₆) -alkyl, (C₁-C₆) -alkylsulfonyl- (C₁-C₆) -alkyl, (C₁-C₆) -alkoxycarbonyl, (C₁-C₆)-haloalkoxycarbonyl, (C₁-C₆)-alkylaminocarbonyl, (C₁-C₆)-alkylcarbonylamino, halo- (C₁-C₆) -alkylthio- (C₁-C₆) -alkyl, halo- (C₁-C₆) -alkylsulfinyl- (C₁-C₆) -alkyl and/or halo-(C₁-C₆) -alkylsulfonyl- (C₁-C₆) -alkyl,
and
D represents a C₁-C₆-alkyl, phenyl, thiophenyl, isoxazolyl, phenyl-(C₁-C₂)-alkyl or benzodioxolyl radical which is unsubstituted or substituted by one or more radicals R⁸,
where the substituent (s) R⁸ are in each case independently of one another selected from:
cyano, halogen, nitro, acetyl, (C₃-C₈)-cycloalkyl, (C₃-C₈) -cycloalkyloxy, (C₃-C₈) -cycloalkyl- (C₃-C₈) - cycloalkyl, (C₁-C₆) -alkyl- (C₃-C₈) -cycloalkyl, halo-(C₃-C₈) -cycloalkyl, (C₁-C₆) -alkyl, (C₁-C₆) -haloalkyl, (C₁-C₆) -cyanoalkyl, (C₁-C₆) -hydroxyalkyl, hydroxycarbonyl- (C₁-C₆) -alkoxy, (C₁-C₆) -alkoxycarbonyl- (C₁-C₆) - alkyl, (C₁-C₆) -alkoxy- (C₁-C₆) -alkyl, (C₂-C₆) -alkenyl, (C₂-C₆) -haloalkenyl, (C₂-C₆) -cyanoalkenyl, (C₂-C₆) - alkynyl, (C₂-C₆) -haloalkynyl, (C₂-C₆) -cyanoalkynyl, (C₁-C₆) -alkoxy, (C₁-C₆) -haloalkoxy, (C₁-C₆) -cyanoalkoxy, (C₁-C₆) -alkoxycarbonyl- (C₁-C₆) -alkoxy, (C₁-C₆) -alkoxy-(C₁-C₆) -alkoxy, (C₁-C₆) -alkylhydroxyimino, (C₁-C₆)-alkoxyimino, (C₁-C₆) -alkyl- (C₁-C₆) -alkoxyimino, (C₁-C₆)-haloalkyl-(C₁-C₆)-alkoxyimino, (C₁-C₆) -alkylthio, (C₁-C₆)-haloalkylthio, (C₁-C₆) -alkoxy- (C₁-C₆) -alkylthio, (C₁-C₆)-alkylthio- (C₁-C₆) -alkyl, (C₁-C₆) -alkylsulfinyl, (C₁-C₆)-haloalkylsulfinyl, (C₁-C₆) -alkoxy- (C₁-C₆) -alkylsulfinyl, (C₁-C₆) -alkylsulfinyl- (C₁-C₆) -alkyl, (C₁-C₆)-alkylsulfonyl, (C₁-C₆)-haloalkylsulfonyl, (C₁-C₆)-alkoxy-(C₁-C₆)-alkylsulfonyl, (C₁-C₆)-alkylsulfonyl- (C₁-C₆)-alkyl, (C₁-C₆) -alkylsulfonyloxy, (C₁-C₆) -alkylcarbonyl, (C₁-C₆)-alkylthiocarbonyl, (C₁-C₆)-haloalkylcarbonyl, (C₁-C₆) -alkylcarbonyloxy, (C₁-C₆) -alkoxycarbonyl, (C₁-C₆)-haloalkoxycarbonyl, aminocarbonyl, (C₁-C₆)-alkylaminocarbonyl, (C₁-C₆) -alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylaminocarbonyl, (C₃-C₈) -cycloalkylaminocarbonyl, (C₁-C₆)-alkylsulfonylamino, (C₁-C₆) -alkylamino, di-(C₁-C₆) - alkylamino, aminosulfonyl, (C₁-C₆)-alkylaminosulfonyl, di-(C₁-C₆)-alkylaminosulfonyl, (C₁-C₆)-alkylsulfoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino, (1-pyrazolyl) (C₁-C₃)-alkyl and/or (C₁-C₃)-alkoxypyrimidinyloxy,
for controlling animal pests.

2. Non-therapeutic use according to Claim 1, **characterized in that**
R¹ represents hydrogen, cyano, halogen, cyclopropyl, (C₁-C₄)-alkyl, halocyclopropyl, halo-(C₁-C₃)-alkylcyclopropyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylthio- (C₁-C₄)-alkyl, (C₁-C₄)-alkylsulfinyl-(C₁-C₄)-alkyl, (C₁-C₄)-alkylsulfonyl- (C₁-C₄)-alkyl, or benzyl which is unsubstituted or mono- or polysubstituted by identical or different substituents, possible substituents being in each case: cyano, halogen, (C₁-C₄)-haloalkyl, (C₁-C₄)-alkoxy, (C₁-C₄)-haloalkoxy, (C₁-C₆)-alkylthio and/or (C₁-C₆)-haloalkylthio,
R² represents hydrogen, cyano, halogen, (C₁-C₄)-haloalkyl, (C₁-C₄)-alkoxy, (C₁-C₄)-haloalkoxy, (C₁-C₆)-alkylthio or (C₁-C₆)-haloalkylthio,
R³ represents hydrogen, cyano, halogen, cyclopropyl, (C₁-C₄)-alkyl, halocyclopropyl, halo-(C₁-C₃)-alkylcyclopropyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylthio- (C₁-C₄)-alkyl, (C₁-C₄)-alkylsulfinyl- (C₁-C₄)-alkyl or (C₁-C₄)-alkylsulfonyl-(C₁-C₄)-alkyl,
R⁴ represents hydrogen, cyano, halogen, (C₁-C₄)-haloalkyl, (C₁-C₄)-alkoxy, (C₁-C₄)-alkyl, (C₃-C₄)-cycloalkyl, (C₁-C₄)-haloalkoxy, (C₁-C₆)-alkylthio or (C₁-C₆)-haloalkylthio,
R⁵ represents hydrogen, cyclopropyl, (C₁-C₄)-alkyl, halocyclopropyl, halo-(C₁-C₃)-alkylcyclopropyl or (C₁-C₄)-haloalkyl,
R⁶ represents hydrogen, cyano, halogen, (C₁-C₄)-haloalkyl, (C₁-C₄)-alkoxy, (C₁-C₄)-haloalkoxy, 4 (C₁-C₆)-alkylthio or (C₁-C₆)-haloalkylthio,
A represents halo-(C₁-C₄)-alkyl-(C₃-C₄)-cycloalkyl, halo-(C₃-C₆)-cycloalkyl, (C₁-C₆) -alkyl, (C₁-C₅)-haloalkyl, (C₁-C₆) -cyanoalkyl, (C₁-C₄)-alkoxy- (C₁-C₄)-alkyl, halo-(C₁-C₃)-alkoxy- (C₁-C₄)-alkyl, (C₁-C₄)-alkylthio- (C₁-C₄)-alkyl, (C₁-C₄)-alkylsulfinyl-(C₁-C₄)-alkyl, halo- (C₃-C₄)-alkenyl or
a benzyl radical which is optionally mono- or polysubstituted by identical or different substituents, where the substituent(s) are in each case independently of one another selected from:
cyano, halogen, (C₃-C₆)-cycloalkyl, (C₃-C₆)-cycloalkyloxy, halo- (C₃-C₈)-cycloalkyl, (C₁-C₆)-alkyl, (C₁-C₆)-haloalkyl, (C₁-C₆) -cyanoalkyl, (C₁-C₆) -alkoxy- (C₁-C₆) - alkyl, (C₁-C₆)-alkoxy, (C₁-C₆)-haloalkoxy, (C₁-C₆)-cyanoalkoxy, (C₁-C₆) -alkoxy- (C₁-C₆) -alkoxy, (C₁-C₆) -alkylthio and/or (C₁-C₆)-haloalkylthio,
Q represents a phenyl or pyridyl radical which is unsubstituted or substituted by one or more radicals R⁷, where the substituent (s) R⁷ are in each case independently of one another selected from:
cyano, halogen, (C₃-C₆)-cycloalkyl, halo-(C₃-C₈)-cycloalkyl, (C₁-C₆)-alkyl, (C₁-C₆)-haloalkyl, (C₁-C₆)-alkoxy-(C₁-C₆)-alkyl, (C₁-C₆)-alkoxy and/or (C₁-C₆)-haloalkoxy, and
D represents a C₁-C₆-alkyl, phenyl, thiophenyl, isoxazolyl, benzyl or benzodioxolyl radical which is unsubstituted or substituted by one or more radicals R⁸, where the substituent (s) R⁸ are in each case independently of one another selected from:
cyano, halogen, acetyl, (C₃-C₆)-cycloalkyl, (C₃-C₆)-cycloalkyloxy, (C₃-C₆)-cycloalkyl- (C₃-C₈) -cycloalkyl, (C₁-C₆)-alkyl- (C₃-C₆)-cycloalkyl, halo- (C₃-C₆)-cycloalkyl, (C₁-C₆) -alkyl, (C₁-C₆) -haloalkyl, (C₁-C₆) -cyanoalkyl, (C₁-C₆) -hydroxyalkyl, hydroxycarbonyl- (C₁-C₆) -alkoxy, (C₁-C₆) -alkoxycarbonyl- (C₁-C₆) -alkyl, (C₁-C₆) -alkoxy-(C₁-C₆)-alkyl, (C₂-C₆)-alkenyl, (C₂-C₆) -haloalkenyl, (C₂-C₆)-cyanoalkenyl, (C₂-C₆) -alkynyl, (C₂-C₆) -haloalkynyl, (C₂-C₆) -cyanoalkynyl, (C₁-C₆) -alkoxy, (C₁-C₆)-haloalkoxy, (C₁-C₆) -cyanoalkoxy, (C₁-C₆) -alkoxycarbonyl-(C₁-C₆) -alkoxy, (C₁-C₆) -alkoxy- (C₁-C₆) -alkoxy, (C₁-C₆)-alkoxyimino, (C₁-C₆) -alkyl- (C₁-C₆) -alkoxyimino, (C₁-C₆)-haloalkyl-(C₁-C₆)-alkoxyimino, (C₁-C₆) -alkylthio, (C₁-C₆)-haloalkylthio, (C₁-C₆) -alkoxy- (C₁-C₆) -alkylthio, (C₁-C₆)-alkylthio- (C₁-C₆) -alkyl, (C₁-C₆) -alkylsulfinyl, (C₁-C₆)-haloalkylsulfinyl, (C₁-C₆) -alkoxy- (C₁-C₆) -alkylsulfinyl, (C₁-C₆) -alkylsulfinyl- (C₁-C₆) -alkyl, (C₁-C₆) -alkylsulfonyl, (C₁-C₆) -haloalkylsulfonyl, (C₁-C₆) -alkoxy-(C₁-C₆) -alkylsulfonyl, (C₁-C₆) -alkylsulfonyl- (C₁-C₆) - alkyl, (C₁-C₆) -alkylsulfonyloxy, (C₁-C₆) -alkylcarbonyl, (C₁-C₆) -haloalkylcarbonyl, (C₁-C₆) -alkylcarbonyloxy, (C₁-C₆) -alkoxycarbonyl, (C₁-C₆) -haloalkoxycarbonyl, aminocarbonyl, (C₁-C₆) -alkylaminocarbonyl, di-(C₁-C₆)-alkylaminocarbonyl, (C₂-C₆)-alkylaminocarbonyl, di-(C₂-C₆) -alkenylaminocarbonyl, (C₃-C₈) -cycloalkylaminocarbonyl, (C₁-C₆)-alkylsulfonylamino, (C₁-C₆)-alkylamino, di-(C₁-C₆) -alkylamino, (C₃-C₈) -cycloalkylamino, (C₁-C₆)-alkylcarbonylamino, (1-pyrazolyl)(C₁-C₃)-alkyl and/or (C₁-C₃)-alkoxypyrimidinyloxy.

3. Non-therapeutic use according to Claim 1, **characterized in that**
R¹ represents hydrogen, halogen, cyclopropyl, (C₁-C₄)-alkyl, (C₁-C₄)-alkylthio, halocyclopropyl, (C₁-C₄)-haloalkyl or benzyl which is unsubstituted or mono- or polysubstituted by identical or different substituents, possible substituents being in each case: cyano, halogen, (C₁-C₄)-haloalkyl and/or (C₁-C₄)-alkoxy,
R² represents hydrogen, halogen, cyano, (C₁-C₄)-alkyl, (C₁-C₄)-haloalkyl,
R³ represents hydrogen, halogen, cyclopropyl, (C₁-C₄)-alkyl, halocyclopropyl or (C₁-C₄)-haloalkyl,
R⁴ represents hydrogen, halogen, cyano, (C₁-C₄)-alkyl, cyclopropyl or (C₁-C₄)-haloalkyl,
R⁵ represents hydrogen, cyclopropyl, (C₁-C₄)-alkyl or halo- (C₁-C₄)-alkyl,
R⁶ represents hydrogen, halogen, cyano, (C₁-C₄)-alkyl or (C₁-C₄)-haloalkyl,
A represents halo-(C₁-C₄)-alkyl-(C₃-C₄)-cycloalkyl, halo-(C₃-C₆)-cycloalkyl, (C₁-C₆)-alkyl, (C₁-C₅)-haloalkyl, (C₁-C₆)-cyanoalkyl, (C₁-C₄)-alkoxy- (C₁-C₄)-alkyl, halo-(C₁-C₃)-alkoxy- (C₁-C₄)-alkyl, (C₁-C₄)-alkylthio- (C₁-C₄)-alkyl, (C₁-C₄)-alkylsulfinyl-(C₁-C₄)-alkyl, halo- (C₃-C₄)-alkenyl or
a benzyl radical which is optionally mono- or polysubstituted by identical or different substituents, where the substituent(s) are in each case independently of one another selected from:
cyano, halogen, (C₃-C₆)-cycloalkyl, (C₃-C₆)-cyclo-alkyloxy, halo-(C₃-C₈)-cycloalkyl, (C₁-C₆)-alkyl, (C₁-C₆)-haloalkyl, (C₁-C₆)-alkoxy-(C₁-C₆)-alkyl, (C₁-C₆)-alkoxy, (C₁-C₆)-haloalkoxy, (C₁-C₆)-alkylthio and/or (C₁-C₆)-haloalkylthio,
Q represents a phenyl or pyridyl radical which is unsubstituted or substituted by one or more radicals R⁷, where the substituent (s) R⁷ are in each case independently of one another selected from:
cyano, halogen, (C₃-C₆)-cycloalkyl, halo-(C₃-C₆)-cycloalkyl, (C₁-C₆)-alkyl, (C₁-C₆)-haloalkyl, (C₁-C₆)-alkoxy-(C₁-C₆)-alkyl, (C₁-C₆)-alkoxy and/or (C₁-C₆)-haloalkoxy, and
D represents a C₁-C₆-alkyl, phenyl, thiophenyl, isoxazolyl, benzyl or benzodioxolyl radical which is unsubstituted or substituted by one or more radicals R⁸, where the substituent (s) R⁸ are in each case independently of one another selected from:
cyano, halogen, acetyl, (C₃-C₆)-cycloalkyl, (C₃-C₆)-cycloalkyloxy, (C₃-C₆)-cycloalkyl-(C₃-C₈)-cycloalkyl, (C₁-C₆)-alkyl- (C₃-C₆)-cycloalkyl, halo- (C₃-C₆)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl- (C₁-C₆)-alkoxy, (C₁-C₆)-alkoxycarbonyl- (C₁-C₆)-alkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy- (C₁-C₆)-alkoxy, (C₁-C₆)-alkoxyimino, (C₁-C₆)-alkyl- (C₁-C₆)-alkoxyimino, (C₁-C₆)-haloalkyl- (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio- (C₁-C₆)-alkyl, (C₁-C₆)-alkylsulfinyl, (C₁-C₆)-haloalkylsulfinyl, (C₁-C₆)-alkoxy-(C₁-C₆)-alkylsulfinyl, (C₁-C₆)-alkylsulfinyl-(C₁-C₆)-alkyl, (C₁-C₆)-alkylsulfonyl, (C₁-C₆)-haloalkylsulfonyl, (C₁-C₆)-alkoxy-(C₁-C₆)-alkylsulfonyl, (C₁-C₆)-alkylsulfonyl- (C₁-C₆)-alkyl, (C₁-C₆)-alkylsulfonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, (C₃-C₈)-cycloalkylaminocarbonyl, (C₁-C₆)-alkylsulfonylamino, (C₁-C₆)-alkylamino, di-(C₁-C₆)-alkylamino, (C₃-C₆)-cycloalkylamino, (C₁-C₆)-alkylcarbonylamino, (1-pyrazolyl)(C₁-C₃)-alkyl and/or (C₁-C₃)-alkoxy-pyrimidinyloxy.

4. Non-therapeutic use according to Claim 1, **characterized in that**
R¹ represents hydrogen, chlorine, bromine, methyl, benzyl, 2-chlorobenzyl, 3-chlorobenzyl, 4-chlorobenzyl, trifluoromethyl, methylthio or isopropylthio,
R² represents hydrogen, cyano, chlorine, bromine or iodine,
R³ represents hydrogen, chlorine, methyl, isopropyl, ethyl or bromine,
R⁴ represents hydrogen, chlorine, bromine, iodine, fluorine, difluoromethyl, isopropyl or cyclopropyl,
R⁵ represents methyl or 2,2,2-trifluoroethyl and
R⁶ represents hydrogen or chlorine,
A represents a radical selected from the radicals of the formulae (III1-III19) : 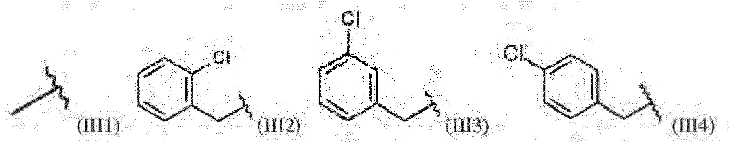 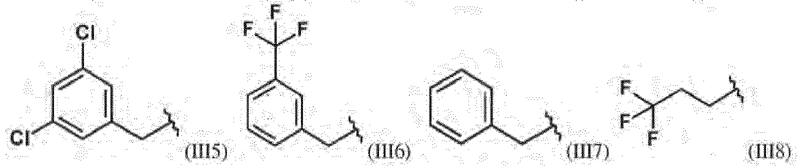 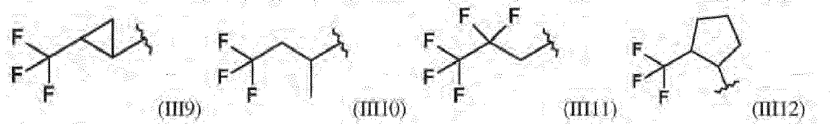 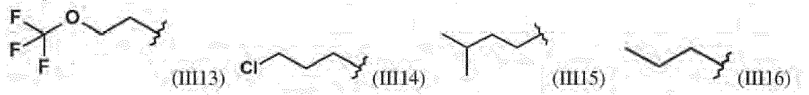 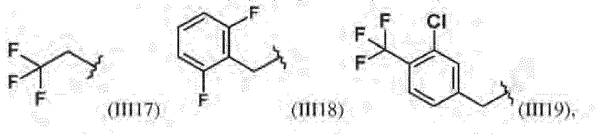
Q represents a radical selected from the radicals of the formulae (IV1-IV40): 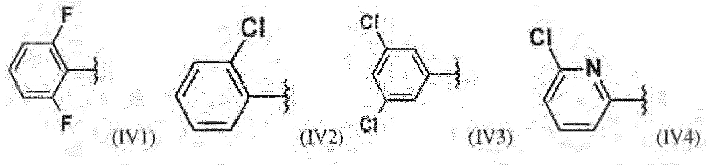 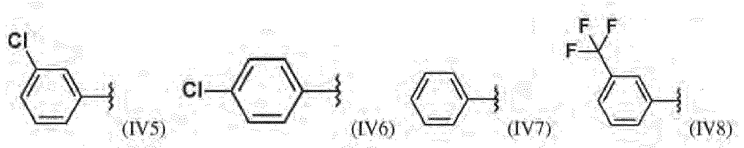 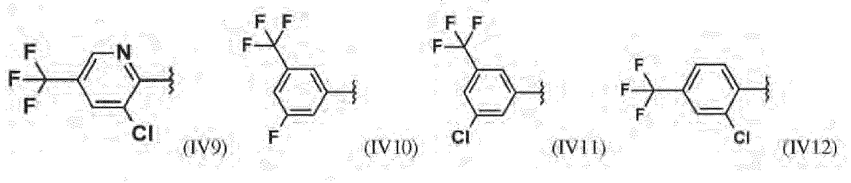 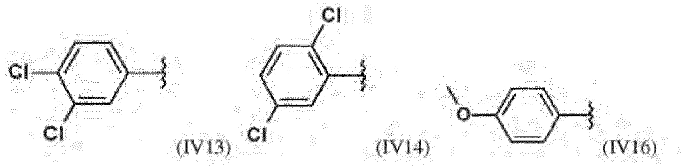 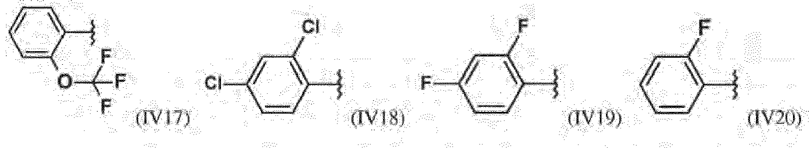 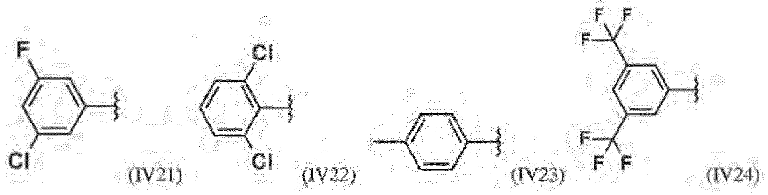 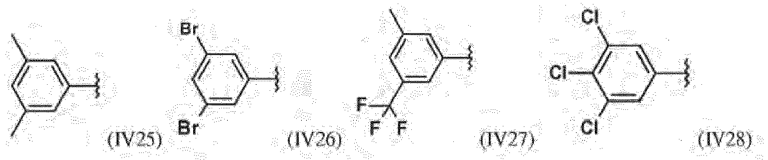 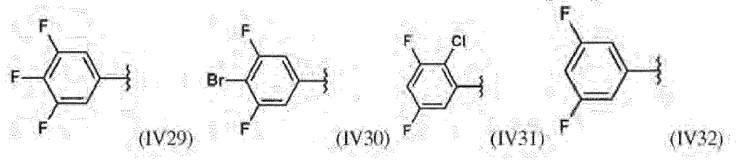 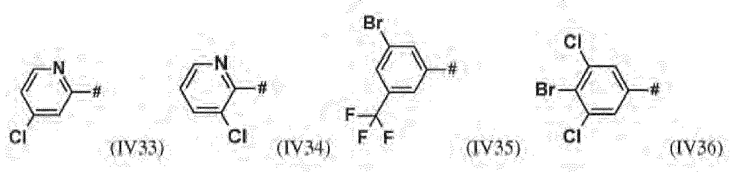 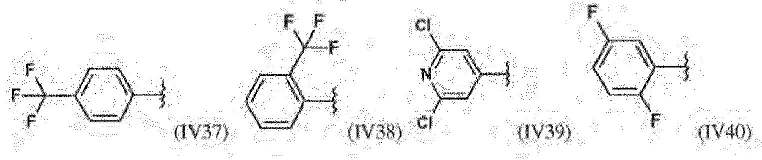 and
D represents a radical selected from the radicals of the formulae (V1-V61) :

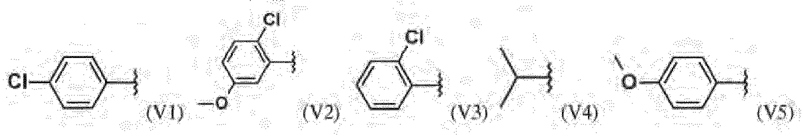

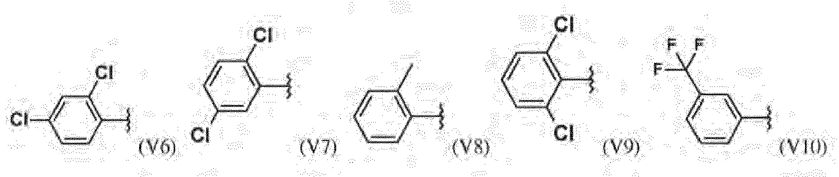

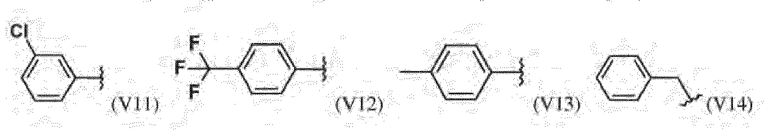

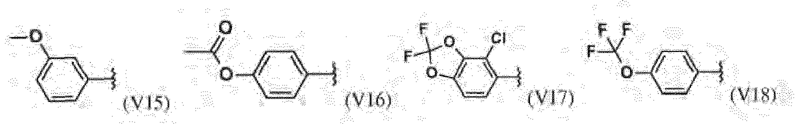

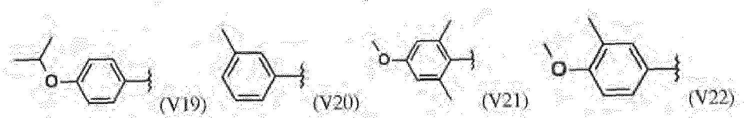

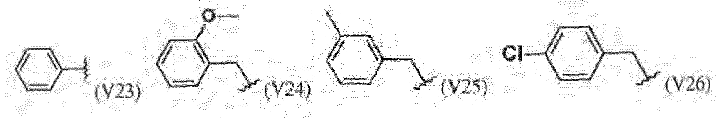

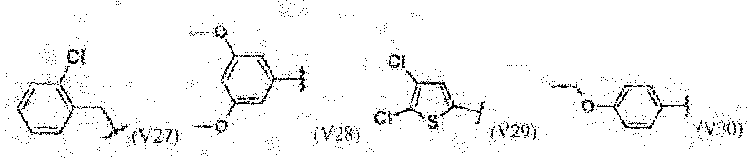

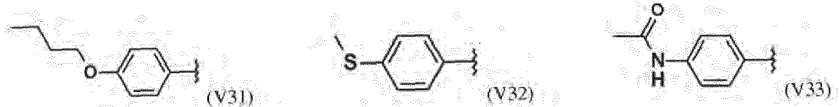

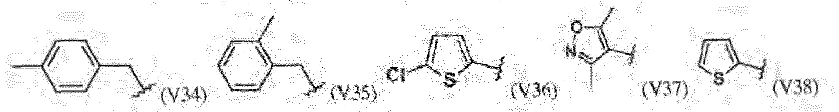

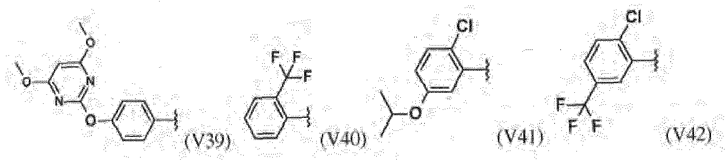

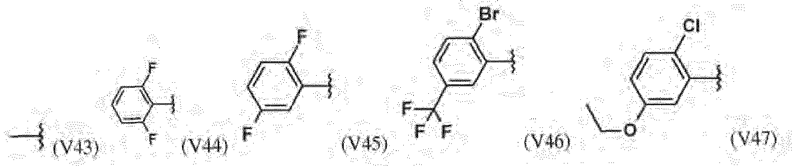

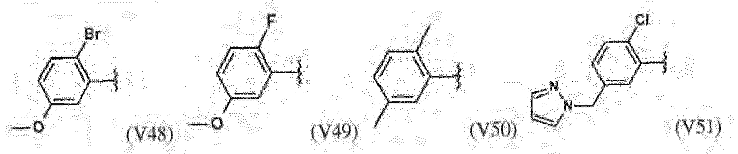

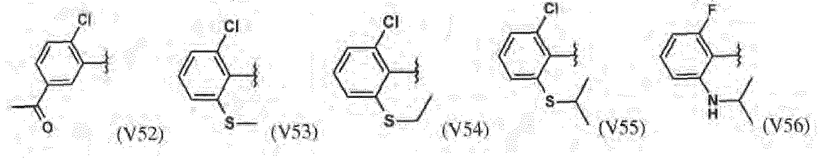

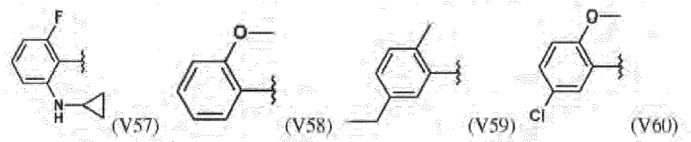

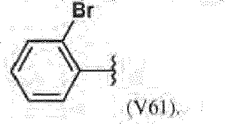

5. Non-therapeutic use according to any of Claims 1 to 4, **characterized in that** in formula (I) M represents a radical selected from the formulae IIa or IIb, where the radicals R¹, R², A, R³, R⁴, R⁷, R⁸, Q and D have the meanings described in Claim 1, 2, 3 or 4.

6. Non-therapeutic use according to any of Claims 1 to 4, **characterized in that** compounds of the formulae (la), (Ib) or (Ic) are used 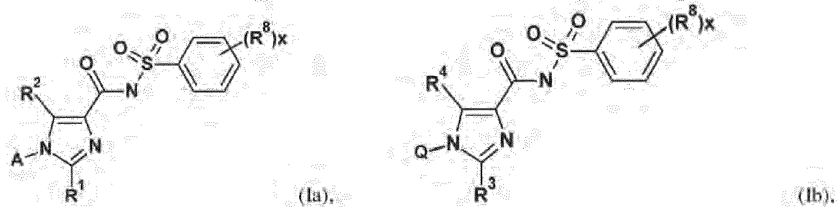 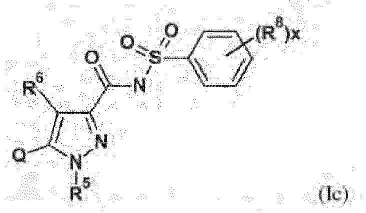 where the radicals R¹, R², R³, R⁴, R⁵, R⁶, R⁷, A, Q and R⁸ have the meanings described in Claim 1, 2, 3 or 4.

7. Non-therapeutic use according to any of Claims 1 to 6, **characterized in that** the animal pests are nematodes.

8. Non-therapeutic use according to any of Claims 1 to 7 for protecting the propagation material of plants.

9. Composition, comprising at least one compound of the formula (I) or the formulae (Ia), (Ib) or (Ic), defined according to any of Claims 1 to 6, and customary extenders and/or surfactants, in particular for controlling animal pests, preferably nematodes.

10. Method for controlling animal pests, preferably nematodes, where at least one compound of the formula (I or of the formulae (Ia), (Ib) or (Ic), defined according to any of Claims 1 to 6, or a composition according to the invention according to Claim 9 is allowed to act on the animal pests, preferably nematodes and/or their habitat, excluding the surgical, therapeutic and diagnostic treatment of the human or animal body.

11. Agrochemical formulation, comprising at least one compound of the formula (I) or of the formulae (Ia), (Ib) or (Ic), defined according to any of Claims 1 to 6, in biologically effective amounts of from 0.00000001 to 98% by weight, based on the weight of the agrochemical formulation, and also extenders and/or surfactants.

12. Agrochemical formulation according to Claim 11, **characterized in that** it additionally comprises a further agrochemically active compound.

13. Compounds of the formula (I') 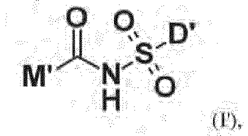 in which
M' represents a radical selected from the formulae (IIa'-IIc') : 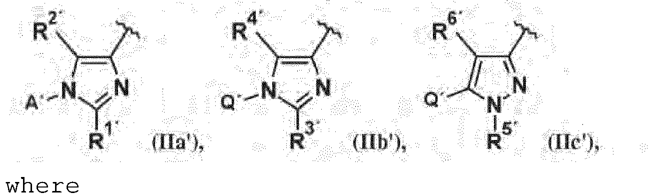
R^{1'} represents cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy- (C₁-C₆)-alkoxy, (C₁-C₆)-alkoxyimino, (C₁-C₆)-alkylthio, (C₁-C₆)-haloalkylthio, (C₁-C₆)-alkoxy- (C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl, (C₁-C₆)-alkylcarbonylamino) or benzyl which is unsubstituted or mono- or polysubstituted by identical or different substituents, possible substituents being in each case:
cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy-(C₁-C₆)-alkoxy, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl and/or (C₁-C₆)-alkylsulfonyl-(C₁-C₆)-alkyl,
R^{2'} represents hydrogen, cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, C₁-C₆) -alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl or (C₁-C₆)-alkylcarbonylamino),
R^{3'} represents cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl, (C₁-C₆)-alkylcarbonylamino or benzyl which is unsubstituted or mono- or polysubstituted by identical or different substituents, possible substituents being in each case:
cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy-(C₁-C₆)-alkoxy, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl and/or (C₁-C₆)-alkylsulfonyl-(C₁-C₆)-alkyl,
R^{4'} represents hydrogen, cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, C₁-C₆) -alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl or (C₁-C₆)-alkylcarbonylamino),
R^{5'} represents (C₃-C₈)-cycloalkyl, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-alkylthio- (C₁-C₆)-alkyl, halo-(C₁-C₆)-alkylthio-(C₁-C₆)-alkyl, (C₁-C₆)-alkylsulfinyl-(C₁-C₆)-alkyl, (C₁-C₆)-alkylsulfonyl- (C₁-C₆)-alkyl, halo-(C₁-C₆)-alkylsulfinyl-(C₁-C₆)-alkyl or halo-(C₁-C₆)-alkylsulfonyl-(C₁-C₆)-alkyl, R^{6'} represents hydrogen, cyano, halogen, nitro, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo- (C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, C₁-C₆)-alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl or (C₁-C₆)-alkylcarbonylamino),
A' represents halo-(C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, halo-(C₁-C₃)-alkoxy-(C₁-C₄)-alkyl, (C₁-C₆)-alkylthio (C₁-C₆)-alkyl, (C₁-C₆)-alkylsulfinyl-(C₁-C₆)-alkyl, halo-(C₁-C₆)-alkylthio-(C₁-C₆)-alkyl, halo-(C₁-C₆)-alkylsulfinyl-(C₁-C₆)-alkyl, halo-(C₁-C₆)-alkylsulfonyl-(C₁-C₆)-alkyl, halo- (C₃-C₆)-alkenyl or
a benzyl radical which is optionally mono- or polysubstituted by identical or different substituents, the substituent(s) being in each case independently of one another selected from:
cyano, halogen, (C₃-C₆)-cycloalkyl, (C₃-C₆)-cyclo-alkyloxy, (C₃-C₆)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, C₁-C₆)-alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, halo-(C₁-C₆)-alkylthio-(C₁-C₆)-alkyl, halo-(C₁-C₆)-alkylsulfinyl-(C₁-C₆)-alkyl and/or halo-(C₁-C₆)-alkylsulfonyl-(C₁-C₆)-alkyl,
Q' represents a phenyl or pyridyl radical which is unsubstituted or substituted by one or more radicals R^{7'}, where the substituent (s) R^{7'} are in each case independently of one another selected from:
cyano, halogen, nitro, (C₃-C₆)-cycloalkyl, (C₃-C₆)-cyclo-alkyloxy, (C₃-C₆)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, (C₁-C₆)-alkoxy-(C₁-C₆)-alkyl, (C₁-C₆)-alkoxy, (C₁-C₆)-haloalkoxy, (C₁-C₆)-cyanoalkoxy, C₁-C₆)-alkoxy-(C₁-C₆)-alkoxy, (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkoxycarbonyl, (C₁-C₆)-haloalkoxycarbonyl, (C₁-C₆)-alkylaminocarbonyl, (C₁-C₆)-alkylcarbonylamino), halo-(C₁-C₆)-alkylthio-(C₁-C₆)-alkyl, halo-(C₁-C₆)-alkylsulfinyl-(C₁-C₆)-alkyl and/or halo-(C₁-C₆)-alkylsulfonyl-(C₁-C₆)-alkyl,
and
D' represents a C₁-C₆-alkyl, phenyl, thiophenyl, isoxazolyl, phenyl-(C₁-C₂)-alkyl or benzodioxolyl radical which is unsubstituted or substituted by one or more radicals R^{8'},
where the substituent (s) R^{8'} are in each case independently of one another selected from:
cyano, halogen, nitro, acetyl, (C₃-C₈)-cycloalkyl, (C₃-C₈)-cycloalkyloxy, (C₃-C₈)-cycloalkyl-(C₃-C₈)-cycloalkyl, (C₁-C₆)-alkyl-(C₃-C₈)-cycloalkyl, halo-(C₃-C₈)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl- (C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl- (C₁-C₆)-alkoxy, (C₁-C₆)-alkoxy-(C₁-C₆)-alkoxy, (C₁-C₆)-alkylhydroxyimino, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl, (C₁-C₆)-haloalkylsulfinyl, (C₁-C₆)-alkoxy- (C₁-C₆)-alkylsulfinyl, (C₁-C₆)-alkylsulfinyl- (C₁-C₆)-alkyl, (C₁-C₆)-alkylsulfonyl, (C₁-C₆)-haloalkylsulfonyl, (C₁-C₆)-alkoxy-(C₁-C₆)-alkylsulfonyl, (C₁-C₆)-alkylsulfonyl- (C₁-C₆)-alkyl, (C₁-C₆)-sulfonyloxy, (C₁-C₆)-alkylcarbonyl, (C₁-C₆)-alkylthiocarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, (C₁-C₆)-alkylaminothiocarbonyl, di-(C₁-C₆)-alkylaminocarbonyl, di-(C₁-C₆)-alkylaminothiocarbonyl, (C₂-C₆)-alkenylaminocarbonyl, di-(C₂-C₆)-alkenylamino-carbonyl, (C₃-C₈)-cycloalkylaminocarbonyl, (C₁-C₆)-alkylsulfonylamino, (C₁-C₆)-alkylamino, di-(C₁-C₆)-alkylamino, aminosulfonyl, (C₁-C₆)-alkylaminosulfonyl, di-(C₁-C₆)-alkylaminosulfonyl, (C₁-C₆)-alkylsulfoximino, aminothiocarbonyl, (C₁-C₆)-alkylaminothiocarbonyl, di-(C₁-C₆)-alkylaminothiocarbonyl, (C₃-C₈)-cycloalkylamino, (C₁-C₆)-alkylcarbonylamino, (1-pyrazolyl)(C₁-C₃)-alkyl and/or (C₁-C₃)-alkoxypyrimidinyloxy.

14. Compounds of the formula (I') according to Claim 13, **characterized in that**
R^{1'} represents cyano, halogen, cyclopropyl, (C₁-C₄)-alkyl, halocyclopropyl, halo-(C₁-C₃)-alkylcyclopropyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylthio-(C₁-C₄)-alkyl, (C₁-C₄)-alkylsulfinyl- (C₁-C₄)-alkyl, (C₁-C₄)-alkylsulfonyl-(C₁-C₄)-alkyl, or benzyl which is unsubstituted or mono- or polysubstituted by identical or different substituents, possible substituents being in each case: cyano, halogen, (C₁-C₄)-haloalkyl, (C₁-C₄)-alkoxy, (C₁-C₄)-haloalkoxy, (C₁-C₆)-alkylthio and/or (C₁-C₆)-haloalkylthio,
R^{2'} represents hydrogen, cyano, halogen, (C₁-C₄)-haloalkyl, (C₁-C₄)-alkoxy, (C₁-C₄)-haloalkoxy, (C₁-C₆)-alkylthio or (C₁-C₆)-haloalkylthio,
R^{3'} represents cyano, halogen, cyclopropyl, (C₁-C₄)-alkyl, halocyclopropyl, halo- (C₁-C₃)-alkylcyclopropyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylthio-(C₁-C₄)-alkyl, (C₁-C₄)-alkylsulfinyl-(C₁-C₄)-alkyl or (C₁-C₄)-alkylsulfonyl- (C₁-C₄)-alkyl,
R^{4'} represents hydrogen, cyano, halogen, (C₁-C₄)-haloalkyl, (C₁-C₄)-alkoxy, (C₁-C₄)-alkyl, (C₃-C₄)-cycloalkyl, (C₁-C₄)-haloalkoxy, (C₁-C₆)-alkylthio or (C₁-C₆)-halo-alkylthio,
R^{5'} represents cyclopropyl, (C₁-C₄)-alkyl, halocyclopropyl, halo-(C₁-C₃)-alkylcyclopropyl or (C₁-C₄)-haloalkyl,
R^{6'} represents hydrogen, cyano, halogen, (C₁-C₄)-haloalkyl, (C₁-C₄)-alkoxy, (C₁-C₄)-haloalkoxy, 4 (C₁-C₆)-alkylthio or (C₁-C₆)-haloalkylthio,
A' represents halo-(C₁-C₄)-alkyl-(C₃-C₄)-cycloalkyl, halo-(C₃-C₆)-cycloalkyl, (C₁-C₆)-alkyl, (C₁-C₅)-haloalkyl, (C₁-C₆)-cyanoalkyl, (C₁-C₄)-alkoxy-(C₁-C₄)-alkyl, halo-(C₁-C₃)-alkoxy- (C₁-C₄)-alkyl, (C₁-C₄)-alkylthio- (C₁-C₄)-alkyl, (C₁-C₄)-alkylsulfinyl-(C₁-C₄)-alkyl, halo- (C₃-C₄)-alkenyl or
a benzyl radical which is optionally mono- or polysubstituted by identical or different substituents, where the substituent(s) are in each case independently of one another selected from:
cyano, halogen, (C₃-C₆)-cycloalkyl, (C₃-C₆)-cyclo-alkyloxy, halo- (C₃-C₈)-cycloalkyl, (C₁-C₆)-alkyl, (C₁-C₆)-haloalkyl, (C₁-C₆)-cyanoalkyl, (C₁-C₆)-alkoxy-(C₁-C₆)-alkyl, (C₁-C₆)-alkoxy, (C₁-C₆)-haloalkoxy, (C₁-C₆)-cyano-alkoxy, C₁-C₆)-alkoxy-(C₁-C₆)-alkoxy, (C₁-C₆)-alkylthio and/or (C₁-C₆)-haloalkylthio,
Q' represents a phenyl or pyridyl radical which is unsubstituted or substituted by one or more radicals R^{7'}, where the substituent (s) R^{7'} are in each case independently of one another selected from:
cyano, halogen, (C₃-C₆)-cycloalkyl, halo- (C₃-C₈)-cycloalkyl, (C₁-C₆)-alkyl, (C₁-C₆)-haloalkyl, (C₁-C₆)-alkoxy-(C₁-C₆)-alkyl, (C₁-C₆)-alkoxy and/or (C₁-C₆)-haloalkoxy, and
D' represents a C₁-C₆-alkyl, phenyl, thiophenyl, isoxazolyl, benzyl or benzodioxolyl radical which is unsubstituted or substituted by one or more radicals R^{8'}, where the substituent (s) R^{8'} are in each case independently of one another selected from:
cyano, halogen, acetyl, (C₃-C₆)-cycloalkyl, (C₃-C₆)-cyclo-alkyloxy, (C₃-C₆)-cycloalkyl-(C₃-C₈)-cycloalkyl, (C₁-C₆)-alkyl-(C₃-C₆)-cycloalkyl, halo-(C₃-C₆)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₂-C₆)-alkenyl, (C₂-C₆)-haloalkenyl, (C₂-C₆)-cyanoalkenyl, (C₂-C₆)-alkynyl, (C₂-C₆)-haloalkynyl, (C₂-C₆)-cyanoalkynyl, (C₁-C₆)-alkoxy, (C₁-C₆)-haloalkoxy, (C₁-C₆)-cyanoalkoxy, (C₁-C₆)-alkoxycarbonyl-(C₁-C₆)-alkoxy, (C₁-C₆)-alkoxy-(C₁-C₆)-alkoxy, (C₁-C₆)-alkoxyimino, (C₁-C₆)-alkyl-(C₁-C₆)-alkoxyimino, (C₁-C₆)-haloalkyl-(C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl, (C₁-C₆)-haloalkylsulfinyl, (C₁-C₆)-alkoxy-(C₁-C₆)-alkylsulfinyl, (C₁-C₆)-alkylsulfinyl-(C₁-C₆)-alkyl, (C₁-C₆)-alkylsulfonyl, (C₁-C₆)-haloalkylsulfonyl, (C₁-C₆)-alkoxy-(C₁-C₆)-alkylsulfonyl, (C₁-C₆)-aklylsulfonyl-(C₁-C₆)-alkyl, (C₁-C₆)-alkylsulfonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, di-(C₂-C₆)-alkenylaminocarbonyl, (C₃-C₈)-cycloalkylamino-carbonyl, (C₁-C₆)-alkylsulfonylamino, (C₁-C₆)-alkylamino, di-(C₁-C₆)-alkylamino, (C₃-C₈)-cycloalkylamino, (C₁-C₆)-alkylcarbonylamino, (1-pyrazolyl)(C₁-C₃)-alkyl and/or (C₁-C₃)-alkoxypyrimidinyloxy.

15. Compounds of the formula (I') according to Claim 13, **characterized in that**
R^{1'} represents halogen, cyclopropyl, (C₁-C₄)-alkyl, (C₁-C₄)-alkylthio, halocyclopropyl, (C₁-C₄)-haloalkyl or benzyl which is unsubstituted or mono- or polysubstituted by identical or different substituents, possible substituents being in each case: cyano, halogen, (C₁-C₄)-haloalkyl and/or (C₁-C₄)-alkoxy,
R^{2'} represents hydrogen, halogen, cyano, (C₁-C₄)-alkyl, (C₁-C₄)-haloalkyl,
R^{3'} represents halogen, cyclopropyl, (C₁-C₄)-alkyl, halocyclopropyl or (C₁-C₄)-haloalkyl,
R^{4'} represents hydrogen, halogen, cyano, (C₁-C₄)-alkyl, cyclopropyl or (C₁-C₄)-haloalkyl,
R^{5'} represents cyclopropyl, (C₁-C₄)-alkyl or halo-(C₁-C₄)-alkyl,
R^{6'} represents hydrogen, halogen, cyano, (C₁-C₄)-alkyl or (C₁-C₄)-haloalkyl,
A' represents halo- (C₁-C₄)-alkyl- (C₃-C₄)-cycloalkyl, halo- (C₃-C₆)-cycloalkyl, (C₁-C₆)-alkyl, (C₁-C₅)-haloalkyl, (C₁-C₆)-cyanoalkyl, (C₁-C₄)-alkoxy- (C₁-C₄)-alkyl, halo-(C₁-C₃)-alkoxy- (C₁-C₄)-alkyl, (C₁-C₄)-alkylthio- (C₁-C₄)-alkyl, (C₁-C₄)-alkylsulfinyl-(C₁-C₄)-alkyl, halo- (C₃-C₄)-alkenyl or
a benzyl radical which is optionally mono- or polysubstituted by identical or different substituents, where the substituent(s) are in each case independently of one another selected from:
cyano, halogen, (C₃-C₆)-cycloalkyl, (C₃-C₆)-cyclo-alkyloxy, halo- (C₃-C₈)-cycloalkyl, (C₁-C₆)-alkyl, (C₁-C₆)-haloalkyl, (C₁-C₆)-alkoxy-(C₁-C₆)-alkyl, (C₁-C₆)-alkoxy, (C₁-C₆)-haloalkoxy, (C₁-C₆)-alkylthio and/or (C₁-C₆)-haloalkylthio,
Q' represents a phenyl or pyridyl radical which is unsubstituted or substituted by one or more radicals R^{7'}, where the substituent (s) R^{7'} are in each case independently of one another selected from:
cyano, halogen, (C₃-C₆)-cycloalkyl, halo-(C₃-C₆)-cycloalkyl, (C₁-C₆)-alkyl, (C₁-C₆)-haloalkyl, (C₁-C₆)-alkoxy-(C₁-C₆)-alkyl, (C₁-C₆)-alkoxy and/or (C₁-C₆)-haloalkoxy, and
D' represents a C₁-C₆-alkyl, phenyl, thiophenyl, isoxazolyl, benzyl or benzodioxolyl radical which is unsubstituted or substituted by one or more radicals R^{8'}, where the substituent (s) R^{8'} are in each case independently of one another selected from:
cyano, halogen, acetyl, (C₃-C₆)-cycloalkyl, (C₃-C₆)-cycloalkyloxy, (C₃-C₆)-cycloalkyl-(C₃-C₈)-cycloalkyl, (C₁-C₆)-alkyl-(C₃-C₆)-cycloalkyl, halo-(C₃-C₆)-cycloalkyl, (C₁-C₆)-alkyl, (C₁-C₆)-haloalkyl, (C₁-C₆)-cyanoalkyl, (C₁-C₆)-hydroxyalkyl, hydroxycarbonyl-(C₁-C₆)-alkoxy, (C₁-C₆)-alkoxycarbonyl-(C₁-C₆)-alkyl, (C₁-C₆)-alkoxy-(C₁-C₆)-alkyl, (C₁-C₆)-alkoxy, (C₁-C₆)-haloalkoxy, (C₁-C₆)-cyanoalkoxy, (C₁-C₆)-alkoxy-(C₁-C₆)-alkoxy, (C₁-C₆)-alkoxyimino, (C₁-C₆)-alkyl- (C₁-C₆)-alkoxyimino, (C₁-C₆)-haloalkyl- (C₁-C₆)-alkoxyimino, (C₁-C₆)-alkylthio, (C₁-C₆)-haloalkylthio, (C₁-C₆)-alkoxy-(C₁-C₆)-alkylthio, (C₁-C₆)-alkylthio-(C₁-C₆)-alkyl, (C₁-C₆)-alkylsulfinyl, (C₁-C₆)-haloalkylsulfinyl, (C₁-C₆)-alkoxy-(C₁-C₆)-alkylsulfinyl, (C₁-C₆)-alkylsulfinyl-(C₁-C₆)-alkyl, (C₁-C₆)-alkylsulfonyl, (C₁-C₆)-haloalkylsulfonyl, (C₁-C₆)-alkoxy-(C₁-C₆)-alkylsulfonyl, (C₁-C₆)-alkylsulfonyl-(C₁-C₆)-alkyl, (C₁-C₆)-alkylsulfonyloxy, (C₁-C₆)-alkylcarbonyl, (C₁-C₆)-haloalkylcarbonyl, (C₁-C₆)-alkylcarbonyloxy, (C₁-C₆)-alkoxycarbonyl, (C₁-C₆)-haloalkoxycarbonyl, aminocarbonyl, (C₁-C₆)-alkylaminocarbonyl, di-(C₁-C₆)-alkylaminocarbonyl, (C₂-C₆)-alkenylaminocarbonyl, (C₃-C₈)-cycloalkylaminocarbonyl, (C₁-C₆)-alkylsulfonylamino, (C₁-C₆)-alkylamino, di-(C₁-C₆)-alkylamino, (C₃-C₆)-cycloalkylamino, (C₁-C₆)-alkylcarbonylamino, (1-pyrazolyl)(C₁-C₃)-alkyl and/or (C₁-C₃)-alkoxy-pyrimidinyloxy.

16. Compounds of the formula (I') according to Claim 13, **characterized in that**
R^{1'} represents chlorine, bromine, methyl, benzyl, 2-chlorobenzyl, 3-chlorobenzyl, 4-chlorobenzyl, trifluoromethyl, methylthio or isopropylthio,
R^{2'} represents hydrogen, cyano, chlorine, bromine or iodine,
R^{3'} represents chlorine, methyl, isopropyl, ethyl or bromine,
R^{4'} represents hydrogen, chlorine, bromine, iodine, fluorine, difluoromethyl, isopropyl or cyclopropyl,
R^{5'} represents methyl or 2,2,2-trifluoroethyl,
R^{6'} represents hydrogen or chlorine,
A' represents a radical selected from the radicals of the formulae (III1-III19) : 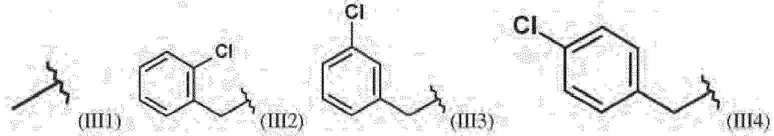 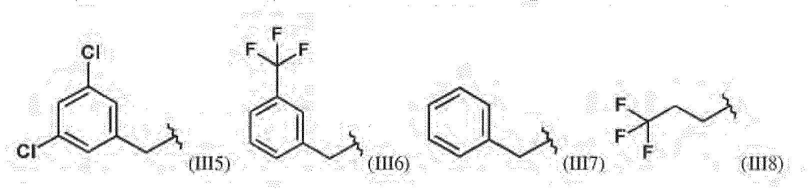 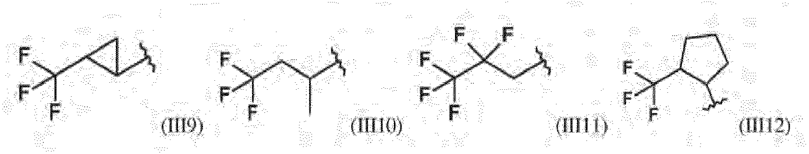 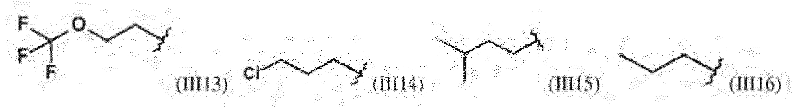 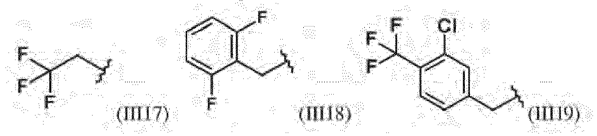
Q' represents a radical selected from the radicals of the formulae (IV1-IV40): 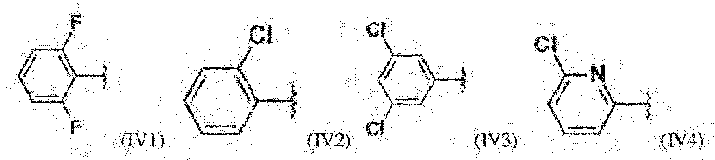 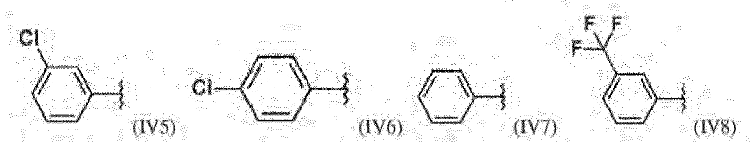 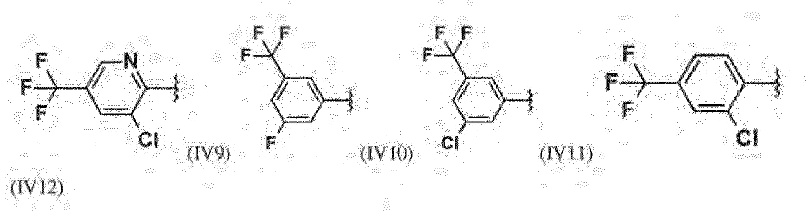 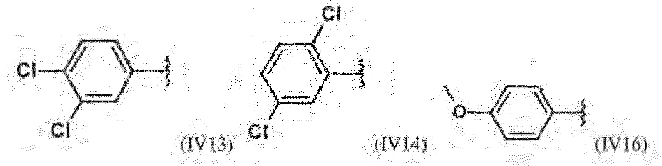 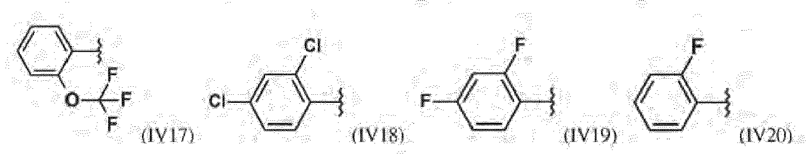 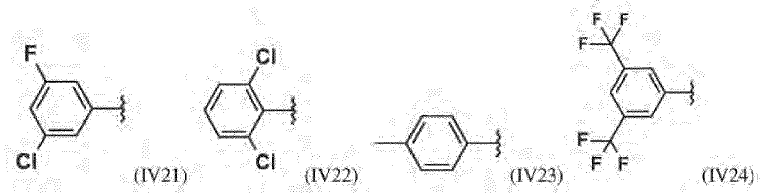 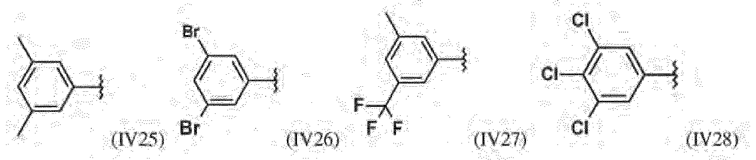 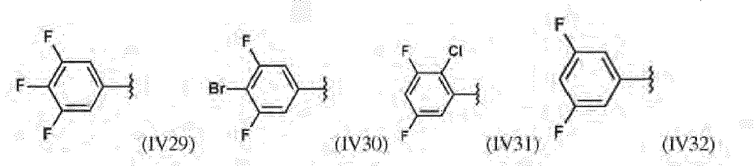 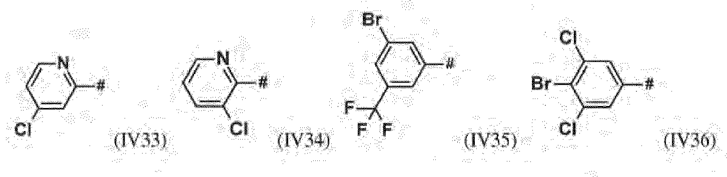 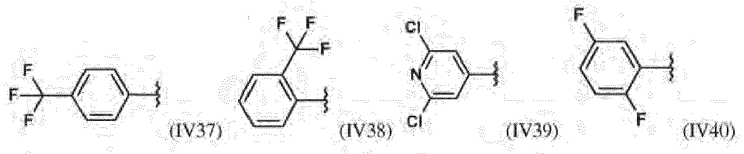 and
D' represents a radical selected from the radicals of the formulae (V1-V61) :

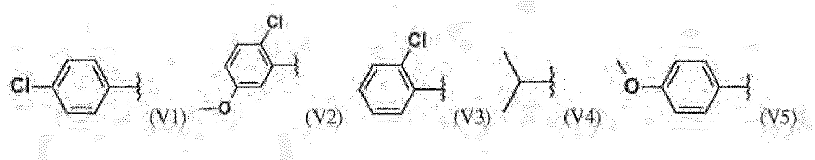

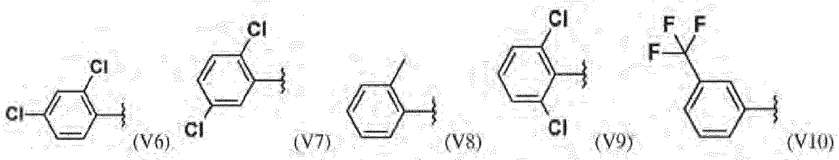

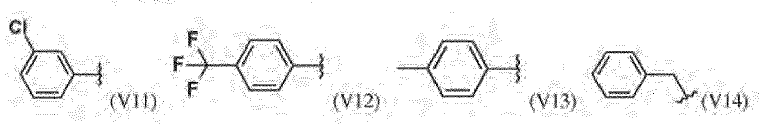

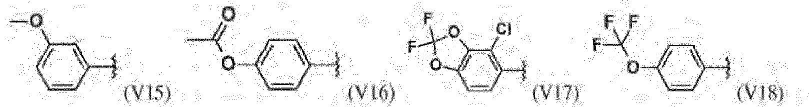

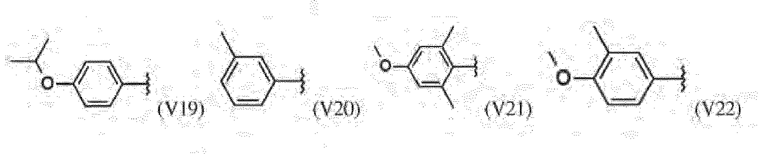

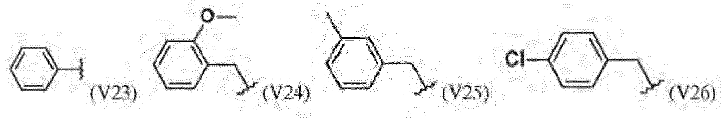

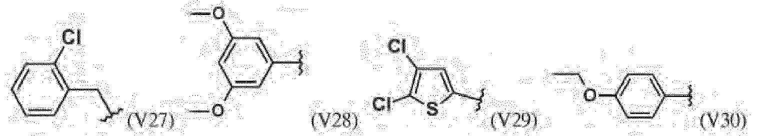

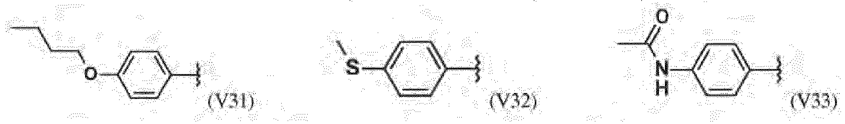

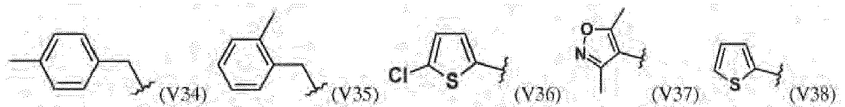

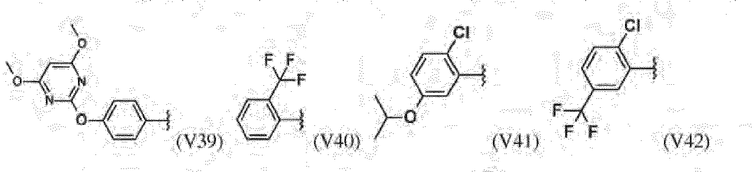

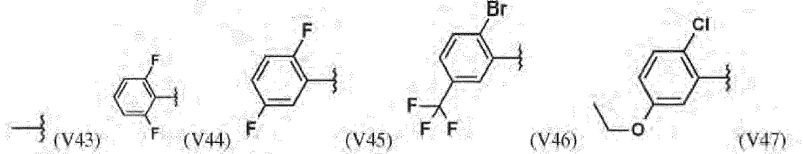

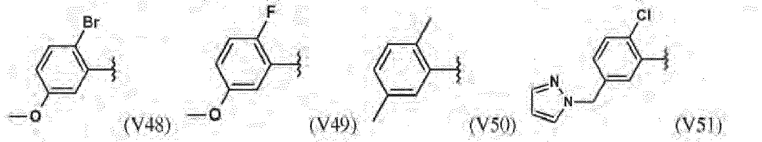

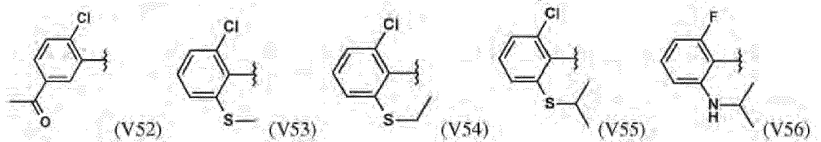

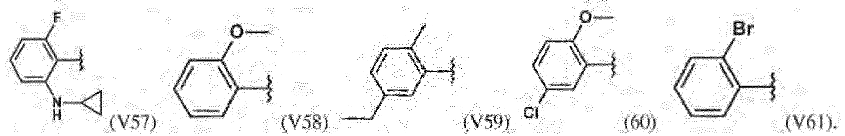

17. Compounds of the formula (I') according to any of Claims 13 to 16, **characterized in that**
in the formulae (I'), M' represents a radical selected from the formulae IIa' or IIb', where the radicals R^{1'}, R^{2'}, A', R^{3'}, R^{4'}, R^{7'}, R^{8'}, Q' and D' have the meanings described in Claim 13, 14, 15 or 16.

18. Compounds of the formulae (Ia'), (Ib') or (Ic') according to any of Claims 13 to 16 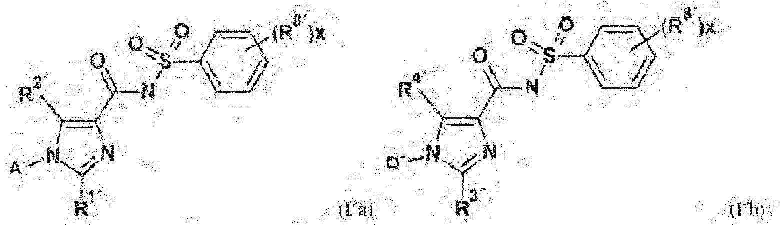 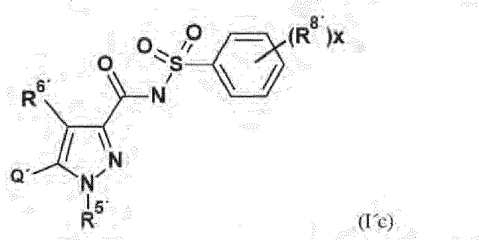 where the radicals R^{1'}, R^{2'}, R^{3'}, R^{4'}, R^{5'}, R^{6'}, R^{7'}, A', Q' and R^{8'} have the meanings described in Claim 13, 14, 15 or 16.

19. Intermediates of the formulae (VIa), (Xa) and (IXa) 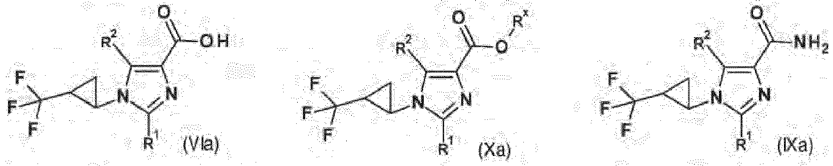 in which R¹ and R² have the meanings mentioned in Claim 2, 3 or 4 and R^{x} represents (C₁-C₆)-alkyl or (C₃-C₈)-cycloalkyl.

## Revendications

1. Utilisation non thérapeutique d'un composé de formule (I) 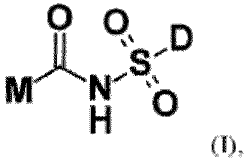 dans laquelle
M représente un radical choisi parmi les formules (IIa à IIc) : 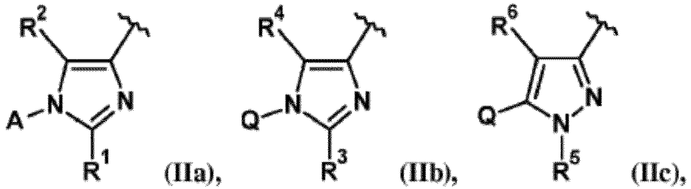 dans lesquelles
R¹ est hydrogène, cyano, halogène, nitro, (C₃-C₈)cycloalkyle, (C₃-C₈)cycloalkyloxy, (C₃-C₈)cycloalkyl-(C₃-C₈)cycloalkyle, (C₁-C₆)alkyl-(C₃-C₈)cycloalkyle, halogéno(C₃-C₈)cycloalkyle, (Ci-C₆)alkyle, (C₁-C₆)halogénoalkyle, (Ci-C₆)cyanoalkyle, (C₁-C₆)hydroxyalkyle, (C₁-C₆)alcoxy-(C₁-C₆)alkyle, (C₁-C₆)alcoxy, (C₁-C₆)halogénoalcoxy, (C₁-C₆)cyanoalcoxy, (C₁-C₆)alcoxy- (C₁-C₆)alcoxy, (Ci-C₆)alcoxyimino, (C₁-C₆)alkylthio, (Ci-C₆)halogénoalkylthio, (C₁-C₆)alcoxy- (Ci-C₆)alkylthio, (C₁-C₆)alkylthio- (C₁-C₆)alkyle, (Ci-C₆)alkylsulfinyl-(C₁-C₆)alkyle, (Ci-C₆)alkylsulfonyl-(C₁-C₆)alkyle, (Ci-C₆)alcoxycarbonyle, (C₁-C₆)halogénoalcoxycarbonyle, (C₁-C₆)alkylaminocarbonyle, (Ci-C₆)alkylcarbonylamino ou benzyle non substitué, monosubstitué ou polysubstitué, de manière identique ou différente, les substituants en question étant à chaque fois :
cyano, halogène, nitro, (C₃-C₈)cycloalkyle, (C₃-C₈)cycloalkyloxy, (C₃-C₈)cycloalkyl- (C₃-C₈)cycloalkyle, (C₁-C₆)alkyl-(C₃-C₈)cycloalkyle, halogéno (C₃-C₈)cycloalkyle, (C₁-C₆)alkyle, (Ci-C₆)halogénoalkyle, (C₁-C₆)cyanoalkyle, (Ci-C₆)alcoxy- (C₁-C₆)alkyle, (C₁-C₆)alcoxy, (Ci-C₆)halogénoalcoxy, (C₁-C₆)cyanoalcoxy, (Ci-C₆)alcoxy- (C₁-C₆)alcoxy, (C₁-C₆)alkylthio, (Ci-C₆)halogénoalkylthio, (C₁-C₆)alcoxy- (Ci-C₆)alkylthio, (C₁-C₆)alkylthio- (C₁-C₆)alkyle, (Ci-C₆)alkylsulfinyl-(C₁-C₆)alkyle et/ou (Ci-C₆)alkylsulfonyl-(C₁-C₆)alkyle,
R² est hydrogène, cyano, halogène, nitro, (C₃-C₈)cycloalkyle, (C₃-C₈)cycloalkyloxy, (C₃-C₈)cycloalkyl-(C₃-C₈)cycloalkyle, (C₁-C₆)alkyl-(C₃-C₈)cycloalkyle, halogéno(C₃-C₈)cycloalkyle, (Ci-C₆)alkyle, (C₁-C₆)halogénoalkyle, (C₁-C₆)cyanoalkyle, (C₁-C₆)hydroxyalkyle, (C₁-C₆)alcoxy-(C₁-C₆)alkyle, (C₁-C₆)alcoxy, (C₁-C₆)halogénoalcoxy, (C₁-C₆)cyanoalcoxy, (C₁-C₆)alcoxy-(C₁-C₆)alcoxy, (Ci-C₆)alcoxyimino, (C₁-C₆)alkylthio, (C₁-C₆)halogénoalkylthio, (C₁-C₆)alcoxy- (Ci-C₆)alkylthio, (C₁-C₆)alkylthio-(C₁-C₆)alkyle, (Ci-C₆)alkylsulfinyl-(C₁-C₆)alkyle, (C₁-C₆)alkylsulfonyl-(C₁-C₆)alkyle, (C₁-C₆)alcoxycarbonyle, (C₁-C₆)halogénoalcoxycarbonyle, (C₁-C₆)alkylaminocarbonyle ou (C₁-C₆)alkylcarbonylamino,
R³ est hydrogène, cyano, halogène, nitro, (C₃-C₈)cycloalkyle, (C₃-C₈)cycloalkyloxy, (C₃-C₈)cycloalkyl-(C₃-C₈)cycloalkyle, (C₁-C₆)alkyl-(C₃-C₈)cycloalkyle, halogéno(C₃-C₈)cycloalkyle, (C₁-C₆) alkyle, (C₁-C₆) halogénoalkyle, (C₁-C₆) cyanoalkyle, (C₁-C₆) hydroxyalkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alcoxyimino, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle, (C₁-C₆) alkylsulfonyl- (C₁-C₆) alkyle, (C₁-C₆)alcoxycarbonyle, (C₁-C₆) halogénoalcoxycarbonyle, (C₁-C₆) alkylaminocarbonyle, (C₁-C₆)alkylcarbonylamino ou benzyle non substitué, monosubstitué ou polysubstitué, de manière identique ou différente, les substituants en question étant à chaque fois :
cyano, halogène, nitro, (C₃-C₈) cycloalkyle, (C₃-C₈)cycloalkyloxy, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆) halogénoalkyle, (C₁-C₆) cyanoalkyle, (C₁-C₆) alcoxy- (C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle et/ou (C₁-C₆) alkylsulfonyl- (C₁-C₆) alkyle,
R⁴ est hydrogène, cyano, halogène, nitro, (C₃-C₈) cycloalkyle, (C₃-C₈) cycloalkyloxy, (C₃-C₈) cycloalkyl- (C₃-C₈) cycloalkyle, (C₁-C₆) alkyl- (C₃-C₈)cycloalkyle, halogéno (C₃-C₈) cycloalkyle, (C₁-C₆) alkyle, (C₁-C₆) halogénoalkyle, (C₁-C₆) cyanoalkyle, (C₁-C₆) hydroxyalkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alcoxyimino, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle, (C₁-C₆) alkylsulfonyl- (C₁-C₆) alkyle, (C₁-C₆)alcoxycarbonyle, (C₁-C₆) halogénoalcoxycarbonyle, (C₁-C₆) alkylaminocarbonyle ou (C₁-C₆)alkylcarbonylamino,
R⁵ est hydrogène, (C₃-C₈) cycloalkyle, (C₃-C₈) cycloalkyl- (C₃-C₈) cycloalkyle, (C₁-C₆) alkyl- (C₃-C₈)cycloalkyle, halogéno (C₃-C₈) cycloalkyle, (C₁-C₆) alkyle, (C₁-C₆) halogénoalkyle, (C₁-C₆) cyanoalkyle, (C₁-C₆) alcoxy- (C₁-C₆) alkyle, (C₁-C₆) alkylthio- (C₁-C₆) alkyle, halogène (C₁-C₆) alkylthio- (C₁-C₆) alkyle, (C₁-C₆) alkylsulfinyl-(C₁-C₆) alkyle, (C₁-C₆) alkylsulfonyl- (C₁-C₆) alkyle, halogéno (C₁-C₆) alkylsulfinyl- (C₁-C₆) alkyle ou halogéno (C₁-C₆) alkylsulfonyl- (C₁-C₆) alkyl,
R⁶ hydrogène, cyano, halogène, nitro, (C₃-C₈) cycloalkyle, (C₃-C₈) cycloalkyloxy, (C₃-C₈) cycloalkyl- (C₃-C₈) cycloalkyle, (C₁-C₆) alkyl- (C₃-C₈)cycloalkyle, halogéno (C₃-C₈) cycloalkyle, (C₁-C₆) alkyle, (C₁-C₆) halogénoalkyle, (C₁-C₆) cyanoalkyle, (C₁-C₆) hydroxyalkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alcoxyimino, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle, (C₁-C₆) alkylsulfonyl- (C₁-C₆) alkyle, (C₁-C₆)alcoxycarbonyle, (C₁-C₆) halogénoalcoxycarbonyle, (C₁-C₆) alkylaminocarbonyle ou (C₁-C₆)alkylcarbonylamino,
A est halogène (C₁-C₆) alkyl- (C₃-C₈) cycloalkyle, halogène (C₃-C₈) cycloalkyle, (C₁-C₆) alkyle, (C₁-C₆) halogénoalkyle, (C₁-C₆) cyanoalkyle, (C₁-C₆) alcoxy- (C₁-C₆) alkyle, halogéno (C₁-C₃) alcoxy- (C₁-C₄) alkyle, (C₁-C₆) alkylthio- (C₁-C₆) alkyle, (C₁-C₆) alkylsulfinyl- (C₁-C₆) alkyle, halogène (C₁-C₆) alkylthio- (C₁-C₆) alkyle, halogène (C₁-C₆) alkylsulfinyl- (C₁-C₆) alkyle, halogène (C₁-C₆) alkylsulfonyl- (C₁-C₆) alkyle, halogène (C₃-C₆) alcényle ou
un radical benzyle éventuellement monosubstitué ou polysubstitué, de manière identique ou différente, le ou les substituants étant à chaque fois indépendamment choisis parmi :
cyano, halogène, (C₃-C₆) cycloalkyle, (C₃-C₆)cycloalkyloxy, (C₃-C₆) cycloalkyl- (C₃-C₈) cycloalkyle, (C₁-C₆) alkyl- (C₃-C₈) cycloalkyle, halogène (C₃-C₈) cycloalkyle, (C₁-C₆) alkyle, (C₁-C₆) halogénoalkyle, (C₁-C₆) cyanoalkyle, (C₁-C₆) alcoxy- (C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alcoxyimino, (C₁-C₆) alkylthio, (C₁-C₆) halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle, (C₁-C₅) alkylsulfonyl- (C₁-C₆) alkyle, halogène (C₁-C₆) alkylthio- (C₁-C₆) alkyle, halogène (C₁-C₆) alkylsulfinyl- (C₁-C₆) alkyle et/ou halogéno (C₁-C₆) alkylsulfonyl- (C₁-C₆) alkyle,
Q est un radical phényle ou pyridinyle non substitué ou substitué par un ou plusieurs radicaux R⁷,
le ou les substituants R⁷ étant à chaque fois indépendamment choisis parmi :
cyano, halogène, nitro, (C₃-C₆) cycloalkyle, (C₃-C₆)cycloalkyloxy, (C₃-C₆) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆) halogénoalkyle, (C₁-C₆) cyanoalkyle, (C₁-C₆) hydroxyalkyle, (C₁-C₆) alcoxy- (C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆) alcoxyimino, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyl- (C₁-C₆) alkyle, (C₁-C₆) alkylsulfonyl- (C₁-C₆) alkyle, (C₁-C₆)alcoxycarbonyle, (C₁-C₆) halogénoalcoxycarbonyle, (C₁-C₆)alkylaminocarbonyle, (C₁-C₆)alkylcarbonylamino, halogène (C₁-C₆)alkylthio- (C₁-C₆)alkyle, halogéno (C₁-C₆)alkylsulfinyl-(C₁-C₆)alkyle et/ou halogéno (C₁-C₆)alkylsulfonyl-(C₁-C₆)alkyle,
et
D est un radical C₁-C₆-alkyle, phényle, thiophényle, isoxazolyle, phényl-(C₁-C₂)alkyle ou benzodioxolyle non substitué ou substitué par un ou plusieurs radicaux R⁸,
le ou les substituants R⁸ étant à chaque fois indépendamment choisis parmi :
cyano, halogène, nitro, acétyle, (C₃-C₈)cycloalkyle, (C₃-C₈) cycloalkyloxy, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆)alkyle, (C₁-C₆)halogénoalkyle, (C₁-C₆) cyanoalkyle, (C₁-C₆)hydroxyalkyle, hydroxycarbonyl-(C₁-C₆)-alcoxy, (C₁-C₆)alcoxycarbonyl- (C₁-C₆)alkyle, (C₁-C₆)alcoxy-(C₁-C₆) alkyle, (C₂-C₆)alcényle, (C₂-C₆)halogénoalcényle, (C₂-C₆)cyanoalcényle, (C₂-C₆) alcynyle, (C₂-C₆)halogénoalcynyle, (C₂-C₆)cyanoalcynyle, (C₁-C₆)alcoxy, (C₁-C₆)halogénoalcoxy, (C₁-C₆) cyanoalcoxy, (C₁-C₆)alcoxycarbonyl- (C₁-C₆)alcoxy, (C₁-C₆)alcoxy- (C₁-C₆)alcoxy, (C₁-C₆)alkylhydroxyimino, (C₁-C₆)alcoxyimino, (C₁-C₆)alkyl- (C₁-C₆)alcoxyimino, (C₁-C₆)halogénoalkyl- (C₁-C₆) alcoxyimino, (C₁-C₆)alkylthio, (C₁-C₆)halogénoalkylthio, (C₁-C₆)alcoxy- (C₁-C₆)alkylthio, (C₁-C₆)alkylthio- (C₁-C₆)alkyle, (C₁-C₆) alkylsulfinyle, (C₁-C₆)halogénoalkylsulfinyle, (C₁-C₆)alcoxy- (C₁-C₆)alkylsulfinyle, (C₁-C₆) alkylsulfinyl-(C₁-C₆) alkyle, (C₁-C₆)alkylsulfonyle, (C₁-C₆)halogénoalkylsulfonyle, (C₁-C₆)alcoxy- (C₁-C₆)alkylsulfonyle, (C₁-C₆) alkylsulfonyl- (C₁-C₆) alkyle, (C₁-C₆)alkylsulfonyloxy, (C₁-C₆) alkylcarbonyle, (C₁-C₆)alkylthiocarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, (C₁-C₆)alkylaminothiocarbonyle, di-(C₁-C₆)alkylaminocarbonyle, di-(C₁-C₆)alkyl-aminothiocarbonyle, (C₂-C₆)alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆)alkylamino, di-(C₁-C₆)alkylamino, aminosulfonyle, (Ci-C₆)alkylaminosulfonyle, di-(C₁-C₆)alkyl-aminosulfonyle, (C₁-C₆)alkylsulfoximino, aminothiocarbonyle, (C₁-C₆)alkylaminothiocarbonyle, di-(C₁-C₆)alkyl-aminothiocarbonyle, (C₃-C₈)cycloalkylamino, (C₁-C₆)alkylcarbonylamino, (1-pyrazolyl) (C₁-C₃)alkyle et/ou (C₁-C₃)alcoxypyrimidinyloxy,
pour la lutte contre des parasites animaux.

2. Utilisation non thérapeutique selon la revendication 1, **caractérisée en ce que**
R¹ est hydrogène, cyano, halogène, cyclopropyle, (C₁-C₄)alkyle, halogénocyclopropyle, halogéno(C₁-C₃)alkylcyclopropyle, (C₁-C₄)halogénoalkyle, (C₁-C₄) alcoxy, (C₁-C₄) halogénoalcoxy, (C₁-C₄) alkylthio, (C₁-C₄)halogénoalkylthio, (C₁-C₄) alkylthio- (C₁-C₄) alkyle, (C₁-C₄)alkylsulfinyl- (C₁-C₄)alkyle, (C₁-C₄)alkylsulfonyl-(C₁-C₄)alkyle, ou benzyle non substitué, monosubstitué ou polysubstitué, de manière identique ou différente, les substituants en question étant à chaque fois : cyano, halogène, (C₁-C₄)halogénoalkyle, (C₁-C₄)alcoxy, (C₁-C₄)halogénoalcoxy, (C₁-C₆)alkylthio et/ou (C₁-C₆)halogénoalkylthio,
R² est hydrogène, cyano, halogène, (C₁-C₄) halogénoalkyle, (C₁-C₄)alcoxy, (C₁-C₄)halogénoalcoxy, (C₁-C₆)alkylthio ou (C₁-C₆)halogénoalkylthio,
R³ est hydrogène, cyano, halogène, cyclopropyle, (C₁-C₄)alkyle, halogénocyclopropyle, halogéno(C₁-C₃)alkylcyclopropyle, (C₁-C₄)halogénoalkyle, (C₁-C₄) alcoxy, (C₁-C₄)halogénoalcoxy, (C₁-C₄) alkylthio, (C₁-C₄)halogénoalkylthio, (C₁-C₄) alkylthio- (C₁-C₄) alkyle, (C₁-C₄)alkylsulfinyl- (C₁-C₄)alkyle ou (C₁-C₄)alkylsulfonyl-(C₁-C₄)alkyle,
R⁴ est hydrogène, cyano, halogène, (C₁-C₄)halogénoalkyle, (C₁-C₄) alcoxy, (C₁-C₄) alkyle, (C₃-C₄)cycloalkyle, (C₁-C₄)halogénoalcoxy, (C₁-C₆)alkylthio ou (C₁-C₆)halogénoalkylthio,
R⁵ est hydrogène, cyclopropyle, (C₁-C₄)alkyle, halogénocyclopropyle, halogéno(C₁-C₃)alkylcyclopropyle ou (C₁-C₄)halogénoalkyle,
R⁶ est hydrogène, cyano, halogène, (C₁-C₄)halogénoalkyle, (C₁-C₄)alcoxy, (C₁-C₄)halogénoalcoxy, (C₁-C₆)alkylthio ou (C₁-C₆)halogénoalkylthio,
A est halogène (C₁-C₄)alkyl- (C₃-C₄)cycloalkyle, halogène (C₃-C₆) cycloalkyle, (C₁-C₆)alkyle, (C₁-C₅)halogénoalkyle, (C₁-C₆) cyanoalkyle, (C₁-C₄)alcoxy- (C₁-C₄)alkyle, halogéno (C₁-C₃)alcoxy- (C₁-C₄)alkyle, (C₁-C₄)alkylthio- (C₁-C₄)alkyle, (C₁-C₄)alkylsulfinyl- (C₁-C₄) alkyle, halogène (C₃-C₄)alcényle ou
un radical benzyle éventuellement monosubstitué ou polysubstitué, de manière identique ou différente, le ou les substituants étant à chaque fois indépendamment choisis parmi :
cyano, halogène, (C₃-C₆) cycloalkyle, (C₃-C₆)cycloalkyloxy, halogéno (C₃-C₈)cycloalkyle, (C₁-C₆)alkyle, (C₁-C₆)halogénoalkyle, (C₁-C₆)cyanoalkyle, (C₁-C₆)alcoxy- (C₁-C₆)alkyle, (C₁-C₆)alcoxy, (C₁-C₆)halogénoalcoxy, (C₁-C₆)cyanoalcoxy, (C₁-C₆)alcoxy- (C₁-C₆)alcoxy, (C₁-C₆)alkylthio et/ou (C₁-C₆)halogénoalkylthio,
Q est un radical phényle ou pyridinyle non substitué ou substitué par un ou plusieurs radicaux R⁷,
le ou les substituants R⁷ étant à chaque fois indépendamment choisis parmi :
cyano, halogène, (C₃-C₆) cycloalkyle, halogéno(C₃-C₈) cycloalkyle, (C₁-C₆) alkyle, (C₁-C₆) halogénoalkyle, (C₁-C₆) alcoxy- (C₁-C₆) alkyle, (C₁-C₆)alcoxy et/ou (C₁-C₆)halogénoalcoxy,
et
D est un radical C₁-C₆-alkyle, phényle, thiophényle, isoxazolyle, benzyle ou benzodioxolyle non substitué ou substitué par un ou plusieurs radicaux R⁸,
le ou les substituants R⁸ étant à chaque fois indépendamment choisis parmi :
cyano, halogène, acétyle, (C₃-C₆) cycloalkyle, (C₃-C₆)cycloalkyloxy, (C₃-C₆) cycloalkyl- (C₃-C₈) cycloalkyle, (C₁-C₆) alkyl- (C₃-C₆) cycloalkyle, halogéno (C₃-C₆) cycloalkyle, (C₁-C₆) alkyle, (C₁-C₆) halogénoalkyle, (C₁-C₆) cyanoalkyle, (C₁-C₆)hydroxyalkyle, hydroxycarbonyl-(C₁-C₆)-alcoxy, (C₁-C₆) alcoxycarbonyl- (C₁-C₆) alkyle, (C₁-C₆) alcoxy-(C₁-C₆) alkyle, (C₂-C₆) alcényle, (C₂-C₆)halogénoalcényle, (C₂-C₆) cyanoalcényle, (C₂-C₆) alcynyle, (C₂-C₆) halogénoalcynyle, (C₂-C₆)cyanoalcynyle, (C₁-C₆) alcoxy, (C₁-C₆)halogénoalcoxy, (C₁-C₆) cyanoalcoxy, (C₁-C₆)alcoxycarbonyl- (C₁-C₆) alcoxy, (C₁-C₆) alcoxy- (C₁-C₆)alcoxy, (C₁-C₆) alcoxyimino, (C₁-C₆) alkyl- (C₁-C₆)alcoxyimino, (C₁-C₆) halogénoalkyl- (C₁-C₆)alcoxyimino, (C₁-C₆) alkylthio, (C₁-C₆)halogénoalkylthio, (C₁-C₆) alcoxy- (C₁-C₆) alkylthio, (C₁-C₆) alkylthio- (C₁-C₆) alkyle, (C₁-C₆) alkylsulfinyle, (C₁-C₆)halogénoalkylsulfinyle, (C₁-C₆) alcoxy- (C₁-C₆) alkylsulfinyle, (C₁-C₆) alkylsulfinyl- (C₁-C₆) alkyle, (C₁-C₆) alkylsulfonyle, (C₁-C₆)halogénoalkylsulfonyle, (C₁-C₆) alcoxy- (C₁-C₆) alkylsulfonyle, (C₁-C₆) alkylsulfonyl- (C₁-C₆) alkyle, (C₁-C₆) alkylsulfonyloxy, (C₁-C₆) alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆) alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆) alkylamino, di-(C₁-C₆)alkylamino, (C₃-C₈)cycloalkylamino, (C₁-C₆)alkylcarbonylamino, (1-pyrazolyl) (C₁-C₃)alkyle et/ou (C₁-C₃)alcoxypyrimidinyloxy.

3. Utilisation non thérapeutique selon la revendication 1, **caractérisée en ce que**
R¹ est hydrogène, halogène, cyclopropyle, (C₁-C₄)alkyle, (C₁-C₄) alkylthio, halogénocyclopropyle, (C₁-C₄)halogénoalkyle ou benzyle non substitué ou monosubstitué ou polysubstitué, de manière identique ou différente, les substituants en question étant à chaque fois : cyano, halogène, (C₁-C₄)halogénoalkyle et/ou (C₁-C₄)alcoxy,
R² est hydrogène, halogène, cyano, (C₁-C₄)alkyle, (C₁-C₄) halogénoalkyle,
R³ est hydrogène, halogène, cyclopropyle, (C₁-C₄)alkyle, halogénocyclopropyle ou (C₁-C₄)halogénoalkyle,
R⁴ est hydrogène, halogène, cyano, (C₁-C₄)alkyle, cyclopropyle ou (C₁-C₄)halogénoalkyle,
R⁵ est hydrogène, cyclopropyle, (C₁-C₄)alkyle ou halogéno (C₁-C₄)alkyle,
R⁶ est hydrogène, halogène, cyano, (C₁-C₄) alkyle ou (C₁-C₄) halogénoalkyle,
A est halogène (C₁-C₄)alkyl- (C₃-C₄) cycloalkyle, halogène (C₃-C₆) cycloalkyle, (C₁-C₆) alkyle, (C₁-C₅) halogénoalkyle, (C₁-C₆) cyanoalkyle, (C₁-C₄)alcoxy- (C₁-C₄)alkyle, halogéno (C₁-C₃)alcoxy- (C₁-C₄)alkyle, (C₁-C₄) alkylthio- (C₁-C₄) alkyle, (C₁-C₄)alkylsulfinyl-(C₁-C₄)alkyle, halogène (C₃-C₄)alcényle ou
un radical benzyle éventuellement monosubstitué ou polysubstitué, de manière identique ou différente, le ou les substituants étant à chaque fois indépendamment choisis parmi :
cyano, halogène, (C₃-C₆) cycloalkyle, (C₃-C₆)cycloalkyloxy, halogéno (C₃-C₈) cycloalkyle, (C₁-C₆)alkyle, (C₁-C₆) halogénoalkyle, (C₁-C₆) alcoxy- (C₁-C₆)alkyle, (C₁-C₆) alcoxy, (C₁-C₆) halogénoalcoxy, (C₁-C₆)alkylthio et/ou (C₁-C₆)halogénoalkylthio,
Q est un radical phényle ou pyridinyle non substitué ou substitué par un ou plusieurs radicaux R⁷,
le ou les substituants R⁷ étant à chaque fois indépendamment choisis parmi :
cyano, halogène, (C₃-C₆) cycloalkyle, halogéno(C₃-C₆)cycloalkyle, (C₁-C₆) alkyle, (Ci-C₆)halogénoalkyle, (C₁-C₆) alcoxy- (C₁-C₆) alkyle, (C₁-C₆)alcoxy et/ou (C₁-C₆)halogénoalcoxy
et
D est un radical C₁-C₆-alkyle, phényle, thiophényle, isoxazolyle, benzyle ou benzodioxolyle non substitué ou substitué par un ou plusieurs radicaux R⁸,
le ou les substituants R⁸ étant à chaque fois indépendamment choisis parmi :
cyano, halogène, acétyle, (C₃-C₆) cycloalkyle, (C₃-C₆)cycloalkyloxy, (C₃-C₆) cycloalkyl- (C₃-C₈) cycloalkyle, (C₁-C₆) alkyl- (C₃-C₆) cycloalkyle, halogène (C₃-C₆) cycloalkyle, (C₁-C₆) alkyle, (C₁-C₆) halogénoalkyle, (C₁-C₆) cyanoalkyle, (C₁-C₆)hydroxyalkyle, hydroxycarbonyl-(C₁-C₆)-alcoxy, (C₁-C₆) alcoxycarbonyl- (C₁-C₆) alkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆)alcoxyimino, (C₁-C₆) alkyl- (C₁-C₆) alcoxyimino, (C₁-C₆)halogénoalkyl- (C₁-C₆) alcoxyimino, (C₁-C₆)alkylthio, (C₁-C₆) halogénoalkylthio, (C₁-C₆)alcoxy- (C₁-C₆) alkylthio, (C₁-C₆) alkylthio- (C₁-C₆)alkyle, (C₁-C₆) alkylsulfinyle, (C₁-C₆)halogénoalkylsulfinyle, (C₁-C₆) alcoxy- (C₁-C₆)alkylsulfinyle, (C₁-C₆)alkylsulfinyl-(Ci-C₆)alkyle, (C₁-C₆)alkylsulfonyle, (C₁-C₆)halogénoalkylsulfonyle, (C₁-C₆)alcoxy- (C₁-C₆)alkylsulfonyle, (C₁-C₆) alkylsulfonyl- (C₁-C₆)alkyle, (C₁-C₆)alkylsulfonyloxy, (C₁-C₆)alkylcarbonyle, (C₁-C₆)halogénoalkylcarbonyle, (C₁-C₆) alkylcarbonyloxy, (C₁-C₆)alcoxycarbonyle, (C₁-C₆) halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, (C₃-C₈)cycloalkylaminocarbonyle, (C₁-C₆)alkylsulfonylamino, (C₁-C₆)alkylamino, di-(C₁-C₆)alkylamino, (C₃-C₆)cycloalkylamino, (C₁-C₆)alkylcarbonylamino, (1-pyrazolyl) (C₁-C₃)alkyle et/ou (C₁-C₃)alcoxypyrimidinyloxy.

4. Utilisation non thérapeutique selon la revendication 1, **caractérisée en ce que**
R¹ est hydrogène, chlore, brome, méthyle, benzyle, 2-chlorobenzyle, 3-chlorobenzyle, 4-chlorobenzyle, trifluorométhyle, méthylthio ou isopropylthio,
R² est hydrogène, cyano, chlore, brome ou iode,
R³ est hydrogène, chlore, méthyle, isopropyle, éthyle ou brome,
R⁴ est hydrogène, chlore, brome, iode, fluor, difluorométhyle, isopropyle ou cyclopropyle,
R⁵ est méthyle ou 2,2,2-trifluoréthyle et
R⁶ est hydrogène ou chlore,
A est un radical choisi parmi les radicaux de formules (III1 à III19) : 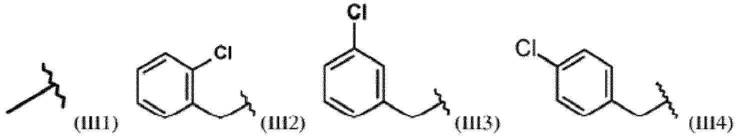 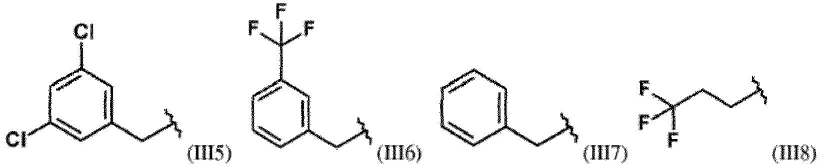 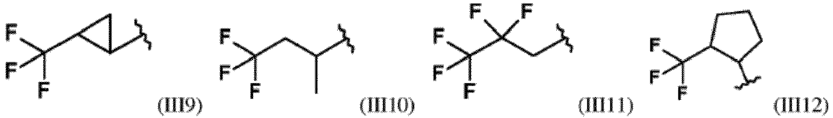 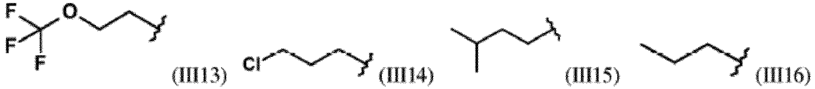 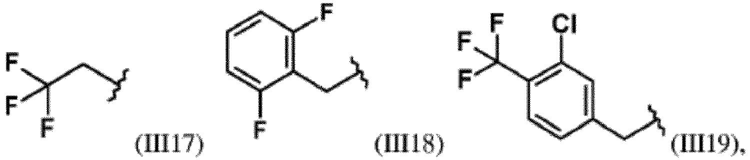
Q est un radical choisi parmi les radicaux de formules (IV1 à IV40) : 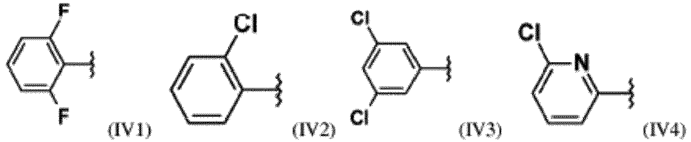 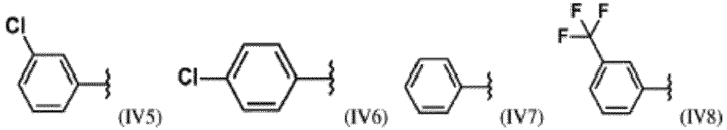 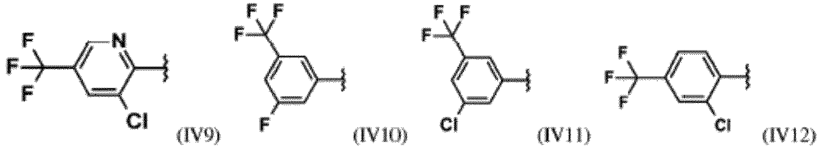 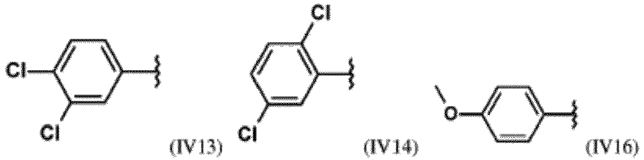 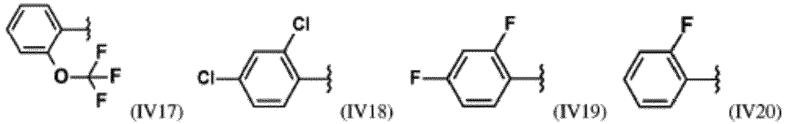 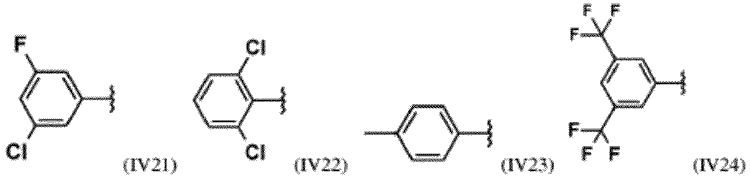 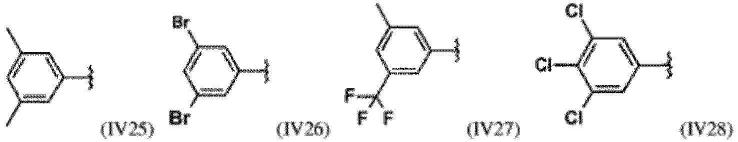 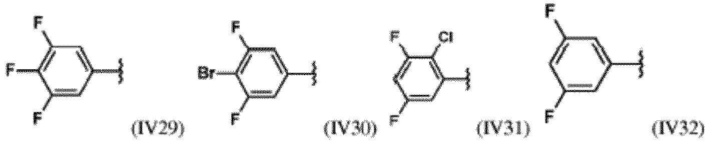 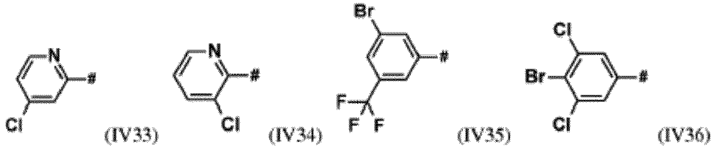 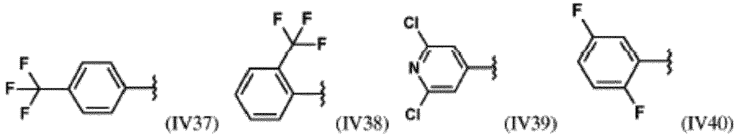 et
D est un radical choisi parmi les radicaux de formules (V1 à V61) :

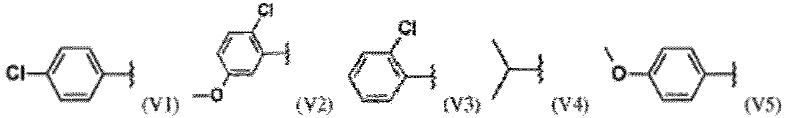

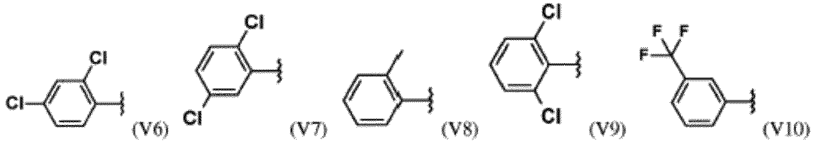

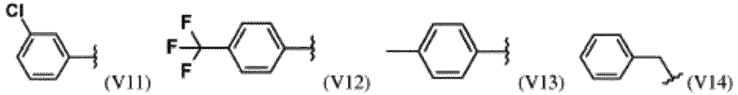

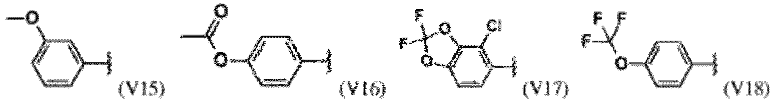

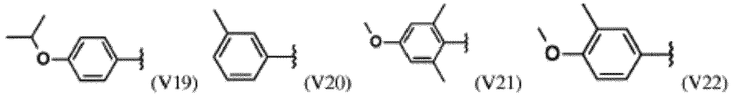

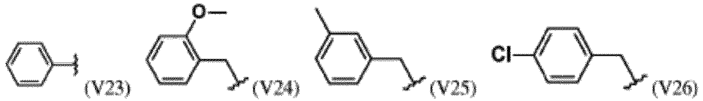

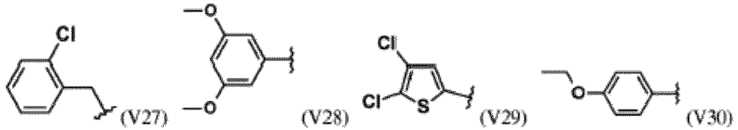

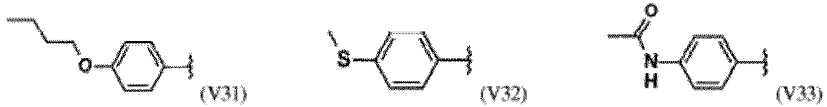

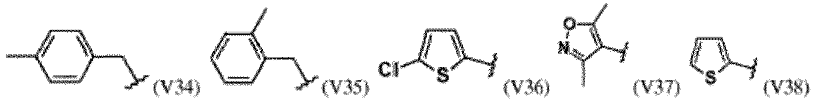

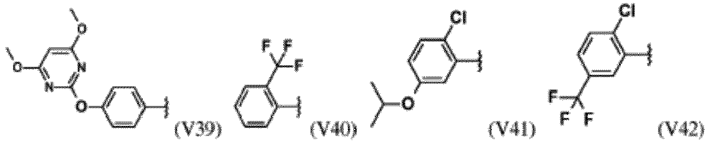

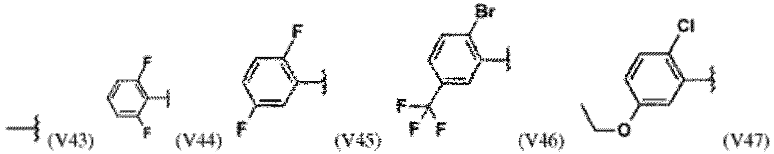

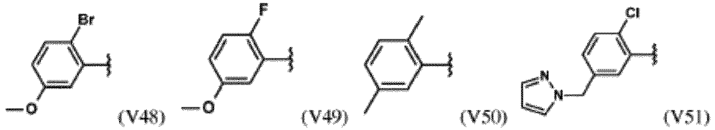

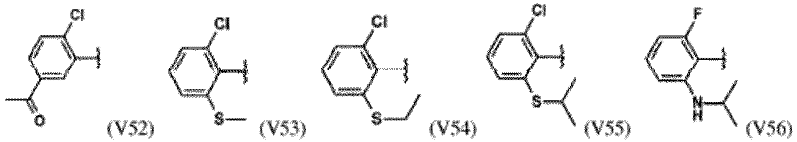

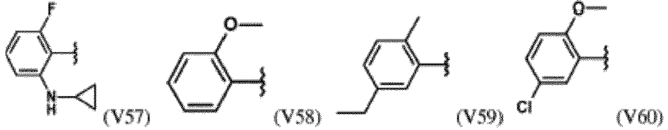

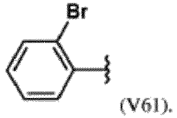

5. Utilisation non thérapeutique selon l'une quelconque des revendications 1 à 4, **caractérisée en ce que** dans la formule (I), M représente un radical choisi parmi les formules IIa et IIb, les radicaux R¹, R², A, R³, R⁴, R⁷, R⁸, Q et D possédant les significations décrites dans la revendication 1, 2, 3 ou 4.

6. Utilisation non thérapeutique selon l'une quelconque des revendications 1 à 4, **caractérisée en ce que** des composés de formule (Ia), (Ib) ou (Ic) sont utilisés, 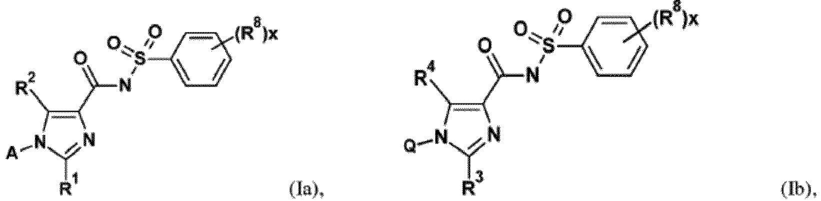 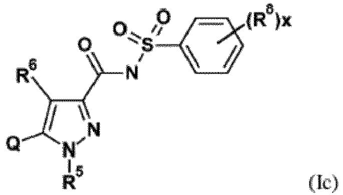 les radicaux R¹, R², R³, R⁴, R⁵, R⁶, R⁷, A, Q et R⁸ possédant les significations décrites dans la revendication 1, 2, 3 ou 4.

7. Utilisation non thérapeutique selon l'une quelconque des revendications 1 à 6, **caractérisée en ce que** les parasites animaux sont des nématodes.

8. Utilisation non thérapeutique selon l'une quelconque des revendications 1 à 7 pour la protection du matériel de propagation de végétaux.

9. Agent, comportant une teneur d'au moins un composé selon la formule (I) ou de formule (Ia), (Ib) ou (Ic), qui est défini selon l'une quelconque des revendications 1 à 6, et des diluants habituels et/ou des substances tensioactives, en particulier pour la lutte contre des parasites animaux, préférablement des nématodes.

10. Procédé pour la lutte contre des parasites animaux, préférablement des nématodes, dans lequel on laisse agir au moins un composé selon la formule (I) ou de formule (Ia), (Ib) ou (Ic), qui est défini selon l'une quelconque des revendications 1 à 6, ou un agent selon l'invention selon la revendication 9, sur les parasites animaux, préférablement des nématodes et/ou sur leur lieu de vie, le traitement chirurgical, thérapeutique et diagnostique du corps humain ou animal étant exclus.

11. Formulation agrochimique contenant au moins un composé selon la formule (I) ou de formule (Ia), (Ib) ou (Ic), qui est défini selon l'une quelconque des revendications 1 à 6, en des teneurs biologiquement efficaces comprises entre 0,00000001 et 98 % en poids, par rapport au poids de la formulation agrochimique, ainsi que des diluants et/ou des substances tensioactives.

12. Formulation agrochimique selon la revendication 11, **caractérisée en ce que** celle-ci contient de plus un principe actif agrochimique supplémentaire.

13. Composés de formule (I') 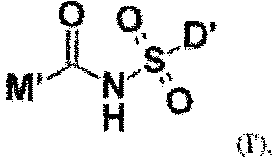 dans laquelle
M' représente un radical choisi parmi les formules (IIa' à IIc') : 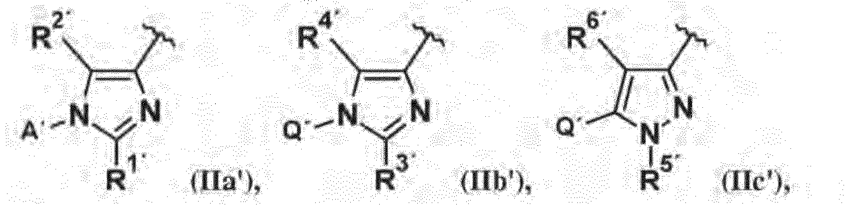 dans lequelles
R^{1'} est cyano, halogène, nitro, (C₃-C₈) cycloalkyle, (C₃-C₈) cycloalkyloxy, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆) halogénoalkyle, (C₁-C₆) cyanoalkyle, (C₁-C₆) hydroxyalkyle, (C₁-C₆) alcoxy- (C₁-C₆) alkyle, (C₁-C₆)alcoxy, (C₁-C₆) halogénoalcoxy, (C₁-C₆)cyanoalcoxy, (C₁-C₆) alcoxy- (C₁-C₆) alcoxy, (C₁-C₆)alcoxyimino, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl- (C₁-C₆) alkyle, (C₁-C₆)alkylsulfonyl- (C₁-C₆) alkyle, (C₁-C₆)alcoxycarbonyle, (C₁-C₆) halogénoalcoxycarbonyle, (C₁-C₆) alkylaminocarbonyle, (C₁-C₆)alkylcarbonylamino ou benzyle non substitué, monosubstitué ou polysubstitué, de manière identique ou différente, les substituants en question étant à chaque fois :
cyano, halogène, nitro, (C₃-C₈) cycloalkyle, (C₃-C₈)cycloalkyloxy, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆)halogénoalkyle, (C₁-C₆) cyanoalkyle, (C₁-C₆)alcoxy- (C₁-C₆) alkyle, (C₁-C₆) alcoxy, (C₁-C₆)halogénoalcoxy, (C₁-C₆) cyanoalcoxy, (C₁-C₆)alcoxy- (C₁-C₆) alcoxy, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl-(C₁-C₆) alkyle et/ou (C₁-C₆)alkylsulfonyl- (C₁-C₆) alkyle,
R^{2'} est hydrogène, cyano, halogène, nitro, (C₃-C₈) cycloalkyle, (C₃-C₈) cycloalkyloxy, (C₃-C₈) cycloalkyl- (C₃-C₈) cycloalkyle, (C₁-C₆) alkyl- (C₃-C₈)cycloalkyle, halogéno (C₃-C₈) cycloalkyle, (C₁-C₆)alkyle, (C₁-C₆) halogénoalkyle, (C₁-C₆)cyanoalkyle, (C₁-C₆) hydroxyalkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆)alcoxyimino, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl-(C₁-C₆) alkyle, (C₁-C₆)alkylsulfonyl-(C₁-C₆) alkyle, (C₁-C₆)alcoxycarbonyle, (C₁-C₆)halogénoalcoxycarbonyle, (C₁-C₆) alkylaminocarbonyle ou (C₁-C₆)alkylcarbonylamino,
R^{3'} est cyano, halogène, nitro, (C₃-C₈) cycloalkyle, (C₃-C₈) cycloalkyloxy, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆)halogénoalkyle, (C₁-C₆) cyanoalkyle, (C₁-C₆)hydroxyalkyle, (C₁-C₆) alcoxy- (C₁-C₆) alkyle, (C₁-C₆)alcoxy, (C₁-C₆) halogénoalcoxy, (C₁-C₆)cyanoalcoxy, (C₁-C₆) alcoxy- (C₁-C₆) alcoxy, (C₁-C₆)alcoxyimino, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl-(C₁-C₆) alkyle, (C₁-C₆)alkylsulfonyl-(C₁-C₆) alkyle, (C₁-C₆)alcoxycarbonyle, (C₁-C₆) halogénoalcoxycarbonyle, (C₁-C₆) alkylaminocarbonyle, (C₁-C₆)alkylcarbonylamino ou benzyle non substitué, monosubstitué ou polysubstitué, de manière identique ou différente, les substituants en question étant à chaque fois :
cyano, halogène, nitro, (C₃-C₈) cycloalkyle, (C₃-C₈)cycloalkyloxy, (C₃-C₈) cycloalkyl- (C₃-C₈)cycloalkyle, (C₁-C₆) alkyl- (C₃-C₈) cycloalkyle, halogéno (C₃-C₈) cycloalkyle, (C₁-C₆) alkyle, (C₁-C₆)halogénoalkyle, (C₁-C₆) cyanoalkyle, (C₁-C₆)alcoxy- (C₁-C₆) alkyle, (C₁-C₆) alcoxy, (C₁-C₆)halogénoalcoxy, (C₁-C₆) cyanoalcoxy, (C₁-C₆)alcoxy- (C₁-C₆) alcoxy, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl-(C₁-C₆) alkyle et/ou (C₁-C₆)alkylsulfonyl-(C₁-C₆) alkyle,
R^{4'} est hydrogène, cyano, halogène, nitro, (C₃-C₈)cycloalkyle, (C₃-C₈) cycloalkyloxy, (C₃-C₈)cycloalkyl- (C₃-C₈) cycloalkyle, (C₁-C₆) alkyl- (C₃-C₈)cycloalkyle, halogéno (C₃-C₈) cycloalkyle, (C₁-C₆)alkyle, (C₁-C₆) halogénoalkyle, (C₁-C₆)cyanoalkyle, (C₁-C₆) hydroxyalkyle, (C₁-C₆) alcoxy-(C₁-C₆) alkyle, (C₁-C₆) alcoxy, (C₁-C₆) halogénoalcoxy, (C₁-C₆) cyanoalcoxy, (C₁-C₆) alcoxy- (C₁-C₆) alcoxy, (C₁-C₆)alcoxyimino, (C₁-C₆) alkylthio, (C₁-C₆)halogénoalkylthio, (C₁-C₆) alcoxy- (C₁-C₆)alkylthio, (C₁-C₆) alkylthio- (C₁-C₆) alkyle, (C₁-C₆)alkylsulfinyl-(C₁-C₆) alkyle, (C₁-C₆)alkylsulfonyl-(C₁-C₆) alkyle, (C₁-C₆)alcoxycarbonyle, (C₁-C₆) halogénoalcoxycarbonyle, (C₁-C₆) alkylaminocarbonyle ou (C₁-C₆)alkylcarbonylamino,
R^{5'} est (C₃-C₈) cycloalkyle, (C₃-C₈) cycloalkyl- (C₃-C₈) cycloalkyle, (C₁-C₆) alkyl- (C₃-C₈) cycloalkyle, halogéno (C₃-C₈) cycloalkyle, (C₁-C₆) alkyle, (C₁-C₆)halogénoalkyle, (C₁-C₆)cyanoalkyle, (C₁-C₆)alcoxy- (C₁-C₆)alkyle, (C₁-C₆)alkylthio- (C₁-C₆)alkyle, halogéno (C₁-C₆)alkylthio- (C₁-C₆)alkyle, (C₁-C₆)alkylsulfinyl-(C₁-C₆) alkyle, (C₁-C₆)alkylsulfonyl-(C₁-C₆)alkyle, halogéno(C₁-C₆)alkylsulfinyl-(C₁-C₆)alkyle ou halogène(C₁-C₆)alkylsulfonyl-(C₁-C₆)alkyle,
R^{6'} est hydrogène, cyano, halogène, nitro, (C₃-C₈)cycloalkyle, (C₃-C₈)cycloalkyloxy, (C₃-C₈)cycloalkyl- (C₃-C₈)cycloalkyle, (C₁-C₆)alkyl-(C₃-C₈)cycloalkyle, halogéno(C₃-C₈)cycloalkyle, (C₁-C₆)alkyle, (C₁-C₆)halogénoalkyle, (C₁-C₆)cyanoalkyle, (C₁-C₆)hydroxyalkyle, (C₁-C₆)alcoxy-(C₁-C₆)alkyle, (C₁-C₆)alcoxy, (C₁-C₆)halogénoalcoxy, (C₁-C₆)cyanoalcoxy, (C₁-C₆)alcoxy- (C₁-C₆) alcoxy, (Ci-C₆)alcoxyimino, (C₁-C₆)alkylthio, (C₁-C₆)halogénoalkylthio, (C₁-C₆) alcoxy- (Ci-C₆)alkylthio, (C₁-C₆)alkylthio- (C₁-C₆)alkyle, (Ci-C₆)alkylsulfinyl-(C₁-C₆)alkyle, (C₁-C₆)alkylsulfonyl-(C₁-C₆)alkyle, (C₁-C₆)alcoxycarbonyle, (C₁-C₆)halogénoalcoxycarbonyle, (C₁-C₆) alkylaminocarbonyle ou (C₁-C₆)alkylcarbonylamino,
A' est halogène(C₁-C₆) alkyl-(C₃-C₈)cycloalkyle, halogéno(C₃-C₈)cycloalkyle, (C₁-C₆)alkyle, (C₁-C₆)halogénoalkyle, (C₁-C₆)cyanoalkyle, (C₁-C₆)alcoxy- (C₁-C₆)alkyle, halogéno(C₁-C₃) alcoxy-(C₁-C₄)alkyle, (C₁-C₆)alkylthio- (C₁-C₆)alkyle, (Ci-C₆)alkylsulfinyl-(C₁-C₆)alkyle, halogène(C₁-C₆)alkylthio-(C₁-C₆)alkyle, halogène(C₁-C₆)alkylsulfinyl-(C₁-C₆)alkyle, halogène(C₁-C₆)alkylsulfonyl-(C₁-C₆)alkyle, halogène(C₃-C₆)alcényle ou
un radical benzyle éventuellement monosubstitué ou polysubstitué, de manière identique ou différente, le ou les substituants étant à chaque fois indépendamment choisis parmi :
cyano, halogène, (C₃-C₆)cycloalkyle, (C₃-C₆)cycloalkyloxy, (C₃-C₆)cycloalkyl- (C₃-C₈)cycloalkyle, (C₁-C₆)alkyl- (C₃-C₈)cycloalkyle, halogéno(C₃-C₈)cycloalkyle, (C₁-C₆)alkyle, (C₁-C₆)halogénoalkyle, (C₁-C₆)cyanoalkyle, (C₁-C₆)alcoxy-(C₁-C₆)alkyle, (C₁-C₆)alcoxy, (C₁-C₆)halogénoalcoxy, (C₁-C₆)cyanoalcoxy, (C₁-C₆)alcoxy-(C₁-C₆)alcoxy, (C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio- (C₁-C₆)alkyle, (C₁-C₆)alkylsulfinyl-(C₁-C₆)alkyle, (C₁-C₆)alkylsulfonyl-(C₁-C₆)alkyle, halogène(C₁-C₆)alkylthio-(C₁-C₆)alkyle, halogène(C₁-C₆)alkylsulfinyl-(C₁-C₆)alkyle et/ou halogéno(C₁-C₆)alkylsulfonyl-(C₁-C₆)alkyle,
Q' est un radical phényle ou pyridinyle non substitué ou substitué par un ou plusieurs radicaux R^{7'},
le ou les substituants R^{7'} étant à chaque fois indépendamment choisis parmi :
cyano, halogène, nitro, (C₃-C₆)cycloalkyle, (C₃-C₆)cycloalkyloxy, (C₃-C₆)cycloalkyl-(C₃-C₈)cycloalkyle, (C₁-C₆)alkyl-(C₃-C₈) cycloalkyle, halogéno (C₃-C₈)cycloalkyle, (C₁-C₆)alkyle, (C₁-C₆)halogénoalkyle, (C₁-C₆)cyanoalkyle, (C₁-C₆)hydroxyalkyle, (C₁-C₆)alcoxy- (C₁-C₆)alkyle, (C₁-C₆)alcoxy, (C₁-C₆)halogénoalcoxy, (C₁-C₆)cyanoalcoxy, (C₁-C₆)alcoxy- (C₁-C₆)alcoxy, (C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio- (C₁-C₆)alkyle, (C₁-C₆)alkylsulfinyl-(C₁-C₆)alkyle, (C₁-C₆)alkylsulfonyl-(C₁-C₆)alkyle, (C₁-C₆)alcoxycarbonyle, (C₁-C₆)halogénoalcoxycarbonyle, (C₁-C₆)alkylaminocarbonyle, (C₁-C₆)alkylcarbonylamino, halogène(C₁-C₆)alkylthio- (C₁-C₆)alkyle, halogéno(C₁-C₆)alkylsulfinyl-(C₁-C₆)alkyle et/ou halogéno (C₁-C₆)alkylsulfonyl-(C₁-C₆)alkyle,
et
D' est un radical C₁-C₆-alkyle, phényle, thiophényle, isoxazolyle, phényl-(C₁-C₂)alkyle ou benzodioxolyle non substitué ou substitué par un ou plusieurs radicaux R^{8'},
le ou les substituants R^{8'} étant à chaque fois indépendamment choisis parmi :
cyano, halogène, nitro, acétyle, (C₃-C₈)cycloalkyle, (C₃-C₈)cycloalkyloxy, (C₃-C₈)cycloalkyl-(C₃-C₈)cycloalkyle, (C₁-C₆)alkyl-(C₃-C₈)cycloalkyle, halogéno (C₃-C₈)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl- (C₁-C₆)alkyle, (C₁-C₆)alcoxy-(C₁-C₆) alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆) cyanoalcoxy, (C₁-C₆)alcoxycarbonyl- (C₁-C₆)alcoxy, (C₁-C₆)alcoxy- (C₁-C₆)alcoxy, (C₁-C₆)alkylhydroxyimino, (C₁-C₆)alcoxyimino, (C₁-C₆)alkyl- (C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio- (C₁-C₆)alkyle, (C₁-C₆)alkylsulfinyle, (C₁-C₆)halogénoalkylsulfinyle, (C₁-C₆)alcoxy- (C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy- (C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)alkylthiocarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, (C₁-C₆)alkylaminothiocarbonyle, di-(C₁-C₆)alkylaminocarbonyle, di-(C₁-C₆)alkyl-aminothiocarbonyle, (C₂-C₆)alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆)alkylamino, di-(C₁-C₆)alkylamino, aminosulfonyle, (Ci-C₆)alkylaminosulfonyle, di-(C₁-C₆)alkyl-aminosulfonyle, (C₁-C₆)alkylsulfoximino, aminothiocarbonyle, (C₁-C₆)alkylaminothiocarbonyle, di-(C₁-C₆)alkyl-aminothiocarbonyle, (C₃-C₈)cycloalkylamino, (C₁-C₆) alkylcarbonylamino, (1-pyrazolyl) (C₁-C₃)alkyle et/ou (Ci-C₃)alcoxypyrimidinyloxy.

14. Composés de formule (I') selon la revendication 13, **caractérisés en ce que**
R^{1'} est cyano, halogène, cyclopropyle, (C₁-C₄)alkyle, halogénocyclopropyle, halogéno(C₁-C₃)alkylcyclopropyle, (C₁-C₄)halogénoalkyle, (C₁-C₄) alcoxy, (C₁-C₄)halogénoalcoxy, (C₁-C₄)alkylthio, (C₁-C₄)halogénoalkylthio, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle, ou benzyle non substitué, monosubstitué ou polysubstitué, de manière identique ou différente, les substituants en question étant à chaque fois :
cyano, halogène, (C₁-C₄)halogénoalkyle, (C₁-C₄)alcoxy, (C₁-C₄)halogénoalcoxy, (C₁-C₆)alkylthio et/ou (C₁-C₆)halogénoalkylthio,
R^{2'} est hydrogène, cyano, halogène, (C₁-C₄)halogénoalkyle, (C₁-C₄)alcoxy, (C₁-C₄)halogénoalcoxy, (C₁-C₆)alkylthio ou (C₁-C₆)halogénoalkylthio,
R³' est cyano, halogène, cyclopropyle, (C₁-C₄)alkyle, halogénocyclopropyle, halogéno(C₁-C₃)alkylcyclopropyle, (C₁-C₄)halogénoalkyle, (C₁-C₄)alcoxy, (C₁-C₄)halogénoalcoxy, (C₁-C₄) alkylthio, (C₁-C₄)halogénoalkylthio, (C₁-C₄) alkylthio- (Ci-C₄)alkyle, (C₁-C₄)alkylsulfinyl-(C₁-C₄)alkyle ou (C₁-C₄)alkylsulfonyl-(C₁-C₄)alkyle,
R^{4'} est hydrogène, cyano, halogène, (C₁-C₄)halogénoalkyle, (C₁-C₄)alcoxy, (C₁-C₄)alkyle, (C₃-C₄)cycloalkyle, (C₁-C₄)halogénoalcoxy, (C₁-C₆)alkylthio ou (C₁-C₆)halogénoalkylthio,
R^{5'} est cyclopropyle, (C₁-C₄)alkyle, halogénocyclopropyle, halogéno(C₁-C₃)alkylcyclopropyle ou (C₁-C₄)halogénoalkyle,
R^{6'} est hydrogène, cyano, halogène, (Ci-C₄)halogénoalkyle, (C₁-C₄)alcoxy, (C₁-C₄)halogénoalcoxy, (C₁-C₆)alkylthio ou (C₁-C₆)halogénoalkylthio,
A' est halogène(C₁-C₄)alkyl- (C₃-C₄)cycloalkyle, halogéno (C₃-C₆)cycloalkyle, (C₁-C₆)alkyle, (C₁-C₅)halogénoalkyle, (C₁-C₆)cyanoalkyle, (C₁-C₄)alcoxy-(C₁-C₄)alkyle, halogéno (C₁-C₃)alcoxy- (C₁-C₄)alkyle, (C₁-C₄)alkylthio- (C₁-C₄)alkyle, (C₁-C₄)alkylsulfinyl-(C₁-C₄)alkyle, halogène(C₃-C₄)alcényle ou
un radical benzyle éventuellement monosubstitué ou polysubstitué, de manière identique ou différente, le ou les substituants étant à chaque fois indépendamment choisis parmi :
cyano, halogène, (C₃-C₆)cycloalkyle, (C₃-C₆)cycloalkyloxy, halogéno (C₃-C₈)cycloalkyle, (C₁-C₆)alkyle, (C₁-C₆)halogénoalkyle, (C₁-C₆)cyanoalkyle, (C₁-C₆)alcoxy- (C₁-C₆)alkyle, (C₁-C₆)alcoxy, (C₁-C₆)halogénoalcoxy, (C₁-C₆)cyanoalcoxy, (C₁-C₆)alcoxy- (C₁-C₆)alcoxy, (C₁-C₆)alkylthio et/ou (C₁-C₆)halogénoalkylthio,
Q' est un radical phényle ou pyridinyle non substitué ou substitué par un ou plusieurs radicaux R^{7'},
le ou les substituants R^{7'} étant à chaque fois indépendamment choisis parmi :
cyano, halogène, (C₃-C₆)cycloalkyle, halogéno(C₃-C₈)cycloalkyle, (C₁-C₆)alkyle, (C₁-C₆)halogénoalkyle, (C₁-C₆)alcoxy- (C₁-C₆)alkyle, (C₁-C₆)alcoxy et/ou (C₁-C₆)halogénoalcoxy,
et
D' est un radical C₁-C₆-alkyle, phényle, thiophényle, isoxazolyle, benzyle ou benzodioxolyle non substitué ou substitué par un ou plusieurs radicaux R^{8'},
le ou les substituants R^{8'} étant à chaque fois indépendamment choisis parmi :
cyano, halogène, acétyle, (C₃-C₆)cycloalkyle, (C₃-C₆)cycloalkyloxy, (C₃-C₆)cycloalkyl- (C₃-C₈)cycloalkyle, (C₁-C₆)alkyl-(C₃-C₆)cycloalkyle, halogéno (C₃-C₆)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl- (C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy- (C₁-C₆)alcoxy, (C₁-C₆)alcoxyimino, (C₁-C₆)alkyl- (C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy- (C₁-C₆)alkylthio, (C₁-C₆)alkylthio- (C₁-C₆)alkyle, (C₁-C₆)alkylsulfinyle, (C₁-C₆)halogénoalkylsulfinyle, (C₁-C₆)alcoxy- (C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆) alkylaminocarbonyle, (C₂-C₆)alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆)alkylamino, di-(C₁-C₆)alkylamino, (C₃-C₈)cycloalkylamino, (C₁-C₆)alkylcarbonylamino, (1-pyrazolyl)(C₁-C₃)alkyle et/ou (C₁-C₃)alcoxypyrimidinyloxy.

15. Composés de formule (I') selon la revendication 13, **caractérisés en ce que**
R¹' est halogène, cyclopropyle, (C₁-C₄)alkyle, (C₁-C₄)alkylthio, halogénocyclopropyle, (C₁-C₄)halogénoalkyle ou benzyle non substitué ou monosubstitué ou polysubstitué, de manière identique ou différente, les substituants en question étant à chaque fois : cyano, halogène, (C₁-C₄)halogénoalkyle et/ou (C₁-C₄)alcoxy,
R^{2'} est hydrogène, halogène, cyano, (C₁-C₄)alkyle, (C₁-C₄)halogénoalkyle,
R^{3'} est halogène, cyclopropyle, (C₁-C₄)alkyle, halogénocyclopropyle ou (C₁-C₄)halogénoalkyle,
R^{4'} est hydrogène, halogène, cyano, (C₁-C₄)alkyle, cyclopropyle ou (C₁-C₄)halogénoalkyle,
R^{5'} est cyclopropyle, (C₁-C₄)alkyle ou halogéno(C₁-C₄)alkyle,
R^{6'} est hydrogène, halogène, cyano, (C₁-C₄)alkyle ou (C₁-C₄)halogénoalkyle,
A' est halogène(C₁-C₄)alkyl- (C₃-C₄)cycloalkyle, halogène(C₃-C₆)cycloalkyle, (C₁-C₆)alkyle, (C₁-C₅)halogénoalkyle, (C₁-C₆)cyanoalkyle, (C₁-C₄)alcoxy-(C₁-C₄)alkyle, halogéno (C₁-C₃)alcoxy-(C₁-C₄)alkyle, (C₁-C₄)alkylthio- (C₁-C₄)alkyle, (C₁-C₄)alkylsulfinyl-(C₁-C₄)alkyle, halogène(C₃-C₄)alcényle ou
un radical benzyle éventuellement monosubstitué ou polysubstitué, de manière identique ou différente, le ou les substituants étant à chaque fois indépendamment choisis parmi :
cyano, halogène, (C₃-C₆)cycloalkyle, (C₃-C₆)cycloalkyloxy, halogéno (C₃-C₈)cycloalkyle, (C₁-C₆)alkyle, (C₁-C₆)halogénoalkyle, (C₁-C₆) alcoxy-(C₁-C₆)alkyle, (C₁-C₆)alcoxy, (C₁-C₆)halogénoalcoxy, (C₁-C₆)alkylthio et/ou (C₁-C₆)halogénoalkylthio,
Q' est un radical phényle ou pyridinyle non substitué ou substitué par un ou plusieurs radicaux R^{7'},
le ou les substituants R^{7'} étant à chaque fois indépendamment choisis parmi :
cyano, halogène, (C₃-C₆)cycloalkyle, halogéno(C₃-C₆)cycloalkyle, (C₁-C₆)alkyle, (C₁-C₆)halogénoalkyle, (C₁-C₆)alcoxy- (C₁-C₆)alkyle, (C₁-C₆)alcoxy et/ou (C₁-C₆)halogénoalcoxy
et
D' est un radical C₁-C₆-alkyle, phényle, thiophényle, isoxazolyle, benzyle ou benzodioxolyle non substitué ou substitué par un ou plusieurs radicaux R^{8'},
le ou les substituants R^{8'} étant à chaque fois indépendamment choisis parmi :
cyano, halogène, acétyle, (C₃-C₆)cycloalkyle, (C₃-C₆)cycloalkyloxy, (C₃-C₆)cycloalkyl-(C₃-C₈)cycloalkyle, (C₁-C₆)alkyl- (C₃-C₆)cycloalkyle, halogéno (C₃-C₆)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl- (C₁-C₆)alkyle, (C₁-C₆)alcoxy-(C₁-C₆)alkyle, (C₁-C₆)alcoxy, (C₁-C₆)halogénoalcoxy, (C₁-C₆) cyanoalcoxy, (C₁-C₆)alcoxy- (C₁-C₆)alcoxy, (C₁-C₆)alcoxyimino, (C₁-C₆)alkyl- (C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio- (C₁-C₆)alkyle, (C₁-C₆)alkylsulfinyle, (C₁-C₆)halogénoalkylsulfinyle, (C₁-C₆)alcoxy- (C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy- (C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, (C₃-C₈)cycloalkylaminocarbonyle, (C₁-C₆)alkylsulfonylamino, (C₁-C₆)alkylamino, di-(C₁-C₆)alkylamino, (C₃-C₆)cycloalkylamino, (C₁-C₆)alkylcarbonylamino, (1-pyrazolyl)(C₁-C₃)alkyle et/ou (C₁-C₃)alcoxypyrimidinyloxy.

16. Composés de formule (I') selon la revendication 13, **caractérisés en ce que**
R^{1'} est chlore, brome, méthyle, benzyle, 2-chlorobenzyle, 3-chlorobenzyle, 4-chlorobenzyle, trifluorométhyle, méthylthio ou isopropylthio,
R^{2'} est hydrogène, cyano, chlore, brome ou iode,
R^{3'} est chlore, méthyle, isopropyle, éthyle ou brome,
R^{4'} est hydrogène, chlore, brome, iode, fluor, difluorométhyle, isopropyle ou cyclopropyle,
R^{5'} est méthyle ou 2,2,2-trifluoréthyle,
R^{6'} est hydrogène ou chlore,
A' est un radical choisi parmi les radicaux de formules (III1 à III19) : 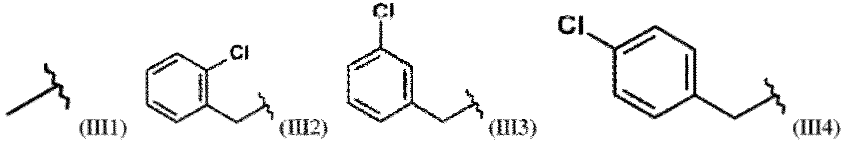 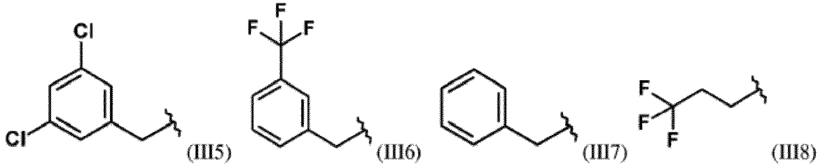 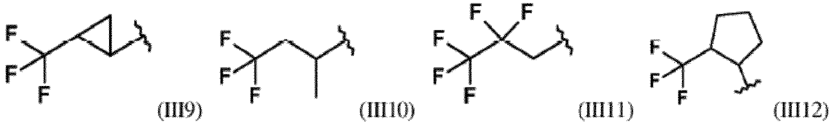 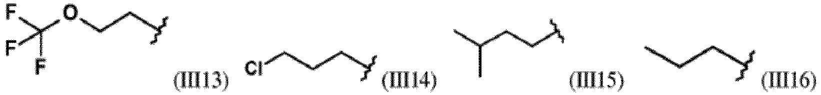 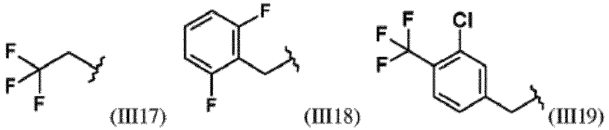
Q' est un radical choisi parmi les radicaux de formules (IV1 à IV40) : 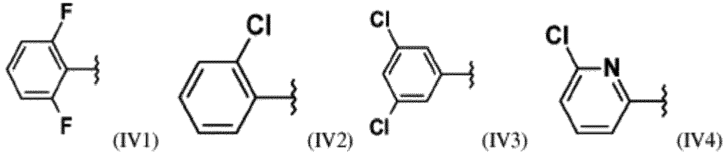 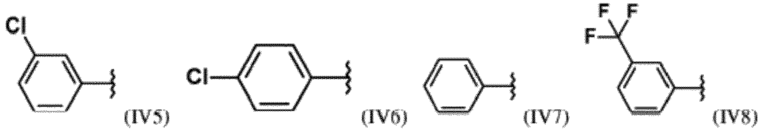 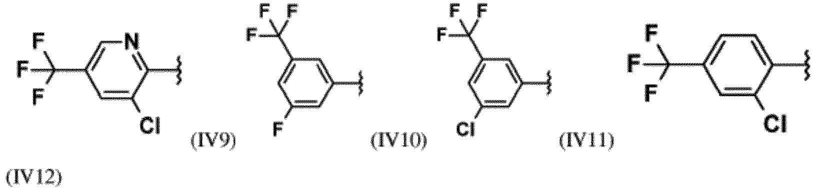 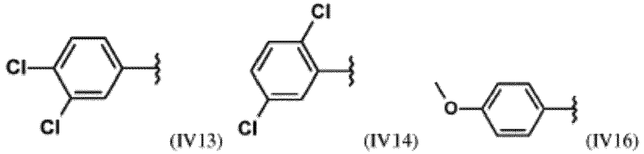 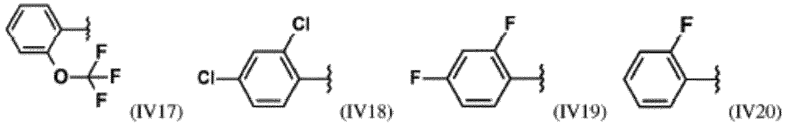 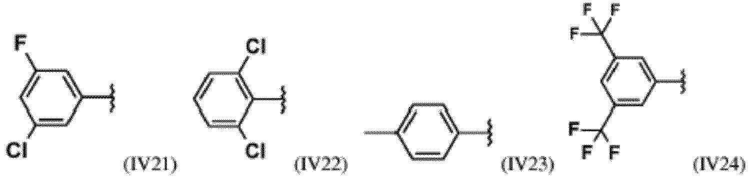 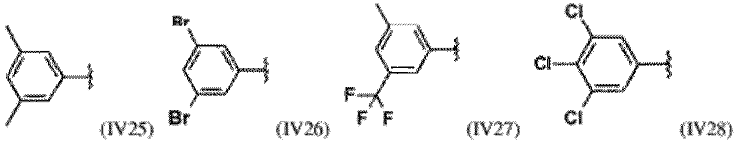 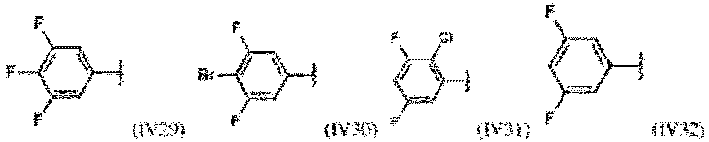 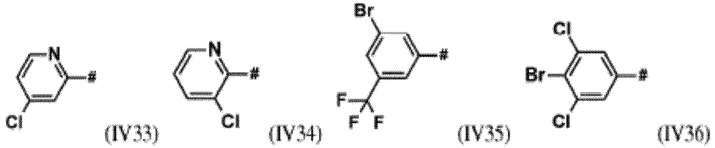 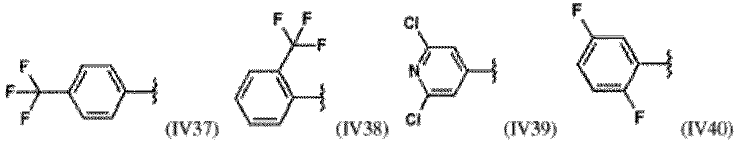 et
D' est un radical choisi parmi les radicaux de formules (V1 à V61) :

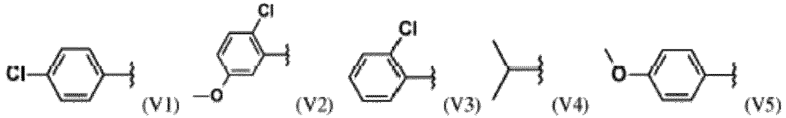

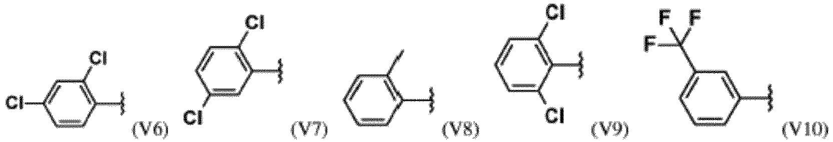

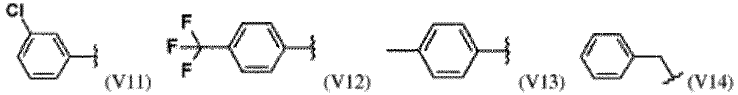

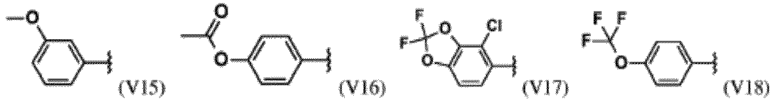

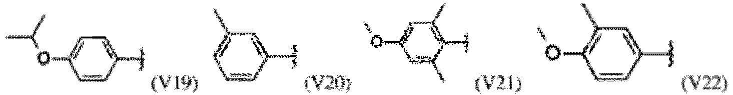

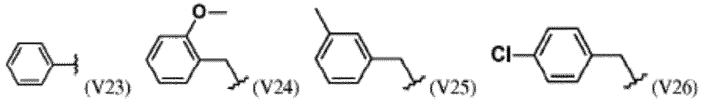

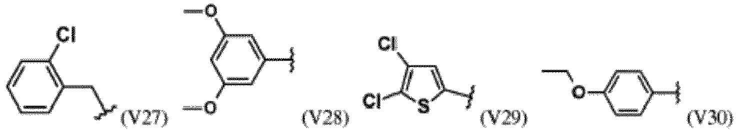

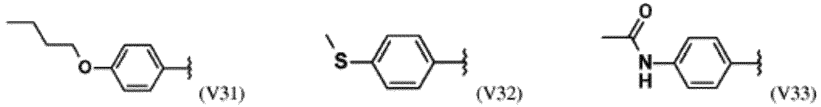

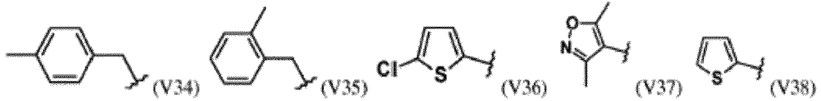

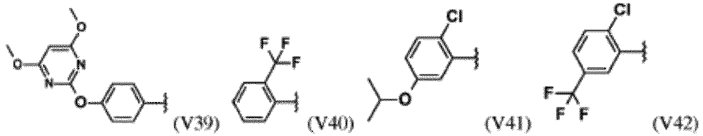

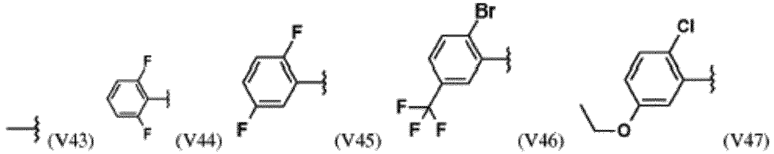

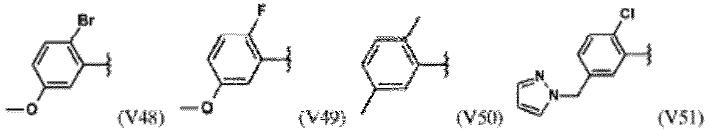

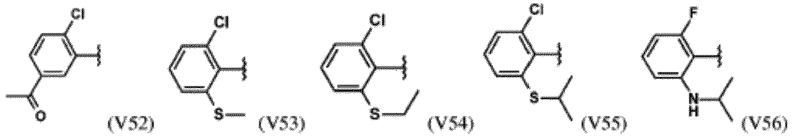

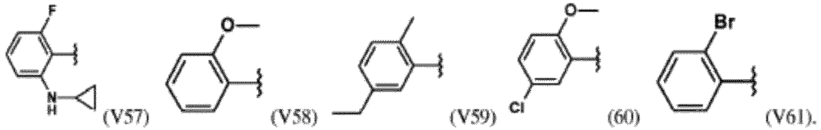

17. Composés de formule (I') selon l'une quelconque des revendications 13 à 16, **caractérisés en ce que** dans les formules (I'), M' représente un radical choisi parmi les formules IIa' et IIb', les radicaux R^{1'}, R^{2'}, A', R^{3'}, R^{4'}, R^{7'}, R^{8'}, Q' et D' possédant les significations décrites dans la revendication 13, 14, 15 ou 16.

18. Composés de formules (Ia'), (Ib') ou (Ic') selon l'une quelconque des revendications 13 à 16, 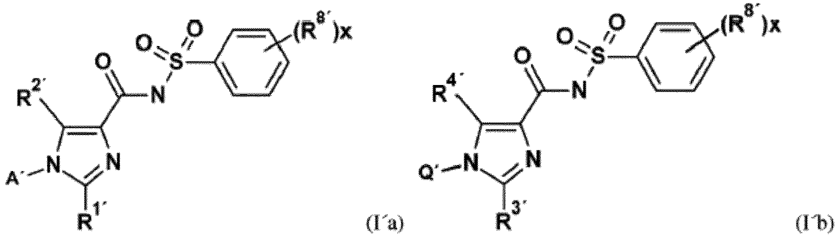 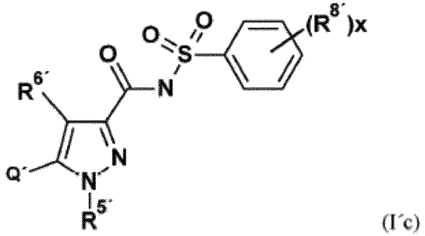 les radicaux R^{1'}, R^{2'}, R^{3'}, R^{4'}, R^{5'}, R^{6'}, R^{7'}, A', Q' et R^{8'} possédant les significations décrites dans la revendication 13, 14, 15 ou 16.

19. Produits intermédiaires de formules (VIa), (Xa) et (IXa), 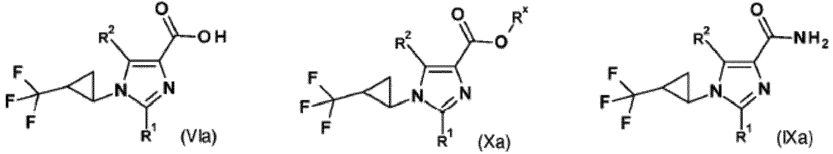 dans lesquelles R¹ et R² possèdent les significations mentionnées dans la revendication 2, 3 ou 4 et R^{x} représente (C₁-C₆)alkyle ou (C₃-C₈)cycloalkyle.
